# EUROPEAN PATENT APPLICATION

(11) **EP 1 798 229 A1**
(43) Date of publication of application: **20.06.2007**
(21) Application number: 05781578.9
(22) Date of filing: 05.09.2005
(51) Int. Cl.: C07D 401/04, A61K 31/4439, A61K 31/496, A61K 31/5377, A61P 1/16, A61P 11/00, A61P 13/12, A61P 17/00, C07D 401/14, C07D 405/14, C07D 409/14, C07D 417/04, C07D 417/14

(54) **SUBSTITUTED BIPHENYL DERIVATIVE**

(30) Priority: 07.09.2004 JP 2004259535
(71) Applicant: Sankyo Company, Limited, Tokyo 103-8426 (JP)
(72) Inventor: KURATA, Hitoshi c/o Sankyo Company Limited, Tokyo 140-8710 (JP); NISHIMATA, Toyoki c/o Sankyo Company Limited, Tokyo 140-8710 (JP); WATANABE, Yukiko c/o Sankyo Company Limited, Tokyo 140-8710 (JP); EBISAWA, Masayuki c/o Sankyo Company Limited, Tokyo 140-8710 (JP); FUJIMOTO, Teppei c/o Sankyo Company Limited, Tokyo 140-8710 (JP); KOBAYASHI, Hideki c/o Sankyo Company Limited, Tokyo 140-8710 (JP)
(74) Representative: Gibson, Christian John Robert
(86) International application number: PCT/JP2005/016212
(87) International publication number: WO 2006/028029

(57) **Abstract**

The present invention relates to a biaryl derivative or a pharmacologically acceptable salt thereof having an excellent collagen-synthesis inhibition activity. A biaryl derivative having a structure represented by the following General Formula (I) or a pharmacologically acceptable salt thereof: 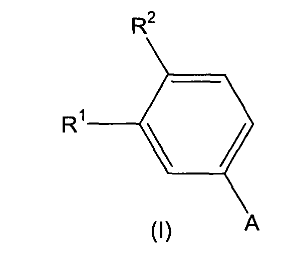 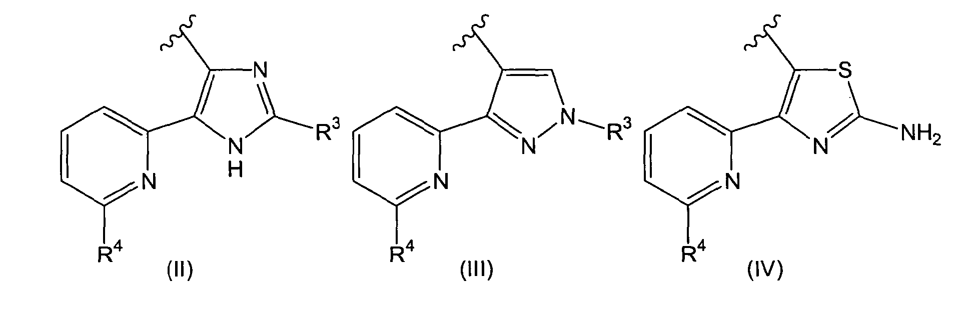 wherein
R¹ represents a C₆-C₁₀ aryl group which is substituted with one to three group(s) each independently selected from the group consisting of a group defined by formula R-L-, a di-(C₁-C₆ alkyl)amino group, a di-(C₁-C₆ alkyl)aminosulfonyl group, a hydroxyaminocarbonyl group, and a halogen atom, and so on; R represents a C₁-C₆ alkyl group, and so on; L represents a sulfonyl group, an aminosulfonyl group, or a sulfonylamino group, and so on; R² represents a hydrogen atom, and so on; A represents a group defined by formula (II), (III), or (IV); R³ represents a C₁-C₆ alkyl group, and so on; and R⁴ represents a C₁-C₆ alkyl group, and so on.

## Description

### Technical Field

The present invention relates to biaryl derivatives or pharmacologically acceptable salts thereof, and pharmaceutical drugs containing the same as an active ingredient. The biaryl derivatives are collagen synthesis inhibitors, and the pharmaceutical drugs are particularly useful for treatment and/or prevention of morbid conditions such as fibrosis that is induced by enhanced production of the extracellular matrix represented by collagen.

### Background Art

Fibrosis is tissue damage due to the accumulation of extracellular fiber or the formation of scars or keloid in the course of repair of tissue injury which is caused by inflammation, high pressure, or high temperature and so on. The tissue damage is caused by excessive generation or suppressed degradation of the extracellular matrix, which is mainly formed by stellate cells of the liver and by fibroblasts and epidermal cells of other organs. These disorders are often intractable, progressive, and irreversible. Hence, establishment of an effective treatment is desired.

The major component of the extracellular matrix in fibrosis is collagen, in particular, collagen type I (Non-Patent Document 1). Collagen is a main component of conjunctive tissue and has a triple helix structure. Collagen is a protein constituting the extracellular matrix to form a high molecular weight assembly. A typical collagen molecule has a triple helix structure formed by three polypeptides. These peptides have a characteristic repeated structure, Gly-X-Y, in which glycine is repeated every three amino acid residues. To date, collagen types I to XX have been reported and are classified into fibrous collagen (type I, II, III, V, and VI), basement membrane collagen (IV), long-chain collagen (VII), short-chain collagen (VIII and X), FACIT collagen (IX, XII, XIV, XVI, and XIX), macrofibrillar collagen (VI), multiplexin (XV and XVIII), and others (VIII and XVII). As factors enhancing collagen synthesis, tumor growth factor (Non-Patent Document 2), oncostatin M (Non-Patent Document 3), interleukin 4 (Non-Patent Document 4), interleukin 6 (Non-Patent Document 5), platelet-derived growth factor (Non-Patent Document 6), connective tissue growth factor (Non-Patent Document 7), histamine (Non-Patent Document 8), and estrogen (Non-Patent Document 9) are known. As factors inhibiting collagen synthesis, tumor necrosis factor α (Non-Patent Document 10), interferon γ (Non-Patent Document 10), interferon α (Non-Patent Document 11), interleukin 1 (Non-Patent Document 12), interleukin 10 (Non-Patent Document 13), basic fibroblast growth factor (Non-Patent Document 14), and glucocorticoid (Non-Patent Document 15) are known.

Activation of collagen synthesis and an increase in the amount of extracellular matrix accompanying the activation are factors causing the occurrence and progress of fibrosis of the kidney, liver, lung, skin, and cardiovascular system.

Overproduction of collagen is involved in the progress of renal fibrosis. Renal fibrosis is a histological change that corresponds with the progress of renal failure. Specifically, renal fibrosis is accumulation of extracellular matrix in the glomus. Collagen is the main component of this accumulated extracellular matrix. Hence, inhibiting collagen production is effective in treating of chronic renal disease.

Overproduction of collagen is involved in the progress of hepatic fibrosis. Hepatic fibrosis is the end-stage of chronic liver disease and causes hepatocellular dysfunction due to excessive fibrous deposition and associated liver cancer complication at a high rate. In hepatic fibrosis, collagen hyperproduction in stellate cells is a principal factor for developing fibrosis. Collagen type I is a main component of the extracellular matrix in fibrosis tissue such as in liver cirrhosis. In liver cirrhosis tissues, collagen type I accounts for 60% to 70% of the total amount of increased collagen. Hence, inhibiting collagen production is effective in treating liver fibrosis and also chronic liver disease.

Overproduction of collagen is involved in lung fibrosis. Lung fibrosis is observed in various inflammatory lung diseases. Lung fibrosis often progresses irreversibly and is therefore a morbid condition for which treatment is crucial. Hence, inhibiting collagen production is effective in treating chronic lung disease.

Overproduction of collagen is involved in skin fibrosis. Examples of skin fibrosis include general scleroderma, local scleroderma, keloid, and discoid lupus erythematosus. These disorders are thought to be caused by an increase in collagen synthesis and a decrease in collagen degradation. Hence, inhibiting collagen production is effective in treating skin fibrosis.

Based on the above-mentioned background, attempts have been made to find compounds having a collagen synthesis inhibiting effect. For example, tranilast (Non-Patent Document 16) and pirfenidone (Non-Patent Document 17) are known as such compounds.
[Non-Patent Document 1]
   Annu. Rev. Biochem. 1980 49: 957.
[Non-Patent Document 2]
   Ann. N. Y. Acad. Sci. 1990 593: 59-72.
[Non-Patent Document 3]
   J. Biol. Chem. 1997 272: 24666-24672.
[Non-Patent Document 4]
   J. Clin. Invest. 1992 90: 1479-1485.
[Non-Patent Document 5]
   Clin. Exp. Immunol. 1994 95: 530-535.
[Non-Patent Document 6]
   Am. J. Pathol. 1996 148: 1169-1180.
[Non-Patent Document 7]
   J. Invest. Dermatol. 1996 107: 404-411.
[Non-Patent Document 8]
   Clin. Exp. Allergy 2002 32: 237-246.
[Non-Patent Document 9]
   DNA 1988 7: 347-354.
[Non-Patent Document 10]
   J. Clin. Invest. 1990 86: 1489-1495.
[Non-Patent Document 11]
   Hepatology 2003 38: 890-899.
[Non-Patent Document 12]
   Biochem. J. 1988 252: 247-255.
[Non-Patent Document 13]
   Int. J. Exp. Pathol. 1997 78: 33-41.
[Non-Patent Document 14]
   Connect Tissue Res. 1991 26: 271-281.
[Non-Patent Document 15]
   Biochemistry 198; ;25 3202-329.
[Non-Patent Document 16]
   Life Sci. 1994 55: PL287-292.
[Non-Patent Document 17]
   Kidney Int. Suppl. 1997 63: S239-243.

### Disclosure of the Invention

### Summary of the Invention

The present inventors have conducted intensive studies on derivatives having a collagen synthesis inhibiting effect and, as a result, have found that biaryl derivatives according to the present invention function as non-peptide inhibitors that strongly and selectively inhibit collagen synthesis and, therefore, are effective in preventing and/or treating morbid conditions (for example, renal disease, liver fibrosis, lung fibrosis, or skin fibrosis mainly caused by fibrosis) that are mainly caused by fibrosis (for example, chronic renal disease, acute renal disease, diabetic renal disorder, liver fibrosis, lung fibrosis, or skin fibrosis). Thus, the present invention has been accomplished.

The present invention relates to (1) a biaryl derivative having General Formula (I) or a pharmacologically acceptable salt thereof, 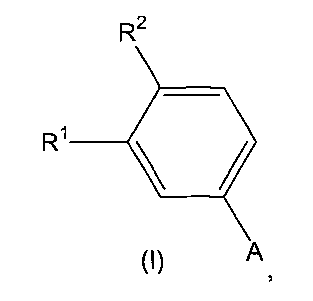 wherein
R¹ represents a C₆-C₁₀ aryl group which is substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, a halogen atom, and a halogenosulfonyl group; or a heterocyclic group which may be substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, a halogen atom, and an oxo group,
R represents a hydrogen atom, a C₁-C₆ alkyl group, a C₁-C₆ halogenated alkyl group, a C₃-C₆ cycloalkyl group, a C₆-C₁₀ aryl group which may be substituted with one to three group(s) each independently selected from substituent group *a*, a heterocyclic group which may be substituted with one group selected from substituent group *a*, a C₁-C₆ alkyl group which is substituted with one group selected from substituent group *b*, a cyano group, a nitro group, a C₁-C₆ alkyl group which is substituted with two hydroxy groups, or a C₁-C₆ alkyl group which is substituted with one to three halogen atom(s) and one hydroxy group,
L represents a single bond, an oxygen atom, an amino group, a sulfur atom, a sulfinyl group, a sulfonyl group, a carbonyl group, an oxycarbonyl group, a carbonyloxy group, an aminocarbonyl group, a carbonylamino group, an aminosulfonyl group, a sulfonylamino group, an aminocarbonylamino group, an aminosulfonylamino group, a hydrazinocarbonylamino group, or an aminocarbonylhydrazino group,
   provided that the case in which R represents a hydrogen atom and L represents a single bond is excluded,
R² represents a hydrogen atom, a C₁-C₆ alkyl group, or a halogen atom,
A represents a group defined by formula (II), (III), or (IV) 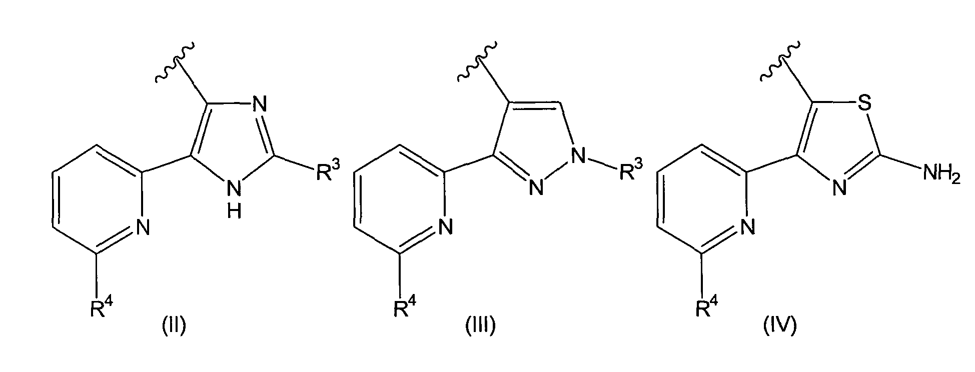
(wherein R³ represents a hydrogen atom, a C₁-C₆ alkyl group, a C₃-C₆ cycloalkyl group, or a C₁-C₆ alkyl group which is substituted with a C₃-C₆ cycloalkyl group, and R⁴ represents a hydrogen atom, a C₁-C₆ alkyl group, or a C₃-C₆ cycloalkyl group),
substituent group *a* represents the group consisting of a halogen atom, a C₁-C₆ alkyl group, and a C₁-C₆ halogenated alkyl group, and
substituent group *b* represents the group consisting of a hydroxy group, an amino group, a carbamoyl group, a C₃-C₆ cycloalkyl group, a C₁-C₆ alkoxy group, a C₂-C₇ alkylcarbonyloxy group, a di-(C₁-C₆ alkyl)amino group, a mono-C₁-C₆ alkylsulfonylamino group, a C₆-C₁₀ aryl group which may be substituted with one to three group(s) each independently selected from substituent group *a*, a heterocyclic group which may be substituted with one group selected from substituent group *a*, a heterocyclic carbonyl group which may be substituted with one group selected from substituent group *a*, a heterocyclic amino group which may be substituted with one group selected from substituent group *a*, a heterocyclic group which is substituted with one oxo group, a C₁-C₆ alkylthio group, a C₁-C₆ alkylsulfinyl group, and a C₁-C₆ alkylsulfonyl group.

The present invention relates, preferably, to
(2) the biaryl derivative or the pharmacologically acceptable salt thereof according to the above (1), in which
   R represents a hydrogen atom, a C₁-C₆ alkyl group, a heterocyclic group which may be substituted with one group selected from substituent group *a,* or a C₁-C₆ alkyl group which is substituted with one group selected from substituent group *b*;
(3) the biaryl derivative or the pharmacologically acceptable salt thereof according to the above (1), in which
   R represents a hydrogen atom, a C₁-C₄ alkyl group, a partially or completely reduced 6-membered heterocyclic group, a C₁-C₆ alkyl group which is substituted with one partially or completely reduced 5-membered heterocyclic group, or a C₁-C₆ alkyl group which is substituted with one hydroxy group;
(4) the biaryl derivative or the pharmacologically acceptable salt thereof according to the above (1), in which
   R represents a hydrogen atom, a methyl group, an ethyl group, a hydroxymethyl group, a 1-hydroxy-1-methylethyl group, or a 2-hydroxyethyl group;
(5) the biaryl derivative or the pharmacologically acceptable salt thereof according to the above (1), in which
   R represents a hydrogen atom, a C₁-C₄ alkyl group, a C₁-C₄ halogenated alkyl group, a C₃-C₄ cycloalkyl group, a phenyl group which may be substituted with one group selected from substituent group *a*, a partially or completely reduced 6-membered heterocyclic group which may be substituted with one group selected from substituent group *a*, a C₁-C₄ alkyl group which is substituted with one group selected from substituent group *b*, or a nitro group;
(6) the biaryl derivative or the pharmacologically acceptable salt thereof according to the above (1), in which
   R represents a hydrogen atom; a methyl group; an ethyl group; a trifluoromethyl group; a cyclopropyl group; a morpholino, piperazinyl, or tetrahydropyranyl group which may be substituted with one group selected from a fluorine atom, a chlorine atom, a methyl group, and an ethyl group; or a C₁-C₄ alkyl group which is substituted with one group selected from a hydroxy group and a C₂-C₇ alkylcarbonyloxy group;
(7) the biaryl derivative or the pharmacologically acceptable salt thereof according to the above (1), in which
   R represents a hydrogen atom, a methyl group, an ethyl group, a cyclopropyl group, a 4-morpholino group, a 4-methyl-1-piperazinyl group, a 4-tetrahydropyranyl group, a 1-hydroxy-1-methylethyl group, a 1-acetoxy-1-methylethyl group, or a 2-hydroxyethyl group;
(8) the biaryl derivative or the pharmacologically acceptable salt thereof according to any one of the above (1) to (7), in which
   L represents a single bond, an oxygen atom, an amino group, a sulfur atom, a sulfinyl group, a sulfonyl group, a carbonyl group, an oxycarbonyl group, an aminocarbonyl group, a carbonylamino group, an aminosulfonyl group, a sulfonylamino group, or an aminocarbonylamino group;
(9) the biaryl derivative or the pharmacologically acceptable salt thereof according to any one of the above (1) to (7), in which
   L represents an oxygen atom, an amino group, a sulfur atom, a sulfinyl group, a sulfonyl group, a carbonyl group, an aminocarbonyl group, a carbonylamino group, an aminosulfonyl group, a sulfonylamino group, or an aminocarbonylamino group;
(10) the biaryl derivative or the pharmacologically acceptable salt thereof according to any one of the above (1) to (7), in which
   L represents an oxygen atom, an amino group, a sulfur atom, a sulfinyl group, a sulfonyl group, an aminocarbonyl group, an aminosulfonyl group, a sulfonylamino group, or an aminocarbonylamino group;
(11) the biaryl derivative or the pharmacologically acceptable salt thereof according to any one of the above (1) to (7), in which
   L represents a single bond, an oxygen atom, a sulfonyl group, an aminocarbonyl group, or an aminosulfonyl group;
(12) the biaryl derivative or the pharmacologically acceptable salt thereof according to any one of the above (1) to (11), in which
   R¹ represents a C₆-C₁₀ aryl group which is substituted with one to three group(s) each independently selected from the group consisting of a group defined by formula R-L- and a di-(C₁-C₆ alkyl)amino group; or a heterocyclic group which may be substituted with one to three group(s) each independently selected from a group defined by formula R-L-;
(13) the biaryl derivative or the pharmacologically acceptable salt thereof according to any one of the above (1) to (11), in which
   R¹ represents a C₆-C₁₀ aryl group which is substituted with one group selected from the group consisting of a group defined by formula R-L- and a di-(C₁-C₆ alkyl)amino group; or a 5-membered aromatic heterocyclic, 6-membered aromatic heterocyclic, or fused bicyclic heterocyclic group which may be substituted with one group selected from a group defined by formula R-L-;
(14) the biaryl derivative or the pharmacologically acceptable salt thereof according to any one of the above (1) to (11), in which
   R¹ represents a phenyl group of which the 4-position is substituted with one group selected from the group consisting of a group defined by formula R-L- and a dimethylamino group; or a thienyl, pyrrolyl, pyrazolyl, imidazolyl, pyridyl, or 1,1-dioxido-2,3-dihydro-1-benzothienyl group which may be substituted with one group selected from a group defined by formula R-L-;
(15) the biaryl derivative or the pharmacologically acceptable salt thereof according to any one of the above (1) to (11), in which
   R¹ represents a 4-methylsulfonylphenyl group, a 4-aminosulfonylphenyl group, a 4-methylaminosulfonylphenyl group, a 4-(4-morpholino)aminocarbonylphenyl group, a 4-[2-(1-pyrrolidinyl)ethoxy]phenyl group, a 4-dimethylaminosulfonylphenyl group, a 5 -amino sulfonyl-2-thienyl group, a 2-pyrrolyl group, a 3-pyrrolyl group, a 4-pyrazolyl group, a 1-methyl-4-pyrazolyl group, a 1-ethyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 2-(2-hydroxyethyl)-5-imidazolyl group, a 5-hydroxymethyl-2-pyridyl group, a 5-(1-hydroxy-1-methylethyl)-2-pyridyl group, a 5-(4-morpholino)-2-pyridyl group, or a 1,1-dioxido-2,3-dihydro-1-benzothien-5-yl group;
(16) the biaryl derivative or the pharmacologically acceptable salt thereof according to any one of the above (1) to (11), in which
   R¹ represents a 4-methylsulfonylphenyl group, a 4-aminosulfonylphenyl group, a 4-methylaminosulfonylphenyl group, a 5-aminosulfonyl-2-thienyl group, a 3-pyrrolyl group, a 1-methyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 5-(1-hydroxy-1-methylethyl)-2-pyridyl group, or a 1,1-dioxido-2,3-dihydro-1-benzothien-5-yl group;
(17) the biaryl derivative or the pharmacologically acceptable salt thereof according to any one of the above (1) to (11), in which
   R¹ represents a C₆-C₁₀ aryl group which is substituted with one or two group(s) selected from the group consisting of a group defined by formula R-L-, a di-(C₁-C₄ alkyl)amino group, a di-(C₁-C₄ alkyl)aminosulfonyl group, a hydroxyaminocarbonyl group, and a halogen atom; or a heterocyclic group which may be substituted with one group selected from the group consisting of a group defined by formula R-L-, a di-(C₁-C₆ alkyl)amino group, a di-(C₁-C₆ alkyl)aminosulfonyl group, a hydroxyaminocarbonyl group, and a halogen atom;
(18) the biaryl derivative or the pharmacologically acceptable salt thereof according to any one of the above (1) to (11), in which
   R¹ represents a phenyl group of which the 4- or 3-position is substituted with one group selected from the group consisting of a group defined by formula R-L-, a di-(C₁-C₂ alkyl)aminosulfonyl group, and a halogen atom; or a thienyl, pyrrolyl, furyl, or pyridyl group which may be substituted with one group selected from the group consisting of a group defined by formula R-L-, a di-(C₁-C₂ alkyl)aminosulfonyl group, and a halogen atom;
(19) the biaryl derivative or the pharmacologically acceptable salt thereof according to any one of the above (1) to (11), in which
   R¹ represents a phenyl group of which the 4-position is substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group; or a thienyl, pyrrolyl, furyl, or pyridyl group which may be substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group;
(20) the biaryl derivative or the pharmacologically acceptable salt thereof according to any one of the above (1) to (11), in which
   R¹ represents a 4-methoxyphenyl group, a 4-aminophenyl group, a 4-methylthiophenyl group, a 4-methylsulfinylphenyl group, a 4-methylsulfonylphenyl group, a 4-ethylsulfonylphenyl group, a 4-acetylaminophenyl group, a 4-(1-hydroxy-1-methylethyl)carbonylaminophenyl group, a 4-(1-acetoxy-1-methylethyl)carbonylaminophenyl group, a 4-carbamoylphenyl group, a 3-carbamoylphenyl group, a 4-(2-hydroxyethyl)aminocarbonylphenyl group, a 4-methylsulfonylaminophenyl group, a 4-ethylsulfonylaminophenyl group, a 4-cyclopropylsulfonylaminophenyl group, a 4-aminosulfonylphenyl group, a 4-methylaminosulfonylphenyl group, a 4-(4-morpholino)carbonylphenyl group, a 4-(4-morpholino)sulfonylphenyl group, a 4-(4-morpholino)aminocarbonylphenyl group, a 4-(4-methyl-1-piperazinyl)aminocarbonylphenyl group, a 4-(4-methyl-1-piperazinyl)sulfonylphenyl group, a 4-(4-tetrahydropyranyl)aminocarbonylphenyl group, a 4-(1-methyl-4-piperidino)aminocarbonylphenyl group, a 4-(4-morpholino)aminocarbonylaminophenyl group, a 4-(4-methyl-1-piperazinyl)aminocarbonylaminophenyl group, a 3-thienyl group, a 2-pyrrolyl group, a 3-furyl group, a 5-carbamoyl-2-pyridyl group, a 2-methoxy-5-pyridyl group, or a 4-pyridyl group;
(21) the biaryl derivative or the pharmacologically acceptable salt thereof according to any one of the above (1) to (11), in which
   R¹ represents a 4-methoxyphenyl group, a 4-aminophenyl group, a 4-methylthiophenyl group, a 4-methylsulfinylphenyl group, a 4-methylsulfonylphenyl group, a 4-methylsulfonylaminophenyl group, a 4-ethylsulfonylaminophenyl group, a 4-cyclopropylsulfonylaminophenyl group, a 4-aminosulfonylphenyl group, a 4-(4-morpholino)aminocarbonylphenyl group, a 4-(4-methyl-1-piperazinyl)aminocarbonylphenyl group, a 4-(4-tetrahydropyranyl)aminocarbonylphenyl group, a 4-(1-methyl-4-piperidino)aminocarbonylphenyl group, a 4-(4-morpholino)aminocarbonylaminophenyl group, a 4-(4-methyl-1-piperazinyl)aminocarbonylaminophenyl group, a 3-thienyl group, a 2-pyrrolyl group, or a 5-carbamoyl-2-pyridyl group;
(22) the biaryl derivative or the pharmacologically acceptable salt thereof according to any one of the above (1) to (21), in which
   R² represents a hydrogen atom, a methyl group, a fluorine atom, or a chlorine atom;
(23) the biaryl derivative or the pharmacologically acceptable salt thereof according to any one of the above (1) to (21), in which
   R² represents a hydrogen atom or a fluorine atom;
(24) the biaryl derivative or the pharmacologically acceptable salt thereof according to any one of the above (1) to (21), in which
   R² represents a fluorine atom;
(25) the biaryl derivative or the pharmacologically acceptable salt thereof according to any one of the above (1) to (24), in which
   A represents a group defined by formula (II);
(26) the biaryl derivative or the pharmacologically acceptable salt thereof according to any one of the above (1) to (24), in which
   A represents a group defined by formula (III);
(27) the biaryl derivative or the pharmacologically acceptable salt thereof according to any one of the above (1) to (26), in which
   R³ represents a C₁-C₆ alkyl group or a C₃-C₆ cycloalkyl group;
(28) the biaryl derivative or the pharmacologically acceptable salt thereof according to any one of the above (1) to (26), in which
   R³ represents an isopropyl group, an isobutyl group, or a cyclopropyl group;
(29) the biaryl derivative or the pharmacologically acceptable salt thereof according to any one of the above (1) to (26), in which
   R³ represents an isopropyl group;
(30) the biaryl derivative or the pharmacologically acceptable salt thereof according to any one of the above (1) to (26), in which
   R³ represents a hydrogen atom;
(31) the biaryl derivative or the pharmacologically acceptable salt thereof according to any one of the above (1) to (30), in which
   R⁴ represents a hydrogen atom or a methyl group;
(32) the biaryl derivative or the pharmacologically acceptable salt thereof according to any one of the above (1) to (30), in which
   R⁴ represents a methyl group;
(33) the biaryl derivative or the pharmacologically acceptable salt thereof according to the above (1), in which
   R¹ represents a C₆-C₁₀ aryl group which is substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, and a halogen atom; or a heterocyclic group which may be substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, and a halogen atom, R represents a hydrogen atom, a C₁-C₆ alkyl group, a C₁-C₆ halogenated alkyl group, a C₃-C₆ cycloalkyl group, a C₆-C₁₀ aryl group which may be substituted with one to three group(s) each independently selected from substituent group *a*, a heterocyclic group which may be substituted with one group selected from substituent group *a*, a C₁-C₆ alkyl group which is substituted with one group selected from substituent group *b*, a cyano group, or a nitro group, L represents a single bond, an oxygen atom, an amino group, a sulfur atom, a sulfinyl group, a sulfonyl group, a carbonyl group, an oxycarbonyl group, a carbonyloxy group, an aminocarbonyl group, a carbonylamino group, an aminosulfonyl group, a sulfonylamino group, an aminocarbonylamino group, an aminosulfonylamino group, a hydrazinocarbonylamino group, or an aminocarbonylhydrazino group, provided that the case in which R represents a hydrogen atom and L represents a single bond is excluded; R² represents a hydrogen atom, a C₁-C₆ alkyl group, or a halogen atom, A represents a group defined by formula (II), (III), or (IV), R³ represents a hydrogen atom, a C₁-C₆ alkyl group, a C₃-C₆ cycloalkyl group, or a C₁-C₆ alkyl group which is substituted with a C₃-C₆ cycloalkyl group, R⁴ represents a hydrogen atom or a C₁-C₆ alkyl group, substituent group *a* is the group consisting of a halogen atom, a C₁-C₆ alkyl group, and a C₁-C₆ halogenated alkyl group, and substituent group *b* is the group consisting of a hydroxy group, an amino group, a carbamoyl group, a C₃-C₆ cycloalkyl group, a C₁-C₆ alkoxy group, a C₂-C₇ alkylcarbonyloxy group, a di-(C₁-C₆ alkyl)amino group, a mono-C₁-C₆ alkylsulfonylamino group, a C₆-C₁₀ aryl group which may be substituted with one to three group(s) each independently selected from substituent group *a,* a heterocyclic group which may be substituted with one group selected from substituent group *a,* and a heterocyclic carbonyl group which may be substituted with one group selected from substituent group *a;*
(34) the biaryl derivative or the pharmacologically acceptable salt thereof according to the above (1), in which
   R¹ represents a C₆-C₁₀ aryl group which is substituted with one group selected from the group consisting of a group defined by formula R-L- and a di-(C₁-C₆ alkyl)amino group, or a 5-membered aromatic heterocyclic, 6-membered aromatic heterocyclic, or fused bicyclic heterocyclic group which may be substituted with one group selected from a group defined by formula R-L-, R² represents a hydrogen atom or a fluorine atom, A represents a group defined by formula (II) or (III), R³ represents an isopropyl group, an isobutyl group, a cyclopropyl group, or a hydrogen atom, and R⁴ represents a hydrogen atom or a methyl group;
(35) the biaryl derivative or the pharmacologically acceptable salt thereof according to the above (1), in which
   R¹ represents a phenyl group of which the 4-position is substituted with one group selected from the group consisting of a group defined by formula R-L- and a dimethylamino group, or a thienyl, pyrrolyl, pyrazolyl, imidazolyl, pyridyl, or 1,1-dioxido-2,3-dihydro-1-benzothienyl group which may be substituted with one group selected from a group defined by formula R-L-, R² represents a fluorine atom, A represents a group defined by formula (II) or (III), R³ represents an isopropyl group or a hydrogen atom, and R⁴ represents a methyl group;
(36) the biaryl derivative or the pharmacologically acceptable salt thereof according to the above (1), in which
   R¹ represents a 4-methylsulfonylphenyl group, a 4-aminosulfonylphenyl group, a 4-methylaminosulfonylphenyl group, a 4-(4-morpholino)aminocarbonylphenyl group, a 4-[2-(1-pyrrolidinyl)ethoxy]phenyl group, a 4-dimethylaminosulfonylphenyl group, a 5-aminosulfonyl-2-thienyl group, a 2-pyrrolyl group, a 3-pyrrolyl group, a 4-pyrazolyl group, a 1-methyl-4-pyrazolyl group, a 1-ethyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 2-(2-hydroxyethyl)-5-imidazolyl group, a 5-hydroxymethyl-2-pyridyl group, a 5-(1-hydroxy-1-methylethyl)-2-pyridyl group, a 5-(4-morpholino)-2-pyridyl group, or a 1,1-dioxido-2,3-dihydro-1-benzothien-5-yl group, R² represents a fluorine atom, A represents a group defined by formula (III), R³ represents a hydrogen atom, and R⁴ represents a methyl group;
(37) the biaryl derivative or the pharmacologically acceptable salt thereof according to the above (1), in which
   R¹ represents a 4-methylsulfonylphenyl group, a 4-aminosulfonylphenyl group, a 4-methylaminosulfonylphenyl group, a 5-aminosulfonyl-2-thienyl group, a 3-pyrrolyl group, a 1-methyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 5-(1-hydroxy-1-methylethyl)-2-pyridyl group, or a 1,1-dioxido-2,3-dihydro-1-benzothien-5-yl group, R² represents a fluorine atom, A represents a group defined by formula (III), R³ represents a hydrogen atom, and R⁴ represents a methyl group;
(38) the biaryl derivative or the pharmacologically acceptable salt thereof according to the above (1), in which
   R¹ represents a phenyl group of which the 4-position is substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group, or a thienyl, pyrrolyl, furyl, or pyridyl group which may be substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group, R² represents a hydrogen atom, a methyl group, a fluorine atom, or a chlorine atom, A represents a group defined by formula (II), (III), or (IV), R³ represents an isopropyl group, an isobutyl group, or a cyclopropyl group, and R⁴ represents a hydrogen atom or a methyl group;
(39) the biaryl derivative or the pharmacologically acceptable salt thereof according to the above (1), in which
   R¹ represents a phenyl group of which the 4-position is substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group, or a thienyl, pyrrolyl, furyl, or pyridyl group which may be substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group, R² represents a hydrogen atom or a fluorine atom, A represents a group defined by formula (II), R³ represents an isopropyl group, and R⁴ represents a methyl group;
(40) the biaryl derivative or the pharmacologically acceptable salt thereof according to the above (1), in which
   R¹ represents a 4-methoxyphenyl group, a 4-aminophenyl group, a 4-methylthiophenyl group, a 4-methylsulfinylphenyl group, a 4-methylsulfonylphenyl group, a 4-ethylsulfonylphenyl group, a 4-acetylaminophenyl group, a 4-(1-hydroxy-1-methylethyl)carbonylaminophenyl group, a 4-(1-acetoxy-1-methylethyl)carbonylaminophenyl group, a 4-carbamoylphenyl group, a 3-carbamoylphenyl group, a 4-(2-hydroxyethyl)aminocarbonylphenyl group, a 4-methylsulfonylaminophenyl group, a 4-ethylsulfonylaminophenyl group, a 4-cyclopropylsulfonylaminophenyl group, a 4-aminosulfonylphenyl group, a 4-methylaminosulfonylphenyl group, a 4-(4-morpholino)carbonylphenyl group, a 4-(4-morpholino)sulfonylphenyl group, a 4-(4-morpholino)aminocarbonylphenyl group, a 4-(4-methyl-1-piperazinyl)aminocarbonylphenyl group, a 4-(4-methyl-1-piperazinyl)sulfonylphenyl group, a 4-(4-tetrahydropyranyl)aminocarbonylphenyl group, a 4-(1-methyl-4-piperidino)aminocarbonylphenyl group, a 4-(4-morpholino)aminocarbonylaminophenyl group, a 4-(4-methyl-1-piperazinyl)aminocarbonylaminophenyl group, a 3-thienyl group, a 2-pyrrolyl group, a 3-furyl group, a 5-carbamoyl-2-pyridyl group, a 2-methoxy-5-pyridyl group, or a 4-pyridyl group, R² represents a hydrogen atom or a fluorine atom, A represents a group defined by formula (II), R³ represents an isopropyl group, and R⁴ represents a methyl group;
(41) the biaryl derivative or the pharmacologically acceptable salt thereof according to the above (1), in which
   R¹ represents a 4-methoxyphenyl group, a 4-aminophenyl group, a 4-methylthiophenyl group, a 4-methylsulfinylphenyl group, a 4-methylsulfonylphenyl group, a 4-methylsulfonylaminophenyl group, a 4-ethylsulfonylaminophenyl group, a 4-cyclopropylsulfonylaminophenyl group, a 4-aminosulfonylphenyl group, a 4-(4-morpholino)aminocarbonylphenyl group, a 4-(4-methyl-1-piperazinyl)aminocarbonylphenyl group, a 4-(4-tetrahydropyranyl)aminocarbonylphenyl group, a 4-(1-methyl-4-piperidino)aminocarbonylphenyl group, a 4-(4-morpholino)aminocarbonylaminophenyl group, a 4-(4-methyl-1-piperazinyl)aminocarbonylaminophenyl group, a 3-thienyl group, a 2-pyrrolyl group, or a 5-carbamoyl-2-pyridyl group, R² represents a hydrogen atom or a fluorine atom, A represents a group defined by formula (II), R³ represents an isopropyl group, and R⁴ represents a methyl group;
(42) the biaryl derivative or the pharmacologically acceptable salt thereof according to the above (1) which is
   2-{4-[6-fluoro-4'-(methylsulfonyl)-1,1'-biphenyl-3-yl]-2-isopropyl-1H-imidazol-5-yl} -6-methylpyridine,
   2'-fluoro-5'-[2-isopropyl-5-(6-methylpyridin-2-yl)-1H-imidazol-4-yl]-1,1'-biphenyl-4-sulfonamide,
   N-(morpholin-4-yl)-4- {2-fluoro-5-[2-isopropyl-5-(6-methylpyridin-2-yl)-1H-imidazol-4-yl]-phenyl}benzamide,
   2-{4-[4-fluoro-3-(1H-pyrrol-2-yl)phenyl]-2-isopropyl-1H-imidazol-5-yl}-6-methylpyridine,
   2-{4-[4-fluoro-3-(1H-pyrrol-3-yl)phenyl]-2-isopropyl-1H-imidazol-5-yl}-6-methylpyridine,
   2-{4-[6-fluoro-4'-(2-pyrrolidin-1-ylethoxy)-1,1'-biphenyl-3-yl]-2-isopropyl-1H-imidazol-5-yl} -6-methylpyridine,
   2-{5-[4-fluoro-3-(1H-pyrazol-4-yl)phenyl]-2-isopropyl-1H-pyrazol-4-yl}-6-methylpyridine,
   2-{5-[4-fluoro-3-(1-methyl-1H-pyrazol-4-yl)phenyl]-2-isopropyl-1H-imidazol-4-yl}-6-methylpyridine,
   2-{5-[3-(1-ethyl-1H-pyrazol-4-yl)-4-fluorophenyl]-2-isopropyl-1H-imidazol-4-yl}-6-methylpyridine,
   2-{4-[4-fluoro-3-(1H-imidazol-4-yl)phenyl]-2-isopropyl-1H-imidazol-5-yl}-6-methylpyridine,
   2-{4-[4-fluoro-3-(1-methyl-1H-imidazol-4-yl)phenyl]-2-isopropyl-1H-imidazol-5-yl} -6-methylpyridine,
   2-{4-[6-fluoro-4'-(methylsulfinyl)-1,1'-biphenyl-3-yl]-1H-pyrazol-3-yl}-6-methylpyridine,
   2'-fluoro-5'-[3-(6-methylpyridin-2-yl)-1H-pyrazol-4-yl]-1,1'-biphenyl-4-sulfonamide,
   2'-fluoro-N-methyl-5'-[3-(6-methylpyridin-2-yl)-1H-pyrazol-4-yl]-1,1'-biphenyl-4-sulfonamide,
   2-(4-[4-fluoro-3-(1H-pyrazol-4-yl)phenyl]-1H-pyrazol-3-yl)-6-methylpyridine,
   2- {4-[4-fluoro-3-(1-methyl-1 H-pyrazol-4-yl)phenyl]-1 H-pyrazol-3-yl} -6-methylpyridine,
   2-{4-[4-fluoro-3-(1H-imidazol-4-yl)phenyl]-1H-pyrazol-3-yl}-6-methylpyridine, (4- {2-fluoro-5-[3-(6-methylpyridin-2-yl)-1 H-pyrazol-4-yl]phenyl } -1 H-imidazol-2-yl)methanol,
   2-{4-[4-fluoro-3-(1-methyl-1H-pyrazol-4-yl)phenyl]-1H-pyrazol-3-yl}pyridine, 5- {2-fluoro-5-[3-(6-methylpyridin-2-yl)-1H-pyrazol-4-yl]phenyl}thiophene-2-sulfonamide,
   2'-fluoro-N,N-dimethyl-5'-[3-(6-methylpyridin-2-yl)-1H-pyrazol-4-yl]-1,1'-biphenyl-4-sulfonamide,
   (6- {2-fluoro-5-[3-(6-methylpyridin-2-yl)-1H-pyrazol-4-yl]phenyl}pyridin-3-yl)methanol,
   2-(6- {2-fluoro-5-[3-(6-methylpyridin-2-yl)-1H-pyrazol-4-yl]phenyl}pyridin-3-yl)propan-2-ol,
   2-(4- {2-fluoro-5-[3-(6-methylpyridin-2-yl)-1H-pyrazol-4-yl]phenyl}- 1H-imidazol-2-yl)propan-2-ol,
   2-(4-{2-fluoro-5-[3-(6-methylpyridin-2-yl)-1H-pyrazol-4-yl]phenyl}-1H-imidazol-2-yl)ethanol,
   2-(4-{2-fluoro-5-[3-(6-methylpyridin-2-yl)-1H-pyrazol-4-yl]phenyl}-1H-pyrazol-1-yl)ethanol,
   4-(6-{2-fluoro-5-[3-(6-methylpyridin-2-yl)-1H-pyrazol-4-yl]phenyl}pyridin-3-yl)morpholine,
   2- {4-[4-fluoro-3-(1-methyl-1H-imidazol-4-yl)phenyl]-1H-pyrazol-3-yl}-6-methylpyridine,
   2- {4-[3-(1,1-dioxido-2,3-dihydro-1-benzothien-5-yl)-4-fluorophenyl]-1 H-pyrazol-3-yl}-6-methylpyridine, or
   5-[4-fluoro-3-(1-methyl-1H-pyrazol-4-yl)phenyl]-4-(6-methylpyridin-2-yl)-1,3-thiazole-2-amine;
(43) the biaryl derivative or the pharmacologically acceptable salt thereof according to the above (1) which is
   2-{4-[4-fluoro-3-(1H-pyrrol-3-yl)phenyl]-2-isopropyl-1H-imidazol-5-yl}-6-methylpyridine,
   2-{5-[4-fluoro-3-(1-methyl-1H-pyrazol-4-yl)phenyl] -2-isopropyl-1H-imidazol-4-yl}-6-methylpyridine,
   2-{4-[4-fluoro-3-(1H-imidazol-4-yl)phenyl]-2-isopropyl-1H-imidazol-5-yl} -6-methylpyridine,
   2-{4-[4-fluoro-3-(1-methyl-1H-imidazol-4-yl)phenyl]-2-isopropyl-1H-imidazol-5-yl}-6-methylpyridine,
   2-{4-[6-fluoro-4'-(methylsulfonyl)-1,1'-biphenyl-3-yl]-1H-pyrazol-3-yl} -6-methylpyridine,
   2'-fluoro-5'-[3-(6-methylpyridin-2-yl)-1H-pyrazol-4-yl]-1,1'-biphenyl-4-sulfonamide,
   2'-fluoro-N-methyl-5'-[3-(6-methylpyridin-2-yl)-1H-pyrazol-4-yl]-1,1'-biphenyl-4-sulfonamide,
   2-(4-[4-fluoro-3-(1-methyl-1H-pyrazol-4-yl)phenyl]-1H-pyrazol-3-yl)-6-methylpyridine,
   (4-{2-fluoro-5-[3-(6-methylpyridin-2-yl)-1H-pyrazol-4-yl]phenyl}-1H-imidazol-2-yl)methanol,
   5-{2-fluoro-5-[3-(6-methylpyridin-2-yl)-1H-pyrazol-4-yl]phenyl}thiophene-2-sulfonamide,
   2-(6-{2-fluoro-5-[3-(6-methylpyridin-2-yl)-1H-pyrazol-4-yl]phenyl}pyridin-3-yl)propan-2-ol,
   2-(4-{2-fluoro-5-[3-(6-methylpyridin-2-yl)-1H-pyrazol-4-yl]phenyl}-1H-imidazol-2-yl)propan-2-ol,
   2-(4-{2-fluoro-5-[3-(6-methylpyridin-2-yl)-1H-pyrazol-4-yl]phenyl}-1H-pyrazol-1-yl)ethanol,
   2-{4-[4-fluoro-3-(1-methyl-1H-imidazol-4-yl)phenyl]-1H-pyrazol-3-yl}-6-methylpyridine, or
   2-{4-[3-(1,1-dioxido-2,3-dihydro-1-benzothien-5-yl)-4-fluorophenyl]-1H-pyrazol-3-yl}-6-methylpyridine;
(44) the biaryl derivative or the pharmacologically acceptable salt thereof according to the above (1) which is
   2-[2-isopropyl-4-(4'-methoxy-1,1'-biphenyl-3-yl)-1H-imidazol-5-yl]-6-methylpyridine,
   2-[2-isopropyl-4-(4'-amino-1,1'-biphenyl-3-yl)-1H-imidazol-5-yl]-6-methylpyridine,
   2-{2-isopropyl-4-[4'-(methylthio)-1,1'-biphenyl-3-yl]-1H-imidazol-5-yl}-6-methylpyridine,
   2-{2-isopropyl-4-[4'-(methylsulfoxy)-1,1'-biphenyl-3-yl]-1H-imidazol-5-yl} -6-methylpyridine,
   2-{2-isopropyl-4-[4'-(methylsulfonyl)-1,1'-biphenyl-3-yl]-1H-imidazol-5-yl} -6-methylpyridine,
   2-{4-[6-fluoro-4'-(methylsulfonyl)-1,1'-biphenyl-3-yl]-2-isopropyl-1H-imidazol-5-yl}-6-methylpyridine,
   N-{3'-[2-isopropyl-5-(6-methylpyridin-2-yl)-1H-imidazol-4-yl]-1,1'-biphenyl-4-yl}methanesulfonamide,
   N-{3'-[2-isopropyl-5-(6-methylpyridin-2-yl)-1H-imidazol-4-yl]-1,1'-biphenyl-4-yl}ethanesulfonamide,
   N- {3'-[2-isopropyl-5-(6-methylpyridin-2-yl)-1H-imidazol-4-yl]-1,1'-biphenyl-4-yl} cyclopropylsulfonamide,
   N- {3'-[2-cylopropyl-5-(6-methylpyridin-2-yl)-1H-imidazol-4-yl]-1,1'-biphenyl-4-yl} cyclopropylsulfonamide,
   N- {2'-fluoro-5'-[2-isopropyl-5-(6-methylpyridin-2-yl)-1H-imidazol-4-yl]-1,1'-biphenyl-4-yl} cyclopropylsulfonamide,
   3'-[2-isopropyl-4-(6-methylpyridin-2-yl)-1H-imidazol-5-yl]-1,1'-biphenyl-4-sulfonamide,
   N-(morpholin-4-yl)-4-{3-[2-isopropyl-5-(6-methylpyridin-2-yl)-1H-imidazol-4-yl]-phenyl}benzamide,
   N-(morpholin-4-yl)-4-{2-fluoro-5-[2-isopropyl-5-(6-methylpyridin-2-yl)-1H-imidazol-4-yl]-phenyl}benzamide,
   N-(4-methylpiperazin-1-yl)-4-{3-[2-isopropyl-5-(6-methylpyridin-2-yl)-1H-imidazol-4-yl]phenyl}benzamide,
   2'-fluoro-5'-[2-isopropyl-5-(6-methylpyridin-2-yl)-1H-imidazol-4-yl]-N-(4-methylpiperazin-1-yl)-1,1'-biphenyl-4-carboxyamide,
   2-{2-isopropyl-4-[3-(thiophen-3-yl)phenyl]-1 H-imidazol-5-yl}-6-methylpyridine,
   2-(2-isopropyl-4-[3-(1H-pyrrol-2-yl)phenyl]-1H-imidazol-5-yl)-6-methylpyridine,
   6-{3-[2-isopropyl-5-(6-methylpyridin-2-yl)-1H-imidazol-4-yl]phenyl}nicotinamide, 1-({3'-[2-isopropyl-5-(6-methylpyridin-2-yl)-1H-imidazol-4-yl]-1,1'-biphenyl-4-yl}sulfonyl)-4-methylpiperazine,
   4-({2'-fluoro-5'-[2-isopropyl-5-(6-methylpyridin-2-yl)-1H-imidazol-4-yl]-1,1'-biphenyl-4-yl}methyl)morpholine,
   N-{3'-[2-isopropyl-5-(6-methylpyridm-2-yl)-1H-imidazol-4-yl]-1,1'-biphenyl-4-yl}-N'-(methylpiperazin-1-yl)urea,
   N-{2'-fluoro-5'-[2-isopropyl-5-(6-methylpyridin-2-yl)-1H-imidazol-4-yl]-1,1'-biphenyl-4-yl}-N'-(4-methylpiperazin-1-yl)urea,
   N-(tetrahydropyran-4-yl)-4-{2-fluoro-5-[2-isopropyl-5-(6-methylpyridin-2-yl)-1H-imidazol-4-yl]phenyl}benzamide,
   N- {3'-[2-isopropyl-5-(6-methylpyridin-2-yl)-1H-imidazol-4-yl]-1,1'-biphenyl-4-yl} - N'-morpholin-4-ylurea,
   N-{2'-fluoro-5'-[2-isopropyl-5-(6-methylpyridin-2-yl)-1H-imidazol-4-yl]-1,1'-biphenyl-4-yl}-N'-motpholin-4-ylurea,
   3'-[2-isopropyl-5-(6-methylpyridin-2-yl)-1H-imidazol-4-yl]-N-(1-methylpiperidin-4-yl)-1,1'-biphenyl-4-carboxyamide,
   2'-fluoro-5'-[2-isopropyl-5-(6-methylpyridin-2-yl)-1H-imidazol-4-yl]-N-(1-methylpiperidin-4-yl)-1,1'-biphenyl-4-carboxyamide,
   N- {3'-[2-isopropyl-5-(6-methylpyridin-2-yl)-1H-imidazol-4-yl]-1,1'-biphenyl-4-yl}-N'-(1-methylpiperidin-4-yl)urea,
   N-{2'-fluoro-5'-[2-isopropyl-5-(6-methylpyridin-2-yl)-1H-imidazol-4-yl]-1,1'-biphenyl-4-yl}-N'-(1-methylpiperidin-4-yl)urea,
   2-{4-[6-fluoro-4'-(methylsulfonyl)-1,1'-biphenyl-3-yl]-1H-pyrazol-3-yl}-6-methylpyridine,
   N-{2'-fluoro-5'-[3-(6-methylpyridin-2-yl)-1H-pyrazol-4-yl]-1,1'-biphenyl-4-yl}cyclopropanesulfonamide,
   2'-fluoro-N-(4-methylpiperazin-1-yl)-5'-[3-(6-methylpyridin-2-yl)-1H-pyrazol-4-yl]-1,1'-biphenyl-4-carboxyamide,
   N-{2'-fluoro-5'-[3-(6-methylpyridin-2-yl)-1H-pyrazol-4-yl]-1,1'-biphenyl-4-yl}-N'-morpholin-4-ylurea,
   N-{2'-fluoro-5'-[3-(6-methylpyridin-2-yl)-1H-pyrazol-4-yl]-1,1'-biphenyl-4-yl}-N'-(4-methylpiperazin-1-yl)urea, or
   4-({2'-fluoro-5'-[3-(6-methylpyridin-2-yl)-1H-pyrazol-4-yl]-1,1'-biphenyl-4-yl}methyl)morpholine;
(45) the biaryl derivative or the pharmacologically acceptable salt thereof according to the above (1) which is
   2-{4-[6-fluoro-4'-(methylsulfonyl)-1,1'-biphenyl-3-yl]-2-isopropyl-1H-imidazol-5-yl}-6-methylpyridine,
   N-{2'-fluoro-5'-[2-isopropyl-5-(6-methylpyridin-2-yl)-1H-imidazol-4-yl]-1,1'-biphenyl-4-yl} cyclopropylsulfonamide,
   N-(morpholin-4-yl)-4- {2-fluoro-5-[2-isopropyl-5-(6-methylpyridin-2-yl)-1 H-imidazol-4-yl]-phenyl}benzamide,
   2'-fluoro-5'-[2-isopropyl-5-(6-methylpyridin-2-yl)-1H-imidazol-4-yl]-N-(4-methylpiperazin-1-yl)-1,1'-biphenyl-4-carboxyamide,
   N-{2'-fluoro-5'-[2-isopropyl-5-(6-methylpyridin-2-yl)-1H-imidazol-4-yl]-1,1'-biphenyl-4-yl}N'-(4-methylpiperazin-1-yl)urea,
   N-(tetrahydropyran-4-yl)-4-{[2-fluoro-5-[2-isopropyl-5-(6-methylpyridin-2-yl)-1H-imidazol-4-yl]phenyl}benzamide,
   N-{2'-fluoro-5'-[2-isopropyl-5-(6-methylpyridin-2-yl)-1H-imidazol-4-yl]-1,1'-biphenyl-4-yl}-N'-morpholin-4-ylurea, or
   2'-fluoro-5'-[2-isopropyl-5-(6-methylpyridin-2-yl)-1H-imidazol-4-yl]-N-(1-methylpiperidin-4-yl)-1,1'-biphenyl-4-carboxyamide;
(46) the biaryl derivative or the pharmacologically acceptable salt thereof according to the above (1) which is
   2-{2-isopropyl-4-[4'-(methylsulfonyl)-1,1'-biphenyl-3-yl]-1H-imidazol-5-yl}-6-methylpyridine,
   N-{3'-[2-isopropyl-5-(6-methylpyridin-2-yl)-1H-imidazol-4-yl]-1,1'-biphenyl-4-yl}cyclopropylsulfonamide,
   N-(4-methylpiperazin-1-yl)-4-{3-[2-isopropyl-5-(6-methylpyridin-2-yl)-1H-imidazol-4-yl]phenyl}benzamide,
   N-{3'-[2-isopropyl-5-(6-methylpyridin-2-yl)-1H-imidazol-4-yl]-1,1'-biphenyl-4-yl}-N'-(methylpiperazin-1-yl)urea,
   N-{3'-[2-isopropyl-5-(6-methylpyridin-2-yl)-1H-imidazol-4-yl]-1,1'-biphenyl-2-yl}-N'-morpholin-4-ylurea, or
   3'-[2-isopropyl-5-(6-methylpyridin-2-yl)-1H-imidazol-4-yl]-N-(1-methylpiperidin-4-yl)-1,1'-biphenyl-4-carboxyamide;
(47) a pharmaceutical composition containing a biaryl derivative or a pharmacologically acceptable salt thereof according to any one of the above (1) to (46) as an active ingredient;
(48) a pharmaceutical composition containing a biaryl derivative or a pharmacologically acceptable salt thereof according to any one of the above (1) to (46) as an active ingredient, which is for suppressing production of an extracellular matrix in glomerular cells;
(49) a pharmaceutical composition containing a biaryl derivative or a pharmacologically acceptable salt thereof according to any one of the above (1) to (46) as an active ingredient, which is for suppressing production of collagen in glomerular cells;
(50) a pharmaceutical composition containing a biaryl derivative or a pharmacologically acceptable salt thereof according to any one of the above (1) to (46) as an active ingredient, which is for suppressing production of an extracellular matrix in liver stellate cells;
(51) a pharmaceutical composition containing a biaryl derivative or a pharmacologically acceptable salt thereof according to any one of the above (1) to (46) as an active ingredient, which is for suppressing production of collagen in liver stellate cells;
(52) a pharmaceutical composition containing a biaryl derivative or a pharmacologically acceptable salt thereof according to any one of the above (1) to (46) as an active ingredient, which is for suppressing production of an extracellular matrix in lung fibroblasts;
(53) a pharmaceutical composition containing a biaryl derivative or a pharmacologically acceptable salt thereof according to any one of the above (1) to (46) as an active ingredient, which is for suppressing production of collagen in lung fibroblasts;
(54) a pharmaceutical composition containing a biaryl derivative or a pharmacologically acceptable salt thereof according to any one of the above (1) to (46) as an active ingredient, which is for suppressing production of an extracellular matrix in skin fibroblasts;
(55) a pharmaceutical composition containing a biaryl derivative or a pharmacologically acceptable salt thereof according to any one of the above (1) to (46) as an active ingredient, which is for suppressing production of collagen in skin fibroblasts;
(56) a pharmaceutical composition containing a biaryl derivative or a pharmacologically acceptable salt thereof according to any one of the above (1) to (46) as an active ingredient, which is for prevention and/or treatment of chronic renal disease, acute renal disease, diabetic renal disorder, or any renal disease mainly caused by fibrosis;
(57) a pharmaceutical composition containing a biaryl derivative or a pharmacologically acceptable salt thereof according to any one of the above (1) to (46) as an active ingredient, which is for prevention and/or treatment of liver fibrosis;
(58) a pharmaceutical composition containing a biaryl derivative or a pharmacologically acceptable salt thereof according to any one of the above (1) to (46) as an active ingredient, which is for prevention and/or treatment of lung fibrosis;
(59) a pharmaceutical composition containing a biaryl derivative or a pharmacologically acceptable salt thereof according to any one of the above (1) to (46) as an active ingredient, which is for prevention and/or treatment of general scleroderma, local scleroderma, keloid, discoid lupus erythematosus, or any skin disease mainly caused by fibrosis;
(60) a pharmaceutical composition containing a biaryl derivative or a pharmacologically acceptable salt thereof according to any one of the above (1) to (46) as an active ingredient, which is for prevention and/or treatment of skin fibrosis;
(61) use of the biaryl derivative or the pharmacologically acceptable salt thereof according to any one of the above (1) to (46) for the manufacture of a pharmaceutical composition;
(62) the use according to the above (61), wherein the pharmaceutical composition is one for prevention and/or treatment of chronic renal disease, acute renal disease, diabetic renal disorder, or any renal disease mainly caused by fibrosis;
(63) the use according to the above (61), wherein the pharmaceutical composition is one for prevention and/or treatment of liver fibrosis;
(64) the use according to the above (61), wherein the pharmaceutical composition is one for prevention and/or treatment of lung fibrosis;
(65) the use according to the above (61), wherein the pharmaceutical composition is one for prevention and/or treatment of general scleroderma, local scleroderma, keloid, discoid lupus erythematosus, or any skin disease mainly caused by fibrosis;
(66) the use according to the above (61), wherein the pharmaceutical composition is one for prevention and/or treatment of skin fibrosis;
(67) a method of preventing and/or treating a disease by administering a pharmaceutically effective dose of the biaryl derivative or the pharmacologically acceptable salt thereof according to any one of the above (1) to (46) to a warm-blooded animal;
(68) the method according to the above (67), wherein the disease is chronic renal disease, acute renal disease, diabetic renal disorder, or any renal disease mainly caused by fibrosis;
(69) the method according to the above (67), wherein the disease is liver fibrosis;
(70) the method according to the above (67), wherein the disease is lung fibrosis;
(71) the method according to the above (67), wherein the disease is general scleroderma, local scleroderma, keloid, discoid lupus erythematosus, or any skin disease mainly caused by fibrosis;
(72) the method according to the above (67), wherein the disease is skin fibrosis; and
(73) the method according to any one of the above (67) to (72), the warm-blooded animal is a human.

In the present invention, a "C₁-C₆ alkyl group" refers to a linear or branched alkyl group having one to six carbon atom(s), examples of which include methyl, ethyl, propyl, isopropyl, butyl, isobutyl, s-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl, and 2-ethylbutyl groups. In R³, it is preferably a branched alkyl group having three or four carbon atoms (C₃-C₄ alkyl group), more preferably an isopropyl group or an isobutyl group, and further more preferably an isopropyl group. In the others, it is preferably a linear or branched alkyl group having one to four carbon atom(s) (C₁-C₄ alkyl group), more preferably a methyl, an ethyl, a propyl, or an isopropyl group (C₁-C₃ alkyl group), and further more preferably a methyl group or an ethyl group (C₁-C₂ alkyl group), particularly preferably, a methyl group.

In the present invention, a "di-(C₁-C₆ alkyl)amino group" refers to a group in which the same or different two "C₁-C₆ alkyl group" described above are bound to an amino groups. Examples of it include dimethylamino, diethylamino, dipropylamino, diisopropylamino, dibutylamino, diisobutylamino, dipentylamino, diisopentylamino, dineopentylamino, dihexylamino, diisohexylamino, N-ethyl-N-methylamino, N-methyl-N-propylamino, N-isopropyl-N-methylamino, N-butyl-N-methylamino, N-isobutyl-N-methylamino, N-methyl-N-pentylamino, N-isopentyl-N-methylamino, N-ethyl-N-propylamino, N-ethyl-N-isopropylamino, N-butyl-N-ethylamino, N-ethyl-N-isobutylamino, N-ethyl-N-pentylamino, and N-ethyl-N-isopentylamino groups. It is preferably a group in which the same or different two "C₁-C₄ alkyl group" described above are bound to an amino group (di-(C₁-C₄ alkyl)amino group), more preferably a dimethylamino group, a diethylamino group, or an N-ethyl-N-methylamino group (di-(C₁-C₂ alkyl)amino group), and further more preferably a dimethylamino group.

In the present invention, a "di-(C₁-C₆ alkyl)aminosulfonyl group" refers to a group in which one "di-(C₁-C₆ alkyl)amino group" described above is bound to a sulfonyl group. Examples of it include dimethylaminosulfonyl, diethylaminosulfonyl, dipropylaminosulfonyl, diisopropylaminosulfonyl, dibutylaminosulfonyl, diisobutylaminosulfonyl, dipentylaminosulfonyl, diisopentylaminosulfonyl, dineopentylaminosulfonyl, dihexylaminosulfonyl, diisohexylaminosulfonyl, N-ethyl-N-methylaminosulfonyl, N-methyl-N-propylaminosulfonyl, N-isopropyl-N-methylaminosulfonyl, N-butyl-N-methylaminosulfonyl, N-isobutyl-N-methylaminosulfonyl, N-methyl-N-pentylaminosulfonyl, N-isopentyl-N-methylaminosulfonyl, N-ethyl-N-propylaminosulfonyl, N-ethyl-N-isopropylaminosulfonyl, N-butyl-N-ethylaminosulfonyl, N-ethyl-N-isobutylaminosulfonyl, N-ethyl-N-pentylaminosulfonyl, and N-ethyl-N-isopentylaminosulfonyl groups. It is preferably a group in which one "di-(C₁-C₄ alkyl)amino group" described above is bound to a sulfonyl group (di-(C₁-C₄ alkyl)aminosulfonyl group), more preferably a dimethylaminosulfonyl group, a diethylaminosulfonyl group, or an N-ethyl-N-methylaminosulfonyl group (di-(C₁-C₂ alkyl)aminosulfonyl group), and further more preferably a dimethylaminosulfonyl group.

In the present invention, a "di-(C₁-C₆ alkyl)aminocarbonylamino group" refers to a group in which a carbonyl group bonded to one "di-(C₁-C₆ alkyl)amino group" described above is bonded to an amino group. Examples of it include dimethylaminocarbonylamino, diethylaminocarbonylamino, dipropylaminocarbonylamino, diisopropylaminocarbonylamino, dibutylaminocarbonylamino, diisobutylaminocarbonylamino, dipentylaminocarbonylamino, diisopentylaminocarbonylamino, dineopentylaminocarbonylamino, dihexylaminocarbonylamino, diisohexylaminocarbonylamino, N-ethyl-N-methylaminocarbonylamino, N-methyl-N-propylaminocarbonylamino, N-isopropyl-N-methylaminocarbonylamino, N-butyl-N-methylaminocarbonylamino, N-isobutyl-N-methylaminocarbonylamino, N-methyl-N-pentylaminocarbonylamino, N-isopentyl-N-methylaminocarbonylamino, N-ethyl-N-propylaminocarbonylamino, N-ethyl-N-isopropylaminocarbonylamino, N-butyl-N-ethylaminocarbonylamino, N-ethyl-N-isobutylaminocarbonylamino, N-ethyl-N-pentylaminocarbonylamino, and N-ethyl-N-isopentylaminocarbonylamino groups. It is preferably a group in which a carbonyl group bonded to one "di-(C₁-C₄ alkyl)amino group" described above is bonded to an amino group (di-(C₁-C₄ alkyl)aminocarbonylamino group), more preferably a dimethylaminocarbonylamino group, a diethylaminocarbonylamino group, or an N-ethyl-N-methylaminocarbonylamino group (di-(C₁-C₂ alkyl)aminocarbonylamino group), and further more preferably a dimethylaminocarbonylamino group.

In the present invention, a "halogen atom" refers to a fluorine atom, a chlorine atom, a bromine atom, and an iodine atom. It is preferably a fluorine atom or a chlorine atom, and more preferably a fluorine atom.

In the present invention, a "halogenosulfonyl group" refers to a group in which one "halogen atom" described above is bound to a sulfonyl group. It is a fluorosulfonyl group, a chlorosulfonyl group, a bromosulfonyl group, or an iodosulfonyl group, preferably a fluorosulfonyl group or a chlorosulfonyl group, and more preferably a fluorosulfonyl group.

In the present invention, a "C₆-C₁₀ aryl group" refers to an aromatic hydrocarbon group having six to ten carbon atoms, examples of which include phenyl, indenyl, and naphthyl groups. It is preferably a phenyl group or a naphthyl group, and more preferably a phenyl group.

In the present invention, a "heterocyclic group" refers to a 5- to 7-membered heterocyclic group containing one to three sulfur atom(s), oxygen atom(s), nitrogen atom(s), sulfinyl group(s), and/or sulfonyl group(s), examples of which include "5-membered aromatic heterocyclic groups" such as furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, and thiadiazolyl groups; "6-membered aromatic heterocyclic groups" such as pyranyl, pyridyl, pyridazinyl, pyrimidinyl, and pyrazinyl groups; "partially or completely reduced 5-membered heterocyclic groups" such as tetrahydrothienyl, pyrrolidinyl, pyrrolinyl, imidazolidinyl, pyrazolidinyl, oxazolidinyl, isoxazolidinyl, thiazolidinyl, and dioxolanyl groups; and "partially or completely reduced 6-membered heterocyclic groups" such as tetrahydropyranyl, morpholinyl, morpholino, thiomorpholinyl, thiomorpholino, piperidinyl, piperidino, piperazinyl, and dioxanyl groups. In addition, the heterocyclic group may be fused with another cyclic group such as a benzene ring ("fused bicyclic heterocyclic group"), examples of which include benzothienyl, benzothiazolyl, benzoxazolyl, isobenzofuranyl, 1,3-dihydroisobenzofuranyl, quinolyl, 1,3-benzodioxolanyl, 1,4-benzodioxanyl, 1,1-dioxido-2,3-dihydro-1-benzothienyl, indolyl, isoindolyl, and indolinyl groups. In R¹, it is preferably a 5-membered aromatic heterocyclic group, 6-membered aromatic heterocyclic group, or fused bicyclic heterocyclic group, more preferably a thienyl, pyrrolyl, pyrazolyl, imidazolyl, pyridyl, or 1,1-dioxido-2,3-dihydro-1-benzothienyl group, further more preferably a 2-thienyl, 2-pyrrolyl, 3-pyrrolyl, 4-pyrazolyl, 4-imidazolyl, 5-imidazolyl, 2-pyridyl, or 1,1-dioxido-2,3-dihydro-1-benzothien-5-yl group, and particularly preferably a 2-thienyl, 3-pyrrolyl, 4-pyrazolyl, 4-imidazolyl, 5-imidazolyl, 2-pyridyl, or 1,1-dioxido-2,3-dihydro-1-benzothien-5-yl group. In R and substituent group *b,* it is preferably a partially or completely reduced 6-membered heterocyclic group or partially or completely reduced 5-membered heterocyclic group, more preferably a pyrrolidinyl, tetrahydropyranyl, morpholino, piperidino, or piperazinyl group, further more preferably a 1-pyrrolidinyl, 4-tetrahydropyranyl, 4-morpholino, 4-piperidino, or 1-piperazinyl group, and particularly preferably a 1-pyrrolidinyl or 4-morpholino group.

In the present invention, a "C₁-C₆ halogenated alkyl group" refers to a group in which the same or different one to five "halogen atom" described above are bound to the above-described "C₁-C₆ alkyl group", examples of which include trifluoromethyl, trichloromethyl, difluoromethyl, dichloromethyl, dibromomethyl, fluoromethyl, 2,2,2-trifluoroethyl, 2,2,2-trichloroethyl, 2-bromoethyl, 2-chloroethyl, 2-fluoroethyl, 2-iodoethyl, pentafluoroethyl, 3-chloropropyl, 4-fluorobutyl, 6-iodohexyl, and 2,2-dibromoethyl groups. It is preferably a group in which the same or different one to five "halogen atom" described above are bound to the above-described "C₁-C₄ alkyl group" (C₁-C₄ halogenated alkyl group), more preferably a group in which the same or different one to five "halogen atom" described above are bound to the above-described "C₁-C₂ alkyl group" (C₁-C₂ halogenated alkyl group), and further more preferably a trifluoromethyl group.

In the present invention, a "C₃-C₆ cycloalkyl group" is a cyclopropyl, cyclobutyl, cyclopentyl, or cyclohexyl group, preferably a cyclopropyl or cyclobutyl group, and more preferably a cyclopropyl group.

In the present invention, a "C₁-C₆ alkyl group substituted with a C₃-C₆ cycloalkyl group" refers to the above-described "C₁-C₆ alkyl group" substituted with one "C₃-C₆ cycloalkyl group" described above, examples of which include cyclopropylmethyl, cyclobutylmethyl, cyclopentylmethyl, cyclohexyhmethyl, 2-cyclopropylethyl, 1-cyclopropylethyl, 2-cyclobutylethyl, 1-cyclobutylethyl, 3-cyclopropylpropyl, and 4-cyclopropylbutyl groups. It is preferably a cyclopropylmethyl, a cyclobutylmethyl, a 2-cyclopropylethyl, a 1-cyclopropylethyl, a 2-cyclobutylethyl, or a 1-cyclobutylethyl group, and more preferably a cyclopropylmethyl group.

In the present invention, a "C₁-C₆ alkyl group substituted with two hydroxy groups" refers to a group in which two hydroxy groups are bound to the above-described "C₁-C₆ alkyl group", and is preferably a 1,2-dihydroxyethyl group.

In the present invention, a "C₁-C₆ alkyl group substituted with one to three halogen atom(s) and one hydroxy group" refers to a group in which the same or different one to three "halogen atom" described above and one hydroxy group are bound to the above-described "C₁-C₆ alkyl group", and is preferably a 2-fluoro-1-hydroxy-1-methylethyl group or a 2,2,2-trifluoro-1-hydroxy-1-methylethyl group.

In the present invention, a "C₁-C₆ alkoxy group" refers to a group in which the above-described "C₁-C₆ alkyl group" is bound to an oxygen atom, and refers to a linear or branched alkoxy group having one to six carbon atom(s). Examples of it include methoxy, ethoxy, propoxy, isopropoxy, butoxy, isobutoxy, s-butoxy, t-butoxy, pentoxy, isopentoxy, 2-methylbutoxy, 1-ethylpropoxy, 2-ethylpropoxy, neopentoxy, hexyloxy, 4-methylpentoxy, 3-methylpentoxy, 2-methylpentoxy, 3,3-dimethylbutoxy, 2,2-dimethylbutoxy, 1,1-dimethylbutoxy, 1,2-dimethylbutoxy, 1,3-dimethylbutoxy, and 2,3-dimethylbutoxy groups. It is preferably a linear or branched alkoxy group having one to four carbon atom(s) (C₁-C₄ alkoxy group), more preferably a methoxy group or ethoxy group (C₁-C₂ alkoxy group), and further more preferably a methoxy group.

In the present invention, a "C₂-C₇ alkylcarbonyloxy group" refers to a group in which a carbonyl group bonded to one "C₁-C₆ alkyl group" described above is bonded to an oxy group, examples of which include acetoxy, propionyloxy, butyryloxy, isobutyryloxy, pentanoyloxy, pivaloyloxy, valeryloxy, and isovaleryloxy groups. It is preferably a group in which a carbonyl group bonded to one "C₁-C₄ alkyl group" described above is bonded to an oxy group (C₂-C₅ alkylcarbonyloxy group), more preferably an acetoxy group or a propionyloxy group (C₂-C₃ alkylcarbonyloxy group), and further more preferably an acetoxy group.

In the present invention, a "mono-C₁-C₆ alkylsulfonylamino group" refers to a group in which a sulfonyl group bonded to one "C₁-C₆ alkyl group" described above is bonded to an amino group, examples of which include methylsulfonylamino, ethylsulfonylamino, propylsulfonylamino, isopropylsulfonylamino, butylsulfonylamino, isobutylsulfonylamino, s-butylsulfonylamino, t-butylsulfonylamino, pentylsulfonylamino, isopentylsulfonylamino, 2-methylbutylsulfonylamino, neopentylsulfonylamino, 1-ethylpropylsulfonylamino, hexylsulfonylamino, isohexylsulfonylamino, 4-methylpentylsulfonylamino, 3-methylpentylsulfonylamino, 2-methylpentylsulfonylamino, 1-methylpentylsulfonylamino, 3,3-dimethylbutylsulfonylamino, 2,2-dimethylbutylsulfonylamino, 1,1-dimethylbutylsulfonylamino, 1,2-dimethylbutylsulfonylamino, 1,3-dimethylbutylsulfonylamino, 2,3-dimethylbutylsulfonylamino, and 2-ethylbutylsulfonylamino groups. It is preferably a group in which a sulfonyl group bonded to one "C₁-C₄ alkyl group" described above is bonded to an amino group (mono-C₁-C₄ alkylsulfonylamino group), more preferably a methylsulfonylamino group or an ethylsulfonylamino group (mono-C₁-C₂ alkylsulfonylamino group), and further more preferably a methylsulfonylamino group.

In the present invention, a "group defined by formula R-L-" refers to a group in which a "group defined by formula R-" is bound to a "group defined by formula - L-", and is preferably a methyl group, ethyl group, hydroxymethyl group, 1-hydroxy-1-methylethyl group, 2-hydroxyethyl group, nitro group, methoxy group, 2-(1-pyrrolidinyl)ethoxy group, amino group, methylthio group, methylsulfinyl group, methylsulfonyl group, ethylsulfonyl group, methoxycarbonyl group, carbamoyl group, (2-hydroxyethyl)aminocarbonyl group, acetylamino group, (1-hydroxy-1-methylethyl)carbonylamino group, (1-acetoxy-1-methylethyl)carbonylamino group, aminosulfonyl group, methylaminosulfonyl group, dimethylaminosulfonyl group, methylsulfonylamino group, ethylsulfonylamino group, cyclopropylsulfonylamino group, (4-morpholino)sulfonyl group, (4-methyl-1-piperazinyl)sulfonyl group, (4-morpholino)carbonyl group, (4-morpholino)aminocarbonyl group, (4-methyl-1-piperazinyl)aminocarbonyl group, (4-tetrahydropyranyl)aminocarbonyl group, (1-methyl-4-piperidino)aminocarbonyl group, (4-morpholino)aminocarbonylamino group, (4-methyl-1-piperazinyl)aminocarbonylamino group, or 4-morpholino group, more preferably a methyl group, ethyl group, hydroxymethyl group, 1-hydroxy-1-methylethyl group, 2-hydroxyethyl group, methoxy group, 2-(1-pyrrolidinyl)ethoxy group, amino group, methylthio group, methylsulfinyl group, methylsulfonyl group, ethylsulfonyl group, carbamoyl group, acetylamino group, (1-hydroxy-1-methylethyl)carbonylamino group, (1-acetoxy-1-methylethyl)carbonylamino group, (2-hydroxyethyl)aminocarbonyl group, methylsulfonylamino group, ethylsulfonylamino group, cyclopropylsulfonylamino group, aminosulfonyl group, methylaminosulfonyl group, (4-morpholino)sulfonyl group, (4-methyl-1-piperazinyl)sulfonyl group, (4-morpholino)carbonyl group, (4-morpholino)aminocarbonyl group, (4-methyl-1-piperazinyl)aminocarbonyl group, (4-tetrahydropyranyl)aminocarbonyl group, (1-methyl-4-piperidino)aminocarbonyl group, (4-morpholino)aminocarbonylamino group, (4-methyl-1-piperazinyl)aminocarbonylamino group, or 4-morpholino group, further more preferably a methyl group, ethyl group, hydroxymethyl group, 1-hydroxy-1-methylethyl group, 2-hydroxyethyl group, methoxy group, 2-(1-pyrrolidinyl)ethoxy group, amino group, methylthio group, methylsulfinyl group, methylsulfonyl group, carbamoyl group, methylsulfonylamino group, ethylsulfonylamino group, cyclopropylsulfonylamino group, aminosulfonyl group, methylaminosulfonyl group, (4-morpholino)aminocarbonyl group, (4-methyl-1-piperazinyl)aminocarbonyl group, (4-tetrahydropyranyl)aminocarbonyl group, (1-methyl-4-piperidino)aminocarbonyl group, (4-morpholino)aminocarbonylamino group, (4-methyl-1-piperazinyl)aminocarbonylamino group, or 4-morpholino group, particularly preferably a methyl group, ethyl group, hydroxymethyl group, 1-hydroxy-1-methylethyl group, 2-hydroxyethyl group, 2-(1-pyrrolidinyl)ethoxy group, methylsulfonyl group, aminosulfonyl group, methylaminosulfonyl group, (4-morpholino)aminocarbonyl group, or 4-morpholino group, and most preferably a methyl group, hydroxymethyl group, 1-hydroxy-1-methylethyl group, 2-hydroxyethyl group, methylsulfonyl group, aminosulfonyl group, or methylaminosulfonyl group.

In the present invention, a "C₆-C₁₀ aryl group which may be substituted with one to three group(s) each independently selected from substituent group *a"* refers to the above-described "C₆-C₁₀ aryl group" which may be substituted with one to three group(s) each independently selected from substituent group *a,* and preferably refers to phenyl group or 4-methylphenyl group.

In the present invention, a "heterocyclic group which may be substituted with one group selected from substituent group *a"* refers to the above-described "heterocyclic group" which may be substituted with one group selected from substituent group *a,* and preferably refers to 4-tetrahydropyranyl group, 4-morpholino group, 4-methyl-1-piperazinyl group, or 1-methyl-4-piperidino group.

In the present invention, a "C₁-C₆ alkyl group substituted with one group selected from substituent group *b*" refers to the above-described "C₁-C₆ alkyl group" which is substituted with one group selected from substituent group *b*, and preferably refers to a C₁-C₄ alkyl group substituted with one group selected from the group consisting of a hydroxy group, C₂-C₅ alkylcarbonyloxy group, and heterocyclic group which may be substituted with one group selected from substituent group *a.* It is more preferably a hydroxymethyl group, 1-hydroxy-1-methylethyl group, 2-hydroxyethyl group, 1-acetoxy-1-methylethyl group, or 2-(1-pyrrolidinyl)ethyl group, and further more preferably a hydroxymethyl group, 1-hydroxy-1-methylethyl group, 2-hydroxyethyl group, or 2-(1-pyrrolidinyl)ethyl group.

In the present invention, a "heterocyclic carbonyl group which may be substituted with one group selected from substituent group *a*" refers to a group in which one "heterocyclic group which may be substituted with one group selected from substituent group *a"* described above is bound to a carbonyl group, and is preferably a (4-tetrahydropyranyl)carbonyl group, (4-morpholino)carbonyl group, (4-methyl-1-piperazinyl)carbonyl group, or (1-methyl-4-piperidino)carbonyl group.

In the present invention, a "heterocyclic amino group which may be substituted with one group selected from substituent group *a"* refers to a group in which one "heterocyclic group which may be substituted with one group selected from substituent group *a*" described above is bound to an amino group, and is preferably a (1-methyl-4-piperidino)amino group.

In the present invention, a "heterocyclic group substituted with one oxo group" refers to a heterocyclic group in which one oxo group is substituted to a methylene group forming the heterocyclic group, and is preferably a 2-oxo-1-pyrrolidinyl group.

In the present invention, a "C₁-C₆ alkylthio group" refers to a group in which the above-described "C₁-C₆ alkyl group" is bound to a sulfur atom and refers to a linear or branched alkylthio group having one to six carbon atom(s), examples of which include methylthio, ethylthio, propylthio, isopropylthio, butylthio, isobutylthio, s-butylthio, t-butylthio, pentylthio, isopentylthio, 2-methylbutylthio, neopentylthio, 1-ethylpropylthio, hexylthio, isohexylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 1-ethylbutylthio, and 2-ethylbutylthio groups. It is preferably a linear or branched alkylthio group having one to four carbon atom(s) (C₁-C₄ alkylthio group), more preferably a methylthio group or an ethylthio group (C₁-C₂ alkylthio group), and further more preferably a methylthio group.

In the present invention, a "C₁-C₆ alkylsulfinyl group" refers to a group in which the above-described "C₁-C₆ alkyl group" is bound to a sulfinyl group, examples of which include methylsulfinyl, ethylsulfinyl, propylsulfinyl, isopropylsulfinyl, butylsulfinyl, isobutylsulfinyl, s-butylsulfinyl, t-butylsulfinyl, pentylsulfinyl, isopentylsulfinyl, 2-methylbutylsulfinyl, neopentylsulfinyl, 1-ethylpropylsulfinyl, hexylsulfinyl, 4-methylpentylsulfinyl, 3-methylpentylsulfinyl, 2-methylpentylsulfinyl, 1-methylpentylsulfinyl, 3,3-dimethylbutylsulfinyl, 2,2-dimethylbutylsulfinyl, 1,1-dimethylbutylsulfinyl, 1,2-dimethylbutylsulfinyl, 1,3-dimethylbutylsulfinyl, 2,3-dimethylbutylsulfinyl, and 2-ethylbutylsulfinyl groups. It is preferably a group in which the above-described "C₁-C₄ alkyl group" is bound to a sulfinyl group (C₁-C₄ alkylsulfinyl group), more preferably a methylsulfinyl group or an ethylsulfinyl group (C₁-C₂ alkylsulfinyl group), and further more preferably a methylsulfinyl group.

In the present invention, a "C₁-C₆ alkylsulfonyl group" refers to a group in which the above-described "C₁-C₆ alkyl group" is bound to a sulfonyl group, examples of which include methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, isopentylsulfonyl, 2-methylbutylsulfonyl, neopentylsulfonyl, 1-ethylpropylsulfonyl, hexylsulfonyl, 4-methylpentylsulfonyl, 3-methylpentylsulfonyl, 2-methylpentylsulfonyl, 1-methylpentylsulfonyl, 3,3-dimethylbutylsulfonyl, 2,2-dimethylbutylsulfonyl, 1,1-dimethylbutylsulfonyl, 1,2-dimethylbutylsulfonyl, 1,3-dimethylbutylsulfonyl, 2,3-dimethylbutylsulfonyl, and 2-ethylbutylsulfonyl groups. It is preferably a group in which the above-described "C₁-C₄ alkyl group" is bound to a sulfonyl group (C₁-C₄ alkylsulfonyl group), more preferably a methylsulfonyl group or an ethylsulfonyl group (C₁-C₂ alkylsulfonyl group), and further more preferably a methylsulfonyl group.

In the present invention, a "C₆-C₁₀ aryl group which is substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, a halogen atom, and a halogenosulfonyl group" refers to the above-described "C₆-C₁₀ aryl group" which is substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, a halogen atom, and a halogenosulfonyl group, and is preferably a C₆-C₁₀ aryl group which is substituted with one or two group(s) each independently selected from the group consisting of a group defined by formula R-L-, a di-(C₁-C₄ alkyl)amino group, a di-(C₁-C₄ alkyl)aminosulfonyl group, a hydroxyaminocarbonyl group, and a halogen atom, more preferably a phenyl group of which the 4- or 3-position is substituted with one group selected from the group consisting of a group defined by formula R-L-, a di-(C₁-C₂ alkyl) aminosulfonyl group, and a halogen atom, further more preferably a phenyl group of which the 4-position is substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, a (4-methyl-1-piperazinyl)aminocarbonylamino group, a 2-(1-pyrrolidinyl)ethoxy group, and a dimethylaminosulfonyl group, particularly preferably a 4-methoxyphenyl group, a 4-aminophenyl group, a 4-methylthiophenyl group, a 4-methylsulfinylphenyl group, a 4-methylsulfonylphenyl group, a 4-ethylsulfonylphenyl group, a 4-acetylaminophenyl group, a 4-(1-hydroxy-1-methylethyl)carbonylaminophenyl group, a 4-(1-acetoxy-1-methylethyl)carbonylaminophenyl group, a 4-carbamoylphenyl group, a 3-carbamoylphenyl group, a 4-(2-hydroxyethyl)aminocarbonylphenyl group, a 4-methylsulfonylaminophenyl group, a 4-ethylsulfonylaminophenyl group, a 4-cyclopropylsulfonylaminophenyl group, a 4-aminosulfonylphenyl group, a 4-methylaminosulfonylphenyl group, a 4-(4-morpholino)carbonylphenyl group, a 4-(4-morpholino)sulfonylphenyl group, a 4-(4-morpholino)aminocarbonylphenyl group, a 4-(4-methyl-1-piperazinyl)aminocarbonylphenyl group, a 4-(4-methyl-1-piperazinyl)sulfonylphenyl group, a 4-(4-tetrahydropyranyl)aminocarbonylphenyl group, a 4-(1-methyl-4-piperidino)aminocarbonylphenyl group, a 4-(4-morpholino)aminocarbonylaminophenyl group, a 4-(4-methyl-1-piperazinyl)aminocarbonylaminophenyl group, a 4-[2-(1-pyrrolidinyl)ethoxy]phenyl group, or a 4-dimethylaminosulfonylphenyl group, most preferably a 4-methoxyphenyl group, a 4-aminophenyl group, a 4-methylthiophenyl group, a 4-methylsulfinylphenyl group, a 4-methylsulfonylphenyl group, a 4-methylsulfonylaminophenyl group, a 4-ethylsulfonylaminophenyl group, a 4-cyclopropylsulfonylaminophenyl group, a 4-aminosulfonylphenyl group, a 4-methylaminosulfonylphenyl group, a 4-(4-morpholino)aminocarbonylphenyl group, a 4-(4-methyl-1-piperazinyl)aminocarbonylphenyl group, a 4-(4-tetrahydropyranyl)aminocarbonylphenyl group, a 4-(1-methyl-4-piperidino)aminocarbonylphenyl group, a 4-(4-morpholino)aminocarbonylaminophenyl group, a 4-(4-methyl-1-piperazinyl)aminocarbonylaminophenyl group, a 4-[2-(1-pyrrolidinyl)ethoxy]phenyl group, or a 4-dimethylaminosulfonylphenyl group, further most preferably a 4-methylsulfonylphenyl group, a 4-aminosulfonylphenyl group, a 4-methylaminosulfonylphenyl group, a 4-(4-morpholino)aminocarbonylphenyl group, a 4-[2-(1-pyrrolidinyl)ethoxy]phenyl group, or a 4-dimethylaminosulfonylphenyl group, and further most preferably a 4-methylsulfonylphenyl group, a 4-aminosulfonylphenyl group, or a 4-methylaminosulfonylphenyl group.

In the present invention, a "heterocyclic group which may be substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, a halogen atom, and an oxo group" refers to the above-mentioned "heterocyclic group" which may be substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, a halogen atom, and an oxo group, and is preferably a heterocyclic group which may be substituted with one group selected from the group consisting of a group defined by formula R-L-, a di-(C₁-C₆ alkyl)amino group, a di-(C₁-C₆ alkyl)aminosulfonyl group, a hydroxyaminocarbonyl group, and a halogen atom, more preferably a thienyl, pyrrolyl, furyl, pyrazolyl, imidazolyl, pyridyl, or 1,1-dioxido-2,3-dihydro-1-benzothienyl group which may be substituted with one group selected from the group consisting of a group defined by formula R-L-, a di-(C₁-C₂ alkyl)aminosulfonyl group, and a halogen atom, further more preferably a thienyl, pyrrolyl, furyl, pyrazolyl, imidazolyl, pyridyl, or 1,1-dioxido-2,3-dihydro-1-benzothienyl group which may be substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group, particularly preferably a 3-thienyl group, a 3-furyl group, a 5-carbamoyl-2-pyridyl group, a 2-methoxy-5-pyridyl group, a 4-pyridyl group, a 5-aminosulfonyl-2-thienyl group, a 2-pyrrolyl group, a 3-pyrrolyl group, a 4-pyrazolyl group, a 1-methyl-4-pyrazolyl group, a 1-ethyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 2-(2-hydroxyethyl)-5-imidazolyl group, a 5-hydroxymethyl-2-pyridyl group, a 5-(1-hydroxy-1-methylethyl)-2-pyridyl group, a 5-(4-morpholino)-2-pyridyl group, or a 1,1-dioxido-2,3-dihydro-1-benzothien-5-yl group, most preferably a 3-thienyl group, a 5-carbamoyl-2-pyridyl group, a 5-aminosulfonyl-2-thienyl group, a 2-pyrrolyl group, a 3-pyrrolyl group, a 4-pyrazolyl group, a 1-methyl-4-pyrazolyl group, a 1-ethyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 2-(2-hydroxyethyl)-5-imidazolyl group, a 5-hydroxymethyl-2-pyridyl group, a 5-(1-hydroxy-1-methylethyl)-2-pyridyl group, a 5-(4-morpholino)-2-pyridyl group, or a 1,1-dioxido-2,3-dihydro-1-benzothien-5-yl group, further most preferably a 5-aminosulfonyl-2-thienyl group, a 2-pyrrolyl group, a 3-pyrrolyl group, a 4-pyrazolyl group, a 1-methyl-4-pyrazolyl group, a 1-ethyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 2-(2-hydroxyethyl)-5-imidazolyl group, a 5-hydroxymethyl-2-pyridyl group, a 5-(1-hydroxy-1-methylethyl)-2-pyridyl group, a 5-(4-morpholino)-2-pyridyl group, or a 1,1-dioxido-2,3-dihydro-1-benzothien-5-yl group, and further most preferably a 5-aminosulfonyl-2-thienyl group, a 3-pyrrolyl group, a 1-methyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 5-(1-hydroxy-1-methylethyl)-2-pyridyl group, or a 1,1-dioxido-2,3-dihydro-1-benzothien-5-yl group.

In the present invention, R is preferably a hydrogen atom, a C₁-C₆ alkyl group, a heterocyclic group which may be substituted with one group selected from substituent group *a*, or a C₁-C₆ alkyl group which is substituted with one group selected from substituent group *b*, more preferably a hydrogen atom, a C₁-C₄ alkyl group, a partially or completely reduced 6-membered heterocyclic group, a C₁-C₆ alkyl group which is substituted with one partially or completely reduced 5-membered heterocyclic group, or a C₁-C₆ alkyl group which is substituted with one hydroxy group, and further more preferably a hydrogen atom, a methyl group, an ethyl group, a hydroxymethyl group, a 1-hydroxy-1-methylethyl group, or a 2-hydroxyethyl group.

In the present invention, L is preferably a single bond, an oxygen atom, an amino group, a sulfur atom, a sulfinyl group, a sulfonyl group, a carbonyl group, an oxycarbonyl group, an aminocarbonyl group, a carbonylamino group, an aminosulfonyl group, a sulfonylamino group, or an aminocarbonylamino group, more preferably a single bond, an oxygen atom, an amino group, a sulfur atom, a sulfinyl group, a sulfonyl group, a carbonyl group, an aminocarbonyl group, a carbonylamino group, an aminosulfonyl group, a sulfonylamino group, or an aminocarbonylamino group, further more preferably a single bond, an oxygen atom, an amino group, a sulfur atom, a sulfinyl group, a sulfonyl group, an aminocarbonyl group, an aminosulfonyl group, a sulfonylamino group, or an aminocarbonylamino group, and particularly preferably a single bond, an oxygen atom, a sulfonyl group, an aminocarbonyl group, or an aminosulfonyl group.

In the present invention, R¹ is preferably a C₆-C₁₀ aryl group which is substituted with one to three group(s) each independently selected from the group consisting of a group defined by formula R-L- and a di-(C₁-C₆ alkyl)amino group; or a heterocyclic group which may be substituted with one to three group(s) each independently selected from a group defined by formula R-L-. R¹ is more preferably a C₆-C₁₀ aryl group which is substituted with one group selected from the group consisting of a group defined by formula R-L- and a di-(C₁-C₆ alkyl)amino group; or a 5-membered aromatic heterocyclic, 6-membered aromatic heterocyclic, or fused bicyclic heterocyclic group which may be substituted with one group selected from a group defined by formula R-L-. R¹ is further more preferably a phenyl group of which the 4-position is substituted with one group selected from the group consisting of a group defined by formula R-L- and a dimethylamino group; or a thienyl, pyrrolyl, pyrazolyl, imidazolyl, pyridyl, or 1,1-dioxido-2,3-dihydro-1-benzothien-5-yl group which may be substituted with one group selected from a group defined by formula R-L-. R¹ is particularly preferably a 4-methylsulfonylphenyl group, a 4-aminosulfonylphenyl group, a 4-methylaminosulfonylphenyl group, a 4-(4-morpholino)aminocarbonylphenyl group, a 4-[2-(1-pyrrolidinyl)ethoxy]phenyl group, a 4-dimethylaminosulfonylphenyl group, a 5-aminosulfonyl-2-thienyl group, a 2-pyrrolyl group, a 3-pyrrolyl group, a 4-pyrazolyl group, a 1-methyl-4-pyrazolyl group, a 1-ethyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 2-(2-hydroxyethyl)-5-imidazolyl group, a 5-hydroxymethyl-2-pyridyl group, a 5-(1-hydroxy-1-methylethyl)-2-pyridyl group, a 5-(4-morpholino)-2-pyridyl group, or a 1,1-dioxido-2,3-dihydro-1-benzothien-5-yl group, and most preferably a 4-methylsulfonylphenyl group, a 4-aminosulfonylphenyl group, a 4-methylaminosulfonylphenyl group, a 5-aminosulfonyl-2-thienyl group, a 3-pyrrolyl group, a 1-methyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 5-(1-hydroxy-1-methylethyl)-2-pyridyl group, or a 1,1-dioxido-2,3-dihydro-1-benzothien-5-yl group.

In the present invention, R² is preferably a hydrogen atom, a methyl group, a fluorine atom, or a chlorine atom, more preferably a hydrogen atom or a fluorine atom; and most preferably a fluorine atom.

In the present invention, A is preferably a group defined by formula (II).

In the present invention, A is preferably a group defined by formula (III).

In the present invention, R³ is preferably a hydrogen atom, a C₁-C₆ alkyl group, or a C₃-C₆ cycloalkyl group. When A is defined by formula (II), R³ is more preferably an isopropyl group, an isobutyl group, or a cyclopropyl group, and further preferably an isopropyl group. When A is defined by formula (III), R³ is more preferably a hydrogen atom.

In the present invention, R⁴ is preferably a hydrogen atom or a methyl group, more preferably a methyl group.

A "pharmacologically acceptable salt thereof' means a salt formed by allowing the biaryl derivative having the General Formula (I) of the present invention to react with acid when the biaryl derivative contains a basic group such as an amino group, or with base when the biaryl derivative contains an acidic group such as a carboxyl group.

The salts derived from a basic group include, for example, inorganic salts such as hydrohalide including hydrofluoride, hydrochloride, hydrobromide and hydroiodide, nitrate, perchlorate, sulfate and phosphate; organic salts such as C₁-C₆ alkyl sulfonate including methanesulfonate, trifluoromethanesulfonate and ethanesulfonate, aryl sulfonate including benzenesulfonate and p-toluenesulfonate, acetate, malate, fumarate, succinate, citrate, ascorbate, tartrate, oxalate and maleate; and amino acid salts such as glycine salt, lysine salt, arginine salt, ornithine salt, glutamate and aspartate.

The salts derived from an acidic group include, for example, alkali metal salts such as sodium salt, potassium salt and lithium salt, alkaline earth metal salts such as calcium salt and magnesium salt, metal salts such as aluminium salt and iron salt; amine salts including inorganic salts such as ammonium salt and organic salts such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt and tris(hydroxymethyl)aminomethane salt; and amino acid salts such as glycine salt, lysine salt, arginine salt, ornithine salt, glutamic acid and aspartate.

The biaryl derivative having the General Formula (I) or a pharmacologically acceptable salt thereof according to the present invention may absorb water, or water may attach thereto to form a hydrate when left in the air or recrystallized. The salt of the present invention also encompasses such hydrates.

The biaryl derivative having the General Formula (I) or a pharmacologically acceptable salt thereof according to the present invention may absorb certain other solvent(s) to form a solvate. The salt of the present invention also encompasses such solvates.

Specific examples of compounds having the General Formula (I) of the present invention are, for example, the compounds shown in the following Table 1 to Table 3, but the present invention is not limited to these compounds.

The meanings of abbreviations in Tables 1 to 3 are as follows:
F: fluorine atom,
Cl: chlorine atom,
Me: methyl group,
Et: ethyl group,
iPr: isopropyl group,
cPr: cyclopropyl group,
iBu: isobutyl group,
tBu: tertiary-butyl group,
4-[SO₂-(4-Morpho)]-C₆H₄: 4-(4-morpholino)sulfonylphenyl group,
4-[SO₂-(4-Me-1-Pipera)]-C₆H₄: 4-(4-methyl-1-piperazinyl)sulfonylphenyl group,
4-[CO-(4-Thiomorpho)]-C₆H₄: 4-(4-thiomorpholino)carbonylphenyl group,
4-[CO-(1-Oxi-4-thiomorpho)]-C₆H₄: 4-(1-oxido-4-thiomorpholino)carbonylphenyl group,
4-[CO-(1,1-Dioxi-4-thiomorpho)]-C₆H₄: 4-(1,1-dioxido-4-thiomorpholino)carbonylphenyl group,
4-[CONH-(4-Tet-pyra)]-C₆H₄: 4-(4-tetrahydropyranyl)aminocarbonylphenyl group,
4-[CONH-(1-Me-4-Piperidi)]-C₆H₄: 4-(1-methyl-4-piperidino)aminocarbonylphenyl group,
4-[NHCO-(4-Tet-pyra)]-C₆H₄: 4-(4-tetrahydropyranyl)carbonylaminophenyl group, 2-Thi: 2-thienyl group,
3-Fur: 3-furyl group,
2-Pyrro: 2-pyrrolyl group,
2-Py: 2-pyridyl group,
1-Me-4-Pyrazo: 1-methyl-4-pyrazolyl group,
1-Me-4-imidazo: 1-methyl-4-imidazolyl group,
2-Pyrimi: 2-pyrimidinyl group,
2-CH₂OH-4-Thiazo: 2-hydroxymethyl-4-thiazolyl group,
2-Oxo-1-Pyrrolidi: 2-oxo-1-pyrrolidinyl group,
2-Furyl: 2-furyl group,
2-Oxazo: 2-oxazolyl group,
2-Thiazo: 2-thiazolyl group,
5-[1,2,3]-Triazo: 5-[1,2,3]-triazolyl group,
1-[1,2,4]-Triazo: 1-[1,2,4]-triazolyl group,
4-SO₂NH₂-3-F-C₆H₄: 4-aminosulfonyl-3-fluorophenyl group,
4-SO₂NH₂-2,5-di-F-C₆H₄: 4-aminosulfonyl-2,5-difluorophenyl group,
Het(A): 1,3-dihydro-2H-imidazol-2-on-4-yl group,
Het(B): 3,4-dihydro-2H-thiochromen-6-yl group,
Het(C): 1-oxido-3,4-dihydro-2H-thiochromen-6-yl group,
Het(D): 1,1-dioxido-3,4-dihydro-2H-thiochromen-6-yl group,
Het(E): 2,3-dihydro-4H-thiochromen-4-on-6-yl group,
Het(F): 1-oxido-2,3-dihydro-4H-thiochromen-4-on-6-yl group,
Het(G): 1,1-dioxido-2,3-dihydro-4H-thiochromen-4-on-6-yl group,
Het(H): 1,1-dioxido-1-benzothien-5-yl group,
Het(I): 1,1-dioxido-2,3-dihydro-1-benzothien-5-yl group,
Het(J): 1-oxido-2,3-dihydroimidazo[2,1-b][1,3]thiazol-6-yl group, and
Het(K): 1,1-dioxido-2,3-dihydroimidazo[2,1-b][1,3]thiazol-6-yl group.

**(Table 1)**

| | | | | |
|---|---|---|---|---|
| 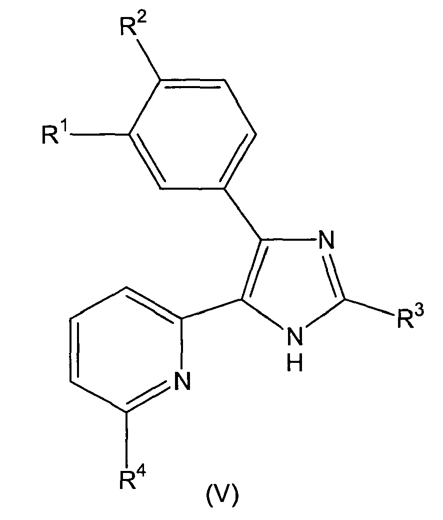 | | | | |

| Compound No. | R¹ | R² | R³ | R⁴ |
|---|---|---|---|---|
| 1-1 | 4-Ome-C₆H₄ | H | iPr | Me |
| 1-2 | 4-Ome-C₆H₄ | H | iPr | H |
| 1-3 | 4-Ome-C₆H₄ | H | cPr | Me |
| 1-4 | 4-Ome-C₆H₄ | H | cPr | H |
| 1-5 | 4-Ome-C₆H₄ | H | iBu | Me |
| 1-6 | 4-Ome-C₆H₄ | H | iBu | H |
| 1-7 | 4-Ome-C₆H₄ | Me | iPr | Me |
| 1-8 | 4-Ome-C₆H₄ | Me | iPr | H |
| 1-9 | 4-Ome-C₆H₄ | Me | cPr | Me |
| 1-10 | 4-Ome-C₆H₄ | Me | cPr | H |
| 1-11 | 4-Ome-C₆H₄ | Me | iBu | Me |
| 1-12 | 4-Ome-C₆H₄ | Me | iBu | H |
| 1-13 | 4-Ome-C₆H₄ | F | iPr | Me |
| 1-14 | 4-Ome-C₆H₄ | F | iPr | H |
| 1-15 | 4-Ome-C₆H₄ | F | cPr | Me |
| 1-16 | 4-Ome-C₆H₄ | F | cPr | H |
| 1-17 | 4-Ome-C₆H₄ | F | iBu | Me |
| 1-18 | 4-Ome-C₆H₄ | F | iBu | H |
| 1-19 | 4-Ome-C₆H₄ | Cl | iPr | Me |
| 1-20 | 4-Ome-C₆H₄ | Cl | iPr | H |
| 1-21 | 4-Ome-C₆H₄ | Cl | cPr | Me |
| 1-22 | 4-Ome-C₆H₄ | Cl | cPr | H |
| 1-23 | 4-Ome-C₆H₄ | Cl | iBu | Me |
| 1-24 | 4-Ome-C₆H₄ | Cl | iBu | H |
| 1-25 | 4-NH₂-C₆H₄ | H | iPr | Me |
| 1-26 | 4-NH₂-C₆H₄ | H | iPr | H |
| 1-27 | 4-NH₂-C₆H₄ | H | cPr | Me |
| 1-28 | 4-NH₂-C₆H₄ | H | cPr | H |
| 1-29 | 4-NH₂-C₆H₄ | H | iBu | Me |
| 1-30 | 4-NH₂-C₆H₄ | H | iBu | H |
| 1-31 | 4-NH₂-C₆H₄ | Me | iPr | Me |
| 1-32 | 4-NH₂-C₆H₄ | Me | iPr | H |
| 1-33 | 4-NH₂-C₆H₄ | Me | cPr | Me |
| 1-34 | 4-NH₂-C₆H₄ | Me | cPr | H |
| 1-35 | 4-NH₂-C₆H₄ | Me | iBu | Me |
| 1-36 | 4-NH₂-C₆H₄ | Me | iBu | H |
| 1-37 | 4-NH₂-C₆H₄ | F | iPr | Me |
| 1-38 | 4-NH₂-C₆H₄ | F | iPr | H |
| 1-39 | 4-NH₂-C₆H₄ | F | cPr | Me |
| 1-40 | 4-NH₂-C₆H₄ | F | cPr | H |
| 1-41 | 4-NH₂-C₆H₄ | F | iBu | Me |
| 1-42 | 4-NH₂-C₆H₄ | F | iBu | H |
| 1-43 | 4-NH₂-C₆H₄ | Cl | iPr | Me |
| 1-44 | 4-NH₂-C₆H₄ | Cl | iPr | H |
| 1-45 | 4-NH₂-C₆H₄ | Cl | cPr | Me |
| 1-46 | 4-NH₂-C₆H₄ | Cl | cPr | H |
| 1-47 | 4-NH₂-C₆H₄ | Cl | iBu | Me |
| 1-48 | 4-NH₂-C₆H₄ | Cl | iBu | H |
| 1-49 | 4-Sme-C₆H₄ | H | iPr | Me |
| 1-50 | 4-Sme-C₆H₄ | H | iPr | H |
| 1-51 | 4-Sme-C₆H₄ | H | cPr | Me |
| 1-52 | 4-Sme-C₆H₄ | H | cPr | H |
| 1-53 | 4-Sme-C₆H₄ | H | iBu | Me |
| 1-54 | 4-Sme-C₆H₄ | H | iBu | H |
| 1-55 | 4-Sme-C₆H₄ | Me | iPr | Me |
| 1-56 | 4-Sme-C₆H₄ | Me | iPr | H |
| 1-57 | 4-Sme-C₆H₄ | Me | cP | Me |
| 1-58 | 4-Sme-C₆H₄ | Me | cPr | H |
| 1-59 | 4-Sme-C₆H₄ | Me | iBu | Me |
| 1-60 | 4-Sme-C₆H₄ | Me | iBu | H |
| 1-61 | 4-Sme-C₆H₄ | F | iPr | Me |
| 1-62 | 4-Sme-C₆H₄ | F | iPr | H |
| 1-63 | 4-Sme-C₆H₄ | F | cPr | Me |
| 1-64 | 4-Sme-C₆H₄ | F | cPr | H |
| 1-65 | 4-Sme-C₆H₄ | F | iBu | Me |
| 1-66 | 4-Sme-C₆H₄ | F | iBu | H |
| 1-67 | 4-Sme-C₆H₄ | Cl | iPr | Me |
| 1-68 | 4-Sme-C₆H₄ | Cl | iPr | H |
| 1-69 | 4-Sme-C₆H₄ | Cl | cPr | Me |
| 1-70 | 4-Sme-C₆H₄ | Cl | cPr | H |
| 1-71 | 4-Sme-C₆H₄ | Cl | iBu | Me |
| 1-72 | 4-Sme-C₆H₄ | Cl | iBu | H |
| 1-73 | 4-SOMe-C₆H₄ | H | iPr | Me |
| 1-74 | 4-SOMe-C₆H₄ | H | iPr | H |
| 1-75 | 4-SOMe-C₆H₄ | H | cPr | Me |
| 1-76 | 4-SOMe-C₆H₄ | H | cPr | H |
| 1-77 | 4-SOMe-C₆H₄ | H | iBu | Me |
| 1-78 | 4-SOMe-C₆H₄ | H | iBu | H |
| 1-79 | 4-SOMe-C₆H₄ | Me | iPr | Me |
| 1-80 | 4-SOMe-C₆H₄ | Me | iPr | H |
| 1-81 | 4-SOMe-C₆H₄ | Me | cPr | Me |
| 1-82 | 4-SOMe-C₆H₄ | Me | cPr | H |
| 1-83 | 4-SOMe-C₆H₄ | Me | iBu | Me |
| 1-84 | 4-SOMe-C₆H₄ | Me | iBu | H |
| 1-85 | 4-SOMe-C₆H₄ | F | iPr | Me |
| 1-86 | 4-SOMe-C₆H₄ | F | iPr | H |
| 1-87 | 4-SOMe-C₆H₄ | F | cPr | Me |
| 1-88 | 4-SOMe-C₆H₄ | F | cPr | H |
| 1-89 | 4-SOMe-C₆H₄ | F | iBu | Me |
| 1-90 | 4-SOMe-C₆H₄ | F | iBu | H |
| 1-91 | 4-SOMe-C₆H₄ | Cl | iPr | Me |
| 1-92 | 4-SOMe-C₆H₄ | Cl | iPr | H |
| 1-93 | 4-SOMe-C₆H₄ | Cl | cPr | Me |
| 1-94 | 4-SOMe-C₆H₄ | Cl | cPr | H |
| 1-95 | 4-SOMe-C₆H₄ | Cl | iBu | Me |
| 1-96 | 4-SOMe-C₆H₄ | Cl | iBu | H |
| 1-97 | 4-SO₂Me-C₆H₄ | H | iPr | Me |
| 1-98 | 4-SO₂Me-C₆H₄ | H | iPr | H |
| 1-99 | 4-SO₂Me-C₆H₄ | H | cPr | Me |
| 1-100 | 4-SO₂Me-C₆H₄ | H | cPr | H |
| 1-101 | 4-SO₂Me-C₆H₄ | H | iBu | Me |
| 1-102 | 4-SO₂Me-C₆H₄ | H | iBu | H |
| 1-103 | 4-SO₂Me-C₆H₄ | Me | iPr | Me |
| 1-104 | 4-SO₂Me-C₆H₄ | Me | iPr | H |
| 1-105 | 4-SO₂Me-C₆H₄ | Me | cPr | Me |
| 1-106 | 4-SO₂Me-C₆H₄ | Me | cPr | H |
| 1-107 | 4-SO₂Me-C₆H₄ | Me | iBu | Me |
| 1-108 | 4-SO₂Me-C₆H₄ | Me | iBu | H |
| 1-109 | 4-SO₂Me-C₆H₄ | F | iPr | Me |
| 1-110 | 4-SO₂Me-C₆H₄ | F | iPr | H |
| 1-111 | 4-SO₂Me-C₆H₄ | F | cPr | Me |
| 1-112 | 4-SO₂Me-C₆H₄ | F | cPr | H |
| 1-113 | 4-SO₂Me-C₆H₄ | F | iBu | Me |
| 1-114 | 4-SO₂Me-C₆H₄ | F | iBu | H |
| 1-115 | 4-SO₂Me-C₆H₄ | Cl | iPr | Me |
| 1-116 | 4-SO₂Me-C₆H₄ | Cl | iPr | H |
| 1-117 | 4-SO₂Me-C₆H₄ | Cl | cPr | Me |
| 1-118 | 4-SO₂Me-C₆H₄ | Cl | cPr | H |
| 1-119 | 4-SO₂Me-C₆H₄ | Cl | iBu | Me |
| 1-120 | 4-SO₂Me-C₆H₄ | Cl | iBu | H |
| 1-121 | 4-NHSO₂Me-C₆H₄ | H | iPr | Me |
| 1-122 | 4-NHSO₂Me-C₆H₄ | H | iPr | H |
| 1-123 | 4-NHSO₂Me-C₆H₄ | H | cPr | Me |
| 1-124 | 4-NHSO₂Me-C₆H₄ | H | cPr | H |
| 1-125 | 4-NHSO₂Me-C₆H₄ | H | iBu | Me |
| 1-126 | 4-NHSO₂Me-C₆H₄ | H | iBu | H |
| 1-127 | 4-NHSO₂Me-C₆H₄ | Me | iPr | Me |
| 1-128 | 4-NHSO₂Me-C₆H₄ | Me | iPr | H |
| 1-129 | 4-NHSO₂Me-C₆H₄ | Me | cPr | Me |
| 1-130 | 4-NHSO₂Me-C₆H₄ | Me | cPr | H |
| 1-131 | 4-NHSO₂Me-C₆H₄ | Me | iBu | Me |
| 1-132 | 4-NHSO₂Me-C₆H₄ | Me | iBu | H |
| 1-133 | 4-NHSO₂Me-C₆H₄ | F | iPr | Me |
| 1-134 | 4-NHSO₂Me-C₆H₄ | F | iPr | H |
| 1-135 | 4-NHSO₂Me-C₆H₄ | F | cPr | Me |
| 1-136 | 4-NHSO₂Me-C₆H₄ | F | cPr | H□ |
| 1-137 | 4-NHSO₂Me-C₆H₄ | F | iBu | Me |
| 1-138 | 4-NHSO₂Me-C₆H₄ | F | iBu | H |
| 1-139 | 4-NHSO₂Me-C₆H₄ | Cl | iPr | Me |
| 1-140 | 4-NHSO₂Me-C₆H₄ | Cl | iPr | H |
| 1-141 | 4-NHSO₂Me-C₆H₄ | Cl | cPr | Me |
| 1-142 | 4-NHSO₂Me-C₆H₄ | Cl | cPr | H |
| 1-143 | 4-NHSO₂Me-C₆H₄ | Cl | iBu | Me |
| 1-144 | 4-NHSO₂Me-C₆H₄ | Cl | iBu | H |
| 1-145 | 4-NHSO₂Et-C₆H₄ | H | iPr | Me |
| 1-146 | 4-NHSO₂Et-C₆H₄ | H | iPr | H |
| 1-147 | 4-N-HSO₂Et-C₆H₄ | H | cPr | Me |
| 1-148 | 4-NHSO₂Et-C₆H₄ | H | cPr | H |
| 1-149 | 4-NHSO₂Et-C₆H₄ | H | iBu | Me |
| 1-150 | 4-NHSO₂Et-C₆H₄ | H | iBu | H |
| 1-151 | 4-NHSO₂Et-C₆H₄ | Me | iPr | Me |
| 1-152 | 4-NHSO₂Et-C₆H₄ | Me | iPr | H |
| 1-153 | 4-NHSO₂Et-C₆H₄ | Me | cPr | Me |
| 1-154 | 4-NHSO₂Et-C₆H₄ | Me | cPr | H |
| 1-155 | 4-NHSO₂Et-C₆H₄ | Me | iBu | Me |
| 1-156 | 4-NHSO₂Et-C₆H₄ | Me | iBu | H |
| 1-157 | 4-NHSO₂Et-C₆H₄ | F | iPr | Me |
| 1-158 | 4-NHSO₂Et-C₆H₄ | F | iPr | H |
| 1-159 | 4-NHSO₂Et-C₆H₄ | F | cPr | Me |
| 1-160 | 4-NHSO₂Et-C₆H₄ | F | cPr | H |
| 1-161 | 4-NHSO₂Et-C₆H₄ | F | iBu | Me |
| 1-162 | 4-NHSO₂Et-C₆H₄ | F | iBu | H |
| 1-163 | 4-NHSO₂Et-C₆H₄ | Cl | iPr | Me |
| 1-164 | 4-NHSO₂Et-C₆H₄ | Cl | iPr | H |
| 1-165 | 4-NHSO₂Et-C₆H₄ | Cl | cPr | Me |
| 1-166 | 4-NHSO₂Et-C₆H₄ | Cl | cPr | H |
| 1-167 | 4-NHSO₂Et-C₆H₄ | Cl | iBu | Me |
| 1-168 | 4-NHSO₂Et-C₆H₄ | Cl | iBu | H |
| 1-169 | 4-NHSO₂cPr-C₆H₄ | H | iPr | Me |
| 1-170 | 4-NHSO₂cPr-C₆H₄ | H | iPr | H |
| 1-171 | 4-NHSO₂cPr-C₆H₄ | H | cPr | Me |
| 1-172 | 4-NHSO₂cPr-C₆H₄ | H | cPr | H |
| 1-173 | 4-NHSO₂cPr-C₆H₄ | H | iBu | Me |
| 1-174 | 4-NHSO₂cPr-C₆H₄ | H | iBu | H |
| 1-175 | 4-NHSO₂cPr-C₆H₄ | Me | iPr | Me |
| 1-176 | 4-NHSO₂cPr-C₆H₄ | Me | iPr | H |
| 1-177 | 4-NHSO₂cPr-C₆H₄ | Me | cPr | Me |
| 1-178 | 4-NHSO₂cPr-C₆H₄ | Me | cPr | H |
| 1-179 | 4-NHSO₂cPr-C₆H₄ | Me | iBu | Me |
| 1-180 | 4-NHSO₂cPr-C₆H₄ | Me | iBu | H |
| 1-181 | 4-NHSO₂cPr-C₆H₄ | F | iPr | Me |
| 1-182 | 4-NHSO₂cPr-C₆H₄ | F | iPr | H |
| 1-183 | 4-NHSO₂cPr-C₆H₄ | F | cPr | Me |
| 1-184 | 4-NHSO₂cPr-C₆H₄ | F | cPr | H |
| 1-185 | 4-NHSO₂cPr-C₆H₄ | F | iBu | Me |
| 1-186 | 4-NHSO₂cPr-C₆H₄ | F | iBu | H |
| 1-187 | 4-NHSO₂cPr-C₆H₄ | Cl | iPr | Me |
| 1-188 | 4-NHSO₂cPr-C₆H₄ | Cl | iPr | H |
| 1-189 | 4-NHSO₂cPr-C₆H₄ | Cl | cPr | Me |
| 1-190 | 4-NHSO₂cPr-C₆H₄ | Cl | cPr | H |
| 1-191 | 4-NHSO₂cPr-C₆H₄ | Cl | iBu | Me |
| 1-192 | 4-NHSO₂cPr-C₆H₄ | Cl | iBu | H |
| 1-193 | 4-SO₂NH₂-C₆H₄ | H | iPr | Me |
| 1-194 | 4-SO₂NH₂-C₆H₄ | H | iPr | H |
| 1-195 | 4-SO₂NH₂-C₆H₄ | H | cPr | Me |
| 1-196 | 4-SO₂NH₂-C₆H₄ | H | cPr | H |
| 1-197 | 4-SO₂NH₂-C₆H₄ | H | iBu | Me |
| 1-198 | 4-SO₂NH₂-C₆H₄ | H | iBu | H |
| 1-199 | 4-SO₂NH₂-C₆H₄ | Me | iPr | Me |
| 1-200 | 4-SO₂NH₂-C₆H₄ | Me | iPr | H |
| 1-201 | 4-SO₂NH₂-C₆H₄ | Me | cPr | Me |
| 1-202 | 4-SO₂NH₂-C₆H₄ | Me | cPr | H |
| 1-203 | 4-SO₂NH₂-C₆H₄ | Me | iBu | Me |
| 1-204 | 4-SO₂N-H₂-C₆H₄ | Me | iBu | H |
| 1-205 | 4-SO₂NH₂-C₆H₄ | F | iPr | Me |
| 1-206 | 4-SO₂NH₂-C₆H₄ | F | iPr | H |
| 1-207 | 4-SO₂NH₂-C₆H₄ | F | cPr | Me |
| 1-208 | 4-SO₂NH₂-C₆H₄ | F | cPr | H |
| 1-209 | 4-SO₂NH₂-C₆H₄ | F | iBu | Me |
| 1-210 | 4-SO₂NH₂-C₆H₄ | F | iBu | H |
| 1-211 | 4-SO₂NH₂-C₆H₄ | Cl | iPr | Me |
| 1-212 | 4-SO₂NH₂-C₆H₄ | Cl | iPr | H |
| 1-213 | 4-SO₂NH₂-C₆H₄ | Cl | cPr | Me |
| 1-214 | 4-SO₂NH₂-C₆H₄ | Cl | cPr | H |
| 1-215 | 4-SO₂NH₂-C₆H₄ | Cl | iBu | Me |
| 1-216 | 4-SO₂NH₂-C₆H₄ | Cl | iBu | H |
| 1-217 | 4-[CONH-(4-Morpho)]-C₆H₄ | H | iPr | Me |
| 1-218 | 4-[CONH-(4-Morpho)]-C₆H₄ | H | iPr | H |
| 1-219 | 4-[CONH-(4-Morpho)]-C₆H₄ | H | cPr | Me |
| 1-220 | 4-[CONH-(4-Morpho)]-C₆H₄ | H | cPr | H |
| 1-221 | 4-[CONH-(4-Morpho)]-C₆H₄ | H | iBu | Me |
| 1-222 | 4-[CONH-(4-Morpho)]-C₆H₄ | H | iBu | H |
| 1-223 | 4-[CONH-(4-Morpho)]-C₆H₄ | Me | iPr | Me |
| 1-224 | 4-[CONH-(4-Morpho)]-C₆H₄ | Me | iPr | H |
| 1-225 | 4-[CONH-(4-Morpho)]-C₆H₄ | Me | cPr | Me |
| 1-226 | 4-[CONH-(4-Morpho)]-C₆H₄ | Me | cPr | H |
| 1-227 | 4-[CONH-(4-Morpho)]-C₆H₄ | Me | iBu | Me |
| 1-228 | 4-[CONH-(4-Morpho)]-C₆H₄ | Me | iBu | H |
| 1-229 | 4-[CONH-(4-Morpho)]-C₆H₄ | F | iPr | Me |
| 1-230 | 4-[CONH-(4-Morpho)]-C₆H₄ | F | iPr | H |
| 1-231 | 4-[CONH-(4-Morpho)]-C₆H₄ | F | cPr | Me |
| 1-232 | 4-[CONH-(4-Morpho)]-C₆H₄ | F | cPr | H |
| 1-233 | 4-[CONH-(4-Morpho)]-C₆H₄ | F | iBu | Me |
| 1-234 | 4-[CONH-(4-Morpho)]-C₆H₄ | F | iBu | H |
| 1-235 | 4-[CONH-(4-Morpho)]-C₆H₄ | Cl | iPr | Me |
| 1-236 | 4-[CONH-(4-Morpho)]-C₆H₄ | Cl | iPr | H |
| 1-237 | 4-[CONH-(4-Morpho)]-C₆H₄ | Cl | cPr | Me |
| 1-238 | 4-[CONH-(4-Morpho)]-C₆H₄ | Cl | cPr | H |
| 1-239 | 4-[CONH-(4-Morpho)]-C₆H₄ | Cl | iBu | Me |
| 1-240 | 4-[CONH-(4-Morpho)]-C₆H₄ | Cl | iBu | H |
| 1-241 | 4-[CONH-(4-Me-1-Pipera)]-C₆H₄ | H | iPr | Me |
| 1-242 | 4-[CONH-(4-Me-1-Pipera)]-C₆H₄ | H | iPr | H |
| 1-243 | 4-[CONH-(4-Me-1-Pipera)]-C₆H₄ | H | cPr | Me |
| 1-244 | 4-[CONH-(4-Me-1-Pipera)]-C₆H₄ | H | cPr | H |
| 1-245 | 4-[CONH-(4-Me-1-Pipera)]-C₆H₄ | H | iBu | Me |
| 1-246 | 4-[CONH-(4-Me-1-Pipera)]-C₆H₄ | H | iBu | H |
| 1-247 | 4-[CONH-(4-Me-1-Pipera)]-C₆H₄ | Me | iPr | Me |
| 1-248 | 4-[CONH-(4-Me-1-Pipera)]-C₆H₄ | Me | iPr | H |
| 1-249 | 4-[CONH-(4-Me-1-Pipera)]-C₆H₄ | Me | cPr | Me |
| 1-250 | 4-[CONH-(4-Me-1-Pipera)]-C₆H₄ | Me | cPr | H |
| 1-251 | 4-[CONH-(4-Me-1-Pipera)]-C₆H₄ | Me | iBu | Me |
| 1-252 | 4-[CONH-(4-Me-1-Pipera)]-C₆H₄ | Me | iBu | H |
| 1-253 | 4-[CONH-(4-Me-1-Pipera)]-C₆H₄ | F | iPr | Me |
| 1-254 | 4-[CONH-(4-Me-1-Pipera)]-C₆H₄ | F | iPr | H |
| 1-255 | 4-[CONH-(4-Me-1-Pipera)]-C₆H₄ | F | cPr | Me |
| 1-256 | 4-[CONH-(4-Me-1-Pipera)]-C₆H₄ | F | cPr | H |
| 1-257 | 4-[CONH-(4-Me-1-Pipera)]-C₆H₄ | F | iBu | Me |
| 1-258 | 4-[CONH-(4-Me-1-Pipera)]-C₆H₄ | F | iBu | H |
| 1-259 | 4-[CONH-(4-Me-1-Pipera)]-C₆H₄ | Cl | iPr | Me |
| 1-260 | 4-[CONH-(4-Me-1-Pipera)]-C₆H₄ | Cl | iPr | H |
| 1-261 | 4-[CONH-(4-Me-1-Pipera)]-C₆H₄ | Cl | cPr | Me |
| 1-262 | 4-[CONH-(4-Me-1-Pipera)]-C₆H₄ | Cl | cPr | H |
| 1-263 | 4-[CONH-(4-Me-1-Pipera)]-C₆H₄ | Cl | iBu | Me |
| 1-264 | 4-[CONH-(4-Me-1-Pipera)]-C₆H₄ | Cl | iBu | H |
| 1-265 | 3-Thi | H | iPr | Me |
| 1-266 | 3-Thi | H | iPr | H |
| 1-267 | 3-Thi | H | cPr | Me |
| 1-268 | 3-Thi | H | cPr | H |
| 1-269 | 3-Thi | H | iBu | Me |
| 1-270 | 3-Thi | H | iBu | H |
| 1-271 | 3-Thi | Me | iPr | Me |
| 1-272 | 3-Thi | Me | iPr | H |
| 1-273 | 3-Thi | Me | cPr | Me |
| 1-274 | 3-Thi | Me | cPr | H |
| 1-275 | 3-Thi | Me | iBu | Me |
| 1-276 | 3-Thi | Me | iBu | H |
| 1-277 | 3-Thi | F | iPr | Me |
| 1-278 | 3-Thi | F | iPr | H |
| 1-279 | 3-Thi | F | cPr | Me |
| 1-280 | 3-Thi | F | cPr | H |
| 1-281 | 3-Thi | F | iBu | Me |
| 1-282 | 3-Thi | F | iBu | H |
| 1-283 | 3-Thi | Cl | iPr | Me |
| 1-284 | 3-Thi | Cl | iPr | H |
| 1-285 | 3-Thi | Cl | cPr | Me |
| 1-286 | 3-Thi | Cl | cPr | H |
| 1-287 | 3-Thi | Cl | iBu | Me |
| 1-288 | 3-Thi | Cl | iBu | H |
| 1-289 | 2-Pyrro | H | iPr | Me |
| 1-290 | 2-Pyrro | H | iPr | H |
| 1-291 | 2-Pyrro | H | cPr | Me |
| 1-292 | 2-Pyrro | H | cPr | H |
| 1-293 | 2-Pyrro | H | iBu | Me |
| 1-294 | 2-Pyrro | H | iBu | H |
| 1-295 | 2-Pyrro | Me | iPr | Me |
| 1-296 | 2-Pyrro | Me | iPr | H |
| 1-297 | 2-Pyrro | Me | cPr | Me |
| 1-298 | 2-Pyrro | Me | cPr | H |
| 1-299 | 2-Pyrro | Me | iBu | Me |
| 1-300 | 2-Pyrro | Me | iBu | H |
| 1-301 | 2-Pyrro | F | iPr | Me |
| 1-302 | 2-Pyrro | F | iPr | H |
| 1-303 | 2-Pyrro | F | cPr | Me |
| 1-304 | 2-Pyrro | F | cPr | H |
| 1-305 | 2-Pyrro | F | iBu | Me |
| 1-306 | 2-Pyrro | F | iBu | H |
| 1-307 | 2-Pyrro | Cl | iPr | Me |
| 1-308 | 2-Pyrro | Cl | iPr | H |
| 1-309 | 2-Pyrro | Cl | cPr | Me |
| 1-310 | 2-Pyrro | Cl | cPr | H |
| 1-311 | 2-Pyrro | Cl | iBu | Me |
| 1-312 | 2-Pyrro | Cl | iBu | H |
| 1-313 | 5-CONH₂-2-Py | H | iPr | Me |
| 1-314 | 5-CONH₂-2-Py | H | iPr | H |
| 1-315 | 5-CONH₂-2-Py | H | cPr | Me |
| 1-316 | 5-CONH₂-2-Py | H | cPr | H |
| 1-317 | 5-CONH₂-2-Py | H | iBu | Me |
| 1-318 | 5-CONH₂-2-Py | H | iBu | H |
| 1-319 | 5-CONH₂-2-Py | Me | iPr | Me |
| 1-320 | 5-CONH₂-2-Py | Me | iPr | H |
| 1-321 | 5-CONH₂-2-Py | Me | cPr | Me |
| 1-322 | 5-CONH₂-2-Py | Me | cPr | H |
| 1-323 | 5-CONH₂-2-Py | Me | iBu | Me |
| 1-324 | 5-CONH₂-2-Py | Me | iBu | H |
| 1-325 | 5-CONH₂-2-Py | F | iPr | Me |
| 1-326 | 5-CONH₂-2-Py | F | iPr | H |
| 1-327 | 5-CONH₂-2-Py | F | cPr | Me |
| 1-328 | 5-CONH₂-2-Py | F | cPr | H |
| 1-329 | 5-CONH₂-2-Py | F | iBu | Me |
| 1-330 | 5-CONH₂-2-Py | F | iBu | H |
| 1-331 | 5-CONH₂-2-Py | Cl | iPr | Me |
| 1-332 | 5-CONH₂-2-Py | Cl | iPr | H |
| 1-333 | 5-CONH₂-2-Py | Cl | cPr | Me |
| 1-334 | 5-CONH₂-2-Py | Cl | cPr | H |
| 1-335 | 5-CONH₂-2-Py | Cl | iBu | Me |
| 1-336 | 5-CONH₂-2-Py | Cl | iBu | H |
| 1-337 | 4-SO₂Et-C₆H₄ | H | iPr | Me |
| 1-338 | 4-SO₂Et-C₆H₄ | H | iPr | H |
| 1-339 | 4-SO₂Et-C₆H₄ | H | cPr | Me |
| 1-340 | 4-SO₂Et-C₆H₄ | H | cPr | H |
| 1-341 | 4-SO₂Et-C₆H₄ | H | iBu | Me |
| 1-342 | 4-SO₂Et-C₆H₄ | H | iBu | H |
| 1-343 | 4-NHCOMe-C₆H₄ | H | iPr | Me |
| 1-344 | 4-NHCOMe-C₆H₄ | H | iPr | H |
| 1-345 | 4-NHCOMe-C₆H₄ | H | cPr | Me |
| 1-346 | 4-NHCOMe-C₆H₄ | H | cPr | H |
| 1-347 | 4-NHCOMe-C₆H₄ | H | iBu | Me |
| 1-348 | 4-NHCOMe-C₆H₄ | H | iBu | H |
| 1-349 | 4-NHCOCMe₂OH-C₆H₄ | H | iPr | Me |
| 1-350 | 4-NHCOCMe₂OH-C₆H₄ | H | iPr | H |
| 1-351 | 4-NHCOCMe₂OH-C₆H₄ | H | cPr | Me |
| 1-352 | 4-NHCOCMe₂OH-C₆H₄ | H | cPr | H |
| 1-353 | 4-NHCOCMe₂OH-C₆H₄ | H | iBu | Me |
| 1-354 | 4-NHCOCMe₂OH-C₆H₄ | H | iBu | H |
| 1-355 | 4-NHCOCMe₂OCOMe-C₆H₄ | H | iPr | Me |
| 1-356 | 4-NHCOCMe₂OCOMe-C₆H₄ | H | iPr | H |
| 1-357 | 4-NHCOCMe₂OCOMe-C₆H₄ | H | cPr | Me |
| 1-358 | 4-NHCOCMe₂OCOMe-C₆H₄ | H | cPr | H |
| 1-359 | 4-NHCOCMe₂OCOMe-C₆H₄ | H | iBu | Me |
| 1-360 | 4-NHCOCMe₂OCOMe-C₆H₄ | H | iBu | H |
| 1-361 | 4-CONH₂-C₆H₄ | H | iPr | Me |
| 1-362 | 4-CONH₂-C₆H₄ | H | iPr | H |
| 1-363 | 4-CONH₂-C₆H₄ | H | cPr | Me |
| 1-364 | 4-CONH₂-C₆H₄ | H | cPr | H |
| 1-365 | 4-CONH₂-C₆H₄ | H | iBu | Me |
| 1-366 | 4-CONH₂-C₆H₄ | H | iBu | H |
| 1-367 | 3-CONH₂-C₆H₄ | H | iPr | Me |
| 1-368 | 3-CONH₂-C₆H₄ | H | iPr | H |
| 1-369 | 3-CONH₂-C₆H₄ | H | cPr | Me |
| 1-370 | 3-CONH₂-C₆H₄ | H | cPr | H |
| 1-371 | 3-CONH₂-C₆H₄ | H | iBu | Me |
| 1-372 | 3-CONH₂-C₆H₄ | H | iBu | H |
| 1-373 | 4-CONHCH₂CH₂OH-C₆H₄ | H | iPr | Me |
| 1-374 | 4-CONHCH₂CH₂OH-C₆H₄ | H | iPr | H |
| 1-375 | 4-CONHCH₂CH₂OH-C₆H₄ | H | cPr | Me |
| 1-376 | 4-CONHCH₂CH₂OH-C₆H₄ | H | cPr | H |
| 1-377 | 4-CONHCH₂CH₂OH-C₆H₄ | H | iBu | Me |
| 1-378 | 4-CONHCH₂CH₂OH-C₆H₄ | H | iBu | H |
| 1-379 | 4-SO₂NHMe-C₆H₄ | H | iPr | Me |
| 1-380 | 4-SO₂NHMe-C₆H₄ | H | iPr | H |
| 1-381 | 4-SO₂NHMe-C₆H₄ | H | cPr | Me |
| 1-382 | 4-SO₂NHMe-C₆H₄ | H | cPr | H |
| 1-383 | 4-SO₂NHMe-C₆H₄ | H | iBu | Me |
| 1-384 | 4-SO₂NHMe-C₆H₄ | H | iBu | H |
| 1-385 | 4-[CO-(4-Morpho)]-C₆H₄ | H | iPr | Me |
| 1-386 | 4-[CO-(4-Morpho)]-C₆H₄ | H | iPr | H |
| 1-387 | 4-[CO-(4-Morpho)]-C₆H₄ | H | cPr | Me |
| 1-388 | 4-[CO-(4-Morpho)]-C₆H₄ | H | cPr | H |
| 1-389 | 4-[CO-(4-Morpho)]-C₆H₄ | H | iBu | Me |
| 1-390 | 4-[CO-(4-Morpho)]-C₆H₄ | H | iBu | H |
| 1-391 | 4-[SO₂-(4-Morpho)]-C₆H₄ | H | iPr | Me |
| 1-392 | 4-[SO₂-(4-Morpho)]-C₆H₄ | H | iPr | H |
| 1-393 | 4-[SO₂-(4-Morpho)]-C₆H₄ | H | cPr | Me |
| 1-394 | 4-[SO₂-(4-Morpho)]-C₆H₄ | H | cPr | H |
| 1-395 | 4-[SO₂-(4-Morpho)]-C₆H₄ | H | iBu | Me |
| 1-396 | 4-[SO₂-(4-Morpho)]-C₆H₄ | H | iBu | H |
| 1-397 | 3-Fur | H | iPr | Me |
| 1-398 | 3-Fur | H | iPr | H |
| 1-399 | 3-Fur | H | cPr | Me |
| 1-400 | 3-Fur | H | cPr | H |
| 1-401 | 3-Fur | H | iBu | Me |
| 1-402 | 3-Fur | H | iBu | H |
| 1-403 | 2-OMe-5-Py | H | iPr | Me |
| 1-404 | 2-OMe-5-Py | H | iPr | H |
| 1-405 | 2-OMe-5-Py | H | cPr | Me |
| 1-406 | 2-OMe-5-Py | H | cPr | H |
| 1-407 | 2-OMe-5-Py | H | iBu | Me |
| 1-408 | 2-OMe-5-Py | H | iBu | H |
| 1-409 | 4-Py | H | iPr | Me |
| 1-410 | 4-Py | H | iPr | H |
| 1-411 | 4-Py | H | cPr | Me |
| 1-412 | 4-Py | H | cPr | H |
| 1-413 | 4-Py | H | iBu | Me |
| 1-414 | 4-Py | H | iBu | H |
| 1-415 | 4-F-C₆H₄ | H | iPr | Me |
| 1-416 | 4-F-C₆H₄ | H | iPr | H |
| 1-417 | 4-F-C₆H₄ | H | cPr | Me |
| 1-418 | 4-F-C₆H₄ | H | cPr | H |
| 1-419 | 4-F-C₆H₄ | H | iBu | Me |
| 1-420 | 4-F-C₆H₄ | H | iBu | H |
| 1-421 | 3-F-C₆H₄ | H | iPr | Me |
| 1-422 | 3-F-C₆H₄ | H | iPr | H |
| 1-423 | 3-F-C₆H₄ | H | cPr | Me |
| 1-424 | 3-F-C₆H₄ | H | cPr | H |
| 1-425 | 3-F-C₆H₄ | H | iBu | Me |
| 1-426 | 3-F-C₆H₄ | H | iBu | H |
| 1-427 | 2-F-C₆H₄ | H | iPr | Me |
| 1-428 | 2-F-C₆H₄ | H | iPr | H |
| 1-429 | 2-F-C₆H₄ | H | cPr | Me |
| 1-430 | 2-F-C₆H₄ | H | cPr | H |
| 1-431 | 2-F-C₆H₄ | H | iBu | Me |
| 1-432 | 2-F-C₆H₄ | H | iBu | H |
| 1-433 | 4-Me-C₆H₄ | H | iPr | Me |
| 1-434 | 4-Me-C₆H₄ | H | iPr | H |
| 1-43 5 | 4-Me-C₆H₄ | H | cPr | Me |
| 1-436 | 4-Me-C₆H₄ | H | cPr | H |
| 1-437 | 4-Me-C₆H₄ | H | iBu | Me |
| 1-438 | 4-Me-C₆H₄ | H | iBu | H |
| 1-439 | 4-NO₂-C₆H₄ | H | iPr | Me |
| 1-440 | 4-NO₂-C₆H₄ | H | iPr | H |
| 1-441 | 4-NO₂-C₆H₄ | H | cPr | Me |
| 1-442 | 4-NO₂-C₆H₄ | H | cPr | H |
| 1-443 | 4-NO₂-C₆H₄ | H | iBu | Me |
| 1-444 | 4-NO₂-C₆H₄ | H | iBu | H |
| 1-445 | 4-CO₂Me-C₆H₄ | H | iPr | Me |
| 1-446 | 4-CO₂Me-C₆H₄ | H | iPr | H |
| 1-447 | 4-CO₂Me-C₆H₄ | H | cPr | Me |
| 1-448 | 4-CO₂Me-C₆H₄ | H | cPr | H |
| 1-449 | 4-CO₂Me-C₆H₄ | H | iBu | Me |
| 1-450 | 4-CO₂Me-C₆H₄ | H | iBu | H |
| 1-451 | 4-SO₂NMe₂-C₆H₄ | H | iPr | Me |
| 1-452 | 4-SO₂NMe₂-C₆H₄ | H | iPr | H |
| 1-453 | 4-SO₂NMe₂-C₆H₄ | H | cPr | Me |
| 1-454 | 4-SO₂NMe₂-C₆H₄ | H | cPr | H |
| 1-455 | 4-SO₂NMe₂-C₆H₄ | H | iBu | Me |
| 1-456 | 4-SO₂NMe₂-C₆H₄ | H | iBu | H |
| 1-457 | 3-NHSO₂Me-C₆H₄ | H | iPr | Me |
| 1-458 | 3-NHSO₂Me-C₆H₄ | H | iPr | H |
| 1-459 | 3-NHSO₂Me-C₆H₄ | H | cPr | Me |
| 1-460 | 3-NHSO₂Me-C₆H₄ | H | cPr | H |
| 1-461 | 3-NHSO₂Me-C₆H₄ | H | iBu | Me |
| 1-462 | 3-NHSO₂Me-C₆H₄ | H | iBu | H |
| 1-463 | 4-[SO₂-(4-Me-1-Pipera)]-C₆H₄ | H | iPr | Me |
| 1-464 | 4-[SO₂-(4-Me-1-Pipera)]-C₆H₄ | H | iPr | H |
| 1-465 | 4-[SO₂-(4-Me-1-Pipera)]-C₆H₄ | H | cPr | Me |
| 1-466 | 4-[SO₂-(4-Me-1-Pipera)]-C₆H₄ | H | cPr | H |
| 1-467 | 4-[SO₂-(4-Me-1-Pipera)]-C₆H₄ | H | iBu | Me |
| 1-468 | 4-[SO₂-(4-Me-1-Pipera)]-C₆H₄ | H | iBu | H |
| 1-469 | 2-Thi | H | iPr | Me |
| 1-470 | 2-Thi | H | iPr | H |
| 1-471 | 2-Thi | H | cPr | Me |
| 1-472 | 2-Thi | H | cPr | H |
| 1-473 | 2-Thi | H | iBu | Me |
| 1-474 | 2-Thi | H | iBu | H |
| 1-475 | 5-CONH₂-2-Thi | H | iPr | Me |
| 1-476 | 5-CONH₂-2-Thi | H | iPr | H |
| 1-477 | 5-CONH₂-2-Thi | H | cPr | Me |
| 1-478 | 5-CONH₂-2-Thi | H | cPr | H |
| 1-479 | 5-CONH₂-2-Thi | H | iBu | Me |
| 1-480 | 5-CONH₂-2-Thi | H | iBu | H |
| 1-481 | 3-Py | H | iPr | Me |
| 1-482 | 3-Py | H | iPr | H |
| 1-483 | 3-Py | H | cPr | Me |
| 1-484 | 3-Py | H | cPr | H |
| 1-485 | 3-Py | H | iBu | Me |
| 1-486 | 3-Py | H | iBu | H |
| 1-487 | 2-CONH₂-5-Py | H | iPr | Me |
| 1-488 | 2-CONH₂-5-Py | H | iPr | H |
| 1-489 | 2-CONH₂-5-Py | H | cPr | Me |
| 1-490 | 2-CONH₂-5-Py | H | cPr | H |
| 1-491 | 2-CONH₂-5-Py | H | iBu | Me |
| 1-492 | 2-CONH₂-5-Py | H | iBu | H |
| 1-493 | 4-CF₃-C₆H₄ | H | iPr | Me |
| 1-494 | 4-CF₃-C₆H₄ | H | cPr | Me |
| 1-495 | 4-CF₃-C₆H₄ | H | iBu | Me |
| 1-496 | 2,4-di-F-C₆H₃ | H | iPr | Me |
| 1-497 | 2,4-di-F-C₆H₃ | H | cPr | Me |
| 1-498 | 2,4-di-F-C₆H₃ | H | iBu | Me |
| 1-499 | 4-OCF₃-C₆H₄ | H | iPr | Me |
| 1-500 | 4-OCF₃-C₆H₄ | H | cPr | Me |
| 1-501 | 4-OCF₃-C₆H₄ | H | iBu | Me |
| 1-502 | 3-SO₂Me-C₆H₄ | H | iPr | Me |
| 1-503 | 3-SO₂Me-C₆H₄ | H | cPr | Me |
| 1-504 | 3-SO₂Me-C₆H₄ | H | iBu | Me |
| 1-505 | 4-COMe-C₆H₄ | H | iPr | Me |
| 1-506 | 4-COMe-C₆H₄ | H | cPr | Me |
| 1-507 | 4-COMe-C₆H₄ | H | iBu | Me |
| 1-508 | 4-CH₂NH₂-C₆H₄ | H | iPr | Me |
| 1-509 | 4-CH₂NH₂-C₆H₄ | H | cPr | Me |
| 1-510 | 4-CH₂NH₂-C₆H₄ | H | iBu | Me |
| 1-511 | 3-SO₂NH₂-C₆H₄ | H | iPr | Me |
| 1-512 | 3-SO₂NH₂-C₆H₄ | H | cPr | Me |
| 1-513 | 3-SO₂NH₂-C₆H₄ | H | iBu | Me |
| 1-514 | 2-Py | H | iPr | Me |
| 1-515 | 2-Py | H | cPr | Me |
| 1-516 | 2-Py | H | iBu | Me |
| 1-517 | 4-CO₂H-C₆H₄ | H | iPr | Me |
| 1-518 | 4-CO₂H-C₆H₄ | H | cPr | Me |
| 1-519 | 4-CO₂H-C₆H₄ | H | iBu | Me |
| 1-520 | 4-[CO-(4-Me-1-Pipera)]-C₆H₄ | H | iPr | Me |
| 1-521 | 4-[CO-(4-Me-1-Pipera)]-C₆H₄ | H | cPr | Me |
| 1-522 | 4-[CO-(4-Me-1-Pipera)]-C₆H₄ | H | iBu | Me |
| 1-523 | 4-[CO-(4-Thiomorpho)]-C₆H₄ | H | iPr | Me |
| 1-524 | 4-[CO-(4-Thiomorpho)]-C₆H₄ | H | cPr | Me |
| 1-525 | 4-[CO-(4-Thiomorpho)]-C₆H₄ | H | iBu | Me |
| 1-526 | 4-[CO-(1-Oxi-4-thiomorpho)]-C₆H₄ | H | iPr | Me |
| 1-527 | 4-[CO-(1-Oxi-4-thiomorpho)]-C₆H₄ | H | cPr | Me |
| 1-528 | 4-[CO-(1-Oxi-4-thiomorpho)]-C₆H₄ | H | iBu | Me |
| 1-529 | 4-[CO-(1,1-Dioxi-4-thiomorpho)]-C₆H₄ | H | iPr | Me |
| 1-530 | 4-[CO-(1,1-Dioxi-4-thiomorpho)]-C₆H₄ | H | cPr | Me |
| 1-531 | 4-[CO-(1,1-Dioxi-4-thiomorpho)]-C₆H₄ | H | iBu | Me |
| 1-532 | 4-[CONH-(4-Tet-pyra)]-C₆H₄ | H | iPr | Me |
| 1-533 | 4-[CONH-(4-Tet-pyra)]-C₆H₄ | H | cPr | Me |
| 1-534 | 4-[CONH-(4-Tet-pyra)]-C₆H₄ | H | iBu | Me |
| 1-535 | 4-[CONH-(4-Tet-pyra)]-C₆H₄ | H | tBu | Me |
| 1-536 | 5-SO₂Me-2-Py | H | iPr | Me |
| 1-537 | 5-SO₂Me-2-Py | H | cPr | Me |
| 1-538 | 5-SO₂Me-2-Py | H | iBu | Me |
| 1-539 | 4-[CH₂-(4-Morpho)]-C₆H₄ | H | iPr | Me |
| 1-540 | 4-[CH₂-(4-Morpho)]-C₆H₄ | H | iPr | H |
| 1-541 | 4-[CH₂-(4-Morpho)]-C₆H₄ | H | cPr | Me |
| 1-542 | 4-[CH₂-(4-Morpho)]-C₆H₄ | H | cPr | H |
| 1-543 | 4-[CH₂-(4-Morpho)]-C₆H₄ | H | iBu | Me |
| 1-544 | 4-[CH₂-(4-Morpho)]-C₆H₄ | H | iBu | H |
| 1-545 | 4-[CH₂-(4-Morpho)]-C₆H₄ | Me | iPr | Me |
| 1-546 | 4-[CH₂-(4-Morpho)]-C₆H₄ | Me | iPr | H |
| 1-547 | 4-[CH₂-(4-Morpho)]-C₆H₄ | Me | cPr | Me |
| 1-548 | 4-[CH₂-(4-Morpho)]-C₆H₄ | Me | cPr | H |
| 1-549 | 4-[CH₂-(4-Morpho)]-C₆H₄ | Me | iBu | Me |
| 1-550 | 4-[CH₂-(4-Morpho)]-C₆H₄ | Me | iBu | H |
| 1-551 | 4-[CH₂-(4-Morpho)]-C₆H₄ | F | iPr | Me |
| 1-552 | 4-[CH₂-(4-Morpho)]-C₆H₄ | F | iPr | H |
| 1-553 | 4-[CH₂-(4-Morpho)]-C₆H₄ | F | cPr | Me |
| 1-554 | 4-[CH₂-(4-Morpho)]-C₆H₄ | F | cPr | H |
| 1-555 | 4-[CH₂-(4-Morpho)]-C₆H₄ | F | iBu | Me |
| 1-556 | 4-[CH₂-(4-Morpho)]-C₆H₄ | F | iBu | H |
| 1-557 | 4-[CH₂-(4-Morpho)]-C₆H₄ | Cl | iPr | Me |
| 1-558 | 4-[CH₂-(4-Morpho)]-C₆H₄ | Cl | iPr | H |
| 1-559 | 4-[CH₂-(4-Morpho)]-C₆H₄ | Cl | cPr | Me |
| 1-560 | 4-[CH₂-(4-Morpho)]-C₆H₄ | Cl | cPr | H |
| 1-561 | 4-[CH₂-(4-Morpho)]-C₆H₄ | Cl | iBu | Me |
| 1-562 | 4-[CH₂-(4-Morpho)]-C₆H₄ | Cl | iBu | H |
| 1-563 | 4-[NHCONH-(4-Me-1-Pipera)]-C₆H₄ | H | iPr | Me |
| 1-564 | 4-[NHCONH-(4-Me-1-Pipera)]-C₆H₄ | H | iPr | H |
| 1-565 | 4-[NHCONH-(4-Me-1-Pipera)]-C₆H₄ | H | cPr | Me |
| 1-566 | 4-[NHCONH-(4-Me-1-Pipera)]-C₆H₄ | H | cPr | H |
| 1-567 | 4-[NHCONH-(4-Me-1-Pipera)]-C₆H₄ | H | iBu | Me |
| 1-568 | 4-[NHCONH-(4-Me-1-Pipera)]-C₆H₄ | H | iBu | H |
| 1-569 | 4-[NHCONH-(4-Me-1-Pipera)]-C₆H₄ | Me | iPr | Me |
| 1-570 | 4-[NHCONH-(4-Me-1-Pipera)]-C₆H₄ | Me | iPr | H |
| 1-571 | 4-[NHCONH-(4-Me-1-Pipera)]-C₆H₄ | Me | cPr | Me |
| 1-572 | 4-[NHCONH-(4-Me-1-Pipera)]-C₆H₄ | Me | cPr | H |
| 1-573 | 4-[NHCONH-(4-Me-1-Pipera)]-C₆H₄ | Me | iBu | Me |
| 1-574 | 4-[NHCONH-(4-Me-1-Pipera)]-C₆H₄ | Me | iBu | H |
| 1-575 | 4-[NHCONH-(4-Me-1-Pipera)]-C₆H₄ | F | iPr | Me |
| 1-576 | 4-[NHCONH-(4-Me-1-Pipera)]-C₆H₄ | F | iPr | H |
| 1-577 | 4-[NHCONH-(4-Me-1-Pipera)]-C₆H₄ | F | cPr | Me |
| 1-578 | 4-[NHCONH-(4-Me-1-Pipera)]-C₆H₄ | F | cPr | H |
| 1-579 | 4-[NHCONH-(4-Me-1-Pipera)]-C₆H₄ | F | iBu | Me |
| 1-580 | 4-[NHCONH-(4-Me-1-Pipera)]-C₆H₄ | F | iBu | H |
| 1-581 | 4-[NHCONH-(4-Me-1-Pipera)]-C₆H₄ | Cl | iPr | Me |
| 1-582 | 4-[NHCONH-(4-Me-1-Pipera)]-C₆H₄ | Cl | iPr | H |
| 1-583 | 4-[NHCONH-(4-Me-1-Pipera)]-C₆H₄ | Cl | cPr | Me |
| 1-584 | 4-[NHCONH-(4-Me-1-Pipera)]-C₆H₄ | Cl | cPr | H |
| 1-585 | 4-[NHCONH-(4-Me-1-Pipera)]-C₆H₄ | Cl | iBu | Me |
| 1-586 | 4-[NHCONH-(4-Me-1-Pipera)]-C₆H₄ | Cl | iBu | H |
| 1-587 | 4-SO₂Me-C₆H₄ | H | iPr | Et |
| 1-588 | 4-[CONH-(4-Tet-pyra)]-C₆H₄ | F | iPr | Me |
| 1-589 | 4-[CONH-(4-Tet-pyra)]-C₆H₄ | F | cPr | Me |
| 1-590 | 4-[CONH-(4-Tet-pyra)]-C₆H₄ | F | iBu | Me |
| 1-591 | 4-[CONH-(4-Tet-pyra)]-C₆H₄ | F | tBu | Me |
| 1-592 | 4-[NHCONH-(4-Morpho)]-C₆H₄ | H | iPr | Me |
| 1-593 | 4-[NHCONH-(4-Morpho)]-C₆H₄ | H | iPr | H |
| 1-594 | 4-[NHCONH-(4-Morpho)]-C₆H₄ | H | cPr | Me |
| 1-595 | 4-[NHCONH-(4-Morpho)]-C₆H₄ | H | cPr | H |
| 1-596 | 4-[NHCONH-(4-Morpho)]-C₆H₄ | H | iBu | Me |
| 1-597 | 4-[NHCONH-(4-Morpho)]-C₆H₄ | H | iBu | H |
| 1-598 | 4-[NHCONH-(4-Morpho)]-C₆H₄ | Me | iPr | Me |
| 1-599 | 4-[NHCONH-(4-Morpho)]-C₆H₄ | Me | iPr | H |
| 1-600 | 4-[NHCONH-(4-Morpho)]-C₆H₄ | Me | cPr | Me |
| 1-601 | 4-[NHCONH-(4-Morpho)]-C₆H₄ | Me | cPr | H |
| 1-602 | 4-[NHCONH-(4-Morpho)]-C₆H₄ | Me | iBu | Me |
| 1-603 | 4-[NHCONH-(4-Morpho)]-C₆H₄ | Me | iBu | H |
| 1-604 | 4-[NHCONH-(4-Morpho)]-C₆H₄ | F | iPr | Me |
| 1-605 | 4-[NHCONH-(4-Morpho)]-C₆H₄ | F | iPr | H |
| 1-606 | 4-[NHCONH-(4-Morpho)]-C₆H₄ | F | cPr | Me |
| 1-607 | 4-[NHCONH-(4-Morpho)]-C₆H₄ | F | cPr | H |
| 1-608 | 4-[NHCONH-(4-Morpho)]-C₆H₄ | F | iBu | Me |
| 1-609 | 4-[NHCONH-(4-Morpho)]-C₆H₄ | F | iBu | H |
| 1-610 | 4-[NHCONH-(4-Morpho)]-C₆H₄ | Cl | iPr | Me |
| 1-611 | 4-[NHCONH-(4-Morpho)]-C₆H₄ | Cl | iPr | H |
| 1-612 | 4-[NHCONH-(4-Morpho)]-C₆H₄ | Cl | cPr | Me |
| 1-613 | 4-[NHCONH-(4-Morpho)]-C₆H₄ | Cl | cPr | H |
| 1-614 | 4-[NHCONH-(4-Morpho)]-C₆H₄ | Cl | iBu | Me |
| 1-615 | 4-[NHCONH-(4-Morpho)]-C₆H₄ | Cl | iBu | H |
| 1-616 | 4-[NHCO-(4-Me-1-Pipera)]-C₆H₄ | H | iPr | Me |
| 1-617 | 4-[NHCO-(4-Me-1-Pipera)]-C₆H₄ | H | iPr | H |
| 1-618 | 4-[NHCO-(4-Me-1-Pipera)]-C₆H₄ | H | cPr | Me |
| 1-619 | 4-[NHCO-(4-Me-1-Pipera)]-C₆H₄ | H | cPr | H |
| 1-620 | 4-[NHCO-(4-Me-1-Pipera)]-C₆H₄ | H | iBu | Me |
| 1-621 | 4-[NHCO-(4-Me-1-Pipera)]-C₆H₄ | H | iBu | H |
| 1-622 | 4-[NHCO-(4-Me-1-Pipera)]-C₆H₄ | F | iPr | Me |
| 1-623 | 4-[NHCO-(4-Me-1-Pipera)]-C₆H₄ | F | iPr | H |
| 1-624 | 4-[NHCO-(4-Me-1-Pipera)]-C₆H₄ | F | cPr | Me |
| 1-625 | 4-[NHCO-(4-Me-1-Pipera)]-C₆H₄ | F | cPr | H |
| 1-626 | 4-[NHCO-(4-Me-1-Pipera)]-C₆H₄ | F | iBu | Me |
| 1-627 | 4-[NHCO-(4-Me-1-Pipera)]-C₆H₄ | F | iBu | H |
| 1-628 | 4-[NHCO-(4-Morpho)]-C₆H₄ | H | iPr | Me |
| 1-629 | 4-[NHCO-(4-Morpho)]-C₆H₄ | H | iPr | H |
| 1-630 | 4-[NHCO-(4-Morpho)]-C₆H₄ | H | cPr | Me |
| 1-631 | 4-[NHCO-(4-Morpho)]-C₆H₄ | H | cPr | H |
| 1-632 | 4-[NHCO-(4-Morpho)]-C₆H₄ | H | iBu | Me |
| 1-633 | 4-[NHCO-(4-Morpho)]-C₆H₄ | H | iBu | H |
| 1-634 | 4-[NHCO-(4-Morpho)]-C₆H₄ | F | iPr | Me |
| 1-635 | 4-[NHCO-(4-Morpho)]-C₆H₄ | F | iPr | H |
| 1-636 | 4-[NHCO-(4-Morpho)]-C₆H₄ | F | cPr | Me |
| 1-637 | 4-[NHCO-(4-Morpho)]-C₆H₄ | F | cPr | H |
| 1-638 | 4-[NHCO-(4-Morpho)]-C₆H₄ | F | iBu | Me |
| 1-639 | 4-[NHCO-(4-Morpho)]-C₆H₄ | F | iBu | H |
| 1-640 | 4-[NHCO-(4-Tet-pyra)]-C₆H₄ | H | iPr | Me |
| 1-641 | 4-[NHCO-(4-Tet-pyra)]-C₆H₄ | H | iPr | H |
| 1-642 | 4-[NHCO-(4-Tet-pyra)]-C₆H₄ | H | cPr | Me |
| 1-643 | 4-[NHCO-(4-Tet-pyra)]-C₆H₄ | H | cPr | H |
| 1-644 | 4-[NHCO-(4-Tet-pyra)]-C₆H₄ | H | iBu | Me |
| 1-645 | 4-[NHCO-(4-Tet-pyra)]-C₆H₄ | H | iBu | H |
| 1-646 | 4-[NHCO-(4-Tet-pyra)]-C₆H₄ | F | iPr | Me |
| 1-647 | 4-[NHCO-(4-Tet-pyra)]-C₆H₄ | F | iPr | H |
| 1-648 | 4-[NHCO-(4-Tet-pyra)]-C₆H₄ | F | cPr | Me |
| 1-649 | 4-[NHCO-(4-Tet-pyra)]-C₆H₄ | F | cPr | H |
| 1-650 | 4-[NHCO-(4-Tet-pyra)]-C₆H₄ | F | iBu | Me |
| 1-651 | 4-[NHCO-(4-Tet-pyra)]-C₆H₄ | F | iBu | H |
| 1-652 | 4-[CONH-(1-Me-4-Piperidi)]-C₆H₄ | H | iPr | Me |
| 1-653 | 4-[CONH-(1-Me-4-Piperidi)]-C₆H₄ | H | iPr | H |
| 1-654 | 4-[CONH-(1-Me-4-Piperidi)]-C₆H₄ | H | cPr | Me |
| 1-655 | 4-[CONH-(1-Me-4-Piperidi)]-C₆H₄ | H | cPr | H |
| 1-656 | 4-[CONH-(1-Me-4-Piperidi)]-C₆H₄ | H | iBu | Me |
| 1-657 | 4-[CONH-(1-Me-4-Piperidi)]-C₆H₄ | H | iBu | H |
| 1-658 | 4-[CONH-(1-Me-4-Piperidi)]-C₆H₄ | F | iPr | Me |
| 1-659 | 4-[CONH-(1-Me-4-Piperidi)]-C₆H₄ | F | iPr | H |
| 1-660 | 4-[CONH-(1-Me-4-Piperidi)]-C₆H₄ | F | cPr | Me |
| 1-661 | 4-[CONH-(1-Me-4-Piperidi)]-C₆H₄ | F | cPr | H |
| 1-662 | 4-[CONH-(1-Me-4-Piperidi)]-C₆H₄ | F | iBu | Me |
| 1-663 | 4-[CONH-(1-Me-4-Piperidi)]-C₆H₄ | F | iBu | H |
| 1-664 | 4-[NHCONH-(1-Me-4-Piperidi)]-C₆H₄ | H | iPr | Me |
| 1-665 | 4-[NHCONH-(1-Me-4-Piperidi)]-C₆H₄ | H | iPr | H |
| 1-666 | 4-[NHCONH-(1-Me-4-Piperidi)]-C₆H₄ | H | cPr | Me |
| 1-667 | 4-[NHCONH-(1-Me-4-Piperidi)]-C₆H₄ | H | cPr | H |
| 1-668 | 4-[NHCONH-(1-Me-4-Piperidi)]-C₆H₄ | H | iBu | Me |
| 1-669 | 4-[NHCONH-(1-Me-4-Piperidi)]-C₆H₄ | H | iBu | H |
| 1-670 | 4-[NHCONH-(1-Me-4-Piperidi)]-C₆H₄ | F | iPr | Me |
| 1-671 | 4-[NHCONH-(1-Me-4-Piperidi)]-C₆H₄ | F | iPr | H |
| 1-672 | 4-[NHCONH-(1-Me-4-Piperidi)]-C₆H₄ | F | cPr | Me |
| 1-673 | 4-[NHCONH-(1-Me-4-Piperidi)]-C₆H₄ | F | cPr | H |
| 1-674 | 4-[NHCONH-(1-Me-4-Piperidi)]-C₆H₄ | F | iBu | Me |
| 1-675 | 4-[NHCONH-(1-Me-4-Piperidi)]-C₆H₄ | F | iBu | H |
| 1-676 | 2-F-4-NHSO₂Me-C₆H₄ | H | iPr | Me |
| 1-677 | 2-F-4-NHSO₂Me-C₆H₄ | F | iPr | Me |
| 1-678 | 2-F-4-NHSO₂cPr-C₆H₄ | H | iPr | Me |
| 1-679 | 2-F-4-NHSO₂cPr-C₆H₄ | F | iPr | Me |
| 1-680 | 2-F-4-SO₂NH₂-C₆H₄ | H | iPr | Me |
| 1-681 | 2-F-4-SO₂NH₂-C₆H₄ | F | iPr | Me |
| 1-682 | 3-Pyrro | H | iPr | Me |
| 1-683 | 3-Pyrro | F | iPr | Me |
| 1-684 | 5-NH₂-2-Py | H | iPr | Me |
| 1-685 | 5-NH₂-2-Py | F | iPr | Me |
| 1-686 | 5-NHSO₂Me-2-Py | H | iPr | Me |
| 1-687 | 5-NHSO₂Me-2-Py | F | iPr | Me |
| 1-688 | 5-NHSO₂cPr-2-Py | H | iPr | Me |
| 1-689 | 5-NHSO₂cPr-2-Py | F | iPr | Me |
| 1-690 | 4-[CH₂-(4-Me-1-Pipera)]-C₆H₄ | H | iPr | Me |
| 1-691 | 4-[CH₂-(4-Me-1-Pipera)]-C₆H₄ | F | iPr | Me |
| 1-692 | 4-[CH₂NH-(1-Me-4-Piperidi)]-C₆H₄ | H | iPr | Me |
| 1-693 | 4-[CH₂NH-(1-Me-4-Piperidi)]-C₆H₄ | F | iPr | Me |
| 1-694 | 5-SO₂Me-2-Py | F | iPr | Me |
| 1-695 | 5-SO₂Me-2-Py | F | iPr | H |
| 1-696 | 4-OH-C₆H₄ | H | iPr | Me |
| 1-697 | 4-OH-C₆H₄ | F | iPr | Me |
| 1-698 | 4-[OCH₂CH₂-(1-Pyrrolidi)]-C₆H₄ | H | iPr | Me |
| 1-699 | 4-[OCH₂CH₂-(1-Pyrrolidi)]-C₆H₄ | F | iPr | Me |
| 1-700 | 4-[OCH₂CH₂-(2-Oxo-1-Pyrrolidi)]-C₆H₄ | H | iPr | Me |
| 1-701 | 4-[OCH₂CH₂-(2-Oxo-1-Pyrrolidi)]-C₆H₄ | F | iPr | Me |
| 1-702 | 4-[OCH₂CH₂-(1-Pyrro)]-C₆H₄ | H | iPr | Me |
| 1-703 | 4-[OCH₂CH₂-(1-Pyrro)]-C₆H₄ | F | iPr | Me |
| 1-704 | 4-[OCH₂CH₂-(1-Piperidi)]-C₆H₄ | H | iPr | Me |
| 1-705 | 4-[OCH₂CH₂-(1-Piperidi)]-C₆H₄ | F | iPr | Me |
| 1-706 | 4-[OCH₂CH₂-(4-Morpho)]-C₆H₄ | H | iPr | Me |
| 1-707 | 4-[OCH₂CH₂-(4-Morpho)]-C₆H₄ | F | iPr | Me |
| 1-708 | 4-[CH₂CO-(4-Morpho)]-C₆H₄ | H | iPr | Me |
| 1-709 | 4-[CH₂CO-(4-Morpho)]-C₆H₄ | F | iPr | Me |
| 1-710 | 4-(4-Morpho)-C₆H₄ | H | iPr | Me |
| 1-711 | 4-(4-Morpho)-C₆H₄ | F | iPr | Me |
| 1-712 | 4-CH₂SMe-C₆H₄ | H | iPr | Me |
| 1-713 | 4-CH₂SMe-C₆H₄ | F | iPr | Me |
| 1-714 | 4-CH₂SOMe-C₆H₄ | H | iPr | Me |
| 1-715 | 4-CH₂SOMe-C₆H₄ | F | iPr | Me |
| 1-716 | 4-CH₂SO₂Me-C₆H₄ | H | iPr | Me |
| 1-717 | 4-CH₂SO₂Me-C₆H₄ | F | iPr | Me |
| 1-718 | 4-SO₂F-C₆H₄ | H | iPr | Me |
| 1-719 | 4-SO₂F-C₆H₄ | F | iPr | Me |
| 1-720 | 4-(OCH₂CH₂NMe₂)-C₆H₄ | H | iPr | Me |
| 1-721 | 4-(OCH₂CH₂NMe₂)-C₆H₄ | F | iPr | Me |
| 1-722 | 4-Pyrazo | H | iPr | Me |
| 1-723 | 4-Pyrazo | F | iPr | Me |
| 1-724 | 1-Me-4-Pyrazo | H | iPr | Me |
| 1-725 | 1-Me-4-Pyrazo | F | iPr | Me |
| 1-726 | 5-Pyrazo | H | iPr | Me |
| 1-727 | 5-Pyrazo | F | iPr | Me |
| 1-728 | 1-Me-5-Pyrazo | H | iPr | Me |
| 1-729 | 1-Me-5-Pyrazo | F | iPr | Me |
| 1-730 | 1-Pyrazo | H | iPr | Me |
| 1-731 | 1-Pyrazo | F | iPr | Me |
| 1-732 | 1-Me-3-Pyrazo | H | iPr | Me |
| 1-733 | 1-Me-3-Pyrazo | F | iPr | Me |
| 1-734 | 1-Me-4-Pyrazo | H | H | Me |
| 1-735 | 1-Me-4-Pyrazo | F | H | Me |
| 1-736 | 1-Et-4-Pyrazo | H | iPr | Me |
| 1-737 | 1-Et-4-Pyrazo | F | iPr | Me |
| 1-738 | 1-Et-4-Pyrazo | H | H | Me |
| 1-739 | 1-Et-4-Pyrazo | F | H | Me |
| 1-740 | 1-iPr-4-Pyrazo | H | iPr | Me |
| 1-741 | 1-iPr-4-Pyrazo | F | iPr | Me |
| 1-742 | 4-SO₂Me-C₆H₄ | F | H | Me |
| 1-743 | 2-Furyl | H | iPr | Me |
| 1-744 | 2-Furyl | F | iPr | Me |
| 1-745 | 2-Oxazo | H | iPr | Me |
| 1-746 | 2-Oxazo | F | iPr | Me |
| 1-747 | 5-Oxazo | H | iPr | Me |
| 1-748 | 5-Oxazo | F | iPr | Me |
| 1-749 | 2-Thiazo | H | iPr | Me |
| 1-750 | 2-Thiazo | F | iPr | Me |
| 1-751 | 4-cO₂H-C₆H₄ | F | iPr | Me |
| 1-752 | 2-SOMe-5-Py | H | iPr | Me |
| 1-753 | 2-SOMe-5-Py | F | iPr | Me |
| 1-754 | 2-SO₂Me-5-Py | H | iPr | Me |
| 1-755 | 2-SO₂Me-5-Py | F | iPr | Me |
| 1-756 | 5-Me-2-Pyrro | H | iPr | Me |
| 1-757 | 5-Me-2-Pyrro | F | iPr | Me□ |
| 1-758 | 5-[1,2,3]-Triazo | H | iPr | Me |
| 1-759 | 5-[1,2,3]-Triazo | F | iPr | Me |
| 1-760 | 5-[1,2,4]-Triazo | H | iPr | Me |
| 1-761 | 5-[1,2,4]-Triazo | F | iPr | Me |
| 1-762 | 1-[1,2,4]-Triazo | H | iPr | Me |
| 1-763 | 1-[1,2,4]-Triazo | F | iPr | Me |
| 1-764 | 1-imidazo | H | iPr | Me |
| 1-765 | 1-imidazo | F | iPr | Me |
| 1-766 | 2-imidazo | H | iPr | Me |
| 1-767 | 2-imidazo | F | iPr | Me |
| 1-768 | 5-imidazo | H | iPr | Me |
| 1-769 | 5-imidazo | F | iPr | Me |
| 1-770 | 2-Me-5-imidazo | H | iPr | Me |
| 1-771 | 2-Me-5-imidazo | F | iPr | Me |
| 1-772 | 2-Et-5-imidazo | H | iPr | Me |
| 1-773 | 2-Et-5-imidazo | F | iPr | Me |
| 1-774 | 1-Me-4-imidazo | H | iPr | Me |
| 1-775 | 1-Me-4-imidazo | F | iPr | Me |
| 1-776 | Het(A) | H | iPr | Me |
| 1-777 | Het(A) | F | iPr | Me |
| 1-778 | Het(B) | H | iPr | Me |
| 1-779 | Het(B) | F | iPr | Me |
| 1-780 | Het(C) | H | iPr | Me |
| 1-781 | Het(C) | F | iPr | Me |
| 1-782 | Het(D) | H | iPr | Me |
| 1-783 | Het(D) | F | iPr | Me |
| 1-784 | Het(E) | H | iPr | Me |
| 1-785 | Het(E) | F | iPr | Me |
| 1-786 | Het(F) | H | iPr | Me |
| 1-787 | Het(F) | F | iPr | Me |
| 1-788 | Het(G) | H | iPr | Me |
| 1-789 | Het(G) | F | iPr | Me |
| 1-790 | Het(H) | H | iPr | Me |
| 1-791 | Het(H) | F | iPr | Me |
| 1-792 | Het(I) | H | iPr | Me |
| 1-793 | Het(I) | F | iPr | Me |
| 1-794 | 5-(4-Morpho)-2-Py | F | H | Me□ |
| 1-795 | 5-(4-Morpho)-2-Py | F | Me | Me |
| 1-796 | 5-(4-Me-1-Pipera)-2-Py | F | H | Me |
| 1-797 | 5-(4-Me-1-Pipera)-2-Py | F | Me | Me |
| 1-798 | 2-(4-Me-1-Pipera)-5-Py | F | H | Me |
| 1-799 | 2-(4-Me-1-Pipera)-5-Py | F | Me | Me |
| 1-800 | 2-(4-Morpho)-5-Py | F | H | Me |
| 1-801 | 2-(4-Morpho)-5-Py | F | Me | Me |
| 1-802 | 5-(4-Morpho)-2-Py | H | H | Me |
| 1-803 | 5-(4-Morpho)-2-Py | F | H | H |
| 1-804 | 5-(4-Morpho)-2-Py | H | H | H |
| 1-805 | 5-(4-Me-1-Pipera)-2-Py | H | H | Me |
| 1-806 | 5-(4-Me-1-Pipera)-2-Py | F | H | H |
| 1-807 | 5-(4-Me-1-Pipera)-2-Py | H | H | H |
| 1-808 | 2-(4-Me-1-Pipera)-5-Py | H | H | Me |
| 1-809 | 2-(4-Me-1-Pipera)-5-Py | F | H | H |
| 1-810 | 2-(4-Me-1-Pipera)-5-Py | H | H | H |
| 1-811 1 | 2-(4-Morpho)-5-Py | H | H | Me |
| 1-812 | 2-(4-Morpho)-5-Py | F | H | H |
| 1-813 | 2-(4-Morpho)-5-Py | H | H | H |

**(Table 2)**

| | | | | |
|---|---|---|---|---|
| 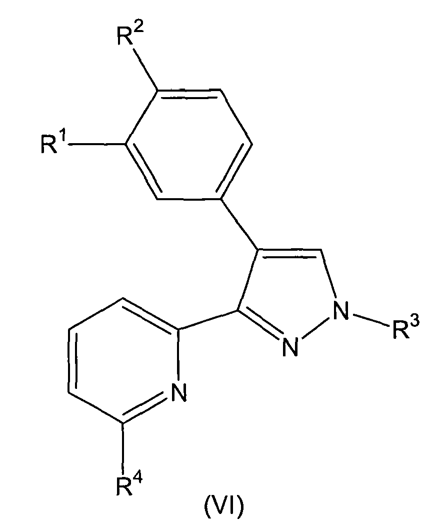 | | | | |

| Compound No. | R¹ | R² | R³ | R⁴ |
|---|---|---|---|---|
| 2-1 | 4-OMe-C₆H₄ | H | iPr | Me |
| 2-2 | 4-OMe-C₆H₄ | H | iPr | H |
| 2-3 | 4-OMe-C₆H₄ | H | cPr | Me |
| 2-4 | 4-OMe-C₆H₄ | H | cPr | H |
| 2-5 | 4-OMe-C₆H₄ | H | iBu | Me |
| 2-6 | 4-OMe-C₆H₄ | H | iBu | H |
| 2-7 | 4-OMe-C₆H₄ | Me | iPr | Me |
| 2-8 | 4-OMe-C₆H₄ | Me | iPr | H |
| 2-9 | 4-OMe-C₆H₄ | Me | cPr | Me |
| 2-10 | 4-OMe-C₆H₄ | Me | cPr | H |
| 2-11 | 4-OMe-C₆H₄ | Me | iBu | Me |
| 2-12 | 4-OMe-C₆H₄ | Me | iBu | H |
| 2-13 | 4-OMe-C₆H₄ | F | iPr | Me |
| 2-14 | 4-OMe-C₆H₄ | F | iPr | H |
| 2-15 | 4-OMe-C₆H₄ | F | cPr | Me |
| 2-16 | 4-OMe-C₆H₄ | F | cPr | H |
| 2-17 | 4-OMe-C₆H₄ | F | iBu | Me |
| 2-18 | 4-OMe-C₆H₄ | F | iBu | H |
| 2-19 | 4-OMe-C₆H₄ | Cl | iPr | Me |
| 2-20 | 4-OMe-C₆H₄ | Cl | iPr | H |
| 2-21 | 4-OMe-C₆H₄ | Cl | cPr | Me |
| 2-22 | 4-OMe-C₆H₄ | Cl | cPr | H |
| 2-23 | 4-OMe-C₆H₄ | Cl | iBu | Me |
| 2-24 | 4-OMe-C₆H₄ | Cl | iBu | H |
| 2-25 | 4-NH₂-C₆H₄ | H | iPr | Me |
| 2-26 | 4-NH₂-C₆H₄ | H | iPr | H |
| 2-27 | 4-NH₂-C₆H₄ | H | cPr | Me |
| 2-28 | 4-NH₂-C₆H₄ | H | cPr | H |
| 2-29 | 4-NH₂-C₆H₄ | H | iBu | Me |
| 2-30 | 4-NH₂-C₆H₄ | H | iBu | H |
| 2-31 | 4-NH₂-C₆H₄ | Me | iPr | Me |
| 2-32 | 4-NH₂-C₆H₄ | Me | iPr | H |
| 2-33 | 4-NH₂-C₆H₄ | Me | cPr | Me |
| 2-34 | 4-NH₂-C₆H₄ | Me | cPr | H |
| 2-35 | 4-NH₂-C₆H₄ | Me | iBu | Me |
| 2-36 | 4-NH₂-C₆H₄ | Me | iBu | H |
| 2-37 | 4-NH₂-C₆H₄ | F | iPr | Me |
| 2-38 | 4-NH₂-C₆H₄ | F | iPr | H |
| 2-39 | 4-NH₂-C₆H₄ | F | cPr | Me |
| 2-40 | 4-NH₂-C₆H₄ | F | cPr | H |
| 2-41 | 4-NH₂-C₆H₄ | F | iBu | Me |
| 2-42 | 4-NH₂-C₆H₄ | F | iBu | H |
| 2-43 | 4-NH₂-C₆H₄ | Cl | iPr | Me |
| 2-44 | 4-NH₂-C₆H₄ | Cl | iPr | H |
| 2-45 | 4-NH₂-C₆H₄ | Cl | cPr | Me |
| 2-46 | 4-NH₂-C₆H₄ | Cl | cPr | H |
| 2-47 | 4-NH₂-C₆H₄ | Cl | iBu | Me |
| 2-48 | 4-NH₂-C₆H₄ | Cl | iBu | H |
| 2-49 | 4-SMe-C₆H₄ | H | iPr | Me |
| 2-50 | 4-SMe-C₆H₄ | H | iPr | H |
| 2-51 | 4-SMe-C₆H₄ | H | cPr | Me |
| 2-52 | 4-SMe-C₆H₄ | H | cPr | H |
| 2-53 | 4-SMe-C₆H₄ | H | iBu | Me |
| 2-54 | 4-SMe-C₆H₄ | H | iBu | H |
| 2-55 | 4-SMe-C₆H₄ | Me | iPr | Me |
| 2-56 | 4-SMe-C₆H₄ | Me | iPr | H |
| 2-57 | 4-SMe-C₆H₄ | Me | cPr | Me |
| 2-58 | 4-SMe-C₆H₄ | Me | cPr | H |
| 2-59 | 4-SMe-C₆H₄ | Me | iBu | Me |
| 2-60 | 4-SMe-C₆H₄ | Me | iBu | H |
| 2-61 | 4-SMe-C₆H₄ | F | iPr | Me |
| 2-62 | 4-SMe-C₆H₄ | F | iPr | H |
| 2-63 | 4-SMe-C₆H₄ | F | cPr | Me |
| 2-64 | 4-SMe-C₆H₄ | F | cPr | H |
| 2-65 | 4-SMe-C₆H₄ | F | iBu | Me |
| 2-66 | 4-SMe-C₆H₄ | F | iBu | H |
| 2-67 | 4-SMe-C₆H₄ | Cl | iPr | Me |
| 2-68 | 4-SMe-C₆H₄ | Cl | iPr | H |
| 2-69 | 4-SMe-C₆H₄ | Cl | cPr | Me |
| 2-70 | 4-SMe-C₆H₄ | Cl | cPr | H |
| 2-71 | 4-SMe-C₆H₄ | Cl | iBu | Me |
| 2-72 | 4-SMe-C₆H₄ | Cl | iBu | H |
| 2-73 | 4-SOMe-C₆H₄ | H | iPr | Me |
| 2-74 | 4-SOMe-C₆H₄ | H | iPr | H |
| 2-75 | 4-SOMe-C₆H₄ | H | cPr | Me |
| 2-76 | 4-SOMe-C₆H₄ | H | cPr | H |
| 2-77 | 4-SOMe-C₆H₄ | H | iBu | Me |
| 2-78 | 4-SOMe-C₆H₄ | H | iBu | H |
| 2-79 | 4-SOMe-C₆H₄ | Me | iPr | Me |
| 2-80 | 4-SOMe-C₆H₄ | Me | iPr | H |
| 2-81 | 4-SOMe-C₆H₄ | Me | cPr | Me |
| 2-82 | 4-SOMe-C₆H₄ | Me | cPr | H |
| 2-83 | 4-SOMe-C₆H₄ | Me | iBu | Me |
| 2-84 | 4-SOMe-C₆H₄ | Me | iBu | H |
| 2-85 | 4-SOMe-C₆H₄ | F | iPr | Me |
| 2-86 | 4-SOMe-C₆H₄ | F | iPr | H |
| 2-87 | 4-SOMe-C₆H₄ | F | cPr | Me |
| 2-88 | 4-SOMe-C₆H₄ | F | cPr | H |
| 2-89 | 4-SOMe-C₆H₄ | F | iBu | Me |
| 2-90 | 4-SOMe-C₆H₄ | F | iBu | H |
| 2-91 | 4-SOMe-C₆H₄ | Cl | iPr | Me |
| 2-92 | 4-SOMe-C₆H₄ | Cl | iPr | H |
| 2-93 | 4-SOMe-C₆H₄ | Cl | cPr | Me |
| 2-94 | 4-SOMe-C₆H₄ | Cl | cPr | H |
| 2-95 | 4-SOMe-C₆H₄ | Cl | iBu | Me |
| 2-96 | 4-SOMe-C₆H₄ | Cl | iBu | H |
| 2-97 | 4-SO₂Me-C₆H₄ | H | iPr | Me |
| 2-98 | 4-SO₂Me-C₆H₄ | H | iPr | H |
| 2-99 | 4-SO₂Me-C₆H₄ | H | cPr | Me |
| 2-100 | 4-SO₂Me-C₆H₄ | H | cPr | H |
| 2-101 | 4-SO₂Me-C₆H₄ | H | iBu | Me |
| 2-102 | 4-SO₂Me-C₆H₄ | H | iBu | H |
| 2-103 | 4-SO₂Me-C₆H₄ | Me | iPr | Me |
| 2-104 | 4-SO₂Me-C₆H₄ | Me | iPr | H |
| 2-105 | 4-SO₂Me-C₆H₄ | Me | cPr | Me |
| 2-106 | 4-SO₂Me-C₆H₄ | Me | cPr | H |
| 2-107 | 4-SO₂Me-C₆H₄ | Me | iBu | Me |
| 2-108 | 4-SO₂Me-C₆H₄ | Me | iBu | H |
| 2-109 | 4-SO₂Me-C₆H₄ | F | iPr | Me |
| 2-110 | 4-SO₂Me-C₆H₄ | F | iPr | H |
| 2-111 | 4-SO₂Me-C₆H₄ | F | cPr | Me |
| 2-112 | 4-SO₂Me-C₆H₄ | F | cPr | H |
| 2-113 | 4-SO₂Me-C₆H₄ | F | iBu | Me |
| 2-114 | 4-SO₂Me-C₆H₄ | F | iBu | H |
| 2-115 | 4-SO₂Me-C₆H₄ | Cl | iPr | Me |
| 2-116 | 4-SO₂Me-C₆H₄ | Cl | iPr | H |
| 2-117 | 4-SO₂Me-C₆H₄ | Cl | cPr | Me |
| 2-118 | 4-SO₂Me-C₆H₄ | Cl | cPr | H |
| 2-119 | 4-SO₂Me-C₆H₄ | Cl | iBu | Me |
| 2-120 | 4-SO₂Me-C₆H₄ | Cl | iBu | H |
| 2-121 | 4-NHSO₂Me-C₆H₄ | H | iPr | Me |
| 2-122 | 4-NHSO₂Me-C₆H₄ | H | iPr | H |
| 2-123 | 4-NHSO₂Me-C₆H₄ | H | cPr | Me |
| 2-124 | 4-NHSO₂Me-C₆H₄ | H | cPr | H |
| 2-125 | 4-NHSO₂Me-C₆H₄ | H | iBu | Me |
| 2-126 | 4-NHSO₂Me-C₆H₄ | H | iBu | H |
| 2-127 | 4-NHSO₂Me-C₆H₄ | Me | iPr | Me |
| 2-128 | 4-NHSO₂Me-C₆H₄ | Me | iPr | H |
| 2-129 | 4-NHSO₂Me-C₆H₄ | Me | cPr | Me |
| 2-130 | 4-NHSO₂Me-C₆H₄ | Me | cPr | H |
| 2-131 | 4-NHSO₂Me-C₆H₄ | Me | iBu | Me |
| 2-132 | 4-NHSO₂Me-C₆H₄ | Me | iBu | H |
| 2-133 | 4-NHSO₂Me-C₆H₄ | F | iPr | Me |
| 2-134 | 4-NHSO₂Me-C₆H₄ | F | iPr | H |
| 2-135 | 4-NHSO₂Me-C₆H₄ | F | cPr | Me |
| 2-136 | 4-NHSO₂Me-C₆H₄ | F | cPr | H |
| 2-137 | 4-NHSO₂Me-C₆H₄ | F | iBu | Me |
| 2-138 | 4-NHSO₂Me-C₆H₄ | F | iBu | H |
| 2-139 | 4-NHSO₂Me-C₆H₄ | Cl | iPr | Me |
| 2-140 | 4-NHSO₂Me-C₆H₄ | Cl | iPr | H |
| 2-141 | 4-NHSO₂Me-C₆H₄ | Cl | cPr | Me |
| 2-142 | 4-NHSO₂Me-C₆H₄ | Cl | cPr | H |
| 2-143 | 4-NHSO₂Me-C₆H₄ | Cl | iBu | Me |
| 2-144 | 4-NHSO₂Me-C₆H₄ | Cl | iBu | H |
| 2-145 | 4-NHSO₂Et-C₆H₄ | H | iPr | Me |
| 2-146 | 4-NHSO₂Et-C₆H₄ | H | iPr | H |
| 2-147 | 4-NHSO₂Et-C₆H₄ | H | cPr | Me |
| 2-148 | 4-NHSO₂Et-C₆H₄ | H | cPr | H |
| 2-149 | 4-NHSO₂Et-C₆H₄ | H | iBu | Me |
| 2-150 | 4-NHSO₂Et-C₆H₄ | H | iBu | H |
| 2-151 | 4-NHSO₂Et-C₆H₄ | Me | iPr | Me |
| 2-152 | 4-NHSO₂Et-C₆H₄ | Me | iPr | H |
| 2-153 | 4-NHSO₂Et-C₆H₄ | Me | cPr | Me |
| 2-154 | 4-NHSO₂Et-C₆H₄ | Me | cPr | H |
| 2-155 | 4-NHSO₂Et-C₆H₄ | Me | iBu | Me |
| 2-156 | 4-NHSO₂Et-C₆H₄ | Me | iBu | H |
| 2-157 | 4-NHSO₂Et-C₆H₄ | F | iPr | Me |
| 2-158 | 4-NHSO₂Et-C₆H₄ | F | iPr | H |
| 2-159 | 4-NHSO₂Et-C₆H₄ | F | cPr | Me |
| 2-160 | 4-NHSO₂Et-C₆H₄ | F | cPr | H |
| 2-161 | 4-NHSO₂Et-C₆H₄ | F | iBu | Me |
| 2-162 | 4-NHSO₂Et-C₆H₄ | F | iBu | H |
| 2-163 | 4-NHSO₂Et-C₆H₄ | Cl | iPr | Me |
| 2-164 | 4-NHSO₂Et-C₆H₄ | Cl | iPr | H |
| 2-165 | 4-NHSO₂Et-C₆H₄ | Cl | cPr | Me |
| 2-166 | 4-NHSO₂Et-C₆H₄ | Cl | cPr | H |
| 2-167 | 4-NHSO₂Et-C₆H₄ | Cl | iBu | Me |
| 2-168 | 4-NHSO₂Et-C₆H₄ | Cl | iBu | H |
| 2-169 | 4-NHSO₂cPr-C₆H₄ | H | iPr | Me |
| 2-170 | 4-NHSO₂cPr-C₆H₄ | H | iPr | H |
| 2-171 | 4-NHSO₂cPr-C₆H₄ | H | cPr | Me |
| 2-172 | 4-NHSO₂cPr-C₆H₄ | H | cPr | H |
| 2-173 | 4-NHSO₂cPr-C₆H₄ | H | iBu | Me |
| 2-174 | 4-NHSO₂cPr-C₆H₄ | H | iBu | H |
| 2-175 | 4-NHSO₂cPr-C₆H₄ | Me | iPr | Me |
| 2-176 | 4-NHSO₂cPr-C₆H₄ | Me | iPr | H |
| 2-177 | 4-NHSO₂cPr-C₆H₄ | Me | cPr | Me |
| 2-178 | 4-NHSO₂cPr-C₆H₄ | Me | cPr | H |
| 2-179 | 4-NHSO₂cPr-C₆H₄ | Me | iBu | Me |
| 2-180 | 4-NHSO₂cPr-C₆H₄ | Me | iBu | H |
| 2-181 | 4-NHSO₂cPr-C₆H₄ | F | iPr | Me |
| 2-182 | 4-NHSO₂cPr-C₆H₄ | F | iPr | H |
| 2-183 | 4-NHSO₂cPr-C₆H₄ | F | cPr | Me |
| 2-184 | 4-NHSO₂cPr-C₆H₄ | F | cPr | H |
| 2-185 | 4-NHSO₂cPr-C₆H₄ | F | iBu | Me |
| 2-186 | 4-NHSO₂cPr-C₆H₄ | F | iBu | H |
| 2-187 | 4-NHSO₂cPr-C₆H₄ | Cl | iPr | Me |
| 2-188 | 4-NHSO₂cPr-C₆H₄ | Cl | iPr | H |
| 2-189 | 4-NHSO₂cPr-C₆H₄ | Cl | cPr | Me□ |
| 2-190 | 4-NHSO₂cPr-C₆H₄ | Cl | cPr | H |
| 2-191 | 4-NHSO₂cPr-C₆H₄ | Cl | iBu | Me |
| 2-192 | 4-NHSO₂cPr-C₆H₄ | Cl | iBu | H |
| 2-193 | 4-SO₂NH₂-C₆H₄ | H | iPr | Me |
| 2-194 | 4-SO₂NH₂-C₆H₄ | H | iPr | H |
| 2-195 | 4-SO₂NH₂-C₆H₄ | H | cPr | Me |
| 2-196 | 4-SO₂NH₂-C₆H₄ | H | cPr | H |
| 2-197 | 4-SO₂NH₂-C₆H₄ | H | iBu | Me |
| 2-198 | 4-SO₂NH₂-C₆H₄ | H | iBu | H |
| 2-199 | 4-SO₂NH₂-C₆H₄ | Me | iPr | Me |
| 2-200 | 4-SO₂NH₂-C₆H₄ | Me | iPr | H |
| 2-201 | 4-SO₂NH₂-C₆H₄ | Me | cPr | Me |
| 2-202 | 4-SO₂NH₂-C₆H₄ | Me | cPr | H |
| 2-203 | 4-SO₂NH₂-C₆H₄ | Me | iBu | Me |
| 2-204 | 4-SO₂NH₂-C₆H₄ | Me | iBu | H |
| 2-205 | 4-SO₂NH₂-C₆H₄ | F | iPr | Me |
| 2-206 | 4-SO₂NH₂-C₆H₄ | F | iPr | H |
| 2-207 | 4-SO₂NH₂-C₆H₄ | F | cPr | Me |
| 2-208 | 4-SO₂NH₂-C₆H₄ | F | cPr | H |
| 2-209 | 4-SO₂NH₂-C₆H₄ | F | iBu | Me |
| 2-210 | 4-SO₂NH₂-C₆H₄ | F | iBu | H |
| 2-211 | 4-SO₂NH₂-C₆H₄ | Cl | iPr | Me |
| 2-212 | 4-SO₂NH₂-C₆H₄ | Cl | iPr | H |
| 2-213 | 4-SO₂NH₂-C₆H₄ | Cl | cPr | Me |
| 2-214 | 4-SO₂NH₂-C₆H₄ | Cl | cPr | H |
| 2-215 | 4-SO₂NH₂-C₆H₄ | Cl | iBu | Me |
| 2-216 | 4-SO₂NH₂-C₆H₄ | Cl | iBu | H |
| 2-217 | 4-[CONH-(4-Morpho)]-C₆H₄ | H | iPr | Me |
| 2-218 | 4-[CONH-(4-Morpho)]-C₆H₄ | H | iPr | H |
| 2-219 | 4-[CONH-(4-Morpho)]-C₆H₄ | H | cPr | Me |
| 2-220 | 4-[CONH-(4-Morpho)]-C₆H₄ | H | cPr | H |
| 2-221 | 4-[CONH-(4-Morpho)]-C₆H₄ | H | iBu | Me |
| 2-222 | 4-[CONH-(4-Morpho)]-C₆H₄ | H | iBu | H |
| 2-223 | 4-[CONH-(4-Morpho)]-C₆H₄ | Me | iPr | Me |
| 2-224 | 4-[CONH-(4-Morpho)]-C₆H₄ | Me | iPr | H |
| 2-225 | 4-[CONH-(4-Morpho)]-C₆H₄ | Me | cPr | Me |
| 2-226 | 4-[CONH-(4-Morpho)]-C₆H₄ | Me | cPr | H |
| 2-227 | 4-[CONH-(4-Morpho)]-C₆H₄ | Me | iBu | Me |
| 2-228 | 4-[CONH-(4-Morpho)]-C₆H₄ | Me | iBu | H |
| 2-229 | 4-[CONH-(4-Morpho)]-C₆H₄ | F | iPr | Me |
| 2-230 | 4-[CONH-(4-Morpho)]-C₆H₄ | F | iPr | H |
| 2-231 | 4-[CONH-(4-Morpho)]-C₆H₄ | F | cPr | Me |
| 2-232 | 4-[CONH-(4-Morpho)]-C₆H₄ | F | cPr | H |
| 2-233 | 4-[CONH-(4-Morpho)]-C₆H₄ | F | iBu | Me |
| 2-234 | 4-[CONH-(4-Morpho)]-C₆H₄ | F | iBu | H |
| 2-235 | 4-[CONH-(4-Morpho)]-C₆H₄ | Cl | iPr | Me |
| 2-236 | 4-[CONH-(4-Morpho)]-C₆H₄ | Cl | iPr | H |
| 2-237 | 4-[CONH-(4-Morpho)]-C₆H₄ | Cl | cPr | Me |
| 2-238 | 4-[CONH-(4-Morpho)]-C₆H₄ | Cl | cPr | H |
| 2-239 | 4-[CONH-(4-Morpho)]-C₆H₄ | Cl | iBu | Me |
| 2-240 | 4-[CONH-(4-Morpho)]-C₆H₄ | Cl | iBu | H |
| 2-241 | 4-[CONH-(4-Me-1-Pipera)]-C₆H₄ | H | iPr | Me |
| 2-242 | 4-[CONH-(4-Me-1-Pipera)]-C₆H₄ | H | iPr | H |
| 2-243 | 4-[CONH-(4-Me-1-Pipera)]-C₆H₄ | H | cPr | Me |
| 2-244 | 4-[CONH-(4-Me-1-Pipera)]-C₆H₄ | H | cPr | H |
| 2-245 | 4-[CONH-(4-Me-1-Pipera)]-C₆H₄ | H | iBu | Me |
| 2-246 | 4-[CONH-(4-Me-1-Pipera)]-C₆H₄ | H | iBu | H |
| 2-247 | 4-[CONH-(4-Me-1-Pipera)]-C₆H₄ | Me | iPr | Me |
| 2-248 | 4-[CONH-(4-Me-1-Pipera)]-C₆H₄ | Me | iPr | H |
| 2-249 | 4-[CONH-(4-Me-1-Pipera)]-C₆H₄ | Me | cPr | Me |
| 2-250 | 4-[CONH-(4-Me-1-Pipera)]-C₆H₄ | Me | cPr | H |
| 2-251 | 4-[CONH-(4-Me-1-Pipera)]-C₆H₄ | Me | iBu | Me |
| 2-252 | 4-[CONH-(4-Me-1-Pipera)]-C₆H₄ | Me | iBu | H |
| 2-253 | 4-[CONH-(4-Me-1-Pipera)]-C₆H₄ | F | iPr | Me |
| 2-254 | 4-[CONH-(4-Me-1-Pipera)]-C₆H₄ | F | iPr | H |
| 2-255 | 4-[CONH-(4-Me-1-Pipera)]-C₆H₄ | F | cPr | Me |
| 2-256 | 4-[CONH-(4-Me-1-Pipera)]-C₆H₄ | F | cPr | H |
| 2-257 | 4-[CONH-(4-Me-1-Pipera)]-C₆H₄ | F | iBu | Me |
| 2-258 | 4-[CONH-(4-Me-1-Pipera)]-C₆H₄ | F | iBu | H□ |
| 2-259 | 4-[CONH-(4-Me-1-Pipera)]-C₆H₄ | Cl | iPr | Me |
| 2-260 | 4-[CONH-(4-Me-1-Pipera)]-C₆H₄ | Cl | iPr | H |
| 2-261 | 4-[CONH-(4-Me-1-Pipera)]-C₆H₄ | Cl | cPr | Me |
| 2-262 | 4-[CONH-(4-Me-1-Pipera)]-C₆H₄ | Cl | cPr | H |
| 2-263 | 4-[CONH-(4-Me-1-Pipera)]-C₆H₄ | Cl | iBu | Me |
| 2-264 | 4-[CONH-(4-Me-1-Pipera)]-C₆H₄ | Cl | iBu | H |
| 2-265 | 3-Thi | H | iPr | Me |
| 2-266 | 3-Thi | H | iPr | H |
| 2-267 | 3-Thi | H | cPr | Me |
| 2-268 | 3-Thi | H | cPr | H |
| 2-269 | 3-Thi | H | iBu | Me |
| 2-270 | 3-Thi | H | iBu | H |
| 2-271 | 3-Thi | Me | iPr | Me |
| 2-272 | 3-Thi | Me | iPr | H |
| 2-273 | 3-Thi | Me | cPr | Me |
| 2-274 | 3-Thi | Me | cPr | H |
| 2-275 | 3-Thi | Me | iBu | Me |
| 2-276 | 3-Thi | Me | iBu | H |
| 2-277 | 3-Thi | F | iPr | Me |
| 2-278 | 3-Thi | F | iPr | H |
| 2-279 | 3-Thi | F | cPr | Me |
| 2-280 | 3-Thi | F | cPr | H |
| 2-281 | 3-Thi | F | iBu | Me |
| 2-282 | 3-Thi | F | iBu | H |
| 2-283 | 3-Thi | Cl | iPr | Me |
| 2-284 | 3-Thi | Cl | iPr | H |
| 2-285 | 3-Thi | Cl | cPr | Me |
| 2-286 | 3-Thi | Cl | cPr | H |
| 2-287 | 3-Thi | Cl | iBu | Me |
| 2-288 | 3-Thi | Cl | iBu | H |
| 2-289 | 2-Pyrro | H | iPr | Me |
| 2-290 | 2-Pyrro | H | iPr | H |
| 2-291 | 2-Pyrro | H | cPr | Me |
| 2-292 | 2-Pyrro | H | cPr | H |
| 2-293 | 2-Pyrro | H | iBu | Me |
| 2-294 | 2-Pyrro | H | iBu | H |
| 2-295 | 2-Pyrro | Me | iPr | Me |
| 2-296 | 2-Pyrro | Me | iPr | H |
| 2-297 | 2-Pyrro | Me | cPr | Me |
| 2-298 | 2-Pyrro | Me | cPr | H |
| 2-299 | 2-Pyrro | Me | iBu | Me |
| 2-300 | 2-Pyrro | Me | iBu | H |
| 2-301 | 2-Pyrro | F | iPr | Me |
| 2-302 | 2-Pyrro | F | iPr | H |
| 2-303 | 2-Pyrro | F | cPr | Me |
| 2-304 | 2-Pyrro | F | cPr | H |
| 2-305 | 2-Pyrro | F | iBu | Me |
| 2-306 | 2-Pyrro | F | iBu | H |
| 2-307 | 2-Pyrro | Cl | iPr | Me |
| 2-308 | 2-Pyrro | Cl | iPr | H |
| 2-309 | 2-Pyrro | Cl | cPr | Me |
| 2-310 | 2-Pyrro | Cl | cPr | H |
| 2-311 | 2-Pyrro | Cl | iBu | Me |
| 2-312 | 2-Pyrro | Cl | iBu | H |
| 2-313 | 5-CONH₂-2-Py | H | iPr | Me |
| 2-314 | 5-CONH₂-2-Py | H | iPr | H |
| 2-315 | 5-CONH₂-2-Py | H | cPr | Me |
| 2-316 | 5-CONH₂-2-Py | H | cPr | H |
| 2-317 | 5-CONH₂-2-Py | H | iBu | Me |
| 2-318 | 5-CONH₂-2-Py | H | iBu | H |
| 2-319 | 5-CONH₂-2-Py | Me | iPr | Me |
| 2-320 | 5-CONH₂-2-Py | Me | iPr | H |
| 2-321 | 5-CONH₂-2-Py | Me | cPr | Me |
| 2-322 | 5-CONH₂-2-Py | Me | cPr | H |
| 2-323 | 5-CONH₂-2-Py | Me | iBu | Me |
| 2-324 | 5-CONH₂-2-Py | Me | iBu | H |
| 2-325 | 5-CONH₂-2-Py | F | iPr | Me |
| 2-326 | 5-CONH₂-2-Py | F | iPr | H |
| 2-327 | 5-CONH₂-2-Py | F | cPr | Me |
| 2-328 | 5-CONH₂-2-Py | F | cPr | H |
| 2-329 | 5-CONH₂-2-Py | F | iBu | Me |
| 2-330 | 5-CONH₂-2-Py | F | iBu | H |
| 2-331 | 5-CONH₂-2-Py | Cl | iPr | Me |
| 2-332 | 5-CONH₂-2-Py | Cl | iPr | H |
| 2-333 | 5-CONH₂-2-Py | Cl | cPr | Me |
| 2-334 | 5-CONH₂-2-Py | Cl | cPr | H |
| 2-335 | 5-CONH₂-2-Py | Cl | iBu | Me |
| 2-336 | 5-CONH₂-2-Py | Cl | iBu | H |
| 2-337 | 4-SO₂Et-C₆H₄ | H | iPr | Me |
| 2-338 | 4-SO₂Et-C₆H₄ | H | cPr | Me |
| 2-339 | 4-SO₂Et-C₆H₄ | H | iBu | Me |
| 2-340 | 4-NHCOMe-C₆H₄ | H | iPr | Me |
| 2-341 | 4-NHCOMe-C₆H₄ | H | cPr | Me |
| 2-342 | 4-NHCOMe-C₆H₄ | H | iBu | Me |
| 2-343 | 4-NHCOCMe₂OH-C₆H₄ | H | iPr | Me |
| 2-344 | 4-NHCOCMe₂OH-C₆H₄ | H | cPr | Me |
| 2-345 | 4-NHCOCMe₂OH-C₆H₄ | H | iBu | Me |
| 2-346 | 4-NHCOCMe₂OCOMe-C₆H₄ | H | iPr | Me |
| 2-347 | 4-NHCOCMe₂OCOMe-C₆H₄ | H | cPr | Me |
| 2-348 | 4-NHCOCMe₂OCOMe-C₆H₄ | H | iBu | Me |
| 2-349 | 4-CONH₂-C₆H₄ | H | iPr | Me |
| 2-350 | 4-CONH₂-C₆H₄ | H | cPr | Me |
| 2-351 | 4-CONH₂-C₆H₄ | H | iBu | Me |
| 2-352 | 3-CONH₂-C₆H₄ | H | iPr | Me |
| 2-353 | 3-CONH₂-C₆H₄ | H | cPr | Me |
| 2-354 | 3-CONH₂-C₆H₄ | H | iBu | Me |
| 2-355 | 4-CONHCH₂CH₂OH-C₆H₄ | H | iPr | Me |
| 2-356 | 4-CONHCH₂CH₂OH-C₆H₄ | H | cPr | Me |
| 2-357 | 4-CONHCH₂CH₂OH-C₆H₄ | H | iBu | Me |
| 2-358 | 4-SO₂NHMe-C₆H₄ | H | iPr | Me |
| 2-359 | 4-SO₂NHMe-C₆H₄ | H | cPr | Me |
| 2-360 | 4-SO₂NHMe-C₆H₄ | H | iBu | Me |
| 2-361 | 4-[CO-(4-Morpho)]-C₆H₄ | H | iPr | Me |
| 2-362 | 4-[CO-(4-Morpho)]-C₆H₄ | H | cPr | Me |
| 2-363 | 4-[CO-(4-Morpho)]-C₆H₄ | H | iBu | Me |
| 2-364 | 4-[SO₂-(4-Morpho)]-C₆H₄ | H | iPr | Me |
| 2-365 | 4-[SO₂-(4-Morpho)]-C₆H₄ | H | cPr | Me |
| 2-366 | 4-[SO₂-(4-Morpho)]-C₆H₄ | H | iBu | Me |
| 2-367 | 3-Fur | H | iPr | Me |
| 2-368 | 3-Fur | H | cPr | Me |
| 2-369 | 3-Fur | H | iBu | Me |
| 2-370 | 2-OMe-5-Py | H | iPr | Me |
| 2-371 | 2-OMe-5-Py | H | cPr | Me |
| 2-372 | 2-OMe-5-Py | H | iBu | Me |
| 2-373 | 4-Py | H | iPr | Me |
| 2-374 | 4-Py | H | cPr | Me |
| 2-375 | 4-Py | H | iBu | Me |
| 2-376 | 4-OMe-C₆H₄ | H | H | Me |
| 2-377 | 4-OMe-C₆H₄ | H | Me | Me |
| 2-378 | 4-OMe-C₆H₄ | Me | H | Me |
| 2-379 | 4-OMe-C₆H₄ | Me | Me | Me |
| 2-380 | 4-OMe-C₆H₄ | F | H | Me |
| 2-381 | 4-OMe-C₆H₄ | F | Me | Me |
| 2-382 | 4-OMe-C₆H₄ | Cl | H | Me |
| 2-383 | 4-OMe-C₆H₄ | Cl | Me | Me |
| 2-384 | 4-NH₂-C₆H₄ | H | H | Me |
| 2-385 | 4-NH₂-C₆H₄ | H | Me | Me |
| 2-386 | 4-NH₂-C₆H₄ | Me | H | Me |
| 2-387 | 4-NH₂-C₆H₄ | Me | Me | Me |
| 2-388 | 4-NH₂-C₆H₄ | F | H | Me |
| 2-389 | 4-NH₂-C₆H₄ | F | Me | Me |
| 2-390 | 4-NH₂-C₆H₄ | Cl | H | Me |
| 2-391 | 4-NH₂-C₆H₄ | Cl | Me | Me |
| 2-392 | 4-SMe-C₆H₄ | H | H | Me |
| 2-393 | 4-SMe-C₆H₄ | H | Me | Me |
| 2-394 | 4-SMe-C₆H₄ | Me | H | Me |
| 2-395 | 4-SMe-C₆H₄ | Me | Me | Me |
| 2-396 | 4-SMe-C₆H₄ | F | H | Me |
| 2-397 | 4-SMe-C₆H₄ | F | Me | Me |
| 2-398 | 4-SMe-C₆H₄ | Cl | H | Me |
| 2-399 | 4-SMe-C₆H₄ | Cl | Me | Me |
| 2-400 | 4-SOMe-C₆H₄ | H | H | Me |
| 2-401 | 4-SOMe-C₆H₄ | H | Me | Me |
| 2-402 | 4-SOMe-C₆H₄ | Me | H | Me |
| 2-403 | 4-SOMe-C₆H₄ | Me | Me | Me |
| 2-404 | 4-SOMe-C₆H₄ | F | H | Me |
| 2-405 | 4-SOMe-C₆H₄ | F | Me | Me |
| 2-406 | 4-SOMe-C₆H₄ | Cl | H | Me |
| 2-407 | 4-SOMe-C₆H₄ | Cl | Me | Me |
| 2-408 | 4-SO₂Me-C₆H₄ | H | H | Me |
| 2-409 | 4-SO₂Me-C₆H₄ | H | Me | Me |
| 2-410 | 4-SO₂Me-C₆H₄ | Me | H | Me |
| 2-411 | 4-SO₂Me-C₆H₄ | Me | Me | Me |
| 2-412 | 4-SO₂Me-C₆H₄ | F | H | Me |
| 2-413 | 4-SO₂Me-C₆H₄ | F | Me | Me |
| 2-414 | 4-SO₂Me-C₆H₄ | Cl | H | Me |
| 2-415 | 4-SO₂Me-C₆H₄ | Cl | Me | Me |
| 2-416 | 4-NHSO₂Me-C₆H₄ | H | H | Me |
| 2-417 | 4-NHSO₂Me-C₆H₄ | H | Me | Me |
| 2-418 | 4-NHSO₂Me-C₆H₄ | Me | H | Me |
| 2-419 | 4-NHSO₂Me-C₆H₄ | Me | Me | Me |
| 2-420 | 4-NHSO₂Me-C₆H₄ | F | H | Me |
| 2-421 | 4-NHSO₂Me-C₆H₄ | F | Me | Me |
| 2-422 | 4-NHSO₂Me-C₆H₄ | Cl | H | Me |
| 2-423 | 4-NHSO₂Me-C₆H₄ | Cl | Me | Me |
| 2-424 | 4-NHSO₂Et-C₆H₄ | H | H | Me |
| 2-425 | 4-NHSO₂Et-C₆H₄ | H | Me | Me |
| 2-426 | 4-NHSO₂Et-C₆H₄ | Me | H | Me |
| 2-427 | 4-NHSO₂Et-C₆H₄ | Me | Me | Me |
| 2-428 | 4-NHSO₂Et-C₆H₄ | F | H | Me |
| 2-429 | 4-NHSO₂Et-C₆H₄ | F | Me | Me |
| 2-430 | 4-NHSO₂Et-C₆H₄ | Cl | H | Me |
| 2-431 | 4-NHSO₂Et-C₆H₄ | Cl | Me | Me |
| 2-432 | 4-NHSO₂cPr-C₆H₄ | H | H | Me |
| 2-433 | 4-NHSO₂cPr-C₆H₄ | H | Me | Me |
| 2-434 | 4-NHSO₂cPr-C₆H₄ | Me | H | Me |
| 2-435 | 4-NHSO₂cPr-C₆H₄ | Me | Me | Me |
| 2-436 | 4-NHSO₂cPr-C₆H₄ | F | H | Me |
| 2-437 | 4-NHSO₂cPr-C₆H₄ | F | Me | Me |
| 2-438 | 4-NHSO₂cPr-C₆H₄ | Cl | H | Me |
| 2-439 | 4-NHSO₂cPr-C₆H₄ | Cl | Me | Me |
| 2-440 | 4-SO₂NH₂-C₆H₄ | H | H | Me |
| 2-441 | 4-SO₂NH₂-C₆H₄ | H | Me | Me |
| 2-442 | 4-SO₂NH₂-C₆H₄ | Me | H | Me |
| 2-443 | 4-SO₂NH₂-C₆H₄ | Me | Me | Me |
| 2-444 | 4-SO₂NH₂-C₆H₄ | F | H | Me |
| 2-445 | 4-SO₂NH₂-C₆H₄ | F | Me | Me |
| 2-446 | 4-SO₂NH₂-C₆H₄ | Cl | H | Me |
| 2-447 | 4-SO₂NH₂-C₆H₄ | Cl | Me | Me |
| 2-448 | 4-[CONH-(4-Morpho)]-C₆H₄ | H | H | Me |
| 2-449 | 4-[CONH-(4-Morpho)]-C₆H₄ | H | Me | Me |
| 2-450 | 4-[CONH-(4-Morpho)]-C₆H₄ | Me | H | Me |
| 2-451 | 4-[CONH-(4-Morpho)]-C₆H₄ | Me | Me | Me |
| 2-452 | 4-[CONH-(4-Morpho)]-C₆H₄ | F | H | Me |
| 2-453 | 4-[CONH-(4-Morpho)]-C₆H₄ | F | Me | Me |
| 2-454 | 4-[CONH-(4-Morpho)]-C₆H₄ | Cl | H | Me |
| 2-455 | 4-[CONH-(4-Morpho)]-C₆H₄ | Cl | Me | Me |
| 2-456 | 4-[CONH-(4-Me-1-Pipera)]-C₆H₄ | H | H | Me |
| 2-457 | 4-[CONH-(4-Me-1-Pipera)]-C₆H₄ | H | Me | Me |
| 2-458 | 4-[CONH-(4-Me-1-Pipera)]-C₆H₄ | Me | H | Me |
| 2-459 | 4-[CONH-(4-Me-1-Pipera)]-C₆H₄ | Me | Me | Me |
| 2-460 | 4-[CONH-(4-Me-1-Pipera)]-C₆H₄ | F | H | Me |
| 2-461 | 4-[CONH-(4-Me-1-Pipera)]-C₆H₄ | F | Me | Me |
| 2-462 | 4-[CONH-(4-Me-1-Pipera)]-C₆H₄ | Cl | H | Me |
| 2-463 | 4-[CONH-(4-Me-1-Pipera)]-C₆H₄ | Cl | Me | Me |
| 2-464 | 3-Thi | H | H | Me |
| 2-465 | 3-Thi | H | Me | Me |
| 2-466 | 3-Thi | Me | H | Me |
| 2-467 | 3-Thi | Me | Me | Me |
| 2-468 | 3-Thi | F | H | Me |
| 2-469 | 3-Thi | F | Me | Me |
| 2-470 | 3-Thi | Cl | H | Me |
| 2-471 | 3-Thi | Cl | Me | Me |
| 2-472 | 2-Pyrro | H | H | Me |
| 2-473 | 2-Pyrro | H | Me | Me |
| 2-474 | 2-Pyrro | Me | H | Me |
| 2-475 | 2-Pyrro | Me | Me | Me |
| 2-476 | 2-Pyrro | F | H | Me |
| 2-477 | 2-Pyrro | F | Me | Me |
| 2-478 | 2-Pyrro | Cl | H | Me |
| 2-479 | 2-Pyrro | Cl | Me | Me |
| 2-480 | 5-CONH₂-2-Py | H | H | Me |
| 2-481 | 5-CONH₂-2-Py | H | Me | Me |
| 2-482 | 5-CONH₂-2-Py | Me | H | Me |
| 2-483 | 5-CONH₂-2-Py | Me | Me | Me |
| 2-484 | 5-CONH₂-2-Py | F | H | Me |
| 2-485 | 5-CONH₂-2-Py | F | Me | Me |
| 2-486 | 5-CONH₂-2-Py | Cl | H | Me |
| 2-487 | 5-CONH₂-2-Py | Cl | Me | Me |
| 2-488 | 4-SO₂Et-C₆H₄ | H | H | Me |
| 2-489 | 4-SO₂Et-C₆H₄ | H | Me | Me |
| 2-490 | 4-SO₂Et-C₆H₄ | F | H | Me |
| 2-491 | 4-SO₂Et-C₆H₄ | F | Me | Me |
| 2-492 | 4-NHCOMe-C₆H₄ | H | H | Me |
| 2-493 | 4-NHCOMe-C₆H₄ | H | Me | Me |
| 2-494 | 4-NHCOMe-C₆H₄ | F | H | Me |
| 2-495 | 4-NHCOMe-C₆H₄ | F | Me | Me |
| 2-496 | 4-NHCOCMe₂OH-C₆H₄ | H | H | Me |
| 2-497 | 4-NHCOCMe₂OH-C₆H₄ | H | Me | Me |
| 2-498 | 4-NHCOCMe₂OH-C₆H₄ | F | H | Me |
| 2-499 | 4-NHCOCMe₂OH-C₆H₄ | F | Me | Me |
| 2-500 | 4-NHCOCMe₂OCOMe-C₆H₄ | H | H | Me |
| 2-501 | 4-NHCOCMe₂OCOMe-C₆H₄ | H | Me | Me |
| 2-502 | 4-NHCOCMe₂OCOMe-C₆H₄ | F | H | Me |
| 2-503 | 4-NHCOCMe₂OCOMe-C₆H₄ | F | Me | Me |
| 2-504 | 4-CONH₂-C₆H₄ | H | H | Me |
| 2-505 | 4-CONH₂-C₆H₄ | H | Me | Me |
| 2-506 | 4-CONH₂-C₆H₄ | F | H | Me |
| 2-507 | 4-CONH₂-C₆H₄ | F | Me | Me |
| 2-508 | 3-CONH₂-C₆H₄ | H | H | Me |
| 2-509 | 3-CONH₂-C₆H₄ | H | Me | Me |
| 2-510 | 3-CONH₂-C₆H₄ | F | H | Me |
| 2-511 | 3-CONH₂-C₆H₄ | F | Me | Me |
| 2-512 | 4-CONHCH₂CH₂OH-C₆H₄ | H | H | Me |
| 2-513 | 4-CONHCH₂CH₂OH-C₆H₄ | H | Me | Me |
| 2-514 | 4-CONHCH₂CH₂OH-C₆H₄ | F | H | Me |
| 2-515 | 4-CONHCH₂CH₂OH-C₆H₄ | F | Me | Me |
| 2-516 | 4-SO₂NHMe-C₆H₄ | H | H | Me |
| 2-517 | 4-SO₂NHMe-C₆H₄ | H | Me | Me |
| 2-518 | 4-SO₂NHMe-C₆H₄ | F | H | Me |
| 2-519 | 4-SO₂NHMe-C₆H₄ | F | Me | Me |
| 2-520 | 4-[CO-(4-Morpho)]-C₆H₄ | H | H | Me |
| 2-521 | 4-[CO-(4-Morpho)]-C₆H₄ | H | Me | Me |
| 2-522 | 4-[CO-(4-Morpho)]-C₆H₄ | F | H | Me |
| 2-523 | 4-[CO-(4-Morpho)]-C₆H₄ | F | Me | Me |
| 2-524 | 4-[SO₂-(4-Morpho)]-C₆H₄ | H | H | Me |
| 2-525 | 4-[SO₂-(4-Morpho)]-C₆H₄ | H | Me | Me |
| 2-526 | 4-[SO₂-(4-Morpho)]-C₆H₄ | F | H | Me |
| 2-527 | 4-[SO₂-(4-Morpho)]-C₆H₄ | F | Me | Me |
| 2-528 | 3-Fur | H | H | Me |
| 2-529 | 3-Fur | H | Me | Me |
| 2-530 | 3-Fur | F | H | Me |
| 2-531 | 3-Fur | F | Me | Me |
| 2-532 | 2-OMe-5-Py | H | H | Me |
| 2-533 | 2-OMe-5-Py | H | Me | Me |
| 2-534 | 2-OMe-5-Py | F | H | Me |
| 2-535 | 2-OMe-5-Py | F | Me | Me |
| 2-536 | 4-Py | H | H | Me |
| 2-537 | 4-Py | H | Me | Me |
| 2-538 | 4-Py | F | H | Me |
| 2-539 | 4-Py | F | Me | Me |
| 2-540 | 4-[NHCONH-(4-Morpho)]-C₆H₄ | H | iPr | Me |
| 2-541 | 4-[NHCONH-(4-Morpho)]-C₆H₄ | H | iPr | H |
| 2-542 | 4-[NHCONH-(4-Morpho)]-C₆H₄ | H | Me | Me |
| 2-543 | 4-[NHCONH-(4-Morpho)]-C₆H₄ | H | Me | H |
| 2-544 | 4-[NHCONH-(4-Morpho)]-C₆H₄ | H | H | Me |
| 2-545 | 4-[NHCONH-(4-Morpho)]-C₆H₄ | H | H | H |
| 2-546 | 4-[NHCONH-(4-Morpho)]-C₆H₄ | F | iPr | Me |
| 2-547 | 4-[NHCONH-(4-Morpho)]-C₆H₄ | F | iPr | H |
| 2-548 | 4-[NHCONH-(4-Morpho)]-C₆H₄ | F | Me | Me |
| 2-549 | 4-[NHCONH-(4-Morpho)]-C₆H₄ | F | Me | H |
| 2-550 | 4-[NHCONH-(4-Morpho)]-C₆H₄ | F | H | Me |
| 2-551 | 4-[NHCONH-(4-Morpho)]-C₆H₄ | F | H | H |
| 2-552 | 4-[NHCONH-(4-Me-1-Pipera)]-C₆H₄ | H | iPr | Me |
| 2-553 | 4-[NHCONH-(4-Me-1-Pipera)]-C₆H₄ | H | iPr | H |
| 2-554 | 4-[NHCONH-(4-Me-1-Pipera)]-C₆H₄ | H | Me | Me |
| 2-555 | 4-[NHCONH-(4-Me-1-Pipera)]-C₆H₄ | H | Me | H |
| 2-556 | 4-[NHCONH-(4-Me-1-Pipera)]-C₆H₄ | H | H | Me |
| 2-567 | 4-[NHCONH-(4-Me-1-Pipera)]-C₆H₄ | H | H | H |
| 2-568 | 4-[NHCONH-(4-Me-1-Pipera)]-C₆H₄ | F | iPr | Me |
| 2-569 | 4-[NHCONH-(4-Me-1-Pipera)]-C₆H₄ | F | iPr | H |
| 2-570 | 4-[NHCONH-(4-Me-1-Pipera)]-C₆H₄ | F | Me | Me |
| 2-571 | 4-[NHCONH-(4-Me-1-Pipera)]-C₆H₄ | F | Me | H |
| 2-572 | 4-[NHCONH-(4-Me-1-Pipera)]-C₆H₄ | F | H | Me |
| 2-573 | 4-[NHCONH-(4-Me-1-Pipera)]-C₆H₄ | F | H | H |
| 2-574 | 4-[CONH-(4-Tet-pyra)]-C₆H₄ | H | iPr | Me |
| 2-575 | 4-[CONH-(4-Tet-pyra)]-C₆H₄ | H | iPr | H |
| 2-576 | 4-[CONH-(4-Tet-pyra)]-C₆H₄ | H | Me | Me |
| 2-577 | 4-[CONH-(4-Tet-pyra)]-C₆H₄ | H | Me | H |
| 2-578 | 4-[CONH-(4-Tet-pyra)]-C₆H₄ | H | H | Me |
| 2-579 | 4-[CONH-(4-Tet-pyra)]-C₆H₄ | H | H | H |
| 2-580 | 4-[CONH-(4-Tet-pyra)]-C₆H₄ | F | iPr | Me |
| 2-581 | 4-[CONH-(4-Tet-pyra)]-C₆H₄ | F | iPr | H |
| 2-582 | 4-[CONH-(4-Tet-pyra)]-C₆H₄ | F | Me | Me |
| 2-583 | 4-[CONH-(4-Tet-pyra)]-C₆H₄ | F | Me | H |
| 2-584 | 4-[CONH-(4-Tet-pyra)]-C₆H₄ | F | H | Me |
| 2-585 | 4-[CONH-(4-Tet-pyra)]-C₆H₄ | F | H | H |
| 2-586 | 4-[CH₂-(4-Morpho)]-C₆H₄ | H | iPr | Me |
| 2-587 | 4-[CH₂-(4-Morpho)]-C₆H₄ | H | iPr | H |
| 2-588 | 4-[CH₂-(4-Morpho)]-C₆H₄ | H | Me | Me |
| 2-589 | 4-[CH₂-(4-Morpho)]-C₆H₄ | H | Me | H |
| 2-590 | 4-[CH₂-(4-Morpho)]-C₆H₄ | H | H | Me |
| 2-591 | 4-[CH₂-(4-Morpho)]-C₆H₄ | H | H | H |
| 2-592 | 4-[CH₂-(4-Morpho)]-C₆H₄ | F | iPr | Me |
| 2-593 | 4-[CH₂-(4-Morpho)]-C₆H₄ | F | iPr | H |
| 2-594 | 4-[CH₂-(4-Morpho)]-C₆H₄ | F | Me | Me |
| 2-595 | 4-[CH₂-(4-Morpho)]-C₆H₄ | F | Me | H |
| 2-596 | 4-[CH₂-(4-Morpho)]-C₆H₄ | F | H | Me |
| 2-597 | 4-[CH₂-(4-Morpho)]-C₆H₄ | F | H | H |
| 2-598 | 4-Pyrazo | F | H | Me |
| 2-599 | 4-Pyrazo | F | Me | Me |
| 2-600 | 1-Me-4-Pyrazo | F | H | Me |
| 2-601 | 1-Me-4-Pyrazo | F | Me | Me |
| 2-602 | 4-OH-C₆H₄ | F | H | Me |
| 2-603 | 4-OH-C₆H₄ | F | Me | Me |
| 2-604 | 3-Pyrro | F | H | Me |
| 2-605 | 3-Pyrro | F | Me | Me |
| 2-606 | 5-Pyrazo | F | H | Me |
| 2-607 | 5-Pyrazo | F | Me | Me |
| 2-608 | 5-imidazo | F | H | Me |
| 2-609 | 5-imidazo | F | Me | Me |
| 2-610 | 4-SO₂Me-C₆H₄ | F | H | cPr |
| 2-611 | 4-SO₂Me-C₆H₄ | F | Me | cPr |
| 2-612 | 4-SO₂Me-C₆H₄ | H | H | cPr |
| 2-613 | 4-SO₂Me-C₆H₄ | H | Me | cPr |
| 2-614 | 2-CH(OH)CH₂OH-5-imidazo | F | H | Me |
| 2-615 | 2-CH(OH)CH₂OH-5-imidazo | F | Me | Me |
| 2-616 | 2-CH₂OH-5-imidazo | F | H | Me |
| 2-617 | 2-CH₂OH-5-imidazo | F | Me | Me |
| 2-618 | 4-SO₂NH₂-3-F-C₆H₄ | F | H | Me |
| 2-619 | 4-SO₂NH₂-3-F-C₆H₄ | F | Me | Me |
| 2-620 | 3-SO₂NH₂-4-OH-C₆H₄ | F | H | Me |
| 2-621 | 3-SO₂NH₂-4-OH-C₆H₄ | F | Me | Me |
| 2-622 | 1-Me-4-Pyrazo | F | H | H |
| 2-623 | 1-Me-4-Pyrazo | F | Me | H |
| 2-624 | 4-SO₂NH₂-C₆H₄ | F | H | cPr |
| 2-625 | 4-SO₂NH₂-C₆H₄ | F | Me | cPr |
| 2-626 | 4-SO₂NH₂-C₆H₄ | H | H | cPr |
| 2-627 | 4-SO₂NH₂-C₆H₄ | H | Me | cPr |
| 2-628 | 5-SO₂NH₂-2-Thi | F | H | Me |
| 2-629 | 5-SO₂NH₂-2-Thi | F | Me | Me |
| 2-630 | 4-SO₂NH₂-2,5-di-F-C₆H₄ | F | H | Me |
| 2-631 | 4-SO₂NH₂-2,5-di-F-C₆H₄ | F | Me | Me |
| 2-632 | 4-SO₂NH₂-3-OCF₃-C₆H₄ | F | H | Me |
| 2-633 | 4-SO₂NH₂-3-OCF₃-C₆H₄ | F | Me | Me |
| 2-634 | 4-SO₂NH₂-3-Cl-C₆H₄ | F | H | Me |
| 2-635 | 4-SO₂NH₂-3-Cl-C₆H₄ | F | Me | Me |
| 2-636 | 4-SO₂NH₂-3-CF₃-C₆H₄ | F | H | Me |
| 2-637 | 4-SO₂NH₂-3-CF₃-C₆H₄ | F | Me | Me |
| 2-638 | 4-SO₂NH₂-3-Me-C₆H₄ | F | H | Me |
| 2-639 | 4-SO₂NH₂-3-Me-C₆H₄ | F | Me | Me |
| 2-640 | 4-SO₂NH₂-2-Me-C₆H₄ | F | H | Me |
| 2-641 | 4-SO₂NH₂-2-Me-C₆H₄ | F | Me | Me |
| 2-642 | 4-SO₂NH₂-3-Et-C₆H₄ | F | H | Me |
| 2-643 | 4-SO₂NH₂-3-Et-C₆H₄ | F | Me | Me |
| 2-644 | 4-SO₂NMe₂-C₆H₄ | F | H | Me |
| 2-645 | 4-SO₂NMe₂-C₆H₄ | F | Me | Me |
| 2-646 | 5-CH₂OH-2-Py | F | H | Me |
| 2-647 | 5-CH₂OH-2-Py | F | Me | Me |
| 2-648 | 2-CMe₂OH-5-Py | F | H | Me |
| 2-649 | 2-CMe₂OH-5-Py | F | Me | Me |
| 2-650 | 5-CMe₂OH-2-Py | F | H | Me |
| 2-651 | 5-CMe₂OH-2-Py | F | Me | Me |
| 2-652 | 2-CMe₂OH-5-imidazo | F | H | Me |
| 2-653 | 2-CMe₂OH-5-imidazo | F | Me | Me |
| 2-654 | 4-SO₂NH₂-C₆H₄ | F | H | H |
| 2-655 | 4-SO₂NH₂-C₆H₄ | F | Me | H |
| 2-656 | 2-CH₂CH₂OH-5-imidazo | F | H | Me |
| 2-657 | 2-CH₂CH₂OH-5-imidazo | F | Me | Me |
| 2-658 | 5-SO₂NMe₂-2-Thi | F | H | Me |
| 2-659 | 5-SO₂NMe₂-2-Thi | F | Me | Me |
| 2-660 | 5-SO₂NHMe-2-Thi | F | H | Me |
| 2-661 | 5-SO₂NHMe-2-Thi | F | Me | Me |
| 2-662 | 1-CH₂CH₂OH-4-Pyrazo | F | H | Me |
| 2-663 | 1-CH₂CH₂-OH-4-Pyrazo | F | Me | Me |
| 2-664 | 5-CMe(CH₂F)OH-2-Py | F | H | Me |
| 2-665 | 5-CMe(CH₂F)OH-2-Py | F | Me | Me |
| 2-666 | 5-CMe(CF₃)OH-2-Py | F | H | Me |
| 2-667 | 5-CMe(CF₃)OH-2-Py | F | Me | Me |
| 2-668 | 5-CH(Me)OH-2-Py | F | H | Me |
| 2-669 | 5-CH(Me)OH-2-Py | F | Me | Me |
| 2-670 | 2-CH₂CMe₂OH-5-imidazo | F | H | Me |
| 2-671 | 2-CH₂CMe₂OH-5-imidazo | F | Me | Me |
| 2-672 | 4-SO₂CF₃-C₆H₄ | F | H | Me |
| 2-673 | 4-SO₂CF₃-C₆H₄ | F | Me | Me |
| 2-674 | 4-SCH₂CH₂OH-C₆H₄ | F | H | Me |
| 2-675 | 4-SCH₂CH₂OH-C₆H₄ | F | Me | Me |
| 2-676 | 4-SO₂CH₂CH₂OH-C₆H₄ | F | H | Me |
| 2-677 | 4-SO₂CH₂CH₂OH-C₆H₄ | F | Me | Me |
| 2-678 | 4-SO₂CHF₂-C₆H₄ | F | H | Me |
| 2-679 | 4-SO₂CHF₂-C₆H₄ | F | Me | Me |
| 2-680 | 5-OCH₂CH₂OH-2-Py | F | H | Me |
| 2-681 | 5-OCH₂CH₂OH-2-Py | F | Me | Me |
| 2-682 | 5-CMe₂OMe-2-Py | F | H | Me |
| 2-683 | 5-CMe₂OMe-2-Py | F | Me | Me |
| 2-684 | 5-CEt₂OH-2-Py | F | H | Me |
| 2-685 | 5-CEt₂OH-2-Py | F | Me | Me |
| 2-686 | 5-[OCH₂CH₂-(4-Morpho)]-2-Py | F | H | Me |
| 2-687 | 5-[OCH₂CH₂-(4-Morpho)]-2-Py | F | Me | Me□ |
| 2-688 | 5-[CH₂-(4-Morpho)]-2-Py | F | H | Me |
| 2-689 | 5-[CH₂-(4-Morpho)]-2-Py | F | Me | Me |
| 2-690 | 5-(4-Morpho)-2-Py | F | H | Me |
| 2-691 | 5-(4-Morpho)-2-Py | F | Me | Me |
| 2-692 | 5-(4-Me-1-Pipera)-2-Py | F | H | Me |
| 2-693 | 5-(4-Me-1-Pipera)-2-Py | F | Me | Me |
| 2-694 | 2-(4-Me-1-Pipera)-5-Py | F | H | Me |
| 2-695 | 2-(4-Me-1-Pipera)-5-Py | F | Me | Me |
| 2-696 | 2-(4-Morpho)-5-Py | F | H | Me |
| 2-697 | 2-(4-Morpho)-5-Py | F | Me | Me |
| 2-698 | 1-Me-4-imidazo | F | H | Me |
| 2-699 | 1-Me-4-imidazo | F | Me | Me |
| 2-700 | 1-CH₂CH₂OH-4-imidazo | F | H | Me |
| 2-701 | 1-CH₂CH₂OH-4-imidazo | F | Me | Me |
| 2-702 | 1-CH₂CH₂NMe₂-4-imidazo | F | H | Me |
| 2-703 | 1-CH₂CH₂NMe₂-4-imidazo | F | Me | Me |
| 2-704 | 1-[CH₂CH₂-(4-Morpho)]-4-imidazo | F | H | Me |
| 2-705 | 1-[CH₂CH₂-(4-Morpho)]-4-imidazo | F | Me | Me |
| 2-706 | 2-CH₂CH₂NMe₂-4-imidazo | F | H | Me |
| 2-707 | 2-CH₂CH₂NMe₂-4-imidazo | F | Me | Me |
| 2-708 | 2-[CH₂CH₂-(4-Morpho)]-4-imidazo | F | H | Me |
| 2-709 | 2-[CH₂CH₂-(4-Morpho)]-4-imidazo | F | Me | Me |
| 2-710 | 1-CH₂CH₂NMe₂-4-Pyrazo | F | H | Me |
| 2-711 | 1-CH₂CH₂NMe₂-4-Pyrazo | F | Me | Me |
| 2-712 | 1-[CH₂CH₂-(4-Morpho)]-4-Pyrazo | F | H | Me |
| 2-713 | 1-[CH₂CH₂-(4-Morpho)]-4-Pyrazo | F | Me | Me |
| 2-714 | 5-CMe₂OH-2-Pyrimi | F | H | Me |
| 2-715 | 5-CMe₂OH-2-Pyrimi | F | Me | Me |
| 2-716 | 2-CMe₂OH-4-Thiazo | F | H | Me |
| 2-717 | 2-CMe₂OH-4-Thiazo | F | Me | Me |
| 2-718 | 2-CH₂OH-4-Thiazo | F | H | Me |
| 2-719 | 2-CH₂OH-4-Thiazo | F | Me | Me |
| 2-720 | 2-CMe₂OH-5-Thiazo | F | H | Me |
| 2-721 | 2-CMe₂OH-5-Thiazo | F | Me | Me |
| 2-722 | Het(H) | F | H | Me |
| 2-723 | Het(H) | F | Me | Me |
| 2-724 | Het(I) | F | H | Me |
| 2-725 | Het(I) | F | Me | Me |
| 2-726 | Het(J) | F | H | Me |
| 2-727 | Het(J) | F | Me | Me |
| 2-728 | Het(K) | F | H | Me |
| 2-729 | Het(K) | F | Me | Me |
| 2-730 | 4-Pyrazo | H | H | Me |
| 2-731 | 4-Pyrazo | F | H | H |
| 2-732 | 4-Pyrazo | H | H | H |
| 2-733 | 1-Me-4-Pyrazo | H | H | Me |
| 2-734 | 1-Me-4-Pyrazo | F | H | H |
| 2-735 | 1-Me-4-Pyrazo | H | H | H |
| 2-736 | 4-OH-C₆H₄ | H | H | Me |
| 2-737 | 4-OH-C₆H₄ | F | H | H |
| 2-738 | 4-OH-C₆H₄ | H | H | H |
| 2-739 | 3-Pyrro | H | H | Me |
| 2-740 | 3-Pyrro | F | H | H |
| 2-741 | 3-Pyrro | H | H | H |
| 2-742 | 5-Pyrazo | H | H | Me |
| 2-743 | 5-Pyrazo | F | H | H |
| 2-744 | 5-Pyrazo | H | H | H |
| 2-745 | 5-imidazo | H | H | Me |
| 2-746 | 5-imidazo | F | H | H |
| 2-747 | 5-imidazo | H | H | H |
| 2-748 | 4-SO₂Me-C₆H₄ | Cl | H | H |
| 2-749 | 4-SO₂Me-C₆H₄ | F | H | H |
| 2-750 | 4-SO₂Me-C₆H₄ | H | H | H |
| 2-751 | 2-CH(OH)CH₂OH-5-imidazo | H | H | Me |
| 2-752 | 2-CH(OH)CH₂OH-5-imidazo | F | H | H |
| 2-753 | 2-CH(OH)CH₂OH-5-imidazo | H | H | H |
| 2-754 | 2-CH₂OH-5-imidazo | H | H | Me |
| 2-755 | 2-CH₂OH-5-imidazo | F | H | H |
| 2-756 | 2-CH₂OH-5-imidazo | H | H | H |
| 2-757 | 4-SO₂NH₂-3-F-C₆H₄ | H | H | Me |
| 2-758 | 4-SO₂NH₂-3-F-C₆H₄ | F | H | H |
| 2-759 | 4-SO₂NH₂-3-F-C₆H₄ | H | H | H |
| 2-760 | 3-SO₂NH₂-4-OH-C₆H₄ | H | H | Me |
| 2-761 | 3-SO₂NH₂-4-OH-C₆H₄ | F | H | H |
| 2-762 | 3-SO₂NH₂-4-OH-C₆H₄ | H | H | H |
| 2-763 | 1-Et-4-Pyrazo | H | H | Me |
| 2-764 | 1-Et-4-Pyrazo | F | H | H |
| 2-765 | 1-Et-4-Pyrazo | H | H | H |
| 2-766 | 4-SO₂NH₂-C₆H₄ | Cl | H | H |
| 2-767 | 4-SO₂NH₂-C₆H₄ | F | H | H |
| 2-768 | 4-SO₂NH₂-C₆H₄ | H | H | H |
| 2-769 | 5-SO₂NH₂-2-Thi | H | H | Me |
| 2-770 | 5-SO₂NH₂-2-Thi | F | H | H |
| 2-771 | 5-SO₂NH₂-2-Thi | H | H | H |
| 2-772 | 4-SO₂NH₂-2,5-di-F-C₆H₄ | H | H | Me |
| 2-773 | 4-SO_{2N}H₂-2,5-di-F-C₆H₄ | F | H | H |
| 2-774 | 4-SO₂NH₂-2,5-di-F-C₆H₄ | H | H | H |
| 2-775 | 4-SO₂NH₂-3-OCF₃-C₆H₄ | H | H | Me |
| 2-776 | 4-SO₂NH₂-3-OCF₃-C₆H₄ | F | H | H |
| 2-777 | 4-SO₂NH₂-3-OCF₃-C₆H₄ | H | H | H |
| 2-778 | 4-SO₂N-H₂-3-Cl-C₆H₄ | H | H | Me |
| 2-779 | 4-SO₂NH₂-3-Cl-C₆H₄ | F | H | H |
| 2-780 | 4-SO₂NH₂-3-Cl-C₆H₄ | H | H | H |
| 2-781 | 4-SO₂NH₂-3-CF₃-C₆H₄ | H | H | Me |
| 2-782 | 4-SO₂NH₂-3-CF₃-C₆H₄ | F | H | H |
| 2-783 | 4-SO₂NH₂-3-CF₃-C₆H₄ | H | H | H |
| 2-784 | 4-SO₂NH₂-3-Me-C₆H₄ | H | H | Me |
| 2-785 | 4-SO₂NH₂-3-Me-C₆H₄ | F | H | H |
| 2-786 | 4-SO₂N-H₂-3-Me-C₆H₄ | H | H | H |
| 2-787 | 4-SO₂NH₂-2-Me-C₆H₄ | H | H | Me |
| 2-788 | 4-SO₂NH₂-2-Me-C₆H₄ | F | H | H |
| 2-789 | 4-SO₂NH₂-2-Me-C₆H₄ | H | H | H |
| 2-790 | 4-SO₂NH₂-3-Et-C₆H₄ | H | H | Me |
| 2-791 | 4-SO₂NH₂-3-Et-C₆H₄ | F | H | H |
| 2-792 | 4-SO₂NH₂-3-Et-C₆H₄ | H | H | H |
| 2-793 | 4-SO₂NMe₂-C₆H₄ | H | H | Me |
| 2-794 | 4-SO₂NMe₂-C₆H₄ | F | H | H |
| 2-795 | 4-SO₂NMe₂-C₆H₄ | H | H | H |
| 2-796 | 5-CH₂OH-2-Py | H | H | Me |
| 2-797 | 5-CH₂OH-2-Py | F | H | H |
| 2-798 | 5-CH₂OH-2-Py | H | H | H |
| 2-799 | 2-CMe₂OH-5-Py | H | H | Me |
| 2-800 | 2-CMe₂OH-5-Py | F | H | H |
| 2-801 | 2-CMe₂OH-5-Py | H | H | H |
| 2-802 | 5-CMe₂OH-2-Py | H | H | Me |
| 2-803 | 5-CMe₂OH-2-Py | F | H | H |
| 2-804 | 5-CMe₂OH-2-Py | H | H | H |
| 2-805 | 2-CMe₂OH-5-imidazo | H | H | Me |
| 2-806 | 2-CMe₂OH-5-imidazo | F | H | H |
| 2-807 | 2-CMe₂OH-5-imidazo | H | H | H |
| 2-808 | 3-SO₂NH₂-C₆H₄ | F | H | Me |
| 2-809 | 3-SO₂NH₂-C₆H₄ | F | H | H |
| 2-810 | 3-SO₂NH₂-C₆H₄ | H | H | H |
| 2-811 | 2-CH₂CH₂OH-5-imidazo | H | H | Me |
| 2-812 | 2-CH₂CH₂OH-5-imidazo | F | H | H |
| 2-813 | 2-CH₂CH₂OH-5-imidazo | H | H | H |
| 2-814 | 5-SO₂NMe₂-2-Thi | H | H | Me |
| 2-815 | 5-SO₂NMe₂-2-Thi | F | H | H |
| 2-816 | 5-SO₂NMe₂-2-Thi | H | H | H |
| 2-817 | 5-SO₂NHMe-2-Thi | H | H | Me |
| 2-818 | 5-SO₂NHMe-2-Thi | F | H | H |
| 2-819 | 5-SO₂NHMe-2-Thi | H | H | H |
| 2-820 | 1-CH₂CH₂OH-4-Pyrazo | H | H | Me |
| 2-821 | 1-CH₂CH₂OH-4-Pyrazo | F | H | H |
| 2-822 | 1-CH₂CH₂OH-4-Pyrazo | H | H | H |
| 2-823 | 5-CMe(CH₂F)OH-2-Py | H | H | Me |
| 2-824 | 5-CMe(CH₂F)OH-2-Py | F | H | H |
| 2-825 | 5-CMe(CH₂F)OH-2-Py | H | H | H |
| 2-826 | 5-CMe(CF₃)OH-2-Py | H | H | Me |
| 2-827 | 5-CMe(CF₃)OH-2-Py | F | H | H |
| 2-828 | 5-CMe(CF₃)OH-2-Py | H | H | H |
| 2-829 | 5-CH(Me)OH-2-Py | H | H | Me |
| 2-830 | 5-CH(Me)OH-2-Py | F | H | H |
| 2-831 | 5-CH(Me)OH-2-Py | H | H | H |
| 2-832 | 2-CH₂CMe₂OH-5-imidazo | H | H | Me |
| 2-833 | 2-CH₂CMe₂OH-5-imidazo | F | H | H |
| 2-834 | 2-CH₂CMe₂OH-5-imidazo | H | H | H |
| 2-835 | 4-SO₂CF₃-C₆H₄ | H | H | Me |
| 2-836 | 4-SO₂CF₃-C₆H₄ | F | H | H |
| 2-837 | 4-SO₂CF₃-C₆H₄ | H | H | H |
| 2-838 | 4-SCH₂CH₂OH-C₆H₄ | H | H | Me |
| 2-839 | 4-SCH₂CH₂OH-C₆H₄ | F | H | H |
| 2-840 | 4-SCH₂CH₂OH-C₆H₄ | H | H | H |
| 2-841 | 4-SO₂CH₂CH₂OH-C₆H₄ | H | H | Me |
| 2-842 | 4-SO₂CH₂CH₂OH-C₆H₄ | F | H | H |
| 2-843 | 4-SO₂CH₂CH₂OH-C₆H₄ | H | H | H |
| 2-844 | 4-SO₂CHF₂-C₆H₄ | H | H | Me |
| 2-845 | 4-SO₂CHF₂-C₆H₄ | F | H | H |
| 2-846 | 4-SO₂CHF₂-C₆H₄ | H | H | H |
| 2-847 | 5-OCH₂CH₂OH-2-Py | H | H | Me |
| 2-848 | 5-OCH₂CH₂OH-2-Py | F | H | H |
| 2-849 | 5-OCH₂CH₂OH-2-Py | H | H | H |
| 2-850 | 5-CMe₂OMe-2-Py | H | H | Me |
| 2-851 | 5-CMe₂OMe-2-Py | F | H | H |
| 2-852 | 5-CMe₂OMe-2-Py | H | H | H |
| 2-853 | 5-CEt₂OH-2-Py | H | H | Me |
| 2-854 | 5-CEt₂OH-2-Py | F | H | H |
| 2-855 | 5-CEt₂OH-2-Py | H | H | H |
| 2-856 | 5-[OCH₂CH₂-(4-Morpho)]-2-Py | H | H | Me |
| 2-857 | 5-[OCH₂CH₂-(4-Morpho)]-2-Py | F | H | H |
| 2-858 | 5-[OCH₂CH₂-(4-Morpho)]-2-Py | H | H | H |
| 2-859 | 5-[CH₂-(4-Morpho)]-2-Py | H | H | Me |
| 2-860 | 5-[CH₂-(4-Morpho)]-2-Py | F | H | H |
| 2-861 | 5-[CH₂-(4-Morpho)]-2-Py | H | H | H |
| 2-862 | 5-(4-Morpho)-2-Py | H | H | Me |
| 2-863 | 5-(4-Morpho)-2-Py | F | H | H |
| 2-864 | 5-(4-Morpho)-2-Py | H | H | H |
| 2-865 | 5-(4-Me-1-Pipera)-2-Py | H | H | Me |
| 2-866 | 5-(4-Me-1-Pipera)-2-Py | F | H | H |
| 2-867 | 5-(4-Me-1-Pipera)-2-Py | H | H | H |
| 2-868 | 2-(4-Me-1-Pipera)-5-Py | H | H | Me |
| 2-869 | 2-(4-Me-1-Pipera)-5-Py | F | H | H |
| 2-870 | 2-(4-Me-1-Pipera)-5-Py | H | H | H |
| 2-871 | 2-(4-Morpho)-5-Py | H | H | Me |
| 2-872 | 2-(4-Morpho)-5-Py | F | H | H |
| 2-873 | 2-(4-Morpho)-5-Py | H | H | H |
| 2-874 | 1-Me-4-imidazo | H | H | Me |
| 2-875 | 1-Me-4-imidazo | F | H | H |
| 2-876 | 1-Me-4-imidazo | H | H | H |
| 2-877 | 1-CH₂CH₂OH-4-imidazo | H | H | Me |
| 2-878 | 1-CH₂CH₂OH-4-imidazo | F | H | H |
| 2-879 | 1-CH₂CH₂OH-4-imidazo | H | H | H |
| 2-880 | 1-CH₂CH₂NMe₂-4-imidazo | H | H | Me |
| 2-881 | 1-CH₂CH₂NMe₂-4-imidazo | F | H | H |
| 2-882 | 1-CH₂CH₂NMe₂-4-imidazo | H | H | H |
| 2-883 | 1-[CH₂CH₂-(4-Morpho)]-4-imidazo | H | H | Me |
| 2-884 | 1-[CH₂CH₂-(4-Morpho)]-4-imidazo | F | H | H |
| 2-885 | 1-[CH₂CH₂-(4-Morpho)]-4-imidazo | H | H | H |
| 2-886 | 2-CH₂CH₂NMe₂-4-imidazo | H | H | Me |
| 2-887 | 2-CH₂CH₂NMe₂-4-imidazo | F | H | H |
| 2-888 | 2-CH₂CH₂NMe₂-4-imidazo | H | H | H |
| 2-889 | 2-[CH₂CH₂-(4-Morpho)]-4-imidazo | H | H | Me |
| 2-890 | 2-[CH₂CH₂-(4-Morpho)]-4-imidazo | F | H | H |
| 2-891 | 2-[CH₂CH₂-(4-Morpho)]-4-imidazo | H | H | H |
| 2-892 | 1-CH₂CH₂NMe₂-4-Pyrazo | H | H | Me |
| 2-893 | 1-CH₂CH₂NMe₂-4-Pyrazo | F | H | H |
| 2-894 | 1-CH₂CH₂NMe₂-4-Pyrazo | H | H | H |
| 2-895 | 1-[CH₂CH₂-(4-Morpho)]-4-Pyrazo | H | H | Me |
| 2-896 | 1-[CH₂CH₂-(4-Morpho)]-4-Pyrazo | F | H | H |
| 2-897 | 1-[CH₂CH₂-(4-Morpho)]-4-Pyrazo | H | H | H |
| 2-898 | 5-CMe₂OH-2-Pyrimi | H | H | Me |
| 2-899 | 5-CMe₂OH-2-Pyrimi | F | H | H |
| 2-900 | 5-CMe₂OH-2-Pyrimi | H | H | H |
| 2-901 | 2-CMe₂OH-4-Thiazo | H | H | Me |
| 2-902 | 2-CMe₂OH-4-Thiazo | F | H | H |
| 2-903 | 2-CMe₂OH-4-Thiazo | H | H | H |
| 2-904 | 2-CH₂OH-4-Thiazo | H | H | Me |
| 2-905 | 2-CH₂OH-4-Thiazo | F | H | H |
| 2-906 | 2-CH₂OH-4-Thiazo | H | H | H |
| 2-907 | 2-CMe₂OH-5-Thiazo | H | H | Me |
| 2-908 | 2-CMe₂OH-5-Thiazo | F | H | H |
| 2-909 | 2-CMe₂OH-5-Thiazo | H | H | H |
| 2-910 | Het(H) | H | H | Me |
| 2-911 | Het(H) | F | H | H |
| 2-912 | Het(H) | H | H | H |
| 2-913 | Het(I) | H | H | Me |
| 2-914 | Het(I) | F | H | H |
| 2-915 | Het(I) | H | H | H |
| 2-916 | Het(J) | H | H | Me |
| 2-917 | Het(J) | F | H | H |
| 2-918 | Het(J) | H | H | H |
| 2-919 | Het(K) | H | H | Me |
| 2-920 | Het(K) | F | H | H |
| 2-921 | Het(K) | H | H | H |
| 2-922 | 1-Et-4-Pyrazo | F | H | Me |
| 2-923 | 1-Et-4-Pyrazo | F | Me | Me |

**(Table 3)**

| | | | |
|---|---|---|---|
| 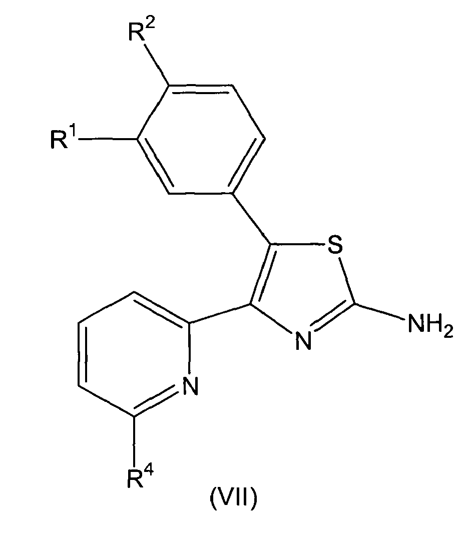 | | | |

| Compound No. | R¹ | R² | R⁴ |
|---|---|---|---|
| 3-1 | 4-OMe-C₆H₄ | H | Me |
| 3-2 | 4-OMe-C₆H₄ | H | H |
| 3-3 | 4-OMe-C₆H₄ | Me | Me |
| 3-4 | 4-OMe-C₆H₄ | Me | H |
| 3-5 | 4-OMe-C₆H₄ | F | Me |
| 3-6 | 4-OMe-C₆H₄ | F | H |
| 3-7 | 4-OMe-C₆H₄ | Cl | Me |
| 3-8 | 4-OMe-C₆H₄ | Cl | H |
| 3-9 | 4-NH₂-C₆H₄ | H | Me |
| 3-10 | 4-NH₂-C₆H₄ | H | H |
| 3-11 | 4-NH₂-C₆H₄ | Me | Me |
| 3-12 | 4-NH₂-C₆H₄ | Me | H |
| 3-13 | 4-NH₂-C₆H₄ | F | Me |
| 3-14 | 4-NH₂-C₆H₄ | F | H |
| 3-15 | 4-NH₂-C₆H₄ | Cl | Me |
| 3-16 | 4-NH₂-C₆H₄ | Cl | H |
| 3-17 | 4-SMe-C₆H₄ | H | Me |
| 3-18 | 4-SMe-C₆H₄ | H | H |
| 3-19 | 4-SMe-C₆H₄ | Me | Me |
| 3-20 | 4-SMe-C₆H₄ | Me | H |
| 3-21 | 4-SMe-C₆H₄ | F | Me |
| 3-22 | 4-SMe-C₆H₄ | F | H |
| 3-23 | 4-SMe-C₆H₄ | Cl | Me |
| 3-24 | 4-SMe-C₆H₄ | Cl | H |
| 3-25 | 4-SOMe-C₆H₄ | H | Me |
| 3-26 | 4-SOMe-C₆H₄ | H | H |
| 3-27 | 4-SOMe-C₆H₄ | Me | Me |
| 3-28 | 4-SOMe-C₆H₄ | Me | H |
| 3-29 | 4-SOMe-C₆H₄ | F | Me |
| 3-30 | 4-SOMe-C₆H₄ | F | H |
| 3-31 | 4-SOMe-C₆H₄ | Cl | Me |
| 3-32 | 4-SOMe-C₆H₄ | Cl | H |
| 3-33 | 4-SO₂Me-C₆H₄ | H | Me |
| 3-34 | 4-SO₂Me-C₆H₄ | H | H |
| 3-35 | 4-SO₂Me-C₆H₄ | Me | Me |
| 3-36 | 4-SO₂Me-C₆H₄ | Me | H |
| 3-37 | 4-SO₂Me-C₆H₄ | F | Me |
| 3-38 | 4-SO₂Me-C₆H₄ | F | H |
| 3-39 | 4-SO₂Me-C₆H₄ | Cl | Me |
| 3-40 | 4-SO₂Me-C₆H₄ | Cl | H |
| 3-41 | 4-NHSO₂Me-C₆H₄ | H | Me |
| 3-42 | 4-NHSO₂Me-C₆H₄ | H | H |
| 3-43 | 4-NHSO₂Me-C₆H₄ | Me | Me |
| 3-44 | 4-NHSO₂Me-C₆H₄ | Me | H |
| 3-45 | 4-NHSO₂Me-C₆H₄ | F | Me |
| 3-46 | 4-NHSO₂Me-C₆H₄ | F | H |
| 3-47 | 4-NHSO₂Me-C₆H₄ | Cl | Me |
| 3-48 | 4-NHSO₂Me-C₆H₄ | Cl | H |
| 3-49 | 4-NHSO₂Et-C₆H₄ | H | Me |
| 3-50 | 4-NHSO₂Et-C₆H₄ | H | H |
| 3-51 | 4-NHSO₂Et-C₆H₄ | Me | Me |
| 3-52 | 4-NHSO₂Et-C₆H₄ | Me | H |
| 3-53 | 4-NHSO₂Et-C₆H₄ | F | Me |
| 3-54 | 4-NHSO₂Et-C₆H₄ | F | H |
| 3-55 | 4-NHSO₂Et-C₆H₄ | Cl | Me |
| 3-56 | 4-NHSO₂Et-C₆H₄ | Cl | H |
| 3-57 | 4-NHSO₂cPr-C₆H₄ | H | Me |
| 3-58 | 4-NHSO₂cPr-C₆H₄ | H | H |
| 3-59 | 4-NHSO₂cPr-C₆H₄ | Me | Me |
| 3-60 | 4-NHSO₂cPr-C₆H₄ | Me | H |
| 3-61 | 4-NHSO₂cPr-C₆H₄ | F | Me |
| 3-62 | 4-NHSO₂cPr-C₆H₄ | F | H |
| 3-63 | 4-NHSO₂cPr-C₆H₄ | Cl | Me |
| 3-64 | 4-NHSO₂cPr-C₆H₄ | Cl | H |
| 3-65 | 4-SO₂NH₂-C₆H₄ | H | Me |
| 3-66 | 4-SO₂NH₂-C₆H₄ | H | H |
| 3-67 | 4-SO₂NH₂-C₆H₄ | Me | Me |
| 3-68 | 4-SO₂NH₂-C₆H₄ | Me | H |
| 3-69 | 4-SO₂NH₂-C₆H₄ | F | Me |
| 3-70 | 4-SO₂NH₂-C₆H₄ | F | H |
| 3-71 | 4-SO₂NH₂-C₆H₄ | Cl | Me |
| 3-72 | 4-SO₂NH₂-C₆H₄ | Cl | H |
| 3-73 | 4-[CONH-(4-Morpho)]-C₆H₄ | H | Me |
| 3-74 | 4-[CONH-(4-Morpho)]-C₆H₄ | H | H |
| 3-75 | 4-[CONH-(4-Morpho)]-C₆H₄ | Me | Me |
| 3-76 | 4-[CONH-(4-Morpho)]-C₆H₄ | Me | H |
| 3-77 | 4-[CONH-(4-Morpho)]-C₆H₄ | F | Me |
| 3-78 | 4-[CONH-(4-Morpho)]-C₆H₄ | F | H |
| 3-79 | 4-[CONH-(4-Morpho)]-C₆H₄ | Cl | Me |
| 3-80 | 4-[CONH-(4-Morpho)]-C₆H₄ | Cl | H |
| 3-81 | 4-[CONH-(4-Me-1-Pipera)]-C₆H₄ | H | Me |
| 3-82 | 4-[CONH-(4-Me-1-Pipera)]-C₆H₄ | H | H |
| 3-83 | 4-[CONH-(4-Me-1-Pipera)]-C₆H₄ | Me | Me |
| 3-84 | 4-[CONH-(4-Me-1-Pipera)]-C₆H₄ | Me | H |
| 3-85 | 4-[CONH-(4-Me-1-Pipera)]-C₆H₄ | F | Me |
| 3-86 | 4-[CONH-(4-Me-1-Pipera)]-C₆H₄ | F | H |
| 3-87 | 4-[CONH-(4-Me-1-Pipera)]-C₆H₄ | Cl | Me |
| 3-88 | 4-[CONH-(4-Me-1-Pipera)]-C₆H₄ | Cl | H |
| 3-89 | 3-Thi | H | Me |
| 3-90 | 3-Thi | H | H |
| 3-91 | 3-Thi | Me | Me |
| 3-92 | 3-Thi | Me | H |
| 3-93 | 3-Thi | F | Me |
| 3-94 | 3-Thi | F | H |
| 3-95 | 3-Thi | Cl | Me |
| 3-96 | 3-Thi | Cl | H |
| 3-97 | 2-Pyrro | H | Me |
| 3-98 | 2-Pyrro | H | H |
| 3-99 | 2-Pyrro | Me | Me |
| 3-100 | 2-Pyrro | Me | H |
| 3-101 | 2-Pyrro | F | Me |
| 3-102 | 2-Pyrro | F | H |
| 3-103 | 2-Pyrro | Cl | Me |
| 3-104 | 2-Pyrro | Cl | H |
| 3-105 | 5-CONH₂-2-Py | H | Me |
| 3-106 | 5-CONH₂-2-Py | H | H |
| 3-107 | 5-CONH₂-2-Py | Me | Me |
| 3-108 | 5-CONH₂-2-Py | Me | H |
| 3-109 | 5-CONH₂-2-Py | F | Me |
| 3-110 | 5-CONH₂-2-Py | F | H |
| 3-111 | 5-CONH₂-2-Py | Cl | Me |
| 3-112 | 5-CONH₂-2-Py | Cl | H |
| 3-113 | 4-SO₂Et-C₆H₄ | H | Me |
| 3-114 | 4-NHCOMe-C₆H₄ | H | Me |
| 3-115 | 4-NHCOCMe₂OH-C₆H₄ | H | Me |
| 3-116 | 4-NHCOCMe₂OCOMe-C₆H₄ | H | Me |
| 3-117 | 4-CONH₂-C₆H₄ | H | Me |
| 3-118 | 3-CONH₂-C₆H₄ | H | Me |
| 3-119 | 4-CONHCH₂CH₂OH-C₆H₄ | H | Me |
| 3-120 | 4-SO₂NHMe-C₆H₄ | H | Me |
| 3-121 | 4-[CO-(4-Morpho)]-C₆H₄ | H | Me |
| 3-122 | 4-[SO₂-(4-Morpho)]-C₆H₄ | H | Me |
| 3-123 | 3-Fur | H | Me |
| 3-124 | 2-OMe-5-Py | H | Me |
| 3-125 | 4-Py | H | Me |
| 3-126 | 1-Me-4-Pyrazo | F | Me |
| 3-127 | 1-Me-4-Pyrazo | F | H |
| 3-128 | 5-(4-Morpho)-2-Py | F | Me |
| 3-129 | 5-(4-Morpho)-2-Py | F | Me |
| 3-130 | 5-(4-Me-1-Pipera)-2-Py | F | Me |
| 3-131 | 5-(4-Me-1-Pipera)-2-Py | F | Me |
| 3-132 | 2-(4-Me-1-Pipera)-5-Py | F | Me |
| 3-133 | 2-(4-Me-1-Pipera)-5-Py | F | Me |
| 3-134 | 2-(4-Morpho)-5-Py | F | Me |
| 3-135 | 2-(4-Morpho)-5-Py | F | Me |
| 3-136 | 1-Me-4-imidazo | F | Me |
| 3-137 | 1-Me-4-imidazo | F | Me |
| 3-138 | 5-(4-Morpho)-2-Py | H | Me |
| 3-139 | 5-(4-Morpho)-2-Py | F | H |
| 3-140 | 5-(4-Morpho)-2-Py | H | H |
| 3-141 | 5-(4-Me-1-Pipera)-2-Py | H | Me |
| 3-142 | 5-(4-Me-1-Pipera)-2-Py | F | H |
| 3-143 | 5-(4-Me-1-Pipera)-2-Py | H | H |
| 3-144 | 2-(4-Me-1-Pipera)-5-Py | H | Me |
| 3-145 | 2-(4-Me-1-Pipera)-5-Py | F | H |
| 3-146 | 2-(4-Me-1-Pipera)-5-Py | H | H |
| 3-147 | 2-(4-Morpho)-5-Py | H | Me |
| 3-148 | 2-(4-Morpho)-5-Py | F | H |
| 3-149 | 2-(4-Morpho)-5-Py | H | H |
| 3-150 | 1-Me-4-imidazo | H | Me |
| 3-151 | 1-Me-4-imidazo | F | H |
| 3-152 | 1-Me-4-imidazo | H | H |

In Tables 1 to 3, preferred compounds are Compound Nos. 1-1, 1-13, 1-15, 1-25, 1-37, 1-39, 1-49, 1-61, 1-63, 1-73, 1-85, 1-87, 1-97, 1-109, 1-111, 1-121, 1-133, 1-135, 1-145, 1-157, 1-159, 1-181, 1-183, 1-193, 1-205, 1-207, 1-217, 1-229, 1-231, 1-241, 1-253, 1-255, 1-265, 1-277, 1-279, 1-289, 1-301, 1-303, 1-313, 1-325, 1-327, 1-343, 1-361, 1-373, 1-403, 1-409, 1-505, 1-508, 1-514, 1-520, 1-523, 1-526, 1-529, 1-532, 1-533, 1-534, 1-535, 1-536, 1-539, 1-551, 1-553, 1-555, 1-563, 1-575, 1-577, 1-588, 1-589, 1-592, 1-604, 1-606, 1-616, 1-622, 1-624, 1-628, 1-634, 1-636, 1-646, 1-652, 1-658, 1-660, 1-670, 1-672, 1-676, 1-679, 1-681, 1-682, 1-683, 1-684, 1-685, 1-686, 1-687, 1-688, 1-689, 1-691, 1-692, 1-693, 1-694, 1-697, 1-699, 1-701, 1-703, 1-705, 1-707, 1-709, 1-711, 1-713, 1-715, 1-717, 1-719, 1-721, 1-723, 1-724, 1-725, 1-727, 1-733, 1-735, 1-737, 1-739, 1-741, 1-742, 1-744, 1-748, 1-750, 1-756, 1-757, 1-759, 1-764, 1-765, 1-767, 1-769, 1-771, 1-773, 1-774, 1-775, 1-777, 1-779, 1-780, 1-781, 1-782, 1-783, 1-784, 1-785, 1-787, 1-789, 1-790, 1-791, 1-792, 1-793, 1-796, 1-797, 1-798, 1-799, 1-800, 1-801, 1^802, 1-803, 1-805, 1-806, 1-808, 1-809, 1-811, 1-812, 1-794, 1-795, 2-109, 2-380, 2-388, 2-396, 2-404, 2-408, 2-412, 2-413, 2-420, 2-428, 2-436, 2-444, 2-452, 2-460, 2-468, 2-476, 2-484, 2-490, 2-494, 2-498, 2-502, 2-506, 2-510, 2-514, 2-518, 2-522, 2-526, 2-530, 2-534, 2-538, 2-550, 2-572, 2-584, 2-596, 2-598, 2-600, 2-602, 2-604, 2-606, 2-608, 2-610, 2-614, 2-616, 2-618, 2-620, 2-622, 2-624, 2-628, 2-630, 2-632, 2-634, 2-636, 2-638, 2-640, 2-642, 2-644, 2-646, 2-648, 2-650, 2-652, 2-654, 2-656, 2-658, 2-660, 2-662, 2-663, 2-664, 2-666, 2-668, 2-670, 2-672, 2-674, 2-676, 2-678, 2-680, 2-682, 2-684, 2-686, 2-688, 2-690, 2-691, 2-692, 2-693, 2-694, 2-695, 2-696, 2-697, 2-698, 2-699, 2-700, 2-702, 2-704, 2-706, 2-708, 2-710, 2-712, 2-716, 2-718, 2-720, 2-722, 2-724, 2-726, 2-728, 2-730, 2-733, 2-734, 2-739, 2-740, 2-749, 2-751, 2-752, 2-754, 2-755, 2-757, 2-758, 2-763, 2-764, 2-767, 2-770, 2-793, 2-794, 2-796, 2-797, 2-799, 2-800, 2-802, 2-803, 2-805, 2-806, 2-811, 2-812, 2-814, 2-815, 2-817, 2-818, 2-820, 2-821, 2-832, 2-833, 2-838, 2-839, 2-847, 2-848, 2-856, 2-857, 2-859, 2-860, 2-862, 2-863, 2-865, 2-866, 2-868, 2-869, 2-871, 2-872, 2-874, 2-875, 2-877, 2-878, 2-880, 2-881, 2-883, 2-884, 2-886, 2-887, 2-889, 2-890, 2-892, 2-893, 2-895, 2-896, 2-904, 2-905, 2-907, 2-908, 2-913, 2-914, 2-919, 2-920, 2-922, 3-5, 3-13, 3-21, 3-29, 3-33, 3-37, 3-45, 3-53, 3-61, 3-69, 3-77, 3-85, 3-93, 3-101, 3-109, 3-126, 3-127, 2-128, 3-129, 3-130, 3-131, 3-132, 3-133, 3-134, 3-136, 3-137, 3-139, 3-140, 3-142, 3-143, 3-145 and 3-146;
more preferred compounds are Compound Nos. 1-109, 1-181, 1-205, 1-229, 1-241, 1-253, 1-301, 1-551, 1-575, 1-588, 1-592, 1-604, 1-622, 1-634, 1-658, 1-670, 1-683, 1-699, 1-701, 1-705, 1-707, 1-711, 1-721, 1-723, 1-725, 1-727, 1-733, 1-735, 1-737, 1-741, 1-744, 1-757, 1-759, 1-767, 1-769, 1-771, 1-773, 1-775, 1-793, 1-794, 1-796, 1-798, 1-800, 2-412, 2-444, 2-452, 2-476, 2-518, 2-550, 2-598, 2-600, 2-602, 2-604, 2-606, 2-608, 2-614, 2-616, 2-620, 2-622, 2-624, 2-628, 2-634, 2-638, 2-642, 2-644, 2-646, 2-648, 2-650, 2-652, 2-656, 2-662, 2-668, 2-674, 2-680, 2-682, 2-686, 2-690, 2-692, 2-694, 2-696, 2-698, 2-700, 2-702, 2-704, 2-706, 2-708, 2-710, 2-712, 2-718, 2-720, 2-724, 2-726, 2-728, 2-922, 3-69, 3-126, 3-128, 3-129, 3-130, 3-131 and 3-132;
further more preferred compounds are
2-{4-[6-fluoro-4'-(methylsulfonyl)-1,1'-biphenyl-3-yl]-2-isopropyl-1H-imidazol-5-yl}-6-methylpyridine (Compound No. 1-109),
2'-fluoro-5'-[2-isopropyl-5-(6-methylpyridin-2-yl)-1H-imidazol-4-yl]-1,1'-biphenyl-4-sulfonamide (Compound No. 1-205),
N-(morpholin-4-yl)-4-{2-fluoro-5-[2-isopropyl-5-(6-methylpyridin-2-yl)-1H-imidazol-4-yl]-phenyl}benzamide (Compound No. 1-229),
2-{4-[4-fluoro-3-(1H-pyrrol-2-yl)phenyl]-2-isopropyl-1H-imidazol-5-yl-6-methylpyridine (Compound No. 1-301),
2-{4-[4-fluoro-3-(1H-pyrrol-3-yl)phenyl]-2-isopropyl-1H-imidazol-5-yl}-6-methylpyridine (Compound No. 1-683),
2-{4-[6-fluoro-4'-(2-pyrrolidin-1-ylethoxy)-1,1'-biphenyl-3-yl]-2-isopropyl-1H-imidazol-5-yl}-6-methylpyridine (Compound No. 1-699),
2-{5-[4-fluoro-3-(1H-pyrazol-4-yl)phenyl]-2-isopropyl-1H-pyrazol-4-yl}-6-methylpyridine (Compound No. 1-723),
2-{5-[4-fluoro-3-(1-methyl-1H-pyrazol-4-yl)phenyl]-2-isopropyl-1H-imidazol-4-yl}-6-methylpyridine (Compound No. 1-725),
2-{5-[3-(1-ethyl-1H-pyrazol-4-yl)-4-fluorophenyl]-2-isopropyl-1H-imidazol-4-yl}-6-methylpyridine (Compound No. 1-737),
2-{4-[4-fluoro-3-(1H-imidazol-4-yl)phenyl]-2-isopropyl-1H-imidazol-5-yl}-6-methylpyridine (Compound No. 1-769),
2-{4-[4-fluoro-3-(1-methyl-1H-imidazol-4-yl)phenyl]-2-isopropyl-1H-imidazol-5-yl}-6-methylpyridine (Compound No. 1-775),
2-{4-[6-fluoro-4'-(methylsulfonyl)-1,1'-biphenyl-3-yl]-1H-pyrazol-3-yl}-6-methylpyridine (Compound No. 2-412),
2'-fluoro-5'-[3-(6-methylpyridin-2-yl)-1 H-pyrazol-4-yl]-1,1'-biphenyl-4-sulfonamide (Compound No. 2-444),
2'-fluoro-N-methyl-5'-[3-(6-methylpyridin-2-yl)-1 H-pyrazol-4-yl]-1,1'-biphenyl-4-sulfonamide (Compound No. 2-518),
2-{4-[4-fluoro-3-(1H-pyrazol-4-yl)phenyl]-1H-pyrazol-3-yl}-6-methylpyridine (Compound No. 2-598),
2-{4-[4-fluoro-3-(1-methyl-1H-pyrazol-4-yl)phenyl]-1H-pyrazol-3-yl}-6-methylpyridine (Compound No. 2-600),
2-{4-[4-fluoro-3-(1H-imidazol-4-yl)phenyl]-1H-pyrazol-3-yl}-6-methylpyridine (Compound No. 2-608),
(4-{2-fluoro-5-[3-(6-methylpyridin-2-yl)-1H-pyrazol-4-yl]phenyl}-1H-imidazol-2-yl)methanol (Compound No. 2-616),
2-{4-[4-fluoro-3-(1-methyl-1H-pyrazol-4-yl)phenyl]-1H-pyrazol-3-yl}pyridine (Compound No. 2-622),
5-{2-fluoro-5-[3-(6-methylpyridin-2-yl)-1H-pyrazol-4-yl]phenyl}thiophene-2-sulfonamide (Compound No. 2-628),
2'-fluoro-N,N-dimethyl-5'-[3-(6-methylpyridin-2-yl)-1H-pyrazol-4-yl]-1,1'-biphenyl-4-sulfonamide (Compound No. 2-644),
(6-12-fluoro-5-[3-(6-methylpyridin-2-yl)-1H-pyrazol-4-yl]phenyl}pyridin-3-yl)methanol (Compound No. 2-646),
2-(6-12-fluoro-5-[3-(6-methylpyridin-2-yl)-1H-pyrazol-4-yl]phenyl}pyridin-3-yl)propan-2-ol (Compound No. 2-650),
2-(4-(2-fluoro-5-[3-(6-methylpyridin-2-yl)-1H-pyrazol-4-yl]phenyl)-1H-imidazol-2-yl)propan-2-ol (Compound No. 2-652),
2-(4-{2-fluoro-5-[3-(6-methylpyridin-2-yl)-1H-pyrazol-4-yl]phenyl}-1H-imidazol-2-yl)ethanol (Compound No. 2-656),
2-(4-{2-fluoro-5-[3-(6-methylpyridin-2-yl)-1H-pyrazol-4-yl]phenyl}-1H-pyrazol-1-yl)ethanol (Compound No. 2-662),
4-(6-{2-fluoro-5-[3-(6-methylpyridin-2-yl)-1H-pyrazol-4-yl]phenyl}pyridin-3-yl)morpholine (Compound No. 2-690),
2-{4-[4-fluoro-3-(1-methyl-1H-imidazol-4-yl)phenyl]-1H-pyrazol-3-yl}-6-methylpyridine (Compound No. 2-698),
2-{4-[3-(1,1-dioxido-2,3-dihydro-1-benzothien-5-yl)-4-fluorophenyl]-1H-pyrazol-3-yl}-6-methylpyridine (Compound No. 2-724), and
5-[4-fluoro-3-(1-methyl-1H-pyrazol-4-yl)phenyl]-4-(6-methylpyridin-2-yl)-1,3-thiazole-2-amine (Compound No. 3-126); and
particularly preferred compounds are
2-{4-[4-fluoro-3-(1H-pyrrol-3-yl)phenyl]-2-isopropyl-1H-imidazol-5-yl}-6-methylpyridine (Compound No. 1-683),
2-{5-[4-fluoro-3-(1-methyl-1H-pyrazol-4-yl)phenyl]-2-isopropyl-1H-imidazol-4-yl}-6-methylpyridine (Compound No. 1-725),
2-{4-[4-fluoro-3-(1H-imidazol-4-yl)phenyl]-2-isopropyl-1H-imidazol-5-yl}-6-methylpyridine (Compound No. 1-769),
2-{4-[4-fluoro-3-(1-methyl-1H-imidazol-4-yl)phenyl]-2-isopropyl-1H-imidazol-5-yl}-6-methylpyridine (Compound No. 1-775),
2-{4-[6-fluoro-4'-(methylsulfonyl)-1,1'-biphenyl-3-yl]-1H-pyrazol-3-yl}-6-methylpyridine (Compound No. 2-412),
2'-fluoro-5'-[3-(6-methylpyridin-2-yl)-1H-pyrazol-4-yl]-1,1'-biphenyl-4-sulfonamide (Compound No. 2-444),
2'-fluoro-N-methyl-5'-[3-(6-methylpyridin-2-yl)-1H-pyrazol-4-yl]-1,1'-biphenyl-4-sulfonamide (Compound No. 2-518),
2-{4-[4-fluoro-3-(1-methyl-1H-pyrazol-4-yl)phenyl]-1H-pyrazol-3-yl}-6-methylpyridine (Compound No. 2-600),
(4 {2-fluoro-5-[3-(6-methylpyridin-2-yl)-1H-pyrazol-4-yl]phenyl}-1H-imidazol-2-yl)methanol (Compound No. 2-616),
5-{2-fluoro-5-[3-(6-methylpyridin-2-yl)-1H-pyrazol-4-yl]phenyl}thiophene-2-sulfonamide (Compound No. 2-628),
2-(6-{2-fluoro-5-[3-(6-methylpyridin-2-yl)-1H-pyrazol-4-yl]phenyl}pyridin-3-yl)propan-2-ol (Compound No. 2-650),
2-(4-{2-fluoro-5-[3-(6-methylpyridin-2-yl)-1H-pyrazol-4-yl]phenyl}-1H-imidazol-2-yl)propan-2-ol (Compound No. 2-652),
2-(4-{2-fluoro-5-[3-(6-methylpyridin-2-yl)-1H-pyrazol-4-yl]phenyl}-1H-pyrazol-1-yl)ethanol (Compound No. 2-662),
2-{4-[4-fluoro-3-(1-methyl-1H-imidazol-4-yl)phenyl]-1H-pyrazol-3-yl}-6-methylpyridine (Compound No. 2-698), and
2-(4-[3-(1,1-dioxido-2,3-dihydro-1-benzothien-5-yl)-4-fluorophenyl]-1H-pyrazol-3-yl}-6-methylpyridine (Compound No. 2-724).

### Effect of the Invention

The biaryl derivatives having the above General Formula (I) or the pharmacologically acceptable salt thereof according to the present invention function as non-peptide inhibitors that strongly and selectively inhibits collagen synthesis. Therefore, they are useful in preventing and/or treating morbid conditions (for example, renal disease, liver fibrosis, lung fibrosis, or skin fibrosis mainly caused by fibrosis) that are mainly caused by fibrosis (for example, chronic renal disease, acute renal disease, diabetic renal disorder, liver fibrosis, lung fibrosis, or skin fibrosis).

### Best Mode for Carrying Out the Invention

The compound having the General Formula (I) according to the present invention can be manufactured according to methods described below.

Method A is a process for manufacturing a compound having the General Formula (I-A).

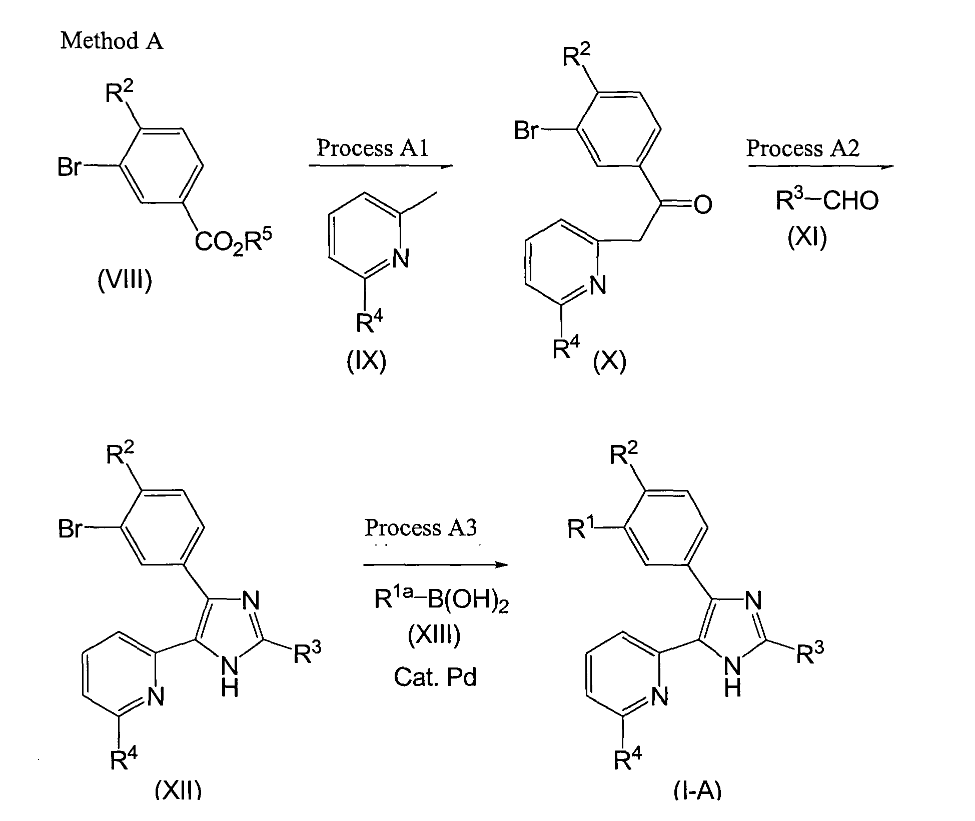

In the present method, R¹, R², R³, and R⁴ are the same as defined above. R^{1a} is the same group as R¹ except that the amino group, the hydroxyl group and/or the carboxyl group contained in R¹ as a substituent is an amino, hydroxyl and/or carboxyl group which may be protected. R⁵ is a C₁-C₂ alkyl group (preferably a methyl group).

### Process A1

In this process, a compound having the General Formula (X) is produced.

In this process, a compound having the General Formula (VIII) reacts with a compound having the General Formula (IX) in an inert solvent in the presence of an organometallic reagent. Each of the compounds having the General Formula (VIII) and (IX) is a known compound or is readily prepared by a known method using a known compound as a starting raw material.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile and isobutyronitrile; nitro compounds such as nitroethane and nitrobenzene; aromatic hydrocarbons such as benzene, toluene, and xylene; and solvent mixtures thereof, preferably ethers, more preferably tetrahydrofuran.

Examples of the organometallic reagent used in this process include butyllithium, sodium hexamethyldisilazane, potassium hexamethyldisilazide, lithium hexamethyldisilazide, lithium diisopropylamide, ethyl magnesium bromide, and isopropyl magnesium bromide, preferably alkali metal hexamethyldisilazane salts, more preferably sodium hexamethyldisilazane.

The reaction temperature of this process depends on the raw material compounds, used inert solvent, and so on, but is usually -78°C to 60°C, and preferably -30°C to 30°C.

The reaction time of this process depends on the raw material compounds, used inert solvent, reaction temperature, and so on, but is usually 0.5 to 48 hr, and preferably 1 to 3 hr.

### Process A2

This process is a process for manufacturing a compound having the General Formula (XII), and includes the following steps (i) to (iii).

In step (i), a compound having the General Formula (X) reacts with sodium nitrite or t-butyl nitrite in an inert solvent in the presence of an acid.

The inert solvent used in this step is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; solvent mixtures thereof; and water, preferably ethers, alcohols, or water, more preferably tetrahydrofuran or water.

Examples of the acid used in this step include hydrogen halides such as a hydrogen chloride gas and hydrogen bromide gas; mineral acids such as sulfuric acid, hydrobromic acid, and hydrochloric acid; organic sulfonic acids such as methanesulfonic acid, p-toluenesulfonic acid, camphorsulfonic acid, and trifluoromethanesulfonic acid; carbonic acids such as acetic acid, formic acid, and trifluoroacetic acid; Lewis acids such as zinc chloride, tin tetrachloride, boron trifluoride, and boron tribromide; or acidic ion-exchange resins, preferably mineral acids, more preferably hydrochloric acid, further more preferably concentrated hydrochloric acid.

The reaction temperature of this step depends on the raw material compounds, used inert solvent, and so on, but is usually -30°C to 100°C, and preferably 0°C to 40°C.

The reaction time of this step depends on the raw material compounds, used inert solvent, reaction temperature, and so on, but is usually 0.5 to 48 hr, and preferably 1 to 24 hr.

In step (ii), the compound obtained in step (i) reacts with a compound having the General Formula (XI) in a carboxylic acid in the presence of ammonium acetate.

Examples of the carboxylic acid used in this step include acetic acid, formic acid, propionic acid, butyric acid, and trifluoroacetic acid, preferably acetic acid.

The reaction temperature of this step depends on the raw material compounds, used carboxylic acid, and so on, but is usually 20°C to 150°C, and preferably 80°C to 120°C.

The reaction time of this step depends on the raw material compounds, used carboxylic acid, reaction temperature, and so on, but is usually 1 to 48 hr, and preferably 2 to 24 hr.

In step (iii), the compound obtained in step (ii) reacts with a reducing agent in an inert solvent.

The inert solvent used in this step is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably alcohols, more preferably methanol.

Examples of the reducing agent used in this step include metal salts such as titanium trichloride; and phosphorus compounds such as phosphorus trichloride and triethylphosphite, preferably metal salts, more preferably 10% titanium trichloride (20 to 30% hydrochloric acid aqueous solution).

The reaction temperature of this step depends on the raw material compounds, used inert solvent, and so on, but is usually -30°C to 100°C, and preferably 0°C to 40°C.

The reaction time of this step depends on the raw material compounds, used inert solvent, reaction temperature, and so on, but is usually 1 to 48 hr, and preferably 2 to 24 hr.

### Process A3

In this process, a compound having the General Formula (I-A) is produced.

In this process, a compound having the General Formula (XII) reacts with a compound having the General Formula (XIII) in an inert solvent in the presence of a palladium catalyst and an inorganic base (for example, "Organometallics in Synthesis, A manual"; Wiley and Sons Ltd.: England; 2002), and then, when necessary, the protecting group of the amino group, hydroxy group, and/or carboxyl group on R^{1a} is removed. The compound having the General Formula (XIII) is a known compound or is readily prepared by a known method using a known compound as a starting raw material.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably alcohols or ethers, more preferably ethers, further more preferably 1,2-dimethoxyethane.

The palladium catalyst used in this process is, for example, a divalent palladium catalyst or zerovalent palladium catalyst, preferably palladium/active carbon, palladium(II) acetate, palladium(II) trifluoroacetate, palladium black, palladium(II) bromide, palladium(II) chloride, palladium(II) iodide, palladium(II) cyanide, palladium(II) nitrate, palladium(II) oxide, palladium(II) sulfate, dichlorobis(acetonitrile)palladium(II), dichlorobis(benzonitrile)palladium(II), dichloro(1,5-cyclooctadiene)palladium(II), acetylacetone palladium(II), palladium(II) sulfide, dichloro[1,1'-bis(diphenylphosphino)ferrocene]palladium(II), tris(dibenzylideneacetone)dipalladium(0), tetrakis(triphenylphosphine)palladium(0), tetrafluoroborate, or palladium allyl chloride dimer, more preferably dichloro[1,1'-bis(diphenylphosphino)ferrocene]palladium(II) or tetrakis(triphenylphosphine)palladium(0), and further more preferably dichloro[1,1'-bis(diphenylphosphino)ferrocene]palladium(II)-dichloromethane complex or tetrakis(triphenylphosphine)palladium(0).

Examples of the inorganic base used in this process include alkali metal hydrides such as lithium hydride, sodium hydride, and potassium hydride; alkali metal carbonates such as sodium carbonate, potassium carbonate, and lithium carbonate; and alkali metal phosphates such as lithium phosphate, sodium phosphate, and potassium phosphate, preferably alkali metal phosphates or alkali metal carbonates, more preferably potassium phosphate or sodium carbonate, further more preferably potassium phosphate hydrate or a 2 N sodium carbonate aqueous solution.

The reaction temperature of this process depends on the raw material compounds, used inert solvent, and so on, but is usually 20°C to 150°C, and preferably 80°C to 120°C.

The reaction time of this process depends on the raw material compounds, used inert solvent, reaction temperature, and so on, but is usually 1 to 72 hr, and preferably 2 to 24 hr.

Method B is a different method from Method A for manufacturing a compound having the General Formula (I-A).

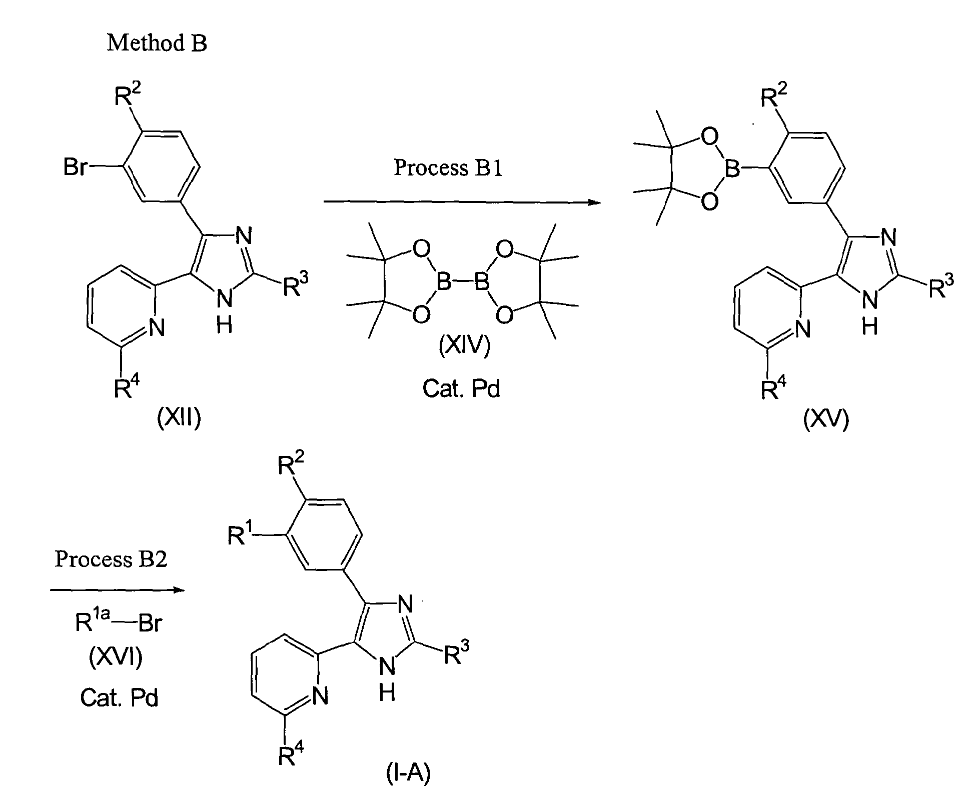

In the present method, R¹ , R², R³, R⁴, and R^{1a} are the same as defined above.

### Process B1

In this process, a compound having the General Formula (XV) is produced.

In this process, a compound having the General Formula (XII) produced in the Process A2 of the above-described Method A reacts with bis(pinacolato)diboron (XIV) in an inert solvent in the presence of a palladium catalyst and an inorganic base (for example, Org. Lett. 20(2), 3201-3204, 2000).

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably sulfoxides or amides, more preferably N,N-dimethylformamide.

The palladium catalyst used in this process is, for example, a divalent palladium catalyst or zerovalent palladium catalyst, preferably palladium/active carbon, palladium(II) acetate, palladium(II) trifluoroacetate, palladium black, palladium(II) bromide, palladium(II) chloride, palladium(II) iodide, palladium(II) cyanide, palladium(II) nitrate, palladium(II) oxide, palladium(II) sulfate, dichlorobis(acetonitrile)palladium(II), dichlorobis(benzonitrile)palladium(II), dichloro(1,5-cyclooctadiene)palladium(II), acetylacetone palladium(II), palladium(II) sulfide, dichloro[1,1'-bis(diphenylphosphino)ferrocene]palladium(II), tris(dibenzylideneacetone)dipalladium(0), tetrakis(triphenylphosphine)palladium(0), tetrafluoroborate, or palladium allyl chloride dimer, more preferably dichloro[1,1'-bis(diphenylphosphino)ferrocene]palladium(II), and further more preferably a dichloro[1,1'-bis(diphenylphosphino)ferrocene]palladium(II)-dichloromethane complex.

Examples of the inorganic base used in this process include alkali metal hydrides such as lithium hydride, sodium hydride, and potassium hydride; alkali metal carbonates such as sodium carbonate, potassium carbonate, and lithium carbonate; alkali metal acetates such as sodium acetate, potassium acetate, and lithium acetate; and alkali metal phosphates such as lithium phosphate, sodium phosphate, and potassium phosphate, preferably an alkali metal acetates, more preferably potassium acetate.

The reaction temperature of this process depends on the raw material compounds, used inert solvent, and so on, but is usually 20°C to 150°C, and preferably 60°C to 120°C.

The reaction time of this process depends on the raw material compounds, used inert solvent, reaction temperature, and so on, but is usually 1 to 72 hr, and preferably 2 to 24 hr.

### Process B2

In this process, a compound having the General Formula (I-A) is produced.

In this process, a compound having the General Formula (XV) reacts with a compound having the General Formula (XVI) in an inert solvent in the presence of a palladium catalyst and an inorganic base as in the Process A3 of the above-described Method A, and then, when necessary, the protecting group of the amino group, hydroxy group, and/or carboxyl group on R^{1a} is removed. The compound having the General Formula (XVI) is a known compound or is readily prepared by a known method using a known compound as a starting raw material.

Method C is a process for converting a substituent on a C₆-C₁₀ aryl group which is substituted and a heterocyclic group which may be substituted in R¹ of the compound having the General Formula (I-A).

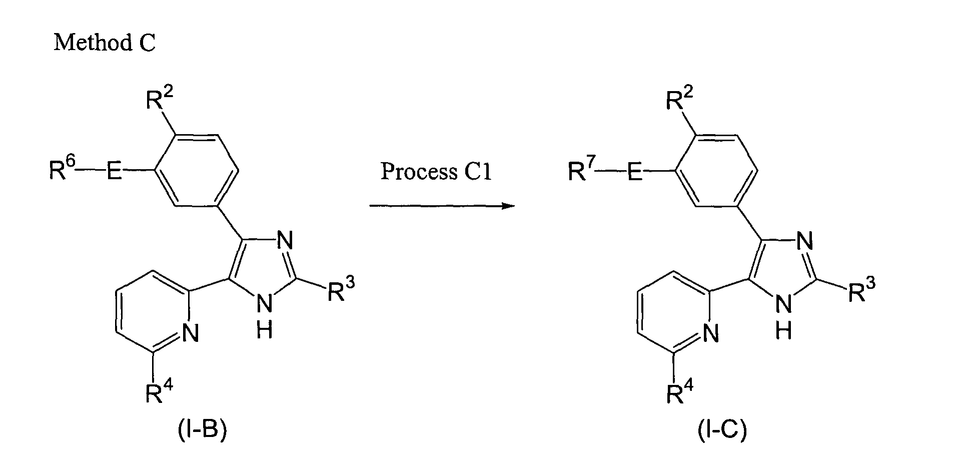

In the present method, R², R³, and R⁴ are the same as defined above, and E represents a C₆-C₁₀ aryl group or a heterocyclic group.

### Process C1

In this process, a substituent R⁶ of a compound having the General Formula (I-B) is converted to a substituent R⁷ of a compound having the General Formula (I-C).

### (a) A case in which R⁶ is a cyano group and R⁷ is a carbamoyl group

In this process, a compound having the General Formula (I-B) reacts with a base in an inert solvent (for example, Tetrahedron Lett. 41, 3747, 2000).

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; esters such as ethyl formate, ethyl acetate, propyl acetate, butyl acetate, and diethyl carbonate; and aromatic hydrocarbons such as benzene, toluene, and xylene, preferably ethers, more preferably dioxane.

Examples of the base used in this process include inorganic bases like alkali metal carbonates such as sodium carbonate, potassium carbonate, and lithium carbonate; alkali metal bicarbonates such as sodium bicarbonate, potassium bicarbonate, and lithium bicarbonate; alkali metal hydrides such as lithium hydride, sodium hydride, and potassium hydride; alkali metal hydroxides such as sodium hydroxide, potassium hydroxide, barium hydroxide, and lithium hydroxide; alkali metal fluorides such as sodium fluoride and potassium fluoride; alkali metal alkoxides such as sodium methoxide, sodium ethoxide, sodium-t-butoxide, potassium methoxide, potassium ethoxide, potassium-t-butoxide, and lithium methoxide; alkali metal trialkylsiloxides such as sodium trimethylsiloxide, potassium trimethylsiloxide, and lithium trimethylsiloxide; alkali metal mercaptans such as sodium methyl mercaptan and sodium ethyl mercaptan; organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]nona-5-ene (DBN), 1,4-diazabicyclo[2.2.2]octane (DABCO), and 1,8-diazabicyclo[5.4.0]undeca-7-ene (DBU); and organometallic bases such as butyllithium, lithium diisopropylamide, and lithium bis(trimethylsilyl)amide, preferably alkali metal trialkylsiloxides, more preferably potassium trimethylsiloxide.

The reaction temperature of this process depends on the raw material compounds, used inert solvent, and so on, but is usually 0°C to 150°C, and preferably 20°C to 120°C.

The reaction time of this process depends on the raw material compounds, used inert solvent, reaction temperature, and so on, but is usually 0.5 to 48 hr, and preferably 1 to 24 hr.

### (b) A case in which R⁶ is represented by formula CO₂R^{6b} (R^{6b} is a C₁-C₆ alkyl group) and R⁷ is a carboxyl group

In this process, a compound having the General Formula (I-B) reacts with a base in an inert solvent.

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile; aromatic hydrocarbons such as benzene, toluene, and xylene; and water, preferably alcohols, ethers, or water; more preferably ethanol, tetrahydrofuran, or water. When necessary, the solvent may be a solvent mixture (mixture ratio is 1 : 100 to 100 : 1).

Examples of the base used in this process include alkali metal carbonates such as sodium carbonate, potassium carbonate, and lithium carbonate; alkali metal bicarbonates such as sodium bicarbonate, potassium bicarbonate, and lithium bicarbonate; and alkali metal hydroxides such as sodium hydroxide, potassium hydroxide, barium hydroxide, and lithium hydroxide, preferably alkali metal hydroxides, more preferably sodium hydroxide. When necessary, the base may be used as an aqueous solution (for example, the content is 1% to 50%, and preferably 3% to 20%).

The reaction temperature of this process depends on the raw material compounds, used inert solvent, and so on, but is usually 0°C to 130°C, and preferably 20°C to 100°C.

The reaction time of this process depends on the raw material compounds, used inert solvent, and so on, but is usually 0.5 to 12 hr, and preferably 1 to 5 hr.

### (c) A case in which R⁶ is a carboxyl group and R⁷ is a chlorocarbonyl group

In this process, a compound having the General Formula (I-B) reacts with a chlorinating agent in an inert solvent.

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile; esters such as ethyl formate, ethyl acetate, propyl acetate, butyl acetate, and diethyl carbonate; halogenated hydrocarbons such as methylene chloride, 1,2-dichloroethane, and dichlorobenzene; and aromatic hydrocarbons such as benzene, toluene, and xylene, preferably aromatic hydrocarbons, more preferably benzene or toluene.

Examples of the chlorinating agent used in this process include inorganic acids such as hydrochloric acid; halogen molecules such as chlorine; phosphorus reagents such as phosphorus trichloride, phosphorus pentachloride, and phosphorus oxychloride; carboxylic chlorides such as oxalyl chloride; sulfinic acid reagents such as thionyl chloride and toluenesulfonyl chloride; and sulfonic acid reagents such as sulfonyl chloride, preferably a sulfinic acid reagents or carboxylic chlorides, more preferably thionyl chloride or oxalic chloride, further more preferably thionyl chloride.

The reaction temperature of this process depends on the raw material compounds, used inert solvent, and so on, but is usually 0°C to 130°C, and preferably 20°C to 100°C.

The reaction time of this process depends on the raw material compounds, used inert solvent, reaction temperature, and so on, but is usually 0.5 to 12 hr, and preferably 1 to 5 hr.

### (d) A case in which R⁶ is a chlorocarbonyl group and R⁷ is a hydroxyamino group, a substituent represented by formula CONHR (R is the same as defined above), or a substituent represented by formula CONR^{6d}R^{7d} (R^{6d} and R^{7d}, together with the nitrogen atom linking them, form a heterocyclic group which may be substituted with one group selected from substituent group a)

In this process, a compound having the General Formula (I-B) reacts with an aminating agent in an inert solvent in the presence of a base.

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile; esters such as ethyl formate, ethyl acetate, propyl acetate, butyl acetate, and diethyl carbonate; halogenated hydrocarbons such as methylene chloride, 1,2-dichloroethane, and dichlorobenzene; and aromatic hydrocarbons such as benzene, toluene, and xylene, preferably halogenated hydrocarbons, more preferably methylene chloride.

Examples of the base used in this process include alkali metal carbonates such as sodium carbonate, potassium carbonate, and lithium carbonate; alkali metal bicarbonates such as sodium bicarbonate, potassium bicarbonate, and lithium bicarbonate; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]nona-5-ene (DBN), 1,4-diazabicyclo[2.2.2]octane (DABCO), and 1,8-diazabicyclo[5.4.0]undeca-7-ene (DBU), preferably organic bases, more preferably triethylamine or pyridine.

The aminating agent used in this process is an amino compound, which is a known compound or is readily prepared by a known method using a known compound as a starting raw material. The aminating agent is represented by, for example, general formula HNHR or HNR^{6d}R^{7d}, and is preferably N-aminomorpholine, 1-amino-4-methylpiperazine, 1-methylpiperazine, morpholine, or ethanolamine.

The reaction temperature of this process depends on the raw material compounds, used inert solvent, and so on, but is usually 0°C to 130°C, and preferably 20°C to 80°C.

The reaction time of this process depends on the raw material compounds, used inert solvent, reaction temperature, and so on, but is usually 0.1 to 12 hr, and preferably 0.5 to 5 hr.

### (e) A case in which R⁶ is a nitro group and R⁷ is an amino group

In this process, a compound having the General Formula (I-B) reacts in an inert solvent in the presence of a palladium catalyst under a hydrogen atmosphere.

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile; esters such as ethyl formate, ethyl acetate, propyl acetate, butyl acetate, and diethyl carbonate; aromatic hydrocarbons such as benzene, toluene, and xylene; and solvent mixtures thereof, preferably alcohols, more preferably methanol.

The palladium catalyst used in this process is, for example, a divalent palladium catalyst or zerovalent palladium catalyst, preferably palladium/active carbon, palladium(II) acetate, palladium(II) trifluoroacetate, palladium black, palladium(II) bromide, palladium(II) chloride, palladium(II) iodide, palladium(II) cyanide, palladium(II) nitrate, palladium(II) oxide, palladium(II) sulfate, dichlorobis(acetonitrile)palladium(II), dichlorobis(benzonitrile)palladium(II), dichloro(1,5-cyclooctadiene)palladium(II), acetylacetone palladium(II), palladium(II) sulfide, tris(dibenzylideneacetone)dipalladium(0), tetrakis(triphenylphosphine)palladium(0) tetrafluoroborate, or palladium allyl chloride dimer; and more preferably palladium/active carbon.

The reaction temperature of this process depends on the raw material compounds, used inert solvent, and so on, but is usually 0°C to 130°C, and preferably 10°C to 60°C.

The reaction time of this process depends on the raw material compounds, used inert solvent, reaction temperature, and so on, but is usually 0.5 to 24 hr, and preferably 1 to 12 hr.

### (f) A case in which R⁶ is an amino group and R⁷ is a substituent represented by formula NHCOR (R is the same as defined above)

In this process, a compound having the General Formula (I-B) reacts with a carbonylating agent in an inert solvent in the presence of a base.

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile; esters such as ethyl formate, ethyl acetate, propyl acetate, butyl acetate, and diethyl carbonate; halogenated hydrocarbons such as methylene chloride, 1,2-dichloroethane, and dichlorobenzene; and aromatic hydrocarbons such as benzene, toluene, and xylene, preferably halogenated hydrocarbons, more preferably methylene chloride.

Examples of the base used in this process include alkali metal carbonates such as sodium carbonate, potassium carbonate, and lithium carbonate; alkali metal bicarbonates such as sodium bicarbonate, potassium bicarbonate, and lithium bicarbonate; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]nona-5-ene (DBN), 1,4-diazabicyclo[2.2.2]octane (DABCO), and 1,8-diazabicyclo[5.4.0]undeca-7-ene (DBU), preferably organic bases, and more preferably triethylamine or pyridine.

The carbonylating agent used in this process is a carbonyl compound, which is a known compound or is readily prepared by a known method using a known compound as a starting raw material. The carbonylating agent is represented by, for example, general formula RCOCI or (RCO)₂O, and is preferably acetic anhydride, acetyl chloride, or 2-acetoxybutyryl chloride.

The reaction temperature of this process depends on the raw material compounds, used inert solvent, and so on, but is usually -30°C to 80°C, and preferably 0°C to 60°C.

The reaction time of this process depends on the raw material compounds, used inert solvent, reaction temperature, and so on, but is usually 0.1 to 24 hr, and preferably 0.5 to 12 hr.

### (g) A case in which R⁶ is an amino group and R⁷ is a formylamino group

This process is carried out according to a known method (for example, a method disclosed in J. Med. Chem. 46(2), 237-243, 2003).

### (h) A case in which R⁶ is an amino group and R⁷ is a substituent represented by formula NHSO₂R (R is the same as defined above)

In this process, a compound having the General Formula (I-B) reacts with a sulfonylating agent in an inert solvent in the presence of a base.

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile; esters such as ethyl formate, ethyl acetate, propyl acetate, butyl acetate, and diethyl carbonate; halogenated hydrocarbons such as methylene chloride, 1,2-dichloroethane, and dichlorobenzene; and aromatic hydrocarbons such as benzene, toluene, and xylene, preferably halogenated hydrocarbons, more preferably methylene chloride.

Examples of the base used in this process include alkali metal carbonates such as sodium carbonate, potassium carbonate, and lithium carbonate; alkali metal bicarbonates such as sodium bicarbonate, potassium bicarbonate, and lithium bicarbonate; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]nona-5-ene (DBN), 1,4-diazabicyclo[2.2.2]octane (DABCO), and 1,8-diazabicyclo[5.4.0]undeca-7-ene (DBU), preferably organic bases, more preferably triethylamine or pyridine.

The sulfonylating agent used in this process is a sulfonyl compound, which is a known compound or is readily prepared by a known method using a known compound as a starting raw material. The sulfonylating agent is represented by, for example, general formula RSO₂Cl or (RSO₂)₂O, and is preferably methanesulfonyl anhydride, methanesulfonyl chloride, ethanesulfonyl chloride, or cyclopropylsulfonyl chloride.

The reaction temperature of this process depends on the raw material compounds, used inert solvent, and so on, but is usually -30°C to 80°C, and preferably 0°C to 60°C.

The reaction time of this process depends on the raw material compounds, used inert solvent, reaction temperature, and so on, but is usually 0.1 to 24 hr, and preferably 0.5 to 12 hr.

### (i) A case in which R⁶ is an amino group and R⁷ is an aminosulfonylamino group

This process is carried out according to a known method (for example, a method disclosed in J. Chem. Soc. Perkin Trans 2, 1851-1854, 1984).

### (j) A case in which R⁶ is a (4-thiomorpholino)carbonyl group and R⁷ is a (1-oxido-4-thiomorpholino)carbonyl group or a (1,1-dioxido-4-thiomorpholino)carbonyl group

In this process, a compound having the General Formula (I-B) reacts with an oxidizing agent in an inert solvent according to a known method (for example, M. Hudlicky, in "Oxidations in organic chemistry", American Chemical Society, Washington, DC, 1990).

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; nitriles such as acetonitrile; esters such as ethyl formate, ethyl acetate, propyl acetate, butyl acetate, and diethyl carbonate; halogenated hydrocarbons such as methylene chloride, 1,2-dichloroethane, and dichlorobenzene; and aromatic hydrocarbons such as benzene, toluene, and xylene, preferably halogenated hydrocarbons, more preferably methylene chloride.

The oxidizing agent used in this process is, for example, an organic peroxide such as m-chloroperbenzoic acid; or an inorganic oxidizing agent such as a hydrogen peroxide solution, metal periodate, or Oxone. The oxidizing agent is preferably m-chloroperbenzoic acid.

The reaction temperature of this process depends on the raw material compounds, used inert solvent, and so on, but is usually -30°C to 100°C, and preferably 0°C to 40°C.

The reaction time of this process depends on the raw material compounds, used inert solvent, reaction temperature, and so on, but is usually 0.5 to 48 hr, and preferably 1 to 24 hr.

### (k) A case in which R⁶ is a substituent represented by formula SR and R⁷ is a substituent represented by formula SOR or a substituent represented by formula SO₂R (R is the same as defined above)

In this process, a compound having the General Formula (I-B) reacts with an oxidizing agent in an inert solvent.

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; nitriles such as acetonitrile; esters such as ethyl formate, ethyl acetate, propyl acetate, butyl acetate, and diethyl carbonate; halogenated hydrocarbons such as methylene chloride, 1,2-dichloroethane, and dichlorobenzene; and aromatic hydrocarbons such as benzene, toluene, and xylene, preferably ethers or halogenated hydrocarbons, more preferably dioxane or methylene chloride.

The oxidizing agent used in this process is, for example, an organic peroxide such as m-chloroperbenzoic acid; or an inorganic oxidizing agent such as a hydrogen peroxide solution, metal periodate, or Oxone. The oxidizing agent is preferably an organic peroxide or metal periodate, and more preferably m-chloroperbenzoic acid or sodium periodate.

The reaction temperature of this process depends on the raw material compounds, used inert solvent, and so on, but is usually -30°C to 150°C, and preferably 20°C to 120°C.

The reaction time of this process depends on the raw material compounds, used inert solvent, reaction temperature, and so on, but is usually 0.5 to 48 hr, and preferably 1 to 24 hr.

Method D is a process for manufacturing a compound having the General Formula (I-C).

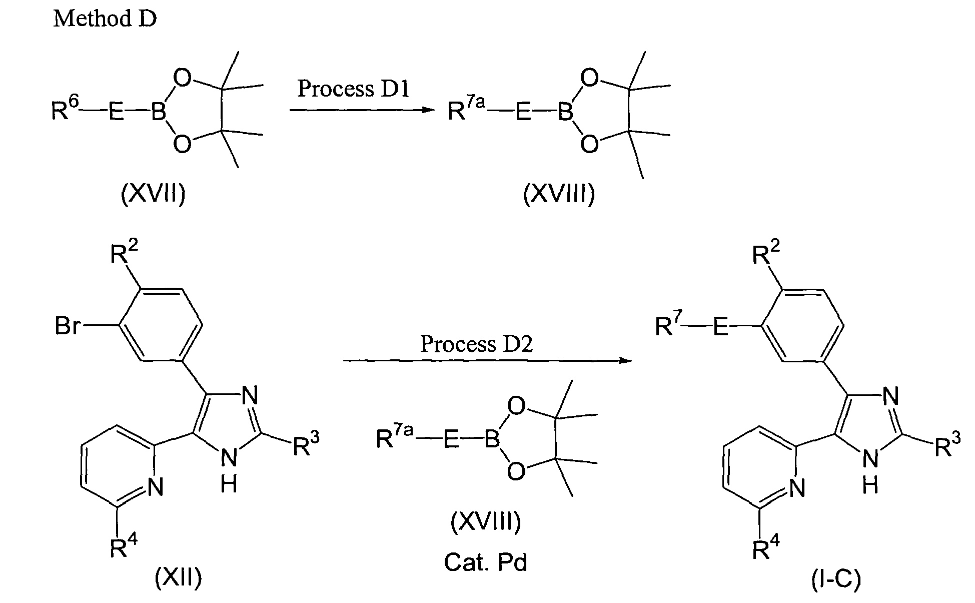

In the present method, R², R³, R⁴, R⁶, R⁷, and E are the same as defined above. R^{7a} is the same group as R⁷ except that the amino group, the hydroxyl group and/or the carboxyl group contained in k⁷ as a substituent is an amino, hydroxyl and/or carboxyl group which may be protected.

### Process D1

In this process, a compound having the General Formula (XVIII) is produced.

In this process, the substituent R⁶ of a compound having the General Formula (XVII) is converted into the substituent R^{7a} of a compound having the General Formula (XVIII). The compound having the General Formula (XVII) is a known compound or is readily prepared by a known method using a known compound as a starting raw material. The process is carried out as in the Process C1 of the above-described Method C, and then, when necessary, the amino group, hydroxy group, and/or carboxyl group on R⁷ is protected.

### Process D2

In this process, a compound having the General Formula (I-C) is produced.

In this process, a compound having the General Formula (XII) produced in the Process A2 of the above-described Method A reacts with a compound having the General Formula (XVIII) as in the Process A3 of the above-described Method A in an inert solvent in the presence of a palladium catalyst and an inorganic base. Then, when necessary, the protecting group of the amino group, hydroxy group, and/or carboxyl group on R^{7a} is removed.

Method E is a process for manufacturing a compound having the General Formula (I-C), apart from the above-described Method D.

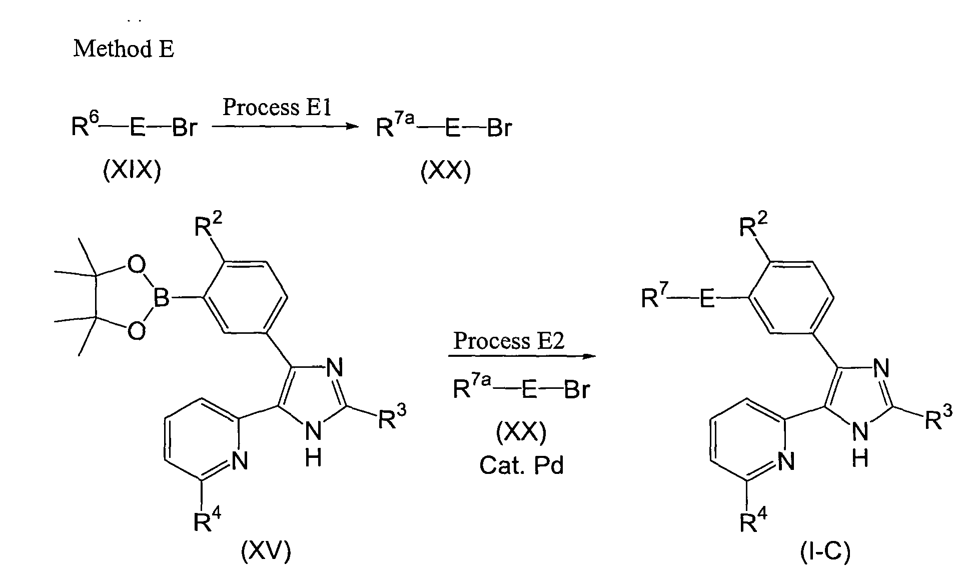

In the present method, R², R³, R⁴, R⁶, R⁷, R^{7a} and E are the same as defined above.

### Process E1

In this process, a compound having the General Formula (XX) is produced.

In this process, the substituent R⁶ of a compound having the General Formula (XIX) is converted to the substituent R7^{a} of a compound having the General Formula (XX). The process is conducted as in the Process C1 of the above-described Method C, and then, when necessary, the amino group, hydroxy group, and/or carboxyl group on R⁷ is protected. The compound having the General Formula (XIX) is a known compound or is readily prepared by a known method using a known compound as a starting raw material.

### Process E2

In this process, a compound having the General Formula (I-C) is produced.

In this process, a compound having the General Formula (XV) produced in the Process B1 of the above-described Method B reacts with a compound having the General Formula (XX) as in the Process A3 of the above-described Method A in an inert solvent in the presence of a palladium catalyst and an inorganic base. Then, when necessary, the protecting group of the amino group, hydroxy group, and/or carboxyl group on R^{7a} is removed.

Method F is a process for manufacturing a compound having the General Formula (I-D).

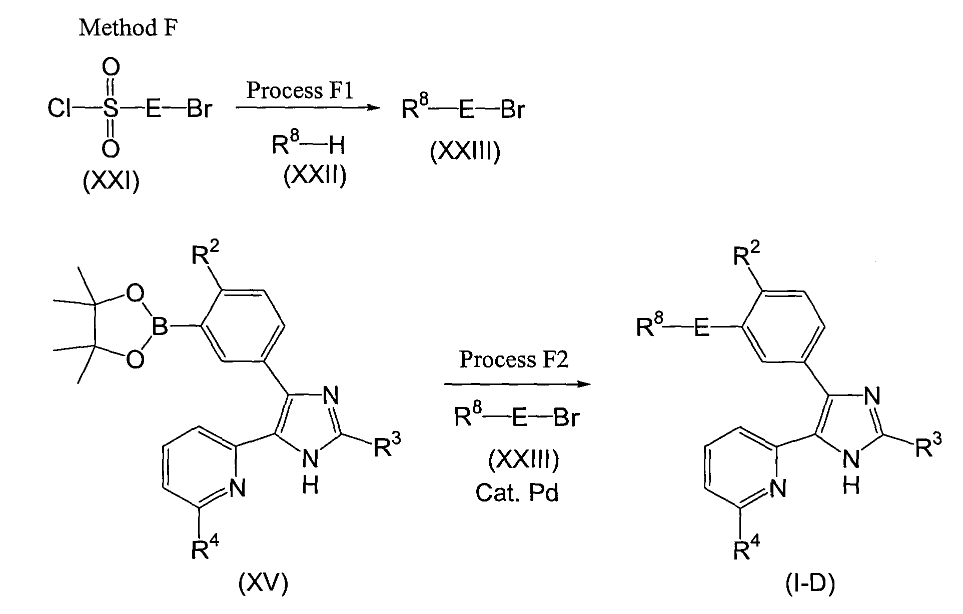

In the present method, R², R³, R⁴, and E are the same as defined above, and R⁸ is a substituent represented by formula SO₂NHR (where R is the same as defined above), formula SO₂NR^{6d}R^{7d} (where R^{6d} and R^{7d} are the same as defined above), or formula SO₂NR^{6f}R^{7f} (where R^{6f} and R^{7f} are the same or different and each is a C₁-C₆ alkyl group).

### Process F 1

In this process, a compound having the General Formula (XXIII) is produced.

In this process, a compound having the General Formula (XXI) reacts with a compound (amino compound) having the General Formula (XXII) in an inert solvent in the presence of a base. Each of the compounds having the General Formula (XXI) or (XXII) is a known compound or is readily prepared by a known method using a known compound as a starting raw material.

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile; esters such as ethyl formate, ethyl acetate, propyl acetate, butyl acetate, and diethyl carbonate; halogenated hydrocarbons such as methylene chloride, 1,2-dichloroethane, and dichlorobenzene; and aromatic hydrocarbons such as benzene, toluene, and xylene, preferably halogenated hydrocarbons, more preferably methylene chloride.

Examples of the base used in this process include alkali metal carbonates such as sodium carbonate, potassium carbonate, and lithium carbonate; alkali metal bicarbonates such as sodium bicarbonate, potassium bicarbonate, and lithium bicarbonate; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]nona-5-ene (DBN), 1,4-diazabicyclo[2.2.2]octane (DABCO), and 1,8-diazabicyclo[5.4.0]undeca-7-ene (DBU), preferably alkali metal bicarbonates or organic bases, more preferably sodium bicarbonate, triethylamine, or pyridine, further more preferably saturated aqueous sodium bicarbonate.

The amino compound used in this process is a known compound or is readily prepared by a known method using a known compound as a starting raw material. For example, the amino compound is represented by general formula HNHR, HNR^{6d}R^{7d}, or HNR^{6f}R^{7f}, and is preferably N-aminomorpholine, 1-amino-4-methylpiperazine, 1-methylpiperazine, morpholine, a 2 M dimethylamine solution (tetrahydrofuran solution), or a 40% methylamine aqueous solution.

The reaction temperature of this process depends on the raw material compounds, used inert solvent, and so on, but is usually -30°C to 100°C, and preferably 0°C to 30°C.

The reaction time of this process depends on the raw material compounds, used inert solvent, reaction temperature, and so on, but is usually 0.5 to 48 hr, and preferably 1 to 24 hr.

### Process F2

In this process, a compound having the General Formula (I-D) is produced.

In this process, a compound having the General Formula (XV) produced in the Process B1 of the above-described Method B reacts with a compound having the General Formula (XXIII) as in the Process A3 of the above-described Method A in an inert solvent in the presence of a palladium catalyst and an inorganic base.

Method G is a process for manufacturing a compound having the General Formula (I-E) and a compound having the General Formula (I-F).

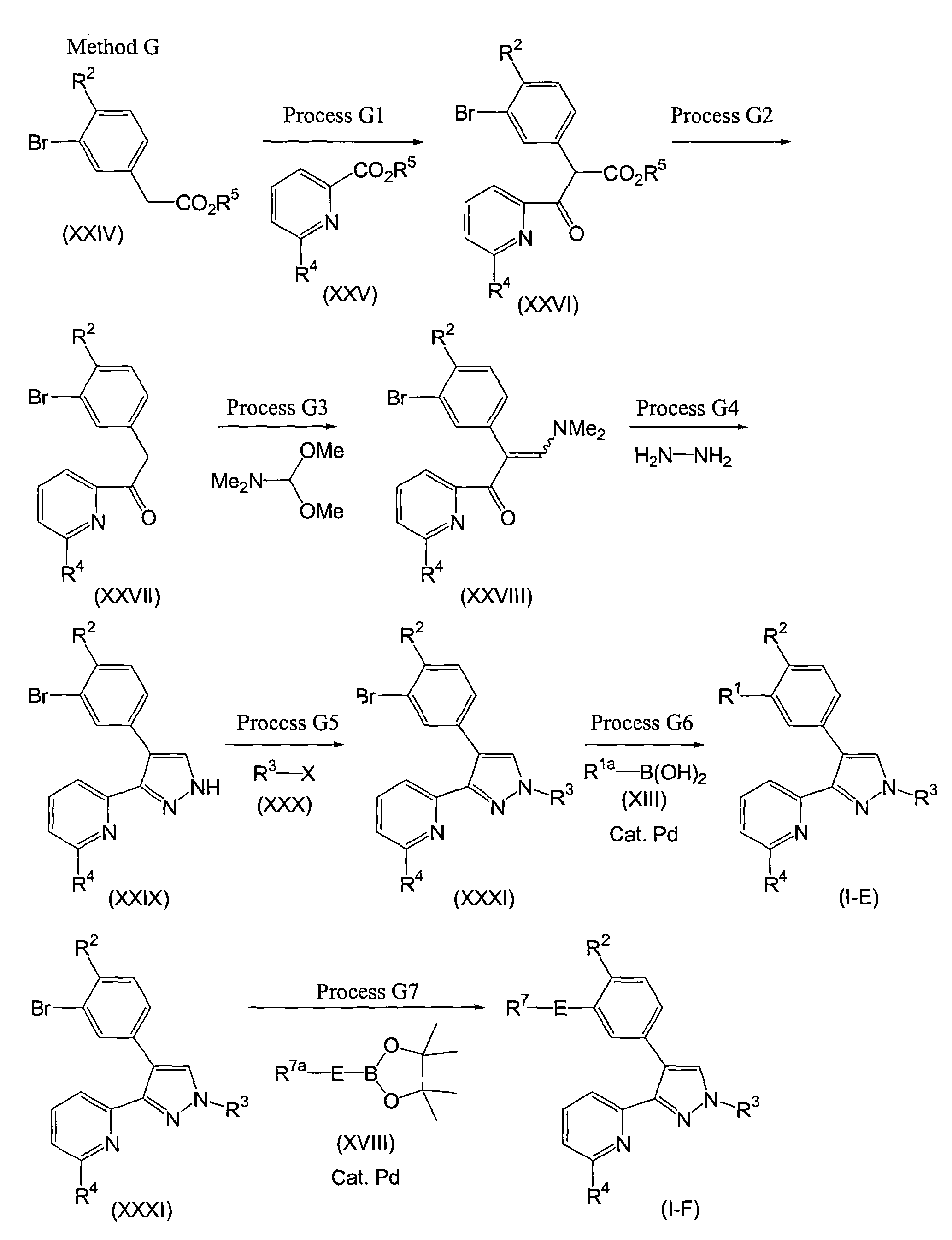

In the present method, R¹, R², R³, R⁴, R⁵, R⁷, R^{1a}, R^{7a}, and E are the same as defined above, and X is a halogen atom (for example, a chlorine atom, bromine atom, or iodine atom, and preferably an iodine atom).

### Process G1

In this process, a compound having the General Formula (XXVI) is produced.

In this process, a compound having the General Formula (XXIV) reacts with a compound having the General Formula (XXV) in an inert solvent in the presence of an organometallic reagent. Each of the compounds having the General Formula (XXIV) or (XXV) is a known compound or is readily prepared by a known method using a known compound as a starting raw material.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; nitro compounds such as nitroethane and nitrobenzene; aromatic hydrocarbons such as benzene, toluene, and xylene; and solvent mixtures thereof, preferably ethers, more preferably tetrahydrofuran.

Examples of the base used in this process include alkali metal carbonates such as sodium carbonate, potassium carbonate, and lithium carbonate; alkali metal bicarbonates such as sodium bicarbonate, potassium bicarbonate, and lithium bicarbonate; alkali metal hydrides such as lithium hydride, sodium hydride, and potassium hydride; alkali metal hydroxides such as sodium hydroxide, potassium hydroxide, barium hydroxide, and lithium hydroxide; alkali metal fluorides such as sodium fluoride and potassium fluoride; alkali metal alkoxides such as sodium methoxide, sodium ethoxide, sodium-t-butoxide, potassium methoxide, potassium ethoxide, potassium-t-butoxide, and lithium methoxide; alkali metal trialkylsiloxides such as sodium trimethylsiloxide, potassium trimethylsiloxide, and lithium trimethylsiloxide; alkali metal mercaptans such as sodium methyl mercaptan and sodium ethyl mercaptan; organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]nona-5-ene (DBN), 1,4-diazabicyclo[2.2.2]octane (DABCO), and 1,8-diazabicyclo[5.4.0]undeca-7-ene (DBU); and organometallic bases such as butyllithium, lithium diisopropylamide, and lithium bis(trimethylsilyl)amide, preferably alkali metal alkoxides, more preferably potassium-t-butoxide. The reaction temperature of this process depends on the raw material compounds, used inert solvent, and so on, but is usually -78°C to 80°C, and preferably 0°C to 65°C.

The reaction time of this process depends on the raw material compounds, used inert solvent, reaction temperature, and so on, but is usually 0.5 to 48 hr, and preferably 1 to 24 hr.

### Process G2

In this process, a compound having the General Formula (XXVII) is produced. In this process, a compound having the General Formula (XXVI) is heated in an inert solvent in the presence or absence of an acid or a base (preferably an acid).

The inert solvent used in this process is not specifically limited as long as the solvent can dissolve the starting material to a certain extent without inhibiting the reaction, examples of which include amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; water; and solvent mixtures thereof, preferably water.

Examples of the acid used in this process include mineral acids such as hydrochloric acid and sulfuric acid, preferably concentrated hydrochloric acid.

Examples of the base used in this process include alkali metal hydroxides such as sodium hydroxide and potassium hydroxide.

The reaction temperature of this process depends on the raw material compounds, used inert solvent, and so on, but is usually 80°C to 180°C, and preferably 100°C to 120°C.

The reaction time of this process depends on the raw material compounds, used inert solvent, reaction temperature, and so on, but is usually 1 to 24 hr, and preferably 6 to 12 hr.

### Process G3

In this process, a compound having the General Formula (XXVIII) is produced.

In this process, a compound having the General Formula (XXVII) reacts with N,N-dimethylformamidedimethylacetal in an inert solvent.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably amides, more preferably N,N-dimethylformamide.

The reaction temperature of this process depends on the raw material compounds, used inert solvent, and so on, but is usually 0°C to 150°C, and preferably 20°C to 120°C.

The reaction time of this process depends on the raw material compounds, used inert solvent, reaction temperature, and so on, but is usually 0.5 to 48 hr, and preferably 1 to 24 hr.

### Process G4

In this process, a compound having the General Formula (XXIX) is produced.

In this process, a compound having the General Formula (XXVIII) reacts with hydrazine in an inert solvent.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably alcohols, more preferably ethanol.

The reaction temperature of this process depends on the raw material compounds, used inert solvent, and so on, but is usually 0°C to 80°C, and preferably 20°C to 40°C.

The reaction time of this process depends on the raw material compounds, used inert solvent, reaction temperature, and so on, but is usually 0.5 to 48 hr, and preferably 1 to 18 hr.

### Process G5

In this process, a compound having the General Formula (XXXI) is produced.

In this process, a compound having the General Formula (XXIX) reacts with a compound having the General Formula (XXX) in an inert solvent in the presence of a base. The compound having the General Formula (XXX) is a known compound or is readily produced by a known method using a known compound as a starting raw material.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably amides, more preferably N,N-dimethylformamide.

Examples of the base used in this process include alkali metal carbonates such as sodium carbonate, potassium carbonate, and lithium carbonate; alkali metal bicarbonates such as sodium bicarbonate, potassium bicarbonate, and lithium bicarbonate; alkali metal hydrides such as lithium hydride, sodium hydride, and potassium hydride; alkali metal hydroxides such as sodium hydroxide, potassium hydroxide, barium hydroxide, and lithium hydroxide; alkali metal fluorides such as sodium fluoride and potassium fluoride; alkali metal alkoxides such as sodium methoxide, sodium ethoxide, sodium-t-butoxide, potassium methoxide, potassium ethoxide, potassium-t-butoxide, and lithium methoxide; alkali metal trialkylsiloxides such as sodium trimethylsiloxide, potassium trimethylsiloxide, and lithium trimethylsiloxide; alkali metal mercaptans such as sodium methyl mercaptan and sodium ethyl mercaptan; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]nona-5-ene (DBN), 1,4-diazabicyclo[2.2.2]octane (DABCO), and 1,8-diazabicyclo[5.4.0]undeca-7-ene (DBU); and organometallic bases such as butyllithium, lithium diisopropylamide, and lithium bis(trimethylsilyl)amide, preferably alkali metal hydrides, more preferably sodium hydride.

The reaction temperature of this process depends on the raw material compounds, used inert solvent, and so on, but is usually -78°C to 80°C, and preferably 0°C to 40°C.

The reaction time of this process depends on the raw material compounds, used inert solvent, reaction temperature, and so on, but is usually 0.5 to 48 hr, and preferably 1 to 12 hr.

### Process G6

In this process, a compound having the General Formula (I-E) is produced.

In this process, a compound having the General Formula (XXXI) reacts with a compound having the General Formula (XIII) in an inert solvent in the presence of a palladium catalyst and an inorganic base as in the Process A3 of the above-described Method A. The compound having the General Formula (XIII) is a known compound or is readily prepared by a known method using a known compound as a starting raw material. Then, when necessary, the protecting group of the amino group, hydroxy group, and/or carboxyl group on R^{1a} is removed.

### Process G7

In this process, a compound having the General Formula (I-F) is produced.

In this process, a compound having the General Formula (XXXI) reacts with a compound having the General Formula (XVIII) produced in the Process D2 of the above-described Method D in an inert solvent in the presence of a palladium catalyst and an inorganic base as in the Process A3 of the above-described Method A. Then, when necessary, the protecting group of the amino group, hydroxy group, and/or carboxyl group on R^{7a} is removed.

Method H is a process for manufacturing a compound having the General Formula (I-E), General Formula (I-F), and General Formula (I-G).

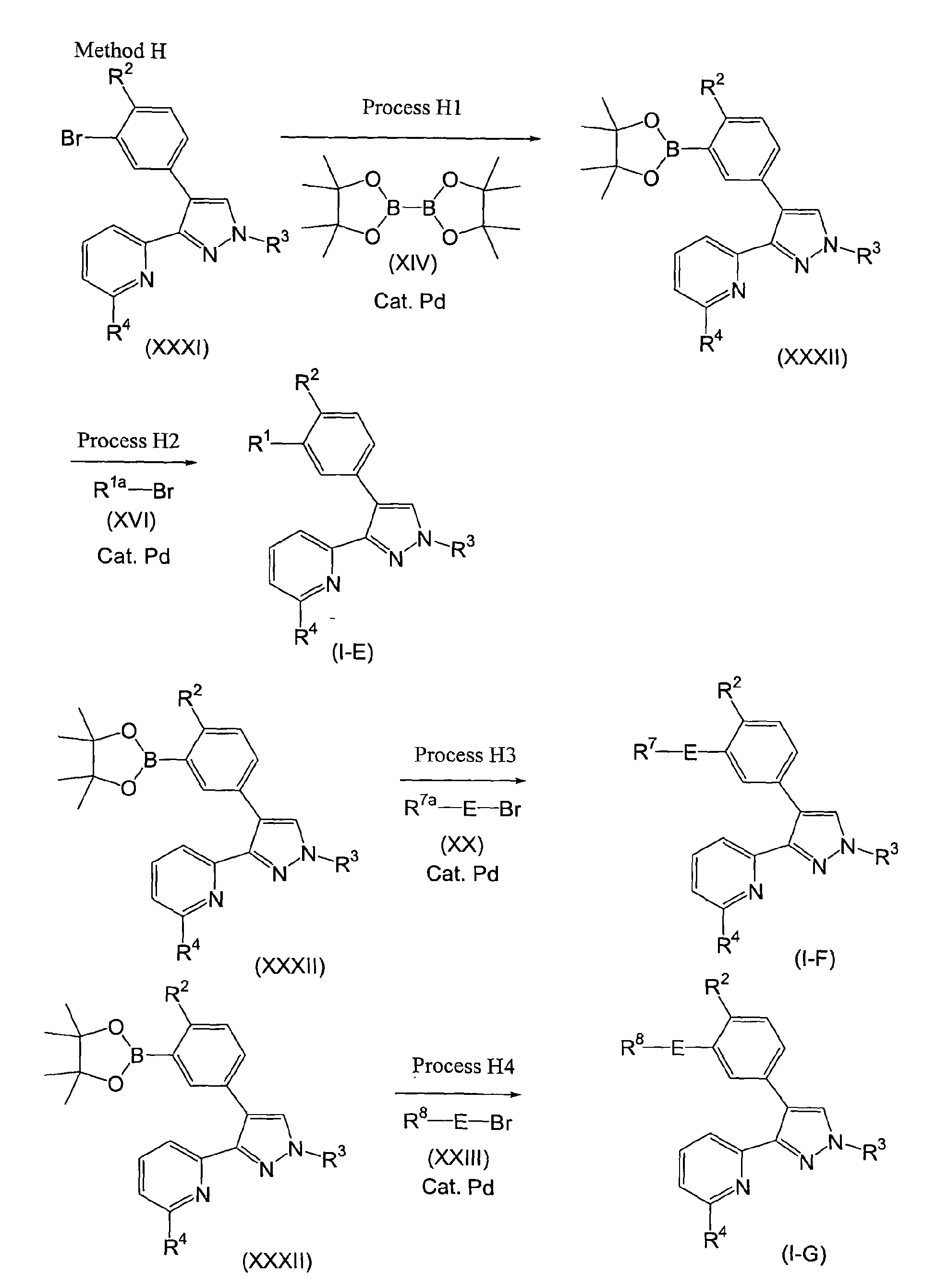

In the present method, R¹, R², R³, R⁴, R⁷, R⁸, R^{1a}, R^{7a}, and E are the same as defined above.

### Process H1

In this process, a compound having the General Formula (XXXII) is produced.

In this process, a compound having the General Formula (XXXI) produced in the Process G4 of the above-described Method G reacts with bis(pinacolato)diboron (XIV) in an inert solvent in the presence of a palladium catalyst and an inorganic base as in the Process B1 of the above-described Method B.

### Process H2

In this process, a compound having the General Formula (I-E) is produced.

In this process, a compound having the General Formula (XXXII) reacts with a compound having the General Formula (XVI) in an inert solvent in the presence of a palladium catalyst and an inorganic base as in the Process A3 of the above-described Method A. The compound having the General Formula (XVI) is a known compound or is readily prepared by a known method using a known compound as a starting raw material. Then, when necessary, the protecting group of the amino group, hydroxy group, and/or carboxyl group on R^{1a} is removed.

### Process H3

In this process, a compound having the General Formula (I-F) is produced.

In this process, a compound having the General Formula (XXXII) reacts with a compound having the General Formula (XX) produced in the Process E1 of the above-described Method E in an inert solvent in the presence of a palladium catalyst and an inorganic base as in the Process A3 of the above-described Method A. Then, when necessary, the protecting group of the amino group, hydroxy group, and/or carboxyl group on R^{7a} is removed.

### Process H4

In this process, a compound having the General Formula (I-G) is produced.

In this process, a compound having the General Formula (XXXII) reacts with a compound having the General Formula (XXIII) produced in the Process F1 of the above-described Method F in an inert solvent in the presence of a palladium catalyst and an inorganic base as in the Process A3 of the above-described Method A.

Method I is a process for converting a substituent on a C₆-C₁₀ aryl group which is substituted and a heterocyclic group which may be substituted in R¹ of the compound having the General Formula (I-H).

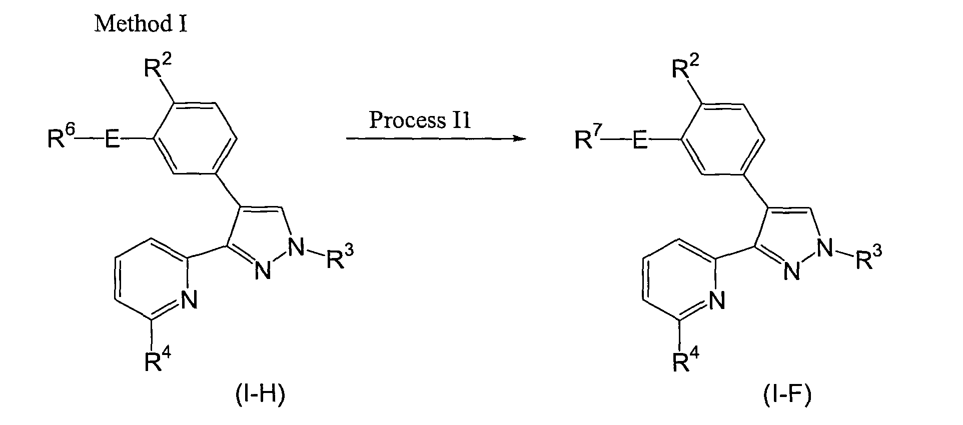

In the present method, R², R³, R⁴, R⁶, R⁷, and E are the same as defined above.

### Process I1

In this process, a compound having the General Formula (I-F) is produced.

In this process, the substituent R⁶ of a compound having the General Formula (I-H) is converted to the substituent R⁷ of a compound having the General Formula (I-F) as in the Process C1 of the above-described Method C.

Method J is a process for manufacturing a compound having the General Formula (I-I) and General Formula (I-J).

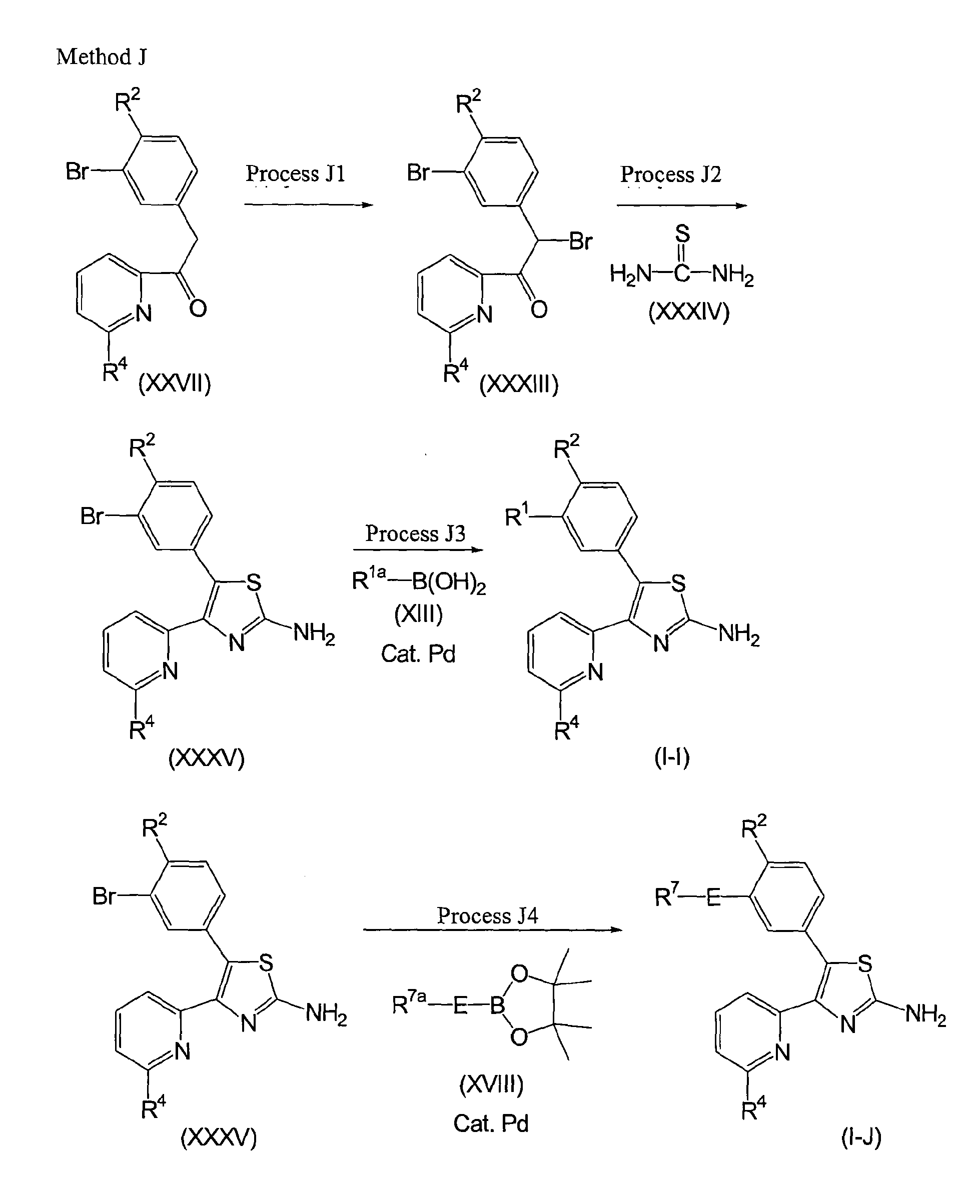

In the present method, R¹, R², R⁴, R⁷, R^{1a}, R^{7a}, and E are the same as defined above.

### Process J1

In this process, a compound having the General Formula (XXXIII) is produced.

In this process, a compound having the General Formula (XXVII) produced in the Process G2 of the above-described Method G reacts with a brominating agent in an inert solvent.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; carboxylic acids such as formic acid and acetic acid; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably halogenated hydrocarbons, more preferably chloroform.

Examples of the brominating agent used in this process include inorganic acids such as hydrobromic acid; halogen molecules such as bromine; phosphorus reagents such as phosphorus tribromide, phosphorus pentabromide, and phosphorus oxybromide; carboxylic bromides such as oxalyl bromide; sulfinic acid reagents such as thionyl bromide and toluenesulfonyl bromide; succinimide reagents such as N-bromosuccinimide; and sulfonic acid reagents such as sulfonyl bromide, preferably halogen molecules, more preferably bromine.

The reaction temperature of this process depends on the raw material compounds, used inert solvent, and so on, but is usually 0°C to 100°C, and preferably 20°C to 65°C.

The reaction time of this process depends on the raw material compounds, used inert solvent, reaction temperature, and so on, but is usually 0.5 to 24 hr, and preferably 1 to 3 hr.

### Process J2

In this process, a compound having the General Formula (XXXV) is produced.

In this process, a compound having the General Formula (XXXIII) reacts with a thiourea (XXXIV) in an inert solvent (for example, Bioorg. Med. Chem. Lett., 8, 3153, 1998).

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably alcohols, more preferably ethanol.

The reaction temperature of this process depends on the raw material compounds, used inert solvent, and so on, but is usually 0°C to 120°C, and preferably 20°C to 80°C.

The reaction time of this process depends on the raw material compounds, used inert solvent, reaction temperature, and so on, but is usually 0.5 to 24 hr, and preferably 1 to 3 hr.

### Process J3

In this process, a compound having the General Formula (I-I) is produced.

In this process, a compound having the General Formula (XXXV) reacts with a compound having the General Formula (XIII) in an inert solvent in the presence of a palladium catalyst and an inorganic base as in the Process A3 of the above-described Method A. The compound having the General Formula (XIII) is a known compound or is readily prepared by a known method using a known compound as a starting raw material. Then, when necessary, the protecting group of the amino group, hydroxy group, and/or carboxyl group on R^{1a} is removed.

### Process J4

In this process, a compound having the General Formula (I-J) is produced.

In this process, a compound having the General Formula (XXXV) reacts with a compound having the General Formula (XVIII) produced in the Process D2 of the above-described Method D in an inert solvent in the presence of a palladium catalyst and an inorganic base as in the Process A3 of the above-described Method A. Then, when necessary, the protecting group of the amino group, hydroxy group, and/or carboxyl group on R^{7a} is removed.

Method K is a process for manufacturing a compound having the General Formula (1-1), General Formula (I-J), and General Formula (I-K).

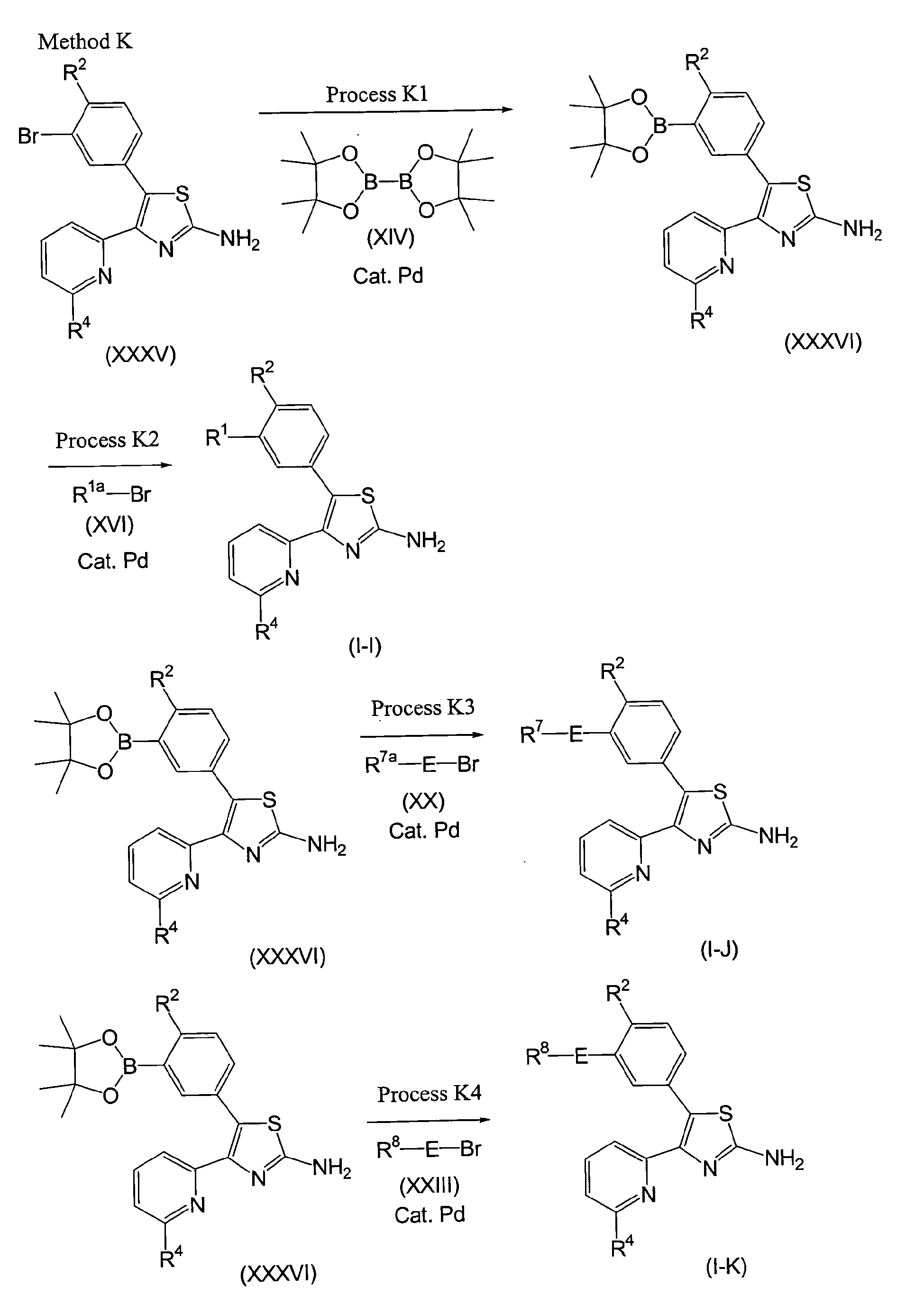

In the present method, R¹, R², R⁴, R⁷, R⁸, R^{1a}, R^{7a}, and E are the same as defined above.

### Process K1

In this process, a compound having the General Formula (XXXVI) is produced.

In this process, a compound having the General Formula (XXXV) produced in the Process J2 of the above-described Method J reacts with bis(pinacolato)diboron (XIV) in an inert solvent in the presence of a palladium catalyst and an inorganic base as in the Process B1 of the above-described Method B.

### Process K2

In this process, a compound having the General Formula (I-I) is produced. In this process, a compound having the General Formula (XXXVI) reacts with a compound having the General Formula (XVI) in an inert solvent in the presence of a palladium catalyst and an inorganic base as in the Process A3 of the above-described Method A. The compound having the General Formula (XVI) is a known compound or is readily prepared by a known method using a known compound as a starting raw material. Then, when necessary, the protecting group of the amino group, hydroxy group, and/or carboxyl group on R^{1a} is removed.

### Process K3

In this process, a compound having the General Formula (I-J) is produced.

In this process, a compound having the General Formula (XXXVI) reacts with a compound having the General Formula (XX) produced in the Process E1 of the above-described Method E in an inert solvent in the presence of a palladium catalyst and an inorganic base as in the Process A3 of the above-described Method A. Then, when necessary, the protecting group of the amino group, hydroxy group, and/or carboxyl group on R^{7a} is removed.

### Process K4

In this process, a compound having the General Formula (I-K) is produced.

In this process, a compound having the General Formula (XXXVI) reacts with a compound having the General Formula (XXIII) produced in the Process F1 of the above-described Method F in an inert solvent in the presence of a palladium catalyst and an inorganic base as in the Process A3 of the above-described Method A.

Method L is a process for converting a substituent on a C₆-C₁₀ aryl group which is substituted and a heterocyclic group which may be substituted in R¹ of the compound having the General Formula (I-L).

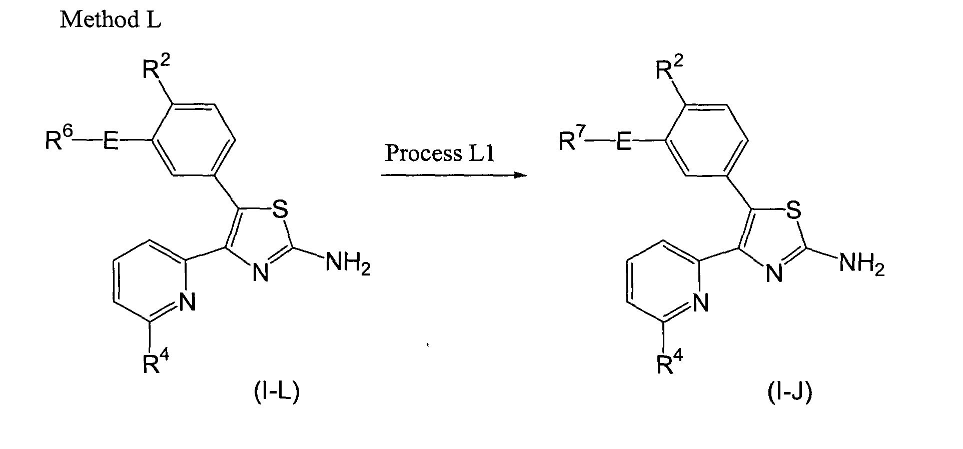

In the present method, R², R⁴, R⁶, R⁷, and E are the same as defined above.

### Process L1

In this process, a compound having the General Formula (I-J) is produced.

In this process, the substituent R⁶ of a compound having the General Formula (I-L) is converted to the substituent R⁷ of a compound having the General Formula (I-J) as in the Process C1 of the above-described Method C. When necessary, the amino group on the thiazole group is protected, and, after the reaction, the protecting group is removed.

Method M is a process for manufacturing a compound having the General Formula (I-M), General Formula (I-N), and General Formula (I-O).

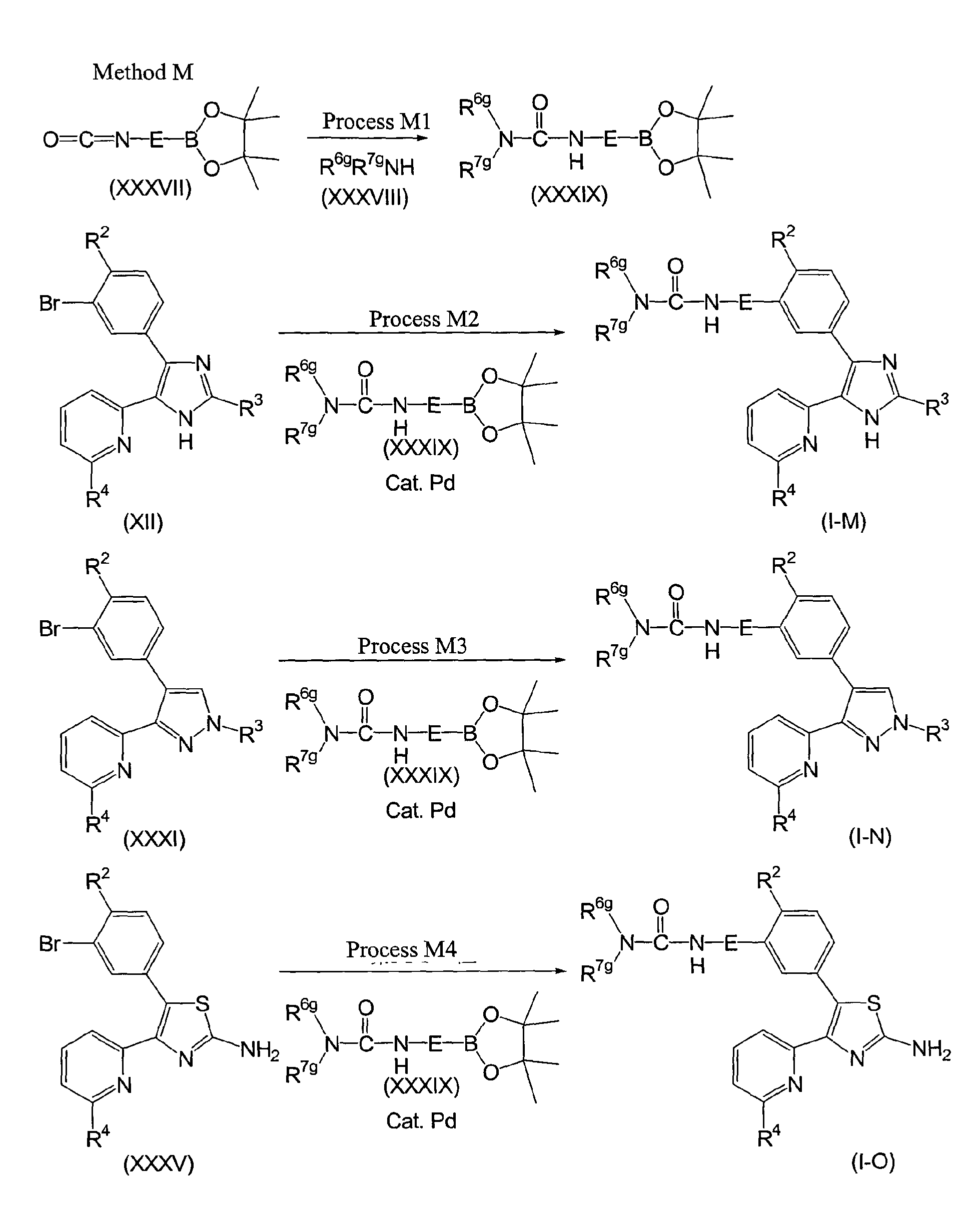

In the present method, R², R³, R⁴, and E are the same as defined above. NR^{6g}R^{7g} is a substituent represented by formula NHR (R is the same as defined above), a substituent represented by formula NR^{6d}R^{7d} (R^{6d} and R^{7d} are the same as defined above), or a substituent represented by formula NR^{6f}R^{7f} (R^{6f} and R^{7f} are the same as defined above).

### Process M1

In this process, a compound having the General Formula (XXXIX) is produced.

In this process, a compound having the General Formula (XXXVII) reacts with a compound having the General Formula (XXXVIII) in an inert solvent in the presence or absence (preferably absence) of a base. Each of the compounds having the General Formula (XXXVII) or General Formula (XXXVIII) is a known compound or is readily prepared by a known method using a known compound as a starting raw material.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably halogenated hydrocarbons, more preferably methylene chloride.

The reaction temperature of this process depends on the raw material compounds, used inert solvent, and so on, but is usually -30°C to 100°C, and preferably 0°C to 40°C.

The reaction time of this process depends on the raw material compounds, used inert solvent, reaction temperature, and so on, but is usually 1 to 24 hr, and preferably 2 to 12 hr.

### Process M2

In this process, a compound having the General Formula (I-M) is produced. In this process, a compound having the General Formula (XII) produced in the Process A2 of the above-described Method A reacts with a compound having the General Formula (XXXIX) in an inert solvent in the presence of a palladium catalyst and an inorganic base as in the Process A3 of the above-described Method A.

### Process M3

In this process, a compound having the General Formula (I-N) is produced.

In this process, a compound having the General Formula (XXXI) produced in the Process G4 of the above-described Method G reacts with a compound having the General Formula (XXXIX) in an inert solvent in the presence of a palladium catalyst and an inorganic base as in the Process A3 of the above-described Method A.

### Process M4

In this process, a compound having the General Formula (I-O) is produced.

In this process, a compound having the General Formula (XXXV) produced in the Process J2 of the above-described Method J reacts with a compound having the General Formula (XXXIX) in an inert solvent in the presence of a palladium catalyst and an inorganic base as in the Process A3 of the above-described Method A.

Method N is a process for manufacturing a compound having the General Formula (I-P) in which a carbon atom of a heterocyclic ring (here, an imidazole ring) binds with a benzene ring, apart from the above-described Methods A, B, D, E, F, G, H, J, and K.

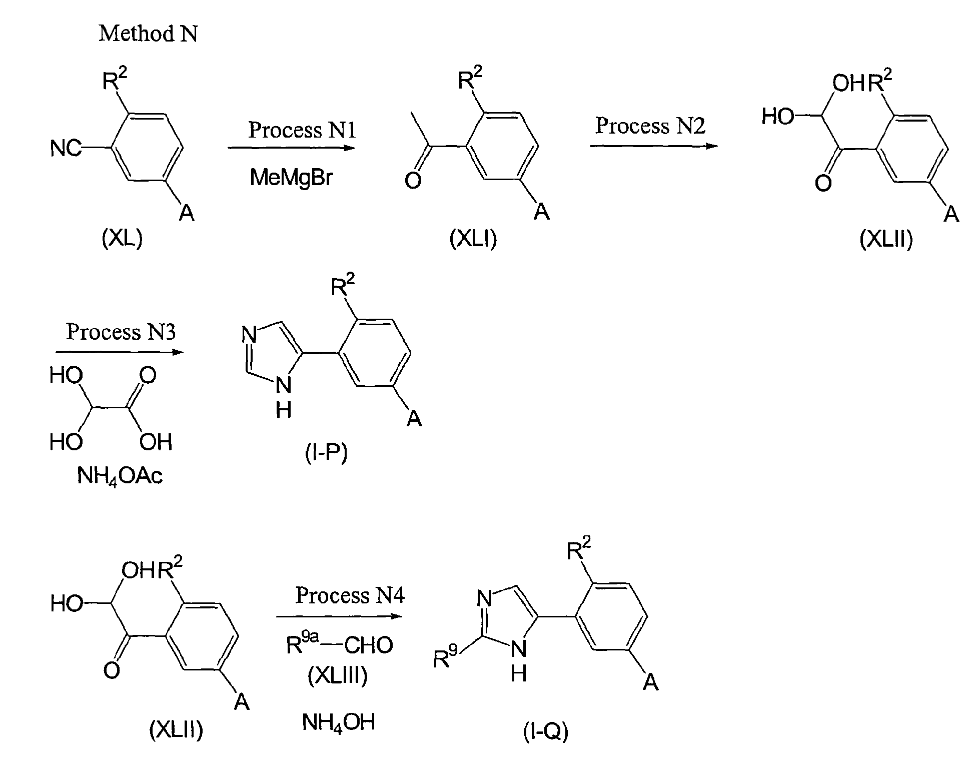

In the present method, R² and A are the same as defined above. R⁹ is a substituent represented by formula R-L-. R^{9a} is the same group as R⁹ except that the amino group, the hydroxyl group and/or the carboxyl group contained in R⁹ as a substituent is an amino, hydroxyl and/or carboxyl group which may be protected.

### Process N1

In this process, a compound having the General Formula (XLI) is produced.

In this process, a compound having the General Formula (XL) reacts with a methyl Grignard reagent in an inert solvent.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; ethers such as diethyl ether, diisopropyl ether, tetrahydrofuran, dioxane, dimethoxyethane, and diethylene glycol dimethyl ether; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably ethers, more preferably diethyl ether or tetrahydrofuran.

The reaction temperature of this process depends on the raw material compounds, used inert solvent, and so on, but is usually -20°C to 100°C, and preferably 20°C to 70°C.

The reaction time of this process depends on the raw material compounds, used inert solvent, reaction temperature, and so on, but is usually 0.5 to 24 hr, and preferably 1 to 8 hr.

### Process N2

In this process, a compound having the General Formula (XLII) is produced.

In this process, a compound having the General Formula (XLI) reacts with dimethylsulfoxide and a hydrogen halide in an inert solvent or in the absence of a solvent.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile and isobutyronitrile; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably sulfoxides, more preferably dimethylsulfoxide. Further, the solvent may be used with water (the mixture ratio is 1 : 100 to 100 : 1, preferably 20 : 80 to 80 : 20), according to need.

The hydrogen halide used in this process is a hydrogen bromide solution or hydrochloric acid, and preferably a hydrogen bromide solution.

The reaction temperature of this process depends on the raw material compounds, used inert solvent, and so on, but is usually 20°C to 130°C, and preferably 60°C to 100°C.

The reaction time of this process depends on the raw material compounds, used inert solvent, reaction temperature, and so on, but is usually 0.5 to 12 hr, and preferably 1 to 6 hr.

### Process N3

In this process, a compound having the General Formula (I-P) is produced. In this process, a compound having the General Formula (XLII) reacts with glyoxylic acid hydrate and ammonium acetate in an inert solvent.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; carboxylic acids such as acetic acid, formic acid, and trifluoroacetic acid; and solvent mixtures thereof, preferably alcohols, more preferably methanol or ethanol. Further, the solvent may be used with water (the mixture ratio is 1 : 100 to 100 : 1, preferably 90 : 10 to 10 : 90), according to need.

The reaction temperature of this process depends on the raw material compounds, used inert solvent, and so on, but is usually 0°C to 100°C, and preferably 25°C to 60°C.

The reaction time of this process depends on the raw material compounds, used inert solvent, reaction temperature, and so on, but is usually 0.5 to 24 hr, and preferably 1 to 6 hr.

### Process N4

In this process, a compound having the General Formula (I-Q) is produced.

In this process, a compound having the General Formula (XLII) reacts with a compound having the General Formula (XLIII) and ammonia water in an inert solvent. The compound having the General Formula (XLIII) is a known compound or is readily prepared by a known method using a known compound as a starting material. Then, when necessary, the protecting group of the amino group, hydroxy group, and/or carboxyl group on R^{9a} is removed.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; carboxylic acids such as acetic acid, formic acid, and trifluoroacetic acid; and solvent mixtures thereof, preferably alcohols, more preferably methanol or ethanol. Further, the solvent may be used with water (the mixture ratio is 1 : 100 to 100 : 1, preferably 90 : 10 to 10 : 90), according to need.

The reaction temperature of this process depends on the raw material compounds, used inert solvent, and so on, but is usually 0°C to 100°C, and preferably 25°C to 60°C.

The reaction time of this process depends on the raw material compounds, used inert solvent, reaction temperature, and so on, but is usually 0.5 to 24 hr, and preferably 1 to 6 hr.

In addition, instead of an imidazole ring, a compound in which a carbon atom of a heterocyclic ring binds with a benzene ring can be synthesized by a known method using a compound having the General Formula (XL) or a compound which is readily prepared by a known method using a compound having the General Formula (XL) as a starting material.

Method O is a process for manufacturing a compound having the General Formula (XL-O). The compound has a substituent represented by formula (II) as the substituent A of a compound having the General Formula (XL) which is the starting compound in the above-described Method N.

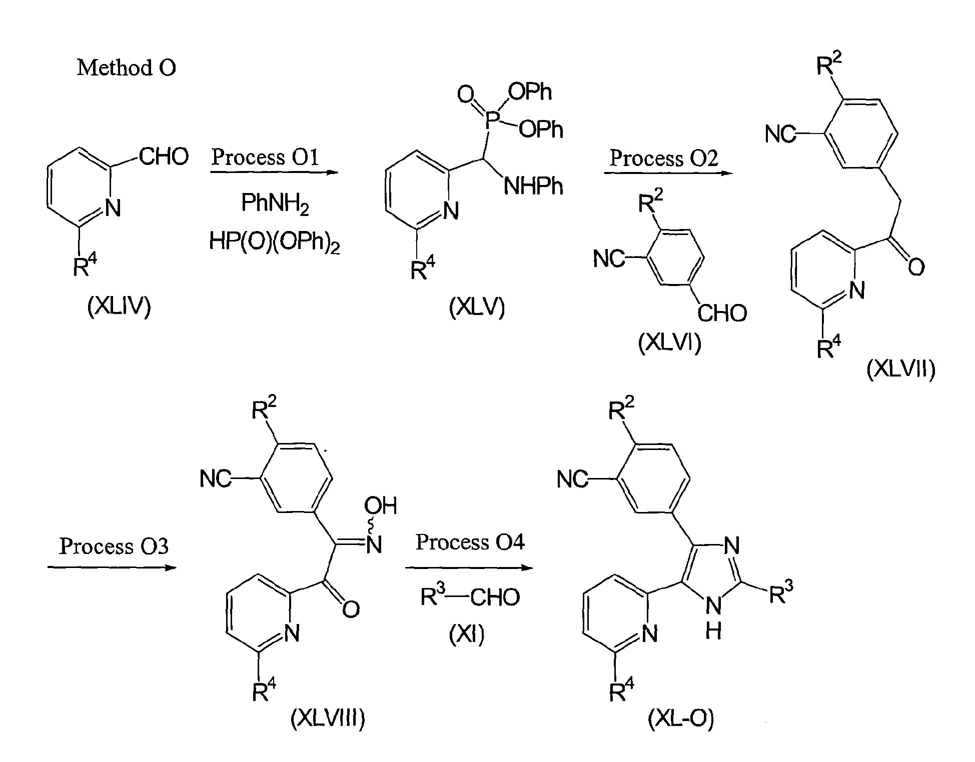

In the present method, R², R³, and R⁴ are the same as defined above.

### Process O1

In this process, a compound having the General Formula (XLV) is produced.

In this process, a compound having the General Formula (XLIV) reacts with aniline and diphenyl phosphite in an inert solvent. The compound having the General Formula (XLIV) is a known compound or is readily prepared by a known method using a known compound as a starting material.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably alcohols, more preferably i-propanol.

The reaction temperature of this process depends on the raw material compounds, used inert solvent, and so on, but is usually 0°C to 100°C, and preferably 20°C to 80°C.

The reaction time of this process depends on the raw material compounds, used inert solvent, reaction temperature, and so on, but is usually 1 to 12 hr, and preferably 2 to 6 hr.

### Process O2

In this process, a compound having the General Formula (XLVII) is produced.

In this process, a compound having the General Formula (XLV) reacts with a compound having the General Formula (XLVI) in an inert solvent in the presence of a base, and then the reaction solution is acidified with a hydrochloric acid solution or the like. The compound having the General Formula (XLVI) is a known compound or is readily prepared by a known method using a known compound as a starting material.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably ethers, alcohols, or a mixture thereof; more preferably tetrahydrofuran, i-propanol, or a mixture thereof; further more preferably a solvent mixture of tetrahydrofuran and i-propanol.

Examples of the base used in this process include inorganic bases such as alkali metal carbonates such as sodium carbonate, potassium carbonate, lithium carbonate, and cesium carbonate; alkali metal bicarbonates such as sodium bicarbonate, potassium bicarbonate, and lithium bicarbonate; alkali metal hydrides such as lithium hydride, sodium hydride, and potassium hydride; alkali metal hydroxides such as sodium hydroxide, potassium hydroxide, barium hydroxide, and lithium hydroxide; alkali metal fluorides such as sodium fluoride and potassium fluoride; alkali metal alkoxides such as sodium methoxide, sodium ethoxide, sodium-t-butoxide, potassium methoxide, potassium ethoxide, potassium-t-butoxide, and lithium methoxide; alkali metal trialkylsiloxides such as sodium trimethylsiloxide, potassium trimethylsiloxide, and lithium trimethylsiloxide; and alkali metal mercaptans such as sodium methyl mercaptan and sodium ethyl mercaptan; organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]nona-5-ene (DBN), 1,4-diazabicyclo[2.2.2]octane (DABCO), and 1,8-diazabicyclo[5.4.0]undeca-7-ene (DBU); and organometallic bases such as butyllithium, lithium diisopropylamide, and lithium bis(trimethylsilyl)amide, preferably alkali metal carbonates, more preferably cesium carbonate.

The reaction temperature of this process depends on the raw material compounds, used inert solvent, and so on, but is usually -20°C to 60°C, and preferably 10°C to 40°C.

The reaction time of this process depends on the raw material compounds, used inert solvent, reaction temperature, and so on, but is usually 0.5 to 6 hr, and preferably 1 to 4 hr.

### Process O3

In this process, a compound having the General Formula (XLVIII) is produced.

In this process, a compound having the General Formula (XLVII) reacts with sodium nitrite or t-butyl nitrite in an inert solvent in the presence of an acid.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; solvent mixtures thereof; and water, preferably ethers, alcohols, or water; more preferably tetrahydrofuran or water.

Examples of the acid used in this process include hydrogen halides such as a hydrogen chloride gas and hydrogen bromide gas; mineral acids such as sulfuric acid, hydrobromic acid, and hydrochloric acid; organic sulfonic acids such as methanesulfonic acid, p-toluenesulfonic acid, camphorsulfonic acid, and trifluoromethanesulfonic acid; carbonic acids such as acetic acid, formic acid, and trifluoroacetic acid; Lewis acids such as zinc chloride, tin tetrachloride, boron trifluoride, and boron tribromide; and acidic ion-exchange resins, preferably mineral acids, more preferably hydrochloric acid, further more preferably concentrated hydrochloric acid.

The reaction temperature of this process depends on the raw material compounds, used inert solvent, and so on, but is usually -30°C to 100°C, and preferably 0°C to 40°C.

The reaction time of this process depends on the raw material compounds, used inert solvent, reaction temperature, and so on, but is usually 0.5 to 48 hr, and preferably 1 to 24 hr.

### Process O4

This process is a process for manufacturing a compound having the General Formula (XL-O), and includes the following steps (i) and (ii).

In step (i), a compound having the General Formula (XLVIII) reacts with a compound having the General Formula (XI) in a carboxylic acid in the presence of ammonium acetate. The compound having the General Formula (XI) is a known compound or is readily prepared by a known method using a known compound as a starting raw material.

The carboxylic acid used in this step is, for example, acetic acid, formic acid, propionic acid, butyric acid, or trifluoroacetic acid, and preferably acetic acid.

The reaction temperature of this step depends on the raw material compounds, used carboxylic acid, and so on, but is usually 20°C to 150°C, and preferably 80°C to 120°C.

The reaction time of this step depends on the raw material compounds, used carboxylic acid, reaction temperature, and so on, but is usually 1 to 48 hr, and preferably 2 to 24 hr.

In step (ii), the compound prepared in step (i) reacts with a reducing agent in an inert solvent.

The inert solvent used in this step is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably alcohols, more preferably methanol.

Examples of the reducing agent used in this step include metal salts such as titanium trichloride; and phosphorus compounds such as phosphorus trichloride and triethylphosphite, preferably metal salts, more preferably 10% titanium trichloride (20 to 30% hydrochloric acid aqueous solution).

The reaction temperature of this step depends on the raw material compounds, used inert solvent, and so on, but is usually -30°C to 100°C, and preferably 0°C to 40°C.

The reaction time of this step depends on the raw material compounds, used inert solvent, reaction temperature, and so on, but is usually 1 to 48 hr, and preferably 2 to 24 hr.

Method P is a process for manufacturing a compound having the General Formula (XL-P). The compound has a substituent represented by formula (III) as the substituent A of a compound having the General Formula (XL) which is the starting compound in the above-described Method N.

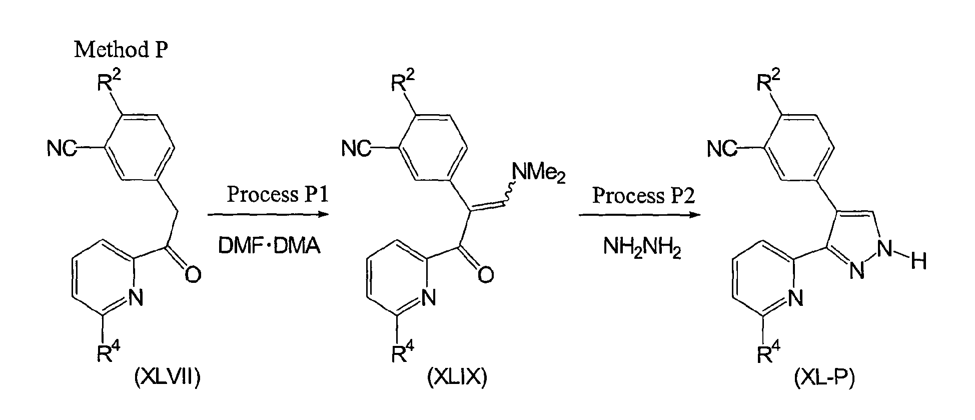

In the present method, R² and R⁴ are the same as defined above.

### Process P1

In this process, a compound having the General Formula (XLIX) is produced.

In this process, a compound having the General Formula (XLVII) prepared in the Process O2 of the above-described Method O reacts with N,N-dimethylformamide dimethyl acetal in an inert solvent.

The inert solvent used in this step is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably amides, more preferably N,N-dimethylformamide.

The reaction temperature of this process depends on the raw material compounds, used inert solvent, and so on, but is usually 0°C to 150°C, and preferably 20°C to 120°C.

The reaction time of this process depends on the raw material compounds, used inert solvent, reaction temperature, and so on, but is usually 0.5 to 48 hr, and preferably 1 to 24 hr.

### Process P2

In this process, a compound having the General Formula (XL-P) is produced. In this process, a compound having the General Formula (XLIX) reacts with hydrazine in an inert solvent.

The inert solvent used in this step is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methylpyrrolidinone, and hexamethylphosphate triamide; ethers such as diethyl ether, diisopropyl ether, tetrahydrofuran, dioxane, dimethoxyethane, and diethylene glycol dimethyl ether; alcohols such as methanol, ethanol, n-propanol, i-propanol, n-butanol, t-butanol, isoamyl alcohol, diethylene glycol, glycerin, octanol, cyclohexanol, and methyl cellosolve; sulfoxides such as dimethylsulfoxide and sulfolane; nitriles such as acetonitrile and isobutyronitrile; esters such as ethyl formate, ethyl acetate, propyl acetate, butyl acetate, and diethyl carbonate; ketones such as acetone, methylethylketone, 4-methyl-2-pentanone, methylisobutylketone, isophorone, and cyclohexanone; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably alcohols, more preferably ethanol.

The reaction temperature of this process depends on the raw material compounds, used inert solvent, and so on, but is usually 0°C to 80°C, and preferably 20°C to 40°C.

The reaction time of this process depends on the raw material compounds, used inert solvent, reaction temperature, and so on, but is usually 0.5 to 48 hr, and preferably 1 to 18 hr.

Method Q is a process for manufacturing a compound having the General Formula (I-R) in which a nitrogen atom of a heterocyclic ring (here, an imidazole ring) binds with a benzene ring, apart from the above-described Methods A, B, D, E, F, G, H, J, and K.

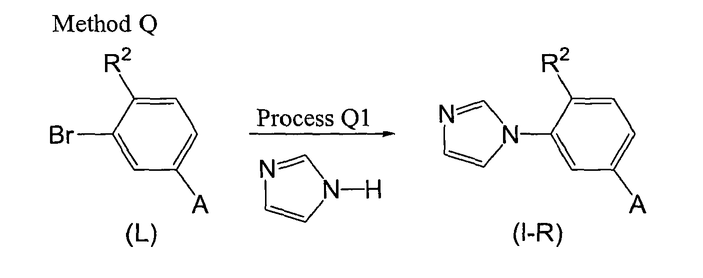

In the present method, R² and A are the same as defined above.

### Process Q1

In this process, a compound having the General Formula (I-R) is produced.

In this process, a compound having the General Formula (L) reacts with a heterocyclic ring (for example, an imidazole ring in the above-mentioned formula) having a substituent represented by formula NH or a heterocyclic ring in which a substituent represented by formula NH is formed in an equilibrium state. The reaction is carried out in an inert solvent in the presence of a catalyst and an inorganic base by heating the reaction mixture in a flask or a closed tube or in a microwave reaction device.

When the substituent A is represented by formula (II), the compound having the General Formula (L) used in this process is a compound having the General Formula (XII) prepared in the Process A2 of the above-described Method A; when the substituent A is represented by formula (III), the compound having the General Formula (L) is a compound having the General Formula (XXIX) prepared in the Process G4 of the above-described Method G and having the General Formula (XXXI) prepared in the Process G5 of the above-described Method G; and when the substituent A is represented by formula (IV), the compound having the General Formula (L) is a compound having the General Formula (XXXV) prepared in the Process J2 of the above-described Method J.

The inert solvent used in this process is not specifically limited as long as the solvent can dissolve the starting material to a certain extent without inhibiting the reaction, examples of which include hydrocarbons such as pentane, hexane, octane, petroleum ether, and ligroin; amides such as formamide, N,N-dimethylformamide, N,N-dimethylacetoamide, N-methyl-2-pyrrolidone, N-methyl-2-pyrrolidinone, and hexamethylphosphate triamide; ethers such as diethyl ether, diisopropyl ether, tetrahydrofuran, dioxane, dimethoxyethane, and diethylene glycol dimethyl ether; sulfoxides such as dimethylsulfoxide and sulfolane; nitriles such as acetonitrile and isobutyronitrile; nitro compounds such as nitroethane and nitrobenzene; halogenated hydrocarbons such as methylene chloride, 1,2-dichloroethane, dichlorobenzene, chloroform, and carbon tetrachloride; aromatic hydrocarbons such as benzene, toluene, and xylene; and solvent mixtures thereof, preferably amides, more preferably N-methyl-2-pyrrolidinone.

Examples of the catalyst used in this process include nickel catalysts and copper catalysts, for example, zerovalent nickel and complexes thereof; zerovalent copper and complexes thereof; monovalent copper salts such as copper(I) chloride, copper(I) bromide, copper(I) iodide, and copper(I) trifluoromethanesulfonate; and bivalent copper salts such as copper(II) bromide, copper(II) acetate, and copper(II) sulfate, preferably monovalent copper salts, more preferably copper(I) iodide.

Examples of the inorganic base used in this process include alkali metal hydrides such as lithium hydride, sodium hydride, and potassium hydride; alkali metal carbonates such as sodium carbonate, potassium carbonate, and lithium carbonate; alkali metal phosphate such as lithium phosphate, sodium phosphate, and potassium phosphate; alkali metal alkoxides such as sodium methoxide, sodium ethoxide, sodium-t-butoxide, potassium methoxide, potassium ethoxide, potassium-t-butoxide, and lithium methoxide; and alkali metal hydroxides such as sodium hydroxide, potassium hydroxide, barium hydroxide, and lithium hydroxid, preferably alkali metal carbonates, more preferably potassium carbonate.

In this process, the heterocyclic ring having a substituent represented by formula NH or the heterocyclic ring in which a substituent represented by formula NH is formed in an equilibrium state is not specifically limited as long as the heterocyclic ring is a 5- to 7-membered heterocyclic ring containing one to three sulfur atom(s), oxygen atom(s), nitrogen atom(s), sulfinyl group(s), and/or sulfonyl group(s) and has a substituent represented by formula NH or in which a substituent represented by formula NH is formed in an equilibrium state. The heterocyclic ring may be fused with another cyclic group such as a benzene ring. Examples of heterocyclic ring include imidazole, pyrazole, triazole, tetrazole, pyrrole, pyrrolidine, piperazine, piperidine, morpholine, thiomorpholine, pyrroline, imidazoline, hydroxypyridine, hydroxypyrimidine, benzimidazole, benztriazole, indazole, indole, and indoline. These heterocyclic rings may each have a substituent.

The reaction temperature of this process depends on the raw material compounds, used inert solvent, and so on, but is usually 60°C to 230°C, and preferably 100°C to 180°C.

The reaction time of this process depends on the raw material compounds, used inert solvent, reaction temperature, and so on, but is usually 0.5 to 24 hr, and preferably 1 to 8 hr.

In each of the above-described processes, the target compound after reaction may be used in a subsequent process after being isolated and purified from the reaction mixture according to ordinary methods or the crude product after reaction may be used in a subsequent process without purification. Generally, the reaction mixture is optionally neutralized and, when necessary, insoluble materials are removed by filtration. Then, water and a water-inmiscible organic solvent (for example, benzene, diethyl ether, or ethyl acetate) are added thereto, and the organic layer containing the target compound is separated. The organic layer is washed with water and is dried with anhydrous magnesium sulfate, anhydrous sodium sulfate, or anhydrous sodium bicarbonate. After filtration, the solvent is removed by evaporation to obtain the target compound. When necessary, the resulting target compound may be isolated and purified by optionally combined ordinary methods which are commonly used for isolation and purification of organic compounds, such as recrystallization and reprecipitation. The target compound can be isolated and purified by employing chromatography to elute the compound with an appropriate eluent. When a target compound is insoluble to a solvent, the target compound can be purified by washing the resulting solid crude product with a solvent.

In the process in which protection and deprotection of a substituent are required, the protection and deprotection are carried out according to known methods (for example, "Protective Groups in Organic Synthesis" (Theodora W. Greene, Peter G. M. Wuts, 1999, A Wiley-Interscience Publication)).

Each of the raw materials (VIII), (IX), (XI), (XIII), (XIV), (XVI), (XVII), (XIX), (XXI), (XXII), (XXIV), (XXV), (XXX), (XXXIV), (XXXVII), (XXXVIII), (XLIII), (XLIV), and (XLVI) is a known compound or is readily prepared by a known method or a similar method thereof using a known compound as a starting raw material.

In the above description, the protecting group of the "amino group which may be protected", the "hydroxyl group which may be protected" and the "carboxyl group which may be protected" in the definition of R^{1a}, R^{7a}, and R^{9a} mean a protecting group which can be cleaved by a chemical method such as hydrogenolysis, hydrolysis, electrolysis or photolysis. This refers to protecting groups generally used in synthetic organic chemistry (see, for example, T. W. Greene et al., Protective Groups in Organic Synthesis, 3rd Edition, John Wiley & Sons, Inc. (1999)).

In the above description, the "protecting group" of the "hydroxyl group which may be protected" in the definition of R^{1a}, R^{7a}, and R^{9a} is not particularly limited as long as it is a protecting group of a hydroxyl group generally used in the field of organic synthetic chemistry. Examples thereof include "general protecting groups associated with an ester based on a hydroxyl group", and are preferably "alkylcarbonyl groups which may be substituted" including alkanoyl groups such as formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl and heneicosanoyl, halogenated alkylcarbonyl groups such as chloroacetyl, dichloroacetyl, trichloroacetyl and trifluoroacetyl, alkoxyalkylcarbonyl groups such as methoxyacetyl and unsaturated alkylcarbonyl groups such as acryloyl, propioloyl, methacryloyl, crotonoyl, isocrotonoyl and (E)-2-methyl-2-butenoyl; "arylacyl groups which may be substituted" including arylcarbonyl groups such as benzoyl, α-naphthoyl and β-naphthoyl, halogenated arylcarbonyl groups such as 2-bromobenzoyl and 4-chlorobenzoyl, C₁-C₆ alkylated arylcarbonyl groups such as 2,4,6-trimethylbenzoyl and 4-toloyl, C₁-C₆ alkoxylated arylcarbonyl groups such as 4-anisoyl, nitrated arylcarbonyl groups such as 4-nitrobenzoyl and 2-nitrobenzoyl, C₂-C₇ alkoxycarbonylated arylcarbonyl groups such as 2-(methoxycarbonyl)benzoyl and arylated arylcarbonyl groups such as 4-phenylbenzoyl; "alkoxycarbonyl groups" including the aforementioned "C₂-C₇ alkoxycarbonyl groups" and C₂-C₇ alkoxycarbonyl groups substituted by halogen or a tri-(C₁-C₆ alkyl)silyl group such as 2,2,2-trichloroethoxycarbonyl and 2-trimethylsilylethoxycarbonyl; "tetrahydropyranyl or tetrahydrothiopyranyl groups" such as tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl; "tetrahydrofuranyl or tetrahydrothiofuranyl groups" such as tetrahydrofuran-2-yl and tetrahydrothiofuran-2-yl; "silyl groups" including tri-(C₁-C₆ alkyl)silyl groups such as trimethylsilyl, triethylsilyl, isopropyldimethylsilyl, t-butyldimethylsilyl, methyldiisopropylsilyl, methyldi-t-butylsilyl and triisopropylsilyl, and (C₁-C₆ alkyl)diarylsilyl or di-(C₁-C₆ alkyl)arylsilyl groups such as diphenylmethylsilyl, diphenylbutylsilyl, diphenylisopropylsilyl and phenyldiisopropylsilyl; "alkoxymethyl groups" including C₁-C₆ alkoxymethyl groups such as methoxymethyl, 1,1-dimethyl-1-methoxymethyl, ethoxymethyl, propoxymethyl, isopropoxymethyl, butoxymethyl and t-butoxymethyl, C₁-C₆ alkoxy C₁-C₆ alkoxymethyl groups such as 2-methoxyethoxymethyl and C₁-C₆ halogenated alkoxymethyl such as 2,2,2-trichloroethoxymethyl and bis(2-chloroethoxy) methyl; "substituted ethyl groups" including C₁-C₆ alkoxyethyl groups such as 1-ethoxyethyl and 1-(isopropoxy)ethyl and halogenated ethyl groups such as 2,2,2-trichloroethyl; "aralkyl groups" including C₁-C₆ alkyl groups substituted by 1 to 3 aryl groups such as benzyl, α-naphthyhnethyl, β-naphthylmethyl, diphenylmethyl, triphenylmethyl, α-naphthyldiphenylmethyl and 9-anthrylmethyl, and C₁-C₆ alkyl groups substituted by 1 to 3 aryl groups, in which the aryl ring is substituted by a C₁-C₆ alkyl, C₁-C₆ alkoxy, nitro, halogen or cyano group, such as 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl and 4-cyanobenzyl; "alkenyloxycarbonyl groups" such as vinyloxycarbonyl and allyloxycarbonyl; and "aralkyloxycarbonyl groups" in which the aryl ring may be substituted by 1 or 2 C₁-C₆ alkoxy or nitro groups, such as benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl, preferably alkylcarbonyl groups which may be substituted, silyl groups or aralkyl groups.

In the above description, the "protecting group" of the "carboxyl group which may be protected" in the definition of R^{1a}, R^{7a}, and R^{9a} is not particularly limited as long as it is a protecting group of a carboxyl group generally used in the field of organic synthetic chemistry. Examples thereof include "general protecting groups associated with an ester based on a carboxyl group", and are preferably the aforementioned "C₁-C₆ alkyl groups"; "C₂-C₆ alkenyl groups" such as ethenyl, 1-propenyl and 2-propenyl; "C₂-C₆ alkynyl groups" such as ethynyl, 1-propynyl and 2-propynyl; the aforementioned "C₁-C₆ halogenated alkyl groups"; hydroxy "C₁-C₆ alkyl groups" such as 2-hydroxyethyl, 2,3-dihydroxypropyl, 3-hydroxypropyl, 3,4-dihydroxybutyl and 4-hydroxybutyl; "C₂-C₇ alkylcarbonyl"-"C₁-C₆ alkyl groups" such as acetylmethyl; the aforementioned "aralkyl groups"; or the aforementioned "silyl groups", preferably C₁-C₆ alkyl groups or aralkyl groups.

In the above description, the "protecting group" of the "amino group which may be protected" in the definition of R^{1a}, R^{7a}, and R^{9a} is not particularly limited as long as it is a protecting group of an amino group generally used in the field of organic synthetic chemistry. Examples thereof include the same "alkylcarbonyl groups"; "arylcarbonyl groups"; "alkoxycarbonyl groups"; "alkenyloxycarbonyl groups"; "aralkyloxycarbonyl groups"; "silyl groups"; or "aralkyl groups" in the aforementioned "general protecting group associated with an ester based on a hydroxyl group", or "substituted methylene groups which form a Schiff base" such as N,N-dimethylaminomethylene, benzylidene, 4-methoxybenzylidene, 4-nitrobenzylidene, salicylidene, 5-chlorosalicylidene, diphenylmethylene and (5-chloro-2-hydroxyphenyl)phenylmethylene, preferably alkylcarbonyl groups, arylcarbonyl groups or alkoxycarbonyl group, most preferably alkoxycarbonyl groups.

When the biaryl derivative having the above general formula (I) or a pharmacologically acceptable salt thereof according to the present invention is used as a medicine, the medicine is administered as is or after mixing with an appropriate pharmacologically acceptable excipient or diluent orally in the form of, for example, tablets, capsules, granules, powder or syrup, or parenterally by injection or in the form of suppository.

These formulations are prepared by a known method using additives such as an excipient (e.g., organic excipients including sugar derivatives such as lactose, sucrose, glucose, mannitol and sorbitol; starch derivatives such as corn starch, potato starch, α-starch and dextrin; cellulose derivatives such as crystalline cellulose; gum arabic; dextran; and pullulan; inorganic excipients including silicate derivatives such as light anhydrous silicic acid, synthetic aluminum silicate, calcium silicate and magnesium aluminometasilicate; phosphates such as calcium hydrogen phosphate; carbonates such as calcium carbonate; and sulfates such as calcium sulfate), a lubricant (e.g., stearic acid and metal stearates such as calcium stearate and magnesium stearate; talc; colloidal silica; waxes such as beegum and spermaceti; boric acid; adipic acid; sulfates such as sodium sulfate; glycol; fumaric acid; sodium benzoate; DL leucine; fatty acid sodium salt; lauryl sulfates such as sodium lauryl sulfate and magnesium lauryl sulfate; silicic acids such as silicic acid anhydride and silicic acid hydrate; and the above starch derivatives), a binder (e.g., hydroxypropyl cellulose, hydroxypropyl methylcellulose, polyvinylpyrrolidone, macrogol and compounds listed above as excipients), a disintegrant (e.g., cellulose derivatives such as low substitution degree hydroxypropyl cellulose, carboxymethylcellulose, calcium carboxymethylcellulose and internally cross-linked sodium carboxymethylcellulose; and chemically modified starch/cellulose such as carboxymethyl starch, sodium carboxymethyl starch and cross-linked polyvinylpyrrolidone), a stabilizer (parahydroxybenzoate such as methylparaben and propylparaben; alcohols such as chlorobutanol, benzyl alcohol and phenylethyl alcohol; benzalkonium chloride; phenols such as phenol and cresol; thimerosal; dehydroacetic acid; and sorbic acid), a corrigent (e.g., sweeteners, acidulants and flavourings generally used), or a diluent.

The dose varies depending on symptoms, age and other factors. Desirably, when administered orally, the compound is administered to an adult human in an amount of 0.0015 mg/kg body weight (preferably 0.008 mg/kg body weight) as a lower limit and 70 mg/kg body weight (preferably 7 mg/kg body weight) as an upper limit, and when administered intravenously, the compound is administered to an adult human in an amount of 0.00015 mg/kg body weight (preferably 0.0008 mg/kg body weight) as a lower limit and 8.5 mg/kg body weight (preferably 5 mg/kg body weight) as an upper limit per dose per day 1 to 6 times per day depending on symptoms.

### Examples

The present invention will now be further described in detail with reference to Examples and Test Examples, but the scope of the present invention is not limited thereto.

### (Example 1) 3'-[2-Isopropyl-4-(6-methylpyridin-2-yl)-1H-imidazol-5-yl]-1,1'-biphenyl-4-carboxyamide (Compound No. 1-361)

### (1a) 1-(3-Bromophenyl)-2-(6-methylpyridin-2-yl)ethanone

2,6-Lutidine (6.4 g, 60 mmol) was dissolved in tetrahydrofuran (5 mL), and sodium hexamethyldisilazane (1 M in tetrahydrofuran solution, 90 mL, 90 mmol) was added thereto at -30°C under a nitrogen atmosphere. The resulting mixture was stirred for 1.5 hr, and then a solution of ethyl 3-bromobenzoate (14 g, 60 mmol) in tetrahydrofuran (50 mL) was added thereto. The resulting solution was stirred at room temperature for 1.5 hr. A saturated ammonium chloride aqueous solution was added to the reaction solution to terminate the reaction. After extraction with ethyl acetate, the organic layer was washed with water and brine, and then dried with anhydrous magnesium sulfate. The solvent was evaporated under reduced pressure, and the resulting crude product was purified by silica gel column chromatography (eluting solvent; hexane : ethyl acetate = 5 : 1) to obtain 9.3 g (yield: 53%) of the title compound as a yellow solid.
¹H-NMR (400 MHz, CDCl₃) δ ppm: 8.20 (1H, t, J = 1.6 Hz), 7.95 (1H, m), 7.65 (1H, m), 7.52 (1H, m), 7.31 (1H, t, J = 7.8 Hz), 7.04 (2H, m), 4.42 (2H, s), 2.53 (3H, s). MS(ES) m/z: 289 (M + H)⁺.

### (1b) 2-[4-(3-Bromophenyl)-2-isopropyl-1H-imidazol-5-yl]-6-methylpyridine

1-(3-Bromophenyl)-2-(6-methylpyridin-2-yl)ethanone (9.3 g, 32 mmol) obtained in Example (1a) was dissolved in tetrahydrofuran (80 mL), and concentrated hydrochloric acid (8 mL) and t-butyl nitrite (4.2 mL, 35 mmol) were added thereto. The resulting mixture was stirred at room temperature for 1 hr. The reaction solution was neutralized with a 10% sodium hydroxide aqueous solution. After extraction with ethyl acetate, the organic layer was washed with water and brine, and then dried with anhydrous magnesium sulfate. The solvent was evaporated under reduced pressure. The resulting crude product (10 g) was dissolved in acetic acid (80 mL), and ammonium acetate (7.5 g, 97 mmol) and isobutylaldehyde (4.6 g, 64 mmol) were added thereto. The resulting mixture was heated under reflux for 1 hr. The reaction solution was evaporated under reduced pressure, and ammonia water was added to the resulting residue. After extraction with ethyl acetate, the organic layer was washed with brine and dried with anhydrous magnesium sulfate. The solvent was evaporated under reduced pressure. The resulting crude product (12 g) was dissolved in methanol (150 mL), and 10% titanium trichloride (20 to 30% hydrochloric acid aqueous solution, 70 mL, 54 mmol) was added thereto. The resulting mixture was stirred at room temperature for 1 hr. Ammonia water was added to the reaction solution under ice cooling, and the reaction mixture was filtered through Celite. The filtrate was extracted with ethyl acetate, and the organic layer was washed with water and brine, and then dried with anhydrous magnesium sulfate. The solvent was evaporated under reduced pressure, and the resulting crude product was purified by silica gel column chromatography (eluting solvent; hexane : ethyl acetate = 1 : 1) to obtain 8.9 g (yield: 78%) of the title compound as a white solid.
¹H-NMR (500 MHz, CDCl₃) δ ppm: 10.35 (1H, brs), 7.82 (1H, t, J = 1.5 Hz), 7.55 (1H, d, J = 7.8 Hz), 7.45 (1H, d, J = 8.8 Hz), 7.42 (1H, t, J = 7.8 Hz), 7.24 (2H, t, J = 7.8 Hz), 6.95 (1H, d, J = 7.8 Hz), 3.14 (1H, m), 2.51 (3H, s), 1.38 (6H, d, J = 6.8 Hz). MS(ES) m/z: 356 (M + H)⁺.

### (1c) 3'-[2-Isopropyl-4-(6-methylpyridin-2-yl)-1H-imidazol-5-yl]-1,1'-biphenyl-4-carboxyamide

2-[4-(3-Bromophenyl)-2-isopropyl-1H-imidazol-5-yl]-6-methylpyridine (0.29 g, 0.80 mmol) obtained in Example (1b), 4-cyanophenylboronic acid (0.24 g, 1.6 mmol), tripotassium phosphate n-hydrate (0.34 g, 1.3 mmol), and dichloro[1,1'-bis(diphenylphosphino)ferrocene]palladium(II)-dichloromethane complex (62 mg, 0.076 mmol) were dissolved in 1,2-dimethoxyethane (5 mL). The resulting mixture was stirred at 80°C for 2 hr. The reaction mixture was diluted with ethyl acetate and filtered through silica gel. The crude product obtained by concentrating the filtrate under reduced pressure was purified by high-performance liquid chromatography (GL Science ODS-3, eluting solvent; water : acetonitrile = 95 : 5 to 5: 95). The resulting compound (0.32 g) was dissolved in 1,4-dioxane (5 mL), and potassium trimethylsiloxide (0.32 g, 2.5 mmol) was added thereto. The resulting mixture was heated under reflux for 4 hr and then cooled to room temperature. A saturated ammonium chloride aqueous solution was added to the reaction mixture to terminate the reaction. Water and acetonitrile were added to the resulting crude product, and the mixture was purified by high-performance liquid chromatography (GL Science ODS-3, eluting solvent; water : acetonitrile = 95 : 5 to 5 : 95) to obtain 0.15 g (yield: 48%) of the title compound as a white powder. Melting point: 130 to 131°C.
¹H-NMR (500 MHz, CDCl₃) δ ppm: 10.07 (1H, brs), 7.91 (1H, s), 7.86 (2H, d, J = 8.3 Hz), 7.69 (2H, d, J = 8.3 Hz), 7.65 (1H, d, J = 7.8 Hz), 7.59 (1H, d, J = 8.3 Hz), 7.49 (1H, t, J = 7.8 Hz), 7.38 (1H, t, J = 7.8 Hz), 7.30 (1H, d, J = 7.8 Hz), 6.94 (1H, d, J = 7.3 Hz), 3.18 (1H, m), 2.56 (3H, s), 1.44 (6H, d, J = 7.3 Hz).
MS(ES) m/z: 397 (M + H)⁺.

### (Example 2) 3'-[2-Isopropyl-5-(6-methylpyridin-2-yl)-1H-imidazol-4-yl]-1,1'-biphenyl-3-carboxyamide (Compound No. 1-367)

The same reaction as in Example (1c) was carried out using 2-[4-(3-bromophenyl)-2-isopropyl-1H-imidazol-5-yl]-6-methylpyridine (0.11 g, 0.30 mmol) obtained in Example (1b) and 3-cyanophenylboronic acid (0.10 g, 0.68 mmol) to obtain 67 mg (yield: 57%) of the title compound as a white amorphous form. Melting point: 114 to 119°C.
¹H-NMR (500 MHz, CDCl₃) δ ppm: 10.35 (1H, brs), 8.06 (1H, s), 7.92 (1H, s), 7.78 (2H, m), 7.62 (1H, d, J = 7.8 Hz), 7.57 (1H, d, J = 7.3 Hz), 7.51-7.45 (2H, m), 7.40 (1H, t, J = 7.8 Hz), 7.30 (1H, d, J = 7.8 Hz), 6.94 (1H, d, J = 7.3 Hz), 6.32 (1H, brs), 5.69 (1H, brs), 3.17 (1H, m), 2.52 (3H, s), 1.41 (6H, d, J = 7.3 Hz).
MS(ES) m/z: 397 (M + H)⁺.

### (Example 3) 2-[2-Isopropyl-4-(4'-methyl-1,1'-biphenyl-3-yl)-1H-imidazol-5-yl]-6-methylpyridine (Compound No. 1-433)

2-[4-(3-Bromophenyl)-2-isopropyl-1H-imidazol-5-yl]-6-methylpyridine (0.11 g, 0.30 mmol) obtained in Example (1b), 4-methylphenylboronic acid (85 mg, 0.63 mmol), tripotassium phosphate n-hydrate (0.13 g, 0.50 mmol), and dichloro[1,1'-bis(diphenylphosphino)ferrocene]palladium(II)-dichloromethane complex (24 mg, 0.029 mmol) were dissolved in 1,2-dimethoxyethane (2 mL). The resulting mixture was stirred at 80°C for 2 hr. The reaction mixture was diluted with ethyl acetate and then filtered through silica gel. The crude product obtained by concentrating the filtrate under reduced pressure was purified by high-performance liquid chromatography (GL Science ODS-3, eluting solvent; water : acetonitrile = 95 : 5 to 5: 95) to obtain 87 mg (yield: 79%) of the title compound as a white amorphous form. Melting point: 76 to 79°C.
¹H-NMR (500 MHz, CDCl₃) δ ppm: 10.78 (1H, brs), 7.86 (1H, s), 7.59-7.32 (7H, m), 7.24-7.21 (2H, m), 6.92 (1H, d, J = 7.3 Hz), 3.14 (1H, m), 2.46 (3H, s), 2.38 (3H, s), 1.35 (6H, d, J = 6.8 Hz).
MS(ES) m/z: 368 (M + H)⁺.

### (Example 4) 2-[2-Isopropyl-4-(4'-methoxy-1,1'-biphenyl-3-yl)-1H-imidazol-5-yl]-6-methylpyridine (Compound No. 1-1)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 4-methoxyphenylboronic acid (95 mg, 0.63 mmol) to obtain 84 mg (yield: 73%) of the title compound as a white amorphous form. Melting point: 75 to 77°C.
¹H-NMR (400 MHz, CDCl₃) δ ppm: 11.46 (1H, brs), 7.82 (1H, s), 7.53-7.37 (6H, m), 7.32 (1H, d, J = 8.3 Hz), 6.94-6.89 (3H, m), 3.81 (3H, s), 3.10 (1H, m), 2.42 (3H, s), 1.28 (6H, d, J = 6.8 Hz).
MS(ES) m/z: 384 (M + H)⁺.

### (Example 5) 2-{2-Isopropyl-4-[4'-(trifluoromethyl)-1,1'-biphenyl-3-yl]-1H-imidazol-5-yl}-6-methylpyridine (Compound No. 1-493)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 4-(trifluoromethyl)phenylboronic acid (0.12 g, 0.63 mmol) to obtain 0.10 g (yield: 80%) of the title compound as a white powder. Melting point: 91 to 93°C.
¹H-NMR (500 MHz, CDCl₃) δ ppm: 10.75 (1H, brs), 7.90 (1H, s), 7.73-7.65 (5H, m), 7.56 (1H, d, J = 7.8 Hz), 7.48 (1H, t, J = 7.8 Hz), 7.41 (1H, t, J = 7.8 Hz), 7.31 (1H, d, J = 7.8 Hz), 6.94 (1H, d, J = 7.3 Hz), 3.15 (1H, m), 2.48 (3H, s), 1.36 (6H, m). MS(ES) m/z: 422 (M + H)⁺.

### (Example 6) 2-[4-(2'-Fluoro-1,1'-biphenyl-3-yl)-2-isopropyl-1H-imidazol-5-yl]-6-methylpyridine (Compound No. 1-427)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 2-fluorophenylboronic acid (90 mg, 0.64 mmol) to obtain 82 mg (yield: 74%) of the title compound as a white amorphous form.
Melting point: 153 to 155°C.
¹H-NMR (500 MHz, CDCl₃) δ ppm: 7.82 (1H, s), 7.65 (1H, d, J = 7.3 Hz), 7.52-7.38 (5H, m), 7.29 (1H, m), 7.20-7.10 (2H, m), 6.93 (1H, d, J = 7.3 Hz), 3.15 (1H, m), 2.48 (3H, s), 1.37 (6H, d, J = 6.8 Hz).
MS(ES) m/z: 372 (M + H)⁺.

### (Example 7) 2-[4-(3'-Fluoro-1,1'-biphenyl-3-yl)-2-isopropyl-1H-imidazol-5-yl]-6-methylpyridine (Compound No. 1-421)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 3-fluorophenylboronic acid (90 mg, 0.64 mmol) to obtain 75 mg (yield: 68%) of the title compound as a white powder. Melting point: 140 to 142°C.
¹H-NMR (500 MHz, CDCl₃) δ ppm: 10.75 (1H, brs), 7.87 (1H, s), 7.63 (1H, d, J = 7.3 Hz), 7.53 (1H, d, J = 7.8 Hz), 7.46 (1H, t, J = 7.8 Hz), 7.42-7.28 (5H, m), 7.02 (1H, m), 6.94 (1H, d, J = 7.8 Hz), 3.15 (1H, m), 2.47 (3H, s), 1.37-1.35 (6H, m). MS(ES) m/z: 372 (M + H)⁺.

### (Example 8) 2-[4-(4'-Fluoro-1,1'-biphenyl-3-yl)-2-isopropyl-1H-imidazol-5-yl]-6-methylpyridine (Compound No. 1-415)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 4-fluorophenylboronic acid (90 mg, 0.64 mmol) to obtain 89 mg (yield: 80%) of the title compound as a white amorphous form. Melting point: 83 to 85°C.
¹H-NMR (500 MHz, CDCl₃) δ ppm: 11.09 (1H, brs), 7.84 (1H, s), 7.59-7.54 (3H, m), 7.49 (1H, d, J = 7.8 Hz), 7.45-7.39 (2H, m), 7.32 (1H, d, J = 7.8 Hz), 7.09 (2H, m), 6.93 (1H, d, J = 7.3 Hz), 3.13 (1H, m), 2.43 (3H, s), 1.34-1.31 (6H, m).
MS(ES) m/z: 372 (M + H)⁺.

### (Example 9) 2-[4-(2',4'-Difluoro-1,1'-biphenyl-3-yl)-2-isopropyl-1H-imidazol-5-yl]-6-methylpyridine (Compound No. 1-496)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 2,4-difluorophenylboronic acid (98 mg, 0.62 mmol) to obtain 0.10 g (yield: 87%) of the title compound as a white amorphous form. Melting point: 70 to 72°C.
¹H-NMR (400 MHz, CDCl₃) δ ppm: 7.77 (1H, s), 7.65 (1H, m), 7.48-7.36 (5H, m), 6.95-6.86 (3H, m), 3.12 (1H, m), 2.43 (3H, s), 1.33-1.30 (6H, m).
MS(ES) m/z: 390 (M + H)⁺.

### (Example 10) 2-{2-Isopropyl-4-[4'-(trifluoromethoxy)-1,1'-biphenyl-3-yl]-1H-imidazol-5-yl}-6-methylpyridine (Compound No. 1-499)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 4-(trifluoromethoxy)phenylboronic acid (0.13 g, 0.61 mmol) to obtain 90 mg (yield: 69%) of the title compound as a white powder. Melting point: 120 to 122°C.
¹H-NMR (500 MHz, CDCl₃) δ ppm: 10.35 (1H, brs), 7.85 (1H, s), 7.63-7.60 (3H, m), 7.52 (1H, d, J = 7.8 Hz), 7.46 (1H, t, J = 7.8 Hz), 7.39 (1H, t, J = 7.8 Hz), 7.30 (1H, d, J = 7.8 Hz), 7.25 (2H, d, J = 8.3 Hz), 6.93 (1H, d, J = 7.3 Hz), 3.16 (1H, m), 2.52 (3H, s), 1.40 (6H, d, J = 6.8 Hz).
MS(ES) m/z: 438 (M + H)⁺.

### (Example 11) 2-[2-Isopropyl-4-(3-pyridin-3-ylphenyl)-1H-imidazol-5-yl]-6-methylpyridine (Compound No. 1-481)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and pyridine-3-boronic acid-1,3-propanediol ester (0.10 g, 0.61 mmol) to obtain 0.10 g (yield: 94%) of the title compound as a white amorphous form.
Melting point: 74 to 77°C.
¹H-NMR (400 MHz, CDCl₃) δ ppm: 10.54 (1H, brs), 8.82 (1H, d, J = 2.0 Hz), 8.54 (1H, dd, J = 1.6, 4.7 Hz), 7.88-7.85 (2H, m), 7.65 (1H, d, J = 7.3 Hz), 7.53-7.45 (2H, m), 7.38 (1H, t, J = 7.8 Hz), 7.34-7.28 (2H, m), 6.92 (1H, d, J = 7.3 Hz), 3.15 (1H, m), 2.49 (3H, s), 1.38 (6H, d, J = 6.8 Hz).
MS(ES) m/z: 355 (M + H)⁺.

### (Example 12) 2-[2-Isopropyl-4-(3-pyridin-4-ylphenyl)-1H-imidazol-5-yl]-6-methylpyridine (Compound No. 1-409)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and pyridine-4-boronic acid pinacol ester (0.13 g, 0.61 mmol) to obtain 82 mg (yield: 78%) of the title compound as a white amorphous form.
Melting point: 84 to 86°C.
¹H-NMR (500 MHz, CDCl₃) δ ppm: 10.65 (1H, brs), 8.63 (2H, d, J = 5.9 Hz), 7.95 (1H, s), 7.70 (1H, d, J = 7.8 Hz), 7.60 (1H, d, J = 7.8 Hz), 7.53-7.49 (3H, m), 7.40 (1H, t, J = 7.8 Hz), 7.29 (1H, d, J = 7.8 Hz), 6.95 (1H, d, J = 7.8 Hz), 3.16 (1H, m), 2.49 (3H, s), 1.38 (6H, d, J = 6.3 Hz).
MS(ES) m/z: 355 (M + H)⁺.

### (Example 13) 5-{3-[2-Isopropyl-5-(6-methylpyridin-2-yl)-1H-imidazol-4-yl]phenyl}-2-methoxypyridine (Compound No. 1-403)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 2-methoxy-5-pyridineboronic acid (98 mg, 0.64 mmol) to obtain 83 mg (yield: 72%) of the title compound as a white amorphous form.
Melting point: 72 to 74°C.
¹H-NMR (500 MHz, CDCl₃) δ ppm: 11.22 (1H, brs), 8.38 (1H, m), 7.82-7.80 (2H, m), 7.61 (1H, d, J = 6.8 Hz), 7.49-7.40 (3H, m), 7.33 (1H, d, J = 8.3 Hz), 6.93 (1H, d, J = 7.8 Hz), 6.79 (1H, d, J = 8.3 Hz), 3.97 (3H, s), 3.13 (1H, m), 2.42 (3H, s), 1.33-1.30 (6H, m).
MS(ES) m/z: 385 (M + H)⁺.

### (Example 14) 2-[2-Isopropyl-4-(3-thien-3-ylphenyl)-1H-imidazol-5-yl]-6-methylpyridine (Compound No. 1-397)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 3-thiopheneboronic acid (80 mg, 0.63 mmol) to obtain 91 mg (yield: 84%) of the title compound as a white amorphous form.
Melting point: 83 to 85°C.
¹H-NMR (500 MHz, CDCl₃) δ ppm: 10.95 (1H, brs), 7.89 (1H, s), 7.55 (2H, m), 7.44-7.30 (6H, m), 6.93 (1H, d, J = 7.3 Hz), 3.14 (1H, m), 2.45 (3H, s), 1.36-1.33 (6H, m).
MS(ES) m/z: 360 (M + H)⁺.

### (Example 15) N-{3'-[2-Isopropyl-5-(6-methylpyridin-2-yl)-1H-imidazol-4-yl]-1,1'-biphenyl-4-yl}methanesulfonamide (Compound No. 1-121)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and (4-methylsulfonyl)aminophenylboronic acid (0.13 g, 0.61 mmol) to obtain 60 mg (yield: 45%) of the title compound as a white amorphous form.
Melting point: 128 to 130°C.
¹H-NMR (400 MHz, CDCl₃) δ ppm: 8.91 (1H, brs), 7.79 (1H, s), 7.56 (1H, d, J = 7.3 Hz), 7.48 (1H, d, J = 7.3 Hz), 7.44-7.39 (4H, m), 7.30 (1H, d, J = 7.8 Hz), 7.24 (2H, d, J = 8.3 Hz), 6.95 (1H, d, J = 7.3 Hz), 3.21 (1H, m), 2.68 (3H, s), 2.50 (3H, s), 1.41 (6H, d, J = 6.9 Hz).
MS(ES) m/z: 447 (M + H)⁺.

### (Example 16) 2-{2-Isopropyl-4-[4'-(methylthio)-1,1'-biphenyl-3-yl]-1H-imidazol-5-yl}-6-methylpyridine (Compound No. 1-49)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 4-(methylthio)phenylboronic acid (0.10 g, 0.60 mmol) to obtain 87 mg (yield: 72%) of the title compound as a white amorphous form.
Melting point: 80 to 82°C.
¹H-NMR (500 MHz, CDCl₃) δ ppm: 10.79 (1H, brs), 7.86 (1H, s), 7.59-7.29 (9H, m), 6.92 (1H, d, J = 7.3 Hz), 3.14 (1H, m), 2.51 (3H, s), 2.46 (3H, s), 1.35 (6H, d, J = 6.8 Hz).
MS(ES) m/z: 400 (M + H)⁺.

### (Example 17) 2-{2-Isopropyl-4-[4'-(methylsulfonyl)-1,1'-biphenyl-3-yl]-1H-imidazol-5-yl}-6-methylpyridine (Compound No. 1-97)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 4-methylsulfonylphenylboronic acid (0.13 g, 0.63 mmol) to obtain 80 mg (yield: 62%) of the title compound as a white amorphous form.
Melting point: 104 to 106°C.
¹H-NMR (500 MHz, CDCl₃) δ ppm: 10.53 (1H, brs), 7.98 (2H, d, J = 7.8 Hz), 7.92 (1H, s), 7.79 (2H, d, J = 7.8 Hz), 7.69 (1H, d, J = 7.3 Hz), 7.57 (1H, d, J = 6.8 Hz), 7.50 (1H, t, J = 7.8 Hz), 7.41 (1H, t, J = 7.8 Hz), 7.30 (1H, d, J = 7.8 Hz), 6.95 (1H, d, J = 7.3 Hz), 3.16 (1H, m), 3.09 (3H, s), 2.50 (3H, s), 1.40-1.37 (6H, m).
MS(ES) m/z: 432 (M + H)⁺.

### (Example 18) 2-{2-Isopropyl-4-[3'-(methylsulfonyl)-1,1'-biphenyl-3-yl]-1H-imidazol-5-yl}-6-methylpyridine (Compound No. 1-502)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 3-methylsulfonylphenylboronic acid (0.12 g, 0.60 mmol) to obtain 0.12 g (yield: 93%) of the title compound as a white powder.
Melting point: 97 to 100°C.
¹H-NMR (500 MHz, CDCl₃) δ ppm: 10.60 (1H, brs), 8.18 (1H, s), 7.93 (1H, s), 7.91-7.89 (2H, m), 7.67 (1H, d, J = 7.3 Hz), 7.63 (1H, t, J = 7.8 Hz), 7.58 (1H, d, J = 7.8 Hz), 7.49 (1H, t, J = 7.8 Hz), 7.43 (1H, t, J = 7.8 Hz), 7.30 (1H, d, J = 7.8 Hz), 6.96 (1H, d, J = 7.8 Hz), 3.16 (1H, m), 3.09 (3H, s), 2.50 (3H, s), 1.39 (6H, d, J = 6.8 Hz). MS(ES) m/z: 432 (M + H)⁺.

### (Example 19) N-{3'-[2-Isopropyl-5-(6-methylpyridin-2-yl)-1H-imidazol-4-yl]-1,1'-biphenyl-3-yl}methanesulfonamide (Compound No. 1-457)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and (3-methylsulfonyl)aminophenylboronic acid (0.14 g, 0.63 mmol) to obtain 0.13 g (yield: 94%) of the title compound as a white amorphous form.
Melting point: 108 to 112°C.
¹H-NMR (500 MHz, CDCl₃) δ ppm: 7.83 (1H, s), 7.56 (1H, m), 7.46 (1H, m), 7.41-7.22 (7H, m), 6.94 (1H, d, J = 7.3 Hz), 3.19 (1H, m), 2.92 (3H, s), 2.52 (3H, s), 1.41 (6H, d, J = 6.8 Hz).
MS(ES) m/z: 447 (M + H)⁺.

### (Example 20) 2-[2-Isopropyl-4-(3-thien-2-ylphenyl)-1H-imidazol-5-yl]-6-methylpyridine (Compound No. 1-469)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 2-thiopheneboronic acid (0.12 g, 0.90 mmol) to obtain 80 mg (yield: 74%) of the title compound as a white amorphous form.
Melting point: 73 to 76°C.
¹H-NMR (500 MHz, CDCl₃) δ ppm: 10.85 (1H, brs), 7.90 (1H, s), 7.58 (1H, d, J = 7.8 Hz), 7.54 (1H, d, J = 7.8 Hz), 7.41-7.38 (2H, m), 7.32-7.30 (2H, m), 7.26 (1H, m), 7.05 (1H, m), 6.93 (1H, d, J = 7.3 Hz), 3.15 (1H, m), 2.46 (3H, s), 1.35 (6H, d, J = 6.8 Hz).
MS(ES) m/z: 360 (M + H)⁺.

### (Example 21) 2-{4-[3-(3-Furyl)phenyl]-2-isopropyl-1H-imidazol-5-yl}-6-methylpyridine (Compound No. 1-397)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 3-furanboronic acid (0.10 g, 0.89 mmol) to obtain 85 mg (yield: 83%) of the title compound as a white amorphous form.
Melting point: 74 to 78°C.
¹H-NMR (500 MHz, CDCl₃) δ ppm: 7.78 (1H, s), 7.72 (1H, s), 7.50 (1H, d, J = 7.3 Hz), 7.46-7.44 (2H, m), 7.41-7.37 (2H, m), 7.30 (1H, d, J = 8.3 Hz), 6.93 (1H, d, J = 7.8 Hz), 6.70 (1H, s), 3.15 (1H, m), 2.47 (3H, s), 1.36 (6H, d, J = 6.8 Hz).
MS(ES) m/z: 344 (M + H)⁺.

### (Example 22) 5-{3-[2-Isopropyl-5-(6-methylpyridin-2-yl)-1H-imidazol-4-yl]phenyl}thiophene-2-carboxyamide (Compound No. 1-475)

2-[4-(3-Bromophenyl)-2-isopropyl-1H-imidazol-5-yl]-6-methylpyridine (0.11 g, 0.30 mmol) obtained in Example (1b), 5-cyanothiophene-2-boronic acid (0.10 g, 0.65 mmol), tripotassium phosphate n-hydrate (0.15 g, 0.57 mmol), 2-(dicyclohexylphosphino)-2',6'-dimethoxy-1,1'-biphenyl (6.5 mg, 0.016 mmol), and tris(dibenzylideneacetone)dipalladium(0) (15 mg, 0.016 mmol) were dissolved in 1,2-dimethoxyethane (2 mL). The resulting mixture was stirred at 80°C for 3 hr, and then the reaction mixture was diluted with ethyl acetate and filtered through silica gel. The crude product obtained by concentrating the filtrate under reduced pressure was purified by high-performance liquid chromatography (GL Science ODS-3, eluting solvent; water : acetonitrile = 95 : 5 to 5 : 95). The resulting compound (88 mg) was dissolved in 1,4-dioxane (3 mL), and potassium trimethylsiloxide (90 mg, 0.70 mmol) was added thereto. The resulting mixture was heated under reflux for 2 hr. The reaction mixture was cooled to room temperature, and a saturated ammonium chloride aqueous solution was added to the reaction mixture to terminate the reaction. To the resulting crude product, water and acetonitrile were added thereto. The mixture was purified by high-performance liquid chromatography (GL Science ODS-3, eluting solvent; water : acetonitrile = 95 : 5 to 5 : 95) to obtain 38 mg (yield: 32%) of the title compound as a light yellow powder.
Melting point: 125 to 128°C.
¹H-NMR (500 MHz, CDCl₃) δ ppm: 7.92 (1H, s), 7.60 (1H, d, J = 7.8 Hz), 7.58 (1H, d, J = 7.8 Hz), 7.49 (1H, d, J = 3.4 Hz), 7.43 (1H, d, J = 7.8 Hz), 7.40 (1H, d, J = 7.8 Hz), 7.30-7.26 (2H, m), 6.95 (1H, d, J = 7.8 Hz), 3.18 (1H, m), 2.53 (3H, s), 1.41 (6H, d, J = 6.8 Hz).
MS(ES) m/z: 403 (M + H)⁺.

### (Example 23) 2-{2-Isopropyl-4-[3-(1H-pyrrol-2-yl)phenyl]-1H-imidazol-5-yl}-6-methylpyridine (Compound No. 1-289)

2-[4-(3-Bromophenyl)-2-isopropyl-1H-imidazol-5-yl]-6-methylpyridine (0.18 g, 0.50 mmol) obtained in Example (1b), 1-(t-butoxycarbonyl)pyrrole-2-boronic acid (0.21 g, 1.0 mmol), tripotassium phosphate n-hydrate (0.25 g, 1.2 mmol), and dichloro [1,1'-bis(diphenylphosphino)ferrocene]palladium(II)-dichloromethane complex (40 mg, 0.050 mmol) were dissolved in 1,2-dimethoxyethane (2 mL). The resulting mixture was stirred at 80°C for 2 hr. The reaction mixture was diluted with ethyl acetate and then filtered through silica gel. The crude product obtained by concentrating the filtrate under reduced pressure was dissolved in tetrahydrofuran (2 mL), and a sodium methoxide solution (25% methanol solution, 0.60 mL, 2.8 mmol) was added thereto. The resulting mixture was stirred at room temperature for 30 min. Water and methylene chloride were added to the reaction solution, and the organic layer was extracted using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.16 g (yield: 91%) of the title compound as a white powder.
Melting point: 103 to 107°C.
¹H-NMR (500 MHz, CDCl₃) δ ppm: 9.08 (1H, brs), 7.80 (1H, s), 7.45 (1H, d, J = 7.8 Hz), 7.40-7.37 (2H, m), 7.32 (2H, t, J = 7.8 Hz), 6.93 (1H, d, J = 7.3 Hz), 6.82 (1H, d, J = 1.5 Hz), 6.49 (1H, s), 6.26 (1H, m), 3.10 (1H, m), 2.46 (3H, s), 1.31 (6H, d, J = 6.8 Hz).
MS(ES) m/z: 343 (M + H)⁺.

### (Example 24) 1-{3'-[2-Isopropyl-5-(6-methylpyridin-2-yl)-1H-imidazol-4-yl]-1,1'-biphenyl-4-yl}ethanone (Compound No.1-505)

The same reaction as in Example 3 was carried out using 2-[4-(3-bromophenyl)-2-isopropyl-1H-imidazol-5-yl]-6-methylpyridine (0.11 g, 0.30 mmol) obtained in Example (1b) and 4-acetylphenylboronic acid (0.10 g, 0.61 mmol) to obtain 0.11 g (yield: 93%) of the title compound as a white amorphous form.
Melting point: 84 to 87°C.
¹H-NMR (500 MHz, CDCl₃) δ ppm: 8.01 (2H, d, J = 7.8 Hz), 7.93 (1H, s), 7.70 (2H, d, J = 7.8 Hz), 7.66 (1H, d, J = 7.8 Hz), 7.59 (1H, d, J = 7.3 Hz), 7.48 (1H, t, J = 7.8 Hz), 7.40 (1H, t, J = 7.8 Hz), 7.31 (1H, d, J = 7.8 Hz), 6.94 (1H, d, J = 7.3 Hz), 3.16 (1H, m), 2.63 (3H, s), 2.48 (3H, s), 1.37 (6H, d, J = 6.8 Hz).
MS(ES) m/z: 396 (M + H)⁺.

### (Example 25) 2-{2-Isopropyl-4-[4'-(ethylsulfonyl)-1,1'-biphenyl-3-yl]-1H-imidazol-5-yl}-6-methylpyridine (Compound No. 1-337)

The same reaction as in Example (1c) was carried out using 2-[4-(3-bromophenyl)-2-isopropyl-1H-imidazol-5-yl]-6-methylpyridine (178 mg, 0.50 mmol) obtained in Example (1b) and 4-ethylsulfonylphenylboronic acid (128 mg, 0.60 mmol) to obtain 20 mg (yield: 10%) of the title compound as a white powder.
Melting point: 103 to 116°C.
¹H-NMR (400 MHz, CDCl₃) δ ppm: 7.91 (3H, brs), 7.76 (2H, d, J = 7.8 Hz), 7.64 (1H, d, J = 7.0 Hz), 7.54 (1H, d, J = 7.4 Hz), 7.48-7.40 (2H, m), 7.29 (1H, d, J = 7.8 Hz), 6.94 (1H, d, J = 7.8 Hz), 3.17-3.08 (3H, m), 2.40 (3H, s), 1.29 (9H, m). MS(ES) m/z: 446 (M + H)⁺.

### (Example 26) 2-{2-Isopropyl-4-[4'-(methylsulfoxy)-1,1'-biphenyl-3-yl]-1H-imidazol-5-yl}-6-methylpyridine (Compound No. 1-73)

2-{2-Isopropyl-4-[4'-(methylthio)-1,1'-biphenyl-3-yl]-1H-imidazol-5-yl}-6-methylpyridine (110 mg, 0.27 mmol) obtained in Example 16 and sodium periodate (128 mg, 0.6 mmol) were suspended in a mixture solution of methanol (3 mL) and water (1 mL). The suspension was stirred at room temperature for 3 hr. The solvent was evaporated under reduced pressure, and the resulting crude product was purified by high-performance liquid chromatography (GL Science ODS-3, eluting solvent; water : acetonitrile = 95 : 5 to 5 : 95) to obtain 61 mg (yield: 54%) of the title compound as a white powder.
Melting point: 95 to 105°C.
¹H-NMR (400 MHz, CDCl₃) δ ppm: 10.84 (1H, brs), 7.88 (1H, s), 7.73 (2H, d, J = 8.4 Hz), 7.67 (2H, d, J = 8.4 Hz), 7.63 (1H, d, J = 7.8 Hz), 7.54 (1H, d, J = 7.8 Hz), 7.46 (1H, d, J = 7.8 Hz), 7.39 (1H, t, J = 7.8 Hz), 7.28 (1H, d, J = 7.8 Hz), 6.93 (1H, d, J = 7.8 Hz), 3.14 (1H, m), 2.75 (3H, s), 2.46 (3H, s), 1.35 (6H, d, J = 7.0 Hz). MS(ES) m/z: 416 (M + H)⁺.

### (Example 27) 2-[2-Isopropyl-4-(4'-nitro-1,1'-biphenyl-3-yl)-1H-imidazol-5-yl]-6-methylpyridine (Compound No. 1-439)

The same reaction as in Example (1c) was carried out using 2-[4-(3-bromophenyl)-2-isopropyl-1H-imidazol-5-yl]-6-methylpyridine (178 mg, 0.50 mmol) obtained in Example (1b) and 4-nitrophenylboronic acid (92 mg, 0.55 mmol) to obtain 142 mg (yield: 72%) of the title compound as a yellow solid.
Melting point: 95 to 105°C.
¹H-NMR (400 MHz, CDCl₃) δ ppm: 8.25 (2H, d, J = 8.6 Hz), 7.91 (1H, s), 7.73 (2H, d, J = 8.6 Hz), 7.68 (1H, d, J = 7.8 Hz), 7.57 (1H, d, J = 7.8 Hz), 7.49 (1H, t, J = 7.8 Hz), 7.37 (1H, t, J = 7.8 Hz), 7.27 (1H, t, d = 7.8 Hz), 6.94 (1H, d, J = 7.8 Hz), 3.16 (1H, m), 2.55 (3H, s), 1.43 (6H, d, J = 7.0 Hz).
MS(ES) m/z: 399 (M + H)⁺.

### (Example 28) 2-[2-Isopropyl-4-(4'-amino-1,1'-biphenyl-3-yl)-1H-imidazol-5-yl]-6-methylpyridine (Compound No. 1-25)

To a methanol solution (10 mL) of 2-[2-isopropyl-4-(4'-nitro-1,1'-biphenyl-3-yl)-1H-imidazol-5-yl]-6-methylpyridine (135 mg, 0.34 mmol) obtained in Example 27, 10% palladium-carbon (50 mg) was added. The resulting mixture was stirred under a hydrogen atmosphere at room temperature for 3 hr. The insoluble matter was removed by filtration, and the solvent of the resulting mother liquid was evaporated under reduced pressure. The resulting crude product was purified by high-performance liquid chromatography (GL Science ODS-3, eluting solvent; water : acetonitrile = 95 : 5 to 5 : 95) to obtain 110 mg (yield: 88%) of the title compound as a white powder.
Melting point: 107 to 117°C.
¹H-NMR (400 MHz, CDCl₃) δ ppm: 7.78 (1H, s), 7.49-7.47 (2H, m), 7.42-7.29 (5H, m), 6.89 (1H, d, J = 7.0 Hz), 6.70 (2H, d, J = 8.6 Hz), 3.15 (1H, m), 2.50 (3H, s), 1.38 (6H, d, J = 7.0 Hz).
MS(ES) m/z: 369 (M + H)⁺.

### (Example 29) 1-{3'-[2-Isopropyl-5-(6-methylpyridin-2-yl)-1H-imidazol-4-yl]-1,1'-biphenyl-4-yl}methanamine (Compound No. 1-508)

The same reaction as in Example (1c) was carried out using 2-[4-(3-bromophenyl)-2-isopropyl-1H-imidazol-5-yl]-6-methylpyridine (712 mg, 2.0 mmol) obtained in Example (1b) and (4-aminomethylphenyl)boronic acid hydrochloride (450 mg, 2.4 mmol) to obtain 208 mg (yield: 27%) of the title compound as a brown solid.
Melting point: 113 to 123°C (decomposition).
¹H-NMR (400 MHz, CDCl₃) δ ppm: 7.84 (1H, s), 7.57-7.50 (4H, m), 7.43 (1H, t, J = 7.8 Hz), 7.37-7.29 (4H, m), 6.91 (1H, d, J = 6.6 Hz), 3.89 (2H, s), 3.17 (1H, m), 2.54 (3H, s), 1.43 (6H, d, J = 7.0 Hz).
MS(ES) m/z: 383 (M + H)⁺.

### (Example 30) N-{3'-[2-Isopropyl-5-(6-methylpyridin-2-yl)-1H-imidazol-4-yl]-1,1'-biphenyl-4-yl}ethanesulfonamide (Compound No. 1-145)

Pyridine (40 µL, 0.5 mmol) and ethanesulfonyl chloride (20 µL) were added to a methylene chloride solution (1 mL) of 2-[2-isopropyl-4-(4'-amino-1,1'-biphenyl-3-yl)-1H-imidazol-5-yl]-6-methylpyridine (18 mg, 0.050 mmol) obtained in Example 28. The resulting mixture was stirred at room temperature overnight. Saturated aqueous sodium bicarbonate was added thereto to terminate the reaction, and the organic layer was extracted using an Empore cartridge. The solvent was evaporated under reduced pressure, and the resulting crude product was purified by high-performance liquid chromatography (GL Science ODS-3, eluting solvent; water : acetonitrile = 95 : 5 to 5 : 95) to obtain 16 mg (yield: 71%) of the title compound as a white powder.
Melting point: 123 to 135°C.
¹H-NMR (400 MHz, CDCl₃) δ ppm: 7.92 (1H, brs), 7.78 (1H, s), 7.54 (1H, d, J = 7.4 Hz), 7.48-7.34 (5H, m), 7.31-7.21 (3H, m), 6.92 (1H, d, J = 7.8 Hz), 3.19 (1H, m), 2.98 (2H, q, J = 7.2 Hz), 2.48 (3H, s), 1.38 (6H, d, J = 7.0 Hz), 1.26 (3H, t, J = 7.2 Hz).
MS(ES) m/z: 461 (M + H)⁺.

### (Example 31) N-{3'-[2-Isopropyl-5-(6-methylpyridin-2-yl)-1H-imidazol-4-yl]-1,1'-biphenyl-4-yl}cyclopropylsulfonamide (Compound No. 1-169)

The same reaction as in Example 30 was carried out using 2-[2-isopropyl-4-(4'-amino-1,1'-biphenyl-3-yl)-1H-imidazol-5-yl]-6-methylpyridine (18 mg, 0.050 mmol) obtained in Example 28 and cyclopropylsulfonyl chloride (20 µL) to obtain 7 mg (yield: 30%) of the title compound as a yellow solid.
Melting point: 135 to 145°C.
¹H-NMR (400 MHz, CDCl₃) δ ppm: 7.84 (1H, brs), 7.58 (1H, d, J = 7.4 Hz), 7.52-7.25 (8H, m), 6.94 (1H, d, J = 7.4 Hz), 3.20 (1H, m), 2.51 (3H, s), 2.38 (1H, brs), 1.40 (6H, d, J = 7.0 Hz), 1.12 (2H, brs), 0.87 (2H, brd, J = 7.4 Hz).
MS(ES) m/z: 473 (M + H)⁺.

### (Example 32) N- {3'-[2-Isopropyl-5-(6-methylpyridin-2-yl)-1H-imidazol-4-yl]-1,1'-biphenyl-4-yl}acetamide (Compound No. 1-343)

The same reaction as in Example 30 was carried out using 2-[2-isopropyl-4-(4'-amino-1,1'-biphenyl-3-yl)-1H-imidazol-5-yl]-6-methylpyridine (18 mg, 0.050 mmol) obtained in Example 28 and acetic anhydride (20 µL) to obtain 14 mg (yield: 68%) of the title compound as a white powder.
Melting point: 140 to 145°C.
¹H-NMR (400 MHz, CDCl₃) δ ppm: 8.14 (1H, brs), 7.77 (1H, s), 7.54-7.28 (9H, m), 6.91 (1H, d, J = 7.8 Hz), 3.15 (1H, m), 2.48 (3H, s), 2.08 (3H, s), 1.37 (6H, d, J = 7.0 Hz).
MS(ES) m/z: 411 (M + H)⁺.

### (Example 33) 2-({3'-[2-Isopropyl-5-(6-methylpyridin-2-yl)-1H-imidazol-4-yl]-1,1'-biphenyl-4-yl}amino)-1,1'-dimethyl-2-oxoethyl acetate (Compound No. 1-355)

The same reaction as in Example 30 was carried out using 2-[2-isopropyl-4-(4'-amino-1,1'-biphenyl-3-yl)-1H-imidazol-5-yl]-6-methylpyridine (18 mg, 0.050 mmol) obtained in Example 28 and 2-acetoxybutyryl chloride (20 µL) to obtain 15 mg (yield: 68%) of the title compound as a yellow solid.
Melting point: 110 to 117°C ¹H-NMR (400 MHz, CDCl₃) δ ppm: 7.90 (1H, brs), 7.85 (1H, brs), 7.58 (4H, s), 7.53 (1H, d, J = 7.4 Hz), 7.44 (1H, t, J = 7.8 Hz), 7.40 (1H, t, J = 7.8 Hz), 7.32 (1H, d, J = 7.8 Hz), 6.93 (1H, d, J = 7.4 Hz), 3.15 (1H, m), 2.46 (3H, s), 2.16 (3H, s), 1.75 (6H, s), 1.35 (6H, d, J = 7.0 Hz).
MS(ES) m/z: 497 (M + H)⁺.

### (Example 34) 2-Hydroxy-N-{3'-[2-isopropyl-5-(6-methylpyridin-2-yl)-1H-imidazol-4-yl]-1,1'-biphenyl-4-yl}-2-methylpropanamide (Compound No. 1-349)

A methanol solution (1 mL) of 2-({3'-[2-isopropyl-5-(6-methylpyridin-2-yl)-1H-imidazol-4-yl]-1,1'-biphenyl-4-yl}amino)-1,1-dimethyl-2-oxoethyl acetate (12 mg, 0.024 mmol) obtained in Example 33 and potassium hydroxide (56 mg, 1.0 mmol) was heated under reflux for 1 hr. The solvent was evaporated under reduced pressure, and the resulting crude product was purified by high-performance liquid chromatography (GL Science ODS-3, eluting solvent; water : acetonitrile = 95 : 5 to 5 : 95) to obtain 12 mg (yield: 100%) of the title compound as a white powder. Melting point: 123 to 135°C.
¹H-NMR (400 MHz, CDCl₃) δ ppm: 8.74 (1H, s), 7.83 (1H, s), 7.52 (6H, brs), 7.40 (1H, t, J = 7.8 Hz), 7.37 (1H, t, J = 7.8 Hz), 7.28 (1H, d, J = 7.8 Hz), 6.91 (1H, d, J = 7.0 Hz), 3.17 (1H, m), 2.47 (3H, s), 1.48 (6H, s), 1.37 (6H, d, J = 7.0 Hz).
MS(ES) m/z: 455 (M + H)⁺.

### (Example 35) 5-{3-[2-Isopropyl-4-(6-methylpyridin-2-yl)-1H-imidazol-5-yl]phenyl}pyridine-2-carboxyamide (Compound No. 1-487)

### (35a) 2-{2-Isopropyl-5-[3-(4,4,5,5-tetramethyl-1,3,2-dioxaborolan-2-yl)phenyl]-1H-imidazol-4-yl}-6-methylpyridine

2-[4-(3-Bromophenyl)-2-isopropyl-1H-imidazol-5-yl]-6-methylpyridine (1.8 g, 5.0 mmol) obtained in Example (1b) bis(pinacolato)diboron (1.5 g, 6.0 mmol), potassium acetate (1.5 g, 15 mmol), and dichloro[1,1'-bis(diphenylphosphino)ferrocene]palladium(II)-dichloromethane complex (0.21 g, 0.25 mmol) were dissolved in N,N-dimethylformamide (15 mL). The resulting mixture was stirred while heating under a nitrogen atmosphere at 90°C for 18 hr. The reaction mixture was diluted with ethyl acetate and filtered through Celite. The filtrate was concentrated under reduced pressure, and the resulting crude product was purified by silica gel column chromatography (eluting solvent; ethyl acetate) to obtain 2.1 g (yield: 100%) of the title compound as a yellow amorphous form.
¹H-NMR (400 MHz, CDCl₃) δ ppm: 8.10 (1H, s), 7.78 (1H, dt, J = 1.2, 7.4 Hz), 7.67 (1H, dt, J = 1.6, 7.8 Hz), 7.38 (1H, t, J = 7.4 Hz), 7.33 (1H, t, J = 7.4 Hz), 7.21 (1H, m), 6.90 (1H, d, J = 7.4 Hz), 3.17 (1H, m), 2.54 (3H, s), 1.42 (6H, d, J = 7.0 Hz), 1.26 (12H, s).
MS(ES) m/z: 404 (M + H)⁺.

### (35b) 5-{3-[2-Isopropyl-4-(6-methylpyridin-2-yl)-1H-imidazol-5-yl]phenyl}pyridine-2-carboxyamide

2-{2-Isopropyl-5-[3-(4,4,5,5-tetramethyl-1,3,2-dioxaborolan-2-yl)phenyl]-1H-imidazol-4-yl}-6-methylpyridine (0.12 g, 0.30 mmol) obtained in Example (35a), 2-cyano-5-bromopyridine (85 mg, 0.46 mmol), tripotassium phosphate n-hydrate (0.19 g, 0.72 mmol), and dichloro[1,1'-bis(diphenylphosphino)ferrocene]palladium(II)-dichloromethane complex (25 mg, 0.030 mmol) were dissolved in 1,2-dimethoxyethane (2 mL). The resulting mixture was stirred at 80°C for 5 hr. The reaction mixture was diluted with ethyl acetate and filtered through silica gel. The filtrate was concentrated under reduced pressure, and the resulting crude product was purified by high-performance liquid chromatography (GL Science ODS-3, eluting solvent; water : acetonitrile = 95 : 5 to 5 : 95). The resulting compound was dissolved in 1,4-dioxane (3 mL), and potassium trimethylsiloxide (0.12 g, 0.90 mmol) was added thereto. The resulting mixture was heated under reflux for 4 hr. The reaction mixture was cooled to room temperature, and then a saturated ammonium chloride aqueous solution was added thereto to terminate the reaction. To the resulting crude product, water and acetonitrile were added. The resulting mixture was purified by high-performance liquid chromatography (GL Science ODS-3, eluting solvent; water : acetonitrile = 95 : 5 to 5 : 95) to obtain 50 mg (yield: 42%) of the title compound as a white powder.
Melting point: 108 to 112°C.
¹H-NMR (500 MHz, CDCl₃) δ ppm: 8.81 (1H, d, J = 1.9 Hz), 8.24 (1H, d, J = 8.3 Hz), 8.06 (1H, dd, J =1.9, 8.3 Hz), 7.94 (1H, s), 7.86 (1H, brs), 7.71 (1H, d, J = 7.3 Hz), 7.58 (1H, d, J = 7.3 Hz), 7.52 (1H, t, J = 7.8 Hz), 7.42 (1H, t, J = 7.8 Hz), 7.31 (1H, d, J = 7.8 Hz), 6.96 (1H, d, J = 7.3 Hz), 5.70 (1H, brs), 3.17 (1H, m), 2.51 (3H, s), 1.39 (6H, d, J = 7.3 Hz).
MS(ES) m/z: 398 (M + H)⁺.

### (Example 36) 6-{3-[2-Isopropyl-5-(6-methylpyridin-2-yl)-1H-imidazol-4-yl]phenyl}nicotinamide (Compound No. 1-313)

2-{2-Isopropyl-5-[3-(4,4,5,5-tetramethyl-1,3,2-dioxaborolan-2-yl)phenyl]-1H-imidazol-4-yl}-6-methylpyridine (0.12 g, 0.30 mmol) obtained in Example (35a), 6-chloronicotinamide (75 mg, 0.48 mmol), tripotassium phosphate n-hydrate (0.19 g, 0.72 mmol), and dichloro[1,1'-bis(diphenylphosphino)ferrocene]palladium(II)-dichloromethane complex (25 mg, 0.030 mmol) were dissolved in 1,2-dimethoxyethane (3 mL). The resulting mixture was stirred at 80°C for 3 hr. The reaction mixture was diluted with ethyl acetate and filtered through silica gel. The filtrate was concentrated under reduced pressure, and the resulting crude product was purified by high-performance liquid chromatography (GL Science ODS-3, eluting solvent; water : acetonitrile = 95 : 5 to 5 : 95) to obtain 36 mg (yield: 30%) of the title compound as a white powder.
Melting point: 138 to 144°C.
¹H-NMR (500 MHz, CDCl₃) δ ppm: 9.05 (1H, d, J = 2.0 Hz), 8.26 (1H, s), 8.16 (1H, dd, J = 2.4, 8.3 Hz), 7.99 (1H, d, J = 7.8 Hz), 7.75 (1H, d, J = 7.8 Hz), 7.65 (1H, d, J = 7.8 Hz), 7.47 (1H, t, J = 7.8 Hz), 7.37 (1H, t, J = 7.8 Hz), 7.25 (1H, m), 6.93 (1H, d, J = 7.3 Hz), 6.83 (1H, brs), 5.99 (1H, brs), 3.17 (1H, m), 2.50 (3H, s), 1.39 (6H, d, J = 7.3 Hz).
MS(ES) m/z: 398 (M + H)⁺.

### (Example 37) 3'-[2-Isopropyl-4-(6-methylpyridin-2-yl)-1H-imidazol-5-yl]-1,1'-biphenyl-4-sulfonamide (Compound No. 1-193)

The same reaction as in Example 36 was carried out using 2-{2-isopropyl-5-[3-(4,4,5,5-tetramethyl-1,3,2-dioxaborolan-2-yl)phenyl]-1H-imidazol-4-yl}-6-methylpyridine (0.12 g, 0.30 mmol) obtained in Example (35a) and 4-bromobenzenesulfonamide (0.18 g, 0.76 mmol) to obtain 0.14 g (yield: 64%) of the title compound as a white powder.
Melting point: 255 to 258°C.
¹H-NMR (400 MHz, DMSO-d₆) δ ppm: 12.05 (1H, s), 8.09 (0.5H, s), 7.92 (0.5H, s), 7.91-7.85 (3H, m), 7.77-7.71 (2H, m), 7.63-7.57 (3H, m), 7.47 (0.5H, t, J = 7.8 Hz), 7.44 (0.5H, t, J = 7.8 Hz), 7.36-7.22 (2H, m), 7.23 (0.5H, d, J = 7.8 Hz), 7.11 (0.5H, d, J = 7.8 Hz), 3.10 (1H, m), 2.49 (3H, s), 1.34-1.30 (6H, m).
MS(ES) m/z: 433 (M + H)⁺.

### (Example 38) 3'-[2-Isopropyl-5-(6-methylpyridin-2-yl)-1H-imidazol-4-yl]-N-methyl-1,1'-biphenyl-4-sulfonamide (Compound No. 1-379)

Saturated aqueous sodium bicarbonate (3 mL) and a 40% methylamine aqueous solution (1 mL, 13 mmol) were added to a solution of 4-bromobenzenesulfonyl chloride (0.26 g, 1.0 mmol) in methylene chloride (5 mL). The resulting mixture was stirred at room temperature for 30 min. Water and methylene chloride were added to the reaction solution, and the organic layer was extracted using an Empore cartridge (GL Science). The compound (0.12 g, 0.48 mmol) obtained by evaporating the solvent under reduced pressure, 2- {2-isopropyl-5-[3-(4,4,5,5-tetramethyl-1,3,2-dioxaborolan-2-yl)phenyl]-1H-imidazol-4-yl}-6-methylpyridine (0.12 g, 0.30 mmol) obtained in Example (35a), tripotassium phosphate n-hydrate (0.19 g, 0.72 mmol), and dichloro[1,1'-bis(diphenylphosphino)ferrocene]palladium(II)-dichloromethane complex (25 mg, 0.030 mmol) were dissolved in 1,2-dimethoxyethane (3 mL). The resulting mixture was stirred at 80°C for 3 hr. The reaction mixture was diluted with ethyl acetate and filtered through silica gel. The filtrate was concentrated under reduced pressure, and the resulting crude product was purified by high-performance liquid chromatography (GL Science ODS-3, eluting solvent; water : acetonitrile = 95 : 5 to 5 : 95) to obtain 60 mg (yield: 45%) of the title compound as a white amorphous form.
Melting point: 96 to 100°C.
¹H-NMR (500 MHz, CDCl₃) δ ppm: 10.72 (1H, brs), 7.86-7.84 (3H, m), 7.72-7.65 (3H, m), 7.53 (1H, m), 7.49 (1H, m), 7.41 (1H, t, J = 7.8 Hz), 7.29 (1H, d, J = 7.8 Hz), 6.96 (1H, d, J = 7.3 Hz), 4.82 (1H, brs), 3.17 (1H, m), 2.65 (3H, s), 2.48 (3H, s), 1.38 (6H, d, J = 7.3 Hz).
MS(ES) m/z: 447 (M + H)⁺.

### (Example 39) 3'-[2-Isopropyl-5-(6-methylpyridin-2-yl)-1H-imidazol-4-yl]-N,N-dimethyl-1, 1'-biphenyl-4-sulfonamide (Compound No. 1-451)

The same reaction as in Example 38 was carried out using a dimethylamine solution (2 M in tetrahydrofuran solution, 3 mL, 6.0 mmol) instead of the 40% methylamine aqueous solution to obtain 72 mg (yield: 52%) of the title compound as a light brown amorphous form.
Melting point: 93 to 96°C.
¹H-NMR (500 MHz, CDCl₃) δ ppm: 10.55 (1H, brs), 7.92 (1H, s), 7.82 (2H, d, J = 8.3 Hz), 7.76 (2H, d, J = 8.3 Hz), 7.68 (1H, m), 7.58 (1H, d, J = 7.8 Hz), 7.50 (1H, t, J = 7.8 Hz), 7.41 (1H, t, J = 7.8 Hz), 7.31 (1H, d, J = 7.8 Hz), 6.96 (1H, d, J = 7.8 Hz), 3.16 (1H, m), 2.74 (6H, s), 2.50 (3H, s), 1.39 (6H, d, J = 6.8 Hz).
MS(ES) m/z: 461 (M + H)⁺.

### (Example 40) 4-({3'-[2-Isopropyl-5-(6-methylpyridin-2-yl)-1H-imidazol-4-yl]-1,1'-biphenyl-4-yl}sulfonyl)morpholine (Compound No. 1-391)

The same reaction as in Example 38 was carried out using morpholine (0.44 g, 5.1 mmol) instead of the 40% methylamine aqueous solution to obtain 93 mg (yield: 62%) of the title compound as a white amorphous form.
Melting point: 88 to 92°C.
¹H-NMR (500 MHz, CDCl₃) δ ppm: 10.63 (1H, brs), 7.93 (1H, s), 7.81-7.75 (4H, m), 7.69 (1H, m), 7.57 (1H, d, J = 7.8 Hz), 7.50 (1H, t, J = 7.8 Hz), 7.41 (1H, t, J = 7.8 Hz), 7.30 (1H, d, J = 7.8 Hz), 6.95 (1H, d, J = 7.8 Hz), 3.77-3.74 (4H, m), 3.16 (1H, m), 3.05-2.99 (4H, m), 2.49 (3H, s), 1.38 (6H, d, J = 6.8 Hz).
MS(ES) m/z: 503 (M + H)⁺.

### (Example 41) 1-({3'-[2-Isopropyl-5-(6-methylpyridin-2-yl)-1H-imidazol-4-yl]-1,1'-biphenyl-4-yl}sulfonyl)-4-methylpiperazine (Compound No. 1-463)

The same reaction as in Example 38 was carried out using 1-methylpiperazine (0.50 g, 5.0 mmol) instead of the 40% methylamine aqueous solution to obtain 82 mg (yield: 53%) of the title compound as a light yellow amorphous form.
Melting point: 110 to 116°C.
¹H-NMR (500 MHz, CDCl₃) δ ppm: 7.87 (1H, s), 7.76 (2H, d, J = 8.3 Hz), 7.72 (2H, d, J = 8.3 Hz), 7.66 (1H, m), 7.55 (1H, m), 7.50 (1H, m), 7.41 (1H, m), 7.31 (1H, d, J = 8.3 Hz), 6.93 (1H, d, J = 7.3 Hz), 3.15 (1H, m), 3.10-3.05 (4H, m), 2.48-2.46 (7H, m), 2.26 (3H, s), 1.38 (6H, d, J = 6.8 Hz).
MS(ES) m/z: 516 (M + H)⁺.

### (Example 42) 3'-[2-Isopropyl-5-(6-methylpyridin-2-yl)-1H-imidazol-4-yl]-1,1'-biphenyl-3-sulfonamide (Compound No. 1-511)

The same reaction as in Example 36 was carried out using 2-{2-isopropyl-5-[3-(4,4,5,5-tetramethyl-1,3,2-dioxaborolan-2-yl)phenyl]-1H-imidazol-4-yl}-6-methylpyridine (0.12 g, 0.30 mmol) obtained in Example (35a) and 3-bromobenzenesulfonamide (0.11 g, 0.45 mmol) to obtain 0.11 g (yield: 84%) of the title compound as a white powder.
Melting point: 123 to 126°C.
¹H-NMR (500 MHz, CDCl₃) δ ppm: 8.12 (1H, s), 7.85 (2H, d, J = 7.8 Hz), 7.77 (1H, d, J = 7.3 Hz), 7.60-7.51 (3H, m), 7.45 (1H, m), 7.41 (1H, t, J = 7.8 Hz), 7.28 (1H, d, J = 8.3 Hz), 6.95 (1H, d, J = 7.3 Hz), 5.22 (2H, brs), 3.18 (1H, m), 2.54 (3H, s), 1.41 (6H, d, J = 7.3 Hz).
MS(ES) m/z: 433 (M + H)⁺.

### (Example 43) 2-[2-Isopropyl-4-(3-pyridin-2-ylphenyl)-1H-imidazol-5-yl]-6-methylpyridine (Compound No. 1-514)

The same reaction as in Example 36 was carried out using 2-{2-isopropyl-5-[3-(4,4,5,5-tetramethyl-1,3,2-dioxaborolan-2-yl)phenyl]-1H-imidazol-4-yl}-6-methylpyridine (0.12 g, 0.30 mmol) obtained in Example (35a) and 2-bromopyridine (55 mg, 0.35 mmol) to obtain 12 mg (yield: 12%) of the title compound as a white powder.
Melting point: 97 to 100°C.
¹H-NMR (500 MHz, CDCl₃) δ ppm: 8.67 (1H, d, J = 4.9 Hz), 8.21 (1H, s), 8.03 (1H, d, J = 7.3 Hz), 7.75-7.71 (2H, m), 7.65 (1H, d, J = 7.8 Hz), 7.50 (1H, t, J = 7.8 Hz), 7.39-7.28 (2H, m), 7.22 (1H, m), 6.92 (1H, d, J = 7.3 Hz), 3.17 (1H, m), 2.51 (3H, s), 1.40 (6H, d, J = 7.3 Hz).
MS(ES) m/z: 355 (M + H)⁺.

### (Example 44) N-(Tetrahydropyran-4-yl)-4-{3-[2-isopropyl-5-(6-methylpyridin-2-yl)-1H-imidazol-4-yl]-phenyl}benzamide (Compound No. 1-532) (44a) 4-(4,4,5,5-Tetramethyl-[1,3,2]dioxaborolan-2-yl)-N-(tetrahydropyran-4-yl)benzamide

4-(4,4,5,5-Tetramethyl-[1,3,2]dioxaborolan-2-yl)benzoic acid (3.7 g, 15 mmol) was suspended in thionyl chloride (10 mL). The resulting mixture was heated under reflux for 2 hr under a nitrogen atmosphere. Then, the thionyl chloride was evaporated. The residue was dissolved in methylene chloride (15 mL), and tetrahydropyran-4-ylamine (2.0 g, 13 mmol) and triethylamine (4 mL) were added thereto. The resulting mixture was stirred at room temperature for 3 hr. To this reaction solution, water was added. After extraction with methylene chloride, the organic layer was washed with water and brine, and then dried with anhydrous magnesium sulfate. The solvent was evaporated under reduced pressure, and the resulting crude product was purified by silica gel column chromatography (eluting solvent; methylene chloride : methanol = 20 : 1) to obtain 4.0 g (yield: 82%) of the title compound as a white powder.
¹H-NMR (400 MHz, CDCl₃) δ ppm: 7.87 (2H, d, J = 7.8 Hz), 7.74 (2H, d, J = 7.8 Hz), 6.01 (1H, m), 4.21 (1H, m), 4.00 (2H, m), 3.54 (2H, m), 2.02 (2H, m), 1.57 (2H, m), 1.36 (12H, s).

### (44b) N-(Tetrahydropyran-4-yl)-4-{3-[2-isopropyl-5-(6-methylpyridin-2-yl)-1H-imidazol-4-yl]phenyl}benzamide

2-[4-(3-Bromophenyl)-2-isopropyl-1H-imidazol-5-yl]-6-methylpyridine (0.20 g, 0.56 mmol) obtained in Example (1b) 4-(4,4,5,5-tetramethyl-[1,3,2]dioxaborolan-2-yl)-N-(tetrahydropyran-4-yl)benzamide (0.20 g, 0.62 mmol) obtained in Example (44a) were dissolved in 1,2-dimethoxyethane (4.8 mL). Water (2.4 mL), a 2 M sodium carbonate aqueous solution (1.1 mL), and tetrakis(triphenylphosphine) palladium (0.022 g, 0.019 mmol) were added thereto. The mixture was heated under reflux for 3 hr. Then, water was added to the reaction solution. After extraction with methylene chloride, the organic layer was washed with water and brine, and then dried with anhydrous magnesium sulfate. The solvent was evaporated under reduced pressure, and the resulting crude product was purified by silica gel column chromatography (eluting solvent; ethyl acetate : methanol = 9 : 1) to obtain 0.25 g (yield: 92%) of the title compound as a white solid.
Melting point: 140°C.
¹H-NMR (400 MHz, CDCl₃) δ ppm: 10.17 (1H, brs), 7.88 (1H, t, J = 1.6 Hz), 7.64 (2H, d, J = 8.6 Hz), 7.65 (2H, d, J = 8.6 Hz), 7.61 (1H, d, J = 7.8 Hz), 7.55 (1H, d, J = 7.8 Hz), 7.46 (1H, t, J = 7.8 Hz), 7.36 (1H, t, J = 7.8 Hz), 7.28 (1H, d, J = 7.8 Hz), 6.92 (1H, d, J = 7.8 Hz), 6.00 (1H, d, J = 7.8 Hz), 4.21 (1H, m), 4.01 (2H, m), 3.54 (2H, m), 3.17 (1H, hp, J = 7.0 Hz), 2.53 (3H, s), 2.04 (2H, m), 1.60 (2H, m), 1.41 (6H, d, J = 7.0 Hz).
MS(ESI) m/z: 481 (M + H)⁺.

### (Example 45) N-(Tetrahydropyran-4-yl)-4-{3-[2-isobutyl-5-(6-methylpyridin-2-yl)-1H-imidazol-4-yl]phenyl}benzamide (Compound No. 1-534)

### (45a) 2-[4-(3-Bromophenyl)-2-isobutyl-1H-imidazol-5-yl]-6-methylpyridine

The same reaction as in Example (1b) was carried out using isovaleraldehyde (0.17 g, 2.0 mmol) instead of isobutylaldehyde. The resulting crude product was purified by high-performance liquid chromatography (GL Science ODS-3, eluting solvent; water : acetonitrile = 95 : 5 to 5 : 95) to obtain 0.23 g (yield: 62%) of the title compound as a light yellow amorphous form.
¹H-NMR (400 MHz, CDCl₃) δ ppm: 10.35 (1H, brs), 7.80 (1H, t, J = 1.6 Hz), 7.54 (1H, m), 7.44 (1H, m), 7.40 (1H, t, J = 7.8 Hz), 7.27-7.20 (2H, m), 6.99 (1H, d, J = 7.8 Hz), 2.62 (2H, d, J = 7.4 Hz), 2.51 (3H, s), 2.12 (1H, m), 0.98 (6H, d, J = 7.0 Hz).

### (45b) N-(Tetrahydropyran-4-yl)-4-{3-[2-isobutyl-5-(6-methylpyridin-2-yl)-1H-imidazol-4-yl]phenyl}benzamide

The same reaction as in Example (44b) was carried out using 2-[4-(3-bromophenyl)-2-isobutyl-1H-imidazol-5-yl]-6-methylpyridine (0.10 g, 0.27 mmol) obtained in Example (45a) instead of 2-[4-(3-bromophenyl)-2-isopropyl-1H-imidazol-5-yl]-6-methylpyridine obtained in Example (1b). The resulting crude product was purified by high-performance liquid chromatography (GL Science ODS-3, eluting solvent; water : acetonitrile = 95 : 5 to 5 : 95) to obtain 0.035 g (yield: 26%) of the title compound as a white solid.
Melting point: 126 to 129°C.
¹H-NMR (400 MHz, CDCl₃) δ ppm: 10.03 (1H, brs), 7.89 (1H, t, J = 1.6 Hz), 7.78 (2H, d, J = 8.2 Hz), 7.65 (2H, d, J = 8.2 Hz), 7.63 (1H, m), 7.56 (1H, m), 7.47 (1H, t, J = 7.8 Hz), 7.36 (1H, t, J = 7.8 Hz), 7.30 (1H, d, J = 7.8 Hz), 6.92 (1H, d, J = 7.8 Hz), 5.99 (1H, d, J = 8.2 Hz), 4.22 (1H, m), 4.00 (2H, m), 3.55 (2H, m), 2.69 (1H, d, J = 7.0 Hz), 2.55 (3H, s), 2.19 (1H, m), 2.03 (2H, m), 1.60 (2H, m), 1.04 (6H, d, J = 6.7 Hz).
MS(ESI) m/z: 495 (M + H)⁺.

### (Example 46) N-(Tetrahydropyran-4-yl)-4-{3-[2-cyclopropyl-5-(6-methylpyridin-2-yl)-1H-imidazol-4-yl]phenyl}benzamide (Compound No. 1-533)

### (46a) 2-[4-(3-Bromophenyl)-2-cyclopropyl-1H-imidazol-5-yl]-6-methylpyridine

The same reaction as in Example (1b) was carried out using cyclopropanecarboxyaldehyde (0.15 g, 2.0 mmol) instead of isobutylaldehyde. The resulting crude product was purified by high-performance liquid chromatography (GL Science ODS-3, eluting solvent; water : acetonitrile = 95 : 5 to 5 : 95) to obtain 0.16 g (yield: 45%) of the title compound as a light yellow amorphous form.
¹H-NMR (400 MHz, CDCl₃) δ ppm: 10.15 (1H, brs), 7.69 (1H, t, J =1.6 Hz), 7.50 (1H, m), 7.43 (1H, m), 7.38 (1H, t, J = 7.8 Hz), 7.22 (1H, d, J = 7.8 Hz), 7.20 (1H, t, J = 7.8 Hz), 6.92 (1H, d, J = 7.8 Hz), 2.52 (3H, s), 1.99 (1H, m), 1.08-0.97 (4H, m).

### (46b) N-(Tetrahydropyran-4-yl)-4-{3-[2-cyclopropyl-5-(6-methylpyridin-2-yl)-1H-imidazol-4-yl]phenyl}benzamide

The same reaction as in Example (44b) was carried out using 2-[4-(3-bromophenyl)-2-cyclopropyl-1H-imidazol-5-yl]-6-methylpyridine (0.097 g, 0.27 mmol) obtained in Example (46a) instead of 2-[4-(3-bromophenyl)-2-isopropyl-1H-imidazol-5-yl]-6-methylpyridine obtained in Example (1b). The resulting crude product was purified by high-performance liquid chromatography (GL Science ODS-3, eluting solvent; water : acetonitrile = 95 : 5 to 5 : 95) to obtain 0.012 g (yield: 10%) of the title compound as a white solid.
Melting point: 127 to 128°C.
¹H-NMR (400 MHz, CDCl₃) δ ppm: 9.95 (1H, brs), 7.85 (1H, t, J = 1.5 Hz), 7.78 (2H, d, J = 8.2 Hz), 7.65 (2H, d, J = 8.2 Hz), 7.62 (1H, m), 7.55 (1H, m), 7.46 (1H, t, J = 7.4 Hz), 7.34 (1H, t, J = 7.8 Hz), 7.25 (1H, d, J = 7.8 Hz), 6.91 (1H, d, J = 7.8 Hz), 5.98 (1H, d, J = 7.8 Hz), 4.22 (1H, m), 4.00 (2H, m), 3.54 (2H, m), 2.55 (3H, s), 2.03 (2H, m), 1.57 (2H, m), 1.19-1.00 (4H, m).
MS(ESI) m/z: 479 (M + H)⁺.

### (Example 47) N-(Tetrahydropyran-4-yl)-4-{3-[2-t-butyl-5-(6-methylpyridin-2-yl)-1H-imidazol-4-yl]phenyl}benzamide (Compound No. 1-535)

### (47a) 2-[4-(3-Bromophenyl)-2-t-butyl-1H-imidazol-5-yl]-6-methylpyridine

The same reaction as in Example (1b) was carried out using pivalaldehyde (0.17 g, 2.0 mmol) instead of isobutylaldehyde. The resulting crude product was purified by high-performance liquid chromatography (GL Science ODS-3, eluting solvent; water : acetonitrile = 95 : 5 to 5 : 95) to obtain 0.14 g (yield: 37%) of the title compound as a colorless amorphous form.
¹H-NMR (400 MHz, CDCl₃) δ ppm: 10.49 (1H, brs), 7.79 (1H, s), 7.51 (1H, d, J = 7.8 Hz), 7.42 (1H, d, J = 7.9 Hz), 7.38 (1H, d, J = 7.8 Hz), 7.25-7.16 (2H, m), 6.92 (1H, d, J = 7.9 Hz), 2.46 (3H, s), 1.42 (9H, s).

### (47b) N-(Tetrahydropyran-4-yl)-4- {3-[2-t-butyl-5-(6-methylpyridin-2-yl)-1H-imidazol-4-yl]phenyl} benzamide

The same reaction as in Example (44b) was carried out using 2-[4-(3-bromophenyl)-2-t-butyl-1H-imidazol-5-yl]-6-methylpyridine (0.13 g, 0.36 mmol) obtained in Example (47a) instead of 2-[4-(3-bromophenyl)-2-isopropyl-1H-imidazol-5-yl]-6-methylpyridine obtained in Example (1b). The resulting crude product was purified by high-performance liquid chromatography (GL Science ODS-3, eluting solvent; water : acetonitrile = 95 : 5 to 5 : 95) to obtain 0.13 g (yield: 72%) of the title compound as a white solid.
Melting point: 146 to 149°C.
¹H-NMR (400 MHz, CDCl₃) δ ppm: 10.23 (1H, brs), 7.87 (1H, s), 7.79 (2H, d, J = 8.2 Hz), 7.65 (2H, d, J = 8.2 Hz), 7.62 (1H, d, J = 7.8 Hz), 7.55 (1H, d, J = 7.8 Hz), 7.46 (1H, t, J = 7.8 Hz), 7.35 (1H, t, J = 7.8 Hz), 7.25 (1H, d, J = 7.8 Hz), 6.91 (1H, d, J = 7.8 Hz), 6.00 (1H, d, J = 7.8 Hz), 4.24 (1H, m), 4.00 (2H, m), 3.54 (2H, m), 2.52 (3H, s), 2.01 (2H, m), 1.60 (2H, m), 1.47 (9H, s).
MS(ESI) m/z: 494 (M + H)⁺.

### (Example 48) N-(4-Methylpiperazin-1-yl)-4-{3-[2-isopropyl-5-(6-methylpyridin-2-yl)-1H-imidazol-4-yl]phenyl}benzamide (Compound No. 1-241)

### (48a) 4-(4,4,5,5-Tetramethyl-[1,3,2]dioxaborolan-2-yl)-N-(4-methylpiperazin-1-yl)benzamide

The same reaction as in Example (44a) was carried out using 1-amino-4-methylpiperazine (0.27 g, 2.7 mmol) instead of tetrahydropyran-4-ylamine to obtain 0.55 g (yield: 79%) of a crude product of the title compound as a white solid.
¹H-NMR (400 MHz, CDCl₃) δ ppm: 7.83 (2H, d, J = 7.8 Hz), 7.69 (2H, d, J = 7.8 Hz), 6.75 (1H, s), 2.98 (4H, brs), 2.67 (4H, brs), 2.35 (3H, s), 1.35 (12H, s).

### (48b) N-(4-Methylpiperazin-1-yl)-4-{3-[2-isopropyl-5-(6-methylpyridin-2-yl)-1H-imidazol-4-yl]phenyl}benzamide

The same reaction as in Example (44b) was carried out using 4-(4,4,5,5-tetramethyl-[1,3,2]dioxaborolan-2-yl)-N-(4-methylpiperazin-1-yl)benzamide (0.19 g, 0.56 mmol) obtained in Example (48a) instead of 4-(4,4,5,5-tetramethyl-[1,3,2]dioxaborolan-2-yl)-N-(tetrahydropyran-4-yl)benzamide obtained in Example (44a). The resulting crude product was purified by high-performance liquid chromatography (GL Science ODS-3, eluting solvent; water : acetonitrile = 95 : 5 to 5 : 95) to obtain 0.11 g (yield: 80%) of the title compound as a light yellow amorphous form.
¹H-NMR (400 MHz, CDCl₃) δ ppm: 7.90 (1H, s), 7.79 (2H, d, J = 7.8 Hz), 7.67-7.63 (3H, m), 7.57 (1H, d, J = 7.8 Hz), 7.48 (1H, t, J = 7.8 Hz), 7.38 (1H, t, J = 7.8 Hz), 7. 31 (1H, d, J = 7. 8 Hz), 6.94 (1H, d, J = 7.8 Hz), 6.74 (1H, brs), 3.18 (1H, hp, J = 7.8 Hz), 2.97 (4H, brs), 2.66 (4H, brs), 2.55 (3H, s), 2.34 (3H, s), 1.43 (6H, d, J = 7.0 Hz).
MS(ESI) m/z: 495 (M + H)⁺.

### (Example 49) 3'-[2-Isopropyl-4-(6-methylpyridin-2-yl)-1H-imidazol-5-yl]-1,1'-biphenyl-4-carboxylic acid methyl (Compound No. 1-445)

The same reaction as in Example (44b) was carried out using 4-(methoxycarbonyl)phenylboronic acid (0.93 g, 5.2 mmol) instead of 4-(4,4,5,5-tetramethyl-[1,3,2]dioxaborolan-2-yl)-N-(tetrahydropyran-4-yl)benzamide obtained in Example (44a). The resulting crude product was purified by silica gel column chromatography (eluting solvent; ethyl acetate : water = 30 : 1) to obtain 1.7 g (yield: 82%) of the title compound as a colorless amorphous form.
¹H-NMR (400 MHz, CDCl₃) 5 ppm: 10.55 (1H, brs), 8.05 (2H, d, J = 8.6 Hz), 7.90 (1H, s), 7.65 (2H, d, J = 8.6 Hz), 7.63 (1H, d, J = 7.8 Hz), 7.56 (1H, d, J = 7.8 Hz), 7.46 (1H, t, J = 7.8 Hz), 7.38 (1H, t, J = 7.8 Hz), 7.28 (1H, d, J = 7.8 Hz), 6.92 (1H, d, J = 7.8 Hz), 3.92 (3H, s), 3.15 (1H, hp, J = 7.0 Hz), 2.49 (3H, s), 1.38 (6H, d, J = 7.0 Hz).
MS(ESI) m/z: 412 (M + H)⁺.

### (Example 50) 3'-[2-Isopropyl-4-(6-methylpyridin-2-yl)-1H-imidazol-5-yl]-1,1'-biphenyl-4-carboxylic acid (Compound No. 1-517)

3'-[2-Isopropyl-4-(6-methylpyridin-2-yl)-1H-imidazol-5-yl]-1,1'-biphenyl-4-carboxylic acid methyl (3.4 g, 8.3 mmol) obtained in Example (49) was dissolved in ethanol (3.0 mL), and a 10% sodium hydroxide aqueous solution (20 mL) was added thereto. The resulting mixture was stirred at room temperature for 1.5 hr. The reaction solution was concentrated and neutralized with concentrated hydrochloric acid under ice cooling. Then, a saturated ammonium chloride aqueous solution was added thereto to produce a viscous material. The water layer was removed by decantation. After the viscous material of the residue was rinsed with ethyl acetate, the organic layer was removed by decantation. Then, the residue was dissolved in methanol and dried with anhydrous magnesium sulfate. The solvent was concentrated to obtain 2.2 g (yield: 51%) of the title compound as a light yellow amorphous form.
¹H-NMR (400 MHz, DMSO-d6) δ ppm: 7.98 (3H, brm), 7.67 (3H, brm), 7.63-7.29 (4H, m), 7.06 (1H, brm), 3.15 (3H, s), 3.07 (1H, hp, J = 7.0 Hz), 1.32 (6H, d, J = 7.0 Hz).
MS(ESI) m/z: 398 (M + H)⁺.

### (Example 51) N-(Morpholin-4-yl)-4- {3-[2-isopropyl-5-(6-methylpyridin-2-yl)-1H-imidazol-4-yl]-phenyl}benzamide (Compound No. 1-217)

3'-[2-Isopropyl-4-(6-methylpyridin-2-yl)-1H-imidazol-5-yl]-1,1'-biphenyl-4-carboxylic acid (0.14 g, 0.36 mmol) obtained in Example 50 was suspended in a mixture of thionyl chloride (1.5 mL) and benzene (3 mL). The resulting mixture was heated under reflux for 2 hr. The reaction solution was evaporated. The residue was dissolved in methylene chloride (2 mL), and N-aminomorpholine (0.5 mL) and triethylamine (0.5 mL) were added thereto. The resulting mixture was stirred at room temperature for 2.5 hr. Water and methylene chloride were added to the reaction solution, and the organic layer was extracted with an Empore cartridge (3M). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.027 g (yield: 16%) of the title compound as a light yellow solid.
Melting point: 145°C.
¹H-NMR (400 MHz, CDCl₃) δ ppm: 10.22 (1H, brs), 7.87 (1H, s), 7.67 (2H, d, J = 8.2 Hz), 7.64 (2H, d, J = 8.2 Hz), 7.62 (1H, d, J = 7.8 Hz), 7.55 (1H, d, J = 7.8 Hz), 7.46 (1H, t, J = 7.8 Hz), 7.36 (1H, t, J = 7.8 Hz), 7.28 (1H, d, J = 7.8 Hz), 6.92 (1H, d, J = 7.8 Hz), 6.85 (1H, brs), 3.87 (4H, brs), 3.18 (1H, hp, J = 7.0 Hz), 2.97 (4H, brs), 2.55 (3H, s), 1.43 (6H, d, J = 7.0 Hz).
MS(ES) m/z: 482 (M + H)⁺.

### (Example 52) 4-({3'-[2-Isopropyl-4-(6-methylpyridin-2-yl)-1H-imidazol-5-yl]-1,1'-biphenyl-4-yl}carbonyl)morpholine (Compound No. 1-385)

The same reaction and purification as in Example 51 was carried out using morpholine (0.5 mL) instead ofN-aminomorpholine to obtain 0.071 g (yield: 42%) of the title compound as a light yellow amorphous form.
¹H-NMR (400 MHz, CDCl₃) δ ppm: 10.23 (1H, brs), 7.89 (1H, s), 7.70-7.64 (3H, m), 7.56 (1H, d, J = 7.8 Hz), 7.50-7.45 (3H, m), 7.39 (1H, t, J = 7.8 Hz), 7.31 (1H, d, J = 7.8 Hz), 6.94 (1H, d, J = 7.8 Hz), 3.90-3.30 (8H, brm), 3.18 (1H, hp, J = 7.0 Hz), 2.54 (3H, s), 1.42 (6H, d, J = 7,0 Hz).
MS(ES) m/z: 467 (M + H)⁺.

### (Example 53) N-(2-Hydroxyethyl)-3'-[2-isopropyl-4-(6-methylpyridin-2-yl)-1H-imidazol-5-yl]-1,1'-biphenyl-4-carboxyamide (Compound No. 1-373)

The same reaction as in Example 51 was carried out using ethanolamine (0.8 mL) instead of N-aminomorpholine to obtain 0.11 g (yield: 43%) of the title compound as a light yellow amorphous form.
¹H-NMR (400 MHz, CDCl₃) δ ppm: 7.83 (1H, s), 7.75 (2H, d, J = 8.2 Hz), 7.63 (1H, m), 7.57 (2H, d, J = 8.2 Hz), 7.51(1H, m), 7.43 (1H, m), 7.36 (1H, t, J = 7.8 Hz), 7.26 (1H, t, J = 7.8 Hz), 6.92 (1H, d, J = 7.8 Hz), 6. 81 (1H, brs), 3.79 (2H, t, J = 5.0 Hz), 3.58 (2H, q, J = 5.0 Hz), 3.17 (1H, hp, J = 7.0 Hz), 2.52 (3H, s), 1.40 (6H, d, J = 7.0 Hz).
MS(ES) m/z: 441 (M + H)⁺.

### (Example 54) 4-({3'-[2-Isopropyl-4-(6-methylpyridin-2-yl)-1H-imidazol-5-yl]-1,1'-biphenyl-4-yl}carbonyl)-1-methylpiperazine (Compound No. 1-520)

3'-[2-Isopropyl-4-(6-methylpyridin-2-yl)-1H-imidazol-5-yl]-1,1'-biphenyl-4-carboxylic acid (0.14 g, 0.36 mmol) obtained in Example 50 was suspended in a mixture of thionyl chloride (1.5 mL) and benzene (3 mL). The resulting mixture was heated under reflux for 2 hr. The reaction solution was evaporated. The residue was dissolved in pyridine (2 mL), and 1-methylpiperazine (0.7 mL) was added thereto. The resulting mixture was stirred at 70°C for 15 hr, and water was added to the reaction solution. After extraction with ethyl acetate, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.079 g (yield: 45%) of the title compound as a light yellow amorphous form.
¹H-NMR (400 MHz, CDCl₃) δ ppm: 10.16 (1H, brs), 7.86 (1H, s), 7.61 (2H, d, J = 8.2 Hz), 7.61 (1H, d, J = 7.8 Hz), 7.53 (1H, d, J = 7.8 Hz), 7.45 (1H, t, J = 7.8 Hz), 7.43 (2H, d, J = 8.2 Hz), 7.36 (1H, t, J = 7.8 Hz), 7.28 (1H, d, J = 7.8 Hz), 6.91 (1H, d, J = 7.8 Hz), 3.80 (2H, brs), 3.50 (2H, brs), 3.17 (1H, hp, J = 7.0 Hz), 2.54 (3H, s), 2.49 (2H, brs), 2.37 (2H, brs), 2.32 (3H, s), 1.42 (6H, d, J = 7.0 Hz).
MS(ES) m/z: 480 (M + H)⁺.

### (Example 55) 4-({3'-[2-Isopropyl-4-(6-methylpyridin-2-yl)-1H-imidazol-5-yl]-1,1'-biphenyl-4-yl}carbonyl)thiomorpholine (Compound No. 1-523)

The same reaction as in Example 51 was carried out using thiomorpholine (1.0 mL) instead of N-aminomorpholine. After purification, 0.48 g (yield: 93%) of the title compound was obtained as a light yellow amorphous form.
¹H-NMR (400 MHz, CDCl₃) δ ppm: 10.48 (1H, brs), 7.86 (1H, s), 7.62 (2H, d, J = 7.8 Hz), 7.61 (1H, d, J = 7.8 Hz), 7.46 (1H, d, J = 7.8 Hz), 7.45 (1H, t, J = 7.8 Hz), 7.40 (2H, d, J = 7.8 Hz), 7.37 (1H, t, J = 7.8 Hz), 7.29 (1H, d, J = 7.8 Hz), 6.92 (1H, d, J = 7.8 Hz), 4.02 (2H, brs), 3.73 (2H, brs), 3.15 (1H, hp, J = 7.0 Hz), 2.66 (4H, brm), 2.50 (3H, s), 1.39 (6H, d, J = 7.0 Hz).
MS(ES) m/z: 483 (M + H)⁺.

### (Example 56) 4-({3'-[2-Isopropyl-4-(6-methylpyridin-2-yl)-1H-imidazol-5-yl]-1,1'-biphenyl-4-yl}carbonyl)thiomorpholine 1-oxide (Compound No. 1-526)

4-({3'-[2-Isopropyl-4-(6-methylpyridin-2-yl)-1H-imidazol-5-yl]-1,1'-biphenyl-4-yl}carbonyl)thiomorpholine (0.15 g, 0.32 mmol) obtained in Example 55 was dissolved in methylene chloride (2 mL), and m-chloroperbenzoic acid (0.061 g, 0.35 mmol) was added thereto. The resulting mixture was stirred at 0°C for 1.5 hr. To this reaction solution, water and methylene chloride were added. The organic layer was extracted with an Empore cartridge (3M). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.064 g (yield: 41%) of the title compound as a white solid.
Melting point: 136 to 140°C.
¹H-NMR (400 MHz, CDCl₃) δ ppm: 7.89 (1H, s), 7.68 (2H, d, J = 8.2 Hz), 7.65 (1H, d, J = 7.8 Hz), 7.56 (1H, d, J = 7.8 Hz), 7.49 (1H, t, J = 7.8 Hz), 7.47 (2H, d, J = 8.2 Hz), 7.39 (1H, t, J = 7.8 Hz), 7.31 (1H, d, J = 7.8 Hz), 6.95 (1H, d, J = 7.8 Hz), 4.09 (4H, br), 3.19 (1H, hp, J = 7.0 Hz), 2.84 (4H, br), 2.56 (3H, s), 1.43 (6H, d, J = 7.0 Hz).
MS(ESI) m/z: 499 (M + H)⁺.

### (Example 57) 4-({3'-[2-Isopropyl-4-(6-methylpyridin-2-yl)-1H-imidazol-5-yl]-1,1'-biphenyl-4-yl}carbonyl)thiomorpholine 1,1-dioxide (Compound No. 1-529)

The same reaction as in Example 56 was carried out using m-chloroperbenzoic acid (0.12 g, 0.67 mmol). The resulting crude product was purified by preparative thin-layer chromatography (developing solvent; ethyl acetate : methanol = 9 : 1) to obtain 0.012 g (yield: 8%) of the title compound as a white solid.
Melting point: 141 to 145°C.
¹H-NMR (400 MHz, CDCl₃) δ ppm: 7.90 (1H, s), 7.70 (2H, d, J = 8.2 Hz), 7.65 (1H, d, J = 7.8 Hz), 7.57 (1H, d, J = 7.8 Hz), 7.49 (1H, t, J = 7.8 Hz), 7.49 (2H, d, J = 8.2 Hz), 7.41 (1H, t, J = 7.8 Hz), 7.31 (1H, d, J = 7.8 Hz), 6.97 (1H, d, J = 7.8Hz), 4.13 (4H, br), 3.21 (1H, hp, J = 7.0 Hz), 3.09 (4H, br), 2.57 (3H, s), 1.44 (6H, d, J = 7.0 Hz).
MS(ESI) m/z: 515 (M + H)⁺.

### (Example 58) 4-({3'-[2-Isopropyl-5-(6-methylpyridin-2-yl)-1H-imidazol-4-yl]-1,1'-biphenyl-4-yl}methyl)morpholine (Compound No. 1-540)

The same reaction as in Example (44b) was carried out using 4-[4-(4,4,5,5-tetramethyl-1,3,2-dioxaborolan-2-yl)benzyl]morpholine (0.094 g) instead of 4-(4,4,5,5-tetramethyl-[1,3,2]dioxaborolan-2-yl)-N-(tetrahydropyran-4-yl)benzamide obtained in Example (44a). The resulting crude product was purified by preparative thin-layer chromatography (developing solvent; ethyl acetate : methanol = 9 : 1) to obtain 0.020 g (yield: 16%) of the title compound as a colorless amorphous form. ¹H-NMR (400 MHz, CDCl₃) δ ppm: 10.13 (1H, brs), 7.87 (1H, s), 7.61-7.53 (4H, m), 7.46 (1H, t, J = 7.4 Hz), 7.35-7.40 (3H, m), 7.31 (1H, d, J = 7.4 Hz), 6.93 (1H, d, J = 7.4 Hz), 3.72 (4H, m), 3.53 (3H, s), 3.18 (1H, hp, J = 7.0 Hz), 2.55 (3H, s), 2.47 (4H, s), 1.43 (6H, d, J = 7.0 Hz).
MS(ESI) m/z: 453 (M + H)⁺.

### (Example 59) N- {3'-[2-Isopropyl-5-(6-methylpyridin-2-yl)-1H-imidazol-4-yl]-1,1'-biphenyl-4-yl}-N'-(methylpiperazin-1-yl)urea (Compound No. 1-563)

### (59a) N-(4-Methylpiperazin-1-yl)-N'-(4,4,5,5-tetramethyl-1,3,2-dioxaborolan-2-yl)urea

(4-Isocyanatephenyl)boronic acid pinacol ester (0.20 g, 0.82 mmol) was dissolved in methylene chloride (2 mL), and 1-aminopiperazine (0.11 mL, 0.10 g, 0.90 mmol) was added thereto. The mixture was stirred at room temperature for 6 hr. The reaction solution was concentrated to obtain 0.29 g (yield: 100%) of a crude product of the title compound as a white solid.
¹H-NMR (400 MHz, CDCl₃) δ ppm: 8.13 (1H. s), 7.72 (2H, d, J = 8.6 Hz), 7.46 (2H, d, J = 8.6 Hz), 5.38 (1H, s), 3.06 (2H, brm), 2.82 (2H, brm), 2.65 (2H, brm), 2.33 (3H, s), 2.30 (2H, brm), 1.34 (12H, s).

### (59b) N-{3'-[2-Isopropyl-5-(6-methylpyridin-2-yl)-1H-imidazol-4-yl]-1,1'-biphenyl-4-yl}-N'-(methylpiperazin-1-yl)urea

The same reaction as in Example (44b) was carried out using N-(4-methylpiperazin-1-yl)-N'-(4,4,5,5-tetramethyl-1,3,2-dioxaborolan-2-yl)urea (0.11 g, 0.31 mmol) obtained in Example (59a) instead of 4-(4,4,5,5-tetramethyl-[1,3,2]dioxaborolan-2-yl)-N-(tetrahydropyran-4-yl)benzamide obtained in Example (44a). The resulting crude product was purified by high-performance liquid chromatography (GL Science ODS-3, eluting solvent; water : acetonitrile = 95 : 5 to 5 : 95) to obtain 0.041 g (yield: 29%) of the title compound as a white solid.
¹H-NMR (400 MHz, CDCl₃) δ ppm: 10.08 (1H, brs), 8.12 (1H, s), 7.85 (1H, s), 7.59-7.51 (6H, m), 7.45 (1H, t, J = 7.4 Hz), 7.40-7.31 (2H, m), 6.93 (1H, d, J = 7.4 Hz), 5.32 (1H, s), 3.18 (1H, hp, J = 7.0 Hz), 3.09 (2H, brm), 2.81 (2H, brm), 2.67 (2H, brm), 2.56 (3H, s), 2.34 (3H, s), 2.31 (2H, brm), 1.44 (6H, d, J = 7.0 Hz).
MS(ESI) m/z: 510 (M + H)⁺.

### (Example 60) 2'-Fluoro-5'-[2-isopropyl-5-(6-methylpyridin-2-yl)-1H-imidazol-4-yl]-N-(4-methylpiperazin-1-yl)-1,1'-biphenyl-4-carboxyamide (Compound No. 1-253)

### (60a) 1-(3-Bromo-4-fluorophenyl)-2-(6-methylpyridin-2-yl)ethanone

The same reaction as in Example (1a) was carried out using methyl 3-bromo-4-fluorobenzoate (5.2 g, 20 mmol) instead of ethyl 3-bromobenzoate. After purification, 1.3 g (yield: 21%) of the title compound was obtained as a yellow solid. ¹H-NMR (400 MHz, CDCl₃) δ ppm: 8.03 (1H, dd, J = 6.7, 2.3 Hz), 7.74 (1H, m), 7.50 (1H, t, J = 7.8 Hz), 7.12 (1H, t, J = 7.4 Hz), 6.85 (1H, d, J = 7.4 Hz), 6.77 (1H, d, J = 7.4 Hz), 5.94 (1H, s), 2.53 (3H, s).

### (60b) 2-[4-(3-Bromo-4-fluorophenyl)-2-isopropyl-1H-imidazol-5-yl]-6-methylpyridine

The same reaction as in Example (1b) was carried out using 1-(3-bromo-4-fluorophenyl)-2-(6-methylpyridin-2-yl)ethanone obtained in Example (60a) instead of 1-(3-bromophenyl)-2-(6-methylpyridin-2-yl)ethanone obtained in Example (1a). After purification, 1.1 g (yield: 66%) of the title compound was obtained as a light yellow amorphous form.
¹H-NMR (400 MHz, CDCl₃) δ ppm: 10.62 (1H, brs), 7.87 (1H, m), 7.52 (1H, m), 7.44 (1H, t, J = 7.8 Hz), 7.19 (1H, d, J = 7.8 Hz), 7.12 (1H, t, J = 8.6 Hz), 6.96 (1H, d, J = 7.8 Hz), 3.12 (1H, hp, J = 7.0 Hz), 2.48 (3H, s), 1.35 (6H, d, J = 7.0 Hz). (60c) 2'-Fluoro-5'-[2-isopropyl-5-(6-methylpyridin-2-yl)-1H-imidazol-4-yl]-N-(4-methylpiperazin-1-yl)-1,1'-biphenyl-4-carboxyamide

The same reaction as in Example (44b) was carried out using 2-[4-(3-bromo-4-fluorophenyl)-2-isopropyl-1H-imidazol-5-yl]-6-methylpyridine (0.11 g, 0.30 mmol) obtained in Example (60b) instead of 2-[4-(3-bromophenyl)-2-isopropyl-1H-imidazol-5-yl]-6-methylpyridine obtained in Example (1b) and using 4-(4,4,5,5-tetramethyl-[1,3,2]dioxaborolan-2-yl)-N-(4-methylpiperazin-1-yl)benzamide (0.12 g, 0.33 mmol) obtained in Example (48a) instead of 4-(4,4,5,5-tetramethyl-[1,3,2]dioxaborolan-2-yl)-N-(tetrahydropyran-4-yl)benzamide obtained in Example (44a). The resulting crude product was purified by preparative thin-layer chromatography (developing solvent; ethyl acetate : methanol : 28% ammonia water = 92 : 5 : 3) and high-performance liquid chromatography (GL Science ODS-3, eluting solvent; water : acetonitrile = 95 : 5 to 5 : 95) to obtain 0.021 g (yield: 14%) of the title compound as a white solid.
Melting point: 143 to 145°C.
¹H-NMR (400 MHz, CDC1₃) δ ppm: 7.79 (2H, d, J = 8.6 Hz), 7.32 (1H, m), 7.63 (2H, d, J = 8.6 Hz), 7.60 (1H, m), 7.40 (1H, t, J = 7.4 Hz), 7.25 (1H, m), 7.19 (1H, m), 6.95 (1H, d, J = 7.4 Hz), 6.78 (1H, brs), 3.16 (1H, hp, J = 7.0 Hz), 2.98 (4H, brs), 2.67 (4H, brs), 2.56 (3H, s), 2.34 (3H, s), 1.42 (6H, d, J = 7.0 Hz).
MS(ESI) m/z: 513 (M + H)⁺.

### (Example 61) 2-{4-[6-Fluoro-4'-(methylsulfonyl)-1,1'-biphenyl-3-yl]-2-isopropyl-1H-imidazol-5-yl}-6-methylpyridine (Compound No. 1-109)

The same reaction as in Example (1c) was carried out using 2-[4-(3-bromo-4-fluorophenyl)-2-isopropyl-1H-imidazol-5-yl]-6-methylpyridine (0.13 g, 0.34 mmol) obtained in Example (60b) instead of 2-[4-(3-bromophenyl)-2-isopropyl-1H-imidazol-5-yl]-6-methylpyridine obtained in Example (1b) and using 4-(methanesulfonyl)phenylboronic acid (0.10 g, 0.51 mmol) instead of 4-cyanophenylboronic acid. The resulting crude product was purified by high-performance liquid chromatography (GL Science ODS-3, eluting solvent; water : acetonitrile = 95 : 5 to 5 : 95) and preparative thin-layer chromatography (developing solvent; ethyl acetate : methanol : 28% ammonia water = 58 : 39 : 3) to obtain 0.016 g (yield: 10%) of the title compound as a white solid.
Melting point: 113 to 115°C.
¹H-NMR (400 MHz, CDC1₃) δ ppm: 10.04 (1H, brs), 7.97 (2H, d, J = 8.6 Hz), 7.74 (2H, d, J = 8.6 Hz), 7.72 (1H, m), 7.62 (1H, m), 7.39 (1H, t, J = 7.8 Hz), 7.25-7.17 (2H, m), 6.94 (1H, d, J = 7.4 Hz), 3.15 (1H, hp, J = 7.0 Hz), 3.08 (3H, s), 2.55 (3H, s), 1.42 (6H, d, J = 7.0 Hz).
MS(ESI) m/z: 450 (M + H)⁺.

### (Example 62) 4-(6-Methylpyridin-2-yl)-5-[4'-(methylsulfonyl)-1,1'-biphenyl-3-yl]-1,3-thiazol-2-amine (Compound No. 3-33)

### (62a) 2-(3-Bromophenyl)-1-(6-methylpyridin-2-yl)ethanone

3-Bromophenyl acetic acid (10 g, 47 mmol) was dissolved in methanol (100 mL), and concentrated sulfuric acid (2.0 mL, 38 mmol) was added thereto at room temperature. Then, the resulting mixture was heated under reflux for 23 hr and then was cooled to room temperature. The reaction solution was evaporated, and saturated aqueous sodium bicarbonate was added thereto. After extraction with ethyl acetate, the organic layer was washed with brine and then dried with anhydrous sodium sulfate. The solvent was evaporated under reduced pressure to obtain a white solid. The resulting white solid was dissolved in tetrahydrofuran (50 mL), and ethyl 6-methylpyridine-2-carboxylate (6.7 mL, 44 mmol) and potassium-t-butoxide (1 M in tetrahydrofuran solution, 132 mL, 132 mmol) were added thereto at room temperature. The resulting mixture was heated under reflux for 5 hr and then cooled to room temperature. The reaction solution was evaporated to obtain a brown oily material. The resulting brown oily material was mixed with water (50 mL) and concentrated hydrochloric acid (25 mL). The resulting mixture was heated under reflux for 6 hr and then cooled to room temperature. The reaction mixture was neutralized with a sodium hydroxide aqueous solution. After extraction with ethyl acetate, the organic layer was washed with brine and then dried with anhydrous sodium sulfate. The solvent was evaporated, and the resulting crude product was purified by silica gel column chromatography (eluting solvent; hexane : ethyl acetate = 20 : 1 to 5 : 1) to obtain 2.4 g (yield: 18%) of the title compound as a yellow oily material.
¹H-NMR (400 MHz, CDCl₃) δ ppm: 7.82 (1H, d, J = 7.8 Hz), 7.68 (1H, t, J = 7.8 Hz), 7.50 (1H, m), 7.45-7.15 (4H, m), 4.50 (2H, s), 2.65 (3H, s).
MS(ES) m/z: 290 (M + H)⁺.

### (62b) 5-(3-Bromophenyl)-4-(6-methylpyridin-2-yl)]-1,3-thiazol-2-amine

2-(3-Bromophenyl)-1-(6-methylpyridin-2-yl)ethanone (600 mg, 2.1 mmol) obtained in Example (62a) was dissolved in chloroform (5 mL), and bromine (0.11 mL, 2.1 mmol) was added thereto at room temperature. The resulting mixture was stirred for 4 hr. To the reaction solution, saturated aqueous sodium bicarbonate and a sodium bisulfite aqueous solution (10%) were added to terminate the reaction. After extraction with ethyl acetate, the organic layer was dried with anhydrous sodium sulfate. The solvent was evaporated under reduced pressure to obtain a brown oily material. The resulting brown oily material was dissolved in ethanol (5 mL), and thiourea (186 mg, 2.5 mmol) was added thereto at room temperature. The resulting mixture was heated under reflux for 4 hr and then cooled to room temperature. The reaction solution was evaporated under reduced pressure, and the resulting crude product was purified by silica gel column chromatography (eluting solvent; hexane : ethyl acetate = 1 : 1 to 1 : 5) to obtain 44 mg (yield: 6%) of the title compound as a yellow solid.
¹H-NMR (400 MHz, CDCl₃) δ ppm: 7.50 (1H, m), 7.45 (1H, t, J = 7.8 Hz), 7.35 (1H, m), 7.18 (1H, m), 7.16 (1H, d, J = 7.8 Hz), 7.09 (1H, t, J = 7.8 Hz), 7.03 (1H, d, J = 7.8 Hz), 5.06 (2H, brs), 2.52 (3H, s).
MS(ES) m/z: 346 (M + H)⁺. (62c) 4-(6-Methylpyridin-2-yl)-5-[4'-(methylsulfonyl)-1,1'-biphenyl-3-yl]-1,3-thiazol-2-amine

The same reaction as in Example 3 was carried out using 5-(3-bromophenyl)-4-(6-methylpyridin-2-yl)]-1,3-thiazol-2-amine (44 mg, 0.13 mmol) obtained in Example (62b) and 4-methylsulfonylphenylboronic acid (51 mg, 0.25 mmol) to obtain 14 mg (yield: 26%) of the title compound as a light yellow solid.
Melting point: 107 to 109°C.
¹H-NMR (500 MHz, CDCl₃) δ ppm: 7.94 (2H, d, J = 8.6 Hz), 7.60 (2H, d, J = 8.6 Hz), 7.52 (1H, m), 7.47-7.44 (2H, m), 7.39-7.32 (2H, m), 7.20 (1H, d, J = 7.6 Hz), 7.05 (1H, d, J = 7.6 Hz), 5.02 (2H, brs), 3.08 (3H, s), 2.52 (3H, s).
MS(ES) m/z: 422 (M + H)⁺.

### (Example 63) 2-Methyl-6-{4-[4'-(methylsulfonyl)-1,1'-biphenyl-3-yl]-1H-pyrazol-2-yl}pyridine (Compound No. 2-408)

### (63a) 2-[4-(3-Bromophenyl)-1H-pyrazol-3-yl]-6-methylpyridine

2-(3-Bromophenyl)-1-(6-methylpyridin-2-yl)ethanone (600 mg, 2.1 mmol) obtained in Example (62a) was dissolved in N,N-dimethylformamide (2 mL), and N,N-dimethylformamide dimethylacetal (0.41 mL, 3.1 mmol) was added thereto at room temperature. The resulting mixture was stirred at 120°C for 4 hr and then cooled to room temperature. The reaction solution was evaporated under reduced pressure to obtain a brown oily material. The resulting brown oily material was dissolved in ethanol (5 mL), and hydrazine hydrate (0.15 mL, 3.1 mmol) was added thereto at room temperature. The resulting mixture was stirred for 4 hr. The reaction solution was evaporated under reduced pressure, and the resulting crude product was purified by silica gel column chromatography (eluting solvent; hexane : ethyl acetate = 1 : 1 to 1 : 5) to obtain 160 mg (yield: 25%) of the title compound as a yellow oily material.
¹H-NMR (400 MHz, CDCl₃) δ ppm: 7.61 (1H, s), 7.58 (1H, t, J = 1.7 Hz), 7.46 (1H, m), 7.45 (1H, t, J = 7.8 Hz), 7.33 (1H, m), 7.24 (1H, t, J = 7.8 Hz), 7.13 (1H, d, J = 7.8 Hz), 7.06 (1H, d, J = 7.8 Hz), 2.57 (3H, s).
MS(ES) m/z: 314 (M + H)⁺.

### (63b) 2-Methyl-6-{4-[4'-(methylsulfonyl)-1,1'-biphenyl-3-yl]-1H-pyrazol-2-yl}pyridine

The same reaction as in Example 3 was carried out using 2-[4-(3-bromophenyl)-1H-pyrazol-3-yl]-6-methylpyridine (50 mg, 0.16 mmol) obtained in Example (63a) and 4-methylsulfonylphenylboronic acid (64 mg, 0.32 mmol) to obtain 6 mg (yield: 10%) of the title compound as a light yellow solid.
Melting point: 95 to 97°C.
¹H-NMR (500 MHz, CDCl₃) δ ppm: 7.98 (2H, d, J = 8.6 Hz), 7.74 (2H, d, J = 8.6 Hz), 7.67 (1H, s), 7.66 (1H, m), 7.58 (1H, m), 7.52 (1H, t, J = 7.8 Hz), 7.49 (1H, m), 7.43 (1H, t, J = 7.8 Hz), 7.19 (1H, d, J = 7.8 Hz), 7.06 (1H, d, J = 7.8 Hz), 3.08 (3H, s), 2.59 (3H, s).
MS(ES) m/z: 422 (M + H)⁺.

### (Example 64) 2-Ethyl-6-{2-isopropyl-4-[4'-(methylsulfonyl)-1,1'-biphenyl-3-yl]-1H-imidazol-5-yl}pyridine (Compound No. 1-587)

### (64a) 1-(3-Bromophenyl)-2-(6-ethylpyridin-2-yl)ethanone

A solution of 2,6-ethylmethylpyridine (3.8 g, 31 mmol) in tetrahydrofuran (30 mL) was added to sodium hexamethyldisilazane (1 M in tetrahydrofuran solution, 66 mL, 66 mmol) under a nitrogen atmosphere at -30°C. The resulting mixture was stirred for 0.5 hr, and then a solution of ethyl 3-bromobenzoate (7.2 g, 31 mmol) in tetrahydrofuran (30 mL) was gradually added dropwise thereto. The resulting mixture was further stirred at -30°C for 3 hr and then left overnight. The reaction solution was evaporated, and a saturated ammonium chloride aqueous solution was added thereto. After extraction with ethyl acetate, the organic layer was washed with brine and then dried with anhydrous sodium sulfate. The solvent was evaporated under reduced pressure, and the resulting crude product was purified by silica gel column chromatography (eluting solvent; hexane : ethyl acetate = 20 : 1 to 5 : 1) to obtain 3.1 g (yield: 32%) of the title compound as a yellow oily material. ¹H-NMR (400 MHz, CDCl₃) δ ppm: 8.00 (1H, brs), 7.77 (1H, d, J = 7.8 Hz), 7.55 (1H, t, J = 7.8 Hz), 7.49 (1H, d, J = 7.8 Hz), 7.28 (1H, t, J = 7.8 Hz), 6.90 (1H, d, J = 7.8 Hz), 6.84 (1H, d, J = 7.8 Hz), 6.04 (1H, s), 2.84 (2H, q, J = 7.4 Hz), 1.38 (3H, t, J = 7.4 Hz).

### (64b) 2-[4-(3-Bromophenyl)-2-isopropyl-1H-imidazol-5-yl]-6-ethylpyridine

The same reaction as in Example (1b) was carried out using 1-(3-bromophenyl)-2-(6-ethylpyridin-2-yl)ethanone (3.7 g, 12 mmol) obtained in Example (64a) to obtain 4.0 g (yield: 89%) of the title compound as a white amorphous form.
¹H-NMR (500 MHz, CDCl₃) δ ppm: 11.52 (1H, brs), 7.83 (1H, brs), 7.54 (1H, d, J = 7.8 Hz), 7.49 (1H, t, J = 7.8 Hz), 7.44 (1H, d, J = 7.8 Hz), 7.26 (1H, d, J = 7.8 Hz), 7.22 (1H, t, J = 7.8 Hz), 6.98 (1H, d, J = 7.8 Hz), 3.08 (1H, m), 2.68 (2H, q, J = 7.4 Hz), 1.26 (6H, d, J = 6.8 Hz), 1.12 (3H, t, J = 7.4 Hz).
MS(ES) m/z: 370 (M + H)⁺.

### (64c) 2-Ethyl-6-{2-isopropyl-4-[4'-(methylsulfonyl)-1,1'-biphenyl-3-yl]-1H-imidazol-5-yl}pyridine

The same reaction as in Example 3 was carried out using 2-[4-(3-bromophenyl)-2-isopropyl-1H-imidazol-5-yl]-6-ethylpyridine (50 mg, 0.14 mmol) obtained in Example (64b) and 4-methylsulfonylphenylboronic acid (64 mg, 0.32 mmol) to obtain 42 mg (yield: 70%) of the title compound as a white solid.
Melting point: 108 to 110°C.
¹H-NMR (500 MHz, CDCl₃) δ ppm: 10.47 (1H, brs), 7.98 (2H, d, J = 8.4 Hz), 7.93 (1H, m), 7.79 (2H, d, J = 8.4 Hz), 7.69 (1H, d, J = 7.8 Hz), 7.57 (1H, d, J = 7.8 Hz), 7.50 (1H, t, J = 7.8 Hz), 7.43 (1H, t, J = 7.8 Hz), 7.31 (1H, d, J = 7.8 Hz), 6.97 (1H, d, J = 7.8 Hz), 3.17 (1H, m), 3.09 (3H, s), 2.80 (2H, q, J = 7.4 Hz), 1.40 (6H, d, J = 7.2 Hz), 1.26 (3H, t, J = 7.4 Hz).
MS(ES) m/z: 446 (M + H)⁺.

### (Example 65) N-(Tetrahydropyran-4-yl)-4-{2-fluoro-5-[2-isopropyl-5-(6-methylpyridin-2-yl)-1H-imidazol-4-yl]phenyl}benzamide (Compound No. 1-588)

The same reaction as in Example 3 was carried out using 2-[4-(3-bromo-4-fluorophenyl)-2-isopropyl-1H-imidazol-5-yl]-6-methylpyridine (0.10 g, 0.27 mmol) obtained in Example (60b) instead of 2-[4-(3-bromophenyl)-2-isopropyl-1H-imidazol-5-yl]-6-methylpyridine obtained in Example (1b) and using 4-(4,4,5,5-tetramethyl-[1,3,2]dioxaborolan-2-yl)-N-(tetrahydropyran-4-yl)benzamide (0.095 g, 0.29 mmol) obtained in Example (44a) instead of 4-methylphenylboronic acid. The resulting crude product was purified by preparative thin-layer chromatography (developing solvent; ethyl acetate : 28% ammonia water = 97 : 3) to obtain 0.080 g (yield: 61 %) of the title compound as a white solid.
Melting point: 130 to 132°C.
¹H-NMR (400 MHz, CDCl₃) δ ppm: 10.11 (1H, brs), 7.81 (2H, d, J = 8.2 Hz), 7.74 (1H, m), 7.64 (2H, d, J = 8.2 Hz), 7.60 (1H, m), 7.41 (1H, t, J = 7.8 Hz), 7.26 (1H, m), 7.19 (1H, m), 6.95 (1H, d, J = 7.4 Hz), 6.03 (1H, m), 4.23 (1H, m), 4.02 (2H, m), 3.51-3.56 (3H, m), 3.16 (1H, sept, J = 7.0 Hz), 2.55 (3H, s), 2.02 (2H, m), 1.58 (2H, m), 1.42 (6H, d, J = 7.0 Hz).
MS(ESI) m/z: 499 (M + H)⁺.

### (Example 66) N-(Morpholin-4-yl)-4-{2-fluoro-5-[2-isopropyl-5-(6-methylpyridin-2-yl)-1H-imidazol-4-yl]phenyl}benzamide (Compound No. 1-229)

### (66a) N-(Morpholin-4-yl)-4-(4,4,5,5,-tetramethyl-[1,3,2]dioxaborolan-2-yl)benzamide

The same reaction as in Example (44a) was carried out using 4-aminomorpholine (3.0 g, 12 mmol) instead of tetrahydropyran-4-ylamine. After purification, 1.8 g (yield: 44%) of the title compound was obtained as a white solid. ¹H-NMR (400 MHz, CDCl₃) δ ppm: 7.83 (2H, d, J = 8.2 Hz), 7.69 (2H, d, J = 8.2 Hz), 7.24 (1H, s), 3.87 (4H, m), 2.96 (4H, m), 1.38 (12H, s).

### (66b) N-(Morpholin-4-yl)-4-{2-fluoro-5-[2-isopropyl-5-(6-methylpyridin-2-yl)-1H-imidazol-4-yl]-phenyl}benzamide

The same reaction as in Example (44b) was carried out using 2-[4-(3-bromo-4-fluorophenyl)-2-isopropyl-1H-imidazol-5-yl]-6-methylpyridine (1.6 g, 0.44 mmol) obtained in Example (60b) instead of 2-[4-(3-bromophenyl)-2-isopropyl-1H-imidazol-5-yl]-6-methylpyridine obtained in Example (1b) and using N-(morpholin-4-yl)-4-(4,4,5,5,-tetramethyl-[1,3,2]dioxaborolan-2-yl)benzamide (0.16 g, 0.48 mmol) obtained in Example (66a) instead of 4-(4,4,5,5-tetramethyl-[1,3,2]dioxaborolan-2-yl)-N-(tetrahydropyran-4-yl)benzamide obtained in Example (44a). The resulting crude product was purified by high-performance liquid chromatography (GL Science ODS-3, eluting solvent; water : acetonitrile = 95 : 5 to 5 : 95) to obtain 0.054 g (yield: 25%) of the title compound as a white solid.
Melting point: 153 to 155°C.
¹H-NMR (400 MHz, CDCl₃) δ ppm: 10.11 (1H, brs), 7.77 (2H, d, J = 8.2 Hz), 7.71 (1H, dd, J = 7.4, 2.0 Hz), 7.61 (2H, d, J = 8.2 Hz), 7.58 (1H, m), 7.39 (1H, t, J = 7.8 Hz), 7.26 (1H, m), 7.17 (1H, m), 6.93 (1H, d, J = 7.4 Hz), 6.85 (1H, s), 3.88 (4H, s), 3.16 (1H, sept, J = 7.0 Hz), 2.97 (4H, s), 2.54 (3H, s), 1.41 (6H, d, J = 7.0 Hz). MS(ES) m/z: 500 (M + H)⁺.

### (Example 67) N-{3'-[2-Isopropyl-5-(6-methylpyridin-2-yl)-1H-imidazol-4-yl]-1,1'-biphenyl-4-yl}-N'-morpholin-4-ylurea (Compound No. 1-592)

### (67a) N-Morpholin-4-yl-N'-(4,4,5,5-tetramethyl-1,3,2-dioxaborolan-2-yl)urea

The same reaction as in Example (59a) was carried out using 4-aminomorpholine instead of 1-aminopiperazine to obtain 0.29 g (yield: 100%) of a crude product of the title compound as a white solid.
¹H-NMR (400 MHz, CDCl₃) δ ppm: 8.10 (1H, s), 7.73 (2H, d, J = 8.6 Hz), 7.47 (2H, d, J = 8.6 Hz), 5.39 (1H, s), 3.91 (2H, brs), 3.75 (2H, brs), 3.03 (2H, brs), 2.64 (2H, brs), 1.34 (12H, s).

### (67b) N-{3'-[2-Isopropyl-5-(6-methylpyridin-2-yl)-1H-imidazol-4-yl]-1,1'-biphenyl-4-yl}-N'-morpholin-4-ylurea

The same reaction as in Example (44b) was carried out using N-morpholin-4-yl-N'-(4,4,5,5-tetramethyl-1,3,2-dioxaborolan-2-yl)urea (0.22 g, 0.62 mmol) obtained in Example (67a) instead of 4-(4,4,5,5-tetramethyl-[1,3,2]dioxaborolan-2-yl)-N-(tetrahydropyran-4-yl)benzamide obtained in Example (44a). The resulting crude product was purified by silica gel column chromatography (eluting solvent; ethyl acetate : methanol : 28% ammonia water = 92 : 5 : 3) and high-performance liquid chromatography (GL Science ODS-3, eluting solvent; water : acetonitrile = 95 : 5 to 5 : 95) to obtain 0.15 g (yield: 55%) of the title compound as a white solid.
Melting point: 146 to 148°C.
¹H-NMR (400 MHz, CDCl₃) δ ppm: 10.38 (1H, brs), 8.07 (1H, s), 7.83 (1H, s), 7.48-7.57 (6H, m), 7.46-7.29 (3H, m), 6.91 (1H, d, J = 7.4 Hz), 5.45 (1H, s), 3.91 (2H, brs), 3.72 (2H, brs), 3.18 (1H, sept, J = 7.0 Hz), 3.03 (2H, brs), 2.67 (2H, brs), 2.51 (3H, s), 1.39 (6H, d, J = 7.0 Hz).
MS(ES) m/z: 497 (M + H)⁺.

### (Example 68) 3'-[2-Isopropyl-4-(6-methylpyridin-2-yl)-1H-imidazol-5-yl]-N-(1-methylpiperidin-4-yl)-1,1'-biphenyl-4-carboxyamide (Compound No. 1-652)

### (68a) N-(1-Methylpiperidin-4-yl)-4-(4,4,5,5-tetramethyl-1,3,2-dioxaborolan-2-yl)benzamide

N-(1-Methylpiperidin-4-yl)-4-(4,4,5,5-tetramethyl-1,3,2-dioxaborolan-2-yl)benzoic acid (2.0 g, 8.0 mmol) was suspended in thionyl chloride (8 mL). The resulting mixture was heated under a nitrogen atmosphere under reflux for 3 hr. The solvent was evaporated under reduced pressure. The residue was dissolved in methylene chloride (20 mL), and 4-amino-1-methylpiperidine (1.0 g, 8.8 mmol) and triethylamine (4 mL) were added thereto. The resulting mixture was stirred at room temperature for 4 hr. To this reaction solution, water was added. After extraction with methylene chloride, the organic layer was washed with water and brine, and then dried with anhydrous magnesium sulfate. The solvent was evaporated under reduced pressure to obtain 2.3 g (yield: 83%) of a crude product of the title compound as a white solid.
¹H-NMR (400 MHz, CDCl₃) δ ppm: 7.83 (2H, d, J = 8.2 Hz), 7.70 (2H, d, J = 8.2 Hz), 6.01 (1H, m), 4.00 (1H, m), 2.87 (2H, m), 2.33 (3H, s), 2.20 (2H, m), 2.05 (2H, m), 1.66 (2H, m), 1.35 (12H, s).

### (68b) 3'-[2-Isopropyl-4-(6-methylpyridin-2-yl)-1H-imidazol-5-yl]-N-(1-methylpiperidin-4-yl)-1,1'-biphenyl-4-carboxyamide

2-[4-(3-Bromophenyl)-2-isopropyl-1H-imidazol-5-yl]-6-methylpyridine (0.20 g, 0.56 mmol) obtained in Example (1b) and N-(1-methylpiperidin-4-yl)-4-(4,4,5,5-tetramethyl-1,3,2-dioxaborolan-2-yl)benzamide (0.21 g, 0.62 mmol) obtained in Example (68a) were dissolved in 1,2-dimethoxyethane (5 mL). Tripotassium phosphate n-hydrate (0.24 g, 1.1 mmol), water (1.0 mL), and dichloro[1,1'-bis(diphenylphosphino)ferrocene]palladium(II)-methylene chloride complex (0.046 g, 0.056 mmol) were added thereto. The resulting mixture was stirred under a nitrogen atmosphere at 90°C for 6 hr. The reaction solution was cooled to room temperature, and water was added thereto. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.16 g (yield: 56%) of the title compound as a white solid.
Melting point: 140°C.
¹H-NMR (400 MHz, CDCl₃) δ ppm: 10.03 (1H, brs), 7.87 (1H, s), 7.78 (2H, d, J = 8.2 Hz), 7.65 (2H, d, J = 8.2 Hz), 7.62 (1H, s), 7.56 (1H, d, J = 7.8 Hz), 7.46 (1H, t, J = 7.8 Hz), 7.35 (1H, t, J = 7.8 Hz), 7.28 (1H, d, J = 7.8 Hz), 6.92 (1H, d, J = 7.8 Hz), 5.98 (1H, d, J = 7.8 Hz), 4.00 (1H, m), 3.17 (1H, hp, J = 7.0 Hz), 2.84 (2H, m), 2.56 (3H, s), 2.30 (3H, s), 2.17 (2H, m), 2,06 (2H, m), 1.58 (2H, m), 1.43 (6H, d, J = 7.0 Hz).
MS(ESI) m/z: 494 (M+H)⁺

### (Example 69) 2-{2-Isopropyl-4-[3-(5-methyl-1H-pyrrol-2-yl)phenyl]-1H-imidazol-5-yl}-6-methylpyridine (Compound No. 1-756)

### (69a) 5-{3-[2-Isopropyl-5-(6-methylpyridin-2-yl)-1H-imidazol-4-yl]phenyl}-1H-pyrrole-2-carboaldehyde

N,N-Dimethylformamide (0.018 mL) was dissolved in 1,2-dichloroethane (1 mL), and oxalyl chloride (0.020 mL) was added thereto. The resulting mixture was stirred at 0°C for 10 min and then further stirred at room temperature for 10 min. Then, the mixture was cooled to 0°C again. To this mixture, a 1,2-dichloroethane solution (1 mL) of 2-{2-isopropyl-4-[3-(1H-pyrrol-2-yl)phenyl]-1H-imidazol-5-yl}-6-methylpyridine (0.23 mmol) obtained in Example 23 was added. The resulting mixture was stirred at 0°C for 10 min and then stirred at room temperature for 24 hr. Saturated aqueous sodium bicarbonate was added to the reaction solution. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.063 g (yield: 73%) of the title compound as a white solid.
¹H-NMR (400 MHz, CDCl₃) δ ppm: 10.26 (1H, brs), 9.92 (1H, brs), 9.50 (1H, s), 8.02 (1H, s), 7.62 (1H, d, J = 7.8 Hz), 7.56 (1H, d, J = 7.8 Hz), 7.44 (1H, t, J = 7.8 Hz), 7.39 (1H, t, J = 7.8 Hz), 7.28 (1H, m), 7.00 (1H, m), 6.95 (1H, d, J = 7.8 Hz), 6.65 (1H, m), 3.13 (1H, hp, J = 7.0 Hz), 2.55 (3H, s), 1.37 (6H, d, J = 7.0 Hz). MS(ESI) m/z: 371 (M+H)⁺

### (69b) 2-{2-Isopropyl-4-[3-(5-methyl-1H-pyrrol-2-yl)phenyl]-1H-imidazol-5-yl}-6-methylpyridine

5-{3-[2-Isopropyl-5-(6-methylpyridin-2-yl)-1H-imidazol-4-yl]phenyl}-1H-pyrrole-2-carboaldehyde (0.24 g, 0.50 mmol) obtained in Example (69a) was dissolved in tetrahydrofuran (3 mL). The resulting mixture was stirred at 0°C, and then aluminum lithium hydride (0.025 g, 0.66 mmol) was added thereto. The mixture was stirred at 0°C for 10 min and then returned to room temperature. After reflux while heating for 6 hr, the mixture was returned to room temperature again. Water and a sodium hydroxide aqueous solution were added thereto. After extraction with ethyl acetate, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.038 g (yield: 65%) of the title compound as a white solid. Melting point: 128 to 130°C.
¹H-NMR (400 MHz, CDCl₃) δ ppm: 10.04 (1H, brs), 8.33 (1H, brs), 7.68 (1H, s), 7.42-7.23 (5H, m), 6.90 (1H, d, J = 7.4 Hz), 6.37 (1H, m), 5.91 (1H, m), 3.16 (1H, hp, J = 7.0 Hz), 2.54 (3H, s), 2.30 (3H, s), 1.41 (6H, d, J = 7.0 Hz).
MS(ESI) m/z: 357 (M+H)⁺

### (Example 70) 2-{2-Isopropyl-5-[3-(1-methyl-1H-pyrazol-4-yl)phenyl]-1H-imidazol-4-yl}-6-methylpyridine (Compound No. 1-724)

The same reaction as in Example (68b) was carried out using 1-methyl-4-(4,4,5,5-tetramethyl-1,3,2-dioxaborolan-2-yl)-1H-pyrazole (520 mg, 2.5 mmol) instead of N-(1-methylpiperidin-4-yl)-4-(4,4,S,S-tetramethyl-1,3,2-dioxaborolan-2-yl)benzamide obtained in Example (68a). After purification, 270 mg (yield: 44%) of the title compound was obtained as a white solid.
Melting point: 84 to 86°C.
¹H-NMR (400 MHz, CDCl₃) δ ppm: 7.78-7.72 (2H, m), 7.62 (1H, s), 7.49-7.27 (5H, m), 6.93 (1H, d, J = 7.4 Hz), 3.93 (3H, s), 3.16 (1H, hp, J = 7.0 Hz), 2.51 (3H, s), 1.40 (6H, d, J = 7.0 Hz).
MS(ESI) m/z: 358 (M+H)⁺.

### (Example 71) 1-({3'-[2-Isopropyl-5-(6-methylpyridin-2-yl)-1H-imidazol-4-yl]-1,1'-biphenyl-4-yl}methyl)-4-methylpiperazine (Compound No. 1-690)

### (71a) 3'-[2-Isopropyl-5-(6-methylpyridin-2-yl)-1H-imidazol-4-yl]-1,1'-biphenyl-4-carboxyaldehyde

2-[4-(3-Bromophenyl)-2-isopropyl-1H-imidazol-5-yl]-6-methylpyridine (2.0 g, 5.6 mmol) obtained in Example (1b) and 4-formylphenylboronic acid (1.0 g, 6.8 mmol) were dissolved in 1,2-dimethoxyethane (25 mL). Water (12 mL), a 2 M sodium carbonate aqueous solution (11 mL), and tetrakis(triphenylphosphine) palladium (0.32 g, 0.28 mmol) were added thereto. The resulting mixture was stirred under a nitrogen atmosphere at 90°C for 5 hr. The reaction solution was cooled to room temperature, and water was added thereto. After extraction with methylene chloride, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 1.6 g (yield: 73%) of the title compound as a light yellow amorphous form.
¹H-NMR (400 MHz, CDCl₃) δ ppm: 10.01 (1H, s), 7.93-7.89 (3H, m), 7.75 (2H, d, J = 8.2 Hz), 7.64 (1H, m), 7.58 (1H, m), 7.47 (1H, t, J = 7.8 Hz), 7.39 (1H, t, J = 7.8 Hz), 7.29 (1H, d, J = 7.8 Hz), 6.93 (1H, d, J = 7.8 Hz), 3.16 (1H, hp, J = 7.0 Hz), 2.49 (3H, s), 1.38 (6H, d, J = 7.0 Hz).
MS(ESI) m/z: 382 (M+H)⁺

### (71b) 1-({3'-[2-Isopropyl-5-(6-methylpyridin-2-yl)-1H-imidazol-4-yl]-1,1'-biphenyl-4-yl}methyl)-4-methylpiperazine

3'-[2-Isopropyl-5-(6-methylpyridin-2-yl)-1H-imidazol-4-yl]-1,1'-biphenyl-4-carboxyaldehyde (0.10 g, 0.26 mmol) obtained in Example (71 a) was dissolved in a mixture solution of 1,2-dichloroethane (1.3 mL) and acetic acid (0.2 mL), and 1-methylpiperazine was added thereto. The resulting mixture was stirred at room temperature for 20 min. After addition of sodium triacetoxyborohydride (0.20 g, 0.94 mmol), the resulting mixture was stirred at room temperature for 6 hr. Saturated aqueous sodium bicarbonate and water were added thereto. After extraction with methylene chloride, the organic layer was separated using an Empore cartridge (GL Science). The solvent was evaporated under reduced pressure, the resulting crude product was purified by high-performance liquid chromatography (GL Science ODS-3, eluting solvent; water : acetonitrile = 95 : 5 to 5 : 95) to obtain 0.048 g (yield: 39%) of the title compound as a colorless amorphous form.
¹H-NMR (400 MHz, CDCl₃) δ ppm: 10.12 (1H, brs), 7.85 (1H, s), 7.59-7.52 (4H, m), 7.43 (1H, t, J = 7.8 Hz), 7.38-7.28 (4H, m), 6.90 (1H, d, J = 7.8 Hz), 3.52 (2H, s), 3.16 (1H, hp, J= 7.0 Hz), 2.53 (3H, s), 2.48 (8H, m), 2.28 (3H, s), 1.41 (6H, d, J = 7.0 Hz).
MS(ESI) m/z: 466 (M+H)⁺

### (Example 72) N-({3'-[2-Isopropyl-5-(6-methylpyridin-2-yl)-1H-imidazol-4-yl]-1,1'-biphenyl-4-yl}methyl)-1-methylpiperidin-4-amine (Compound No. 1-692)

The same reaction as in Example (71b) was carried out using 4-amino-1-methylpiperidine (0.10 mL, 0.90 mmol) instead of 1-methylpiperazine. After purification, 0.048 g (yield: 39%) of the title compound was obtained as a yellow amorphous form.
¹H-NMR (400 MHz, CDCl₃) δ ppm: 10.17 (1H, brs), 7.85 (1H, m), 7.59-7.52 (4H, m), 7.43 (1H, t, J = 7.8 Hz), 7.39-7.33 (3H, m), 7.29 (1H, d, J = 7.8 Hz), 6.90 (1H, d, J = 7.8 Hz), 3.83 (2H, s), 3.16 (1H, hp, J = 7.0 Hz), 2.81 (2H, m), 2.52 (3H, s), 2.51 (1H, m), 2.26 (3H, s), 1.98 (2H, m), 1.91 (2H, m), 1.45 (2H, m), 1.41 (6H, d, J = 7.0 Hz).
MS(ESI) m/z: 480 (M+H)⁺

### (Example 73) N-{2-Fluoro-3'-[2-isopropyl-5-(6-methylpyridin-2-yl)-1H-imidazol-4-yl]-1,1'-biphenyl-4-yl}methanesulfonamide (Compound No. 1-676)

4-Bromo-3-fluoroaniline (0.19 g, 1.0 mmol) was dissolved in methylene chloride (3 mL), and pyridine (1 mL) and methanesulfonyl chloride (0.14 g, 1.2 mmol) were added thereto. The resulting mixture was stirred at room temperature for 1 hr, and then water was added to the reaction solution. After extraction with methylene chloride, the organic layer was separated using an Empore cartridge (GL Science). The solvent was evaporated under reduced pressure to obtain 0.25 g (yield: 93%) of N-(4-bromo-3-fluorophenyl)methanesulfonamide as a crude product. The resulting N-(4-bromo-3-fluorophenyl)methanesulfonamide (0.065 g, 0.24 mmol) and 2-{2-isopropyl-4-[3-(4,4,5,5-tetramethyl-1,3,2-dioxaborolan-2-yl)phenyl]-1H-imidazol-5-yl}-6-methylpyridine (0.12 g, 0.30 mmol) obtained in Example (35a) were dissolved in 1,2-dimethoxyethane (2 mL). Tripotassium phosphate n-hydrate (0.14 g, 0.66 mmol) and dichloro[1,1'-bis(diphenylphosphino)ferrocene]palladium(II)-methylene chloride complex (0.025 g, 0.031 mmol) were added thereto. The resulting mixture was heated under a nitrogen atmosphere under reflux for 6 hr. The reaction solution was cooled to room temperature and diluted with ethyl acetate. After filtration through silica gel, the filtrate was concentrated under reduced pressure. The resulting crude product was purified by high-performance liquid chromatography (GL Science ODS-3, eluting solvent; water : acetonitrile = 95 : 5 to 5 : 95) to obtain 0.038 g (yield: 34%) of the title compound as a white solid.
Melting point: 179 to 184°C.
¹H-NMR (500 MHz, CDCl₃) δ ppm: 7.73 (1H, s), 7.60 (1H, s), 7.51 (1H, m), 7.43-7.35 5 (4H, m), 7.24 (1H, m), 7.08 (1H, m), 7.01 (1H, m), 6.96 (1H, d, J = 7.3 Hz), 3.22 (1H, m), 3.03 (3H, s), 2.57 (3H, s), 1.47 (6H, d, J = 7.3 Hz).
MS(ESI) m/z: 465 (M+H)⁺

### (Example 74) N-{2-Fluoro-3'-[2-isopropyl-5-(6-methylpyridin-2-yl)-1H-imidazol-4-yl]-1,1'-biphenyl-4-yl}cyclopropanesulfonamide (Compound No. 1-678)

The same reaction as in Example 73 was carried out using cyclopropanesulfonyl chloride (0.28 g, 2.0 mmol) instead of methanesulfonyl chloride. After purification, 0.12 g (yield: 51%) of the title compound was obtained as a white solid.
Melting point: 124 to 128°C.
¹H-NMR (500 MHz, CDCl₃) δ ppm: 10.49 (1H, brs), 9.06 (1H, brs), 7.76 (1H, s), 7.59 (1H, d, J = 6.8 Hz), 7.45-7.40 (3H, m), 7.35 (1H, d, J = 8.3 Hz), 7.23 (1H, m), 7.11 (1H, m), 7.02 (1H, m), 6.95 (1H, d, J = 7.3 Hz), 3.22 (1H, m), 2.52 (3H, s), 2.15 (1H, m), 1.42 (6H, d, J = 7.3 Hz), 1.06-1.04 (2H, m), 0.80-0.78 (2H, m).
MS(ESI) m/z: 491 (M+H)⁺

### (Example 75) 2-Fluoro-3'-[2-isopropyl-5-(6-methylpyridin-2-yl)-1H-imidazol-4-y1]-1,1'-biphenyl-4-sulfonamide (Compound No. 1-680)

2-{2-Isopropyl-4-[3-(4,4,5,5-tetramethyl-1,3,2-dioxaborolan-2-yl)phenyl]-1H-imidazol-5-yl}-6-methylpyridine (0.12 g, 0.30 mmol) obtained in Example (35a) and 4-bromo-3-fluorobenzenesulfonamide (0.078 g, 0.31 mmol) were dissolved in 1,2-dimethoxyethane (2 mL). Tripotassium phosphate n-hydrate (0.014 g, 0.67 mmol) and dichloro[1,1'-bis(diphenylphosphino)ferrocene]palladium(II)-methylene chloride complex (0.028 g, 0.034 mmol) were added thereto. The resulting mixture was heated under a nitrogen atmosphere under reflux for 18 hr. The reaction solution was cooled to room temperature and then diluted with ethyl acetate. After filtration through silica gel, the filtrate was concentrated under reduced pressure. The resulting crude product was purified by silica gel column chromatography (NH, eluting solvent; hexane : ethyl acetate = 1 : 9) to obtain 0.060 g (yield: 44%) of the title compound as a white solid.
Melting point: 246 to 248°C.
¹H-NMR (500 MHz, DMSO-d₆) δ ppm: 12.11 (0.5H, s), 12.06 (0.5H, s), 8.00 (0.5H, s), 7.83 (0.5H, s), 7.80-7.46 (9.5H, m), 7.26 (0.5H, d, J = 7.8 Hz), 7.12 (0.5H, d, J = 7.8 Hz), 7.02 (0.5H, dd, J = 1.5, 6.8 Hz), 3.08 (1H, m), 2.49 (3H, s), 1.33-1.31 (6H, m).
MS(ESI) m/z: 451 (M+H)⁺.

### (Example 76) 6-{3-[2-Isopropyl-5-(6-methylpyridin-2-yl)-1H-imidazol-4-yl]phenyl}pyridine-3-amine (Compound No. 1-684)

### (76a) 2-{3-[2-Isopropyl-5-(6-methylpyridin-2-yl)-1H-imidazol-4-yl]phenyl}-5-nitropyridine

The same reaction as in Example 75 was carried out using 2-chloro-5-nitropyridine (0.090 g, 0.57 mmol) instead of 4-bromo-3-fluorobenzenesulfonamide. After purification, 0.13 g (yield: 63%) of the title compound was obtained as a yellow amorphous form.
¹H-NMR (400 MHz, CDCl₃) δ ppm: 9.45 (1H, d, J = 2.3 Hz), 8.48 (1H, dd, J = 2.3, 8.6 Hz), 8.33 (1H, m), 8.09 (1H, d, J = 7.4 Hz), 7.92 (1H, d, J = 8.6 Hz), 7.73 (1H, d, J = 7.4 Hz), 7.52 (1H, m), 7.37 (1H, m), 7.25 (1H, d, J = 7.8 Hz), 6.93 (1H, d, J = 7.4 Hz), 3.16 (1H, m), 2.50 (3H, s), 1.39 (6H, d, J = 7.0 Hz).
MS(ESI) m/z: 400 (M+H)⁺

### (76b) 6-{3-[2-Isopropyl-5-(6-methylpyridin-2-yl)-1H-imidazol-4-yl]phenyl}pyridine-3-amine

2-{3-[2-Isopropyl-5-(6-methylpyridin-2-yl)-1H-imidazol-4-yl]phenyl}-5-nitropyridine (0.12 g, 0.31 mmol) obtained in Example (76a) was dissolved in ethanol (3 mL), and 20% palladium hydroxide-carbon (0.025 g) was added thereto. The resulting mixture was stirred under a hydrogen atmosphere at room temperature for 4 hr. The reaction mixture was filtered, and the filtrate was concentrated under reduced pressure. The resulting crude product was purified by high-performance liquid chromatography (GL Science ODS-3, eluting solvent; water : acetonitrile = 95 : 5 to 5 : 95) to obtain 0.059 g (yield: 52%) of the title compound as a reddish brown amorphous form.
Melting point: 119 to 124°C.
¹H-NMR (500 MHz, DMSO-d₆) δ ppm: 8.23 (1H, s), 8.05 (1H, d, J = 7.8 Hz), 8.03 (1H, d, J = 2.9 Hz), 7.80 (1H, m), 7.72 (1H, m), 7.57 (1H, m), 7.51 (1H, m), 7.32 (1H, d, J = 7.8 Hz), 7.25 (1H, d, J = 7.8 Hz), 7.15 (1H, m), 3.45 (1H, m), 2.49 (3H, s), 1.44 (6H, d, J = 6.8 Hz).
MS(ESI) m/z: 370 (M+H)⁺

### (Example 77) N-(6-{3-[2-Isopropyl-5-(6-methylpyridin-2-yl)-1H-imidazol-4-yl]phenyl}pyridine-3-yl)methanesulfonamine (Compound No. 1-686)

6-{3-[2-Isopropyl-5-(6-methylpyridin-2-yl)-1H-imidazol-4-yl]phenyl}pyridine-3-amine (0.050, 0.14 mmol) obtained in Example (76b) was dissolved in methylene chloride (0.5 mL), and pyridine (0.5 mL) and methanesulfonyl chloride (0.025 g, 0.22 mmol) were added thereto. The resulting mixture was stirred at room temperature for 1 hr. The reaction solution was concentrated under reduced pressure. To the resulting crude product, acetonitrile, water, and saturated aqueous sodium bicarbonate were added for neutralization. The resulting mixture was purified by high-performance liquid chromatography (GL Science ODS-3, eluting solvent; water : acetonitrile = 95 : 5 to 5 : 95) to obtain 0.022 g (yield: 36%) of the title compound as a pink solid.
Melting point: 128 to 131°C.
¹H-NMR (500 MHz, CDCl₃) δ ppm: 8.54 (1H, d, J = 2.5 Hz), 8.11 (1H, s), 7.83 (1H, d, J = 7.8 Hz), 7.60-7.57 (2H, m), 7.48 (1H, d, J = 8.3 Hz), 7.44 (1H, m), 7.35 (1H, m), 7.20 (1H, d, J = 7.8 Hz), 6.93 (1H, d, J = 7.3 Hz), 3.23 (1H, m), 2.59 (3H, s), 2.55 (3H, s), 1.48 (6H, d, J = 6.8 Hz).
MS(ESI) m/z: 448 (M+H)⁺

### (Example 78) 2-{2-Isopropyl-4-[3-(1H-pyrrol-3-yl)phenyl]-1H-imidazol-5-yl}-6-methylpyridine (Compound No. 1-682)

2-{2-Isopropyl-4-[3-(4,4,5,5-tetramethyl-1,3,2-dioxaborolan-2-yl)phenyl]-1H-imidazol-5-yl}-6-methylpyridine (0.21 g, 0.51 mmol) obtained in Example (35a) and t-butyl 3-bromo-1H-pyrrole-1-carboxylate (0.13 g, 0.53 mmol) were dissolved in 1,2-dimethoxyethane (5 mL). Tripotassium phosphate n-hydrate (0.22 g, 1.0 mmol) and dichloro[1,1'-bis(diphenylphosphino)ferrocene]palladium(II)-methylene chloride complex (0.050 g, 0.061 mmol) were added thereto. The resulting mixture was heated under a nitrogen atmosphere under reflux for 4 hr. The reaction solution was diluted with ethyl acetate. After filtration through silica gel, the filtrate was concentrated under reduced pressure. The resulting crude product was purified by silica gel column chromatography (NH, eluting solvent; hexane : ethyl acetate = 3 : 2) to obtain 0.14 g (yield: 62%) of t-butyl 3-{3-[2-isopropyl-5-(6-methylpyridin-2-yl)-1H-imidazol-4-yl]phenyl}-1H-pyrrole-1-carboxylate as a white amorphous form. The resulting t-butyl 3-{3-[2-isopropyl-5-(6-methylpyridin-2-yl)-1H-imidazol-4-yl]phenyl}-1H-pyrrole-1-carboxylate (0.14 g, 0.30 mmol) was dissolved in tetrahydrofuran (3 mL), and a 25% sodium methoxide-methanol solution (0.50 mL, 2.3 mmol) was added thereto. The resulting mixture was stirred at room temperature for 1 hr. To this reaction solution, water was added. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.077 g (yield: 76%) of the title compound as a white solid.
Melting point: 112 to 114°C.
¹H-NMR (500 MHz, CDC1₃) δ ppm: 8.59 (1H, brs), 7.79 (1H, s), 7.50 (1H, d, J = 7.3 Hz), 7.41-7.31 (4H, m), 7.06 (1H, s), 6.90 (1H, d, J = 7.3 Hz), 6.79 (1H, d, J = 2.0 Hz), 6.52 (1H, s), 3.17 (1H, m), 2.49 (3H, s), 1.38 (6H, d, J = 6.8 Hz).
MS(ESI) m/z: 343 (M+H)⁺

### (Example 79) 6-{3-[2-Isopropyl-5-(6-methylpyridin-2-yl)-1H-imidazol-4-yl]phenyl}-2,3-dihydro-4H-thiochromen-4-one (Compound No. 1-784)

The same reaction as in Example 75 was carried out using 6-bromo-2,3-dihydro-4H-thiochromen-4-one (0.027 g, 1.1 mmol) instead of 4-bromo-3-fluorobenzenesulfonamide. After purification, 0.26 g (yield: 59%) of the title compound was obtained as a yellow amorphous form.
¹H-NMR (500 MHz, CDCl₃) δ ppm: 8.36 (1H, d, J = 2.0 Hz), 7.89 (1H, s), 7.66 (1H, dd, J = 2.0, 7.8 Hz), 7.62 (1H, d, J = 7.3 Hz), 7.56 (1H, d, J = 7.8 Hz), 7.46 (1H, m), 7.42 (1H, m), 7.34-7.31 (2H, m), 6.95 (1H, d, J = 7.8 Hz), 3.27 (2H, t, J = 6.3 Hz), 3.17 (1H, m), 3.01 (2H, t, J = 6.3 Hz), 2.52 (3H, s), 1.41 (6H, d, J = 6.8 Hz). MS(ESI) m/z: 440 (M+H)⁺

### (Example 80) 6-{3-[2-Isopropyl-5-(6-methylpyridin-2-yl)-1H-imidazol-4-yl]phenyl}-2,3-dihydro-4H-thiochromen-4-one 1-oxide (Compound No. 1-786)

Water (1 mL) and sodium periodate (0.049 g, 0.23 mmol) were added to a methanol solution (2 mL) of 6-{3-[2-isopropyl-5-(6-methylpyridin-2-yl)-1H-imidazol-4-yl]phenyl}-2,3-dihydro-4H-thiochromen-4-one (0.089 g, 0.20 mmol) obtained in Example 79. The resulting mixture was stirred at room temperature for 20 hr. The reaction solution was concentrated under reduced pressure, and acetonitrile and water were added to the resulting crude product. Then, the crude product was purified by high-performance liquid chromatography (GL Science ODS-3, eluting solvent; water : acetonitrile = 95 : 5 to 5 : 95) to obtain 0.049 g (yield: 53%) of the title compound as a white solid.
Melting point: 145 to 148°C.
¹H-NMR (400 MHz, CDCl₃) δ ppm: 8.38 (1H, s), 8.00 (1H, d, J = 8.3 Hz), 7.93-7.91 (2H, m), 7.68 (1H, d, J = 7.4 Hz), 7.62 (1H, d, J = 7.4 Hz), 7.54-7.46 (2H, m), 7.31 (1H, d, J = 7.8 Hz), 7.02 (1H, d, J = 7.8 Hz), 3.64-3.50 (3H, m), 3.22 (1H, m), 2.94 (1H, m), 2.52 (3H, s), 1.39 (6H, d, J = 7.0 Hz).
MS(ESI) m/z: 456 (M+H)⁺.

### (Example 81) 6-{3-[2-Isopropyl-5-(6-methylpyridin-2-yl)-1H-imidazol-4-yl]phenyl}-2,3-dihydro-4H-thiochromen-4-one 1,1-dioxide (Compound No. 1-788)

6-{3-[2-Isopropyl-5-(6-methylpyridin-2-yl)-1H-imidazol-4-yl]phenyl}-2,3-dihydro-4H-thiochromen-4-one (0.045 g, 0.10 mmol) obtained in Example 79 was dissolved in methylene chloride (3 mL), and m-chloroperbenzoic acid (0.036 g, 0.21 mmol) was added thereto. The resulting mixture was stirred at room temperature for 20 hr. To this reaction solution, saturated aqueous sodium bicarbonate was added. After extraction with methylene chloride, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.0047 g (yield: 10%) of the title compound as a white solid.
Melting point: 115 to 119°C.
¹H-NMR (400 MHz, CDCl₃) δ ppm: 10.31 (1H, brs), 8.35 (1H, s), 8.08-8.01 (2H, m), 7.94 (1H, s), 7.73 (1H, d, J = 7.4 Hz), 7.59 (1H, d, J = 7.8 Hz), 7.52 (1H, m), 7.43 (1H, m), 7.30 (1H, d, J = 7.8 Hz), 6.97 (1H, d, J = 7.4 Hz), 3 . 73 (2H, t, J = 6.3 Hz), 3.45 (2H, t, J = 6.3 Hz), 3.17 (1H, m), 2.54 (3H, s), 1.42 (6H, d, J = 7.0 Hz). MS(ESI) m/z: 472 (M+H)⁺

### (Example 82) 2-{4-[3-(3,4-Dihydro-2H-thiochromen-6-yl)phenyl]-2-isopropyl-1H-imidazol-5-yl}-6-methylpyridine (Compound No. 1-778)

The same reaction as in Example 75 was carried out using 6-bromothiochromane (0.35 g, 1.5 mmol) instead of 4-bromo-3-fluorobenzenesulfonamide. After purification, 0.38 g (yield: 60%) of the title compound was obtained as a white solid.
Melting point: 115 to 117°C.
¹H-NMR (500 MHz, CDCl₃) δ ppm: 7.82 (1H, s), 7.56 (1H, d, J = 7.3 Hz), 7.52 (1H, d, J = 8.3 Hz), 7.43 (1H, m), 7.37 (1H, m), 7.32-7.29 (3H, m), 7.12 (1H, d, J = 8.3 Hz), 6.92 (1H, d, J = 7.8 Hz), 3.19 (1H, m), 3.05 (2H, t, J = 5.9 Hz), 2.86 (2H, t, J = 5.9 Hz), 2.56 (3H, s), 2.17-2.12 (2H, m), 1.44 (6H, d, J = 6.8 Hz).
MS(ESI) m/z: 426 (M+H)⁺

### (Example 83) 2-{2-Isopropyl-4-[3-(1-oxido-3,4-dihydro-2H-thiochromen-6-yl)phenyl]-1H-imidazol-5-yl}-6-methylpyridine (Compound No. 1-780)

The same reaction as in Example 80 was carried out using 2-{4-[3-(3,4-dihydro-2H-thiochromen-6-yl)phenyl]-2-isopropyl-1H-imidazol-5-yl}-6-methylpyridine (0.092 g, 0.22 mmol) obtained in Example 82 instead of 6-{3-[2-isopropyl-5-(6-methylpyridin-2-yl)-1H-imidazol-4-yl]phenyl}-2,3-dihydro-4H-thiochromen-4-one obtained in Example 79. After purification, 0.049 g (yield: 51%) of the title compound was obtained as a white solid.
Melting point: 136 to 139°C ¹H-NMR (500 MHz, CDCl₃) δ ppm: 7.83 (1H, s), 7.66 (1H, d, J = 8.3 Hz), 7.62-7.56 (2H, m), 7.52-7.49 (4H, m), 7.28 (1H, d, J = 6.8 Hz), 7.08 (1H, d, J = 7.8 Hz), 3.37 (1H, m), 3.18 (1H, m), 3.11 (1H, m), 3.00 (1H, m), 2.90 (1H, m), 2.55 (3H, s), 2.51 (1H, m), 2.08 (1H, m), 1.40 (6H, d, J = 6.8 Hz).
MS(ESI) m/z: 442 (M+H)⁺

### (Example 84) 2-{4-[3-(1,1-Dioxido-3,4-dihydro-2H-thiochromen-6-yl)phenyl]-2-isopropyl-1H-imidazol-5-yl}-6-methylpyridine (Compound No. 1-782)

Water (2 mL) and sodium periodate (0.17 g, 0.77 mmol) were added to a methanol solution (6 mL) of 2-{4-[3-(3,4-dihydro-2H-thiochromen-6-yl)phenyl]-2-isopropyl-1H-imidazol-5-yl}-6-methylpyridine (0.11 g, 0.25 mmol) obtained in Example 82. The resulting mixture was heated under reflux for 8 hr. The reaction solution was cooled to room temperature, and then water was added thereto. After extraction with methylene chloride, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.082 g (yield: 73%) of the title compound as a white amorphous form.
Melting point: 138 to 141%
¹H-NMR (500 MHz, CDCl₃) δ ppm: 7.94 (1H, d, J = 8.3 Hz), 7.87 (1H, s), 7.63 (1H, d, J = 7.8 Hz), 7.59 (1H, d, J = 8.3 Hz), 7.54 (1H, d, J = 7.8 Hz), 7.49-7.42 (3H, m), 7.29 (1H, d, J = 7.8 Hz), 6.98 (1H, d, J = 7.8 Hz), 3.39-3.37 (2H, m), 3.17 (1H, m), 3.07 (2H, t, J = 6.3 Hz), 2.55-2.50 (2H, m), 2.47 (3H, s), 1.35 (6H, d, J = 6.8 Hz). MS(ESI) m/z: 458 (M+H)⁺

### (Example 85) 2-{4-[3-(1,1-Dioxido-1-benzothien-5-yl)phenyl]-2-isopropyl-1H-imidazol-5-yl}-6-methylpyridine (Compound No. 1-790)

The same reaction as in Example 75 was carried out using 5-bromo-1-benzothiophene 1,1-dioxide (0.27 g, 1.1 mmol) instead of 4-bromo-3-fluorobenzenesulfonamide. After purification, 0.15 g (yield: 35%) of the title compound was obtained as a white solid.
Melting point: 137 to 138°C ¹H-NMR (500 MHz, CDCl₃) δ ppm: 7.89 (1H, s), 7.77-7.73 (2H, m), 7.69 (1H, d, J = 7.8 Hz), 7.59 (1H, s), 7.55-7.48 (2H, m), 7.39 (1H, m), 7.29-7.24 (2H, m), 6.95 (1H, d, J = 7.8 Hz), 6.75 (1H, d, J = 6.8 Hz), 3.18 (1H, m), 2.56 (3H, s), 1.43 (6H, d, J = 6.8 Hz).
MS(ESI) m/z: 442 (M+H)⁺.

### (Example 86) 2-{4-[3-(1,1-Dioxido-2,3-dihydro-1-benzothien-5-yl)phenyl]-2-isopropyl-1H-imidazol-5-yl}-6-methylpyridine (Compound No. 1-792)

A 10% palladium-carbon (0.050 g) was added to an ethanol solution (10 mL) of 2- {4-[3-(1,1-dioxido-1-benzothien-5-yl)phenyl]-2-isopropyl-1H-imidazol-5-yl} -6-methylpyridine (0.095 g, 0.22 mmol) obtained in Example 85. The resulting mixture was stirred under a hydrogen atmosphere at room temperature for 2 hr. The reaction solution was filtered, and the filtrate was concentrated under reduced pressure. The resulting crude product was purified by high-performance liquid chromatography (GL Science ODS-3, eluting solvent; water : acetonitrile = 95 : 5 to 5 : 95) to obtain 0.086 g (yield: 90%) of the title compound as a white solid.
Melting point: 128 to 131°C
¹H-NMR (500 MHz, CDCl₃) δ ppm: 10.57 (1H, brs), 7.89 (1H, s), 7.77 (1H, d, J = 8.3 Hz), 7.69-7.67 (2H, m), 7.60 (1H, s), 7.53 (1H, d, J = 7.3 Hz), 7.48 (1H, m), 7.40 (1H, m), 7.29 (1H, d, J = 7.8 Hz), 6.95 (1H, d, J = 7.3 Hz), 3.53 (2H, t, J = 6.8 Hz), 3.43 (2H, t, J = 6.8 Hz), 3.16 (1H, m), 2.50 (3H, s), 1.39 (6H, d, J = 7.3 Hz). MS(ESI) m/z: 444 (M+H)⁺

### (Example 87) N-(6-{3-[2-Isopropyl-5-(6-methylpyridin-2-yl)-1H-imidazol-4-yl]phenyl}pyridin-3-yl)cyclopropanesulfonamide (Compound No. 1-688)

The same reaction as in Example 77 was carried out using cyclopropanesulfonyl chloride (0.078 g, 0.56 mmol) instead of methanesulfonyl chloride. After purification, 0.079 g (yield: 61%) of the title compound was obtained as a white solid.
Melting point: 133 to 136°C ¹H-NMR (500 MHz, CDCl₃) δ ppm: 8.54 (1H, d, J = 2.4 Hz), 8.11 (1H, s), 7.90 (1H, d, J = 7.8 Hz), 7.65 (1H, dd, J = 2.4, 8.3 Hz), 7.57 (1H, d, J = 8.3 Hz), 7.55 (1H, d, J = 7.8 Hz), 7.42 (1H, m), 7.36 (1H, m), 7.20 (1H d, J = 7.8 Hz), 6.94 (1H, d, J = 7.8 Hz), 3.30 (1H, m), 2.51 (3H, s), 2.34 (1H, m), 1.46 (6H, d, J = 7.3 Hz), 1.10-1.07 (2H, m), 0.89-0.85 (2H, m).
MS(ESI) m/z: 474 (M+H)⁺

### (Example 88) 2-{3-[2-Isopropyl-5-(6-methylpyridin-2-yl)-1H-imidazol-4-yl]phenyl}-5-(methylsulfonyl)pyridine (Compound No. 1-536)

The same reaction as in Example 75 was carried out using 2-bromo-5-(methylsulfonyl)pyridine (0.13 g, 0.50 mmol) instead of 4-bromo-3-fluorobenzenesulfonamide. After purification, 0.11 g (yield: 52%) of the title compound was obtained as a white solid.
Melting point: 112 to 114°C
¹H-NMR (500 MHz, CDCl₃) δ ppm: 10.87 (1H, brs), 9.18 (1H, d, J = 2.4 Hz), 8.34 (1H, s), 8.23 (1H, dd, J = 2.4, 8.3 Hz), 8.10 (1H, d, J = 7.3 Hz), 7.94 (1H, d, J = 8.3 Hz), 7.75 (1H, d, J = 7.8 Hz), 7.54 (1H, m), 7.39 (1H, m), 7.27 (1H, d, J = 7.3 Hz), 6.95 (1H, d, J = 7.3 Hz), 3.15 (1H, m), 3.14 (3H, s), 2.47 (3H, s), 1.37 (6H, d, J = 6.8 Hz).
MS(ESI) m/z: 433 (M+H)⁺

### (Example 89) 2-{2-Isopropyl-4-[3-(1H-pyrazol-1-yl)phenyl]-1H-imidazol-5-yl}-6-methylpyridine (Compound No. 1-730)

2-[4-(3-Bromophenyl)-2-isopropyl-1H-imidazol-5-yl]-6-methylpyridine (0.36 g, 1.0 mmol) obtained in Example (1b) was dissolved in N-methyl-2-pyrrolidinone (2 mL), and 1H-pyrazole (0.34 g, 5.0 mmol), potassium carbonate (0.32 g, 2.3 mmol), and copper(I) iodide (0.022 g, 0.12 mmol) were added thereto. The resulting mixture was stirred at 195°C for 1 hr in a microwave reaction device. The reaction mixture was cooled to room temperature, and then water was added thereto. After extraction with ethyl acetate, the organic layer was washed with water and brine, and then dried with anhydrous magnesium sulfate. The solvent was evaporated under reduced pressure. The resulting crude product was filtered through silica gel and then purified by high-performance liquid chromatography (GL Science ODS-3, eluting solvent; water : acetonitrile = 95 : 5 to 5 : 95) to obtain 0.066 g (yield: 19%) of the title compound as a white amorphous form.
¹H-NMR (500 MHz, CDCl₃) δ ppm: 10.81 (1H, brs), 7.95-7.92 (2H, m), 7.76 (1H, m), 7.74 (1H, d, J = 2.0 Hz), 7.53 (1H, d, J = 7.8 Hz), 7.46 (1H, m), 7.41 (1H, m), 7.28 (1H, m), 6.94 (1H, d, J = 7.8 Hz), 6.44 (1H, t, J = 2.0 Hz), 3.13 (1H, m), 2.46 (3H, s), 1.35 (6H, d, J = 7.3 Hz).
MS(ESI) m/z: 344 (M+H)⁺

### (Example 90) 2-{4-[3-(1H-Imidazol-1-yl)phenyl]-2-isopropyl-1H-imidazol-5-yl}-6-methylpyridine (Compound No. 1-764)

The same reaction as in Example 89 was carried out using 1H-imidazole (0.096 g, 1.4 mmol) instead of 1H-pyrazole. After purification, 0.12 g (yield: 56%) of the title compound was obtained as a white solid.
Melting point: 158 to 161°C ¹H-NMR (500 MHz, CDCl₃) δ ppm: 11.02 (1H, s), 7.86 (1H, s), 7.72 (1H, s), 7.63 (1H, d, J = 7.3 Hz), 7.49-7.43 (2H, m), 7.34-7.29 (3H, m), 7.19 (1H, s), 6.97 (1H, d, J = 7.3 Hz), 3.13 (1H, m), 2.45 (3H, s), 1.33 (6H, d, J = 6.8 Hz).
MS(ESI) m/z: 344 (M+H)⁺.

### (Example 91) 2-{2-Isopropyl-4-[3-(1H-1,2,4-triazol-1-yl)phenyl]-1H-imidazol-5-yl}-6-methylpyridine (Compound No. 1-762)

The same reaction as in Example 89 was carried out using 1H-1,2,4-triazole (0.077 g, 1.1 mmol) instead of 1H-pyrazole. After purification, 0.069 g (yield: 39%) of the title compound was obtained as a white amorphous form.
¹H-NMR (500 MHz, CDCl₃) δ ppm: 11.06 (1H, brs), 8.58 (1H, s), 8.10 (1H, s), 7.97 (1H, d, J = 2.0 Hz), 7.69 (1H, d, J = 7.8 Hz), 7.65 (1H, d, J = 7.8 Hz), 7.51 (1H, m), 7.44 (1H, m), 7.28 (1H, d, J = 7.8 Hz), 6.97 (1H, d, J = 7.3 Hz), 3.13 (1H, m), 2.44 (3H, s), 1.33 (6H, d, J = 6.8 Hz).
MS(ESI) m/z: 345 (M+H)⁺

### (Example 92) N-{2'-Fluoro-5'-[2-isopropyl-5-(6-methylpyridin-2-yl)-1H-imidazol-4-yl]-1,1'-biphenyl-4-yl}-N'-(4-methylpiperazin-1-yl)urea (Compound No. 1-575)

2-[4-(3-Bromo-4-fluorophenyl)-2-isopropyl-1H-imidazol-5-yl]-6-methylpyridine (0.10 g, 0.27 mmol) obtained in Example (60b) and N-(4-methylpiperazin-1-yl)-N'-[4-(4,4,5,5-tetramethyl-1,3,2-dioxaborolan-2-yl)phenyl]urea (0.11 g, 0.29 mmol) obtained in Example (59a) were dissolved in 1,2-dimethoxyethane (3 mL), and water (1.5 mL), a 2 M sodium carbonate aqueous solution (0.80 mL), and tetrakis(triphenylphosphine) palladium (0.016 g, 0.014 mmol) were added thereto. The resulting mixture was stirred under a nitrogen atmosphere at 90°C for 3 hr. The reaction solution was cooled to room temperature, and water was added thereto. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.087 g (yield: 61%) of the title compound as a white solid.
Melting point: 145°C ¹H-NMR (400 MHz, CDCl₃) δ ppm: 10.27 (1H, brs), 8.14 (1H, s), 7.70 (1H, dd, J = 2.3, 7.8 Hz), 7.56-7.49 (5H, m), 7.40 (1H, t, J = 7.4 Hz), 7.28 (1H, d, J = 7.8 Hz), 7.16 (1H, m), 6.94 (1H, d, J = 7.4 Hz), 5.37 (1H, s), 3.15 (1H, hp, J = 7.0 Hz), 3.08 (2H, m), 2.81 (2H, m), 2.66 (2H, m), 2.53 (3H, s), 2.34 (3H, s), 2.33 (2H, m), 1.40 (6H, d, J = 7.0 Hz).
MS(ESI) m/z: 528 (M+H)⁺

### (Example 93) 2-{4-[4-Fluoro-3-(1H-pyrrol-2-yl)phenyl]-2-isopropyl-1H-imidazol-5-yl}-6-methylpyridine (Compound No. 1-301)

### (93a) t-Butyl 2-{2-fluoro-5-[2-isopropyl-5-(6-methylpyridin-2-yl)-1H-imidazol-4-yl]phenyl}-1H-pyrrole-1-carboxylate

2-[4-(3-Bromo-4-fluorophenyl)-2-isopropyl-1H-imidazol-5-yl]-6-methylpyridine (0.10 g, 0.27 mmol) obtained in Example (60b) and 1-(t-butoxycarbonyl)pyrrole-2-boronic acid (0.062 g, 0.29 mmol) were dissolved in 1,2-dimethoxyethane (2 mL), and water (0.5 mL), tripotassium phosphate n-hydrate (0.12 g, 0.54 mmol), and dichloro[1,1'-bis(diphenylphosphino)ferrocene]palladium(II)-methylene chloride complex (0.022 g, 0.027 mmol) were added thereto. The resulting mixture was stirred under a nitrogen atmosphere at 90°C for 6 hr. The reaction solution was cooled to room temperature, and water was added thereto. After extraction with ethyl acetate, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.66 g (yield: 53%) of the title compound as a white solid.
¹H-NMR (400 MHz, CDCl₃) δ ppm: 9.95 (1H, brs), 7.61 (1H, m), 7.57 (1H, m), 7.41 (1H, m), 7.36 (1H, t, J = 7.8 Hz), 7.27 (1H, m), 7.09 (1H, t, J = 7.8 Hz), 6.93 (1H, d, J = 7.4 Hz), 6.25-6.21 (2H, m), 3.17 (1H, hp, J = 7.0 Hz), 2.55 (3H, s), 1.42 (6H, d, J = 7.0 Hz), 1.41 (9H, s).
MS(ESI) m/z: 461 (M+H)⁺

### (93b) 2-{4-[4-Fluoro-3-(1H-pyrrol-2-yl)phenyl]-2-isopropyl-1H-imidazol-5-yl}-6-methylpyridine

t-Butyl 2-{2-fluoro-5-[2-isopropyl-5-(6-methylpyridin-2-yl)-1H-imidazol-4-yl]phenyl}-1H-pyrrole-1-carboxylate obtained in Example (93 a) was dissolved in tetrahydrofuran (2 mL), and a 25% sodium methoxide-methanol solution (0.3 mL) was added thereto. The resulting mixture was stirred at room temperature for 2 hr. The reaction solvent was evaporated under reduced pressure, and water was added to the residue. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.044 g (yield: 90%) of the title compound as a white solid.
Melting point: 103 to 106°C ¹H-NMR (400 MHz, CDCl₃) δ ppm: 10.06 (1H, brs), 9.05 (1H, brs), 7.85 (1H, m), 7.40-7.33 (2H, m), 7.24-7.20 (1H, m), 7.11 (1H, m), 6.91 (1H, d, J = 7.0 Hz), 6.88 (1H, m), 6.58 (1H, s), 6.26 (1H, m), 3.15 (1H, hp, J = 7.0 Hz), 2.54 (3H, s), 1.41 (6H, d, J = 7.0 Hz).
MS(ESI) m/z: 361 (M+H)⁺

### (Example 94) N-{2'-Fluoro-5'-[2-isopropyl-4-(6-methylpyridin-2-yl)-1H-imidazol-4-yl]-1,1'-biphenyl-4-yl}-N'-morpholin-4-ylurea (Compound No. 1-604)

The same reaction as in Example 92 was carried out using N-morpholin-4-yl-N'-[4-(4,4,5,5-tetramethyl-1,3,2-dioxaborolan-2-yl)phenyl]urea (0.069 g, 0.20 mmol) obtained in Example (67a) instead of N-(4-methylpiperazin-1-yl)-N'-[4-(4,4,5,5-tetramethyl-1,3,2-dioxaborolan-2-yl)phenyl]urea obtained in Example (59a). After purification, 0.034 g (yield: 34%) of the title compound was obtained as a white solid. Melting point: 141 to 143°C ¹H-NMR (400 MHz, CDCl₃) δ ppm: 9.99 (1H, brs), 8.11 (1H, s), 7.70 (1H, m), 7.56-7.52 (5H, m), 7.40 (1H, t, J = 7.8 Hz), 7.27 (1H, m), 7.16 (1H, m), 6.94 (1H, d, J = 7.8 Hz), 5.34 (1H, s), 3.93 (2H, m), 3.73 (2H, m), 3.17 (1H, hp, J = 7.0 Hz), 3.03 (2H, m), 2.65 (2H, m), 2.55 (3H, s), 1.42 (6H, d, J = 7.0 Hz).
MS(ESI) m/z: 515 (M+H)⁺

### (Example 95) N-{2'-Fluoro-S'-[2-isopropyl-5-(6-methylpyridin-2-yl)-1H-imidazol-4-yl]-1,1'-biphenyl-4-yl}-N'-(1-methylpiperidin-4-yl)urea (Compound No. 1-670)

### (95a) N-(1-Methylpiperidin-4-yl)-N'-[4-(4,4,S,S-tetramethyl-1,3,2-dioxaborolan-2-yl)phenyl]urea

2-(4-Isocyanatephenyl)-4,4,5,5-tetramethyl-1,3,2-dioxaborolane (0.20 g, 0.82 mmol) was dissolved in methylene chloride (2 mL), and 4-amino-1-methylpiperidine (0.10 g, 0.90 mmol) was added thereto. The resulting mixture was stirred at room temperature for 5 hr. The reaction solvent was evaporated under reduced pressure to obtain 0.29 g (yield: 98%) of a crude product of the title compound as a white solid.
¹H-NMR (400 MHz, CDCl₃) δ ppm: 7.72 (2H, d, J = 8.2 Hz), 7.27 (2H, d, J = 8.2 Hz), 6.35 (1H, s), 4.64 (1H, m), 3.69 (1H, m), 2.75 (2H, m), 2.26 (3H, s), 2.08 (2H, m), 1.97 (2H, m), 1.44 (2H, m), 1.33 (12H, s).

### (95b) N-{2'-Fluoro-5'-[2-isopropyl-5-(6-methylpyridin-2-yl)-1H-imidazol-4-yl]-1,1'-biphenyl-4-yl} -N'-(1-methylpiperidin-4-yl)urea

The same reaction as in Example 92 was carried out using N-(1-methylpiperidin-4-yl)-N'-[4-(4,4,5,5-tetramethyl-1,3,2-dioxaborolan-2-yl)phenyl]urea (0.11 g, 0.29 mmol) obtained in Example (95a) instead of N-(4-methylpiperazin-1-yl)-N'-[4-(4,4,5,5-tetramethyl-1,3,2-dioxaborolan-2-yl)phenyl]urea obtained in Example (59a). After purification, 0.039 g (yield: 27%) of the title compound was obtained as a light yellow solid.
Melting point: 145°C ¹H-NMR (400 MHz, CDCl₃) δ ppm: 10.10 (1H, brs), 7,59 (1H, m), 7.47 (1H, m), 7.42 (1H, m), 7.38 (1H, t, J = 7.8 Hz), 7.31-7.25 (3H, m), 7.21 (1H, d, J = 7.8 Hz), 7.09 (1H, m), 6.93 (1H, d, J = 7.8 Hz), 5.03 (1H, m), 3.61 (1H, m), 3.16 (1H, hp, J = 7.0 Hz), 2.71 (2H, m), 2.55 (3H, s), 2.23 (3H, s), 2.07 (2H, m), 1.88 (2H, m), 1.43 (6H, d, J = 7.0 Hz), 1.35 (2H, m).
MS(ESI) m/z: 527 (M+H)⁺.

### (Example 96) N- {2'-Fluoro-5'-[2-isopropyl-5-(6-methylpyridin-2-yl)-1H-imidazol-4-yl]-1,1'-biphenyl-4-yl}morpholine-4-carboxyamide (Compound No. 1-634)

### (96a) N-[4-(4,4,5,5-Tetramethyl-1,3,2-dioxaborolan-2-yl)phenyl]morpholine-4-carboxyamide

The same reaction as in Example (95a) was carried out using morpholine (0.078 g, 0.90 mmol) instead of 4-amino-1-methylpiperidine to obtain 0.27 g (yield: 92%) of a crude product of the title compound as a white solid.
¹H-NMR (400 MHz, CDCl₃) δ ppm: 7.72 (2H, d, J = 8.2 Hz), 7.35 (2H, d, J = 8.2 Hz), 6.36 (1H, s), 3.73 (4H, m), 3.48 (4H, m), 1.33 (12H, s). (96b) N-{2'-Fluoro-5'-[2-isopropyl-5-(6-methylpyridin-2-yl)-1H-imidazol-4-yl]-1,1'-biphenyl-4-yl}morpholine-4-carboxyamide

The same reaction as in Example 92 was carried out using N-[4-(4,4,5,5-tetramethyl-1,3,2-dioxaborolan-2-yl)phenyl]morpholine-4-carboxyamide (0.096 g, 0.29 mmol) obtained in Example (32a) instead of N-(4-methylpiperazin-1-yl)-N'-[4-(4,4,5,5-tetramethyl-1,3,2-dioxaborolan-2-yl)phenyl]urea obtained in Example (59a). After purification, 0.035 g (yield: 26%) of the title compound was obtained as a colorless solid.
Melting point: 140°C ¹H-NMR (400 MHz, CDCl₃) δ ppm: 10.25 (1H, brs), 7,66 (1H, m), 7.53-7.45 (3H, m), 7.40-7.35 (3H, m), 7.25 (1H, d, J = 7.8 Hz), 7.13 (1H, m), 6.91 (1H, d, J = 7.4 Hz), 6.48 (1H, s), 3.73 (4H, m), 3.48 (4H, m), 3.14 (1H, hp, J = 7.0 Hz), 2.15 (3H, s), 1.39 (6H, d, J = 7.0 Hz).
MS(ESI) m/z: 500 (M+H)⁺

### (Example 97) N-{2'-Fluoro-5'-[2-isopropyl-5-(6-methylpyridin-2-yl)-1H-imidazol-4-yl]-1,1'-biphenyl-4-yl}-4-methylpiperazine-1-carboxyamide (Compound No. 1-622)

### (97a) 4-Methyl-N-[4-(4,4,5,5-tetramethyl-1,3,2-dioxaborolan-2-yl)phenyl]piperazine-1-carboxyamide

The same reaction as in Example (95a) was carried out using 4-amino-1-methylpiperidine (0.090 g, 0.90 mmol) instead of N-aminomorpholine to obtain 0.27 g (yield: 100%) of a crude product of the title compound as a colorless amorphous form.
¹H-NMR (400 MHz, CDCl₃) δ ppm: 7.73 (2H, d, J = 7.8 Hz), 7.37 (2H, d, J = 7.8 Hz), 6.45 (1H, s), 3.52 (4H, m), 2.44 (4H, m), 2.33 (3H, s), 1.33 (12H, s). (97b) N-{2'-Fluoro-5'-[2-isopropyl-5-(6-methylpyridin-2-yl)-1H-imidazol-4-yl]-1,1'-biphenyl-4-yl}-4-methylpiperazine-1-carboxyamide

The same reaction as in Example 92 was carried out using 4-methyl-N-[4-(4,4,5,5-tetramethyl-1,3,2-dioxaborolan-2-yl)phenyl]piperazine-1 -carboxyamide (0.10 g, 0.29 mmol) obtained in Example (97a) instead of N-(4-methylpiperazin-1-yl)-N'-[4-(4,4,S,S-tetramethyl-1,3,2-dioxaborolan-2-yl)phenyl]urea obtained in Example (59a). After purification, 0.034 g (yield: 24%) of the title compound was obtained as a white solid.
Melting point: 136 to 138°C ¹H-NMR (400 MHz, CDCl₃) δ ppm: 10.03 (1H, brs), 7,67 (1H, dd, J = 2.3, 7.8 Hz), 7.53-7.46 (3H, m), 7.40-7.34 (3H, m), 7.25 (1H, m), 7.13 (1H, m), 6.91 (1H, d, J = 7.0 Hz), 6.39 (1H, s), 3.52 (4H, m), 3.14 (1H, hp, J = 7.0 Hz), 2.53 (3H, s), 2.45 (4H, m), 2.33 (3H, s), 1.41 (6H, d, J = 7.0 Hz).
MS(ESI) m/z: 512 (M+H)⁺

### (Example 98) 2'-Fluoro-5'-[2-isopropyl-5-(6-methylpyridin-2-yl)-1H-imidazol-4-yl]-N-(1-methylpiperidin-4-yl)-1,1'-biphenyl-4-carboxyamide (Compound No. 1-658)

The same reaction as in Example 92 was carried out using N-(1-methylpiperidin-4-yl)-4-(4,4,5,5-tetramethyl-1,3,2-dioxaborolan-2-yl)benzamide (0.10 g, 0.29 mmol) obtained in Example (68a) instead of N-(4-methylpiperazin-1-yl)-N'-[4-(4,4,5,5-tetramethyl-1,3,2-dioxaborolan-2-yl)phenyl]urea obtained in Example (59a). After purification, 0.074 g (yield: 53%) of the title compound was obtained as a white solid.
Melting point: 134 to 136°C ¹H-NMR (400 MHz, CDCl₃) δ ppm: 10.05 (1H, brs), 7,81 (2H, d, J = 8.3 Hz), 7.74 (1H, m), 7.65 (2H, d, J = 8.3 Hz), 7.60 (1H, m), 7.40 (1H, t, J = 7.8 Hz), 7.27 (1H, m), 7.19 (1H, m), 6.95 (1H, d, J = 7.8 Hz), 5.98 (1H, d, J = 7.8 Hz), 4.02 (1H, brs), 3.16 (1H, hp, J = 7.0 Hz), 2.82 (2H, m), 2.55 (3H, s), 2.31 (3H, s), 2.19 (2H, m), 2.05 (2H, m), 1.60 (2H, m), 1.41 (6H, d, J = 7.0 Hz).
MS(ESI) m/z: 512 (M+H)⁺

### (Example 99) 2- {2'-Fluoro-5'-[2-isopropyl-5-(6-methylpyridin-2-yl)-1H-imidazol-4-yl]-1,1'-biphenyl-4-yl}-N-tetrahydro-2H-pyran-4-ylacetamide (Compound No. 1-709)

### (99a) N-Tetrahydro-2H-pyran-4-yl-2-[4-(4,4,5,5-tetramethyl-1,3,2-dioxaborolan-2-yl)phenyl]acetamide

[4-(4,4,5,5-Tetramethyl-1,3,2-dioxaborolan-2-yl)phenyl]acetic acid was dissolved in methylene chloride (2 mL), and 1-ethyl-3-(3-dimethylaminopropyl)carbodiimide hydrochloride (0.29 g, 1.5 mmol) and morpholine (0.099 g, 1.1 mmol) were added thereto. The resulting mixture was stirred at room temperature for 12 hr, and water was added to the reaction solution. After extraction with methylene chloride, the organic layer was separated using an Empore cartridge (GL Science). The solvent was evaporated under reduced pressure to obtain 0.23 g (yield: 90%) of a crude product of the title compound as a white solid.
¹H-NMR (400 MHz, CDCl₃) δ ppm: 7.74 (2H, d, J = 8.2 Hz), 7.23 (2H, d, J = 8.2 Hz), 3.74 (2H, s), 3.62 (4H, m), 3.39 (4H, m), 1.34 (12H, s). (99b) 2-{2'-Fluoro-5'-[2-isopropyl-5-(6-methylpyridin-2-yl)-1H-imidazol-4-yl]-1,1'-biphenyl-4-yl}-N-tetrahydro-2H-pyran-4-ylacetamide

The same reaction as in Example 92 was carried out using N-tetrahydro-2H-pyran-4-yl-2-[4-(4,4,5,5-tetramethyl-1,3,2-dioxaborolan-2-yl)phenyl]acetamide (0.22 g, 0.67 mmol) obtained in Example (99a) instead of N-(4-methylpiperazin-1-yl)-N'-[4-(4,4,5,5-tetramethyl-1,3,2-dioxaborolan-2-yl)phenyl]urea obtained in Example (59a). After purification, 0.19 g (yield: 61%) of the title compound was obtained as a colorless amorphous form.
¹H-NMR (400 MHz, CDC1₃) δ ppm: 10.19 (1H, brs), 7.69 (1H, m), 7.56-7.50 (3H, m), 7.38 (1H, t, J = 7.4 Hz), 7.29-7.23 (3H, m), 7.14 (1H, m), 6.92 (1H, d, J = 7.4 Hz), 3.75 (2H, s), 3.65 (4H, m), 3.48 (4H, m), 3.14 (1H, hp, J = 7.0 Hz), 2.52 (3H, s), 1.39 (6H, d, J = 7.0 Hz).
MS(ESI) m/z: 499 (M+H)⁺

### (Example 100) 4-{2'-Fluoro-5'-[2-isopropyl-5-(6-methylpyridin-2-yl)-1H-imidazol-4-yl]-1,1'-biphenyl-4-yl}morpholine (Compound No. 1-711)

The same reaction as in Example 92 was carried out using 4-[4-(4,4,5,5-tetramethyl-1,3,2-dioxaborolan-2-yl)phenyl]morpholine (0.26 g, 0.88 mmol) instead of N-(4-methylpiperazin-1-yl)-N-[4-(4,4,5,5-tetramethyl-1,3,2-dioxaborolan-2-yl)phenyl]urea obtained in Example (59a). After purification, 0.35 g (yield: 93%) of the title compound was obtained as a white solid.
Melting point: 109 to 111°C
¹H-NMR (400 MHz, CDCl₃) δ ppm: 10.08 (1H, brs), 7.67 (1H, m), 7.50-7.45 (3H, m), 7.37 (1H, t, J = 7.8 Hz), 7.25 (1H, m), 7.12 (1H, m), 6.95-6.89 (3H, m), 3.86 (4H, m), 3.20 (4H, m), 3.13 (1H, hp, J = 7.0 Hz), 2.53 (3H, s), 1.40 (6H, d, J = 7.0 Hz). MS(ESI) m/z: 457 (M+H)⁺.

### (Example 101) 2'-Fluoro-5'-[2-isopropyl-5-(6-methylpyridin-2-yl)-1H-imidazol-4-yl]-1,1'-biphenyl-4-carboxylic acid (Compound No. 1-751)

The same reaction as in Example 92 was carried out using 4-methoxycarbonylphenylboronic acid (1.3 g, 7.0 mmol) instead of N-(4-methylpiperazin-1-yl)-N'-[4-(4,4,5,5-tetramethyl-1,3,2-dioxaborolan-2-yl)phenyl]urea obtained in Example (59a). The resulting crude product was purified by silica gel column chromatography (NH, eluting solvent; hexane : ethyl acetate = 1 : 1) to obtain 1.7 g (yield: 59%) of a methyl ester of the title compound as a colorless amorphous form. The obtained methyl ester (1.7 g, 3.9 mmol) was dissolved in ethanol (20 mL), and a 10% sodium hydroxide aqueous solution (10 mL) was added thereto. The resulting mixture was stirred at room temperature for 2 hr. The reaction solvent was evaporated under reduced pressure, and water was added to the residue. The resulting mixture was neutralized with a 2 N hydrochloric acid aqueous solution. After extraction with ethyl acetate, the organic layer was washed with water and brine, and then dried with anhydrous magnesium sulfate. The solvent was evaporated under reduced pressure to obtain 1.2 g (yield: 75%) of a crude product of the title compound as a white solid.
Melting point: 160 to 161°C
¹H-NMR (400 MHz, CDCl₃) δ ppm: 7.98 (2H, d, J = 8.2 Hz), 7.90 (1H, brs), 7.72 (1H, brs), 7.64-7.59 (4H, m), 7.29 (1H, m), 7.06 (1H, m), 3.16 (3H, s), 3.06 (1H, hp, J = 7.0 Hz), 1.30 (6H, d, J = 7.0 Hz).
MS(ESI) m/z: 416 (M+H)⁺

### (Example 102) 2-{4-[4-Fluoro-3-(5-methyl-1H-pyrrol-2-yl)phenyl]-2-isopropyl-1H-imidazol-5-yl}-6-methylpyridine (Compound No. 1-757)

### (102a) 5-{2-Fluoro-5-[2-isopropyl-5-(6-methylpyridin-2-yl)-1H-imidazol-4-yl]phenyl}-1H-pyrrole-2-carboaldehyde

The same reaction as in Example (69a) was carried out using 2-{4-[4-fluoro-3-(1H-pyrrol-2-yl)phenyl]-2-isopropyl-1H-imidazol-5-yl}-6-methylpyridine (0.17 g, 0.47 mmol) obtained in Example (93b) instead of 2-{2-isopropyl-4-[3-(1H-pyrrol-2-yl)phenyl]-1H-imidazol-5-yl}-6-methylpyridine obtained in Example 23. After purification, 0.14 g (yield: 76%) of the title compound was obtained as a white solid. ¹H-NMR (400 MHz, CDCl₃) δ ppm: 10.14 (1H, brs), 9.95 (1H, brs), 9.51 (1H, s), 7.99 (1H, m), 7.53 (1H, m), 7.38 (1H, t, J = 7.8 Hz), 7.21-7.14 (2H, m), 6.97 (1H, m), 6.94 (1H, d, J = 7.8 Hz), 6.71 (1H, m), 3.14 (1H, hp, J = 7.0 Hz), 2.54 (3H, s), 1.40 (6H, d, J = 7.0 Hz).

### (102b) 2-{4-[4-Fluoro-3-(5-methyl-1H-pyrrol-2-yl)phenyl]-2-isopropyl-1H-imidazol-5-yl}-6-methylpyridine

The same reaction as in Example (69b) was carried out using 5-{2-fluoro-5-[2-isopropyl-5-(6-methylpyridin-2-yl)-1H-imidazol-4-yl]phenyl}-1H-pyrrole-2-carboaldehyde (0.14 g, 0.34 mmol) obtained in Example (102a) instead of 5-{3-[2-isopropyl-5-(6-methylpyridin-2-yl)-1H-imidazol-4-yl]phenyl}-1H-pyrrole-2-carboaldehyde obtained in Example (69a). After purification, 0.095 g (yield: 73%) of the title compound was obtained as a white solid.
Melting point: 118 to 121°C
¹H-NMR (400 MHz, CDCl₃) δ ppm: 10.10 (1H, brs), 8.74 (1H, brs), 7.80 (1H, m), 7.38-7.29 (2H, m), 7.23 (1H, m), 7.08 (1H, m), 6.90 (1H, d, J = 7.4 Hz), 6.45 (1H, m), 5.92 (1H, m), 3.15 (1H, hp, J = 7.0 Hz), 2.53 (3H, s), 2.33 (3H, s), 1.40 (6H, d, J = 7.0 Hz).
MS(ESI) m/z: 374 (M+H)⁺

### (Example 103) 2-(4-{6-Fluoro-4'-[(methylthio)methyl]-1,1'-biphenyl-3-yl}-2-isopropyl-1H-imidazol-5-yl)-6-methylpyridine (Compound No. 1-713)

### (103a) 4,4,5,5-Tetramethyl-2-{4-[(methylthio)methyl]phenyl}-1,3,2-dioxaborolane

2-[4-(Bromomethyl)phenyl]-4,4,5,5-tetramethyl-1,3,2-dioxaborolane (0.90 g, 3.0 mmol) was dissolved in N,N-dimethylformamide (8 mL), and sodium thiomethoxide (0.25 g, 3.5 mmol) was added thereto. The resulting mixture was stirred at room temperature for 18 hr. The reaction solution was concentrated under reduced pressure, and water was added thereto. After extraction with ethyl acetate, the organic layer was washed with water and brine, and then dried with anhydrous magnesium sulfate. The solvent was evaporated under reduced pressure to obtain 0.77 g (yield: 96%) of a crude product of the title compound as a yellow amorphous form.
¹H-NMR (500 MHz, CDCl₃) δ ppm: 7.74 (2H, d, J = 7.8 Hz), 7.29 (2H, d, J = 7.8 Hz), 3.66 (2H, s), 1.96 (3H, s), 1.33 (12H, s).

### (103b) 2-(4-{6-Fluoro-4'-[(methylthio)methyl]-1,1'-biphenyl-3-yl}-2-isopropyl-1H-imidazol-5-yl)-6-methylpyridine

The same reaction as in Example 92 was carried out using 4,4,5,5-tetramethyl-2-{4-[(methylthio)methyl]phenyl}-1,3,2-dioxaborolane (0.77 g, 2.9 mmol) obtained in Example (103a) instead of N-(4-methylpiperazin-1-yl)-N'-[4-(4,4,5,5-tetramethyl-1,3,2-dioxaborolan-2-yl)phenyl]urea obtained in Example (59a). The resulting crude product was purified by silica gel column chromatography (NH, eluting solvent; hexane : ethyl acetate = 7 : 3) to obtain 0.71 g (yield: 82%) of the title compound as a white amorphous form.
¹H-NMR (500 MHz, CDCl₃) δ ppm: 10.46 (1H, brs), 7.72 (1H, dd, J = 2.0, 7.8 Hz), 7.56-7.51 (3H, m), 7.42 (1H, m), 7.35 (2H, d, J = 8.3 Hz), 7.28 (1H, d, J = 7.8 Hz), 7.17 (1H, m), 6.94 (1H, d, J = 7.8 Hz), 3.70 (2H, s), 3.14 (1H, m), 2.50 (3H, s), 2.02 (3H, s), 1.38 (6H, d, J = 6.8 Hz).
MS(ESI) m/z: 432 (M+H)⁺

### (Example 104) 2-(4-{6-Fluoro-4'-[(methylsulfinyl)methyl]-1,1'-biphenyl-3-yl}-2-isopropyl-1H-imidazol-5-yl)-6-methylpyridine (Compound No. 1-715)

Water (0.8 mL) and sodium periodate (0.078g, 0.37 mmol) were added to a methanol solution (3 mL) of 2-(4-{6-fluoro-4'-[(methylthio)methyl]-1,1'-biphenyl-3-yl}-2-isopropyl-1H-imidazol-5-yl)-6-methylpyridine (0.13 g, 0.30 mmol) obtained in Example (103b). The resulting mixture was stirred at room temperature for 3 hr. The reaction solution was concentrated under reduced pressure, and water was added thereto. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; hexane : ethyl acetate = 1 : 9) to obtain 0.082 g (yield: 61%) of the title compound as a white amorphous form.
¹H-NMR (500 MHz, CDCl₃) δ ppm: 10.80 (1H, brs), 7.72 (1H, dd, J = 2.0, 7.3 Hz), 7.59-7.57 (3H, m), 7.43 (1H, m), 7.34 (2H, d, J = 7.3 Hz), 7.28 (1H, d, J = 7.8 Hz), 7.17 (1H, m), 6.95 (1H, d, J = 7.8 Hz), 4.08, 3.97 (2H, ABq, J = 12.7 Hz), 3.13 (1H, m), 2.49 (3H, s), 2.46 (3H, s), 1.35 (6H, d, J = 7.3 Hz).
MS(ESI) m/z: 448 (M+H)⁺

### (Example 105) 2-(4-{6-Fluoro-4'-[(methylsulfonyl)methyl]-1,1'-biphenyl-3-yl}-2-isopropyl-1H-imidazol-5-yl)-6-methylpyridine (Compound No. 1-717)

Water (2 mL) and sodium periodate (0.43 g, 2.0 mmol) were added to a methanol solution (5 mL) of 2-(4-{6-fluoro-4'-[(methylthio)methyl]-1,1'-biphenyl-3-yl}-2-isopropyl-1H-imidazol-5-yl)-6-methylpyridine (0.17 g, 0.40 mmol) obtained in Example (103b). The resulting mixture was heated under reflux for 4 hr. The reaction solution was concentrated under reduced pressure, and water was added thereto. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; hexane : ethyl acetate = 2 : 3) to obtain 0.12 g (yield: 64%) of the title compound as a white amorphous form. ¹H-NMR (500 MHz, CDCl₃) δ ppm: 10.86 (1H, brs), 7.72 (1H, dd, J = 2.0, 7.3 Hz), 7.62-7.57 (3H, m), 7.47-7.42 (3H, m), 7.28 (1H, d, J = 7.8 Hz), 7.18 (1H, m), 6.95 (1H, d, J = 7.8 Hz), 4.28 (2H, s), 3.12 (1H, m), 2.79 (3H, s), 2.46 (3H, s), 1.34 (6H, d, J = 6.8 Hz).
MS(ESI) m/z: 464 (M+H)⁺.

### (Example 106) 2-{4-[4-Fluoro-3-(1H-1,2,3-triazol-4-yl)phenyl]-2-isopropyl-1H-imidazol-5-yl}-6-methylpyridine (Compound No. 1-759)

### (106a) 2-[4-(3-Ethynyl-4-fluorophenyl)-2-isopropyl-1H-imidazol-5-yl]-6-methylpyridine

2-[4-(3-Bromo-4-fluorophenyl)-2-isopropyl-1H-imidazol-5-yl]-6-methylpyridine (0.56 g, 1.5 mmol) obtained in Example (60b) was dissolved in tetrahydrofuran (3 mL), and triethylamine (1.5 mL, 11 mmol), trimethylsilylacetylene (0.35 g, 3.6 mmol), copper(I) iodide (0.016 g, 0.081 mmol), and bis(triphenylphosphine)palladium(II) dichloride (0.012 g, 0.017 mmol) were added thereto. The resulting mixture was heated under reflux for 8 hr. To the reaction solution, ethyl acetate was added. The resulting mixture was filtered through Celite, and water was added to the filtrate. After extraction with ethyl acetate, the organic layer was washed with water and brine, and then dried with anhydrous magnesium sulfate. The solvent was evaporated under reduced pressure. The resulting crude product was dissolved in methanol (5 mL), and potassium carbonate (0.050 g, 0.36 mmol) was added thereto. The resulting mixture was stirred at room temperature for 30 min. The reaction solution was concentrated under reduced pressure, and water was added thereto. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; hexane : ethyl acetate = 3 : 2) to obtain 0.38 g (yield: 80%) of the title compound as a light yellow amorphous form.
¹H-NMR (500 MHz, CDCl₃) δ ppm: 10.36 (1H, brs), 7.78 (1H, dd, J = 2.0, 6.8 Hz), 7.58 (1H, m), 7.42 (1H, m), 7.17 (1H, d, J = 7.8 Hz), 7.10 (1H, m), 6.95 (1H, d, J = 7.3 Hz), 3.28 (1H, s), 3.13 (1H, m), 2.50 (3H, s), 1.38 (6H, d, J = 7.3 Hz).
MS(ESI) m/z: 320 (M+H)⁺

### (106b) 2-{4-[4-Fluoro-3-(1H-1,2,3-triazol-4-yl)phenyl]-2-isopropyl-1H-imidazol-5-yl}-6-methylpyridine

Trimethylsilyl azide (2.0 mL, 15 mmol) was added to 2-[4-(3-ethynyl-4-fluorophenyl)-2-isopropyl-1H-imidazol-5-yl]-6-methylpyridine (0.32 g, 1.0 mmol) obtained in Example (106a). The resulting mixture was heated under reflux for 24 hr. The reaction solution was cooled to room temperature, and saturated aqueous sodium bicarbonate and diethyl ether were added thereto. The resulting mixture was stirred at room temperature for 20 min. The precipitate was collected by filtration, and the resulting crude product was purified by high-performance liquid chromatography (GL Science ODS-3, eluting solvent; water : acetonitrile = 95 : 5 to 5 : 95) to obtain 0.12 g (yield: 33%) of the title compound as a white solid.
Melting point: 233 to 235°C
¹H-NMR (500 MHz, CD₃OD) δ ppm: 8.24 (1H, d, J = 6.4 Hz), 8.13 (1H, m), 7.58 (1H, m), 7.47 (1H, m), 7.25-7.21 (2H, m), 7.12 (1H, d, J = 7.3 Hz), 3.18 (1H, m), 2.50 (3H, s), 1.40 (6H, d, J = 6.8 Hz).
MS(ESI) m/z: 363 (M+H)⁺

### (Example 107) 2- {5-[3-(1-Ethyl-1H-pyrazol-4-yl)-4-fluorophenyl]-2-isopropyl-1H-imidazol-4-yl}-6-methylpyridine (Compound No. 1-737)

### (107a) 1-Ethyl-4-(4,4,5,5-tetramethyl-1,3,2-dioxaborolan-2-yl)-1H-pyrazole

Sodium hydride (55%, oil, 0.54 g, 12 mmol) was suspended in N,N-dimethylformamide (20 mL), and a solution of 4-(4,4,5,5-tetramethyl-1,3,2-dioxaborolan-2-yl)-1H-pyrazole (2.0 g, 10 mmol) in N,N-dimethylformamide (5 mL) was added thereto. The resulting mixture was stirred at room temperature for 1 hr, and then iodoethane (2.4 g, 16 mmol) was slowly added dropwise thereto. The resulting mixture was stirred at room temperature for 3 days. To this reaction solution, water was added. After extraction with diethyl ether, the organic layer was washed with water and brine, and then dried with anhydrous sodium sulfate. The solvent was evaporated under reduced pressure, and the resulting crude product was purified by silica gel column chromatography (Yamazen, eluting solvent: hexane and ethyl acetate) to obtain 1.7 g (yield: 74%) of the title compound as a colorless oily material.
¹H-NMR (400 MHz, CDCl₃) δ ppm: 7.78 (1H, s), 7.70 (1H, s), 4.19 (2H, q, J = 7.3 Hz), 1.49 (3H, t, J = 7.3 Hz), 1.32 (12H, s).

### (107b) 2-(5-[3-(1-Ethyl-1H-pyrazol-4-yl)-4-fluorophenyl]-2-isopropyl-3H-imidazol-4-yl}-6-methylpyridine

2-[4-(3-Bromo-4-fluorophenyl)-2-isopropyl-1H-imidazol-5-yl]-6-methylpyridine (1.0 g, 2.7 mmol) obtained in Example (60b) and 1-ethyl-4-(4,4,5,5-tetramethyl-1,3,2-dioxaborolan-2-yl)-1H-pyrazole (0.89 g, 4.0 mmol) obtained in Example (107a) were dissolved in 1,2-dimethoxyethane (30 mL), and tripotassium phosphate n-hydrate (1.1 g, 5.3 mmol), water (3 mL), and dichloro[1,1'-bis(diphenylphosphino)ferrocene]palladium(II)-methylene chloride complex (0.22 g, 0.27 mmol) were added thereto. The resulting mixture was stirred under a nitrogen atmosphere at 90°C for 24 hr. The reaction solution was cooled to room temperature, and water was added thereto. After extraction with ethyl acetate, the organic layer was washed with water and brine, and then dried with anhydrous sodium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.87 g (yield: 83%) of the title compound as a white solid.
Melting point: 76 to 78°C
¹H-NMR (400 MHz, CDCl₃) δ ppm: 7.87-7.80 (3H, m), 7.41 (1H, m), 7.38 (1H, d, J = 7.8 Hz), 7.24 (1H, d, J = 7.8 Hz), 7.13 (1H, m), 6.93 (1H, d, J = 7.8 Hz), 4.21 (2H, q, J = 7.4 Hz), 3.16 (1H, hp, J = 7.0 Hz), 2.50 (3H, s), 1.53 (3H, t, J = 7.4 Hz), 1.39 (6H, d, J = 7.0 Hz).
MS(ESI) m/z: 390 (M+H)⁺.

### (Example 108) 2-{S-[4-Fluoro-3-(1-isopropyl-1H-pyrazol-4-yl)phenyl]-2-isopropyl-1H-imidazol-4-yl}-6-methylpyridine (Compound No. 1-741)

### (108a) 1-Isopropyl-4-(4,4,5,5-tetramethyl-1,3,2-dioxaborolan-2-yl)-1H-pyrazole

The same reaction as in Example (107a) was carried out using 2-iodopropane (1.3 g, 16 mmol) instead of iodoethane. After purification, 0.77 g (yield: 63%) of the title compound was obtained as a colorless oily material.
¹H-NMR (400 MHz, CDC1₃) δ ppm: 7.77 (1H, s), 7.72 (1H, s), 4.51 (1H, hp, J = 6.7 Hz), 1.50 (6H, d, J = 6.7 Hz), 1.32 (12H, s).

### (108b) 2-{5-[4-Fluoro-3-(1-isopropyl-1H-pyrazol-4-yl)phenyl]-2-isopropyl-1H-imidazol-4-yl}-6-methylpyridine

The same reaction as in Example (107b) was carried out using 1-isopropyl-4-(4,4,5,5-tetramethyl-1,3,2-dioxaborolan-2-yl)-1H-pyrazole (190 mg, 0.80 mmol) obtained in Example (108a) instead of 1-ethyl-4-(4,4,5,5-tetramethyl-1,3,2-dioxaborolan-2-yl)-1H-pyrazole obtained in Example (107a). The resulting crude product was purified by high-performance liquid chromatography (GL Science ODS-3, eluting solvent; water : acetonitrile = 95 : 5 to 5 : 95) to obtain 44 mg (yield: 21 %) of the title compound as a white solid.
Melting point: 84 to 85°C
¹H-NMR (400 MHz, CDCl₃) δ ppm: 7.84-7.76 (3H, m), 7.42-7.34 (2H, m), 7.24 (1H, d, J = 8.2 Hz), 7.09 (1H, m), 6.91 (1H, d, J = 8.2 Hz), 4.52 (1H, hp, J = 6.7 Hz), 3.11 (1H, hp, J = 7.0 Hz), 2.39 (3H, s), 1.54 (6H, d, J = 6.7 Hz), 1.29 (6H, d, J = 7.0 Hz). MS(ESI) m/z: 404 (M+H)⁺

### (Example 109) 4-({2'-Fluoro-5'-[2-isopropyl-4-(6-methylpyridin-2-yl)-1H-imidazol-5-yl]-1,1'-biphenyl-4-yl}methylmorpholine (Compound No. 1-551)

The same reaction as in Example (1 07b) was carried out using 2-[4-(3-bromo-4-fluorophenyl)-2-isopropyl-1H-imidazol-5-yl]-6-methylpyridine (75 mg, 0.20 mmol) obtained in Example (60b) and 4-[4-(4,4,5,5-tetramethyl-1,3,2-dioxaborolan-2-yl)benzyl]morpholine (91 mg, 0.30 mmol). The resulting crude product was purified by high-performance liquid chromatography (GL Science ODS-3, eluting solvent; water : acetonitrile = 95 : 5 to 5 : 95) to obtain 17 mg (yield: 18%) of the title compound as a white solid.
Melting point: 147 to 161°C
¹H NMR (400 MHz, CDCl₃) δ ppm: 9.95 (1H, s), 7.70 (1H, dd, J = 2.2, 7.9 Hz), 7.56 (1H, m), 7.51 (1H, d, J = 8.5 Hz), 7.38 (1H, t, J = 7.9 Hz), 7.36 (1H, d, J = 8.5 Hz), 7.27 (1H, m), 7.25-7.24 (2H, m), 7.16 (1H, dd, J = 8.5, 10.3 Hz), 6.92 (1H, d, J = 7.9 Hz), 3.72 (4H, m), 3.52 (2H, s), 3.18 (1H, hp, J = 7.0 Hz), 2.55 (3H, s), 2.48-2.47 (4H, m), 1.42 (6H, d, J = 7.0 Hz).
MS(ESI) m/z: 471 (M+H)⁺

### (Example 110) 2-{5-[4-Fluoro-3-(1-methyl-1H-pyrazol-4-yl)phenyl]-2-isopropyl-1H-imidazol-4-yl}-6-methylpyridine (Compound No. 1-725)

The same reaction as in Example (107b) was carried out using 2-[4-(3-bromo-4-fluorophenyl)-2-isopropyl-1H-imidazol-5-yl]-6-methylpyridine (75 mg, 0.20 mmol) obtained in Example (60b) and 1-methyl-4-(4,4,5,5-tetramethyl-1,3,2-dioxaborolan-2-yl)-1H-pyrazole (63 mg, 0.30 mmol). The resulting crude product was purified by high-performance liquid chromatography (GL Science ODS-3, eluting solvent; water : acetonitrile = 95 : 5 to 5 : 95) to obtain 17 mg (yield: 23%) of the title compound as a yellow amorphous form.
Melting point: 77 to 97°C
¹H NMR (400 MHz, CDCl₃) δ ppm: 10.1 (1H, s), 7.81 (1H, m), 7.77 (1H, d, J = 2.0 Hz), 7.43 (1H, m), 7.35 (1H, t, J = 7.8 Hz), 7.25 (1H, s), 7.22 (1H, d, J = 8.5 Hz), 7.12 (1H, dd, J=8.5, 10.9 Hz), 6.92 (1H, d, J = 7.8 Hz), 3.94 (3H, s), 3.19 (1H, hp, J = 7.0 Hz), 2.54 (3H, s), 1.42 (6H, d, J = 7.0 Hz).
MS(ESI) m/z: 376 (M+H)⁺.

### (Example 111) -N-{2'-Fluoro-5'-[2-isopropyl-4-(6-methylpyridin-2-yl)-1H imidazol-5-yl]-1,1'-biphenyl-4-yl}cyclopropanesulfonamide (Compound No. 1-181)

4-(4,4,5,5-Tetramethyl-1,3,2-dioxaborolan-2-yl)aniline (4.4 g, 20 mmol) was dissolved in 1,2-dichloroethane (50 mL) under a nitrogen atmosphere, and cyclopropanesulfonyl chloride (2.1 mL, 20 mmol) and pyridine (1 mL) were added thereto. The resulting mixture was stirred at room temperature for 12 hr. To this reaction solution, saturated aqueous sodium bicarbonate was added. After extraction with methylene chloride, the organic layer was separated using an Empore cartridge (GL Science). The solvent was concentrated, and the residue was purified by silica gel column chromatography (hexane : ethyl acetate = 2 : 1) and recrystallized with hexane-ethyl acetate to obtain 3.8 g (yield: 59%) of N-[4-(4,4,5,5-tetramethyl-1,3,2-dioxaborolan-2-yl)phenyl]cyclopropanesulfonamide as a white solid.

The same reaction as in Example (107b) was carried out using 2-[4-(3-bromo-4-fluorophenyl)-2-isopropyl-1H-imidazol-5-yl]-6-methylpyridine (0.38 g, 1.0 mmol) obtained in Example (60b) and N-[4-(4,4,5,5-tetramethyl-1,3,2-dioxaborolan-2-yl)phenyl]cyclopropanesulfonamide (0.39 g, 1.2 mmol). The resulting crude product was purified by high-performance liquid chromatography (GL Science ODS-3, eluting solvent; water : acetonitrile = 95 : 5 to 5 : 95) to obtain 0.060 g (yield: 12%) of the title compound as a deep purple amorphous form.
Melting point: 119 to 129°C
¹H NMR (400 MHz, CDCl₃) δ ppm: 10.3 (1H, s), 7.68 (1H, dd, J = 2.2, 7.6 Hz), 7.55 (1H, m), 7.49 (2H, d, J = 7.6 Hz), 7.40 (1H, t, J = 7.6 Hz), 7.28 (1H, m), 7.25 (1H, s), 7.15 (1H, dd, J = 8.6, 10.5 Hz), 6.94 (2H, d, J = 7.6 Hz), 3.19 (1H, hp, J = 7.0 Hz), 2.52 (3H, s), 2.50 (1H, m), 1.40 (6H, d, J = 7.0 Hz), 1.17 (2H, d, J = 7.4 Hz), 0.95 (2H, d, J = 7.4 Hz).
MS(ESI) m/z: 491 (M+H)⁺

### (Example 112) 2-{5-[4-Fluoro-3-(1H-pyrazol-4-yl)phenyl]-2-isopropyl-1H-pyrazol-4-yl}-6-methylpyridine (Compound No. 1-723)

The same reaction as in Example (107b) was carried out using 2-[4-(3-bromo-4-fluorophenyl)-2-isopropyl-1H-imidazol-5-yl]-6-methylpyridine (75 mg, 0.20 mmol) obtained in Example (60b) and t-butyl 4-(4,4,5,5-tetramethyl-1,3,2-dioxaborolan-2-yl)-1H-pyrazole-1-carboxylate (88 mg, 0.30 mmol). The reaction solvent was evaporated under reduced pressure. The residue was filtered using silica gel and then dissolved in tetrahydrofuran (5 mL). A 25% sodium methoxide methanol solution (0.6 mL) was added thereto, and the resulting mixture was stirred at room temperature for 2 hr. The reaction solvent was evaporated under reduced pressure. Water was added to the residue. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 32 mg (yield: 44%) of the title compound as a white solid.
Melting point: 129 to 136°C
¹H NMR (400 MHz, CDCl₃) δ ppm: 10.2 (1H, s), 7.94 (2H, s), 7.83 (1H, dd, J = 2.2, 7.4 Hz), 7.44 (1H, m), 7.36(1H, t, J = 7.4 Hz), 7.25 (1H, s), 7.23 (1H, d, J = 7.4 Hz), 7.13(1H, dd, J = 8.4, 10.8 Hz), 6.93(1H, d, J = 7.4 Hz), 3.20 (1H, hp, J = 7.0 Hz), 2.54(3H, s), 1.42 (6H, d, J = 7.0 Hz).
MS(ESI) m/z: 362 (M+H)⁺

### (Example 113) 2-{5-[4-Fluoro-3-(2-furyl)phenyl]-2-isopropyl-1H-imidazol-4-yl}-6-methylpyridine (Compound No. 1-744)

The same reaction as in Example (107b) was carried out using 2-[4-(3-bromo-4-fluorophenyl)-2-isopropyl-1H-imidazol-5-yl]-6-methylpyridine (75 mg, 0.20 mmol) obtained in Example (60b) and 2-(3-furyl)-4,4,5,5-tetramethyl-1,3,2-dioxaborolane (58 mg, 0.30 mmol). The resulting crude product was purified by high-performance liquid chromatography (GL Science ODS-3, eluting solvent; water : acetonitrile = 95 : 5 to 5 : 95) to obtain 50 mg (yield: 69%) of the title compound as a white amorphous form.
Melting point: 78 to 90°C
¹H NMR (400 MHz, CDCl₃) δ ppm: 8.09 (1H, dd, J = 2.0, 7.4 Hz), 7.47 (1H, m), 7.43 (1H, d, J = 2.0 Hz), 7.38 (1H, t, J = 7.4 Hz), 7.24 (1H, d, J = 7.4 Hz), 7.12 (1H, dd, J = 8.6, 11.0 Hz), 6.92 (1H, d, J = 7.4 Hz), 6.85 (1H, t, J = 3.5 Hz), 6.49 (1H, dd, J = 2.0, 3.5 Hz), 3.17 (1H, hp, J = 7.0 Hz), 2.45 (3H, s), 1.34 (6H, d, J = 7.0 Hz). MS(ESI) m/z: 362 (M+H)⁺

### (Example 114) 2'-Fluoro-5'-[2-isopropyl-5-(6-methylpyridin-2-yl)-1H-imidazol-4-yl]-1,1'-biphenyl-4-ol (Compound No. 1-697)

2-[4-(3-Bromo-4-fluorophenyl)-2-isopropyl-1H-imidazol-5-yl]-6-methylpyridine (0.20 g, 0.53 mmol) obtained in Example (60b) and 4-hydroxyphenylboronic acid (0.081 g, 0.59 mmol) were dissolved in 1,2-dimethoxyethane (4 mL), and water (2 mL), a 2 M sodium carbonate aqueous solution (1.2 mL), and tetrakis(triphenylphosphine) palladium (0.031 g, 0.027 mmol) were added thereto. The resulting mixture was stirred under a nitrogen atmosphere at 90°C for 5 hr. The reaction solution was cooled to room temperature, and water was added thereto. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.14 g (yield: 67%) of the title compound as a white solid.
Melting point: 143 to 145°C
¹H-NMR (400 MHz, CDCl₃) δ ppm: 7.65 (1H, m), 7.42-7.36 (2H, m), 7.26 (1H, m), 7.19 (2H, d, J = 7.8 Hz), 7.06 (1H, m), 6.94 (1H, d, J = 7.8 Hz), 6.81 (2H, d, J = 7.8 Hz), 3.50 (1H, brs), 3.24 (1H, hp, J = 7.0 Hz), 2.55 (3H, s), 1.43 (6H, d, J = 7.0 Hz). MS(ESI) m/z: 388 (M+H)⁺

### (Example 115) 2-{4-[6-Fluoro-4'-(2-pyrrolidin-1-ylethoxy)-1,1'-biphenyl-3-yl]-2-isopropyl-1H-imidazol-5-yl}-6-methylpyridine (Compound No. 1-699)

2'-Fluoro-5'-[2-isopropyl-5-(6-methylpyridin-2-yl)-1H-imidazol-4-yl]-1,1'-biphenyl-4-ol obtained in Example 114 was dissolved in tetrahydrofuran (3 mL), and di-t-butyl diazocarboxylate (0.090 g, 0.39 mmol) and triphenylphosphine (0.10 g, 0.39 mmol) were added thereto. The resulting mixture was stirred at room temperature for 10 min. 1-(2-Hydroxyethyl)pyrrolidine (0.030 g, 0.26 mmol) was added thereto, and the resulting mixture was stirred at room temperature for 20 hr. Water was added to the reaction solution. After extraction with methylene chloride, the organic layer was separated using an Empore cartridge (GL Science). The solvent was concentrated, and the resulting crude product was purified by high-performance liquid chromatography (GL Science ODS-3, eluting solvent; water : acetonitrile = 95 : 5 to 5 : 95) to obtain 0.054 g (yield: 43%) of the title compound as a colorless amorphous form.
¹H-NMR (400 MHz, CDCl₃) δ ppm: 10.06 (1H, brs), 7.66 (1H, m), 7.51-7.44 (3H, m), 7.37 (1H, t, J = 7.4 Hz), 7.26 (1H, m), 7.13 (1H, m), 6.96-6.90 (3H, m), 4.13 (2H, t, J = 6.3 Hz), 3.14 (1H, hp, J = 7.0 Hz), 2.91 (2H, t, J = 6.3 Hz), 2.62 (4H, m), 2.53 (3H, s), 1.81 (4H, m), 1.40 (6H, d, J = 7.0 Hz).
MS(ESI) m/z: 485 (M+H)⁺.

### (Example 116) 4-[2-({2'-Fluoro-5'-[2-isopropyl-5-(6-methylpyridin-2-yl)-1H-imidazol-4-yl]-1,1'-biphenyl-4-yl}oxy)ethyl]morpholine (Compound No. 1-707)

The same reaction as in Example 115 was carried out using 2-N-morpholinoethanol (0.039g, 0.30 mmol) instead of 1-(2-hydroxyethyl)pyrrolidine. After purification, 0.10 g (yield: 79%) of the title compound was obtained as a colorless amorphous form.
¹H-NMR (400 MHz, CDCl₃) δ ppm: 10.16 (1H, brs), 7.66 (1H, m), 7.51-7.45 (3H,
m), 7.38 (1H, t, J = 7.8 Hz), 7.25 (1H, m), 7.13 (1H, m), 6.96-6.90 (3H, m), 4.14 (2H, t, J = 5.8 Hz), 3.73 (4H, m), 3.14 (1H, hp, J = 7.0 Hz), 2.81 (2H, t, J = 5.8 Hz), 2.58 (4H, m), 2.52 (3H, s), 1.40 (6H, d, J = 7.0 Hz).
MS(ESI) m/z: 501 (M+H)⁺

### (Example 117) 2-{4-[6-Fluoro-4'-(2-piperidin-1-ylethoxy)-1,1'-biphenyl-3-yl]-2-isopropyl-1H-imidazol-5-yl}-6-methylpyridine (Compound No. 1-705)

The same reaction as in Example 115 was carried out using 1-piperidineethanol (0.056 g, 0.43 mmol) instead of 1-(2-hydroxyethyl)pyrrolidine. After purification, 0.060 g (yield: 34%) of the title compound was obtained as a colorless amorphous form.
¹H-NMR (400 MHz, CDCl₃) δ ppm: 10.09 (1H, brs), 7.66 (1H, m), 7.51-7.45 (3H, m), 7.37 (1H, t, J = 7.8 Hz), 7.25 (1H, m), 7.13 (1H, m), 6.95-6.90 (3H, m), 4.13 (2H, t, J = 5.9 Hz), 3.14 (1H, hp, J = 7.0 Hz), 2.78 (2H, t, J = 5.9 Hz), 2.53 (3H, s), 2.51 (4H, m), 1.61 (4H, m), 1.44 (2H, m), 1.40 (6H, d, J = 7.0 Hz).
MS(ESI) m/z: 499 (M+H)⁺

### (Example 118) N-[2-({2'-Fluoro-5'-[2-isopropyl-5-(6-methylpyridin-2-yl)-1H-imidazol-4-yl]-1,1'-biphenyl-4-yl}oxy)ethyl]-N,N-dimethylamine (Compound No. 1-721)

The same reaction as in Example 115 was carried out using N,N-dimethylamine ethanol (0.028 g, 0.31 mmol) instead of 1-(2-hydroxyethyl)pyrrolidine. After purification, 0.0099 g (yield: 8.3%) of the title compound was obtained as a colorless amorphous form.
¹H-NMR (400 MHz, CDCl₃) δ ppm: 10.19 (1H, brs), 7.68 (1H, m), 7.51-7.47 (3H, m), 7.40 (1H, t, J = 7.8 Hz), 7.28 (1H, m), 7.16 (1H, m), 6.98-6.92 (3H, m), 4.10 (2H, t, J = 5.5 Hz), 3.15 (1H, hp, J = 7.0 Hz), 2.75 (2H, t, J = 5.5 Hz), 2.53 (3H, s), 2.35 (6H, s), 1.40 (6H, d, J = 7.0 Hz).
MS(ESI) m/z: 459 (M+H)⁺

### (Example 119) 2-(4-{6-Fluoro-4'-[2-(1H-pyrrol-1-yl)ethoxy]-1,1'-biphenyl-3-yl}-2-isopropyl-1H-imidazol-5-yl)-6-methylpyridine (Compound No. 1-703)

The same reaction as in Example 115 was carried out using 1-(2-hydroxyethyl)pyrrole (0.035 g, 0.31 mmol) instead of 1-(2-hydroxyethyl)pyrrolidine. After purification, 0.052 g (yield: 42%) of the title compound was obtained as a colorless amorphous form.
¹H-NMR (400 MHz, CDCl₃) δ ppm: 10.13 (1H, brs), 7.68 (1H, m), 7.53-7.47 (3H, m), 7.39 (1H, t, J = 7.8 Hz), 7.27 (1H, m), 7.15 (1H, m), 6.95-6.90 (3H, m), 6.78-6.76 (2H, m), 6.18-6.16 (2H, m), 4.29 (2H, t, J = 5.4 Hz), 4.25 (2H, t, J = 5.4 Hz), 3.15 (1H, hp, J = 7.0 Hz), 2.53 (3H, s), 1.40 (6H, d, J = 7.0 Hz).
MS(ESI) m/z: 481 (M+H)⁺

### (Example 120) 1-[2-({2'-Fluoro-5'-[2-isopropyl-5-(6-methylpyridin-2-yl)-1H-imidazol-4-yl]-1,1'-biphenyl-4-yl}oxy)ethyl]pyrrolidin-2-one (Compound No. 1-701)

The same reaction as in Example 115 was carried out using 1-(2-hydroxyethyl)pyrrolidin-2-one (0.040 g, 0.31 mmol) instead of 1-(2-hydroxyethyl)pyrrolidine. After purification, 0.024 g (yield: 18%) of the title compound was obtained as a white solid.
Melting point: 95°C
¹H-NMR (400 MHz, CDCl₃) δ ppm: 10.08 (1H, brs), 7.66 (1H, m), 7.52-7.46 (3H, m), 7.38 (1H, t, J = 7.8 Hz), 7.38 (1H, m), 7.13 (1H, m), 6.92-6.89 (3H, m), 4.14 (2H, t, J = 5.1 Hz), 3.69 (2H, t, J = 5.1 Hz), 3.59 (2H, t, J = 7.8 Hz), 3.14 (1H, hp, J = 7.0 Hz), 2.53 (3H, s), 2.39 (2H, t, J = 7.8 Hz), 2.02 (2H, q, J = 7.8 Hz), 1.40 (6H, d, J = 7.0 Hz).
MS(ESI) m/z: 498 (M+H)⁺.

### (Example 121) 2-{5-[4-Fluoro-3-(1-methyl-1H-pyrazol-4-yl)phenyl]-1H-imidazol-4-yl}-6-methylpyridine (Compound No. 1-735)

### (121a) 2-[4-(3-Bromo-4-fluorophenyl)-1H-imidazol-5-yl]-6-methylpyridine

1-(3-Bromo-4-fluorophenyl)-2-(6-methylpyridin-2-yl)ethanone (3.6 g, 12 mmol) obtained in Example (60a) was dissolved in dimethylsulfoxide (50 mL). The resulting mixture was stirred at 60°C, and a 48% hydrobromic acid aqueous solution (12 mL) was gradually added dropwise thereto. The resulting mixture was stirred at 60°C for 3 hr. The reaction solution was cooled to room temperature and was put into water (100 mL). The pH of the resulting mixture was adjusted to 8 with saturated aqueous sodium bicarbonate. After extraction with ethyl acetate, the organic layer was washed with water and brine, and then dried with anhydrous magnesium sulfate. The solvent was evaporated under reduced pressure. The residue was dissolved in acetic acid (40 mL), and ammonium acetate (5.0 g, 65 mmol) and hexamethylenetetramine (0.30 g, 2.1 mmol) were added thereto. The resulting mixture was heated under reflux for 1.5 hr. The reaction solution was cooled to room temperature and was put into water (400 mL). The insoluble matter was removed by filtration. The filtrate was neutralized with 28% ammonia water. After extraction with ethyl acetate, the organic layer was washed with water and brine, and then dried with anhydrous magnesium sulfate. The solvent was evaporated under reduced pressure, and the resulting crude product was purified by silica gel column chromatography (eluting solvent; ethyl acetate : methanol = 50 : 1) to obtain 1.1 g (yield: 27%) of the title compound as a yellow solid.
Melting point: 205°C
¹H-NMR (400 MHz, CDCl₃) δ ppm: 10.43 (1H, brs), 7.90 (1H, m), 7.72 (1H, s), 7.57 (1H, m), 7.46 (1H, t, J = 7.8 Hz), 7.26 (1H, m), 7.16 (1H, t, J = 7.8 Hz), 7.01 (1H, d, J = 7.8 Hz), 2.56 (3H, s).
MS(ESI) m/z: 332 (M+H)⁺

### (121b) 2-(4-(3-Bromo-4-fluorophenyl)-1-{[2-(trimethylsilyl)ethoxy]methyl}-1H-imidazol-5-yl)-6-methylpyridine and 2-(5-(3-bromo-4-fluorophenyl)-1-{[2-(trimethylsilyl)ethoxy]methyl}-1H-imidazol-5-yl)-6-methylpyridine

Sodium hydride (60%, oil, 0.11 g, 2.8 mmol) was washed with hexane and dried. The resulting sodium hydride was suspended in tetrahydrofuran (10 mL). Then, a tetrahydrofuran solution (10 mL) of 2-[4-(3-bromo-4-fluorophenyl)-1H-imidazol-5-yl]-6-methylpyridine (0.61 g, 1.8 mmol) obtained in Example (121a) was added thereto. The resulting mixture was stirred at room temperature for 15 min. 2-(Trimethylsilyl)ethoxymethyl chloride (0.37 g, 2.2 mmol) was added thereto. The resulting mixture was stirred at room temperature for 2 hr. To this reaction solution, water was added. After extraction with methylene chloride, the organic layer was separated using an Empore cartridge (GL Science). The solvent was evaporated under reduced pressure, and the resulting crude product was purified by silica gel column chromatography (NH, eluting solvent; ethyl acetate : hexane = 2 : 1) to obtain 0.81 g (yield: 95%) of a mixture of the title compound as a yellow amorphous form.
MS(ESI) m/z: 462 (M+H)⁺

### (121c) 2-{5-[4-Fluoro-3-(1-methyl-1H-pyrazol-4-yl)phenyl]-1H-imidazol-4-yl}-6-methylpyridine

A mixture (0.40 g, 0.86 mmol) of 2-(4-(3-bromo-4-fluorophenyl)-1-{[2-(trimethylsilyl)ethoxy]methyl}-1H-imidazol-5-yl)-6-methylpyridine and 2-(5-(3-bromo-4-fluorophenyl)-1-{[2-(trimethylsilyl)ethoxy]methyl}-1H-imidazol-5-yl)-6-methylpyridine obtained in Example (121b) and 1-methyl-4-(4,4,5,5-tetramethyl-1,3,2-dioxaborolan-2-yl)-1H-pyrazole (0.27 g, 1.3 mmol) were dissolved in 1,2-dimethoxyethane (4 mL), and a 2M sodium carbonate aqueous solution (2 mL) and tetrakis(triphenylphosphine) palladium (0.047 g, 0.043 mmol) were added thereto. The resulting mixture was heated under a nitrogen atmosphere under reflux for 4 hr in a shield tube. The reaction solution was cooled to room temperature, and water was added thereto. After extraction with methylene chloride, the organic layer was separated using an Empore cartridge (GL Science). The solvent was evaporated under reduced pressure, and the resulting crude product was purified by silica gel column chromatography (NH, eluting solvent; ethyl acetate : hexane = 7 : 3) to obtain a protected form (0.37 g) of the title compound as a light yellow amorphous form. The resulting protected form (0.37 g, 0.79 mmol) was dissolved in ethanol (6 mL), and a 3 N hydrochloric acid aqueous solution (3 mL) was added thereto. The resulting mixture was stirred at room temperature for 1 hr and then stirred at 80°C for 12 hr. The reaction solution was cooled to room temperature, and saturated aqueous sodium bicarbonate was added thereto. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; ethyl acetate : methanol = 30 : 1) to obtain 0.17 g (yield: 59%) of the title compound as a colorless amorphous form.
¹H-NMR (400 MHz, CDCl₃) δ ppm: 10.49 (1H, brs), 7.84 (1H, m), 7.80 (1H, s), 7.76 (1H, d, J = 2.0 Hz), 7.72 (1H, s), 7.45-7.37 (2H, m), 7.30 (1H, d, J = 7.8 Hz), 7.13 (1H, m), 6.96 (1H, d, J = 7.8 Hz), 3.94 (3H, s), 2.55 (3H, s).
MS(ESI) m/z: 334 (M+H)⁺

### (Example 122) 2-{5-[6-Fluoro-4'-(methylsulfonyl)-1,1'-biphenyl-3-yl]-1H-imidazol-4-yl}-6-methylpyridine (Compound No. 1-742)

The same reaction as in Example (121c) was carried out using 4-(methylsulfonyl)phenylboronic acid (0.26 g, 1.3 mmol) instead of 1-methyl-4-(4,4,5,5-tetramethyl-1,3,2-dioxaborolan-2-yl)-1H-pyrazole. After purification, 0.27 g (yield: 78%) of the title compound was obtained as a white solid.
Melting point: 202°C
¹H-NMR (400 MHz, CDCl₃) δ ppm: 10.39 (1H, brs), 7.97 (2H, d, J = 8.6 Hz), 7.79-7.72 (4H, m), 7.66 (1H, m), 7.43 (1H, t, J = 7.8 Hz), 7.31 (1H, d, J = 7.8 Hz), 7.22 (1H, m), 6.99 (1H, d, J = 7.8 Hz), 3.09 (3H, s), 2.56 (3H, s).
MS(ESI) m/z: 407 (M+H)⁺.

### (Example 123) 2- {4-[4-Fluoro-3-(1H-pyrrol-3-yl)phenyl]-2-isopropyl-1H-imidazol-5-yl}-6-methylpyridine (Compound No. 1-683)

### (123a) 2-{4-[4-Fluoro-3-(4,4,5,5-tetramethyl-1,3,2-dioxaborolan-2-yl)phenyl]-2-isopropyl-1H-imidazol-5-yl}-6-methylpyridine

The same reaction as in Example (35a) was carried out using 2-[4-(3-bromo-4-fluorophenyl)-2-isopropyl-1H-imidazol-5-yl]-6-methylpyridine (3.8 g, 10 mmol) obtained in Example (60b) instead of 2-[4-(3-bromophenyl)-2-isopropyl-1H-imidazol-5-yl]-6-methylpyridine obtained in Example (1b). After purification, 2.0 g (yield: 47%) of the title compound was obtained as a white amorphous form.
¹H-NMR (500 MHz, CDCl₃) δ ppm: 8.02 (1H, m), 7.63 (1H, m), 7.34 (1H, m), 7.12 (1H, d, J = 8.3 Hz), 7.04 (1H, m), 6.91 (1H, d, J = 7.3 Hz), 3.15 (1H, m), 2.54 (3H, s), 1.41 (6H, d, J = 7.3 Hz), 1.24 (12H, s).
MS(ESI) m/z: 422 (M+H)⁺

### (123b) 2- {4-[4-Fluoro-3-(1H-pyrrol-3-yl)phenyl]-2-isopropyl-1H-imidazol-5-yl}-6-methylpyridine

The same reaction as in Example 78 was carried out using 2-{4-[4-fluoro-3-(4,4,5,5-tetramethyl-1,3,2-dioxaborolan-2-yl)phenyl]-2-isopropyl-1H-imidazol-5-yl}-6-methylpyridine (0.21 g, 0.50 mmol) obtained in Example (123a) instead of 2-{2-isopropyl-4-[3-(4,4,5,5-tetramethyl-1,3,2-dioxaborolan-2-yl)phenyl]-1H-imidazol-5-yl}-6-methylpyridine obtained in Example (35a). After purification, 0.057 g (yield: 31%) of the title compound was obtained as a white amorphous form. Melting point: 106 to 110°C
¹H-NMR (500 MHz, CDCl₃) δ ppm: 10.56 (1H, brs), 8.58 (1H, brs), 7.83 (1H, d, J = 6.3 Hz), 7.39-7.36 (2H, m), 7.28-7.26 (2H, m), 7.10 (1H, m), 6.92 (1H, d, J = 7.3 Hz), 6.80 (1H, d, J = 2.4 Hz), 6.57 (1H, s), 3.15 (1H, m), 2.49 (3H, s), 1.38 (6H, d, J = 6.8 Hz).
MS(ESI) m/z: 361 (M+H)⁺

### (Example 124) 2-{2-Fluoro-5-[2-isopropyl-5-(6-methylpyridin-2-yl)-1H-imidazol-4-yl]phenyl}-5-(methylsulfonyl)pyridine (Compound No. 1-694)

2-{4-[4-Fluoro-3-(4,4,5,5-tetramethyl-1,3,2-dioxaborolan-2-yl)phenyl]-2-isopropyl-1H-imidazol-5-yl}-6-methylpyridine (0.21 g, 0.50 mmol) obtained in Example (123a) and 2-bromo-5-(methylsulfonyl)pyridine (0.12 g, 0.46 mmol) were dissolved in 1,2-dimethoxyethane (5 mL), and tripotassium phosphate n-hydrate (0.22 g, 1.0 mmol) and dichloro[1,1'-bis(diphenylphosphino)ferrocene]palladium(II)-methylene chloride complex (0.043 g, 0.053 mmol) were added thereto. The resulting mixture was heated under reflux under a nitrogen atmosphere for 2 hr. The reaction solution was cooled to room temperature and then diluted with ethyl acetate. After filtration using silica gel, the filtrate was concentrated under reduced pressure. The resulting crude product was purified by silica gel column chromatography (NH, eluting solvent; hexane : ethyl acetate = 3 : 2) to obtain 0.12 g (yield: 56%) of the title compound as a white amorphous form.
Melting point: 105 to 108°C
¹H-NMR (500 MHz, CDCl₃) δ ppm: 10.73 (1H, brs), 9.18 (1H, d, J = 2.4 Hz), 8.35 (1H, d, J = 7.8 Hz), 8.26 (1H, dd, J = 2.4, 8.3 Hz), 8.02 (1H, d, J = 8.3 Hz), 7.72 (1H, m), 7.41 (1H, m), 7.27 (1H, d, J = 7.3 Hz), 7.22 (1H, m), 6.95 (1H, d, J = 7.3 Hz), 3.15 (3H, s), 3.12 (1H, m), 2.48 (3H, s), 1.35 (6H, d, J = 6.8 Hz).
MS(ESI) m/z: 451 (M+H)⁺

### (Example 125) 2-{4-[3-(1,1-Dioxido-2,3-dihydro-1-benzothien-5-yl)-4-fluorophenyl]-2-isopropyl-1H-imidazol-5-yl}-6-methylpyridine (Compound No. 1-793)

### (125a) 2-{4-[3-(1,1-Dioxido-1-benzothien-5-yl)-4-fluorophenyl]-2-isopropyl-1H-imidazol-5-yl}-6-methylpyridine

The same reaction as in Example 124 was carried out using 5-bromo-1-benzothiophene 1,1-dioxide (0.13 g, 0.51 mmol) instead of 2-bromo-5-(methylsulfonyl)pyridine. After purification, 0.13 g (yield: 54%) of the title compound was obtained as a white amorphous form.
¹H-NMR (500 MHz, CDCl₃) δ ppm: 7.76 (1H, d, J = 7.8 Hz), 7.74-7.70 (2H, m), 7.65 (1H, m), 7.58 (1H, s), 7.41 (1H, m), 7.25-7.20 (3H, m), 6.96 (1H, d, J = 7.8 Hz), 6.76 (1H, d, J = 6.8 Hz), 3.16 (1H, m), 2.56 (3H, s), 1.42 (6H, d, J = 7.3 Hz).
MS(ESI) m/z: 460 (M+H)⁺

### (125b) 2-{4-[3-(1,1-Dioxido-2,3-dihydro-1-benzothien-5-yl)-4-fluorophenyl]-2-isopropyl-1H-imidazol-5-yl}-6-methylpyridine

The same reaction as in Example 86 was carried out using 2-{4-[3-(1,1-dioxido-1-benzothien-5-yl)-4-fluorophenyl]-2-isopropyl-1H-imidazol-5-yl}-6-methylpyridine (0.10 g, 0.22 mmol) obtained in Example (125a) instead of 2-{4-[3-(1,1-dioxido-1-benzothien-5-yl)phenyl]-2-isopropyl-1H-imidazol-5-yl}-6-methylpyridine obtained in Example 85. After purification, 0.036 g (yield: 35%) of the title compound was obtained as a white solid.
Melting point: 119 to 123°C
¹H-NMR (500 MHz, CDCl₃) δ ppm: 10.27 (1H, brs), 7.78 (1H, d, J = 7.8 Hz), 7.72 (1H, dd, J = 2.0, 7.8 Hz), 7.66-7.62 (2H, m), 7.58 (1H, s), 7.42 (1H, m), 7.25 (1H, d, J = 7.8 Hz), 7.20 (1H, m), 6.96 (1H, d, J = 7.8 Hz), 3.54 (2H, t, J = 6.8 Hz), 3.43 (2H, t, J = 6.8 Hz), 3.15 (1H, m), 2.53 (3H, s), 1.40 (6H, d, J = 7.3 Hz).
MS(ESI) m/z: 462 (M+H)⁺.

### (Example 126) 2'-Fluoro-5'-[2-isopropyl-5-(6-methylpyridin-2-yl)-1H-imidazol-4-yl]-1,1'-biphenyl-4-sulfonyl fluoride (Compound No. 1-719)

2- {4-[4-Fluoro-3-(4,4,5,5-tetramethyl-1,3,2-dioxaborolan-2-yl)phenyl]-2-isopropyl-1H-imidazol-5-yl}-6-methylpyridine (0.17 g, 0.41 mmol) obtained in Example (123a) and 4-bromobenzenesulfonyl fluoride (0.11 g, 0.46 mmol) were dissolved in 1,2-dimethoxyethane (3 mL), and a 2 M sodium carbonate aqueous solution (0.50 mL) and tetrakis(triphenylphosphine) palladium (0.025 g, 0.022 mmol) were added thereto. The resulting mixture was heated under reflux under a nitrogen atmosphere for 3 hr. The reaction solution was cooled to room temperature, and water was added thereto. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; hexane : ethyl acetate = 3 : 2) to obtain 0.057 g (yield: 31%) of the title compound as a white amorphous form.
¹H-NMR (500 MHz, CDCl₃) δ ppm: 10.35 (1H, brs), 8.06 (2H, d, J = 8.3 Hz), 7.82 (2H, d, J = 8.3 Hz), 7.77 (1H, m), 7.66 (1H, m), 7.43 (1H, m), 7.26-7.21 (2H, m), 6.98 (1H, d, J = 7.8 Hz), 3.15 (1H, m), 2.52 (3H, s), 1.39 (6H, d, J = 6.8 Hz). MS(ESI) m/z: 454 (M+H)⁺

### (Example 127) 2'-Fluoro-5'-[2-isopropyl-5-(6-methylpyridin-2-yl)-1H-imidazol-4-yl]-1,1'-biphenyl-4-sulfonamide (Compound No. 1-205)

The same reaction as in Example 126 was carried out using 4-bromobenzenesulfonamide (0.12 g, 0.51 mmol) instead of 4-bromobenzenesulfonyl fluoride. After purification, 0.16 g (yield: 69%) of the title compound was obtained as a white solid.
Melting point: 245 to 246°C
¹H-NMR (500 MHz, DMSO-d₆) δ ppm: 12.14 (0.5H, s), 12.08 (0.5H, s), 8.00 (0.5H, m), 7.95-7.90 (2H, m), 7.85 (1H, m), 7.80 (1H, d, J = 7.8 Hz), 7.72-7.62 (3H, m), 7.43 (2H, s), 7.39-7.28 (1.5H, m), 7.13 (0.5H, d, J = 7.3 Hz), 7.03 (0.5H, d, J = 7.3 Hz), 3.06 (1H, m), 2.50 (3H, s), 1.34-1.30 (6H, m).
MS(ESI) m/z: 451 (M+H)⁺

### (Example 128) 5-{2-Fluoro-5-[2-isopropyl-5-(6-methylpyridin-2-yl)-1H-imidazol-4-yl]phenyl}-2-(methylsulfinyl)pyridine (Compound No. 1-753)

### (128a) 5-{2-Fluoro-5-[2-isopropyl-5-(6-methylpyridin-2-yl)-1H-imidazol-4-yl]phenyl}-2-(methylthio)pyridine

The same reaction as in Example 126 was carried out using 5-bromo-2-(methylthio)pyridine (0.25 g, 1.2 mmol) instead of 4-bromobenzenesulfonyl fluoride. After purification, 0.34 g (yield: 68%) of the title compound was obtained as a white amorphous form.
¹H-NMR (400 MHz, CDCl₃) δ ppm: 9.92 (1H, brs), 8.60 (1H, s), 7.70-7.67 (2H, m), 7.59 (1H, m), 7.38 (1H, m), 7.25-7.15 (3H, m), 6.92 (1H, d, J = 7.4 Hz), 3.15 (1H, m), 2.59 (3H, s), 2.55 (3H, s), 1.42 (6H, d, J = 7.0 Hz).
MS(ESI) m/z: 419 (M+H)⁺

### (128b) 5-{2-Fluoro-5-[2-isopropyl-5-(6-methylpyridin-2-yl)-1H-imidazol-4-yl]phenyl}-2-(methylsulfinyl)pyridine

The same reaction as in Example 104 was carried out using 5-{2-fluoro-5-[2-isopropyl-5-(6-methylpyridin-2-yl)-1 H-imidazol-4-yl]phenyl -2-(methylthio)pyridine (0.14 g, 0.34 mmol) obtained in Example (128a) instead of 2-(4-{6-fluoro-4'-[(methylthio)methyl]-1,1'-biphenyl-3-yl}-2-isopropyl-1H-imidazol-5-yl)-6-methylpyridine obtained in Example (103b). After purification, 0.089 g (yield: 61 %) of the title compound was obtained as a white amorphous form.
Melting point: 100 to 102°C
¹H-NMR (500 MHz, CDCl₃) δ ppm: 10.69 (1H, brs), 8.81 (1H, s), 8.13 (1H, m), 8.08 (1H, d, J = 8.3 Hz), 7.78 (1H, dd, J = 2.0, 7.8 Hz), 7.67 (1H, m), 7.44 (1H, m), 7.28-7.22 (2H, m), 6.98 (1H, d, J = 7.8 Hz), 3.13 (1H, m), 2.89 (3H, s), 2.48 (3H, s), 1.36 (6H, d, J = 7.3 Hz).
MS(ESI) m/z: 435 (M+H)⁺

### (Example 129) 5-{2-Fluoro-5-[2-isopropyl-5-(6-methylpyridin-2-yl)-1H-imidazol-4-yl]phenyl}-2-(methylsulfonyl)pyridine (Compound No. 1-755)

The same reaction as in Example 105 was carried out using 5-{2-fluoro-5-[2-isopropyl-5-(6-methylpyridin-2-yl)-1H-imidazol-4-yl]phenyl}-2-(methylthio)pyridine (0.20 g, 0.48 mmol) obtained in Example (128a) instead of 2-(4-{6-fluoro-4'-[(methylthio)methyl]-1,1'-biphenyl-3-yl}-2-isopropyl-1H-imidazol-5-yl)-6-methylpyridine obtained in Example (103b). After purification, 0.16 g (yield: 73%) of the title compound was obtained as a white amorphous form.
Melting point: 102 to 105°C
¹H-NMR (400 MHz, CDCl₃) δ ppm: 10.78 (1H, brs), 8.87 (1H, s), 8.13-8.12 (2H, m), 7.75 (1H, dd, J = 2.4, 7.4 Hz), 7.68 (1H, m), 7.43 (1H, m), 7.26-7.21 (2H, m), 6.96 (1H, d, J = 7.4 Hz), 3.26 (3H, s), 3.12 (1H, m), 2.46 (3H, s), 1.34 (6H, d, J = 7.0 Hz). MS(ESI) m/z: 451 (M+H)⁺

### (Example 130) 2-{4-[4-Fluoro-3-(1-methyl-1H-imidazol-4-yl)phenyl]-2-isopropyl-1H-imidazol-5-yl}-6-methylpyridine (Compound No. 1-775)

The same reaction as in Example 126 was carried out using 4-iodo-1-methyl-1H-imidazole (0.12 g, 0.57 mmol) instead of 4-bromobenzenesulfonyl fluoride. After purification, 0.035 g (yield: 18%) of the title compound was obtained as a white solid.
Melting point: 100 to 103°C
¹H-NMR (500 MHz, CDC1₃) δ ppm: 10.31 (1H, brs), 8.39 (1H, m), 7.46-7.25 (5H, m), 7.11 (1H, m), 6.88 (1H, d, J = 6.8 Hz), 3.73 (3H, s), 3.12 (1H, m), 2.49 (3H, s), 1.38 (6H, d, J = 6.8 Hz).
MS(ESI) m/z: 376 (M+H)⁺.

### (Example 131) 2-{4-[4-Fluoro-3-(1,3-oxazol-2-yl)phenyl]-2-isopropyl-1H-imidazol-5-yl}-6-methylpyridine (Compound No. 1-746)

### (131a) 2-Fluoro-5-[2-(6-methylpyridin-2-yl)-2-oxoethyl]benzonitrile

The same reaction as in Example (143a) was carried out using 2-fluoro-5-formylbenzonitrile (15 g, 0.10 mol) instead of 3-bromo-4-fluorobenzaldehyde. After purification, 24 g (yield: 94%) of the title compound was obtained as a white solid.
¹H-NMR (400 MHz, CDCl₃) δ ppm: 7.83 (1H, d, J = 7.8 Hz), 7.71 (1H, m), 7.60 (1H, dd, J = 2.4, 5.9 Hz), 7.55 (1H, m), 7.35 (1H, d, J = 7.4 Hz), 7.14 (1H, m), 4.54 (2H, s), 2.65 (3H, s).
MS(ESI) m/z: 255 (M+H)⁺

### (131b) 2-Fluoro-5-[2-isopropyl-5-(6-methylpyridin-2-yl)-1H-imidazol-4-yl]benzonitrile

The same reaction as in Example (1b) was carried out using 2-fluoro-5-[2-(6-methylpyridin-2-yl)-2-oxoethyl]benzonitrile (10 g, 40 mmol) obtained in Example (131a) instead of 1-(3-bromophenyl)-2-(6-methylpyridin-2-yl)ethanone obtained in Example (1a). After purification, 8.3 g (yield: 65%) of the title compound was obtained as a white solid.
¹H-NMR (400 MHz, CDCl₃) δ ppm: 10.39 (1H, brs), 7.90 (1H, dd, J = 2.4, 5.9 Hz), 7.86 (1H, m), 7.44 (1H, m), 7.20 (1H, m), 7.11 (1H, d, J = 7.8 Hz), 6.98 (1H, d, J = 7.4 Hz), 3.11 (1H, m), 2.50 (3H, s), 1.37 (6H, d, J = 7.0 Hz).
MS(ESI) m/z: 321 (M+H)⁺

### (131c) 2-Fluoro-5-[2-isopropyl-5-(6-methylpyridin-2-yl)-1H-imidazol-4-yl]benzamide

Potassium trimethylsiloxide (0.39 g, 3.0 mmol) was added to a 1,4-dioxane solution (5 mL) of 2-fluoro-5-[2-isopropyl-5-(6-methylpyridin-2-yl)-1H-imidazol-4-yl]benzonitrile (0.32 g, 1.0 mmol) obtained in Example (131b). The resulting mixture was heated under reflux for 2 hr. The reaction solution was concentrated under reduced pressure, and a saturated ammonium chloride aqueous solution was added thereto. After extraction with ethyl acetate, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.20 g (yield: 60%) of the title compound as a white amorphous form.
¹H-NMR (400 MHz, CDCl₃) δ ppm: 8.33 (1H, m), 7.77 (1H, m), 7.37 (1H, m), 7.18-7.13 (2H, m), 6.92 (1H, d, J = 7.4 Hz), 6.64 (1H, brs), 5.77 (1H, brs), 3.13 (1H, m), 2.52 (3H, s), 1.40 (6H, d, J = 7.0 Hz).
MS(ESI) m/z: 339 (M+H)⁺

### (131d) 2-{4-[4-Fluoro-3-(1,3-oxazol-2-yl)phenyl]-2-isopropyl-1H-imidazol-5-yl}-6-methylpyridine

Vinylene carbonate (0.10 g, 1.2 mmol) and polyphosphoric acid (2.0 g) were added to 2-fluoro-5-[2-isopropyl-5-(6-methylpyridin-2-yl)-1H-imidazol-4-yl]benzamide (0.086 g, 0.25 mmol) obtained in Example (131c). The resulting mixture was stirred at 160°C for 3 hr. The reaction solution was cooled to room temperature and was neutralized with a 10% sodium hydroxide aqueous solution. After extraction with ethyl acetate, the organic layer was washed with water and brine, and then dried with anhydrous magnesium sulfate. The solvent was evaporated under reduced pressure. The resulting crude product was purified by high-performance liquid chromatography (GL Science ODS-3, eluting solvent; water : acetonitrile = 95 : 5 to 5 : 95) to obtain 0.037 g (yield: 40%) of the title compound as a white amorphous form.
¹H-NMR (500 MHz, CDCl₃) δ ppm: 8.32 (1H, dd, J = 2.0, 7.4 Hz), 7.74 (1H, s), 7.69 (1H, m), 7.41 (1H, m), 7.30 (1H, s), 7.25-7.21 (2H, m), 6.95 (1H, d, J = 7.3 Hz), 3.15 (1H, m), 2.50 (3H, s), 1.38 (6H, d, J = 6.9 Hz).
MS(ESI) m/z: 363 (M+H)⁺

### (Example 132) 2-{4-[4-Fluoro-3-(1,3-thiazol-2-yl)phenyl]-2-isopropyl-1H-imidazol-5-yl}-6-methylpyridine (Compound No. 1-750)

Thioacetamide (0.078 g, 1.0 mmol) and a 4 N hydrochloric acid-1,4-dioxane solution (5 mL) were added to a N,N-dimethylformamide solution (1 mL) of 2-fluoro-5-[2-isopropyl-5-(6-methylpyridin-2-yl)-1H-imidazol-4-yl]benzonitrile (0.13 g, 0.41 mmol) obtained in Example (131b). The resulting mixture was stirred at 100°C for 3 hr. The reaction solution was cooled to room temperature and then neutralized with saturated aqueous sodium bicarbonate. After extraction with ethyl acetate, the organic layer was washed with water and brine, and then dried with anhydrous magnesium sulfate. The solvent was evaporated under reduced pressure. The resulting crude product was dissolved in ethanol (5 mL). To this solution, bromoacetaldehyde diethylacetal (0.15 mL, 0.98 mmol) and a 3 N hydrochloric acid aqueous solution (0.1 mL) were added. The resulting mixture was heated under reflux for 1 hr. The reaction solution was concentrated under reduced pressure, and saturated aqueous sodium bicarbonate was added thereto. After extraction with methylene chloride, the organic layer was separated using an Empore cartridge (GL Science). The solvent was evaporated under reduced pressure. The resulting crude product was purified by high-performance liquid chromatography (GL Science ODS-3, eluting solvent; water : acetonitrile = 95 : 5 to 5 : 95) to obtain 0.081 g (yield: 52%) of the title compound as a light yellow amorphous form.
¹H-NMR (500 MHz, CDCl₃) δ ppm: 10.04 (1H, brs), 8.55 (1H, dd, J = 2.0, 7.3 Hz), 7.91 (1H, m), 7.65 (1H, m), 7.44 (1H, m), 7.37 (1H, m), 7.26-7.22 (2H, m), 6.93 (1H, d, J = 7.3 Hz), 3.15 (1H, m), 2.55 (3H, s), 1.42 (6H, d, J = 6.8 Hz).
MS(ESI) m/z: 379 (M+H)⁺.

### (Example 133) 2-{4-[4-Fluoro-3-(1H-1,2,4-triazol-3-yl)phenyl]-2-isopropyl-1H-imidazol-5-yl;-6-methylpyridine (Compound No. 1-761)

N,N-Dimethylformamide dimethylacetal (1.0 mL, 7.5 mmol) was added to 2-fluoro-5-[2-isopropyl-5-(6-methylpyridin-2-yl)-1H-imidazol-4-yl]benzamide (0.20 g, 0.60 mmol) obtained in Example (131c). The resulting mixture was stirred at 120°C for 1 hr. The reaction solution was concentrated under reduced pressure. The resulting crude product was dissolved in acetic acid (2 mL), and hydrazine-hydrate (0.038 g, 0.76 mmol) was added thereto. The resulting mixture was stirred at 90°C for 1.5 hr. The reaction solution was concentrated under reduced pressure, and saturated aqueous sodium bicarbonate was added thereto. After extraction with methylene chloride, the organic layer was washed with water and brine, and then dried with anhydrous magnesium sulfate. The solvent was evaporated under reduced pressure. The resulting crude product was purified by high-performance liquid chromatography (GL Science ODS-3, eluting solvent; water : acetonitrile = 95 : 5 to 5 : 95) to obtain 0.068 g (yield: 31%) of the title compound as a white solid. Melting point: 184 to 186°C
¹H-NMR (400 MHz, DMSO-d₆) δ ppm: 12.10 (1H, s), 8.62 (0.5H, brs), 8.47 (0.5H, d, J = 6.3 Hz), 8.37 (0.5H, d, J = 6.3 Hz), 7.76 (0.5H, m), 7.66-7.58 (2H, m), 7.30-7.22 (2H, m), 7.09 (0.5H, d, J = 7.8 Hz), 6.99 (0.5H, d, J = 7.8 Hz), 3.09 (1H, m), 2.49 (3H, s), 1.31 (6H, d, J = 7.0 Hz).
MS(ESI) m/z: 363 (M+H)⁺

### (Example 134) 2-{4-[4-Fluoro-3-(1H-imidazol-2-yl)phenyl]-2-isopropyl-1H-imidazol-5-yl}-6-methylpyridine (Compound No. 1-765)

2-Fluoro-5-[2-isopropyl-5-(6-methylpyridin-2-yl)-1H-imidazol-4-yl]benzonitrile (0.48 g, 1.5 mmol) obtained in Example (131b) was dissolved in methylene chloride (1 mL) and toluene (3 mL), and a diisobutylaluminum hydride solution (1.0 M in toluene, 1.5 mL, 1.5 mmol) was added thereto under a nitrogen atmosphere at -78°C. The resulting mixture was stirred at -78°C for 1 hr. Then, a diisobutylaluminum hydride solution (1.5 mL, 1.5 mmol) was further added thereto, and the resulting mixture was stirred at -78°C for 1 hr. The reaction solution was returned to room temperature, and a saturated ammonium chloride aqueous solution was added thereto to terminate the reaction. After extraction with methylene chloride, the organic layer was washed with water and brine, and then dried with anhydrous magnesium sulfate. The solvent was evaporated under reduced pressure, and the resulting crude product was dissolved in ethanol (3 mL). To this solution, 28% ammonia water (0.50 mL) and a 40% glyoxal aqueous solution (0.16 mL, 1.4 mmol) were added. The resulting mixture was stirred at room temperature for 18 hr. The reaction solution was concentrated under reduced pressure. The resulting crude product was purified by high-performance liquid chromatography (GL Science ODS-3, eluting solvent; water : acetonitrile = 95 : 5 to 5 : 95) to obtain 0.083 g (yield: 50%) of the title compound as a white solid.
Melting point: 201 to 205°C
¹H-NMR (500 MHz, DMSO-d₆) δ ppm: 12.20 (0.5H, s), 12.17 (0.5H, s), 12.13 (0.5H, s), 12.10 (0.5H, s), 8.45 (0.5H, dd, J = 2.0, 7.3 Hz), 8.36 (0.5H, dd, J = 2.0, 7.3 Hz), 7.72 (0.5H, m), 7.67-7.57 (2H, m), 7.35-7.22 (2.5H, m), 7.12-7.01 (2H, m), 3.04 (1H, m), 2.50 (3H, s), 1.33-1.31 (6H, m).
MS(ESI) m/z: 362 (M+H)⁺

### (Example 135) 2-{4-[4-Fluoro-3-(1H-pyrazol-3-yl)phenyl]-2-isopropyl-1H-imidazol-5-yl}-6-methylpyridine (Compound No. 1-727)

### (135a) 1-{2-Fluoro-5-[2-isopropyl-5-(6-methylpyridin-2-yl)-1H-imidazol-4-yl]phenyl} ethanone

A methylmagnesium bromide solution (3.0 M in diethyl ether, 1.0 mL, 3.0 mmol) was added to a tetrahydrofuran solution (5 mL) of 2-fluoro-5-[2-isopropyl-5-(6-methylpyridin-2-yl)-1H-imidazol-4-yl]benzonitrile (0.32 g, 1.0 mmol) obtained in Example (131b). The resulting mixture was stirred at 60°C for 4 hr. To this reaction solution, water was added under ice cooling. A 3 N hydrochloric acid aqueous solution was added thereto. The resulting mixture was stirred for 15 min and then neutralized with a saturated sodium carbonate aqueous solution. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; hexane : ethyl acetate = 1 : 1) to obtain 0.27 g (yield: 81%) of the title compound as a white amorphous form.
¹H-NMR (500 MHz, CDCl₃) δ ppm: 10.49 (1H, brs), 8.14 (1H, dd, J = 2.4, 7.3 Hz), 7.79 (1H, m), 7.40 (1H, m), 7.19-7.15 (2H, m), 6.95 (1H, d, J = 7.8 Hz), 3.12 (1H, m), 2.65 (3H, d, J = 4.4 Hz), 2.49 (3H, s), 1.36 (6H, d, J = 7.3 Hz).
MS(ESI) m/z: 338 (M+H)⁺

### (135b) 2-{4-[4-Fluoro-3-(1H-pyrazol-3-yl)phenyl]-2-isopropyl-1H-imidazol-5-yl}-6-methylpyridine

N,N-Dimethylformamide dimethylacetal (1.0 mL, 7.5 mmol) was added to 1-{2-fluoro-5-[2-isopropyl-5-(6-methylpyridin-2-yl)-1H-imidazol-4-yl]phenyl}ethanone (0.15 g, 0.43 mmol) obtained in Example (135a). The resulting mixture was stirred at 90°C for 1.5 hr. The reaction solution was concentrated under reduced pressure. The resulting crude product was dissolved in methanol (3 mL), and acetic acid (0.5 mL) and hydrazine-hydrate (0.035 g, 0.70 mmol) were added thereto. The resulting mixture was stirred at 60°C for 1 hr. The reaction solution was concentrated under reduced pressure, and saturated aqueous sodium bicarbonate was added thereto. After extraction with methylene chloride, the organic layer was washed with water and brine, and then dried with anhydrous magnesium sulfate. The solvent was evaporated under reduced pressure, and the resulting product was purified by high-performance liquid chromatography (GL Science ODS-3, eluting solvent; water : acetonitrile = 95 : 5 to 5 : 95) to obtain 0.082 g (yield: 53%) of the title compound as a white solid.
Melting point: 195 to 198°C
¹H-NMR (500 MHz, CDCl₃) δ ppm: 8.18 (1H, m), 7.61 (1H, d, J = 1.5 Hz), 7.52 (1H, m), 7.42 (1H, m), 7.28 (1H, d, J = 7.3 Hz), 7.11 (1H, m), 6.95 (1H, d, J = 7.8 Hz), 6.68 (1H, s), 3.14 (1H, m), 2.49 (3H, s), 1.34 (6H, d, J = 7.3 Hz).
MS(ESI) m/z: 362 (M+H)⁺.

### (Example 136) 2-{4-[4-Fluoro-3-(1,3-oxazol-5-yl)phenyl]-2-isopropyl-1H-imidazol-5-yl}-6-methylpyridine (Compound No. 1-748)

2-Fluoro-5-[2-isopropyl-5-(6-methylpyridin-2-yl)-1H-imidazol-4-yl]benzonitrile (0.48 g, 1.5 mmol) obtained in Example (131b) was dissolved in methylene chloride (1 mL) and toluene (3 mL), and a diisobutylaluminum hydride solution (1.0 M in toluene, 3.0 mL, 3.0 mmol) was added thereto under a nitrogen atmosphere at -78°C. The resulting mixture was stirred at -78°C for 1 hr. The reaction solution was returned to room temperature, and a saturated ammonium chloride aqueous solution was added thereto to terminate the reaction. After extraction with methylene chloride, the organic layer was washed with water and brine, and then dried with anhydrous magnesium sulfate. The solvent was evaporated under reduced pressure, and the resulting crude product was dissolved in methanol (5 mL). To this solution, p-toluenesulfonylmethyl isocyanide (0.060 g, 0.31 mmol) and sodium methoxide (0.042 g, 0.78 mmol) were added, and the resulting mixture was heated under reflux for 2 hr. The reaction solution was cooled to room temperature, and water was added thereto. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; hexane : ethyl acetate = 3 : 2) to obtain 0.034 g (yield: 40%) of the title compound as a white amorphous form.
¹H-NMR (500 MHz, CDCl₃) δ ppm: 10.52 (1H, brs), 8.07 (1H, dd, J = 2.0, 7.3 Hz), 7.92 (1H, s), 7.57 (1H, m), 7.53 (1H, d, J = 3.9 Hz), 7.41 (1H, m), 7.23-7.17 (2H, m), 6.95 (1H, d, J = 7.8 Hz), 3.15 (1H, m), 2.50 (3H, s), 1.38 (6H, d, J = 6.8 Hz). MS(ESI) m/z: 363 (M+H)⁺

### (Example 137) 2-{4-[4-Fluoro-3-(1H-imidazol-4-yl)phenyl]-2-isopropyl-1H-imidazol-5-yl}-6-methylpyridine (Compound No. 1-769)

A 48% hydrogen bromide aqueous solution (0.20 mL, 1.8 mmol) was added to a dimethylsulfoxide solution (1 mL) of 1-{2-fluoro-5-[2-isopropyl-5-(6-methylpyridin-2-yl)-1H-imidazol-4-yl]phenyl}ethanone (0.20 g, 0.60 mmol) obtained in Example (135a). The resulting mixture was stirred at 60°C for 4 hr. The reaction solution was cooled to room temperature and then neutralized with saturated aqueous sodium bicarbonate. After extraction with ethyl acetate, the organic layer was washed with water and brine, and then dried with anhydrous magnesium sulfate. The solvent was evaporated under reduced pressure, and the resulting crude product was dissolved in methanol (4 mL). To this solution, acetic acid (1 mL), ammonium acetate (0.19 g, 2.4 mmol), and glyoxylic acid-hydrate (0.11 g, 1.2 mmol) were added. The resulting mixture was stirred at room temperature for 2 hr. The reaction solution was concentrated under reduced pressure, and the resulting crude product was purified by high-performance liquid chromatography (GL Science ODS-3, eluting solvent; water : acetonitrile = 95 : 5 to 5 : 95) to obtain 0.099 g (yield: 45%) of the title compound as a white solid.
Melting point: 268 to 270°C
¹H-NMR (500 MHz, DMSO-d₆) δ ppm: 8.39 (1H, brs), 7.76 (1H, s), 7.59-7.47 (3.5H, m), 7.21-7.19 (1.5H, m), 7.06 (1H, brs), 3.08 (1H, m), 2.50 (3H, s), 1.32 (6H, d, J = 6.8 Hz).
MS(ESI) m/z: 362 (M+H)⁺

### (Example 138) 2-{4-[4-Fluoro-3-(1-methyl-1H-pyrazol-5-yl)phenyl]-2-isopropyl-1H-imidazol-5-yl}-6-methylpyridine (Compound No. 1-729)

N,N-Dimethylformamide dimethylacetal (1.0 mL, 7.5 mmol) was added to 1-{2-fluoro-5-[2-isopropyl-5-(6-methylpyridin-2-yl)-1H-imidazol-4-yl]phenyl}ethanone (0.26 g, 0.77 mmol) obtained in Example (135a). The resulting mixture was stirred at 90°C for 1.5 hr. The reaction solution was concentrated under reduced pressure. The resulting crude product was dissolved in methanol (3 mL), and acetic acid (0.5 mL) and methylhydrazine (0.055 g, 1.2 mmol) were added thereto. The resulting mixture was stirred at room temperature for 18 hr. The reaction solution was concentrated under reduced pressure, and saturated aqueous sodium bicarbonate was added thereto. After extraction with methylene chloride, the organic layer was separated using an Empore cartridge (GL Science). The solvent was concentrated under reduced pressure. The resulting crude product (a mixture of two structural isomers) was subjected to high-performance liquid chromatography (GL Science ODS-3, eluting solvent; water : acetonitrile = 95 : 5 to 5 : 95) for separation and purification. By preparatively isolating a highly polar component, 0.065 g (yield: 23%) of the title compound was obtained as a white amorphous form.
¹H-NMR (500 MHz, CDCl₃) δ ppm: 7.68 (1H, m), 7.63 (1H, dd, J = 2.0, 7.3 Hz), 7.52 (1H, d, J = 2.0 Hz), 7.44 (1H, m), 7.25 (1H, m), 7.20 (1H, m), 6.96 (1H, d, J = 7.3 Hz), 6.31 (1H, d, J = 2.0 Hz), 3.81 (3H, s), 2.46 (3H, s), 3.12 (1H, m), 1.33 (6H, d, J = 6.8 Hz).
MS(ESI) m/z: 376 (M+H)⁺

### (Example 139) 2-{4-[4-Fluoro-3-(1-methyl-1H-pyrazol-3-yl)phenyl]-2-isopropyl-1H-imidazol-5-yl}-6-methylpyridine (Compound No. 1-733)

The same reaction as in Example 138 was carried out. After separation and purification for preparatively isolating a low polar component, 0.062 g (yield: 22%) of the title compound was obtained as a white amorphous form.
¹H-NMR (500 MHz, CDCl₃) δ ppm: 10.46 (1H, brs), 8.20 (1H, m), 7.47 (1H, m), 7.39 (1H, d, J = 2.0 Hz), 7.36 (1H, m), 7.22 (1H, d, J = 7.8 Hz), 7.13 (1H, m), 6.90 (1H, d, J = 7.8 Hz), 6.67 (1H, t, J = 2.0 Hz), 3.93 (3H, s), 3.13 (1H, m), 2.49 (3H, s), 1.37 (6H, d, J = 6.8 Hz).
MS(ESI) m/z: 376 (M+H)⁺

### (Example 140) 4-{2-Fluoro-5-[2-isopropyl-5-(6-methylpyridin-2-yl)-1H-imidazol-4-yl]phenyl}-1,3-dihydro-2H-imidazol-2-one (Compound No. 1-777)

A 33% hydrogen bromide-acetic acid solution (0.10 mL) and bromine (0.045 mL, 0.88 mmol) were added to an acetic acid solution (3 mL) of 1-{2-fluoro-5-[2-isopropyl-5-(6-methylpyridin-2-yl)-1H-imidazol-4-yl]phenyl}ethanone (0.26 g, 0.77 mmol) obtained in Example (135a). The resulting mixture was stirred at 60°C for 2 hr. The reaction solution was concentrated under reduced pressure, and saturated aqueous sodium bicarbonate was added thereto. After extraction with methylene chloride, the organic layer was separated using an Empore cartridge (GL Science). The solvent was evaporated under reduced pressure. The resulting crude product was dissolved in acetic acid (5 mL), and water (1 mL), urea (0.051 g, 0.85 mmol), and ammonium acetate (0.12 g, 1.6 mmol) were added thereto. The resulting mixture was heated under reflux for 3 hr. The reaction solution was concentrated under reduced pressure, and the resulting crude product was purified by high-performance liquid chromatography (GL Science ODS-3, eluting solvent; water : acetonitrile = 95 : 5 to 5 : 95) to obtain 0.029 g (yield: 10%) of the title compound as a flesh-colored solid.
Melting point: 177 to 180°C
¹H-NMR (500 MHz, DMSO-d₆) δ ppm: 12.05 (0.5H, s), 11.94 (0.5H, s), 10.49 (0.5H, s), 10.45 (0.5H, s), 10.18 (0.5H, s), 10.16 (0.5H, s), 7.91 (0.5H, dd, J = 1.5, 7.8 Hz), 7.82 (0.5H, dd, J = 1.5, 7.8 Hz), 7.66-7.53 (2H, m), 7.33-6.99 (3H, m), 6.74 (0.5H, s), 6.71 (0.5H, s), 3.08 (1H, m), 2.49 (3H, s), 1.30 (6H, d, J = 7.3 Hz).
MS(ESI) m/z: 378 (M+H)⁺.

### (Example 141) 2-{4-[4-Fluoro-3-(2-methyl-1H-imidazol-4-yl)phenyl]-2-isopropyl-1H-imidazol-5-yl}-6-methylpyridine (Compound No. 1-771)

A 48% hydrogen bromide aqueous solution (0.20 mL, 1.8 mmol) was added to a dimethylsulfoxide solution (1 mL) of 1- {2-fluoro-5-[2-isopropyl-5-(6-methylpyridin-2-yl)-1H-imidazol-4-yl]phenyl}ethanone (0.20 g, 0.60 mmol) obtained in Example (135a). The resulting mixture was stirred at 60°C for 2 hr. The reaction solution was cooled to room temperature and then neutralized with saturated aqueous sodium bicarbonate. After extraction with ethyl acetate, the organic layer was washed with water and brine, and then dried with anhydrous magnesium sulfate. The solvent was evaporated under reduced pressure. The resulting crude product was dissolved in ethanol (4 mL), and 28% ammonia water (1.0 mL) and acetaldehyde (0.10 mL, 1.8 mmol) were added thereto. The resulting mixture was stirred at room temperature for 1 hr. The reaction solution was concentrated under reduced pressure, and water was added thereto. After extraction with methylene chloride, the organic layer was separated using an Empore cartridge (GL Science). The solvent was evaporated under reduced pressure, and the resulting crude product was purified by silica gel column chromatography (NH, eluting solvent: ethyl acetate) to obtain 0.13 g (yield: 56%) of the title compound as a white solid.
Melting point: 230 to 234°C
¹H-NMR (500 MHz, CD₃OD) δ ppm: 8.03 (1H, brs), 7.53 (1H, m), 7.31-7.28 (2H, m), 7.19 (1H, m), 7.14 (1H, m), 7.08 (1H, d, J = 7.8 Hz), 3.16 (1H, m), 2.50 (3H, s), 2.38 (3H, s), 1.40 (6H, d, J = 6.8 Hz).
MS(ESI) m/z: 376 (M+H)⁺

### (Example 142) 2-{4-[3-(2-Ethyl-1H-imidazol-4-yl)-4-fluorophenyl]-2-isopropyl-1H-imidazol-5-yl}-6-methylpyridine (Compound No. 1-773)

The same reaction as in Example 141 was carried out using propionaldehyde (0.11 g, 1.9 mmol) instead of acetaldehyde. After purification, 0.12 g (yield: 49%) of the title compound was obtained as a white solid.
Melting point: 170 to 175°C
¹H-NMR (500 MHz, CD₃OD) δ ppm: 8.06 (1H, brs), 7.53 (1H, m), 7.32 (1H, d, J = 3.4 Hz), 7.28 (1H, m), 7.20 (1H, m), 7.13 (1H, m), 7.08 (1H, d, J = 7.8 Hz), 3.16 (1H, m), 2.74 (2H, q, J = 7.8 Hz), 2.50 (3H, s), 1.40 (6H, d, J = 6.8 Hz), 1.29 (3H, t, J = 7.8 Hz).
MS(ESI) m/z: 390 (M+H)⁺.

### (Example 143) 5-{2-Fluoro-5-[3-(6-methylpyridin-2-yl)-1H-pyrazol-4-yl]phenyl}thiophene-2-sulfonamide (Compound No. 2-628)

### (143a) 2-(3-Bromo-4-fluorophenyl)-1-(6-methylpyridin-2-yl)ethanone

6-Methylpyridine-2-carboxyaldehyde (50 g, 410 mmol) was dissolved in isopropyl alcohol (400 mL), and aniline (46 g, 500 mmol) and diphenyl phosphite (150 g, 660 mmol) were added thereto. The resulting mixture was stirred under a nitrogen atmosphere at room temperature for 1 hr. The reaction solution was heated to 50°C and stirred for 3 hr. The reaction solvent was evaporated under reduced pressure. To the residue, diisopropyl ether was added. The precipitate was collected by filtration and washed with diisopropyl ether. The precipitate was dried to obtain 150 g (yield: 85%) of diphenylanilino(6-methylpyridin-2-yl)methylphosphonate as a white solid. The resulting diphenylanilino(6-methylpyridin-2-yl)methylphosphonate (110 g, 230 mmol) and 3-bromo-4-fluorobenzaldehyde (50 g, 250 mmol) were dissolved in tetrahydrofuran (375 mL) and isopropyl alcohol (75 mL), and cesium carbonate (110 g, 340 mmol) was added thereto. The resulting mixture was stirred under a nitrogen atmosphere at room temperature for 2 hr and acidified with a 3 N hydrochloric acid aqueous solution. The resulting mixture was stirred at room temperature for 1 hr. The reaction solvent was evaporated under reduced pressure, and water (150 mL) was added to the residue. The resulting mixture was neutralized with potassium carbonate and extracted with ethyl acetate. The organic layer was washed with water and brine, and then dried with anhydrous magnesium sulfate. The solvent was evaporated under reduced pressure, and the resulting crude product was purified by silica gel column chromatography (eluting solvent; hexane : ethyl acetate = 9 : 1) and then crystallized from hexane to obtain 58 g (yield: 83%) of the title compound as a white solid.
¹H-NMR (400 MHz, CDCl₃) δ ppm: 7.85 (1H, d, J = 7.8 Hz), 7.71 (1H, t, J = 7.8 Hz), 7.56 (1H, m), 7.34 (1H, d, J = 7.8 Hz), 7.24 (1H, m), 7.05 (1H, t, J = 8.6 Hz), 4.49 (2H, s), 2.65 (3H, s).

### (143b) 2-[4-(3-Bromo-4-fluorophenyl)-1H-pyrazol-3-yl]-6-methylpyridine

2-(3-Bromo-4-fluorophenyl)-1-(6-methylpyridin-2-yl)ethanone (29 g, 93 mmol) obtained in Example (143a) was dissolved in N,N-dimethylformamide (60 mL), and N,N-dimethylformamide dimethylacetal (14 mL, 100 mmol) was added thereto at room temperature. The resulting mixture was stirred at 90°C for 2 hr and then cooled to room temperature. The reaction solution was evaporated under reduced pressure to obtain a reddish brown oily material. The resulting reddish brown oily material was dissolved in ethanol (70 mL), and hydrazine monohydrate (5.0 mL, 100 mmol) was added thereto. The resulting mixture was stirred at room temperature for 3 hr. The reaction solution was evaporated under reduced pressure, and the resulting crude product was purified by silica gel column chromatography (eluting solvent; hexane : ethyl acetate = 3 : 7 to 1 : 9) to obtain 31 g (yield: 99%) of the title compound as a yellow amorphous form.
¹H-NMR (400 MHz, CDCl₃) δ ppm: 11.57 (brs, 1H), 7.65 (1H, m), 7,62 (1H, s), 7.48 (1H, t, J = 7.8 Hz), 7.32 (1H, m), 7.17-7.07 (3H, m), 2.57 (3H, s).
MS(ESI) m/z: 332 (M+H)⁺

### (143c) 2-(4-(3-Bromo-4-fluorophenyl)-1-([2-(trimethylsilyl)ethoxy]methyl)-1H-pyrazol-3-yl)-6-methylpyridine

Sodium hydride (60%, oil, 5.4 g, 140 mmol) was washed with a small amount of hexane, dried, and then suspended in tetrahydrofuran (50 mL). The resulting mixture was stirred at 0°C. To this mixture, a tetrahydrofuran solution (100 mL) of 2-[4-(3-bromo-4-fluorophenyl)-1H-pyrazol-3-yl]-6-methylpyridine (30 g, 90 mmol) obtained in Example (143b) was gradually added while stirring. After completion of the addition, the reaction solution was warmed to room temperature and stirred for 20 min. Then, 2-(trimethylsilyl)ethoxymethyl chloride (19 mL, 110 mmol) was added to the reaction solution. The resulting mixture was stirred at room temperature for 3 hr. The reaction solvent was evaporated under reduced pressure, and water was added to the residue. After extraction with ethyl acetate, the organic layer was washed with water and brine, and then dried with anhydrous magnesium sulfate. The solvent was evaporated under reduced pressure, and the resulting crude product was purified by silica gel column chromatography (eluting solvent; hexane : ethyl acetate = 7 : 3) to obtain 42 g (yield: 100%) of the title compound as a light yellow solid.
¹H-NMR (400 MHz, CDCl₃) δ ppm: 7.80 (1H, m), 7.75 (1H, s), 7.62 (1H, t, J = 7.8 Hz), 7.44 (1H, d, J = 7.8 Hz), 7.31 (1H, m), 7.15 (1H, d, J = 7.8 Hz), 7.09 (1H, t, J = 8.6 Hz), 5.57 (2H, s), 3.74 (2H, t, J = 7.8 Hz), 2.60 (3H, s), 1.04 (2H, t, J = 7.8 Hz), 0.08 (9H, s).

### (143d) 2-(4-[4-Fluoro-3-(4,4,5,5-tetramethyl-1,3,2-dioxaborolan-2-yl)phenyl]-1-{[2-(trimethylsilyl)ethoxy]methyl}-1H-pyrazol-3-yl)-6-methylpyridine

2-(4-(3-Bromo-4-fluorophenyl)-1- { [2-(trimethylsilyl)ethoxy]methyl} -1 H-pyrazol-3-yl)-6-methylpyridine (25 g, 54 mmol) obtained in Example (143c) and bis(pinacolato)diboron (20 g, 81 mmol) were dissolved in N,N-dimethylformamide (200 mL), and potassium acetate (16 g, 160 mmol) and dichloro[1,1'-bis(diphenylphosphino)ferrocene]palladium(II)-methylene chloride complex (4.4 g, 5.4 mmol) were added thereto. The resulting mixture was stirred under a nitrogen atmosphere at 100°C for 8 hr. The reaction solution was cooled to room temperature, and the reaction solvent was evaporated under reduced pressure. The resulting residue was purified by silica gel column chromatography (eluting solvent; hexane : ethyl acetate = 6 : 4) to obtain 19 g (yield: 69%) of the title compound as a yellow solid.
¹H-NMR (400 MHz, CDCl₃) δ ppm: 7.80 (1H, m), 7.70 (1H, s), 7.48 (1H, t, J = 7.8 Hz), 7.35 (1H, m), 7.26 (1H, d, J = 7.8 Hz), 7.04 (1H, d, J = 7.8 Hz), 6.91 (1H, m), 5.50 (2H, s), 3.66 (2H, t, J = 8.2 Hz), 2.52 (3H, s), 1.35 (12H, s), 0.96 (2H, t, J = 8.2 Hz), 0.00 (9H, s).
MS(ESI) m/z: 510 (M+H)⁺

### (143e) 5-Bromothiophene-2-sulfonamide

5-Bromothiophene-2-sulfonyl chloride (0.30 g, 1.2 mmol) was dissolved in 1,4-dioxane (2 mL). The resulting mixture was stirred at 0°C, and 28% ammonia water (4 mL) was added thereto. The resulting mixture was stirred at 0°C for 30 min. The reaction solvent was evaporated under reduced pressure, and water was added to the residue. After extraction with methylene chloride, the organic layer was washed with water and brine, and then dried with anhydrous magnesium sulfate. The solvent was evaporated under reduced pressure to obtain 0.19 g (yield: 68%) of a crude product of the title compound as a white solid.
¹H-NMR (400 MHz, CDCl₃) δ ppm: 7.42 (1H, m), 7.06 (1H, m), 5.02 (2H, s).

### (143f) 5-[2-Fluoro-5-(3-(6-methylpyridin-2-yl)-1-{[2-(trimethylsilyl)ethoxy]methyl}-1H-pyrazol-4-yl)phenyl]thiophene-2-sulfonamide

2-(4-[4-Fluoro-3-(4,4,5,5-tetramethyl-1,3,2-dioxaborolan-2-yl)phenyl]-1-{[2-(trimethylsilyl)ethoxy]methyl}-1H-pyrazol-3-yl)-6-methylpyridine (0.37 g, 0.70 mmol) obtained in Example (143d) and 5-bromothiophene-2-sulfonamide (0.19 g, 0.77 mmol) obtained in Example (143e) were dissolved in 1,2-dimethoxyethane (8 mL), and water (3 mL), tripotassium phosphate n-hydrate (0.30 g, 1.4 mmol), and dichloro[1,1'-bis(diphenylphosphino)ferrocene]palladium(II)-methylene chloride complex (0.057 g, 0.070 mmol) were added thereto. The resulting mixture was stirred under a nitrogen atmosphere at 90°C for 4 hr. The reaction solution was cooled to room temperature, and water was added thereto. After extraction with ethyl acetate, the organic layer was washed with water and brine, and then dried with anhydrous magnesium sulfate. The solvent was evaporated under reduced pressure, and the resulting crude product was purified by silica gel column chromatography (Biotage, eluting solvent: hexane/ethyl acetate) to obtain 0.36 g (yield: 95%) of the title compound as a light yellow solid.
¹H-NMR (400 MHz, CDCl₃) δ ppm: 7.74 (1H, s), 7.65 (1H, m), 7.60-7.56 (2H, m), 7.40 (1H, d, J = 7.8 Hz), 7.34 (1H, m), 7.27 (1H, m), 7.13-7.08 (2H, m), 5.53 (2H, s), 5.21 (2H, s), 3.71 (2H, t, J = 7.8 Hz), 2.50 (3H, s), 1.25 (9H, s), 0.97 (2H, t, J = 7.8 Hz), 0.00 (9H, s).
MS(ESI) m/z: 545 (M+H)⁺

### (143g) 5-{2-Fluoro-5-[3-(6-methylpyridin-2-yl)-1H-pyrazol-4-yl]phenyl}thiophene-2-sulfonamide

5-[2-Fluoro-5-(3-(6-methylpyridin-2-yl)-1-{[2-(trimethylsilyl)ethoxy]methyl}-1H-pyrazol-4-yl)phenyl]thiophene-2-sulfonamide (0.36 g, 0.66 mmol) obtained in Example (143f) was dissolved in ethanol (5 mL), and a 3 N hydrochloric acid aqueous solution (2.5 mL) was added thereto. The resulting mixture was stirred at 80°C for 6 hr. The reaction solution was cooled to room temperature and neutralized with saturated aqueous sodium bicarbonate. After extraction with ethyl acetate, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.14 g (yield: 51 %) of the title compound as a white solid.
Melting point: 125°C
¹H-NMR (400 MHz, CDCl₃) δ ppm: 11.1 (1H, brs), 7.66 (1H, m), 7.62 (1H, s), 7.61 (1H, m), 7.44 (1H, t, J = 7.8 Hz), 7.38 (1H, m), 7.36 (1H, m), 7.21 (1H, m), 7.13 (1H, d, J = 7.8 Hz), 7.07 (1H, d, J = 7.8 Hz), 5.05 (2H, s), 3.48 (3H, s).
MS(ESI) m/z: 414 (M+H)⁺.

### (Example 144) 5-{2-Fluoro-5-[3-(6-methylpyridin-2-yl)-1H-pyrazol-4-yl]phenyl}-N,N-dimethylthiophene-2-sulfonamide (Compound No. 2-658)

### (144a) 5-Bromo-N,N-dimethylthiophene-2-sulfonamide

5-Bromothiophene-2-sulfonyl chloride (0.30 g, 1.2 mmol) was dissolved in methylene chloride (4 mL), and a dimethylamine solution (2.0 M in tetrahydrofuran, 2 mL) and saturated aqueous sodium bicarbonate (2.5 mL) were added thereto. The resulting mixture was stirred at room temperature for 6 hr, and brine was added to the reaction solution. After extraction with methylene chloride, the organic layer was separated using an Empore cartridge (GL Science). The solvent was evaporated under reduced pressure to obtain a crude product of the title compound 0.31 g (yield: 100%) as a reddish brown oily material.
¹H-NMR (400 MHz, CDCl₃) δ ppm: 7.27 (1H, d, J = 3.9 Hz), 7.11 (1H, d, J = 3.9 Hz), 2.76 (6H, s).

### (144b) 5-[2-Fluoro-5-(3-(6-methylpyridin-2-yl)-1-{[2-(trimethylsilyl)ethoxy]methyl}-1H-pyrazol-4-yl)phenyl]-N,N-dimethylthiophene-2-sulfonamide

The same reaction as in Example (143f) was carried out using 5-bromo-N,N-dimethylthiophene-2-sulfonamide (0.31 g, 1.2 mmol) obtained in Example (144a) instead of 5-bromothiophene-2-sulfonamide obtained in Example (143e). After purification, 0.60 g (yield: 100%) of the title compound was obtained as a yellow amorphous form.
¹H-NMR (400 MHz, CDCl₃) δ ppm: 7.73 (1H, s), 7.68 (1H, m), 7.56 (1H, t, J = 7.8 Hz), 7.49 (1H, d, J = 3.9 Hz), 7.40 (1H, d, J = 7.8 Hz), 7.38 (1H, d J = 3.9 Hz), 7.33 (1H, m), 7.13-7.08 (2H, m), 5.52 (2H, s), 3.69 (2H, t, J = 8.6 Hz), 2.79 (6H, s), 2.49 (3H, s), 0.98 (2H, t, J = 8.6 Hz), 0.01 (9H, s).
MS(ESI) m/z: 573 (M+H)⁺

### (144c) 5-{2-Fluoro-5-[3-(6-methylpyridin-2-yl)-1H-pyrazol-4-yl]phenyl}-N,N-dimethylthiophene-2-sulfonamide

The same reaction as in Example (143g) was carried out using 5-{2-fluoro-5-[3-(6-methylpyridin-2-yl)-1H-pyrazol-4-yl]phenyl}-N,N-dimethylthiophene-2-sulfonamide (0.69 g, 1.0 mmol) obtained in Example (144b) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). After purification, 0.23 g (51 %) of the title compound was obtained as a colorless amorphous form.
¹H-NMR (400 MHz, CDCl₃) δ ppm: 11.20 (1H, brs), 7.69 (1H, m), 7.63 (1H, s), 7.50 (1H, d, J = 3.9 Hz), 7.47-7.42 (2H, m), 7.38 (1H, m), 7.20 (1H, m), 7.13 (1H, m), 7.07 (1H, d, J = 7.8 Hz), 3.48 (1H, s), 2.79 (3H, s).
MS(ESI) m/z 442 (M+H)⁺

### (Example 145) 5-{2-Fluoro-5-[3-(6-methylpyridin-2-yl)-1H-pyrazol-4-yl]phenyl}-N-methylthiophene-2-sulfonamide (Compound No. 2-660)

### (145a) 5-Bromo-N-methylthiophene-2-sulfonamide

The same reaction as in Example (144a) was carried out using methylamine hydrochloride (0.23 g, 3.5 mmol) instead of dimethylamine solution (2.0 M in tetrahydrofuran) to obtain a crude product 0.28 g (yield: 96%) of the title compound as a colorless oily material.
¹H-NMR (400 MHz, CDCl₃) δ ppm: 7.37 (1H, d, J = 3.9 Hz), 7.09 (1H, d, J = 3.9 Hz), 4.39 (1H, br), 2.76 (3H, d, J = 5.1 Hz).

### (145b) 5-[2-Fluoro-5-(3-(6-methylpyridin-2-yl)-1-{[2-(trimethylsilyl)ethoxy]methyl}-1H-pyrazol-4-yl)phenyl]-N-methylthiophene-2-sulfonamide

The same reaction as in Example (143f) was carried out using 5-bromo-N-methylthiophene-2-sulfonamide (0.28 g, 1.1 mmol) obtained in Example (145a) instead of 5-bromothiophene-2-sulfonamide obtained in Example (143e). After purification, 0.57 g (yield: 100%) of the title compound was obtained as a yellow amorphous form.
¹H-NMR (400 MHz, CDCl₃) δ ppm: 7.73 (1H, s), 7.68 (1H, m), 7.56-7.48 (2H, m), 7.40 (1H, d, J = 7.8 Hz), 7.32 (1H, m), 7.31 (1H, m), 7.10-7.07 (2H, m), 5.52 (2H, s), 4.44 (1H, m), 3.69 (2H, t, J = 8.6 Hz), 2.79 (3H, s), 2.49 (3H, s), 0.97 (2H, t, J = 8.6 Hz), 0.01 (9H, s).
MS(ESI) m/z: 559 (M+H)⁺

### (145c) 5-{2-Fluoro-5-[3-(6-methylpyridin-2-yl)-1H-pyrazol-4-yl]phenyl}-N-methylthiophene-2-sulfonamide

The same reaction as in Example (143g) was carried out using 5-[2-fluoro-5-(3-(6-methylpyridin-2-yl)-1-{[2-(trimethylsilyl)ethoxy]methyl}-1H-pyrazol-4-yl)phenyl]-N-methylthiophene-2-sulfonamide (0.57 g, 1.0 mmol) obtained in Example (145b) instead of 5-[2-fluoro-5-(3-(6-methylpyridin-2-yl)-1-{[2-(trimethylsilyl)ethoxy]methyl} -1H-pyrazol-4-yl)phenyl]thiophene-2-sulfonamide obtained in Example (143f). After purification, 0.13 g (31%) of the title compound was obtained as a light yellow amorphous form.
¹H-NMR (400 MHz, CDCl₃) δ ppm: 11.28 (1H, brs), 7.67 (1H, m), 7.63 (1H, s), 7.56 (1H, m), 7.44 (1H, m), 7.40-7.35 (2H, m), 7.20 (1H, m), 7.12 (1H, m), 7.07 (1H, d, J = 7.8 Hz), 4.49 (1H, d, J = 5.1 Hz), 2.78 (3H, d, J = 5.1 Hz), 2.58 (3H, s).
MS(ESI) m/z 428 (M+H)⁺.

### (Example 146) 2-{4-[3-(1,1-Dioxido-2,3-dihydro-1-benzothien-5-yl)-4-fluorophenyl]-1H-pyrazol-3-yl}-6-methylpyridine (Compound No. 2-724)

### (146a) 2-(4-[3-(1,1-Dioxido-1-benzothien-5-yl)-4-fluorophenyl]-1-{[2-(trimethylsilyl)ethoxy]methyl} -1 H-pyrazol-3-yl)-6-methylpyridine

The same reaction as in Example (143f) was carried out using 5-bromo-1-benzothiophene 1,1-dioxide (0.50 g, 2.0 mmol) instead of 5-bromothiophene-2-sulfonamide obtained in Example (143e). After purification, 0.77 g (yield: 84%) of the title compound was obtained as a yellow amorphous form.
¹H-NMR (500 MHz, CDCl₃) δ ppm: 7.84-7.80 (2H, m), 7.70-7.63 (2H, m), 7.58-7.56 (2H, m), 7.50-7.47 (2H, m), 7.30 (1H, d, J = 7.3 Hz), 7.22-7.17 (2H, m), 6.84 (1H, d, J = 6.8 Hz), 5.60 (2H, s), 3.77 (2H, t, J = 8.3 Hz), 2.56 (3H, s), 1.04 (2H, t, J = 8.3 Hz), 0.08 (9H, s).
MS(ESI) m/z: 548 (M+H)⁺

### (146b) 2-{4-[3-(1,1-Dioxido-2,3-dihydro-1-benzothien-5-yl)-4-fluorophenyl]-1H-pyrazol-3-yl} -6-methylpyridine

To an ethanol solution (30 mL) of 2-(4-[3-(1,1-dioxido-1-benzothien-5-yl)-4-fluorophenyl]-1- {[2-(trimethylsilyl)ethoxy]methyl}-1H-pyrazol-3-yl)-6-methylpyridine (0.77 g, 1.4 mmol) obtained in Example (146a), 10% of palladium-carbon (0.095 g) was added. The resulting mixture was stirred under a hydrogen atmosphere at room temperature for 1.5 hr. The reaction mixture was filtered, and the filtrate was concentrated under reduced pressure. The resulting crude product was dissolved in ethanol (20 mL), and a 3 N hydrochloric acid aqueous solution (10 mL) was added thereto. The resulting mixture was heated under reflux for 12 hr. The reaction solution was cooled to room temperature and then neutralized with saturated aqueous sodium bicarbonate. After extraction with methylene chloride, the organic layer was washed with water and brine, and then dried with anhydrous magnesium sulfate. The solvent was evaporated under reduced pressure, and the resulting crude product was purified by silica gel column chromatography (Biotage, eluting solvent: hexane/ethyl acetate) to obtain 0.37 g (yield: 63%) of the title compound as a white amorphous form.
Melting point: 107 to 109°C
¹H-NMR (500 MHz, CDCl₃) δ ppm: 7.80 (1H, d, J = 8.3 Hz), 7.65 (1H, s), 7.64 (1H, d, J = 7.8 Hz), 7.56 (1H, s), 7.51-7.44 (3H, m), 7.23 (1H, m), 7.18 (1H, d, J = 7.8 Hz), 7.09 (1H, d, J = 7.3 Hz), 3.55 (2H, t, J = 6.8 Hz), 3.44 (2H, t, J = 6.8 Hz), 2.59 (3H, s).
MS(ESI) m/z: 420 (M+H)⁺

### (Example 147) 2',3-Difluoro-5'-[3-(6-methylpyridin-2-yl)-1H-pyrazol-4-yl]-1,1'-biphenyl-4-sulfonamide (Compound No. 2-618)

2-(4-[4-Fluoro-3-(4,4,5,5-tetramethyl-1,3,2-dioxaborolan-2-yl)phenyl]-1-{[2-(trimethylsilyl)ethoxy]methyl}-1H-pyrazol-3-yl)-6-methylpyridine (0.27 g, 0.53 mmol) obtained in Example (143d) and 4-bromo-2-fluorobenzenesulfonamide (0.20 g, 0.79 mmol) were dissolved in 1,2-dimethoxyethane (5 mL), and water (1 mL), tripotassium phosphate n-hydrate (0.23 g, 1.1 mmol), and dichloro[1,1'-bis(diphenylphosphino)ferrocene]palladium(II)-methylene chloride complex (0.058 g, 0.071 mmol) were added thereto. The resulting mixture was heated under a nitrogen atmosphere under reflux for 1 hr. The reaction solution was diluted with ethyl acetate and then filtered. The filtrate was evaporated under reduced pressure. The resulting crude product was dissolved in ethanol (5 mL), and a 3 N hydrochloric acid aqueous solution (3 mL) was added thereto. The resulting mixture was heated under reflux for 4 hr. The reaction solution was concentrated under reduced pressure, and saturated aqueous sodium bicarbonate was added thereto. After extraction with methylene chloride, the organic layer was separated using an Empore cartridge (GL Science). The solvent was evaporated under reduced pressure, and the resulting crude product was purified by silica gel column chromatography (Biotage, eluting solvent: hexane/ethyl acetate) to obtain 0.092 g (yield: 41%) of the title compound as a white amorphous form.
Melting point: 119 to 121°C
¹H-NMR (400 MHz, CDCl₃) δ ppm: 7.94 (1H, m), 7.63 (1H, s), 7.49-7.41 (5H, m), 7.21 (1H, m), 7.14 (1H, d, J = 7.8 Hz), 7.07 (1H, d, J = 7.8 Hz), 5.14 (2H, s), 2.58 (3H, s).
MS(ESI) m/z: 427 (M+H)⁺

### (Example 148) 2'-Fluoro-4-hydroxy-5'-[3-(6-methylpyridin-2-yl)-1H-pyrazol-4-yl]-1,1'-biphenyl-3-sulfonamide (Compound No. 2-620)

The same reaction as in Example 147 was carried out using 5-bromo-2-hydroxybenzenesulfonamide (0.19 g, 0.73 mmol) instead of 4-bromo-2-fluorobenzenesulfonamide. After purification by high-performance liquid chromatography (GL Science ODS-3, eluting solvent; water : acetonitrile = 95 : 5 to 5 : 95), 0.079 g (yield: 41 %) of the title compound was obtained as a white solid. Melting point: 104 to 109°C
¹H-NMR (500 MHz, DMSO-d₆) δ ppm: 13.40 (0.5H, s), 13.17 (0.5H, s), 8.10 (0.5H, s), 7.79-6.90 (12.5H, m), 2.50 (3H, s).
MS(ESI) m/z: 425 (M+H)⁺

### (Example 149) 2,2',5-Trifluoro-5'-[3-(6-methylpyridin-2-yl)-1H-pyrazol-4-yl]-1,1'-biphenyl-4-sulfonamide (Compound No. 2-630)

### (149a) 4-Bromo-2,5-difluorobenzenesulfonamide

To a tetrahydrofuran solution (6 mL) of 4-bromo-2,5-difluorobenzenesulfonyl chloride (0.88 g, 3.0 mmol), 28% ammonia water (2 mL) was added. The resulting mixture was stirred at room temperature for 2 hr. To this reaction solution, water was added. After extraction with ethyl acetate, the organic layer was washed with water and brine, and then dried with anhydrous magnesium sulfate. The solvent was evaporated under reduced pressure to obtain a crude product 0.78 g (yield: 95%) of the title compound as a white solid.
¹H-NMR (400 MHz, CDCl₃) δ ppm: 7.66 (1H, m), 7.47 (1H, dd, J = 5.9, 8.3 Hz), 5.09 (2H, s).

### (149b) 2,2',5-Trifluoro-5'-[3-(6-methylpyridin-2-yl)-1H-pyrazol-4-yl]-1,1'-biphenyl-4-sulfonamide

The same reaction as in Example 147 was carried out using 4-bromo-2,5-difluorobenzenesulfonamide (0.14 g, 0.52 mmol) obtained in Example (149a) instead of 4-bromo-2-fluorobenzenesulfonamide. After purification, 0.065 g (yield: 37%) of the title compound was obtained as a white solid.
Melting point: 122 to 125°C
¹H-NMR (500 MHz, CDCl₃) δ ppm: 7.71 (1H, dd, J = 5.9, 8.3 Hz), 7.63 (1H, s), 7.52-7.47 (2H, m), 7.43 (1H, m), 7.30 (1H, m), 7.25 (1H, m), 7.18 (1H, d, J = 7.8 Hz), 7.09 (1H, d, J = 7.3 Hz), 5.31 (2H, s), 2.58 (3H, s).
MS(ESI) m/z: 445 (M+H)⁺

### (Example 150) 2'-Fluoro-5'-[3-(6-methylpyridin-2-yl)-1H-pyrazol-4-yl]-3-(trifluoromethoxy)-1,1'-biphenyl-4-sulfonamide (Compound No. 2-632)

### (150a) 4-Bromo-2-(trifluoromethoxy)benzenesulfonamide

The same reaction as in Example (149a) was carried out using 4-bromo-2-(trifluoromethoxy)benzenesulfonyl chloride (0.75 g, 2.2 mmol) instead of 4-bromo-2,5-difluorobenzenesulfonyl chloride to obtain a crude product 0.65 g (yield: 92%) of the title compound as a white solid.
¹H-NMR (400 MHz, CDCl₃) δ ppm: 7.89 (1H, d, J = 8.3 Hz), 7.56-7.53 (2H, m), 4.99 (2H, s).

### (150b) 2'-Fluoro-5'-[3-(6-methylpyridin-2-yl)-1H-pyrazol-4-yl]-3-(trifluoromethoxy)-1,1'-biphenyl-4-sulfonamide

The same reaction as in Example 147 was carried out using 4-bromo-2-(trifluoromethoxy)benzenesulfonamide (0.17 g, 0.52 mmol) obtained in Example (150a) instead of 4-bromo-2-fluorobenzenesulfonamide. After purification, 0.089 g (yield: 45%) of the title compound was obtained as a white amorphous form.
¹H-NMR (500 MHz, CDCl₃) δ ppm: 8.09 (1H, d, J = 8.3 Hz), 7.65 (1H, s), 7.58-7.55 (2H, m), 7.50-7.47 (3H, m), 7.25 (1H, m), 7.19 (1H, d, J = 7.8 Hz), 7.11 (1H, d, J = 7.3 Hz), 5.14 (2H, s), 2.58 (3H, s).
MS(ESI) m/z: 493 (M+H)⁺.

### (Example 151) 3-Chloro-2'-fluoro-5'-[3-(6-methylpyridin-2-yl)-1H-pyrazol-4-yl]-1,1'-biphenyl-4-sulfonamide (Compound No. 2-634)

The same reaction as in Example 147 was carried out using 4-bromo-2-chlorobenzenesulfonamide (0.14 g, 0.50 mmol) instead of 4-bromo-2-fluorobenzenesulfonamide. After purification, 0.099 g (yield: 55%) of the title compound was obtained as a white amorphous form.
¹H-NMR (500 MHz, CDCl₃) δ ppm: 11.49 (1H, brs), 8.13 (1H, d, J = 8.3 Hz), 7.70 (1H, s), 7.63 (1H, s), 7.54 (1H, d, J = 8.3 Hz), 7.50-7.43 (3H, m), 7.21 (1H, m), 7.17 (1H, d, J = 7.8 Hz), 7.09 (1H, d, J = 7.8 Hz), 5.46 (2H, s), 2.57 (3H, s).
MS(ESI) m/z: 443 (M+H)⁺

### (Example 152) 2'-Fluoro-5'-[3-(6-methylpyridin-2-yl)-1H-pyrazol-4-yl]-3-(trifluoromethyl)-1,1'-biphenyl-4-sulfonamide (Compound No. 2-636)

### (152a) 4-Bromo-2-(trifluoromethyl)benzenesulfonamide

The same reaction as in Example (149a) was carried out using 4-bromo-2-(trifluoromethyl)benzenesulfonyl chloride (0.33 g, 1.0 mmol) instead of 4-bromo-2,5-difluorobenzenesulfonyl chloride to obtain a crude product 0.30 g (yield: 99%) of the title compound as a white solid.
¹H-NMR (400 MHz, CDCl₃) δ ppm: 8.15 (1H, d, J = 8.3 Hz), 8.00 (1H, s), 7.86 (1H, m), 5.01 (2H, s).

### (152b) 2'-Fluoro-5'-[3-(6-methylpyridin-2-yl)-1H-pyrazol-4-yl]-3-(trifluoromethyl)-1,1'-biphenyl-4-sulfbnamide

The same reaction as in Example 147 was carried out using 4-bromo-2-(trifluoromethyl)benzenesulfonamide obtained in Example (152a) instead of 4-bromo-2-fluorobenzenesulfonamide. After purification, 0.10 g (yield: 53%) of the title compound was obtained as a white amorphous form.
Melting point: 107 to 110°C
¹H-NMR (500 MHz, CDCl₃) δ ppm: 8.33 (1H, d, J = 8.3 Hz), 8.00 (1H, s), 7.87 (1H, d, J = 8.3 Hz), 7.66 (1H, s), 7.52-7.48 (3H, m), 7.26 (1H, m), 7.19 (1H, d, J = 7.8 Hz), 7.11 (1H, d, J = 7.8 Hz), 5.12 (2H, s), 2.59 (3H, s).
MS(ESI) m/z: 477 (M+H)⁺

### (Example 153) 2'-Fluoro-2-methyl-5'-[3-(6-methylpyridin-2-yl)-1H-pyrazol-4-yl]-1,1'-biphenyl-4-sulfonamide (Compound No. 2-640)

### (153a) 4-Bromo-3-methylbenzenesulfonamide

The same reaction as in Example (149a) was carried out using 4-bromo-3-methylbenzenesulfonyl chloride (0.32 g, 1.2 mmol) instead of 4-bromo-2,5-difluorobenzenesulfonyl chloride to obtain a crude product 0.30 g (yield: 99%) of the title compound as a white solid.
¹H-NMR (400 MHz, CDCl₃) δ ppm: 7.80 (1H, s), 7.69 (1H, d, J = 8.3 Hz), 7.60 (1H, m), 4.78 (2H, s), 2.48 (3H, s).

### (153b) 2'-Fluoro-2-methyl-5'-[3-(6-methylpyridin-2-yl)-1H-pyrazol-4-yl]-1,1'-biphenyl-4-sulfonamide

The same reaction as in Example 147 was carried out using 4-bromo-3-methylbenzenesulfonamide (0.14 g, 0.54 mmol) obtained in Example (153a) instead of 4-bromo-2-fluorobenzenesulfonamide. After purification, 0.067 g (yield: 39%) of the title compound was obtained as a white amorphous form.
¹H-NMR (500 MHz, CDCl₃) δ ppm: 11.32 (1H, brs), 7.85 (1H, s), 7.78 (1H, m), 7.63 (1H, s), 7.47 (1H, m), 7.45 (1H, m), 7.35 (1H, d, J = 8.3 Hz), 7.26 (1H, m), 7.22-7.17 (2H, m), 7.08 (1H, d, J = 7.8 Hz), 5.11 (2H, s), 2.56 (3H, s), 2.29 (3H, s).
MS(ESI) m/z: 423 (M+H)⁺.

### (Example 154) 2'-Fluoro-3-methyl-5'-[3-(6-methylpyridin-2-yl)-1H-pyrazol-4-yl]-1,1'-biphenyl-4-sulfonamide (Compound No. 2-638)

### (154a) 4-Bromo-2-methylbenzenesulfonamide

The same reaction as in Example (149a) was carried out using 4-bromo-2-methylbenzenesulfonyl chloride (0.38 g, 1.4 mmol) instead of 4-bromo-2,5-difluorobenzenesulfonyl chloride to obtain a crude product 0.35 g (quantitative yield) of the title compound as a white solid. ¹H-NMR (400 MHz, CDCl₃) δ ppm: 7.85 (1H, d, J = 8.3 Hz), 7.48 (1H, s), 7.44 (1H, m), 4.75 (2H, s), 2.66 (3H, s).

### (154b) 2'-Fluoro-3-methyl-5'-[3-(6-methylpyridin-2-yl)-1H-pyrazol-4-yl]-1,1'-biphenyl-4-sulfonamide

The same reaction as in Example 147 was carried out using 4-bromo-2-methylbenzenesulfonamide (0.13 g, 0.52 mmol) obtained in Example (154a) instead of 4-bromo-2-fluorobenzenesulfonamide. After purification, 0.099 g (yield: 58%) of the title compound was obtained as a white amorphous form.
¹H-NMR (500 MHz, CDCl₃) δ ppm: 8.05 (1H, d, J = 8.3 Hz), 7.64 (1H, s), 7.49-7.46 (4H, m), 7.41 (1H, m), 7.22-7.18 (2H, m), 7.08 (1H, d, J = 7.8 Hz), 5.08 (2H, s), 2.72 (3H, s), 2.58 (3H, s).
MS(ESI) m/z: 423 (M+H)⁺

### (Example 155) 3-Ethyl-2'-fluoro-5'-[3-(6-methylpyridin-2-yl)-1H-pyrazol-4-yl]-1,1'-biphenyl-4-sulfonamide (Compound No. 2-642)

### (155a) 4-Bromo-2-ethylbenzenesulfonamide

The same reaction as in Example (149a) was carried out using 4-bromo-2-ethylbenzenesulfonyl chloride (0.38 g, 1.3 mmol) instead of 4-bromo-2,5-difluorobenzenesulfonyl chloride to obtain a crude product 0.34 g (yield: 95%) of the title compound.
¹H-NMR (400 MHz, CDCl₃) δ ppm: 7.85 (1H, d, J = 8.6 Hz), 7.52 (1H, d, J = 2.0 Hz), 7.43 (1H, dd, J = 2.0, 8.6 Hz), 4.80 (2H, s), 3.03 (2H, q, J = 7.4 Hz), 1.33 (3H, t, J = 7.4 Hz).

### (155b) 3-Ethyl-2'-fluoro-5'-[3-(6-methylpyridin-2-yl)-1H-pyrazol-4-yl]-1,1'-biphenyl-4-sulfonamide

The same reaction as in Example 147 was carried out using 4-bromo-2-ethylbenzenesulfonamide (0.13 g, 0.49 mmol) obtained in Example (155a) instead of 4-bromo-2-fluorobenzenesulfonamide. After purification, 0.12 g (yield: 66%) of the title compound was obtained as a white amorphous form.
¹H-NMR (500 MHz, CDCl₃) δ ppm: 11.59 (1H, brs), 8.04 (1H, m), 7.64 (1H, s), 7.52 (1H, s), 7.49-7.39 (4H, m), 7.21-7.17 (2H, m), 7.08 (1H, d, J = 7.8 Hz), 5.31 (2H, s), 3.10 (2H, q, J = 7.4 Hz), 2.56 (3H, s), 1.33 (3H, t, J = 7.4 Hz).
MS(ESI) m/z: 437 (M+H)⁺.

### (Example 156) 2'-Fluoro-N-methyl-5'-[3-(6-methylpyridin-2-yl)-1H-pyrazol-4-yl]-1,1'-biphenyl-4-sulfonamide (Compound No. 2-518)

The same reaction as in Example 147 was carried out using 4-bromo-N-methylbenzenesulfonamide (0.14 g, 0.54 mmol) instead of 4-bromo-2-fluorobenzenesulfonamide. After purification, 0.095 g (yield: 56%) of the title compound was obtained as a white amorphous form.
¹H-NMR (500 MHz, CDCl₃) δ ppm: 11.25 (1H, brs), 7.92 (2H, d, J = 8.3 Hz), 7.70 (2H, d, J = 8.3 Hz), 7.66 (1H, s), 7.52 (1H, m), 7.47 (1H, m), 7.44 (1H, m), 7.23 (1H, m), 7.19 (1H, d, J = 7.8 Hz), 7.09 (1H, d, J = 7.8 Hz), 4. 51 (1H, d, J = 5.4 Hz), 2.72 (3H, d, J = 5.4 Hz), 2.58 (3H, s).
MS(ESI) m/z: 423 (M+H)⁺

### (Example 157) 2'-Fluoro-N,N-dimethyl-5'-[3-(6-methylpyridin-2-yl)-1H-pyrazol-4-yl]-1,1'-biphenyl-4-sulfonamide (Compound No. 2-644)

The same reaction as in Example 147 was carried out using 4-bromo-N,N-dimethylbenzenesulfonamide (0.14 g, 0.51 mmol) instead of 4-bromo-2-fluorobenzenesulfonamide. After purification, 0.11 g (yield: 64%) of the title compound was obtained as a white amorphous form.
¹H-NMR (500 MHz, CDCl₃) δ ppm: 11.45 (1H, brs), 7.84 (2H, d, J = 8.3 Hz), 7.71 (2H, d, J = 8.3 Hz), 7.66 (1H, s), 7.53 (1H, dd, J = 2.0, 7.8 Hz), 7.48 (1H, m), 7.44 (1H, m), 7.26-7.19 (2H, m), 7.09 (1H, d, J = 7.8 Hz), 2.76 (6H, s), 2.58 (3H, s). MS(ESI) m/z: 437 (M+H)⁺

### (Example 158) (6-{2-Fluoro-5-[3-(6-methylpyridin-2-yl)-1H-pyrazol-4-yl]phenyl}pyridin-3-yl)methanol (Compound No. 2-646)

The same reaction as in Example 147 was carried out using 2-chloro-5-hydroxymethylpyridine (0.088 g, 0.61 mmol) instead of 4-bromo-2-fluorobenzenesulfonamide. After purification, 0.11 g (yield: 73%) of the title compound was obtained as a white solid.
Melting point: 209 to 211°C
¹H-NMR (500 MHz, DMSO-d₆) δ ppm: 13.40 (0.5H, s), 13.17 (0.5H, s), 8.61 (1H, s), 8.13 (0.5H, d, J = 6.8 Hz), 8.10 (0.5H, s), 8.04 (0.5H, d, J = 6.8 Hz), 7.83-7.66 (3.5H, m), 7.55 (0.5H, d, J = 7.8 Hz), 7.52-7.45 (1H, m), 7.33-7.21 (2H, m), 7.14 (0.5H, d, J = 7.8 Hz), 5.36 (1H, t, J = 5.9 Hz), 4.58 (2H, d, J = 5.9 Hz), 2.49 (3H, s).
MS(ESI) m/z: 361 (M+H)⁺

### (Example 159) 2-(S-{2-Fluoro-5-[3-(6-methylpyridin-2-yl)-1H-pyrazol-4-yl]phenyl}pyridin-2-yl)propan-2-ol (Compound No. 2-648)

### (159a) 2-(5-Bromopyridin-2-yl)propan-2-ol

An n-butyllithium solution (1.6 M in hexane, 8.5 mL, 13 mmol) was added to a toluene solution (60 mL) of 2,5-dibromopyridine (2.6 g, 11 mmol) under a nitrogen atmosphere at -78°C. The resulting mixture was stirred at -78°C for 30 min, and then acetone (1.2 mL, 16 mmol) was added thereto. The resulting mixture was further stirred for 30 min. The reaction solution was returned to room temperature, and a saturated ammonium chloride aqueous solution was added thereto to terminate the reaction. After extraction with ethyl acetate, the organic layer was washed with water and brine, and then dried with anhydrous magnesium sulfate. The solvent was evaporated under reduced pressure, and the resulting crude product was purified by silica gel column chromatography (Biotage, eluting solvent; hexane/ethyl acetate) to obtain 1.7 g (yield: 73%) of the title compound as a light yellow oily material.
¹H-NMR (500 MHz, CDCl₃) δ ppm: 8.58 (1H, d, J = 2.4 Hz), 7.82 (1H, dd, J = 2.4, 8.3 Hz), 7.31 (1H, d, J = 8.3 Hz), 4.42 (1H, s), 1.54 (6H, s).
MS(ESI) m/z: 216 (M+H)⁺

### (159b) 2-(5-{2-Fluoro-5-[3-(6-methylpyridin-2-yl)-1H-pyrazol-4-yl]phenyl}pyridin-2-yl)propan-2-ol

The same reaction as in Example 147 was carried out using 2-(5-bromopyridin-2-yl)propan-2-ol (0.12 g, 0.56 mmol) obtained in Example (159a) instead of 4-bromo-2-fluorobenzenesulfonamide. After purification, 0.089 g (yield: 57%) of the title compound was obtained as a white amorphous form.
¹H-NMR (500 MHz, CDCl₃) δ ppm: 8.69 (1H, s), 7.92 (1H, d, J = 7.8 Hz), 7.68 (1H, s), 7.54-7.47 (3H, m), 7.42 (1H, m), 7.27-7.22 (2H, m), 7.12 (1H, d, J = 7.3 Hz), 2.61 (3H, s), 1.60 (6H, s).
MS(ESI) m/z: 389 (M+H)⁺

### (Example 160) 2-(6-{2-Fluoro-5-[3-(6-methylpyridin-2-yl)-1H-pyrazol-4-yl]phenyl}pyridin-3-yl)propan-2-ol (Compound No. 2-650)

### (160a) 2-(6-Bromopyridin-3-yl)propan-2-ol

An n-butyllithium solution (1.6 M in hexane, 8.5 mL, 13 mmol) was added to a diethylether solution (130 mL) of 2,5-dibromopyridine (2.6 g, 11 mmol) under a nitrogen atmosphere at -78°C. The resulting mixture was stirred at -78°C for 40 min, and acetone (1.5 mL, 20 mmol) was added thereto. The resulting mixture was further stirred for 30 min. The reaction solution was returned to room temperature, and a saturated ammonium chloride aqueous solution was added thereto to terminate the reaction. After extraction with ethyl acetate, the organic layer was washed with water and brine, and then dried with anhydrous magnesium sulfate. The solvent was evaporated under reduced pressure, and the resulting crude product was purified by silica gel column chromatography (Biotage, eluting solvent: hexane/ethyl acetate) to obtain 1.6 g (yield: 66%) of the title compound as a light yellow solid.
¹H-NMR (500 MHz, CDCl₃) δ ppm: 8.48 (1H, d, J = 2.4 Hz), 7.69 (1H, dd, J = 2.4, 8.3 Hz), 7.45 (1H, m), 1.76 (1H, s), 1.60 (6H, s).
MS(ESI) m/z: 216 (M+H)⁺

### (160b) 2-(6-{2-Fluoro-5-[3-(6-methylpyridin-2-yl)-1H-pyrazol-4-yl]phenyl}pyridin-3-yl)propan-2-ol

The same reaction as in Example 147 was carried out using 2-(6-bromopyridin-3-yl)propan-2-ol (0.12 g, 0.56 mmol) obtained in Example (160a) instead of 4-bromo-2-fluorobenzenesulfonamide. After purification, 0.095 g (yield: 61%) of the title compound was obtained as a white solid.
Melting point: 169 to 172°C
¹H-NMR (500 MHz, CDCl₃) δ ppm: 11.22 (1H, brs), 8.83 (1H, d, J = 2.4 Hz), 8.06 (1H, dd, J = 2.4, 8.3 Hz), 7.91 (1H, dd, J = 2.0, 8.3 Hz), 7.79 (1H, dd, J = 2.0, 8.3 Hz), 7.66 (1H, s), 7.43 (1H, m), 7.39 (1H, m), 7.21-7.17 (2H, m), 7.04 (1H, d, J = 7.8 Hz), 2.57 (3H, s), 1.91 (1H, s), 1.65 (6H, s).
MS(ESI) m/z: 389 (M+H)⁺.

### (Example 161) 2-(4-{2-Fluoro-5-[3-(6-methylpyridin-2-yl)-1H-pyrazol-4-yl]phenyl}-1H-imidazol-2-yl)propan-2-ol (Compound No. 2-652)

### (161a) 2-(1-{[2-(Trimethylsilyl)ethoxy]methyl}-1H-imidazol-2-yl)propan-2-ol

An n-butyllithium solution (1.5 M in hexane, 15 mL, 23 mmol) was added to a tetrahydrofuran solution (60 mL) of 1-{[2-(trimethylsilyl)ethoxy]methyl}-1H-imidazole (3.1 g, 15 mmol) under a nitrogen atmosphere at -40°C. The resulting mixture was stirred for 30 min, and acetone (5.0 mL, 68 mmol) was added thereto. The resulting mixture was further stirred for 10 min. The reaction solution was returned to room temperature, and water was added thereto to terminate the reaction. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; hexane : ethyl acetate = 1: 1) to obtain 3.0 g (yield: 76%) of the title compound as a colorless oily material.
¹H-NMR (500 MHz, CDCl₃) δ ppm: 6.95 (1H, d, J = 1.5 Hz), 6.93 (1H, d, J = 1.5 Hz), 5.52 (2H, s), 3.58 (2H, t, J = 8.3 Hz), 3.07 (1H, s), 1.70 (6H, s), 0.95 (2H, t, J = 8.3 Hz), 0.02 (9H, s).

### (161b) 2-(4-Bromo-1-{[2-(trimethylsilyl)ethoxy]methyl}-1H-imidazol-2-yl)propan-2-ol and 2-(5-bromo-1-{[2-(trimethylsilyl)ethoxy]methyl}-1H-imidazol-2-yl)propan-2-ol

N-Bromosuccinimide (2.1 g, 12 mmol) was added to a tetrahydrofuran solution (50 mL) of 2-(1-{[2-(trimethylsilyl)ethoxy]methyl}-1H-imidazol-2-yl)propan-2-ol (3.0 g, 12 mmol) obtained in Example (161a). The resulting mixture was stirred at room temperature for 18 hr, and water was added to the reaction solution. After extraction with ethyl acetate, the organic layer was washed with water and brine, and then dried with anhydrous magnesium sulfate. The solvent was evaporated under reduced pressure, and the resulting crude product was purified by silica gel column chromatography (NH, eluting solvent; hexane : ethyl acetate = 3 : 2) to obtain 1.9 g (yield: 47%) of the title compound as a colorless oily material. ¹H-NMR (400 MHz, CDCl₃) δ ppm: 6.89 (0.6H, s), 6.88 (0.4H, s), 5.55 (1.2H, s), 5.45 (0.8H, s), 3.62 (1.2H, t, J = 8.3 Hz), 3.56 (0.8H, t, J = 8.3 Hz), 3.03 (0.6H, s), 2.69 (0.4H, s), 1.67 (6H, s), 0.97-0.91 (2H, m), 0.01 (9H, s).
MS(ESI) m/z: 335 (M+H)⁺

### (161c) 2-(4-{2-Fluoro-5-[3-(6-methylpyridin-2-yl)-1H-pyrazol-4-yl]phenyl}-1H-imidazol-2-yl)propan-2-ol

The same reaction as in Example 147 was carried out using a mixture (1.8 g, 5.4 mmol) of 2-(4-bromo-1- {[2-(trimethylsilyl)ethoxy]methyl} -1H-imidazol-2-yl)propan-2-ol and 2-(5-bromo-1-{[2-(trimethylsilyl)ethoxy]methyl}-1H-imidazol-2-yl)propan-2-ol obtained in Example (161b) instead of 4-bromo-2-fluorobenzenesulfonamide. After purification, 0.81 g (yield: 40%) of the title compound was obtained as a white solid.
Melting point: 135 to 137°C
¹H-NMR (500 MHz, DMSO-d₆) δ ppm: 13.34 (0.5H, s), 13.11 (0.5H, s), 11.94 (0.5H, s), 11.88 (0.5H, s), 8.15 (0.5H, dd, J = 2.0, 7.3 Hz), 8.07 (0.5H, d, J = 7.3 Hz), 8.02 (0.5H, s), 7.68 (0.5H, s), 7.66-7.62 (1H, m), 7.47 (0.5H, d, J = 7.8 Hz), 7.29 (0.5H, m), 7.25 (0.5H, m), 7.22-7.07 (3.5H, m), 5.29 (1H, s), 2.49 (3H, s), 1.46 (6H, s). MS(ESI) m/z: 378 (M+H)⁺

### (Example 162) 2-(4-{2-Fluoro-5-[3-(6-methylpyridin-2-yl)-1H-pyrazol-4-yl]phenyl}-1H-pyrazol-1-yl)ethanol (Compound No. 2-662)

### (162a) 4-Iodo-1-[2-(tetrahydro-2H-pyran-2-yloxy)ethyl]-1H-pyrazole

Sodium hydride (55% oil, 0.51 g, 12 mmol) was added to an N,N-dimethylformamide solution (15 mL) of 4-iodo-1H-pyrazole (1.5 g, 7.7 mmol). The resulting solution was stirred at room temperature for 15 min, and then 2-(2-bromoethoxy)tetrahydro-2H-pyran (1.5 mL, 9.9 mmol) was added thereto. The resulting mixture was stirred at room temperature for 5 hr. Water was added to the reaction solution to terminate the reaction. After extraction with ethyl acetate, the organic layer was washed with water and brine, and then dried with anhydrous magnesium sulfate. The solvent was evaporated under reduced pressure, and the resulting crude product was purified by silica gel column chromatography (Biotage, eluting solvent: hexane/ethyl acetate) to obtain 1.6 g (yield: 64%) of the title compound as a light yellow gum-like material.
¹H-NMR (500 MHz, CDCl₃) δ ppm: 7.55 (1H, s), 7.51 (1H, s), 4.52 (1H, m), 4.36-4.29 (2H, m), 4.04 (1H, m), 3.72 (1H, m), 3.61 (1H, m), 3.45 (1H, m), 1.80-1.47 (6H, m).

### (162b) 2-(4-{2-Fluoro-5-[3-(6-methylpyridin-2-yl)-1H-pyrazol-4-yl]phenyl}-1H-pyrazol-1-yl)ethanol

The same reaction as in Example 147 was carried out using 4-iodo-1-[2-(tetrahydro-2H-pyran-2-yloxy)ethyl]-1H-pyrazole (0.37 g, 1.1 mmol) obtained in Example (162a) instead of 4-bromo-2-fluorobenzenesulfonamide. After purification, 0.10 g (yield: 28%) of the title compound was obtained as a white solid. Melting point: 132 to 135°C
¹H-NMR (400 MHz, CDCl₃) δ ppm: 11.11 (1H, brs), 7.86 (1H, d, J = 2.4 Hz), 7.84 (1H, s), 7.64 (1H, s), 7.60 (1H, dd, J = 2.4, 7.8 Hz), 7.43 (1H, m), 7.23 (1H, m), 7.19-7.14 (2H, m), 7.07 (1H, d, J = 7.8 Hz), 4.31-4.29 (2H, m), 4.07-4.05 (2H, m), 2.98 (1H, brs), 2.59 (3H, s).
MS(ESI) m/z: 364 (M+H)⁺.

### (Example 163) 1-Fluoro-2-(6-{2-fluoro-5-[3-(6-methylpyridin-2-yl)-1H-pyrazol-4-yl]phenyl}pyridin-3-yl)propan-2-ol (Compound No. 2-664)

### (163a) 2-(6-Bromopyridin-3-yl)-1-fluoropropan-2-ol

The same reaction as in Example (160a) was carried out using fluoroacetone (0.73 g, 9.6 mmol) instead of acetone. After purification, 1.2 g (yield: 80%) of the title compound was obtained as a yellow solid.
¹H-NMR (400 MHz, CDCl₃) δ ppm: 8.48 (1H, d, J = 2.4 Hz), 7.70 (1H, dd, J = 2.4, 8.3 Hz), 7.50 (1H, d, J = 8.3 Hz), 4.50, 4.38 (2H, ABqd, J = 9.3, 47.9 Hz), 2.49 (1H, s), 1.61 (3H, d, J = 2.4 Hz).
MS(ESI) m/z: 233 (M+H)⁺

### (163b) 1-Fluoro-2-(6-{2-fluoro-5-[3-(6-methylpyridin-2-yl)-1H-pyrazol-4-yl]phenyl}pyridine-3-yl)propan-2-ol

The same reaction as in Example 147 was carried out using 2-(6-bromopyridin-3-yl)-1-fluoropropan-2-ol (0.15 g, 0.62 mmol) obtained in Example (163a) instead of 4-bromo-2-fluorobenzenesulfonamide. After purification, 0.10 g (yield: 50%) of the title compound was obtained as a white solid.
Melting point: 174 to 176°C
¹H-NMR (500 MHz, DMSO-d₆) δ ppm: 13.40 (0.5H, s), 13.17 (0.5H, s), 8.79 (1H, m), 8.12 (0.5H, dd, J = 2.4, 7.8 Hz), 8.10 (0.5H, s), 8.04 (0.5H, dd, J = 2.4, 7.8 Hz), 7.99-7.95 (1H, m), 7.77-7.68 (2.5H, m), 7.55 (0.5H, d, J = 7.8 Hz), 7.52 (0.5H, m), 7.48 (0.5H, m), 7.32 (0.5H, m), 7.25-7.21 (1.5H, m), 7.14 (0.5H, d, J = 7.8 Hz), 5.72 (1H, s), 4.50, 4.40 (2H, ABqd, J = 9.3, 47.9 Hz), 2.49 (3H, s), 1.51 (3H, s).
MS(ESI) m/z: 407 (M+H)⁺

### (Example 164) 1,1,1-Trifluoro-2-(6-{2-fluoro-5-[3-(6-methylpyridin-2-yl)-1H-pyrazol-4-yl]phenyl}pyridin-3-yl)propan-2-ol (Compound No. 2-666)

### (164a) 2-(6-Bromopyridin-3-yl)-1,1,1-trifluoropropan-2-ol

The same reaction as in Example (160a) was carried out using 1,1,1-trifluoroacetone (1.1 g, 9.8 mmol) instead of acetone. After purification, 1.1 g (yield: 63%) of the title compound was obtained as a yellow solid.
¹H-NMR (400 MHz, CDCl₃) δ ppm: 8.56 (1H, d, J = 2.4 Hz), 7.78 (1H, dd, J = 2.4, 8.3 Hz), 7.53 (1H, d, J = 8.3 Hz), 2.66 (1H, s), 1.81 (3H, s).
MS(ESI) m/z: 269 (M+H)⁺

### (164b) 1,1,1-Trifluoro-2-(6-{2-fluoro-5-[3-(6-methylpyridin-2-yl)-1H-pyrazol-4-yl]phenyl}pyridin-3-yl)propan-2-ol

The same reaction as in Example 147 was carried out using 2-(6-bromopyridin-3-yl)-1,1,1-trifluoropropan-2-ol (0.17 g, 0.63 mmol) obtained in Example (164a) instead of 4-bromo-2-fluorobenzenesulfonamide. After purification, 0.12 g (yield: 54%) of the title compound was obtained as a white solid. Melting point: 197 to 198°C
¹H-NMR (500 MHz, DMSO-d₆) δ ppm: 13.41 (0.5H, s), 13.18 (0.5H, s), 8.88 (1H, s), 8.19 (0.5H, dd, J = 2.0, 7.8 Hz), 8.11-8.07 (2H, m), 7.85-7.80 (1H, m), 7.76 (0.5H, d, J = 1.5 Hz), 7.70-7.66 (1H, m), 7.56 (0.5H, d, J = 7.8 Hz), 7.54 (0.5H, m), 7.50 (0.5H, m), 7.34-7.21 (2H, m), 7.14 (0.5H, d, J = 7.8 Hz), 6.89 (1H, s), 2.48 (3H, s), 1.77 (3H, s).
MS(ESI) m/z: 443 (M+H)⁺

### (Example 165) 1-(6-{2-Fluoro-5-[3-(6-methylpyridin-2-yl)-1H-pyrazol-4-yl]phenyl}pyridin-3-yl)ethanol (Compound No. 2-668)

### (165a) 1-(6-Bromopyridin-3-yl)ethanol

Water (4 mL) and sodium borohydride (0.23 g, 6.0 mmol) were added to an ethanol solution (10 mL) of 3-acetyl-6-bromopyridine (0.60 g, 3.0 mmol). The resulting mixture was stirred at room temperature for 30 min. The reaction solution was concentrated under reduced pressure, and then water was added thereto. After extraction with ethyl acetate, the organic layer was washed with water and brine, and then dried with anhydrous magnesium sulfate. The solvent was evaporated under reduced pressure to obtain a crude product 0.59 g (yield: 97%) of the title compound as a light yellow oily material.
¹H-NMR (500 MHz, CDCl₃) δ ppm: 8.35 (1H, d, J = 2.4 Hz), 7.61 (1H, dd, J = 2.4, 7.8 Hz), 7.47 (1H, d, J = 7.8 Hz), 4.94 (1H, m), 2.04 (1H, s), 1.51 (3H, d, J = 6.3 Hz).

### (165b) 1-(6-{2-Fluoro-5-[3-(6-methylpyridin-2-yl)-1H-pyrazol-4-yl]phenyl}pyridin-3-yl)ethanol

The same reaction as in Example 147 was carried out using 1-(6-bromopyridin-3-yl)ethanol (0.13 g, 0.64 mmol) obtained in Example (165a) instead of 4-bromo-2-fluorobenzenesulfonamide. After purification, 0.12 g (yield: 65%) of the title compound was obtained as a white solid.
Melting point: 149 to 151°C
¹H-NMR (500 MHz, DMSO-d₆) δ ppm: 13.40 (0.5H, s), 13.17 (0.5H, s), 8.64 (1H, d, J = 2.0 Hz), 8.12 (0.5H, dd, J = 2.0, 7.8 Hz), 8.09 (0.5H, s), 8.03 (0.5H, dd, J = 2.0, 7.8 Hz), 7.85-7.83 (1H, m), 7.75 (0.5H, s), 7.74-7.66 (2H, m), 7.55 (0.5H, d, J = 7.8 Hz), 7.51 (0.5H, m), 7.46 (0.5H, m), 7.30 (0.5H, m), 7.25-7.20 (1.5H, m), 7.14 (0.5H, d, J = 7.3 Hz), 5.36 (1H, d, J = 4.4 Hz), 4.84 (1H, m), 2.49 (3H, s), 1.39 (3H, d, J = 6.3 Hz).
MS(ESI) m/z: 375 (M+H)⁺.

### (Example 166) 2-[(6-{2-Fluoro-5-[3-(6-methylpyridin-2-yl)-1H-pyrazol-4-yl]phenyl}pyridin-3-yl)oxy]ethanol (Compound No. 2-680)

### (166a) 2-Chloro-5-[2-(tetrahydro-2H-pyran-2-yloxy)ethoxy]pyridine

Potassium carbonate (0.68 g, 4.9 mmol) and 2-(2-bromoethoxy)tetrahydro-2H-pyran (0.82 g, 3.9 mmol) were added to an acetonitrile solution (15 mL) of 2-chloro-5-hydroxypyridine (0.39 g, 3.0 mmol). The resulting mixture was stirred at 70°C for 6 hr. The reaction solution was concentrated under reduced pressure, and water was added thereto. After extraction with methylene chloride, the organic layer was washed with water and brine, and then dried with anhydrous magnesium sulfate. The solvent was evaporated under reduced pressure, and the resulting crude product was purified by silica gel column chromatography (Biotage, eluting solvent; hexane : ethyl acetate = 4 : 1) to obtain 0.52 g (yield: 67%) of the title compound as a yellow oily material.
¹H-NMR (500 MHz, CDCl₃) δ ppm: 8.10 (1H, d, J = 2.4 Hz), 7.27-7.21 (2H, m), 4.69 (1H, m), 4.23-4.16 (2H, m), 4.06 (1H, m), 3.87 (1H, m), 3.82 (1H, m), 3.53 (1H, m), 1.85-1.71 (2H, m), 1.64-1.52 (4H, m).
MS(ESI) m/z: 258 (M+H)⁺

### (166b) 2-[(6-{2-Fluoro-5-[3-(6-methylpyridin-2-yl)-1H-pyrazol-4-yl]phenyl}pyridin-3-yl)oxy]ethanol

The same reaction as in Example 147 was carried out using 2-chloro-5-[2-(tetrahydro-2H-pyran-2-yloxy)ethoxy]pyridine (0.24 g, 0.92 mmol) obtained in Example (166a) instead of 4-bromo-2-fluorobenzenesulfonamide. After purification, 0.15 g (yield: 41 %) of the title compound was obtained as a white solid. Melting point: 148 to 151°C
¹H-NMR (500 MHz, CDCl₃) δ ppm: 8.42 (1H, s), 8.03 (1H, m), 7.77 (1H, d, J = 8.3 Hz), 7.66 (1H, s), 7.42 (1H, m), 7.35 (1H, m), 7.30 (1H, m), 7.19-7.15 (2H, m), 7.04 (1H, d, J = 7.8 Hz), 4.18-4.17 (2H, m), 4.02-4.01 (2H, m), 2.57 (3H, s), 2.34 (1H, brs).
MS(ESI) m/z: 391 (M+H)⁺

### (Example 167) 4-{2-[(6-{2-Fluoro-5-[3-(6-methylpyridin-2-yl)-1H-pyrazol-4-yl]phenyl}pyridin-3-yl)oxy]ethyl}morpholine (Compound No. 2-686)

### (167a) 4-{2-[(6-Chloropyridin-3-yl)oxy]ethyl}morpholine

The same reaction as in Example (166a) was carried out using N-(2-chloroethyl)morpholine hydrochloride (0.68 g, 3.6 mmol) instead of 2-(2-bromoethoxy)tetrahydro-2H-pyran. After purification, 0.67 g (yield: 92%) of the title compound was obtained as a light yellow oily material.
¹H-NMR (500 MHz, CDCl₃) δ ppm: 8.07 (1H, d, J = 2.4 Hz), 7.24-7.19 (2H, m), 4.15-4.12 (2H, m), 3.75-3.72 (4H, m), 2.82-2.80 (2H, m), 2.58-2.55 (4H, m). MS(ESI) m/z: 243 (M+H)⁺

### (167b) 4-{2-[(6-{2-Fluoro-5-[3-(6-methylpyridin-2-yl)-1H-pyrazol-4-yl]phenyl}pyridin-3-yl)oxy]ethyl}morpholine

The same reaction as in Example 147 was carried out using 4-{2-[(6-chloropyridin-3-yl)oxy]ethyl}morpholine obtained in Example (167a) instead of 4-bromo-2-fluorobenzenesulfonamide. After purification, 0.13 g (yield: 30%) of the title compound was obtained as a pink amorphous form.
¹H-NMR (500 MHz, CDCl₃) δ ppm: 8.40 (1H, d, J = 2.4 Hz), 8.03 (1H, m), 7.77 (1H, d, J = 8.3 Hz), 7.66 (1H, s), 7.42 (1H, m), 7.36 (1H, m), 7.30 (1H, dd, J = 2.4, 8.3 Hz), 7.19-7.15 (2H, m), 7.04 (1H, d, J = 7.3 Hz), 4.25-4.24 (2H, m), 3.79-3.77 (4H, m), 2.90-2.88 (2H, m), 2.66-2.64 (4H, m), 2.57 (3H, s).
MS(ESI) m/z: 460 (M+H)⁺

### (Example 168) 2-(4-{6-Fluoro-4'-[(trifluoromethyl)sulfonyl]-1,1'-biphenyl-3-yl}-1H-pyrazol-3-yl)-6-methylpyridine (Compound No. 2-672)

### (168a) 1-Bromo-4-[(trifluoromethyl)sulfonyl]benzene

4-Bromophenyltrifluoromethylsulfide (1.0 g, 3.9 mmol) was dissolved in chloroform (50 mL), and m-chloroperbenzoic acid (1.4 g, 8.2 mmol) was added thereto. The resulting mixture was stirred at room temperature for 2 hr and then stirred at 60°C for 4 hr. To this reaction solution, m-chloroperbenzoic acid (0.67 g, 3.9 mmol) was added, and the resulting mixture was further stirred at 60°C for 8 hr. The reaction solution was cooled to room temperature, and saturated aqueous sodium bicarbonate was added thereto. After extraction with methylene chloride, the organic layer was washed with water and brine, and then dried with anhydrous sodium sulfate. The solvent was evaporated under reduced pressure, and the resulting crude product was purified by silica gel column chromatography (Yamazen, eluting solvent: hexane/ethyl acetate) to obtain 1.0 g (yield: 92%) of the title compound as a white solid.
¹H-NMR (400 MHz, CDCl₃) δ ppm: 7.93-7.82 (4H, m)

### (168b) 2-(4-{6-Fluoro-4'-[(trifluoromethyl)sulfonyl]-1,1'-biphenyl-3-yl}-1-{[2-(trimethylsilyl)ethoxy]methyl}-1H-pyrazol-3-yl)-6-methylpyridine

The same reaction as in Example (143f) was carried out using 1-bromo-4-[(trifluoromethyl)sulfonyl]benzene (260 mg, 0.88 mmol) obtained in Example (168a) instead of 5-bromothiophene-2-sulfonamide obtained in Example (143e). The resulting crude product was purified by silica gel column chromatography (Yamazen, eluting solvent: hexane/ethyl acetate) to obtain 340 mg (yield: 86%) of the title compound as a yellow oily material.
¹H-NMR (400 MHz, CDCl₃) δ ppm: 8.07 (2H, d, J = 8.3 Hz),7.79 (2H, d, J = 8.3 Hz), 7.75(1H, s), 7.65-7.57 (2H, m), 7.47-7.42 (2H, m), 7.16 (1H, dd, J = 8.3,10.3 Hz), 7.11 (1H, d, J = 8.3 Hz), 5.52 (2H, s), 3.69 (2H, t, J = 8.3 Hz), 2.46 (3H, s), 0.96 (2H, t, J = 8.3 Hz), 0.00 (9H, s).

### (168c) 2-(4-{6-Fluoro-4'-[(trifluoromethyl)sulfonyl]-1,1'-biphenyl-3-yl}-1H-pyrazol-3-yl)-6-methylpyridine

The same reaction as in Example (143g) was carried out using 2-(4-{6-fluoro-4'-[(trifluoromethyl)sulfonyl]-1,1'-biphenyl-3-yl}-1-{[2-(trimethylsilyl)ethoxy]methyl}-1H-pyrazol-3-yl)-6-methylpyridine (340 mg, 0.58 mmol) obtained in Example (168b) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). After purification, 78 mg (yield: 29%) of the title compound was obtained as a white solid.
Melting point: 81 to 84°C
¹H-NMR (400 MHz, CDCl₃) δ ppm: 8.08 (2H, d, J = 8.6 Hz), 7.85-7.80 (2H, J = 8.6 Hz), 7.64 (1H, s), 7.54 (1H, m), 7.50-7.46 (2H, m), 7.25 (1H, dd, J = 8.6, 10.2 Hz), 7.16 (1H, d, J = 7.8 Hz), 7.08 (1H, d, J = 7.8 Hz), 2.58 (3H, s).
MS(ESI) m/z: 462 (M+H)⁺.

### (Example 169) 2-({2'-Fluoro-5'-[3-(6-methylpyridin-2-yl)-1H-pyrazol-4-yl]-1,1'-biphenyl-4-yl}thio)ethanol (Compound No. 2-674)

### (169a) {2-[(4-Bromophenyl)thio]ethoxy}(t-butyl)dimethylsilane

4-Bromobenzenethiol (1.0 g, 5.3 mmol) was dissolved in tetrahydrofuran (50 mL), and sodium hydride (55%, oil, 0.28 g) was added thereto. The resulting mixture was stirred at room temperature for 5 min, and then (2-bromoethyl)-t-butyldimethylsilane was added thereto. The resulting mixture was stirred at room temperature for 3 hr. To this reaction solution, saturated aqueous sodium bicarbonate was added. After extraction with methylene chloride, the organic layer was washed with water and brine, and then dried with anhydrous sodium sulfate. The solvent was evaporated under reduced pressure, and the resulting crude product was purified by silica gel column chromatography (Yamazen, eluting solvent: hexane/ethyl acetate) to obtain 1.7 g (yield: 88%) of the title compound as a colorless oily material.
¹H-NMR (400 MHz, CDCl₃) δ ppm: 7.34 (2H, d, J = 8.8 Hz), 7.17 (2H, d, J = 8.8 Hz), 3.74 (2H, t, J = 6.8 Hz), 3.00 (2H, t, J = 6.8 Hz), 0.84 (9H, s), 0.00 (6H, s).

### (169b) 2-(4-{4'-[(2-{[t-Butyl(dimethyl)silyl]oxy}ethyl)thio]-6-fluoro-1,1'-biphenyl-3-yl}-1-{[2-(trimethylsilyl)ethoxy]methyl}-1H-pyrazol-3-yl)-6-methylpyridine

The same reaction as in Example (143f) was carried out using {2-[(4-bromophenyl)thio]ethoxy}(t-butyl)dimethylsilane (200 mg, 0.59 mmol) obtained in Example (169a) instead of 5-bromothiophene-2-sulfonamide obtained in Example (143e). The resulting crude product was purified by silica gel column chromatography (Yamazen, eluting solvent: hexane/ethyl acetate) to obtain 55 mg (yield: 21 %) of the title compound as a light yellow oily material.
¹H-NMR (400 MHz, CDCl₃) δ ppm: 7.70 (1H, s), 7.53 (1H, t, J = 7.8 Hz), 7.44 (1H, dd, J = 2.4, 7.8 Hz), 7.40-7.30 (5H, m), 7.27 (1H, m), 7.10-7.01 (2H, m), 5.51 (2H, s), 3.81 (2H, t, J = 7.4 Hz), 3.68 (2H, t, J = 7.4 Hz), 3.09 (2H, t, J = 7.4 Hz), 2.52 (3H, s), 0.96 (2H, t, J = 7.4 Hz), 0.89 (9H, s), 0.06 (6H, s), 0.00 (9H, s).

### (169c) 2-({2'-Fluoro-5'-[3-(6-methylpyridin-2-yl)-1H-pyrazol-4-yl]-1,1'-biphenyl-4-yl}thio)ethanol

The same reaction as in Example (143g) was carried out using 2-(4-{4'-[(2-{[t-butyl(dimethyl)silyl]oxy}ethyl)thio]-6-fluoro-1,1'-biphenyl-3-yl}-1- {[2-(trimethylsilyl)ethoxy]methyl}-1H-pyrazol-3-yl)-6-methylpyridine (55 mg, 0.085 mmol) obtained in Example (169b) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). After purification, 33 mg (yield: 96%) of the title compound was obtained as a white solid.
Melting point: 150 to 152°C
¹H-NMR (400 MHz, CDCl₃) δ ppm: 7.62 (1H, s), 7.49-7.37 (6H, m), 7.31 (1H, m), 7.20-7.11 (2H, m), 7.08 (1H, d, J = 7.4 Hz), 3.77 (2H, t, J = 6.3 Hz), 3.14 (2H, t, J = 6.3 Hz), 2.58 (3H, s).
MS(ESI) m/z: 406 (M+H)⁺

### (Example 170) 2-({2'-Fluoro-5'-[3-(6-methylpyridin-2-yl)-1H-pyrazol-4-yl]-1,1'-biphenyl-4-yl}sulfonyl)ethanol (Compound No. 2-676)

### (170a) {2-[(4-Bromophenyl)sulfonyl]ethoxy}(t-butyl)dimethylsilane

The same reaction as in Example (168a) was carried out using {2-[(4-bromophenyl)thio]ethoxy}(t-butyl)dimethylsilane (0.99 g, 2.8 mmol) obtained in Example (169a) instead of 4-bromophenyltrifluoromethylsulfide. After purification, 0.44 g (yield: 40%) of the title compound was obtained as a white solid.
¹H-NMR (400 MHz, CDCl₃) δ ppm: 7.82 (2H, d, J = 8.3 Hz), 7.73 (2H, d, J = 8.3 Hz), 4.04 (2H, t, J = 6.3 Hz), 3.39 (2H, t, J = 6.3 Hz), 0.81 (9H, s), 0.00 (6H, s).

### (170b) 2-(4-{4'-[(2-{[t-Butyl(dimethyl)silyl]oxy}ethyl)sulfonyl]-6-fluoro-1,1'-biphenyl-3-yl}-1-{[2-(trimethylsilyl)ethoxy]methyl}-1H-pyrazol-3-yl)-6-methylpyridine

The same reaction as in Example (143f) was carried out using {2-[(4-bromophenyl)sulfonyl]ethoxy}(t-butyl)dimethylsilane (220 mg, 0.59 mmol) obtained in Example (170a) instead of 5-bromothiophene-2-sulfonamide obtained in Example (143e). The resulting crude product was purified by silica gel column chromatography (Yamazen, eluting solvent: hexane/ethyl acetate) to obtain 190 mg (yield: 70%) of the title compound as a colorless oily material.
¹H-NMR (400 MHz, CDCl₃) δ ppm: 7.99 (2H, d, J = 8.3 Hz), 7.79 (1H, s), 7.69 (2H, d, J = 8.3 Hz), 7.62 (1H, t, J = 7.8 Hz), 7.54 (1H, dd, J = 2.0, 7.8 Hz), 7.47-7.42 (2H, m), 7.20-7.12 (2H, m), 5.57 (2H, s), 4.07 (2H, t, J = 6.3 Hz), 3.73 (2H, t, J = 7.8 Hz), 3.44 (2H, t, J = 6.3 Hz), 2.54 (3H, s), 1.01 (2H, t, J = 7.8 Hz), 0.80 (9H, s), 0.04 (9H, s), 0.00 (6H, s).

### (170c) 2-({2'-Fluoro-5'-[3-(6-methylpyridin-2-yl)-1H-pyrazol-4-yl]-1,1'-biphenyl-4-yl}sulfonyl)ethanol

The same reaction as in Example (143g) was carried out using 2-(4-{4'-[(2-{[t-butyl(dimethyl)silyl]oxyl ethyl)sulfonyl]-6-fluoro-1,1'-biphenyl-3-yl}-1-{[2-(trimethylsilyl)ethoxy]methyl}-1H-pyrazol-3-yl)-6-methylpyridine (180 mg, 0.26 mmol) obtained in Example (170b) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). After purification, 72 mg (yield: 63%) of the title compound was obtained as a white solid.
Melting point: 88 to 90°C
¹H-NMR (400 MHz, CDCl₃) δ ppm: 7.98 (2H, d, J = 8.2 Hz), 7.74 (2H, d, J = 8.2 Hz), 7.63 (1H, s), 7.50 (1H, dd, J = 2.4, 7.4 Hz), 7.48-7.41 (2H, m), 7.24 (1H, m), 7.16 (1H, d, J = 7.4 Hz), 7.08 (1H, d, J = 7.4 Hz), 4.08-4.02 (2H, m), 3.39 (2H, t, J = 5.1 Hz), 2.76 (1H, brs), 2.58 (3H, s).
MS(ESI) m/z: 438 (M+H)⁺.

### (Example 171) 2-(4-{4'-[(Difluoromethyl)sulfonyl]-6-fluoro-1,1'-biphenyl-3-yl}-1H-pyrazol-3-yl)-6-methylpyridine (Compound No. 2-678)

### (171a) 2-(4-{4'-[(Difluoromethyl)sulfonyl]-6-fluoro-1,1'-biphenyl-3-yl}-1-{[2-(trimethylsilyl)ethoxy]methyl}-1H-pyrazol-3-yl)-6-methylpyridine

The same reaction as in Example (143f) was carried out using 4-(difluoromethylsulfonyl)chlorobenzene (80 mg, 0.35 mmol) instead of 5-bromothiophene-2-sulfonamide obtained in Example (143e). The resulting crude product was purified by silica gel column chromatography (Yamazen, eluting solvent: hexane/ethyl acetate) to obtain 91 mg (yield: 81%) of the title compound as a colorless oily material.
¹H-NMR (400 MHz, CDCl₃) δ ppm: 8.03-7.97 (2H, m), 7.77-7.70 (3H, m), 7.58-7.52 (2H, m), 7.43-7.38 (2H, m), 7.14 (1H, d, J = 8.2 Hz), 7.10 (1H, t, J = 8.2 Hz), 6.22 (1H, t, J = 53.2 Hz), 5.51 (2H, s), 3.68 (2H, t, J = 8.2 Hz), 2.47 (3H, s), 0.96 (2H, t, J = 8.2 Hz), 0.00 (9H, s).

### (171b) 2-(4-{4'-[(Difluoromethyl)sulfonyl]-6-fluoro-1,1'-biphenyl-3-yl}-1H-pyrazol-3-yl)-6-methylpyridine

The same reaction as in Example (143g) was carried out using 2-(4-{4'-[(difluoromethyl)sulfonyl]-6-fluoro-1,1'-biphenyl-3-yl}-1-{[2-(trimethylsilyl)ethoxy]methyl}-1H-pyrazol-3-yl)-6-methylpyridine (91 mg, 0.16 mmol) obtained in Example (171a) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). After purification, 44 mg (yield: 63%) of the title compound was obtained as a white solid.
Melting point: 80 to 82°C
¹H-NMR (400 MHz, CDCl₃) δ ppm: 8.05 (2H, d, J = 8.8 Hz), 7.82 (2H, dd, J = 2.4, 8.8 Hz), 7.66 (1H, s), 7.55 (1H, dd, J = 2.4, 7.8 Hz), 7.50-7.46 (2H, m), 7.27 (1H, m), 7.18 (1H, d, J = 7.8 Hz), 7.10 (1H, d, J = 7.8 Hz), 6.21 (1H, t, J = 53.2 Hz), 2.59 (3H, s).
MS(ESI) m/z: 444 (M+H)⁺

### (Example 172) 2-{4-[4-Fluoro-3-(1-methyl-1H-imidazol-4-yl)phenyl]-1H-pyrazol-3-yl}-6-methylpyridine (Compound 2-698)

### (172a) 2-{4-[4-Fluoro-3-(1-methyl-1H-imidazol-4-yl)phenyl]-1-{[2-(trimethylsilyl)ethoxy]methyl}-1H-pyrazol-3-yl}-6-methylpyridine

2-(4-[4-Fluoro-3-(4,4,5,5-tetramethyl-1,3,2-dioxaborolan-2-yl)phenyl]-1-{[2-(trimethylsilyl)ethoxy]methyl}-1H-pyrazol-3-yl)-6-methylpyridine (0.31 g, 0.60 mmol) obtained in Example (143d) and 4-iodo-1-methyl-1H-imidazole (0.16 g, 0.75 mmol) were dissolved in 1,2-dimethoxyethane (5 mL), and water (0.5 mL), tripotassium phosphate n-hydrate (0.52 g, 1.8 mmol), and dichloro[1,1'-bis(diphenylphosphino)ferrocene]palladium(II)-methylene chloride complex (0.044 g, 0.060 mmol) were added thereto. The resulting mixture was heated under a nitrogen atmosphere under reflux for 4 hr. The reaction solution was cooled to room temperature, and water added thereto. After extraction with ethyl acetate, the organic layer was dried with anhydrous sodium sulfate. The solvent was evaporated under reduced pressure, and the resulting crude product was purified by silica gel column chromatography (Yamazen, eluting solvent: hexane/ethyl acetate) to obtain 0.15 g (yield: 53%) of the title compound as a yellow oily material.
¹H-NMR (400 MHz, CDCl₃) δ ppm: 8.23 (1H, dd, J = 2.0, 7.8 Hz), 7.79 (1H, s), 7.49 (1H, s), 7.47 (1H, t, J = 7.8 Hz), 7.39 (1H, d, J = 3.9 Hz), 7.24 (1H, d, J = 7.8 Hz), 7.09 (1H, ddd, J = 2.0, 4.9, 8.8 Hz), 7.06 (1H, d, J = 7.8 Hz), 6.97 (1H, dd, J = 8.8, 11.2 Hz), 5.52 (2H, s), 3.75 (3H, s), 3.66 (2H, t, J = 8.3 Hz), 2.56 (3H, s), 0.97 (2H, t, J = 8.3 Hz), 0.01 (9H, s).

### (172b) 2-{4-[4-Fluoro-3-(1-methyl-1H-imidazol-4-yl)phenyl]-1H-pyrazol-3-yl}-6-methylpyridine

2- {4-[4-Fluoro-3-(1-methyl-1H-imidazol-4-yl)phenyl]-1-{[2-(trimethylsilyl)ethoxy]methyl}-1H-pyrazol-3-yl}-6-methylpyridine (0.15 g, 0.32 mmol) obtained in Example (172a) was dissolved in ethanol (3 mL), and a 3 N hydrochloric acid aqueous solution (3 mL) was added thereto. The resulting mixture was heated under reflux for 5 hr. The reaction solution was cooled to room temperature and then neutralized with saturated aqueous sodium bicarbonate. After extraction with ethyl acetate, the organic layer was dried with anhydrous sodium sulfate. The solvent was evaporated under reduced pressure, and the resulting crude product was purified by silica gel column chromatography (Yamazen, eluting solvent: hexane/ethyl acetate). The resulting product was further purified by high-performance liquid chromatography (GL Science ODS-3, eluting solvent; water : acetonitrile = 95 : 5 to 5 : 95) to obtain 0.060 g (yield: 56%) of the title compound as a white solid.
Melting point: 168 to 169°C
¹H-NMR (400 MHz, CDCl₃) δ ppm: 11.14 (1H, br), 8.22 (1H, dd, J = 2.0, 7.4 Hz), 7.64 (1H, s), 7.47 (1H, s), 7.41 (1H, d, J = 4.3 Hz), 7.37 (1H, t, J = 7.4 Hz), 7.21-7.13 (2H, m), 7.09 (1H, dd, J = 8.2, 10.9 Hz), 7.00 (1H, d, J = 7.4 Hz), 3.75 (3H, s), 2.56 (3H, s).
MS(ESI) m/z: 334 (M+H)⁺

### (Example 173) 2-{2-Fluoro-5-[3-(6-methylpyridin-2-yl)-1H-pyrazol-4-yl]phenyl}-5-(1-methoxy-1-methylethyl)pyridine (Compound No. 2-682)

### (173a) 2-Bromo-5-(1-methoxy-1-methylethyl)pyridine

2-(6-Bromopyridin-3-yl)propan-2-ol (1.1 g, 5.0 mmol) obtained in Example (160a) was dissolved in N,N-dimethylformamide (10 mL), and iodomethane (0.62 mL, 10 mmol) was added thereto. The resulting mixture was cooled to 0°C, and sodium hydride (60%, oil, 0.40 g, 10 mmol) was added thereto. The mixture was stirred at 0°C for 1 hr and then stirred at room temperature for 3 hr. The reaction solution was cooled to 0°C, and water was gradually added thereto. After extraction with ethyl acetate, the organic layer was dried with anhydrous sodium sulfate. The solvent was evaporated under reduced pressure, and the residue was purified by silica gel column chromatography (Yamazen, eluting solvent: hexane/ethyl acetate) to obtain 1.1 g (yield: 95%) of the title compound as a white solid.
¹H-NMR (400 MHz, CDCl₃) δ ppm: 8.40 (1H, d, J = 2.7 Hz), 7.61 (1H, d, J = 2.7, 8.2 Hz), 7.46 (1H, d, J = 8.2 Hz), 3.10 (3H, s), 1.53 (6H, s).

### (173b) 2-(2-Fluoro-5-[3-(6-methylpyridin-2-yl)-1-{[2-(trimethylsilyl)ethoxy]methyl}-1H-pyrazol-4-yl]phenyl}-5-(1-methoxy-1-methylethyl)pyridine

The same reaction as in Example (172a) was carried out using 2-bromo-5-(1-methoxy-1-methylethyl)pyridine (0.28 g, 1.2 mmol) obtained in Example (173a) instead of 4-iodo-1-methyl-1H-imidazole. The resulting crude product was purified by silica gel column chromatography (Yamazen, eluting solvent: hexane/ethyl acetate) to obtain 0.35 g (quantitative yield) of the title compound as a yellow oily material.
¹H-NMR (400 MHz, CDC1₃) δ ppm: 8.72 (1H, d, J = 2.3 Hz), 8.04 (1H, dd, J = 2.3, 7.4 Hz), 7.78 (1H, dd, J = 2.3, 8.4 Hz), 7.76 (1H, s), 7.74 (1H, m), 7.49 (1H, t, J = 7.4 Hz), 7.29 (1H, m), 7.28 (1H, d, J = 7.4 Hz), 7.05 (1H, d, J = 7.4 Hz), 7.04 (1H, dd, J = 8.6, 11.0 Hz), 5.51 (2H, s), 3.66 (2H, t, J = 8.2 Hz), 3.13 (3H, s), 2.53 (3H, s), 1.59 (6H, s), 0.97 (2H, t, J = 8.2 Hz), 0.01 (9H, s).

### (173c) 2-{2-Fluoro-5-[3-(6-methylpyridin-2-yl)-1H-pyrazol-4-yl]phenyl}-5-(1-methoxy-1-methylethyl)pyridine

The same reaction as in Example (172b) was carried out using 2-{2-fluoro-5-[3-(6-methylpyridin-2-yl)-1-{[2-(trimethylsilyl)ethoxy]methyl}-1H-pyrazol-4-yl]phenyl}-5-(1-methoxy-1-methylethyl)pyridine (0.35 g, 0.60 mmol) obtained in Example (173b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.074 g (yield: 31%) of the title compound was obtained as a light yellow solid.
Melting point: 168 to 169°C
¹H-NMR (400 MHz, CDCl₃) δ ppm: 8.75 (1H, s), 8.08 (1H, dd, J = 2.4, 7.8 Hz), 7.84-7.79 (2H, m), 7.67 (1H, s), 7.43 (1H, t, J = 7.8 Hz), 7.39 (1H, ddd, J = 2.4, 4.9, 8.3 Hz), 7.19 (1H, dd, J = 8.3, 11.2 Hz), 7.18 (1H, d, J = 7.8 Hz), 7.05 (1H, d, J = 7.8 Hz), 3.14 (3H, s), 2.56 (3H, s), 1.59 (6H, s).
MS(ESI) m/z: 403 (M+H)⁺.

### (Example 174) 3-(6-{2-Fluoro-5-[3-(6-methylpyridin-2-yl)-1H-pyrazol-4-yl]phenyl}pyridin-3-yl)pentan-3-ol (Compound No. 2-684)

### (174a) 3-(6-Bromopyridin-3-yl)pentan-3-ol

The same reaction as in Example (160a) was carried out using 3-pentanone (2.1 mL, 20 mmol) instead of acetone. The resulting crude product was purified by silica gel column chromatography (Yamazen, eluting solvent: hexane/ethyl acetate) to obtain 2.2 g (yield: 89%) of the title compound as a yellow oily material.
¹H-NMR (400 MHz, CDCl₃) δ ppm: 8.35 (1H, d, J = 2.4 Hz), 7.57 (1H, dd, J = 2.4, 8.2 Hz), 7.42 (1H, d, J = 8.2 Hz), 1.85 (2H, dq, J = 7.0, 14.1 Hz), 1.81 (2H, dq, J = 7.0, 14.1 Hz), 0.74 (6H, t, J = 7.0 Hz).

### (174b) 3-(6-{2-Fluoro-5-[3-(6-methylpyridin-2-yl)-1-{[2-(trimethylsilyl)ethoxy]methyl}-1H-pyrazol-4-yl]phenyl}pyridin-3-yl)pentan-3-ol

The same reaction as in Example (172a) was carried out using 3-(6-bromopyridin-3-yl)pentan-3-ol (0.29 g, 1.2 mmol) obtained in Example (174a) instead of 4-iodo-1-methyl-1H-imidazole. The resulting crude product was purified by silica gel column chromatography (Yamazen, eluting solvent: hexane/ethyl acetate) to obtain 0.36 g (quantitative yield) of the title compound as a yellow oily material.
¹H-NMR (400 MHz, CDCl₃) δ ppm: 8.71 (1H, d, J = 2.3 Hz), 8.11 (1H, dd, J = 2.3, 7.4 Hz), 7.80 (1H, dd, J = 2.3, 8.2 Hz), 7.79 (1H, s), 7.76 (1H, m), 7.51 (1H, t, J = 7.8 Hz), 7.33-7.29 (2H, m), 7.07 (1H, d, J = 7.8 Hz), 7.06 (1H, dd, J = 8.6, 10.9 Hz), 5.53 (2H, s), 3.68 (2H, t, J = 8.2 Hz), 2.53 (3H, s), 1.94 (2H, dq, J = 7.4, 14.9 Hz), 1.87 (2H, dq, J = 7.4, 14.9 Hz), 0.97 (2H, t, J = 8.2 Hz), 0.83 (6H, t, J = 7.4 Hz), 0.01 (9H, s).

### (174c) 3-(6-{2-Fluoro-5-[3-(6-methylpyridin-2-yl)-1H-pyrazol-4-yl]phenyl}pyridin-3-yl)pentan-3-ol

The same reaction as in Example (172b) was carried out using 3-(6-{2-fluoro-5-[3-(6-methylpyridin-2-yl)-1-{[2-(trimethylsilyl)ethoxy]methyl}-1H-pyrazol-4-yl]phenyl}pyridin-3-yl)pentan-3-ol (0.36 g, 0.60 mmol) obtained in Example (174b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.15 g (yield: 62%) of the title compound was obtained as a white solid.
Melting point: 94 to 95°C
¹H-NMR (400 MHz, CDCl₃) δ ppm: 11.12 (1H, brs), 8.71 (1H, t, J = 1.6 Hz), 8.11 (1H, dd, J = 7.4, 2.3 Hz), 7.82-7.80 (2H, m), 7.67 (1H, s), 7.43 (1H, t, J = 7.8 Hz), 7.39 (1H, ddd, J = 2.3, 4.7, 8.2 Hz), 7.20 (1H, dd, J = 8.2, 10.9 Hz), 7.18 (1H, d, J = 7.8 Hz), 7.05 (1H, d, J = 7.8 Hz), 2.58 (3H, s), 1.93 (2H, dq, J = 7.4, 14.9 Hz), 1.87 (2H, dq, J = 7.4, 14.9 Hz), 0.82 (6H, t, J = 7.4 Hz).
MS(ESI) m/z: 417 (M+H)⁺

### (Example 175) 4-[2-(4-{2-Fluoro-5-[3-(6-methylpyridin-2-yl)-1H-pyrazol-4-yl]phenyl}-1H-pyrazol-1-yl)ethyl]morpholine (Compound No. 2-712)

### (175a) 4-[2-(4-Iodo-1H-pyrazol-1-yl)ethyl]morpholine

N,N-Dimethylformamide (10 mL) was added to a mixture of 4-iodopyrazole (0.97 g, 5.0 mmol), N-(2-chloroethyl)morpholine hydrochloride (1.4 g, 7.5 mmol), and potassium carbonate (2.1 g, 15 mmol). The resulting mixture was stirred at 95°C for 5 hr. The reaction solution was cooled to room temperature, and water was added thereto. After extraction with ethyl acetate, the organic layer was dried with anhydrous sodium sulfate. The solvent was evaporated under reduced pressure, and the residue was purified by silica gel column chromatography (Yamazen, eluting solvent: hexane/ethyl acetate) to obtain 0.98 g (yield: 64%) of the title compound as a white solid.
¹H-NMR (400 MHz, CDCl₃) δ ppm: 7.53 (1H, s), 7.50 (1H, s), 4.24 (2H, t, J = 6.7 Hz), 3.69 (4H, t, J = 4.7 Hz), 2.78 (2H, d, J = 6.7 Hz), 2.47 (4H, d, J = 4.7 Hz).

### (175b) 4-[2-(4-{2-Fluoro-5-[3-(6-methylpyridin-2-yl)-1-{[2-(trimethylsilyl)ethoxy]methyl}-1H-pyrazol-4-yl]phenyl}-1H-pyrazol-1-yl)ethyl]morpholine

The same reaction as in Example (172a) was carried out using 4-[2-(4-iodo-1H-pyrazol-1-yl)ethyl]morpholine (0.40 g, 1.3 mmol) obtained in Example (175a) instead of 4-iodo-1-methyl-1H-imidazole. The resulting crude product was purified by silica gel column chromatography (Yamazen, eluting solvent: ethyl acetate) to obtain 0.27 g (yield: 78%) of the title compound as a yellow oily material.
¹H-NMR (400 MHz, CDCl₃) δ ppm: 7.83 (1H, d, J = 2.4 Hz), 7.73 (1H, s), 7.70 (1H, s), 7.57 (1H, dd, J = 2.4, 7.3 Hz), 7.53 (1H, t, J = 7.8 Hz), 7.33 (1H, d, J = 7.8 Hz), 7.16 (1H, ddd, J = 2.4, 4.9, 8.3 Hz), 7.09 (1H, d, J = 7.8 Hz), 7.04 (1H, dd, J = 8.3, 10.7 Hz), 5.53 (2H, s), 4.27 (2H, t, J = 6.8 Hz), 3.71-3.68 (6H, m), 2.84 (2H, t, J = 6.8 Hz), 2.53 (3H, s), 2.49 (4H, t, J = 4.9 Hz), 0.97 (2H, t, J = 8.3 Hz), 0.01 (9H, s).

### (175c) 4-[2-(4-{2-Fluoro-5-[3-(6-methylpyridin-2-yl)-1H-pyrazol-4-yl]phenyl}-1H-pyrazol-1-yl)ethyl]morpholine

The same reaction as in Example (172b) was carried out using 4-[2-(4-{2-fluoro-5-[3-(6-methylpyridin-2-yl)-1-{[2-(trimethylsilyl)ethoxy]methyl}-1H-pyrazol-4-yl]phenyl}-1H-pyrazol-1-yl)ethyl]morpholine (0.27 g, 0.47 mmol) obtained in Example (175b) instead of 2-{4-[4-fluoro-3-(1-methyl-1H-imidazol-4-yl)phenyl]-1- {[2-(trimethylsilyl) ethoxy]methyl}-1H-pyrazol-3-yl}-6-methylpyridine obtained in Example (172a). After purification, 0.23 g (yield: 48%) of the title compound was obtained as a light yellow solid.
Melting point: 124°C
¹H-NMR (400 MHz, CDCl₃) δ ppm: 11.1 (1H, brs), 7.89 (1H, brs), 7.80 (1H, s), 7.64 (1H, s), 7.60 (1H, dd, J = 2.0, 7.3 Hz), 7.43 (1H, t, J = 7.8 Hz), 7.23 (1H, m), 7.18-7.14 (2H, m), 7.06 (1H, d, J = 7.8 Hz), 4.28 (2H, t, J = 6.3 Hz), 3.69 (4H, t, J = 4.4 Hz), 2.85 (2H, t, J = 6.3 Hz), 2.59 (3H, s), 2.50 (4H, brs).
MS(ESI) m/z: 433 (M+H)⁺.

### (Example 176) 2-(4-{2-Fluoro-5-[3-(6-methylpyridin-2-yl)-1H-pyrazol-4-yl]phenyl}-1H-pyrazol-1-yl)-N,N-dimethylethylamine (Compound No. 2-710)

### (176a) 2-(4-Iodo-1H-pyrazol-1-yl)-N,N-dimethylethylamine

N,N-Dimethylformamide (15 mL) was added to a mixture of 4-iodopyrazole (0.97 g, 5.0 mmol), 2-dimethylaminoethyl chloride hydrochloride (1.4 g, 10 mmol), and potassium carbonate (2.8 g, 15 mmol). The resulting mixture was stirred at 100°C for 24 hr. The reaction solution was cooled to room temperature, and water was added thereto. After extraction with ethyl acetate, the organic layer was dried with anhydrous sodium sulfate. The solvent was evaporated under reduced pressure, and the residue was purified by silica gel column chromatography (Yamazen, eluting solvent: methanol/ethyl acetate) to obtain 0.42 g (yield: 32%) of the title compound as a brown oily material.
¹H-NMR (400 MHz, CDCl₃) δ ppm: 7.53 (1H, s), 7.50 (1H, s), 4.22 (2H, t, J = 6.3 Hz), 2.73 (2H, t, J = 6.3 Hz), 2.27 (6H, s).

### (176b) 2-(4-{2-Fluoro-5-[3-(6-methylpyridin-2-yl)-1-{[2-(trimethylsilyl)ethoxy]methyl}-1H-pyrazol-4-yl]phenyl}-1H-pyrazol-1-yl)ethyl]-N,N-dimethylethylamine

The same reaction as in Example (172a) was carried out using 2-(4-iodo-1H-pyrazol-1-yl)-N,N-dimethylethylamine (0.32 g, 1.2 mmol) obtained in Example (176a) instead of 4-iodo-1-methyl-1H-imidazole. The resulting crude product was purified by silica gel column chromatography (Yamazen, eluting solvent: methanol/ethyl acetate) to obtain 0.33 g (yield: 80%) of the title compound as a brown oily material.
¹H-NMR (400 MHz, CDCl₃) δ ppm: : 7.81 (1H, d, J = 2.0 Hz), 7.73 (1H, s), 7.71 (1H, s), 7.56 (1H, dd, J = 2.0, 7.3 Hz), 7.53 (1H, t, J = 7.8 Hz), 7.31 (1H, d, J = 7.8 Hz), 7.15 (1H, ddd, J = 2.0, 4.9, 8.3 Hz), 7.09 (1H, d, J = 7.8 Hz), 7.03 (1H, dd, J = 8.3, 10.7 Hz), 5.53 (2H, s), 4.27 (2H, t, J = 6.8 Hz), 3.70 (2H, t, J = 8.3 Hz), 2.82 (2H, t. J = 6.8 Hz), 2.54 (3H, s), 2.30 (6H, s), 0.97 (2H, t, J = 8.3 Hz), -0.01 (9H, s).

### (176c) 2-(4-{2-Fluoro-5-[3-(6-methylpyridin-2-yl)-1H-pyrazol-4-yl]phenyl}-1H-pyrazol-1-yl)-N,N-dimethylethylamine

The same reaction as in Example (172b) was carried out using 2-(4-{2-fluoro-5-[3-(6-methylpyridin-2-yl)-1-{[2-(trimethylsilyl)ethoxy]methyl}1H-pyrazol-4-yl]phenyl}-1H-pyrazol-1-yl)ethyl]-N,N-dimethylethylamine (0.33 g, 0.63 mmol) obtained in Example (176b) instead of 2-{4-[4-fluoro-3-(1-methyl-1H-imidazol-4-yl)phenyl]-1- { [2-(trimethylsilyl)ethoxy]methyl} -1 H-pyrazol-3-yl} -6-methylpyridine obtained in Example (172a). After purification, 0.080 g (yield: 32%) of the title compound was obtained as a colorless amorphous form.
¹H-NMR (400 MHz, CDCl₃) δ ppm: 7.84 (1H, d, J = 2.3 Hz), 7.79 (1H, s), 7.62 (1H, s), 7.58 (1H, dd, J = 2.3, 7.4 Hz), 7.41 (1H, t, J = 7.8 Hz), 7.19 (1H, ddd, J = 2.3, 5.1, 8.2 Hz), 7.15-7.10 (2H, m), 7.04 (1H, d, J = 7.8 Hz), 4.25 (2H, t, J = 6.6 Hz), 2.80 (2H, t, J = 6.6 Hz), 2.58 (3H, s), 2.29 (6H, s).
MS(ESI) m/z: 391 (M+H)⁺

### (Example 177) 2-(4-{2-Fluoro-5-[3-(6-methylpyridin-2-yl)-1H-pyrazol-4-yl]phenyl}-1H-imidazo1-1-yl)ethanol (Compound No. 2-700)

### (177a) 4-Bromo-1-{2-(trityloxy)ethyl}-1H-imidazole

2-Bromoethanol (1.4 mL, 20 mmol) was dissolved in methylene chloride (50 mL), and triethylamine (3.5 mL, 25 mmol) and trityl chloride (7.0 g, 25 mmol) were added thereto. The resulting mixture was stirred at room temperature for 6 hr. To this reaction solution, water was added. After extraction with ethyl acetate, the organic layer was dried with anhydrous sodium sulfate. The solvent was evaporated under reduced pressure, and the residue was purified by silica gel column chromatography (Yamazen, eluting solvent: hexane/ethyl acetate) to obtain 4.4 g (yield: 60%) of 2-bromoethyl trityl ether as a white solid. N,N-Dimethylformamide (10 mL) was added to a mixture of the resulting 2-bromoethyl trityl ether (1.9 g, 5.1 mmol) and 4-bromoimidazole (0.68 g, 4.6 mmol). The resulting mixture was cooled to 0°C, and sodium hydride (60%, oil, 0.22g, 5.5 mmol) was added thereto. The mixture was warmed to room temperature and then stirred for 6 hr. To this reaction solution, water was added. After extraction with ethyl acetate, the organic layer was dried with anhydrous sodium sulfate. The solvent was evaporated under reduced pressure, and the residue was purified by silica gel column chromatography (Yamazen, eluting solvent: hexane/ethyl acetate) to obtain 1.4 g (yield: 69%) of the title compound as a white solid.
¹H-NMR (400 MHz, CDCl₃) δ ppm: 7.45 (1H, s), 7.29-7.22 (15H, m), 6.89 (1H, s), 4.00 (2H, t, J = 4.9 Hz), 3.36 (2H, t, J = 4.9 Hz).

### (177b) 2-(4-(4-Fluoro-3-{1-[2-(trityloxy)ethyl]-1H-imidazol-4-yl}phenyl)-1-{[2-(trimethylsilyl)ethoxy]methyl}-1H-pyrazol-3-yl)-6-methylpyridine

The same reaction as in Example (172a) was carried out using 4-bromo-1-{2-(trityloxy)ethyl}-1H-imidazole (0.69 g, 1.6 mmol) obtained in Example (177a) instead of 4-iodo-1-methyl-1H-imidazole. The resulting crude product was purified by silica gel column chromatography (Yamazen, eluting solvent: hexane/ethyl acetate) to obtain 0.46 g (yield: 78%) of the title compound as a light yellow solid. ¹H-NMR (400 MHz, CDCl₃) δ ppm: 8.28 (1H, dd, J = 2.4, 7.3 Hz), 7.81 (1H, s), 7.65 (1H, s), 7.49-7.45 (2H, m), 7.32-7.31 (6H, m), 7.27-7.20 (10H, m), 7.10 (1H, ddd, J = 2.4, 4.8, 8.3 Hz), 7.04 (1H, d, J = 7.8 Hz), 6.98 (1H, dd, J = 8.3, 10.7 Hz), 5.52 (2H, s), 4.11 (2H, t, J = 4.9 Hz), 3.66 (2H, t, J = 8.3 Hz), 3.41 (2H, t, J = 4.9 Hz), 2.55 (3H, s), 0.97 (2H, t, J = 8.3 Hz), 0.01 (9H, s).

### (177c) 2-(4-{2-Fluoro-5-[3-(6-methylpyridin-2-yl)-1H-pyrazol-4-yl]phenyl}-1H-imidazol-1-yl)ethanol

The same reaction as in Example (172b) was carried out using 2-(4-(4-fluoro-3- {1-[2-(trityloxy)ethyl]-1H-imidazol-4-yl}phenyl)-1-{[2-(trimethylsilyl)ethoxy]methyl}-1H-pyrazol-3-yl)-6-methylpyridine (0.33 g, 0.63 mmol) obtained in Example (177b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.14 g (yield: 64%) of the title compound was obtained as a colorless solid.
Melting point: 248°C
¹H-NMR (400 MHz, DMSO-d₆) δ ppm: 8.22 (1H, d, J = 7.4 Hz), 8.11 (1H, d, J = 7.0 Hz), 8.02 (1H, brs), 7.69-7.48 (3H, m), 7.23-7.10 (3H, m), 4.98 (2H, t, J = 5.1 Hz), 4.06 (2H, t, J = 5.1 Hz), 2.50 (3H, s).
MS(ESI) m/z: 364 (M+H)⁺

### (Example 178) 2-(4-{2-Fluoro-5-[3-(6-methylpyridin-2-yl)-1H-pyrazol-4-yl]phenyl}-1,3-thiazol-2-yl)propan-2-ol (Compound No. 2-716)

### (178a) 2-(4-Bromo-1,3-thiazol-2-yl)propan-2-ol

The same reaction as in Example (160a) was carried out using 2,4-dibromothiazole (1.0 g, 4.1 mmol) instead of acetone. The resulting crude product was purified by silica gel column chromatography (Yamazen, eluting solvent: hexane/ethyl acetate) to obtain 0.76 g (yield: 84%) of the title compound as a light yellow oily material.
¹H-NMR (400 MHz, CDC1₃) δ ppm: 7.15 (1H, s), 2.62 (1H, s), 1.68 (6H, s).

### (178b) 2-{4-[2-Fluoro-5-(3-(6-methylpyridin-2-yl)-1-{[2-(trimethylsilyl)ethoxy]methyl}-1H-pyrazol-4-yl)phenyl]-1,3-thiazol-2-yl}propan-2-o1

The same reaction as in Example (172a) was carried out using 2-(4-bromo-1,3-thiazol-2-yl)propan-2-ol (0.28 g, 1.2 mmol) obtained in Example (178a) instead of 4-iodo-1-methyl-1H-imidazole. The resulting crude product was purified by silica gel column chromatography (Yamazen, eluting solvent: hexane/ethyl acetate) to obtain 0.47 g (quantitative yield) of the title compound as a yellow oily material. ¹H-NMR (400 MHz, CDCl₃) δ ppm: 8.21 (1H, dd, J = 7.4, 8.3 Hz), 7.78 (1H, s), 7.68 (1H, d, J = 2.3 Hz), 7.52 (1H, t, J = 7.8 Hz), 7.31 (1H, d, J = 7.8 Hz), 7.25 (1H, ddd, J = 2.3, 4.7, 8.6 Hz), 7.08 (1H, d, J = 7.8 Hz), 7.06 (1H, dd, J = 8.6, 11.3 Hz), 5.55 (2H, s), 3.71 (2H, t, J = 8.2 Hz), 2.94 (1H, s), 2.52 (3H, s), 1.67 (6H, s), 0.97 (2H, t, J = 8.2 Hz), 0.01 (9H, s).

### (178c) 2-(4-{2-Fluoro-5-[3-(6-methylpyridin-2-yl)-1H-pyrazol-4-yl]phenyl}-1,3-thiazol-2-yl)propan-2-ol

The same reaction as in Example (172b) was carried out using 2-{4-[2-fluoro-5-(3-(6-methylpyridin-2-yl)-1-{[2-(trimethylsilyl)ethoxy]methyl}-1H-pyrazol-4-yl)phenyl]-1,3-thiazol-2-yl}propan-2-ol (0.47 g, 0.80 mmol) obtained in Example (178b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.12 g (yield: 37%) of the title compound was obtained as a white solid.
Melting point: 78 to 80°C
¹H-NMR (400 MHz, CDCl₃) δ ppm: 8.31 (1H, dd, J = 2.3, 7.4 Hz), 7.74 (1H, d, J = 2.3 Hz), 7.68 (1H, s), 7.42 (1H, t, J = 7.4 Hz), 7.32(1H, ddd, J = 2.3, 5.1, 8.6 Hz), 7.20-7.16 (2H, m), 7.06 (1H, d, J = 7.4 Hz), 2.94 (1H, brs), 2.59 (3H, s), 1.68 (6H, s). MS(ESI) m/z: 395 (M+H)⁺.

### (Example 179) 4-(6-{2-Fluoro-5-[3-(6-methylpyridin-2-yl)-1H-pyrazol-4-yl]phenyl}pyridin-3-yl)morpholine (Compound No. 2-690)

### (179a) 4-(6-Chloropyridin-3-yl)morpholine

Toluene (50 mL), morpholine (0.87 mL, 10 mmol), and tris(dibenzylideneacetone)dipalladium (0.46 g, 0.50 mmol) were added to a mixture of 2-chloro-5-bromopyridine (2.5 g, 13 mmol), 4,5-bis(diphenylphosphino)-9,9-dimethylxanthene (0.58 g, 1.0 mmol), and sodium t-butoxide (1.4 g, 15 mmol). The reaction solution was heated to 100°C under a nitrogen atmosphere and then stirred for 4 hr. The solvent was evaporated under reduced pressure, and the residue was purified by silica gel column chromatography (Yamazen, eluting solvent: hexane/ethyl acetate) to obtain 2.4 g (quantitative yield) of the title compound as a yellow solid.
¹H-NMR (400 MHz, CDCl₃) δ ppm: 8.02 (1H, d, J = 2.7 Hz), 7.20 (1H, d, J = 8.6 Hz), 7.16 (1H, dd, J = 2.7, 8.6 Hz), 3.87 (4H, t, J = 4.7 Hz), 3.16 (4H, t, J = 4.7 Hz).

### (179b) 4-(6-{2-Fluoro-5-[3-(6-methylpyridin-2-yl)-1-{[2-(trimethylsilyl)ethoxy]methyl}-1H-pyrazol-4-yl]phenyl}pyridine-3-yl)morpholine

The same reaction as in Example (172a) was carried out using 4-(6-chloropyridin-3-yl)morpholine (0.29 g, 1.2 mmol) obtained in Example (179a) instead of 4-iodo-1-methyl-1H-imidazole. The resulting crude product was purified by silica gel column chromatography (Yamazen, eluting solvent: hexane/ethyl acetate) to obtain 0.35 g (yield: 80%) of the title compound as a light yellow solid. ¹H-NMR (400 MHz, CDCl₃) δ ppm: 8.38 (1H, d, J = 2.9 Hz), 8.02 (1H, dd, J = 2.0, 7.8 Hz), 7.77 (1H, s), 7.69 (1H, dd, J = 2.0, 8.8 Hz), 7.49 (1H, t, J = 7.8 Hz), 7.29-7.22 (3H, m), 7.06 (1H, d, J = 7.8 Hz), 7.03 (1H, dd, J = 8.3, 10.7 Hz), 5.53 (2H, s), 3.90 (4H, t, J = 4.9 Hz), 3.67 (2H, t, J = 8.3 Hz), 3.25 (4H, t, J = 4.9 Hz), 2.54 (3H, s), 0.96 (2H, t, J = 8.3 Hz), -0.01 (9H, s).

### (179c) 4-(6-{2-Fluoro-5-[3-(6-methylpyridin-2-yl)-1H-pyrazol-4-yl]phenyl}pyridin-3-yl)morpholine

The same reaction as in Example (172b) was carried out using 4-(6-{2-fluoro-5-[3-(6-methylpyridin-2-yl)-1- { [2-(trimethylsilyl) ethoxy]methyl} -1H-pyrazol-4-yl]phenyl}pyridine-3-yl)morpholine (0.35 g, 0.64 mmol) obtained in Example (179b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.15 g (yield: 58%) of the title compound was obtained as a white solid.
Melting point: 88 to 90°C
¹H-NMR (400 MHz, CDCl₃) δ ppm: 11.10 (1H, brs), 8.38 (1H, d, J = 2.9 Hz), 8.04 (1H, dd, J = 2.4, 7.8 Hz), 7.74 (1H, d, J = 8.8 Hz), 7.66 (1H, s), 7.42 (1H, t, J = 7.8 Hz), 7.33 (1H, ddd, J = 2.4, 4.4, 8.3 Hz), 7.24 (1H, dd, J = 2.9, 8.8 Hz), 7.18 (1H, d, J = 7.8 Hz), 7.16 (1H, dd, J = 8.3, 11.2 Hz), 7.04 (1H, d, J = 7.8 Hz), 3.90 (4H, t, J = 4.9 Hz), 3.25 (4H, t, J = 4.9 Hz), 2.58 (3H, s).
MS(ESI) m/z: 416 (M+H)⁺

### (Example 180) 4-(5-{2-Fluoro-5-[3-(6-methylpyridin-2-yl)-1H-pyrazol-4-yl]phenyl}pyridin-2-yl)morpholine (Compound No. 2-696)

### (180a) 4-(5-Bromopyridin-2-yl)morpholine

The same reaction as in Example (179a) was carried out using 2,5-dibromopyridine (3.1 g, 13 mmol) instead of 2-chloro-5-bromopyridine. The resulting crude product was purified by silica gel column chromatography (Yamazen, eluting solvent: hexane/ethyl acetate) to obtain 1.8 g (yield: 72%) of the title compound as a light yellow solid.
¹H-NMR (400 MHz, CDCl₃) δ ppm: 8.18 (1H, d, J = 2.7 Hz), 7.54 (1H, dd, J = 2.7, 9.0 Hz), 6.52 (1H, d, J = 9.0 Hz), 3.80 (4H, t, J = 5.1 Hz), 3.46 (4H, t, J = 5.1 Hz).

### (180b) 4-(5-{2-Fluoro-5-[3-(6-methylpyridin-2-yl)-1-{[2-(trimethylsilyl)ethoxy]methyl}-1H-pyrazol-4-yl]phenyl}pyridin-2-yl)morpholine

The same reaction as in Example (172a) was carried out using 4-(5-bromopyridin-2-yl)morpholine (0.29 g, 1.2 mmol) obtained in Example (180a) instead of 4-iodo-1-methyl-1H-imidazole. The resulting crude product was purified by silica gel column chromatography (Yamazen, eluting solvent: hexane/ethyl acetate) to obtain 0.41 g (yield: 94%) of the title compound as a yellow oily material.
¹H-NMR (400 MHz, CDCl₃) δ ppm: 8.32 (1H, brs), 7.72 (1H, s), 7.65 (1H, dd, J = 2.0, 9.0 Hz), 7.54 (1H, t, J = 7.8 Hz), 7.42 (1H, dd, J = 2.0, 7.8 Hz), 7.34 (1H, d, J = 7.8 Hz), 7.28 (1H, m), 7.09 (1H, d, J = 7.8 Hz), 7.07 (1H, dd, J = 8.6, 10.6 Hz), 6.68 (1H, d, J = 9.0 Hz), 5.53 (2H, s), 3.84 (4H, t, J = 5.1 Hz), 3.69 (2H, t, J = 8.2 Hz), 3.56 (4H, t, J = 5.1 Hz), 2.53 (3H, s), 0.96 (2H, t, J = 8.2 Hz), -0.01 (9H, s). (180c) 4-(5-{2-Fluoro-5-[3-(6-methylpyridin-2-yl)-1H-pyrazol-4-yl]phenyl}pyridin-2-yl)morpholine

The same reaction as in Example (172b) was carried out using 4-(5-{2-fluoro-5-[3-(6-methylpyridin-2-yl)-1-{[2-(trimethylsilyl)ethoxy]methyl}-1H-pyrazol-4-yl]phenyl}pyridin-2-yl)morpholine (0.41 g, 0.76 mmol) obtained in Example (180b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.15 g (yield: 46%) of the title compound was obtained as a white solid.
Melting point: 85 to 87°C
¹H-NMR (400 MHz, CDCl₃) δ ppm: 11.10 (1H, brs), 8.38 (1H, brs), 7.72 (1H, dt, J = 2.4, 8.8 Hz), 7.64 (1H, s), 7.47-7.44 (2H, m), 7.33 (1H, ddd, J = 2.4, 4.4, 8.3 Hz), 7.22-7.17 (2H, m), 7.07 (1H, d, J = 7.8 Hz), 6.70 (1H, d, J = 8.8 Hz), 3.84 (4H, t, J = 5.4 Hz), 3.56 (4H, t, J = 5.4 Hz), 2.58 (3H, s).
MS(ESI) m/z: 415 (M+H)⁺.

### (Example 181) 4-[(6-{2-Fluoro-5-[3-(6-methylpyridin-2-yl)-1H-pyrazol-4-yl]phenyl}pyridin-3-yl)methyl]morpholine (Compound No. 2-688)

### (181a) 4-[(6-Bromopyridin-3-yl)methyl]morpholine

2-Bromo-5-formylpyridine (0.86 g, 4.6 mmol) was dissolved in ethanol (20 mL), and morpholine (0.48 mL, 5.5 mmol), acetic acid (0.37 mL, 6.4 mmol), and sodium cyanoborohydride (0.43 g, 6.9 mmol) were added thereto. The resulting mixture was stirred at room temperature for 3 hr. The solvent was evaporated under reduced pressure. To the residue, a saturated sodium carbonate aqueous solution was added. After extraction with methylene chloride, the organic layer was dried with anhydrous sodium sulfate. The solvent was evaporated under reduced pressure, and the residue was purified by silica gel column chromatography (Yamazen, eluting solvent: ethyl acetate) to obtain 0.51 g (yield: 43%) of the title compound as a white solid.
¹H-NMR (400 MHz, CDCl₃) δ ppm: 8.28 (1H, d, J = 2.3 Hz), 7.54 (1H, dd, J = 2.3, 8.0 Hz), 7.43 (1H, d, J = 8.0 Hz), 3.69 (4H, t, J = 4.7 Hz), 3.45 (2H, s), 2.43 (4H, t, J = 4.7 Hz).

### (181b) 4-[(6-{2-Fluoro-5-[3-(6-methylpyridin-2-yl)-1-{[2-(trimethylsilyl)ethoxy]methyl}-1H-pyrazol-4-yl]phenyl}pyridin-3-yl)methyl]morpholine

The same reaction as in Example (172a) was carried out using 4-[(6-bromopyridin-3-yl)methyl]morpholine (0.50 g, 2.0 mmol) obtained in Example (181a) instead of 4-iodo-1-methyl-1H-imidazole. The resulting crude product was purified by silica gel column chromatography (Yamazen, eluting solvent: hexane/ethyl acetate) to obtain 0.81 g (quantitative yield) of the title compound as a yellow oily material.
¹H-NMR (400 MHz, CDCl₃) δ ppm: 8.60 (1H, brs), 8.03 (1H, dd, J = 2.3, 7.4 Hz), 7.75 (1H, s), 7.73-7.71 (2H, m), 7.48 (1H, t, J = 7.4 Hz), 7.32-7.27 (2H, m), 7.06-7.01 (2H, m), 5.51 (2H, s), 3.73-3.64 (6H, m), 3.54 (2H, s), 2.52 (3H, s), 2.48 (4H, t, J = 4.7 Hz), 0.96 (2H, t, J = 8.6 Hz), 0.01 (9H, s).

### (181c) 4-[(6-{2-Fluoro-5-[3-(6-methylpyridin-2-yl)-1H-pyrazol-4-yl]phenyl}pyridin-3-yl)methyl]morpholine

The same reaction as in Example (172b) was carried out using 4-[(6-{2-fluoro-5-[3-(6-methylpyridin-2-yl)-1-{[2-(trimethylsilyl)ethoxy]methyl}-1H-pyrazol-4-yl]phenyl}pyridin-3-yl)methyl]morpholine (0.81 g, 1.3 mmol) obtained in Example (181b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.29 g (yield: 52%) of the title compound was obtained as a light brown solid.
Melting point: 68 to 70°C
¹H-NMR (400 MHz, CDCl₃) δ ppm: 8.64 (1H, brs), 8.07 (1H, dd, J = 2.3, 7.8 Hz), 7.80-7.78 (2H, m), 7.67 (1H, s), 7.43 (1H, t, J = 7.8 Hz), 7.40 (1H, ddd, J = 2.3, 4.7, 8.6 Hz), 7.20 (1H, dd, J = 8.6, 10.9 Hz), 7.18 (1H, d, J = 7.8 Hz), 7.05 (1H, d, J = 7.8 Hz), 3.73 (4H, t, J = 4.7 Hz), 3.57 (2H, s), 2.58 (3H, s), 2.50 (4H, t, J = 4.7 Hz). MS(ESI) m/z: 430 (M+H)⁺

### (Example 182) (4-{2-Fluoro-5-[3-(6-methylpyridin-2-yl)-1H-pyrazol-4-yl]phenyl}-1,3-thiazol-2-yl)methanol (Compound No. 2-718)

### (182a) (4-Bromo-1,3-thiazol-2-yl)methanol

2,4-Dibromothiazole (0.49 g, 2.0 mmol) was dissolved in diethylether (20 mL), and the resulting mixture was cooled to -78°C. To this mixture, an n-butyllithium hexane solution (1.6 M, 1.4 mL, 2.2 mmol) was added dropwise. The resulting mixture was stirred for 30 min, and then paraformaldehyde (0.13 g, 4.0 mmol) was added thereto. The resulting mixture was stirred at -78°C for 30 min and then gradually warmed to room temperature. To this reaction solution, water was added. After extraction with ethyl acetate, the organic layer was dried with anhydrous sodium sulfate. The solvent was evaporated under reduced pressure, and the residue was purified by silica gel column chromatography (Yamazen, eluting solvent: hexane/ethyl acetate) to obtain 0.070 g (yield: 19%) of the title compound as a brown oily material.
¹H-NMR (400 MHz, CDCl₃) δ ppm: 7.22 (1H, s), 4.95 (2H, s), 3.23 (1H, brs).

### (182b) (4-{2-Fluoro-5-[3-(6-methylpyridin-2-yl)-1-{[2-(trimethylsilyl)ethoxy]methyl}-1H-pyrazol-4-yl]phenyl}-1,3-thiazol-2-yl)methanol

The same reaction as in Example (172a) was carried out using (4-bromo-1,3-thiazol-2-yl)methanol (0.070 g, 0.38 mmol) obtained in Example (182a) instead of 4-iodo-1-methyl-1H-imidazole. The resulting crude product was purified by silica gel column chromatography (Yamazen, eluting solvent: hexane/ethyl acetate) to obtain 0.11 g (yield: 72%) of the title compound as a yellow oily material.
¹H-NMR (400 MHz, CDCl₃) δ ppm: 8.21 (1H, dd, J = 2.3, 7.4 Hz), 7.77 (1H, s), 7.68 (1H, d, J = 2.3 Hz), 7.51 (1H, t, J = 7.4 Hz), 7.28 (1H, d, J = 7.4 Hz), 7.20 (1H, ddd, J = 2.3, 4.7, 8.6 Hz), 7.07 (1H, d, J = 7.4 Hz), 7.03 (1H, dd, J = 8.6, 11.3 Hz), 5.52 (2H, s), 4.92 (2H, s), 3.69 (2H, t, J = 8.2 Hz), 2.53 (3H, s), 2.13 (1H, brs), 0.97 (2H, t, J = 8.2 Hz), -0.01 (9H, s).

### (182c) (4-{2-Fluoro-5-[3-(6-methylpyridin-2-yl)-1H-pyrazol-4-yl]phenyl}-1,3-thiazol-2-yl)methanol

The same reaction as in Example (172b) was carried out using (4-{2-fluoro-5-[3-(6-methylpyridin-2-yl)-1-{[2-(trimethylsilyl)ethoxy]methyl}-1H-pyrazol-4-yl]phenyl}-1,3-thiazol-2-yl)methanol (0.11 g, 0.22 mmol) obtained in Example (182b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.040 g (yield: 37%) of the title compound was obtained as a white solid.
Melting point: 89 to 92°C
¹H-NMR (400 MHz, CDCl₃) δ ppm: 8.26 (1H, dd, J = 2.3, 7.0 Hz), 7.78 (1H, d, J = 2.3 Hz), 7.63 (1H, s), 7.40 (1H, t, J = 7.8 Hz), 7.31 (1H, m), 7.17 (1H, dd, J = 8.2, 11.0 Hz), 7.13 (1H, d, J = 7.8 Hz), 7.03 (1H, d, J = 7.8 Hz), 4.98 (2H, s), 2.57 (3H, s). MS(ESI) m/z: 367 (M+H)⁺

### (Example 183) 2-(2-{2-Fluoro-5-[3-(6-methylpyridin-2-yl)-1H-pyrazol-4-yl]phenyl}pyrimidin-5-yl)propan-2-ol (Compound No. 2-714)

### (183a) 2-(2-Chloropyrimidin-5-yl)propan-2-ol

5-Bromo-2-chloropyrimidine (1.9 g, 10 mmol) was dissolved in diethylether (50 mL) and tetrahydrofuran (50 mL), and then acetone (1.5 mL, 20 mmol) was added thereto. The resulting mixture was cooled to -78°C. To this mixture, an n-butyllithium hexane solution (1.6 M, 9.5 mL, 15 mmol) was added dropwise over 20 min. The resulting mixture was stirred for 20 min, and saturated aqueous sodium bicarbonate was added to this reaction solution. After extraction with ethyl acetate, the organic layer was dried with anhydrous sodium sulfate. The solvent was evaporated under reduced pressure. The residue was purified by silica gel column chromatography (Yamazen, eluting solvent: hexane/ethyl acetate) to obtain 0.60 g (yield: 35%) of the title compound as a light yellow solid.
¹H-NMR (400 MHz, CDC1₃) δ ppm: 8.74 (2H, s), 2.63 (1H, s), 1.64 (6H, s).

### (183b) 2-(2-{2-Fluoro-5-[3-(6-methylpyridin-2-yl)-1-{[2-(trimethylsilyl)ethoxy]methyl}-1H-pyrazol-4-yl]phenyl]pyrimidin-5-yl}propan-2-ol

The same reaction as in Example (172a) was carried out using 2-(2-chloropyrimidin-5-yl)propan-2-ol (0.21 g, 1.2 mmol) obtained in Example (183a) instead of 4-iodo-1-methyl-1H-imidazole. The resulting crude product was purified by silica gel column chromatography (Yamazen, eluting solvent: hexane/ethyl acetate) to obtain 0.39 g (yield: 94%) of the title compound as a yellow oily material.
¹H-NMR (400 MHz, CDCl₃) δ ppm: 8.97 (2H, s), 8.16 (1H, dd, J = 2.3, 7.4 Hz), 7.78 (1H, s), 7.51 (1H, t, J = 7.8 Hz), 7.40 (1H, ddd, J = 2.3, 4.7, 8.6 Hz), 7.32 (1H, d, J = 7.8 Hz), 7.11 (1H, dd, J = 8.6, 10.9 Hz), 7.07 (1H, d, J = 7.8 Hz), 5.53 (2H, s), 3.68 (2H, t, J = 8.2 Hz), 2.53 (3H, s), 1.68 (6H, s), 0.97 (2H, t, J = 8.2 Hz), -0.01 (9H, s).

### (183c) 2-(2-{2-Fluoro-5-[3-(6-methylpyridin-2-yl)-1H-pyrazol-4-yl]phenyl}pyrimidin-5-yl}propan-2-ol

The same reaction as in Example (172b) was carried out using 2-(2-{2-fluoro-5-[3-(6-methylpyridin-2-yl)-1-{[2-(trimethylsilyl)ethoxy]methyl}-1H-pyrazol-4-yl]phenyl)pyrimidin-5-yl)propan-2-ol (0.39 g, 0.75 mmol) obtained in Example (183b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.15 g (yield: 52%) of the title compound was obtained as a white solid.
Melting point: 162°C ¹H-NMR (400 MHz, CDCl₃) δ ppm: 8.99 (2H, s), 8.13 (1H, dd, J = 2.3, 7.4 Hz), 7.67 (1H, s), 7.47 (1H, ddd, J = 2.3, 4.3, 8.6 Hz), 7.43 (1H, t, J = 7.8 Hz), 7.24 (1H, dd, J = 8.6, 10.9 Hz), 7.16 (1H, d, J = 7.8 Hz), 7.05 (1H, d, J = 7.8 Hz), 2.58 (3H, s), 1.68 (6H, s).
MS(ESI) m/z: 390 (M+H)⁺.

### (Example 184) 2-(5-{2-Fluoro-5-[3-(6-methylpyridin-2-yl)-1H-pyrazol-4-yl]phenyl}-1,3-thiazol-2-yl)propan-2-ol (Compound No. 2-720)

### (184a) 2-(5-Bromo-1,3-thiazol-2-yl)propan-2-ol

The same reaction as in Example (183a) was carried out using 2,5-dibromothiazole (1.2 g, 5.0 mmol) instead of 5-bromo-2-chloropyrimidine. The resulting crude product was purified by silica gel column chromatography (Yamazen, eluting solvent: hexane/ethyl acetate) to obtain 0.19 g (yield: 17%) of the title compound as a brown oily material.
¹H-NMR (400 MHz, CDCl₃) δ ppm: 7.38 (1H, s), 2.20 (1H, s), 1.66 (6H, s).

### (184b) 2-{5-[2-Fluoro-5-(3-(6-methylpyridin-2-yl)-1-{[2-(trimethylsilyl)ethoxy]methyl}-1H-pyrazol-4-yl)phenyl]-1,3-thiazol-2-yl} propan-2-ol

The same reaction as in Example (172a) was carried out using 2-(5-bromo-1,3-thiazol-2-yl)propan-2-ol (0.19 g, 0.86 mmol) obtained in Example (184a) instead of 4-iodo-1-methyl-1H-imidazole. The resulting crude product was purified by silica gel column chromatography (Yamazen, eluting solvent: hexane/ethyl acetate) to obtain 0.24 g (yield: 63%) of the title compound as a brown oily material.
¹H-NMR (400 MHz, CDCl₃) δ ppm: 8.33 (1H, dd, J = 2.0, 7.0 Hz), 7.79 (1H, s), 7.73 (1H, d, J = 2.0 Hz), 7.53 (1H, t, J = 7.8 Hz), 7.37-7.33 (2H, m), 7.11-7.07 (2H, m), 5.53 (2H, s), 3.68 (2H, t, J = 8.2 Hz), 2.52 (3H, s), 2.14 (1H, brs), 1.74 (6H, s), 0.97 (2H, t, J = 8.2 Hz), -0.01 (9H, s).

### (184c) 2-(5-{2-Fluoro-5-[3-(6-methylpyridin-2-yl)-1H-pyrazol-4-yl]phenyl}-1,3-thiazol-2-yl)propan-2-ol

The same reaction as in Example (172b) was carried out using 2-{5-[2-fluoro-5-(3-(6-methylpyridin-2-yl)-1-{[2-(trimethylsilyl)ethoxy]methyl}-1H-pyrazol-4-yl)phenyl]-1,3-thiazol-2-yl}propan-2-ol (0.24 g, 0.45 mmol) obtained in Example (184b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.017 g (yield: 9.0%) of the title compound was obtained as a light yellow solid.
Melting point: 208 to 210°C
¹H-NMR (400 MHz, CDCl₃+DMSO-d₆) δ ppm: 8.31 (1H, dd, J = 2.3, 7.0 Hz), 7.70 (1H, d, J = 2.3 Hz), 7.68 (1H, s), 7.48 (1H, t, J = 7.8 Hz), 7.39 (1H, m), 7.23-7.15 (2H, m), 7.07 (1H, d, J = 7.8 Hz), 2.56 (3H, s), 1.70 (6H, s).
MS(ESI) m/z: 345 (M+H)⁺

### (Example 185) 4-[2-(4-{2-Fluoro-5-[3-(6-methylpyridin-2-yl)-1H-pyrazol-4-yl]phenyl}-1H-imidazol-1-yl)ethyl]morpholine (Compound No. 2-704) (185a) 4-[2-(4-Bromo-1H-imidazol-1-yl)ethyl]morpholine

4-Bromo-1-{2-(trityloxy)ethyl}-1H-imidazole (1.4 g, 3.2 mmol) obtained in Example (177a) was dissolved in ethanol (10 mL) and tetrahydrofuran (4 mL), and then a 3N hydrochloric acid aqueous solution (10 mL) was added thereto. The resulting mixture was stirred at room temperature for 3 hr. To this reaction solution, methylene chloride was added. The water layer was washed and then basified with sodium hydroxide. After extraction with methylene chloride, the organic layer was dried with anhydrous sodium sulfate. The solvent was evaporated under reduced pressure to obtain a crude product 0.42 g (yield: 69%) of 2-(4-bromo-1H-imidazol-1-yl)ethanol. The obtained 2-(4-bromo-1H-imidazol-1-yl)ethanol (0.26 g, 1.4 mmol) was dissolved in methylene chloride (10 mL). The resulting mixture was cooled to 0°C. To this reaction solution, triphenylphosphine (0.69 g, 2.6 mmol) and N-bromosuccinimide (0.47 g, 2.6 mmol) were added. The resulting mixture was warmed to room temperature and then stirred for 3 hr. To this mixture, a 1 N hydrochloric acid aqueous solution was added. The water layer was washed with methylene chloride and then neutralized with saturated aqueous sodium bicarbonate. After extraction with ethyl acetate, the organic layer was dried with anhydrous sodium sulfate. The solvent evaporated under reduced pressure to obtain a crude product of 4-bromo-1-(2-bromoethyl)-1H-imidazole. The resulting 4-bromo-1-(2-bromoethyl)-1H-imidazole (0.34 g, 1.4 mmol) was dissolved in acetonitrile (10 mL), and morpholine (1.2 mL, 14 mmol) and potassium carbonate (1.4 g, 10 mmol) were added thereto. The resulting mixture was heated under reflux for 4 hr. The reaction solution was cooled to room temperature, and water was added thereto. After extraction with ethyl acetate, the organic layer was dried with anhydrous sodium sulfate. The solvent was evaporated under reduced pressure, and the residue was purified by silica gel column chromatography (Yamazen, eluting solvent: methanol/ethyl acetate) to obtain 0.31 g (yield: 88%) of the title compound as a light yellow oily material.
¹H-NMR (400 MHz, CDCl₃) δ ppm: 7.40 (1H, d, J =1.6 Hz), 6.96 (1H, d, J = 1.6 Hz), 3.98 (2H, t, J = 6.3 Hz), 3.69 (4H, t, J = 4.7 Hz), 2.66 (2H, t, J = 6.3 Hz), 2.46 (4H, t, J = 4.7 Hz).

### (185b) 4-[2-(4-{2-Fluoro-5-[3-(6-methylpyridin-2-yl)-1-{[2-(trimethylsilyl)ethoxy]methyl}-1H-pyrazol-4-yl]phenyl}-1H-imidazol-1-yl)ethyl]morpholine

The same reaction as in Example (172a) was carried out using 4-[2-(4-bromo-1H-imidazol-1-yl)ethyl]morpholine (0.31 g, 1.2 mmol) obtained in Example (185a) instead of 4-iodo-1-methyl-1H-imidazole. The resulting crude product was purified by silica gel column chromatography (Yamazen, eluting solvent: methanol/ethyl acetate) to obtain 0.44 g (yield: 98%) of the title compound as a brown oily material. ¹H-NMR (400 MHz, CDCl₃) δ ppm: 8.22 (1H, dd, J = 2.3, 7.4 Hz), 7.77 (1H, s), 7.59 (1H, s), 7.47-7.43 (2H, m), 7.22 (1H, d, J = 7.8 Hz), 7.07 (1H, ddd, J = 2.3, 5.1, 8.2 Hz), 7.04 (1H, d, J = 7.8 Hz), 6.95 (1H, dd, J = 8.2, 10.9 Hz), 5.50 (2H, s), 4.07 (2H, t, J = 6.3 Hz), 3.70 (4H, t, J = 4.7 Hz), 3.65 (2H, t, J = 8.2 Hz), 2.73 (2H, t, J = 6.3 Hz), 2.55 (3H, s), 2.49 (4H, t, J = 4.7 Hz), 0.96 (2H, t, J = 8.2 Hz), 0.01 (9H, s).

### (185c) 4-[2-(4-{2-Fluoro-5-[3-(6-methylpyridin-2-yl)-1H-pyrazol-4-yl]phenyl}-1H-imidazol-1-yl)ethyl]morpholine

The same reaction as in Example (172b) was carried out using 4-[2-(4-{2-fluoro-5-[3-(6-methylpyridin-2-yl)-1-{[2-(trimethylsilyl)ethoxy]methyl}-1H-pyrazol-4-yl]phenyl}-1H-imidazol-1-yl)ethyl]morpholine (0.44 g, 0.79 mmol) obtained in Example (185b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.14 g (yield: 41%) of the title compound was obtained as a white solid.
Melting point: 144 to 145°C
¹H-NMR (400 MHz, CDCl₃) δ ppm: 11.10 (1H, brs), 8.22 (1H, dd, J = 2.3, 7.4 Hz), 7.64 (1H, s), 7.59 (1H, s), 7.50 (1H, d, J = 4.3 Hz), 7.37 (1H, t, J = 7.8 Hz), 7.18 (1H, ddd, J = 2.3, 5.1, 8.2 Hz), 7.14 (1H, d, J = 7.8 Hz), 7.10 (1H, dd, J = 8.2, 11.3 Hz), 7.00 (1H, d, J = 7.8 Hz), 4.08 (2H, t, J = 6.3 Hz), 3.71 (4H, t, J = 4.7 Hz), 2.74 (2H, t, J = 6.3 Hz), 2.56 (3H, s), 2.50 (4H, t, J = 4.7 Hz).
MS(ESI) m/z: 433 (M+H)⁺.

### (Example 186) 2-(4-{2-Fluoro-5-[3-(6-methylpyridin-2-yl)-1H-pyrazol-4-yl]phenyl}-1H-imidazol-1-yl)-N,N-dimethylethylamine (Compound No. 2-702) (186a) 2-(4-Bromo-1H-imidazol-1-yl)-N,N-dimethylethylamine

The same reaction as in Example (185a) was carried out using dimethylamine hydrochloride (1.6 g, 19 mmol) instead of morpholine. After extraction, a crude product 0.42 g (yield: 88%) of the title compound was obtained as a light yellow oily material. This material was used in the subsequent reaction without purification. ¹H-NMR (400 MHz, CDC1₃) δ ppm: 7.40 (1H, d, J = 1.6 Hz), 6.97 (1H, d, J = 1.6 Hz), 3.97 (2H, t, J = 6.6 Hz), 2.62 (2H, t, J = 6.6 Hz), 2.27 (6H, s). (186b) 2-(4-{2-Fluoro-5-[3-(6-methylpyridin-2-yl)-1-{[2-(trimethylsilyl)ethoxy]methyl}-1H-pyrazol-4-yl]phenyl}-1H-imidazol-1-yl)-N,N-dimethylethylamine

The same reaction as in Example (172a) was carried out using 2-(4-bromo-1H-imidazol-1-yl)-N,N-dimethylethylamine (0.42 g, 1.9 mmol) obtained in Example (186a) instead of 4-iodo-1-methyl-1H-imidazole. The resulting crude product was purified by silica gel column chromatography (Yamazen, eluting solvent: methanol/ethyl acetate) to obtain 0.57 g (yield: 85%) of the title compound as a brown oily material.
¹H-NMR (400 MHz, CDCl₃) δ ppm: 8.23 (1H, dd, J = 2.3, 7.4 Hz), 7.79 (1H, s), 7.59 (1H, s), 7.47-7.44 (2H, m), 7.22 (1H, d, J = 7.8 Hz), 7.08 (1H, ddd, J = 2.3, 5.1, 8.6 Hz), 7.06 (1H, d, J = 7.8 Hz), 6.97 (1H, dd, J = 8.6, 10.1 Hz), 5.52 (2H, s), 4.07 (2H, t, J = 6.6 Hz), 3.66 (2H, t, J = 8.2 Hz), 2.70 (2H, t, J = 6.6 Hz), 2.56 (3H, s), 2.30 (6H, s), 0.97 (2H, t, J = 8.2 Hz), -0.01 (9H, s).

### (186c) 2-(4-{2-Fluoro-5-[3-(6-methylpyridin-2-yl)-1H-pyrazol-4-yl]phenyl}-1H-imidazol-1-yl)-N,N-dimethylethylamine

The same reaction as in Example (172b) was carried out using 2-(4-{2-fluoro-5-[3-(6-methylpyridin-2-yl)-1-{[2-(trimethylsilyl)ethoxy]methyl}-1H-pyrazol-4-yl]phenyl}-1H-imidazol-1-yl)-N,N-dimethylethylamine (0.57 g, 1.1 mmol) obtained in Example (186b) instead of 2-{4-[4-fluoro-3-(1-methyl-1H-imidazol-4-yl)phenyl]-1-{[2-(trimethylsilyl)ethoxy]methyl}-1H-pyrazol-3-yl}-6-methylpyridine obtained in Example (172a). After purification, 0.18 g (yield: 42%) of the title compound was obtained as a white solid.
Melting point: 130 to 131°C
¹H-NMR (400 MHz, CDCl₃) δ ppm: 11.5 (1H, brs), 8.22 (1H, dd, J = 2.3, 7.4 Hz), 7.63 (1H, s), 7.56 (1H, s), 7.46 (1H, d, J = 3.9 Hz), 7.37 (1H, t, J = 7.8 Hz), 7.17 (1H, ddd, J = 2.3, 5.1, 8.2 Hz), 7.13 (1H, d, J = 7.8 Hz), 7.09 (1H, d, J = 8.2, 10.9 Hz), 7.00 (1H, d, J = 7.8 Hz), 4.06 (2H, t, J = 6.6 Hz), 2.69 (2H, t, J = 6.6 Hz), 2.56 (3H, s), 2.29 (6H, s).
MS(ESI) m/z: 391 (M+H)⁺

### (Example 187) 1-(5-{2-Fluoro-5-[3-(6-methylpyridin-2-yl)-1H-pyrazol-4-yl]phenyl}pyridin-2-yl)-4-methylpiperazine (Compound No. 2-694)

### (187a) 1-(5-Bromopyridin-2-yl)-4-methylpiperazine

The same reaction as in Example (180a) was carried out using 1-methylpiperazine (0.78 mL, 6.9 mmol) instead of morpholine. The resulting crude product was purified by silica gel column chromatography (NH, eluting solvent; methylene chloride : ethyl acetate = 19 : 1) to obtain 1.7 g (yield: 96%) of the title compound as a yellow crystal.
¹H-NMR (400 MHz, CDCl₃) δ ppm: 8.17 (1H, d, J = 2.6 Hz), 7.51 (1H, dd, J = 2.6, 9.0 Hz), 6.53 (1H, d, J = 9.0 Hz), 3.52 (4H, t, J = 5.1 Hz), 2.50 (4H, t, J = 5.1 Hz), 2.34 (3H, s).

### (187b) 1-{5-[2-Fluoro-5-(3-(6-methylpyridin-2-yl)-1-{[2-(trimethylsilyl)ethoxy]methyl}-1H-pyrazol-4-yl)phenyl]pyridin-2-yl)-4-methylpiperazine

The same reaction as in Example (143f) was carried out using 1-(5-bromopyridin-2-yl)-4-methylpiperazine (270 mg, 1.1 mmol) obtained in Example (187a) instead of 5-bromothiophene-2-sulfonamide obtained in Example (143e). The resulting crude product was purified by silica gel column chromatography (NH, eluting solvent; hexane : ethyl acetate = 3 : 2) to obtain 200 mg (yield: 50%) of the title compound as a light yellow oily material.
¹H-NMR (500 MHz, CDCl₃) δ ppm: 8.31 (1H, s), 7.71 (1H, s), 7.63 (1H, d, J = 8.8 Hz), 7.54 (1H, t, J = 7.8 Hz), 7.42 (1H, m), 7.33 (1H, d, J = 7.8 Hz), 7.25 (1H, m), 7.10-7.05 (2H, m), 6.69 (1H, d, J = 8.8 Hz), 3.70 (2H, s), 3.68 (2H, t, J = 8.1 Hz), 3.61 (4H, t, J = 5.1 Hz), 2.54 (4H, t, J = 5.1 Hz), 2.53 (3H, s), 2.36 (3H, s), 0.96 (2H, t, J = 8.1 Hz), 0.00 (9H, s).

### (187c) 1-(5-{2-Fluoro-5-[3-(6-methylpyridin-2-yl)-1H-pyrazol-4-yl]phenyl}pyridin-2-yl)-4-methylpiperazine

The same reaction as in Example (143g) was carried out using 1-{5-[2-fluoro-5-(3-(6-methylpyridin-2-yl)-1-{[2-(trimethylsilyl)ethoxy]methyl}-1H-pyrazol-4-yl)phenyl]pyridin-2-yl}-4-methylpiperazine (200 mg, 0.36 mmol) obtained in Example (187b) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). The resulting crude product was purified by silica gel column chromatography (NH, eluting solvent; methylene chloride : methanol = 24 : 1) to obtain 110 mg (yield: 73%) of the title compound as a colorless crystal.
Melting point: 86°C
¹H-NMR (500 MHz, CDCl₃) δ ppm: 8.37 (1H, s), 7.70 (1H, d, J = 8.8 Hz), 7.63 (1H, s), 7.47-7.44 (2H, m), 7.33 (1H, m), 7.22-7.16 (2H, m), 7.07 (1H, d, J = 7.8 Hz), 6.71 (1H, d, J = 8.8 Hz), 3.62 (4H, t, J = 5.1 Hz), 2.59 (3H, s), 2.53 (4H, t, J = 5.1 Hz), 2.36 (3H, s).
MS(FAB) m/z: 429 (M+H)⁺

### (Example 188) 1-(6-{2-Fluoro-5-[3-(6-methylpyridin-2-yl)-1H-pyrazol-4-yl]phenyl}pyridin-3-yl)-4-methylpiperazine (Compound No. 2-692)

### (188a) 1-(6-Bromopyridin-3-yl)-4-methylpiperazine

The same reaction as in Example (179a) was carried out using 1-methylpiperazine (0.78 mL, 6.9 mmol) instead of morpholine. The resulting crude product was purified by silica gel column chromatography (NH, eluting solvent; methylene chloride : ethyl acetate = 19 : 1) to obtain 1.1 g (yield: 53%) of the title compound as a light yellow powder.
¹H-NMR (400 MHz, CDCl₃) δ ppm: 8.03 (1H, m), 7.19-7.17 (2H, m), 3.22 (4H, t, J = 5.0 Hz), 2.57 (4H, t, J = 5.0 Hz), 2.36 (3H, s).

### (188b) 1-{6-[2-Fluoro-5-(3-(6-methylpyridin-2-yl)-1-{[2-(trimethylsilyl)ethoxy]methyl}-1H-pyrazol-4-yl)phenyl]pyridin-3-yl}-4-methylpiperazine

The same reaction as in Example (143f) was carried out using 1-(6-bromopyridin-3-yl)-4-methylpiperazine (300 mg, 1.4 mmol) obtained in Example (188a) instead of 5-bromothiophene-2-sulfonamide obtained in Example (143e). The resulting crude product was purified by silica gel column chromatography (NH, eluting solvent; hexane : ethyl acetate = 3 : 2) to obtain 230 mg (yield: 25%) of the title compound as a yellow oily material.
¹H-NMR (500 MHz, CDCl₃) δ ppm: 8.39 (1H, d, J = 1.0 Hz), 8.01 (1H, dd, J = 1.0, 6.4 Hz), 7.77 (1H, s), 7.67 (1H, m), 7.49 (1H, t, J = 7.6 Hz), 7.28-7.22 (3H, m), 7.07-7.01 (2H, m), 5.52 (2H, s), 3.67 (2H, t, J = 7.6 Hz), 3.31 (4H, t, J = 4.9 Hz), 2.60 (4H, t, J = 4.9 Hz), 2.54 (3H, s), 2.37 (3H, s), 1.26 (2H, t, J = 7.6 Hz), 0.00 (9H, s).

### (188c) 1-(6-{2-Fluoro-5-[3-(6-methylpyridin-2-yl)-1H-pyrazol-4-yl]phenyl}pyridin-3-yl)-4-methylpiperazine

The same reaction as in Example (143g) was carried out using 1-{6-[2-fluoro-5-(3-(6-methylpyridin-2-yl)-1-{[2-(trimethylsilyl)ethoxy]methyl}-1H-pyrazol-4-yl)phenyl]pyridin-3-yl}-4-methylpiperazine (230 mg, 0.42 mmol) obtained in Example (188b) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). The resulting crude compound was purified by silica gel column chromatography (NH, eluting solvent; methylene chloride : methanol = 24 : 1) to obtain 96 mg (yield: 54%) of the title compound as a colorless crystal. Melting point: 97°C
¹H-NMR (400 MHz, CDCl₃) δ ppm: 8.40 (1H, d, J = 2.8 Hz), 8.04 (1H, dd, J = 2.8, 8.5 Hz), 7.73 (1H, dd, J = 2.8, 8.5 Hz), 7.67 (1H, s), 7.42 (1H, t, J = 7.8 Hz), 7.33 (1H, m), 7.26 (1H, d, J = 10.9 Hz), 7.20-7.13 (2H, m), 7.05 (1H, d, J = 7.8 Hz), 3.31 (4H, t, J = 5.0 Hz), 2.60 (4H, t, J= 5.0 Hz), 2.58 (3H, s), 2.37 (3H, s).
MS(FAB) m/z: 429 (M+H)⁺.

### (Example 189) 2-(4-[4-Fluoro-3-(1H-pyrrol-3-yl)phenyl]-1H-pyrazol-3-yl}-6-methylpyridine (Compound No. 2-604)

### (189a) 4-(3-Bromo-4-fluorophenyl)-N,N-dimethyl-3-(6-methylpyridin-2-yl)-1H-pyrazol-1-sulfonamide

2-[4-(3-Bromo-4-fluorophenyl)-1H-pyrazol-3-yl]-6-methylpyridine (1.0 g, 3.0 mmol) obtained in Example (143b) was dissolved in chloroform (6 mL), and dimethylaminosulfonyl chloride (0.39 mL, 3.6 mmol) and triethylamine (0.63 mL, 4.5 mmol) were added thereto. The resulting mixture was heated under reflux for 18 hr. The reaction solution was cooled to room temperature, and saturated aqueous sodium bicarbonate was added thereto. After extraction with ethyl acetate, the organic layer was washed with brine and then dried with anhydrous sodium sulfate. The solvent was evaporated under reduced pressure, and the resulting crude product was purified by silica gel column chromatography (Yamazen, eluting solvent; hexane : ethyl acetate = 85 : 15 to 65 : 35) to obtain 0.80 g (yield: 61%) of the title compound as a light yellow solid.
¹H-NMR (400 MHz, CDCl₃) δ ppm: 8.05 (1H, s), 7.82 (1H, dd, J = 2.0, 6.7 Hz), 7.64-7.59 (2H, m) 7.30(1H, ddd, J = 2.4, 4.7, 8.6 Hz), 7.13 (1H, dd, J = 2.0, 6.7 Hz), 7.04 (1H, t, J = 8.6 Hz), 3.03 (6H, s), 2.45 (3H, s).

### (189b) 4-[4-Fluoro-3-(4,4,5,5-tetramethyl-1,3,2-dioxaborolan-2-yl)phenyl]-N,N-dimethyl-3-(6-methylpyridin-2-yl)-1H-pyrazol-1-sulfonamide

4-(3-Bromo-4-fluorophenyl)-N,N-dimethyl-3-(6-methylpyridin-2-yl)-1H-pyrazol-1-sulfonamide (0.80 g, 1.8 mmol) obtained in Example (189a) and bis(pinacolato)diboron (0.55 g, 2.3 mmol) were dissolved in N,N-dimethylformamide (20 mL), and potassium acetate (0.54 g, 5.4 mmol) and dichloro[1,1'-bis(diphenylphosphino)ferrocene]palladium(II)-methylene chloride complex (0.15 g, 0.18 mmol) were added thereto. The resulting mixture was stirred under a nitrogen atmosphere at 100°C for 4 hr. The reaction solution was cooled to room temperature, and then the reaction solvent was evaporated under reduced pressure. The resulting crude product was purified by silica gel column chromatography (Yamazen, eluting solvent; hexane : ethyl acetate = 75 : 25 to 55 : 45) to obtain 0.72 g (yield: 81%) of the title compound as a yellow solid.
¹H-NMR (400 MHz, CDC1₃) δ ppm: 8.10 (1H, s), 7.87 (1H, dd, J = 2.4, 5.4 Hz), 7.63-7.57 (2H, m) 7.44(1H, m), 7.12 (1H, d, J = 7.0 Hz), 6.96 (1H, t, J = 8.6 Hz), 3.03 (6H, s), 2.44 (3H, s), 1.35 (12H, s).

### (189c) t-Butyl 3-{5-[1-[(dimethylamino)sulfonyl]-3-(6-methylpyridin-2-yl)-1H-pyrazol-4-yl]-2-fluorophenyl}-1H-pyrrol-1-carboxylate

4-[4-Fluoro-3-(4,4,5,5-tetramethyl-1,3,2-dioxaborolan-2-yl)phenyl]-N,N-dimethyl-3-(6-methylpyridin-2-yl)-1H-pyrazol-1-sulfonamide (0.35 g, 0.72 mmol) obtained in Example (189b) and t-butyl 3-bromo-1H-pyrrol-1-carboxylate (0.27 g, 1.1 mmol) were dissolved in 1,2-dimethoxyethane (5.0 mL), and water (0.5 mL), tripotassium phosphate n-hydrate (0.30 g, 1.4 mmol), and dichloro[1,1'-bis(diphenylphosphino)ferrocene]palladium(II)-methylene chloride complex (0.059 g, 0.072 mmol) were added thereto. The resulting mixture was stirred under a nitrogen atmosphere at 90°C for 24 hr. The reaction solution was cooled to room temperature, and then the reaction solvent was evaporated under reduced pressure. The resulting crude product was purified by silica gel column chromatography (Yamazen, eluting solvent; hexane : ethyl acetate = 85 : 15 to 65 : 35) to obtain 0.22 g (yield: 57%) of the title compound as a light yellow solid.
¹H-NMR (400 MHz, CDCl₃) δ ppm: 8.08 (1H, s), 7.63-7.55 (3H, m), 7.33 (1H, m), 7.25 (1H, m), 7.18 (1H, m), 7.12 (1H, d, J = 7.4 Hz), 7.02 (1H, dd, J = 8.6, 10.6 Hz), 6.45(1H, m), 3.04 (6H, s), 2.43 (3H, s), 1.61 (9H, s).

### (189d) 2-{4-[4-Fluoro-3-(1H-pyrrol-3-yl)phenyl]-1H-pyrazol-3-yl}-6-methylpyridine

t-Butyl 3-{5-[1-[(dimethylamino)sulfonyl]-3-(6-methylpyridin-2-yl)-1H-pyrazol-4-yl]-2-fluorophenyl}-1H-pyrrol-1-carboxylate (0.22 g, 0.41 mmol) obtained in Example (189c) was dissolved in methanol (5.0 mL), and sodium methoxide (0.32 g, 6.0 mmol) was added thereto. The resulting mixture was heated under reflux for 24 hr and then cooled to room temperature. The reaction solvent was evaporated under reduced pressure, and a saturated ammonium chloride aqueous solution was added thereto. After extraction with ethyl acetate, the organic layer was washed with brine and then dried with anhydrous sodium sulfate. The solvent was evaporated under reduced pressure, and the resulting crude product was purified by silica gel column chromatography (Yamazen, eluting solvent; hexane : ethyl acetate = 40 : 60 to 20 : 80) and then by high-performance liquid chromatography (GL Science ODS-3, eluting solvent; water : acetonitrile = 95 : 5 to 5 : 95) to obtain 0.017 g (yield: 13%) of the title compound as a white solid.
Melting point: 162 to 164°C
¹H-NMR (400 MHz, CDCl₃) δ ppm: 8.37 (1H, brs), 7.65 (1H, s), 7.64 (1H, m), 7.43 (1H, t, J = 7.8 Hz), 7.31(1H, m), 7.21 (1H, d, J = 7.8 Hz), 7.16-7.10 (2H, m), 7.06 (1H, d, J = 7.8 Hz), 6.85 (1H, d, J = 2.4 Hz), 6.57 (1H, d, J = 2.4 Hz), 2.60 (3H, s). MS(ESI) m/z: 319 (M+H)⁺

### (Example 190) 2'-Fluoro-5'-[3-(6-methylpyridin-2-yl)-1H-pyrazol-4-yl]-1,1'-biphenyl-4-sulfonamide (Compound No. 2-444)

### (190a) 4-[4'-(Aminosulfonyl)-6-fluoro-1,1'-biphenyl-3-yl]-N,N-dimethyl-3-(6-methylpyridin-2-yl)-1H-pyrazole-1-sulfonamide

The same reaction as in Example (189c) was carried out using 4-bromobenzenesulfonamide (0.26 g, 1.1 mmol) instead of t-butyl 3-bromo-1H-pyrrol-1-carboxylate and using 4-[4-fluoro-3-(4,4,5,5-tetramethyl-1,3,2-dioxaborolan-2-yl)phenyl]-N,N-dimethyl-3-(6-methylpyridin-2-yl)-1H-pyrazol-1-sulfonamide (0.35 g, 0.72 mmol) obtained in Example (189b). After purification, 0.34 g (yield: 92%) of the title compound was obtained as a white solid.
¹H-NMR (400 MHz, CDCl₃) δ ppm: 8.10 (1H, s), 7.94 (2H, d, J = 7.4 Hz), 7.64 (1H, t, J = 7.6 Hz), 7.60 (1H, m), 7.59 (2H, d, J = 7.4 Hz), 7.49 (1H, d, J = 7.6 Hz), 7.39 (1H, m), 7.16-7.10 (2H, m), 5.25(2H, brs), 3.04 (6H, s), 2.42 (3H, s).

### (190b) 2'-Fluoro-5'-[3-(6-methylpyridin-2-yl)-1H-pyrazol-4-yl]-1,1'-biphenyl-4-sulfonamide

The same reaction as in Example (189d) was carried out using 4-[4'-(aminosulfonyl)-6-fluoro-1,1'-biphenyl-3-yl]-N,N-dimethyl-3-(6-methylpyridin-2-yl)-1H-pyrazole-1-sulfonamide (0.17 g, 0.33 mol) obtained in Example (190a) instead of t-butyl 3-{5-[1-[(dimethylamino)sulfonyl]-3-(6-methylpyridin-2-yl)-1H-pyrazol-4-yl]-2-fluorophenyl}-1H-pyrrol-1-carboxylate. After purification, 0.050 g (yield: 37%) of the title compound was obtained as a white solid.
Melting point: 115 to 118°C
¹H-NMR (400 MHz, CDCl₃) δ ppm: 7.97 (2H, d, J = 8.6 Hz), 7.67 (2H, d, J = 8.6 Hz), 7.63 (1H, s), 7.48 (1H, dd, J = 2.4, 7.4 Hz), 7.45 (1H, t, J = 7.8 Hz), 7.42 (1H, ddd, J = 2.4, 4.7, 8.2 Hz), 7.21 (1H, dd, J = 8.2, 10.6 Hz), 7.12 (1H, d, J = 7.8 Hz), 7.07 (1H, d, J = 7.8 Hz), 4.88 (2H, brs), 2.58 (3H, s).
MS(ESI) m/z: 409 (M+H)⁺.

### (Example 191) 2-{4-[6-Fluoro-4'-(methylsulfonyl)-1,1-biphenyl-3-yl]-1H-pyrazol-3-yl}-methylpyridine (Compound No. 2-412)

2-[4-(3-Bromo-4-fluorophenyl)-1H-pyrazol-3-yl]-6-methylpyridine (10 g, 30 mmol) obtained in Example (143b) and 4-(methylsulfonyl)phenylboronic acid (7.8 g, 39 mmol) were dissolved in 1,2-dimethoxyethane (150 mL), and water (30 mL), tripotassium phosphate n-hydrate (19 g, 90 mmol), and dichloro[1,1'-bis(diphenylphosphino)fenrocene]palladium(II)-methylene chloride complex (1.2 g, 1.5 mmol) were added thereto. The resulting mixture was stirred under a nitrogen atmosphere at 90°C for 48 hr. The reaction solution was cooled to room temperature, and the reaction solvent was evaporated under reduced pressure. The resulting crude product was purified by silica gel column chromatography (Yamazen, eluting solvent; hexane : ethyl acetate = 40 : 60 to 0 : 100) and then by high-performance liquid chromatography (GL Science ODS-3, eluting solvent; water : acetonitrile = 95 : 5 to 5 : 95) to obtain 5.9 g (yield: 48%) of the title compound as a white solid.
Melting point: 100 to 102°C
¹H-NMR (400 MHz, CDCl₃) δ ppm: 7.99 (2H, d, J = 8.6 Hz), 7.72 (2H, d, J = 8.6 Hz), 7.63 (1H, s), 7.50 (1H, dd, J = 2.0, 7.4 Hz), 7.45 (1H, t, J = 7.6 Hz), 7.42 (1H, m), 7.22 (1H, dd, J = 8.6, 10.6 Hz), 7.17 (1H, d, J = 7.6 Hz), 7.07 (1H, d, J = 7.6 Hz), 3.09 (3H, s), 2.58 (3H, s).
MS(ESI) m/z: 408 (M+H)⁺

### (Example 192) N-{2'-Fluoro-5'-[3-(6-methylpyridin-2-yl)-1H-pyrazol-4-yl]-1,1'-biphenyl-4-yl}-N'-morpholine-4-ylurea (Compound No. 2-550)

2-[4-(3-Bromo-4-fluorophenyl)-1H-pyrazol-3-yl]-6-methylpyridine (0.18 g, 0.54 mmol) obtained in Example (143b) and N-morpholin-4-yl-N'-[4-(4,4,5,5-tetramethyl-1,3,2-dioxaborolan-2-yl)phenyl]urea (0.21 g, 0.60 mmol) obtained in Example (67a) were dissolved in 1,2-dimethoxyethane (10 mL), and water (5 mL), a 2 M sodium carbonate aqueous solution (1.1 mL), and tetrakis(triphenylphosphine)palladium (0.031 g, 0.027 mmol) were added thereto. The resulting mixture was stirred under a nitrogen atmosphere at 90°C for 48 hr. The reaction solution was cooled to room temperature, and the reaction solvent was evaporated under reduced pressure. The resulting crude product was purified by silica gel column chromatography (Yamazen, eluting solvent; hexane/ethyl acetate) and then by high-performance liquid chromatography (GL Science ODS-3, eluting solvent; water : acetonitrile = 95 : 5 to 5 : 95) to obtain 0.030 g (yield: 12%) of the title compound as a white solid.
Melting point: 131 to 133°C
¹H-NMR (400 MHz, CDCl₃) δ ppm: 8.10 (1H, brs), 7.62 (1H, s), 7.55-7.53 (2H, m), 7.49-7.42 (4H, m), 7.31 (1H, ddd, J = 2.4, 4.3, 8.2 Hz), 7.18 (1H, dd, J = 7.8, 8.2 Hz), 7.14 (1H, d, J = 7.8 Hz), 7.05 (1H, d, J = 7.8 Hz), 5.34 (1H, s), 3.90 (2H, brs), 3.73 (2H, brs), 3.05 (2H, brs), 2.69 (2H, brs), 2.58 (3H, s).
MS(ESI) m/z: 473 (M+H)⁺

### (Example 193) 2- {4-[4-Fluoro-3-(1H-pyrrol-2-yl)phenyl]-1H-pyrazol-3-yl}-6-methylpyridine (Compound No. 2-476)

The same reaction as in Example 191 was carried out using 1-(t-butoxycarbonyl)pyrrole-2-boronic acid (0.17 g, 0.82 mmol) instead of 4-(methylsulfonyl)phenylboronic acid and using 2-[4-(3-bromo-4-fluorophenyl)-1H-pyrazol-3-yl]-6-methylpyridine (0.18 g, 0.54 mmol) obtained in Example (143b). After purification, a light yellow solid was obtained. The obtained light yellow solid was dissolved in tetrahydrofuran (5.0 mL), and a 25% sodium methoxide methanol solution (0.13 mL) was added thereto. The resulting mixture was stirred at room temperature for 4 hr. The reaction solvent was evaporated under reduced pressure, and a saturated ammonium chloride aqueous solution was added thereto. After extraction with ethyl acetate, the organic layer was washed with brine and then dried with anhydrous sodium sulfate. The solvent was evaporated under reduced pressure, and the resulting crude product was purified by silica gel column chromatography (Yamazen, eluting solvent; hexane/ethyl acetate) and then by high-performance liquid chromatography (GL Science ODS-3, eluting solvent; water : acetonitrile = 95 : 5 to 5 : 95) to obtain 0.015 g (yield: 25%) of the title compound as a white solid.
Melting point: 141 to 142°C
¹H-NMR (400 MHz, CDCl₃) δ ppm: 9.08 (1H, brs), 7.68 (1H, dd, J = 2.0, 7.8 Hz), 7.64 (1H, s), 7.43 (1H, t, J = 7.8 Hz), 7.20-7.12 (3H, m), 7.06 (1H, d, J = 7.8 Hz), 6.92 (1H, m), 6.59 (1H, m), 6.30 (1H, m), 2.59 (3H, s).
MS(ESI) m/z: 319 (M+H)⁺

### (Example 194) 2-{4-[4-Fluoro-3-(1H-pyrazol-4-yl)phenyl]-1H-pyrazol-3-yl}-6-methylpyridine (Compound No. 2-598)

The same reaction as in Example 191 was carried out using 1-(t-butoxycarbonyl)-4-(4,4,5,5-tetramethyl-1,3,2-dioxaborolan-2-yl)pyrazole (0.44 g, 1.5 mmol) instead of 4-(methylsulfonyl)phenylboronic acid and using 2-[4-(3-bromo-4-fluorophenyl)-1H-pyrazol-3-yl]-6-methylpyridine (0.33 g, 1.0 mmol) obtained in Example (143b). After purification, 0.040 g (yield: 13%) of the title compound was obtained as a white solid.
Melting point: 110 to 112°C
¹H-NMR (400 MHz, CDCl₃) δ ppm: 7.95 (2H, d, J = 2.0 Hz), 7.64 (1H, s), 7.61 (1H, dd, J = 2.0, 7.4 Hz), 7.42 (1H, t, J = 7.8 Hz), 7.23(1H, m), 7.16 (1H, d, J = 7.8 Hz), 7.15 (1H, m), 7.05 (1H, d, J = 7.8 Hz), 2.58 (3H, s).
MS(ESI) m/z: 320 (M+H)⁺

### (Example 195) 2-{4-[4-Fluoro-3-(1-methyl-1H-pyrazol-4-yl)phenyl]-1H-pyrazol-3-yl}-6-methylpyridine (Compound No. 2-600)

The same reaction as in Example 191 was carried out using 1-methyl-4-(4,4,5,5-tetramethyl-1,3,2-dioxaborolan-2-yl)-1H-pyrazole (0.31 g, 1.5 mmol) instead of 4-(methylsulfonyl)phenylboronic acid and using 2-[4-(3-bromo-4-fluorophenyl)-1H-pyrazol-3-yl]-6-methylpyridine (0.33 g, 1.0 mmol) obtained in Example (143b). After purification, 0.10 g (yield: 30%) of the title compound was obtained as a white solid.
Melting point: 155 to 156°C
¹H-NMR (400 MHz, CDCl₃) δ ppm: 7.80 (1H, s), 7.78 (1H, d, J = 2.4 Hz), 7.64 (1H, s), 7.60 (1H, dd, J = 2.4, 7.4 Hz), 7.43 (1H, t, J = 7.8 Hz), 7.22(1H, ddd, J = 2.4, 4.7, 8.2 Hz), 7.17 (1H, d, J = 7.8 Hz), 7.15 (1H, dd, J = 8.2, 10.6 Hz), 7.05 (1H, d, J = 7.8 Hz), 3.96 (3H, s), 2.59 (3H, s).
MS(ESI) m/z: 334 (M+H)⁺.

### (Example 196) 2'-Fluoro-5'-[3-(6-methylpyridin-2-yl)-1H-pyrazol-4-yl]-N-morpholin-4-yl-1,1'-biphenyl-4-carboxyamide (Compound No. 2-452)

The same reaction as in Example 192 was carried out using N-morpholin-4-yl-4-(4,4,5,5-tetramethyl-1,3,2-dioxaborolan-2-yl)benzamide (0.33 g, 1.0 mmol) obtained in Example (66a) instead of N-morpholin-4-yl-N'-[4-(4,4,5,5-tetramethyl-1,3,2-dioxaborolan-2-yl)phenyl]urea obtained in Example (67a) and using 2-[4-(3-bromo-4-fluorophenyl)-1H-pyrazol-3-yl]-6-methylpyridine (0.30 g, 0.91 mmol) obtained in Example (143b). After purification, 0.018 g (yield: 4%) of the title compound was obtained as a white solid.
Melting point: 170 to 172°C
¹H-NMR (400 MHz, CDCl₃) δ ppm: 8.09 (1H, brs), 7.80 (2H, d, J = 7.8 Hz), 7.63 (1H, s), 7.57 (2H, d, J = 7.8 Hz), 7.45 (1H, t, J = 7.8 Hz), 7.37 (1H, m), 7.20-7.16 (2H, m), 7.06 (1H, d, J = 7.8 Hz), 6.77 (1H, d, J = 7.8 Hz), 3.85 (2H, brs), 3.80 (2H, brs), 2.98 (2H, brs), 2.91 (2H, brs), 2.57 (3H, s).
MS(ESI) m/z: 458 (M+H)⁺

### (Example 197) 2'-Fluoro-5'-[3-(6-methylpyridin-2-yl)-1H-pyrazol-4-yl]-1,1'-biphenyl-4-ol (Compound No. 2-602)

The same reaction as in Example 191 was carried out using 4-hydroxyphenylboronic acid (0.10 g, 0.75 mmol) instead of 4-(methylsulfonyl)phenylboronic acid and using 2-[4-(3-bromo-4-fluorophenyl)-1H-pyrazol-3-yl]-6-methylpyridine (0.17 g, 0.50 mmol) obtained in Example (143b). After purification, 0.030 g (yield: 17%) of the title compound was obtained as a white solid.
Melting point: 72 to 74°C
¹H-NMR (400 MHz, CDCl₃) δ ppm: 7.64 (1H, s), 7.48-7.43 (4H, m), 7.31 (1H, ddd, J = 2.4, 4.7, 8.6 Hz), 7.22-7.15 (2H, m), 7.07 (1H, d, J = 7.8 Hz), 6.90 (2H, d, J = 8.6 Hz), 2.59 (3H, s).
MS(ESI) m/z: 346 (M+H)⁺

### (Example 198) 2-{4-[3-(1-Ethyl-1H-pyrazol-4-yl)-4-fluorophenyl]-1H-pyrazol-3-yl}-6-methylpyridine (Compound No. 2-922)

2-[4-(3-Bromo-4-fluorophenyl)-1H-pyrazol-3-yl]-6-methylpyridine (0.23 g, 0.68 mmol) obtained in Example (143b) and 1-ethyl-4-(4,4,5,5-tetramethyl-1,3,2-dioxaborolan-2-yl)-1H-pyrazole (0.23 g, 1.0 mmol) obtained in Example (107a) were dissolved in 1,2-dimethoxyethane (5 mL), and tripotassium phosphate n-hydrate (0.30 g, 1.4 mmol) and dichloro[1,1'-bis(diphenylphosphino)ferrocene]palladium(II)-methylene chloride complex (0.056 g, 0.069 mmol) were added thereto. The resulting mixture was stirred under a nitrogen atmosphere for 2 hr at 100°C in a microwave. Water (0.5 mL) and tetrakis(triphenylphosphine)palladium (0.039 mg, 0.034 mmol) were added thereto, and the resulting mixture was further stirred for 2 hr at 100°C in the microwave. The reaction solution was cooled to room temperature, and water was added thereto. After extraction with ethyl acetate, the organic layer was washed with water and brine, and then dried with anhydrous sodium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.0050 g (yield: 2.0%) of the title compound as a light yellow amorphous form.
¹H-NMR (400 MHz, CDCl₃) δ ppm: 7.83-7.78 (2H, m), 7.63 (1H, s), 7.59 (1H, dd, J = 2.0, 7.4 Hz), 7.41 (1H, t, J = 7.4 Hz), 7.22-7.09 (3H, m), 7.04 (1H, d, J = 7.4 Hz), 4.21 (2H, q, J = 7.4 Hz), 2.56 (3H, s), 1.53 (3H, t, J = 7.4 Hz).
MS(ESI) m/z: 348 (M+H)⁺

### (Example 199) 2-{4-[6-Fluoro-4'-(methylsulfonyl)-1,1'-biphenyl-3-yl]-1-methyl-1H-pyrazol-3-yl}-6-methylpyridine (Compound No. 2-413)

### (199a) 2-[4-(3-Bromo-4-fluorophenyl)-1-methyl-1H-pyrazol-3-yl]-6-methylpyridine

2-[4-(3-Bromo-4-fluorophenyl)-1H-pyrazol-3-yl]-6-methylpyridine (0.17 g, 0.51 mmol) obtained in Example (143b) was dissolved in N,N-dimethylformamide (2.0 mL), and sodium hydride (55%, oil, 0.027 g, 0.67 mmol) was added thereto. The resulting mixture was stirred for 30 min. The reaction solution was cooled to 0°C, and iodomethane (0.047 mL, 0.76 mmol) was added thereto. The mixture was stirred for 1 hr. To this reaction solution, a saturated ammonium chloride aqueous solution was added. After extraction with ethyl acetate, the organic layer was washed with brine and then dried with anhydrous sodium sulfate. The solvent was evaporated under reduced pressure, and the resulting crude product was purified by silica gel column chromatography (Yamazen, eluting solvent: hexane/ethyl acetate) to obtain 0.10 g (yield: 61%) of the title compound as a light yellow solid.
¹H-NMR (400 MHz, CDCl₃) δ ppm: 7.69 (1H, dd, J = 2.4, 6.8 Hz), 7.52 (1H, t, J = 7.8 Hz), 7.47 (1H, s), 7.27 (1H, d, J = 7.8 Hz), 7.23 (1H, ddd, J = 2.4, 4.4, 8.3 Hz), 7.07 (1H, d, J = 7.8 Hz), 7.03 (1H, t, J = 8.3 Hz), 4.00 (3H, s), 2.56 (3H, s). (199b) 2-{4-[6-Fluoro-4'-(methylsulfonyl)-1,1'-biphenyl-3-yl]-1-methyl-1H-pyrazol-3-yl}-6-methylpyridine

The same reaction as in Example 191 was carried out using 2-[4-(3-bromo-4-fluorophenyl)-1-methyl-1H-pyrazol-3-yl]-6-methylpyridine (0.10 g, 0.29 mmol) obtained in Example (199a) instead of 2-[4-(3-bromo-4-fluorophenyl)-1H-pyrazol-3-yl]-6-methylpyridine. After purification, 0.090 g (yield: 90%) of the title compound was obtained as a white solid.
Melting point: 66 to 67°C
¹H-NMR (400 MHz, CDCl₃) δ ppm: 7.96 (2H, d, J = 8.6 Hz), 7.65 (2H, d, J = 8.6 Hz), 7.51 (1H, t, J = 7.8 Hz), 7.51 (1H, s), 7.45 (1H, dd, J = 2.0, 7.4 Hz), 7.35 (1H, ddd, J = 2.0, 4.7, 8.6 Hz), 7.27 (1H, d, J = 7.8 Hz), 7.12 (1H, d, J = .7.8 Hz), 7.11 (1H, dd, J = 8.6, 10.6 Hz), 4.01 (3H, s), 3.09 (3H, s), 2.53 (3H, s).
MS(ESI) m/z: 422 (M+H)⁺

### (Example 200) 2-{4-[6-Fluoro-4'-(methylsulfonyl)-1,1'-biphenyl-3-yl]-1-isopropyl-1H-pyrazol-3-yl}-6-methylpyridine (Compound No. 2-109)

### (200a) 2-[4-(3-Bromo-4-fluorophenyl)-1-isopropyl-1H-pyrazol-3-yl]-6-methylpyridine

The same reaction as in Example (199a) was carried out using 2-iodopropane (0.070 mL, 0.70 mmol) instead of iodomethane and using 2-[4-(3-bromo-4-fluorophenyl)-1H-pyrazol-3-yl]-6-methylpyridine (0.16 g, 0.47 mmol) obtained in Example (143b). After purification, 0.11 g (yield: 63%) of the title compound was obtained as a light yellow solid.
¹H-NMR (400 MHz, CDCl₃) δ ppm: 7.73 (1H, dd, J = 2.4, 6.8 Hz), 7.55 (1H, s), 7.54 (1H, t, J = 7.8 Hz), 7.35 (1H, d, J = 7.8 Hz), 7.25 (1H, ddd, J = 2.0, 4.9, 8.3 Hz), 7.07 (1H, d, J = 7.8 Hz), 7.01 (1H, t, J = 8.3 Hz), 4.63 (1H, hp, J = 6.8 Hz), 2.53 (3H, s), 1.57 (6H, d, J = 6.8 Hz).

### (200b) 2-{4-[6-Fluoro-4'-(methylsulfonyl)-1,1'-biphenyl-3-yl]-1-isopropyl-1H-pyrazol-3-yl} -6-methylpyridine

The same reaction as in Example 191 was carried out using 2-[4-(3-bromo-4-fluorophenyl)-1-isopropyl-1H-pyrazol-3-yl]-6-methylpyridine (0.11 g, 0.29 mmol) obtained in Example (200a) instead of 2-[4-(3-bromo-4-fluorophenyl)-1H-pyrazol-3-yl]-6-methylpyridine. After purification, 0.11 g (yield: 100%) of the title compound was obtained as a white solid.
Melting point: 172 to 173°C
¹H-NMR (400 MHz, CDCl₃) δ ppm: 7.96 (2H, d, J = 8.2 Hz), 7.66 (2H, d, J = 8.2 Hz), 7.57 (1H, s), 7.53 (1H, t, J = 7.4 Hz), 7.48 (1H, dd, J = 2.0, 7.4 Hz), 7.37 (1H, m), 7.10 (1H, dd, J = 8.6, 10.6 Hz), 7.35 (1H, d, J = 7.4 Hz), 7.07 (1H, d, J = 7.4 Hz), 4.64 (1H, hp, J = 6.8 Hz), 3.09 (3H, s), 2.50 (3H, s), 1.59 (6H, d, J = 6.8 Hz). MS(ESI) m/z: 450 (M+H)⁺.

### (Example 201) 2-{4-[4-Fluoro-3-(1-methyl-1H-pyrazol-4-yl)phenyl]-1H-pyrazol-3-yl}pyridine (Compound No. 2-622)

### (201a) 2-(3-Bromo-4-fluorophenyl)-1-pyridin-2-ylethanone

The same reaction as in Example (143a) was carried out using 2-pyridinecarboxyaldehyde (12 g, 0.11 mmol) instead of 6-methylpyridine-2-carboxyaldehyde. After purification, 29 g (yield: 89%) of the title compound was obtained as a yellow oily material.
¹H-NNR (400 MHz, CDCl₃) δ ppm: 8.74 (1H, d, J = 4.3 Hz), 8.07 (1H, d, J = 7.8 Hz), 7.86 (1H, m), 7.57-7.49 (2H, m), 7.24 (1H, m), 7.07 (1H, t, J = 8.6 Hz), 4.51 (2H, s).

### (201b) 2-[4-(3-Bromo-4-fluorophenyl)-1H-pyrazol-3-yl]-pyridine

2-(3-Bromo-4-fluorophenyl)-1-pyridin-2-ylethanone (29 g, 0.10 mmol) obtained in Example (201 a) was dissolved in tetrahydrofuran (1.0 L), and N,N-dimethylformamide dimethylacetal (51 g, 0.43 mol) was added thereto at room temperature. The resulting mixture was stirred at 90°C for 3 hr. The reaction solution was cooled to room temperature and evaporated under reduced pressure to obtain a reddish brown oily material. The obtained reddish brown oily material was dissolved in ethanol (0.60 L), and hydrazine monohydrate (16 mL, 0.32 mol) was added thereto. The resulting mixture was stirred at room temperature for 5 hr. The reaction solution was evaporated under reduced pressure, and the resulting crude product was purified by silica gel column chromatography (eluting solvent; acetone : methylene chloride = 1 : 6) to obtain 19 g (yield: 55%) of the title compound as an orange solid.
¹H-NMR (400 MHz, CDCl₃) δ ppm: 8.61 (1H, d, J = 4.3 Hz), 7.67-7.57 (3H, m), 7.36-7.12 (4H, m).

### (201c) 2-(4-(3-Bromo-4-fluorophenyl)-1-{[2-(trimethylsilyl)ethoxy]methyl}-1H-pyrazol-3-yl)pyridine

The same reaction as in Example (143c) was carried out using 2-[4-(3-bromo-4-fluorophenyl)-1H-pyrazol-3-yl]pyridine (5.0 g, 16 mmol) obtained in Example (201b) instead of 2-[4-(3-bromo-4-fluorophenyl)-1H-pyrazol-3-yl]-6-methylpyridine obtained in Example (143b). After purification, 6.1 g (yield: 86%) of the title compound was obtained as a yellow oily material.
¹H-NMR (400 MHz, CDCl₃) δ ppm: 8.60 (1H, d, J = 4.7 Hz), 7.69 (1H, s), 7.66 (1H, dd, J = 1.6,7.8 Hz), 7.62-7.55 (2H, m), 7.27-7.19 (2H, m), 7.05 (1H, t, J = 7.8 Hz), 5.52 (2H, s), 3.69 (2H, t, J = 8.2 Hz), 0.98 (2H, t, J = 8.2 Hz), 0.02 (9H, s).

### (201d) 2-(4-[4-Fluoro-3-(1-methyl-1H-pyrazol-4-yl)phenyl]-1-{[2-(trimethylsilyl)ethoxy]methyl}-1H-pyrazol-3-yl)pyridine

The same reaction as in Example 191 was carried out using 2-(4-(3-bromo-4-fluorophenyl)-1-{[2-(trimethylsilyl)ethoxy]methyl}-1H-pyrazol-3-yl)pyridine (0.24 g, 0.53 mmol) obtained in Example (201c) instead of 2-[4-(3-bromo-4-fluorophenyl)-1H-pyrazol-3-yl]-6-methylpyridine obtained in Example (143b) and using 1-methyl-4-(4,4,5,5-tetramethyl-1,3,2-dioxaborolan-2-yl)-1H-pyrazole (0.17 g, 0.80 mmol) instead of 4-(methylsulfonyl)phenylboronic acid. After purification, 0.16 g (yield: 68%) of the title compound was obtained as a light yellow oily material.
¹H-NMR (400 MHz, CDCl₃) δ ppm: 8.65 (1H, m), 7.74-7.64 (3H, m), 7.65 (1H, m), 7.57 (1H, m), 7.54 (1H, d, J = 7.8 Hz), 7.22 (1H, m), 7.15 (1H, m), 7.06 (1H, m), 5.54 (2H, s), 3.94 (3H, s), 3.70 (2H, t, J = 7.8 Hz), 0.97 (2H, t, J = 7.8 Hz), 0.00 (9H, s).

### (201 e) 2-{4-[4-Fluoro-3-(1-methyl-1H-pyrazol-4-yl)phenyl]-1H-pyrazol-3-yl}pyridine

The same reaction as in Example (143g) was carried out using 2-(4-[4-fluoro-3-(1-methyl-1H-pyrazol-4-yl)phenyl]-1-{[2-(trimethylsilyl)ethoxy]methyl}-1H-pyrazol-3-yl)pyridine (0.16 g, 0.36 mmol) obtained in Example (201d) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). After purification, 0.056 g (yield: 48%) of the title compound was obtained as a white solid. Melting point: 134 to 136°C
¹H-NMR (400 MHz, CDCl₃) δ ppm: 8.65 (1H, d, J = 4.7 Hz), 7.80 (1H, s), 7.79 (1H, d, J = 2.4 Hz), 7.66 (1H, s), 7.61 (1H, dd, J = 2.4, 7.4 Hz), 7.56 (1H, m), 7.38 (1H, d, J = 7.8 Hz), 7.26-7.12 (3H, m), 3.96 (3H, s).
MS(ESI) m/z: 320 (M+H)⁺

### (Example 202) 2'-Fluoro-5'-(3-pyridin-2-yl-1H-pyrazol-4-yl)-1,1'-biphenyl-4-sulfonamide (Compound No. 2-654)

### (202a) 4-(4,4,5,5-Tetramethyl-1,3,2-dioxaborolan-2-yl)benzenesulfonamide

The same reaction as in Example (143d) was carried out using 4-bromobenzenesulfonamide (11 g, 47 mmol) instead of 2-(4-(3-bromo-4-fluorophenyl)-1- {[2-(trimethylsilyl)ethoxy]methyl}-1H-pyrazol-3-yl)-6-methylpyridine obtained in Example (143c). After purification, 7.1 g (yield: 53%) of the title compound was obtained as a white solid.
¹H-NMR (400 MHz, CDCl₃) δ ppm: 7.94-7.86 (4H, m), 4.87 (2H, brs), 1.36 (12H, s).

### (202b) 2'-Fluoro-5'-(3-pyridin-2-yl-1-{[2-(trimethylsilyl)ethoxy]methyl}-1H-pyrazol-4-yl)-1,1'-biphenyl-4-sulfonamide

The same reaction as in Example 191 was carried out using 2-(4-(3-bromo-4-fluorophenyl)-1-{[2-(trimethylsilyl)ethoxy]methyl}-1H-pyrazol-3-yl)pyridine (0.25 g, 0.56 mmol) obtained in Example (201c) instead of 2-[4-(3-bromo-4-fluorophenyl)-1H-pyrazol-3-yl]-6-methylpyridine obtained in Example (143b) and using 4-(4,4,5,5-tetramethyl-1,3,2-dioxaborolan-2-yl)benzenesulfonamide (0.24 g, 0.84 mmol) obtained in Example (202a) instead of 4-(methylsulfonyl)phenylboronic acid. After purification, 0.11 g (yield: 38%) of the title compound was obtained as a light yellow oily material.
¹H-NMR (400 MHz, CDCl₃) δ ppm: 8.60 (1H, d, J = 4.4 Hz), 7.94-7.89 (2H, m), 7.75-7.05 (9H, m), 5.51 (2H, s), 5.27 (2H, brs), 3.69 (2H, t, J = 7.8 Hz), 0.95 (2H, t, J = 7.8 Hz), 0.00 (9H, s).

### (202c) 2'-Fluoro-5'-(3-pyridin-2-yl-1H-pyrazol-4-yl)-1,1'-biphenyl-4-sulfonamide

The same reaction as in Example (143g) was carried out using 2'-fluoro-5'-(3-pyridin-2-yl-1-{[2-(trimethylsilyl)ethoxy]methyl}-1H-pyrazol-4-yl)-1,1'-biphenyl-4-sulfonamide (0.11 g, 0.21 mmol) obtained in Example (202b) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). After purification, 0.044 g (yield: 52%) of the title compound was obtained as a white solid.
Melting point: 128°C
¹H-NMR (400 MHz, CDCl₃) δ ppm: 8.61 (1H, d, J = 4.7 Hz), 7.98-7.92 (2H, m), 7.68-7.58 (4H, m), 7.45 (1H, dd, J = 2.4, 7.4 Hz), 7.43-7.35 (2H, m), 7.27-7.15 (2H, m), 2.38 (2H, brs).
MS(ESI) m/z: 395 (M+H)⁺.

### (Example 203) 2-Cyclopropyl-6-{4-[6-fluoro-4'-(methylsulfonyl)-1,1'-biphenyl-3-yl]-1H-pyrazol-3-yl}pyridine (Compound No. 2-610)

### (203 a) 2-(3-Bromo-4-fluorophenyl)-1-(6-bromopyridin-2-yl)ethanone

The same reaction as in Example (143a) was carried out using 6-bromopyridine-2-carboxyaldehyde (9.3 g, 50 mmol) instead of 6-methylpyridine-2-carboxyaldehyde. After purification, 11 g (yield: 58%) of the title compound was obtained as a yellow solid.
¹H-NMR (400 MHz, CDCl₃) δ ppm: 7.98 (1H, dd, J = 2.0, 6.6 Hz), 7.71-7.65 (2H, m), 7.52 (1H, dd, J = 2.0, 6.6 Hz), 7.22 (1H, m), 7.05 (1H, t, J = 8.6 Hz), 4.44 (2H, s).

### (203b) 2-Bromo-6-[4-(3-bromo-4-fluorophenyl)-1H-pyrazol-3-yl]pyridine

The same reaction as in Example (143b) was carried out using 2-(3-bromo-4-fluorophenyl)-1-(6-bromopyridin-2-yl)ethanone obtained in Example (203 a) instead of 2-(3-bromo-4-fluorophenyl)-1-(6-methylpyridin-2-yl)ethanone obtained in Example (143a). After purification, 8.9 g (yield: 77%) of the title compound was obtained as a white solid.
¹H-NMR (400 MHz, CDCl₃) δ ppm: 7.61 (1H, dd, J = 2.4, 6.6 Hz), 7,60 (1H, s), 7.43 (1H, t, J = 7.8 Hz), 7.38 (1H, d, J = 7.8 Hz), 7.29 (1H, ddd, J = 2.4, 4.7, 8.6 Hz), 7.26 (1H, d, J = 7.8 Hz), 7.15 (1H, t, J = 8.6 Hz).

### (203c) 2-Bromo-6-(4-(3-bromo-4-fluorophenyl)-1-{[2-(trimethylsilyl)ethoxy]methyl}-1H-pyrazol-3-yl)pyridine and 2-bromo-6-(4-(3-bromo-4-fluorophenyl)-1-{[2-(trimethylsilyl)ethoxy]methyl}-1H-pyrazol-5-yl)pyridine

The same reaction as in Example (143c) was carried out using 2-bromo-6-[4-(3-bromo-4-fluorophenyl)-1H-pyrazol-3-yl]pyridine (1.0 g, 2.5 mmol) obtained in Example (203b) instead of 2-[4-(3-bromo-4-fluorophenyl)-1H-pyrazol-3-yl]-6-methylpyridine obtained in Example (143b). After purification, 1.3 g (yield: 97%) of a mixture of the title compound was obtained as a light yellow solid.
MS(ESI) m/z: 528 (M+H)⁺

### (203d) 2-(4-(3-Bromo-4-fluorophenyl)-1-{[2-(trimethylsilyl)ethoxy]methyl}-1H-pyrazol-3-yl)-6-cyclopropylpyridine and 2-(4-(3-bromo-4-fluorophenyl)-1-{[2-(trimethylsilyl)ethoxy]methyl}-1H-pyrazol-5-yl)-6-cyclopropylpyridine

A mixture (0.75 g, 1.4 mmol) of 2-bromo-6-(4-(3-bromo-4-fluorophenyl)-1-{[2-(trimethylsilyl)ethoxy]methyl}-1H-pyrazol-3-yl)pyridine and 2-bromo-6-(4-(3-bromo-4-fluorophenyl)-1-{[2-(trimethylsilyl)ethoxy]methyl}-1H-pyrazol-5-yl)pyridine obtained in Example (203c) and cyclopropylboronic acid (0.16 g, 1.9 mmol) were dissolved in toluene (10 mL), and water (0.50 mL), tripotassium phosphate n-hydrate (1.1 g, 5.0 mmol), palladium acetate (0.032 g, 0.14 mmol), and tricyclohexylphosphine (0.080 g, 0.29 mmol) were added thereto. The resulting mixture was heated under a nitrogen atmosphere under reflux for 24 hr. The reaction solution was cooled to room temperature, and then water was added thereto. After extraction with ethyl acetate, the organic layer was washed with brine and then dried with anhydrous sodium sulfate. The solvent was evaporated under reduced pressure, and the resulting crude product was purified by silica gel column chromatography (Yamazen, eluting solvent; hexane : ethyl acetate = 100 : 0 to 85 : 15) to obtain 0.45 g (yield: 65%) of a mixture of the title compound as a light yellow oily material.
MS(ESI) m/z: 489 (M+H)⁺

### (203e) 2-Cyclopropyl-6-(4-[6-fluoro-4'-(methylsulfonyl)-1,1'-biphenyl-3-yl]-1-{[2-(trimethylsilyl)ethoxy]methyl}-1H-pyrazol-3-yl)pyridine and 2-cyclopropyl-6-(4-[6-fluoro-4'-(methylsulfonyl)-1,1'-biphenyl-3-yl]-1-{[2-(trimethylsilyl)ethoxy]methyl}-1H-pyrazol-5-yl)pyridine

The same reaction as in Example 191 was carried out using a mixture (0.27 g, 0.55 mmol) of 2-(4-(3-bromo-4-fluorophenyl)-1-{[2-(trimethylsilyl)ethoxy]methyl}-1H-pyrazol-3-yl)-6-cyclopropylpyridine and 2-(4-(3-bromo-4-fluorophenyl)-1-{[2-(trimethylsilyl)ethoxy]methyl}-1H-pyrazol-5-yl)-6-cyclopropylpyridine obtained in Example (203d) instead of 2-[4-(3-bromo-4-fluorophenyl)-1H-pyrazol-3-yl]-6-methylpyridine obtained in Example (143b). After purification, 0.24 g (yield: 77%) of a mixture of the title compound was obtained as a white solid.
MS(ESI) m/z: 564 (M+H)⁺

### (203f) 2-Cyclopropyl-6-{4-[6-fluoro-4'-(methylsulfonyl)-1,1'-biphenyl-3-yl]-1H-pyrazol-3-yl}pyridine

The same reaction as in Example (143g) was carried out using a mixture (0.20 g, 0.35 mmol) of 2-cyclopropyl-6-(4-[6-fluoro-4'-(methylsulfonyl)-1,1'-biphenyl-3-yl]-1-{[2-(trimethylsilyl)ethoxy]methyl}-1H-pyrazol-3-yl)pyridine and 2-cyclopropyl-6-(4-[6-fluoro-4'-(methylsulfonyl)-1,1'-biphenyl-3-yl]-1-{[2-(trimethylsilyl)ethoxy]methyl}-1H-pyrazol-5-yl)pyridine obtained in Example (203e) instead of 5-[2-fluoro-5-(3-(6-methylpyridin-2-yl)-1-{[2-(trimethylsilyl)ethoxy]methyl}-1H-pyrazol-4-yl)phenyl]thiophene-2-sulfonamide obtained in Example (143f). After purification, 0.070 g (yield: 45%) of the title compound was obtained as a white solid.
Melting point: 102 to 104°C
¹H-NMR (400 MHz, CDCl₃) δ ppm: 7.99 (2H, d, J = 8.6 Hz), 7.73 (2H, d, J = 8.6 Hz), 7.61 (1H, s), 7.48 (1H, dd, J = 2.4, 7.0 Hz), 7.42 (1H, m), 7.41 (1H, t, J = 7.8 Hz), 7.24 (1H, m), 7.13 (1H, d, J = 7.8 Hz), 7.06 (1H, d, J = 7.8 Hz), 3.09 (3H, s), 2.07 (1H, m), 1.05-1.01 (4H, m).
MS(ESI) m/z: 434 (M+H)⁺.

### (Example 204) 5'-[3-(6-Cyclopropylpyridin-2-yl)-1H-pyrazol-4-yl]-2'-fluoro-1,1'-biphenyl-4-sulfonamide (Compound No. 2-624)

The same reaction as in Example (143b) was carried out using a mixture (200 mg, 0.44 mmol) of 2-(4-(3-bromo-4-fluorophenyl)-1-{[2-(trimethylsilyl)ethoxy]methyl}-1H-pyrazol-3-yl)-6-cyclopropylpyridine and 2-(4-(3-bromo-4-fluorophenyl)-1-{[2-(trimethylsilyl)ethoxy]methyl}-1H-pyrazol-5-yl)-6-cyclopropylpyridine obtained in Example (203d) and 4-(4,4,5,5-tetramethyl-1,3,2-dioxaborolan-2-yl)benzenesulfonamide (130 mg, 0.44 mmol) obtained in Example (202a). The reaction solvent was evaporated under reduced pressure, and the residue was purified by silica gel column chromatography (hexane : ethyl acetate = 4 : 1) to obtain 140 mg (yield: 56%) of a protected compound of the title compound as a yellow amorphous form. The obtained compound in a protected compound (140 mg, 0.25 mmol) was dissolved in ethanol (6 mL), and a 3 N hydrochloric acid aqueous solution (3 mL) was added thereto. The resulting mixture was stirred at room temperature for 1 hr and then at 80°C for 12 hr. The reaction solution was cooled to room temperature, and saturated aqueous sodium bicarbonate was added thereto. After extraction with ethyl acetate, the organic layer was washed with water and brine, and then dried with anhydrous magnesium sulfate. The solvent was evaporated under reduced pressure, and the resulting crude product was purified by silica gel column chromatography (hexane : ethyl acetate = 2 : 1) to obtain 63 mg (yield: 58%) of the title compound as a white solid.
Melting point: 225 to 226°C
¹H NMR (400 MHz, CDCl₃) δ ppm: 7.94 (2H, d, J = 6.7 Hz), 7.61 (1H, s), 7.58 (2H, d, J = 6.7 Hz), 7.40-7.36 (4H, m), 7.15 (1H, d, J = 10.2 Hz), 7.11 (1H, d, J = 7.8 Hz), 7.02 (1H, d, J = 7.8 Hz), 5.70 (2H, s), 1.99 (1H, m), 0.99 (2H, d, J = 5.9 Hz), 0.93 (2H, d, J = 5.9 Hz).
MS(ESI) m/z: 435 (M+H)⁺.

### (Example 205) 2-{4-[4-Fluoro-3-(1H-pyrazol-3-yl)phenyl]-1H-pyrazol-3-yl}-6-methylpyridine (Compound No. 2-606)

### (205a) 2-Fluoro-5-[3-(6-methylpyridin-2-yl)-1H-pyrazol-4-yl]benzonitrile

The same reaction as in Example (143b) was carried out using 2-fluoro-5-[2-(6-methylpyridin-2-yl)-2-oxoethyl]benzonitrile (10 g, 41 mmol) obtained in Example (131 a) instead of 2-(3-bromo-4-fluorophenyl)-1-(6-methylpyridin-2-yl)ethanone obtained in Example (143a). After purification, 7.1 g (yield: 63%) of the title compound was obtained as a white solid.
¹H-NMR (500 MHz, CDCl₃) δ ppm: 7.72 (1H, m), 7.65 (1H, m), 7.63 (1H, s), 7.50 (1H, m), 7.25 (1H, m), 7.12 (1H, d, J = 7.8 Hz), 7.05 (1H, m), 2.58 (3H, s).
MS(ESI) m/z: 279 (M+H)⁺

### (205b) 1-{2-Fluoro-5-[3-(6-methylpyridin-2-yl)-1H-pyrazol-4-yl]phenyl}ethanone

A methylmagnesium bromide solution (3.0 M in diethylether, 7.5 mL, 23 mmol) was added to a tetrahydrofuran solution (50 mL) of 2-fluoro-5-[3-(6-methylpyridin-2-yl)-1H-pyrazol-4-yl]benzonitrile (2.5 g, 9.0 mmol) obtained in Example (205a). The resulting mixture was stirred at 60°C for 1 hr. To this reaction solution, water was added under ice cooling and then a 3 N hydrochloric acid aqueous solution was further added. The resulting mixture was stirred for 15 min and then neutralized with a saturated sodium carbonate aqueous solution. After extraction with ethyl acetate, the organic layer was washed with water and brine, and then dried with anhydrous magnesium sulfate. The solvent was evaporated under reduced pressure, and the resulting crude product was purified by silica gel column chromatography (Biotage, eluting solvent; hexane/ethyl acetate) to obtain 1.7 g (yield: 64%) of the title compound as a light yellow amorphous form.
¹H-NMR (500 MHz, CDCl₃) δ ppm: 11.16 (1H, brs), 7.96 (1H, dd, J = 2.4, 7.3 Hz), 7.63 (1H, s), 7.56 (1H, m), 7.45 (1H, m), 7.17 (1H, m), 7.09 (1H, m), 7.07 (1H, d, J = 7.8 Hz), 2.67 (3H, d, J = 4.9 Hz), 2.58 (3H, s).
MS(ESI) m/z: 296 (M+H)⁺

### (205c) 2-{4-[4-Fluoro-3-(1H-pyrazol-3-yl)phenyl]-1H-pyrazol-3-yl}-6-methylpyridine

The same reaction as in Example (135b) was carried out using 1-{2-fluoro-5-[3-(6-methylpyridin-2-yl)-1H-pyrazol-4-yl]phenyl}ethanone (0.44 g, 1.5 mmol) obtained in Example (205b) instead of 1-{2-fluoro-5-[2-isopropyl-5-(6-methylpyridin-2-yl)-1H-imidazol-4-yl]phenyl}ethanone obtained in Example (135a). After purification, 0.14 g (yield: 30%) of the title compound was obtained as a white amorphous form.
Melting point: 105 to 108°C
¹H-NMR (500 MHz, CDCl₃) δ ppm: 7.92 (1H, brs), 7.65 (2H, s), 7.43 (1H, m), 7.34 (1H, m), 7.20 (1H, m), 7.15 (1H, d, J = 7.8 Hz), 7.06 (1H, d, J = 7.8 Hz), 6.72 (1H, brs), 2.58 (3H, s).
MS(ESI) m/z: 320 (M+H)⁺.

### (Example 206) 2-{4-[4-Fluoro-3-(1H-imidazol-4-yl)phenyl]-1H-pyrazol-3-yl}-6-methylpyridine (Compound No. 2-608)

The same reaction as in Example 137 was carried out using 1-{2-fluoro-5-[3-(6-methylpyridin-2-yl)-1H-pyrazol-4-yl]phenyl}ethanone (0.46 g, 1.6 mmol) obtained in Example (205b) instead of 1-{2-fluoro-5-[2-isopropyl-5-(6-methylpyridin-2-yl)-1H-imidazol-4-yl]phenyl}ethanone obtained in Example (135a). After purification by silica gel column chromatography (NH, eluting solvent; ethyl acetate : methanol = 9:1), 0.22 g (yield: 43%) of the title compound was obtained as a white solid.
Melting point: 229 to 233°C
¹H-NMR (500 MHz, CD₃OD) δ ppm: 8.00 (1H, d, J = 5.8 Hz), 7.75 (1H, s), 7.62 (1H, m), 7.47 (1H, s), 7.25-7.21 (5H, m), 2.52 (3H, s).
MS(ESI) m/z: 320 (M+H)⁺

### (Example 207) 1-(4-{2-Fluoro-5-[3-(6-methylpyridin-2-yl)-1H-pyrazol-4-yl]phenyl}-1H-imidazol-2-yl)ethane-1,2-diol (Compound No. 2-614)

A 48% hydrogen bromide aqueous solution (0.20 mL, 1.8 mmol) was added to a dimethylsulfoxide solution (1 mL) of 1-{2-fluoro-5-[3-(6-methylpyridin-2-yl)-1H-pyrazol-4-yl]phenyl}ethanone (0.23 g, 0.79 mmol) obtained in Example (205b). The resulting mixture was stirred at 60°C for 2 hr. The reaction solution was cooled to room temperature and then neutralized with saturated aqueous sodium bicarbonate. After extraction with ethyl acetate, the organic layer was washed with water and brine, and then dried with anhydrous magnesium sulfate. The solvent was evaporated under reduced pressure. The resulting crude product was dissolved in ethanol (4 mL), and 28% ammonia water (0.5 mL) and DL-glyceraldehyde dimer (0.20 g, 1.1 mmol) were added thereto. The resulting mixture was stirred at room temperature for 1.5 hr. This reaction solution was concentrated under reduced pressure, and then water was added thereto. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.047 g (yield: 15%) of the title compound as a white solid.
Melting point: 125 to 127°C
¹H-NMR (500 MHz, DMSO-d₆) δ ppm: 13.35 (0.5H, s), 13.13 (0.5H, s), 12.07 (1H, s), 8.25-8.03 (2H, m), 7.65-7.15 (6H, m), 5.55 (1H, m), 4.77 (1H, s), 4.63 (1H, s), 3.69 (1H, m), 3.56 (1H, m), 2.49 (3H, s).
MS(ESI) m/z: 380 (M+H)⁺

### (Example 208) (4-{2-Fluoro-5-[3-(6-methylpyridin-2-yl)-1H-pyrazol-4-yl]phenyl}-1H-imidazol-2-yl)methanol (Compound No. 2-616)

A 48% hydrogen bromide aqueous solution (0.34 mL, 3.0 mmol) was added to a dimethylsulfoxide solution (1 mL) of 1-{2-fluoro-5-[3-(6-methylpyridin-2-yl)-1H-pyrazol-4-yl]phenyl}ethanone (0.30 g, 1.0 mmol) obtained in Example (205b). The resulting mixture was stirred at 60°C for 2 hr. The reaction solution was cooled to room temperature and then neutralized with saturated aqueous sodium bicarbonate. After extraction with ethyl acetate, the organic layer was washed with water and brine, and then dried with anhydrous magnesium sulfate. The solvent was evaporated under reduced pressure. The resulting crude product was dissolved in ethanol (4 mL), and 28% ammonia water (0.5 mL) and (t-butyldimethylsilyloxy)acetaldehyde (0.22 g, 1.3 mmol) were added thereto. The resulting mixture was stirred at room temperature for 1 hr. This reaction solution was concentrated under reduced pressure, and then water was added thereto. After extraction with methylene chloride, the organic layer was separated using an Empore cartridge (GL Science). The solvent was evaporated under reduced pressure, and the resulting crude product was dissolved in tetrahydrofuran (4 mL), and a 3 N hydrochloric acid aqueous solution (1 mL) was added thereto. The resulting mixture was stirred at room temperature for 3.5 hr. The reaction solution was concentrated under reduced pressure and then neutralized with saturated aqueous sodium bicarbonate. To this solution, water and acetonitrile were added. The resulting mixture was purified by high-performance liquid chromatography (GL Science ODS-3, eluting solvent; water : acetonitrile = 95 : 5 to 5 : 95) to obtain 0.097 g (yield: 27%) of the title compound as a white solid.
Melting point: 243 to 246°C
¹H-NMR (500 MHz, DMSO-d₆) δ ppm: 13.34 (0.5H, s), 13.12 (0.5H, s), 12.17 (0.5H, s), 12.12 (0.5H, s), 8.22 (0.5H, dd, J = 2.0, 7.3 Hz), 8.10 (0.5H, dd, J = 2.0, 7.3 Hz), 8.02 (0.5H, s), 7.68-7.62 (1.5H, m), 7.48 (0.5H, d, J = 7.8 Hz), 7.38 (0.5H, m), 7.34 (0.5H, m), 7.21-7.07 (3.5H, m), 5.39 (1H, t, J = 5.9 Hz), 4.49 (2H, d, J = 5.9 Hz), 2.50 (3H, s).
MS(ESI) m/z: 350 (M+H)⁺

### (Example 209) 2-(4-{2-Fluoro-5-[3-(6-methylpyridin-2-yl)-1H-pyrazol-4-yl]phenyl}-1H-imidazol-2-yl)ethanol (Compound No. 2-656)

The same reaction as in Example 208 was carried out using 3-[(t-butyldimethylsilyl)oxy]-1-propanal (0.23 g, 1.2 mmol) instead of (t-butyldimethylsilyloxy)acetaldehyde. After purification, 0.14 g (yield: 36%) of the title compound was obtained as a white solid.
Melting point: 219 to 220°C
¹H-NMR (500 MHz, DMSO-d₆) δ ppm: 13.35 (0.5H, s), 13.13 (0.5H, s), 11.95 (1H, s), 8.18 (0.5H, brs), 8.08 (0.5H, brs), 8.03 (0.5H, s), 7.68-7.62 (1.5H, m), 7.49 (0.5H, m), 7.33-7.30 (1H, m), 7.19-7.08 (3.5H, m), 4.76 (1H, s), 3.70 (2H, d, J = 6.3 Hz), 2.79 (2H, t, J = 6.3 Hz), 2.50 (3H, s).
MS(ESI) m/z: 364 (M+H)⁺

### (Example 210) 1-(4-{2-Fluoro-5-[3-(6-methylpyridin-2-yl)-1H-pyrazol-4-yl]phenyl}-1H-imidazol-2-yl)-2-methylpropan-2-ol (Compound No. 2-670)

### (210a) 3-Hydroxy-3-methylbutanal

Pyridinium chlorochromate (2.6 g, 12 mmol) and Celite (5 g) were sufficiently ground in a mortar and were suspended in methylene chloride (50 mL). To this mixture, a methylene chloride solution (10 mL) of 3-methyl-1,3-butanediol (1.1 g, 10 mmol) was added. The resulting mixture was stirred at room temperature for 3 hr and then filtered. The filtrate was concentrated under reduced pressure. The resulting crude product was purified by silica gel column chromatography (Biotage, eluting solvent; hexane : ethyl acetate = 2 : 3) to obtain 0.47 g (yield: 45%) of the title compound as a colorless oily material.
¹H-NMR (400 MHz, CDCl₃) δ ppm: 9.85 (1H, t, J = 2.0 Hz), 2.63 (2H, d, J = 2.0 Hz), 1.34 (6H, s).

### (210b) 1-(4-{2-Fluoro-5-[3-(6-methylpyridin-2-yl)-1H-pyrazol-4-yl]phenyl}-1H-imidazol-2-yl)-2-methylpropan-2-ol

The same reaction as in Example 207 was carried out using 3-hydroxy-3-methylbutanal (0.23 g. 2.3 mmol) obtained in Example (210a) instead of DL-glyceraldehyde dimer. After purification, 0.093 g (yield: 16%) of the title compound was obtained as a white solid.
Melting point: 136 to 140°C
¹H-NMR (500 MHz, DMSO-d₄) δ ppm: 13.33 (0.5H, s), 13.11 (0.5H, s), 11.81 (0.5H, s), 11.77 (0.5H, s), 8.14 (0.5H, d, J = 6.8 Hz), 8.06 (0.5H, d, J = 6.8 Hz), 8.01 (0.5H, s), 7.67-7.60 (1.5H, m), 7.48 (0.5H, d, J = 7.3 Hz), 7.35-7.32 (1H, m), 7.19-7.11 (3.5H, m), 4.68 (1H, s), 2.71 (2H, s), 2.50 (3H, s), 1.13-1.08 (6H, m).
MS(ESI) m/z: 392 (M+H)⁺.

### (Example 211) 6-{2-Fluoro-5-[3-(6-methylpyridin-2-yl)-1H-pyrazol-4-yl]phenyl}-2,3-dihydroimidazo[2,1-b][1,3]thiazole 1-oxide (Compound No. 2-726)

A 33% hydrogen bromide-acetic acid solution (0.30 mL) and bromine (0.070 mL, 1.4 mmol) were added to an acetic acid solution (5 mL) of 1-{2-fluoro-5-[3-(6-methylpyridin-2-yl)-1H-pyrazol-4-yl]phenyl}ethanone (0.36 g, 1.2 mmol) obtained in Example (205b). The resulting mixture was stirred at 60°C for 2 hr. The reaction solution was concentrated under reduced pressure, and saturated aqueous sodium bicarbonate was added thereto. After extraction with ethyl acetate, the organic layer was washed with water and brine, and then dried with anhydrous magnesium sulfate. The solvent was evaporated under reduced pressure. The resulting crude product was dissolved in ethanol (5 mL), and 2-amino-2-thiazoline (0.088 g, 0.86 mmol) was added thereto. The resulting mixture was heated under reflux for 3 hr and then concentrated under reduced pressure, and saturated aqueous sodium bicarbonate was added thereto. After extraction with ethyl acetate, the organic layer was washed with water and brine, and then dried with anhydrous magnesium sulfate. The solvent was evaporated under reduced pressure. The resulting crude product was dissolved in methanol (4 mL), and water (1 mL) and sodium periodate (0.22 g, 1.0 mmol) were added thereto. The resulting mixture was heated under reflux for 1.5 hr. The reaction solution was concentrated under reduced pressure, and water was added thereto. After extraction with ethyl acetate, the organic layer was washed with water and brine, and then dried with anhydrous magnesium sulfate. The solvent was evaporated under reduced pressure, and the resulting crude product was purified by high-performance liquid chromatography (GL Science ODS-3, eluting solvent; water : acetonitrile = 95 : 5 to 5 : 95) to obtain 0.029 g (yield: 6.2%) of the title compound as a flesh-colored amorphous form. Melting point: 140 to 144°C
¹H-NMR (500 MHz, CDCl₃) δ ppm: 8.27 (1H, dd, J = 2.4, 7.8 Hz), 7.70 (1H, d, J = 3.4 Hz), 7.66 (1H, s), 7.43 (1H, m), 7.29 (1H, m), 7.17-7.13 (2H, m), 7.05 (1H, d, J = 7.8 Hz), 4.88 (1H, m), 4.48 (1H, m), 3.82 (1H, m), 3.73 (1H, m), 2.59 (3H, s). MS(ESI) m/z: 394 (M+H)⁺

### (Example 212) 6-{2-Fluoro-5-[3-(6-methylpyridin-2-yl)-1H-pyrazol-4-yl]phenyl}-2,3-dihydroimidazo[2,1-b][1,3]thiazole 1,1-dioxide (Compound No. 2-728)

Water (1 mL) and sodium periodate (0.12 g, 0.56 mmol) were added to a methanol solution (3 mL) of 6-{2-fluoro-5-[3-(6-methylpyridin-2-yl)-1H-pyrazol-4-yl]phenyl}-2,3-dihydroimidazo[2,1-b][1,3]thiazole 1-oxide (0.016 g, 0.041 mmol) obtained in Example 211. The resulting mixture was heated under reflux for 30 hr. The reaction solution was concentrated under reduced pressure, and water was added thereto. After extraction with methylene chloride, the organic layer was separated using an Empore cartridge (GL Science). The solvent was evaporated under reduced pressure, and the resulting crude product was purified by high-performance liquid chromatography (GL Science ODS-3, eluting solvent; water : acetonitrile = 95 : 5 to 5 : 95) to obtain 0.0024 g (yield: 14%) of the title compound as a white solid. Melting point: 134 to 138°C
¹H-NMR (500 MHz, CDCl₃) δ ppm: 8.28 (1H, dd, J = 2.0, 7.8 Hz), 7.66 (1H, s), 7.56 (1H, d, J = 3.4 Hz), 7.41 (1H, m), 7.31 (1H, m), 7.17-7.13 (2H, m), 7.05 (1H, d, J = 7.8 Hz), 4.61 (2H, t, J = 6.8 Hz), 3.98 (2H, t, J = 6.8 Hz), 2.59 (3H, s).
MS(ESI) m/z: 410 (M+H)⁺

### (Example 213) 4-[2-(4-{2-Fluoro-5-[3-(6-methylpyridin-2-yl)-1H-pyrazol-4-yl]phenyl}-1H-imidazol-2-yl)ethyl]morpholine (Compound No. 2-708)

2-(4-{2-Fluoro-5-[3-(6-methylpyridin-2-yl)-1H-pyrazol-4-yl]phenyl}-1H-imidazol-2-yl)ethanol (0.85 g, 2.3 mmol) obtained in Example 209 was dissolved in N,N-dimethylformamide (10 mL). This reaction solution was cooled to 0°C, and sodium hydride (60%, oil, 0.12 g, 3.0 mmol) and methanesulfonyl chloride (0.22 mL, 2.8 mmol) were added thereto. The resulting mixture was stirred at 0°C for 30 min and then warmed to room temperature and further stirred for 2 hr. To this reaction solution, water was added. After extraction with ethyl acetate, the organic layer was dried with anhydrous sodium sulfate. The solvent was evaporated under reduced pressure. The residue was purified by silica gel column chromatography (Yamazen, eluting solvent: methanol/ethyl acetate) to obtain 0.30 g (29%) of 2-(4-{2-fluoro-5-[3-(6-methylpyridin-2-yl)-1H-pyrazol-4-yl]phenyl}-1H-imidazol-2-yl)ethyl methanesulfonate. This product was dissolved in N,N-dimethylformamide (10 mL), and morpholine (0.30 mL, 3.4 mmol) was added thereto. The resulting mixture was stirred at 100°C for 3 hr. The reaction solution was cooled to room temperature, and then water was added thereto. After extraction with ethyl acetate, the organic layer was dried with anhydrous sodium sulfate. The solvent was evaporated under reduced pressure. The residue was purified by silica gel column chromatography (Yamazen, eluting solvent: methanol/ethyl acetate) and crystallized from acetonitrile to obtain 0.073 g (25%) of the title compound as a white solid. Melting point: 239 to 240°C
¹H-NMR (400 MHz, CDCl₃) δ ppm: 8.17 (1H, brs), 7.65 (1H, s), 7.42-7.38 (2H, m), 7.25-7.10 (3H, m), 7.04 (1H, d, J = 7.4 Hz), 3.81 (4H, t, J = 4.7 Hz), 3.01 (2H, t, J = 6.3 Hz), 2.78 (2H, t, J = 6.3 Hz), 2.62-2.56 (4H, m), 2.57 (3H, s).
MS(ESI) m/z: 433 (M+H)⁺.

### (Example 214) 2-(4-{2-Fluoro-5-[3-(6-methylpyridin-2-yl)-1H-pyrazol-4-yl]phenyl}-1H-imidazol-2-yl)-N,N-dimethylamine (Compound No. 2-706)

### (214a) t-Butyl 4-{3-(1-t-butoxycarbonyl)-2-(2-{[t-butyl(dimethyl)silyl]oxy}ethyl)-1H-imidazol-4-yl}-4-fluorophenyl}-3-(6-methylpyridin-2-yl)-1H-pyrazol-1-carboxylate

1-[2-Fluoro-5-[3-(6-methylpyridin-2-yl)-1H-pyrazol-4-yl]phenyl]ethanone (2.0 g, 6.8 mmol) obtained in Example (205b) was dissolved in dimethylsulfoxide (10 mL), and a 48% bromic acid aqueous solution (2.3 mL, 20 mmol) was added thereto. The resulting mixture was stirred at 60°C for 3 hr. The reaction solution was cooled to room temperature and then neutralized with saturated aqueous sodium bicarbonate. After extraction with ethyl acetate, the organic layer was dried with anhydrous sodium sulfate. The solvent was evaporated under reduced pressure. The residue was dissolved in ethanol (35 mL), and a 28% ammonia aqueous solution (2.5 mL) and 3-[(t-butyldimethylsilyl)oxy]propanal (0.95 g, 5.0 mmol) were added thereto. The mixture was stirred at room temperature for 2 hr. To this reaction solution, water was added. After extraction with ethyl acetate, the organic layer was dried with anhydrous sodium sulfate. The solvent was evaporated under reduced pressure. The residue was purified by silica gel column chromatography (Yamazen, eluting solvent: hexane/ethyl acetate) to obtain 1.43 g (44%) of 2-(4-[3-[2-(2-[(t-butyldimethylsilyl)oxy]ethyl)-1H-imidazol-4-yl]-4-fluorophenyl]-1H-pyrazol-3-yl)-6-methylpyridine. This compound (0.96 g, 2.0 mmol) was dissolved in tetrahydrofuran (20 mL) and N,N-dimethylformamide (5 mL). The resulting mixture was cooled to 0°C. To this reaction solution, di-t-butyl dicarbonate (1.8 g, 8.0 mmol) and sodium hydride (60%, oil, 0.32 g, 8.0 mmol) were added. The resulting mixture was stirred at room temperature for 1 hr and then was heated to 70°C and stirred for 2 hr. The reaction solution was cooled to room temperature, and water was added thereto. After extraction with ethyl acetate, the organic layer was dried with anhydrous sodium sulfate. The solvent was evaporated under reduced pressure. The residue was purified by silica gel column chromatography (Yamazen, eluting solvent: hexane/ethyl acetate) to obtain 1.0 g (yield: 65%) of the title compound as a light yellow solid.
¹H-NMR (400 MHz, CDCl₃) δ ppm: 8.25 (1H, s), 8.21 (1H, dd, J= 2.3, 7.0 Hz), 7.75 (1H, d, J = 3.5 Hz), 7.53 (1H, t, J = 7.8 Hz), 7.44 (1H, d, J = 7.8 Hz), 7.19 (1H, ddd, J = 2.3, 5.1, 8.6 Hz), 7.09 (1H, d, J = 7.8 Hz), 7.01 (1H, d, J = 8.6, 10.9 Hz), 4.02 (2H, t, J = 7.0 Hz), 3.31 (2H, t, J = 7.0 Hz), 2.49 (3H, s), 1.69 (9H, s), 1.65 (9H, s), 0.87 (9H, s), 0.03 (6H, s).

### (214b) t-Butyl 4-{3-(1-t-butoxycarbonyl)-2-(2-{[methanesulfonyl]oxy}ethyl)-1H-imidazol-4-yl}-4-fluorophenyl}-3-(6-methylpyridin-2-yl)-1H-pyrazol-1-carboxylate

t-Butyl 4-{3-(1-t-butoxycarbonyl)-2-(2- {[t-butyl(dimethyl)silyl]oxy}ethyl)-1H-imidazol-4-yl}-4-fluorophenyl}-3-(6-methylpyridin-2-yl)-1H-pyrazol-1-carboxylate (0.94 g, 1.4 mmol) obtained in Example (214a) was dissolved in tetrahydrofuran (10 mL). The resulting mixture was cooled to 0°C, and pyridine (5 mL) and pyridine-hydrogen fluoride (3.4 mL) were added thereto. The resulting mixture was stirred at 0°C for 1 hr and then was warmed to room temperature and stirred for 2 hr. The reaction solution was neutralized with saturated aqueous sodium bicarbonate. After extraction with ethyl acetate, the organic layer was dried with anhydrous sodium sulfate. The solvent was evaporated under reduced pressure. The residue was dissolved in methylene chloride (10 mL), and the mixture was cooled to 0°C. To this reaction solution, triethylamine (0.71 mL, 5.0 mmol) and methanesulfonyl chloride (0.38 mL, 5.0 mmol) were added. The resulting mixture was stirred at 0°C for 2 hr, and then saturated aqueous sodium bicarbonate was added thereto. After extraction with ethyl acetate, the organic layer was dried with anhydrous sodium sulfate. The solvent was evaporated under reduced pressure. The residue was purified by silica gel column chromatography (Yamazen, eluting solvent: hexane/ethyl acetate) to obtain 1.3 g (yield: 91%) of the title compound as a light yellow solid.
¹H-NMR (400 MHz, CDCl₃) δ ppm: 8.21 (1H, s), 8.12 (1H, dd, J= 2.3, 7.4 Hz), 7.74 (1H, d, J = 3.9 Hz), 7.54 (1H, t, J = 7.4 Hz), 7.46 (1H, d, J = 7.4 Hz), 7.21 (1H, ddd, J = 2.3, 5.1, 8.6 Hz), 7.10 (1H, d, J = 7.4 Hz), 7.02 (1H, dd, J = 8.6, 10.6 Hz), 4.68 (2H, t, J = 6.7 Hz), 3.50 (2H, t, J = 6.7 Hz), 2.97 (3H, s), 2.47 (3H, s), 1.69 (9H, s), 1.65 (9H, s).

### (214c) 2-(4-{2-Fluoro-5-[3-(6-methylpyridin-2-yl)-1H-pyrazol-4-yl]phenyl}-1H-imidazol-2-yl)-N,N-dimethylethylamine

t-Butyl 4-{3-(1-t-butoxycarbonyl)-2-(2-{[methanesulfonyl]oxy}ethyl)-1H-imidazol-4-yl}-4-fluorophenyl}-3-(6-methylpyridin-2-yl)-1H-pyrazol-1-carboxylate (0.32 g, 0.50 mmol) obtained in Example (214b) was dissolved in acetonitrile (20 mL), and potassium carbonate (2.8 g, 20 mmol) and dimethylamine hydrochloride (0.82 g, 10 mmol) were added thereto. The resulting mixture was stirred at 70°C for 1 hr. The reaction solution was cooled to room temperature, and water was added thereto. After extraction with ethyl acetate, the organic layer was dried with anhydrous sodium sulfate. The solvent was evaporated under reduced pressure. The residue was purified by silica gel column chromatography (Yamazen, eluting solvent: methanol/ethyl acetate) and crystallized from acetonitrile to obtain 0.12 g (yield: 60%) of the title compound as a white solid.
Melting point: 187 to 188°C
¹H-NMR (400 MHz, CDCl₃) δ ppm: 8.04 (1H, brs), 7.63 (1H, s), 7.39-7.35 (2H, m), 7.15 (1H, ddd, J = 2.0, 4.7, 8.2 Hz), 7.11 (1H, d, J = 7.8 Hz), 7.09 (1H, dd, J = 8.2, 11.0 Hz), 7.00 (1H, d, J = 7.8 Hz), 2.96 (2H, t, J = 5.9 Hz), 2.67 (2H, t, J = 5.9 Hz), 2.56 (3H, s), 2.34 (6H, s).
MS(ESI) m/z: 391 (M+H)⁺

### (Example 215) 5-[6-Fluoro-4'-(methylsulfonyl)-1,1'-biphenyl-3-yl]-4-(6-methylpyridin-2-yl)-1,3-thiazol-2-amine (Compound No. 3-37)

### (215a) 5-(3-Bromo-4-fluorophenyl)-4-(6-methylpyridin-2-yl)-1,3-thiazol-2-amine

2-(3-Bromo-4-fluorophenyl)-1-(6-methylpyridin-2-yl)ethanone (3.0 g, 9.7 mmol) obtained in Example (143a) was dissolved in chloroform (20 mL), and bromine (0.55 mL, 11 mmol) was added thereto at room temperature. The resulting mixture was stirred at 65°C for 7 hr. The reaction solution was cooled to room temperature, and saturated aqueous sodium bicarbonate-5% sodium hydrogen thiosulfate aqueous solution was added thereto. After extraction with methylene chloride, the organic layer was washed with brine and then dried with anhydrous sodium sulfate. The solvent was evaporated under reduced pressure to obtain a white solid.

The obtained white solid was dissolved in ethanol (40 mL), and thiourea (0.89 g, 12 mmol) was added thereto. The resulting mixture was heated under reflux for 3 hr. The reaction solution was cooled to room temperature, and the reaction solvent was evaporated under reduced pressure. The resulting crude product was washed with ethyl acetate-methylene chloride to obtain 1.8 g (yield: 51%) of the title compound as a white solid.
¹H-NMR (400 MHz, CDCl₃) δ ppm: 7.58 (1H, dd, J = 2.3, 6.7 Hz), 7.47 (1H, t, J = 7.8 Hz), 7.21 (1H, d, J = 7.8 Hz), 7.19 (1H, m), 7.03 (1H, d, J = 7.8 Hz), 6.98 (1H, t, J = 8.6 Hz), 5.05 (2H, brs), 2.50 (3H, s).

### (215b) 5-[6-Fluoro-4'-(methylsulfonyl)-1,1'-biphenyl-3-yl]-4-(6-methylpyridin-2-yl)-1,3-thiazol-2-amine

5-(3-Bromo-4-fluorophenyl)-4-(6-methylpyridin-2-yl)-1,3-thiazol-2-amine (0.73 g, 2.0 mmol) obtained in Example (215a) and 4-(methylsulfonyl)phenylboronic acid (0.52 g, 2.6 mmol) were dissolved in 1,2-dimethoxyethane (20 mL), and water (2 mL), tripotassium phosphate n-hydrate (0.85 g, 4.0 mmol), and dichloro[1,1'-bis(diphenylphosphino)ferrocene]palladium(II)-methylene chloride complex (0.16 g, 0.20 mmol) were added thereto. The resulting mixture was stirred at 90°C for 24 hr. The reaction solution was cooled to room temperature, and the reaction solvent was evaporated under reduced pressure. The resulting crude product was purified by silica gel column chromatography (Yamazen, eluting solvent; hexane : ethyl acetate = 30 : 70 to 0 : 100) and then by high-performance liquid chromatography (GL Science ODS-3, eluting solvent; water : acetonitrile = 95 to 5 to 5 :95) to obtain 0.41 g (yield: 46%) of the title compound as a white solid.
Melting point: 202 to 204°C
¹H-NMR (400 MHz, CDCl₃) δ ppm: 7.96 (2H, d, J = 8.6 Hz), 7.61 (2H, d, J = 8.6 Hz), 7.48 (1H, t, J = 7.8 Hz), 7.40 (1H, dd, J = 2.4, 7.4 Hz), 7.32 (1H, ddd, J =2.4, 4.7, 8.6 Hz), 7.25 (1H, d, J = 7.8 Hz), 7.08 (1H, dd, J = 8.6, 10.2 Hz), 7.05 (1H, d, J = 7.8 Hz), 4.98 (2H, brs), 3.08 (3H, s), 2.49 (3H, s).
MS(ESI) m/z: 440 (M+H)⁺.

### (Example 216) 5'-[2-Amino-4-(6-methylpyridin-2-yl)-1,3-thiazol-5-yl]-2'-fluoro-1,1'-biphenyl-4-sulfonamide (Compound No. 3-69)

The same reaction as in Example (215b) was carried out using 4-(4,4,5,5-tetramethyl-1,3,2-dioxaborolan-2-yl)benzenesulfonamide (0.30 g, 1.2 mmol) instead of 4-(methylsulfonyl)phenylboronic acid and using 5-(3-bromo-4-fluorophenyl)-4-(6-methylpyridin-2-yl)-1,3-thiazol-2-amine (0.30 g, 0.82 mmol) obtained in Example (215a). After purification, 0.26 g (yield: 72%) of the title compound was obtained as a white solid.
Melting point: 190 to 192°C
¹H-NMR (400 MHz, CDCl₃) δ ppm: 7.96 (2H, d, J = 8.6 Hz), 7.58 (2H, d, J = 8.6 Hz), 7.50 (1H, t, J = 7.8 Hz), 7.40 (1H, dd, J = 2.4, 7.4 Hz), 7.33 (1H, ddd, J = 2.4, 4.7, 8.6 Hz), 7.25 (1H, d, J = 7.8 Hz), 7.09 (1H, dd, J = 8.6, 10.2 Hz), 7.06 (1H, d, J = 7.8 Hz), 5.01 (2H, brs), 4.86 (2H, brs), 2.49 (3H, s).
MS(ESI) m/z: 441 (M+H)⁺

### (Example 217) 5-[4-Fluoro-3-(1-methyl-1H-pyrazol-4-yl)phenyl]-4-(6-methylpyridin-2-yl)-1,3-thiazol-2-amine (Compound No. 3-126)

The same reaction as in Example (215b) was carried out using 1-methyl-4-(4,4,5,5-tetramethyl-1,3,2-dioxaborolan-2-yl)-1H-pyrazole (0.22 g, 1.2 mmol) instead of 4-(methylsulfonyl)phenylboronic acid and using 5-(3-bromo-4-fluorophenyl)-4-(6-methylpyridin-2-yl)-1,3-thiazol-2-amine (0.73 g, 2.0 mmol) obtained in Example (215a). After purification, 0.080 g (yield: 27%) of the title compound was obtained as a white solid.
Melting point: 153 to 155°C
¹H-NMR (400 MHz, CDCl₃) 7.68 (1H, d, J = 2.4 Hz), 7.62 (1H, s), 7.46 (1H, dd, J = 2.4,7.4 Hz), 7.42 (1H, t, J = 7.8 Hz), 7.15 (1H, d, J = 7.8 Hz), 7.08(1H, ddd, J = 2.4, 4.7, 8.6 Hz), 7.02 (1H, d, J = 7.8 Hz), 7.01 (1H, dd, J = 8.6,10.6 Hz), 5.01 (2H, brs), 3.93 (3H, s), 2.53 (3H, s).
MS(ESI) m/z: 366 (M+H)⁺.

### Test Example 1: Collagen synthesis inhibition test

Collagen synthesis inhibiting activity was evaluated by the following assay.

NRK-49F cells (Dainippon Pharmaceutical) are seeded into a 96-well plate. After becoming confluent, the medium is changed to a serum-free Dulbecco's modified MEM medium (Invitrogen) containing 5 µg/mL insulin, 5 µg/mL transferin, and 5 ng/mL selenious acid. One day later, the medium is changed to a serum-free Dulbecco's modified MEM medium (80 µl) containing 10 ng/mL IGF-II (Sigma) and 50 µg/mL L-ascorbic acid (Sigma), and a ten times concentration of a test compound (10µl) is added thereto. Thirty minutes later, 25 ng/mL TGF-β (R&D System: 10 µL) is added to the medium, and the cells are cultured for about 44 hr. Twenty four hours before the termination of the culturing, ³H-labeled proline (Amersham: 1 µCi) is added so as to be taken into collagen in the cells. When the culturing is terminated, 30% trichloroacetic acid (50 µL) is added to the medium. After leaving for 20 min, proteins are precipitated by centrifugation at 3000 rpm for 10 min. The supernatant is removed, and the residue is twice washed with 200 µL of a washing solution (ethanol : ether = 3 : 1). The precipitate is air-dried, and the washing solution is sufficiently removed. After addition of 60 µL of a collagenase solution (2 mg/mL collagenase VII (Sigma), 50 mM Tris-HCl (pH 7.4), 5 mM CaCl₂), the resulting mixture is treated at 37°C for 2 hr to decompose collagen. Then, 90 µL of 15% trichloroacetic acid and 5% tannic acid are added thereto. The resulting mixture is left for 20 min and then centrifuged at 3000 rpm for 10 min. The supernatant (50 µL) is transferred to LumaPlate (PerkinElmer Lifescience) and is air-dried. Then, the amount of ³H is measured by a Top Count. Table 4 shows IC₅₀ values which are the concentrations of tested compounds at which each of the compounds inhibits collagen synthesis by 50%, provided that the collagen synthesis when TGF-β is not added is 100% inhibition and that the collagen synthesis when a test compound is not added is 0% inhibition.

**(Table 4)**

| Tested compound | Collagen synthesis inhibiting activity IC₅₀ (µM) |
|---|---|
| Example 17 | 0.35 |
| Example 48 | 1.49 |
| Example 65 | 0.52 |
| Example 109 | 0.13 |
| Example 110 | 0.05 |
| Example 112 | 0.03 |
| Example 117 | 1.17 |
| Example 121 | 0.01 |
| Example 125 | 0.23 |
| Example 127 | 0.79 |
| Example 130 | 0.18 |
| Example 137 | 0.07 |
| Example 146 | 0.08 |
| Example 156 | 0.10 |
| Example 160 | 0.19 |
| Example 161 | 0.10 |
| Example 162 | 0.03 |
| Example 172 | 0.02 |
| Example 177 | 0.05 |
| Example 179 | 0.04 |
| Example 181 | 0.02 |
| Example 189 | 0.02 |
| Example 190 | 0.04 |
| Example 194 | 0.01 |
| Example 195 | 0.01 |
| Example 202 | 0.29 |
| Example 206 | 0.01 |
| Example 209 | 0.05 |
| Example 212 | 0.11 |
| Example 217 | 0.01 |

The above-mentioned results demonstrate that the compounds according to the present invention have an excellent collagen synthesis inhibiting effect.

Drug Formulation Example 1: Capsules
Compound of Example 15 or 16: 50 mg
Lactose: 128 mg
Cornstarch: 70 mg
Magnesium stearate: 2 mg
250 mg
The above-prescribed powders are mixed and filtered though a 60 mesh sieve. This powder mixture is filled into a No. 3 gelatin capsule of 250 mg to form a capsule.

Drug Formulation Example 2: Tablets
Compound of Example 15 or 16: 50 mg
Lactose: 126 mg
Cornstarch: 23 mg
Magnesium stearate: 1 mg
200 mg
The above-prescribed powders are mixed. The powder mixture is wet granulated using cornstarch paste, dried and made into tablets, each weighing 200 mg, with a tableting machine. The tablets may be coated with sugar if necessary.

### Industrial Applicability

The biaryl derivative having a structure represented by General Formula (I) or a pharmacologically acceptable salt thereof according to the present invention functions as an excellent non-peptide inhibitor that strongly and selectively inhibits collagen synthesis and, therefore, is useful as a therapeutic or preventive drug for diseases that are mainly caused by fibrosis (for example, chronic renal disease, acute renal disease, diabetic renal disorder, liver fibrosis, lung fibrosis, or skin fibrosis).

## Claims

1. A biaryl derivative having General Formula (I) or a pharmacologically acceptable salt thereof, 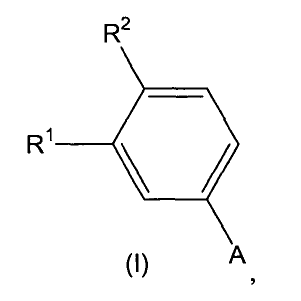 wherein
R¹ represents a C₆-C₁₀ aryl group which is substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, a halogen atom, and a halogenosulfonyl group; or a heterocyclic group which may be substituted with one to three group(s) each independently selected from the group consisting of a group defined by formula R-L-, a di-(C₁-C₆ alkyl)amino group, a di-(C₁-C₆ alkyl)aminosulfonyl group, a di-(C₁-C₆ alkyl)aminocarbonylamino group, a hydroxyaminocarbonyl group, a halogen atom, and an oxo group,
R represents a hydrogen atom, a C₁-C₆ alkyl group, a C₁-C₆ halogenated alkyl group, a C₃-C₆ cycloalkyl group, a C₆-C₁₀ aryl group which may be substituted with one to three group(s) each independently selected from substituent group *a*, a heterocyclic group which may be substituted with one group selected from substituent group *a*, a C₁-C₆ alkyl group which is substituted with one group selected from substituent group *b*, a cyano group, a nitro group, a C₁-C₆ alkyl group which is substituted with two hydroxy groups, or a C₁-C₆ alkyl group which is substituted with one to three halogen atom(s) and one hydroxy group,
L represents a single bond, an oxygen atom, an amino group, a sulfur atom, a sulfinyl group, a sulfonyl group, a carbonyl group, an oxycarbonyl group, a carbonyloxy group, an aminocarbonyl group, a carbonylamino group, an aminosulfonyl group, a sulfonylamino group, an aminocarbonylamino group, an aminosulfonylamino group, a hydrazinocarbonylamino group, or an aminocarbonylhydrazino group,
provided that the case in which R represents a hydrogen atom and L represents a single bond is excluded,
R² represents a hydrogen atom, a C₁-C₆ alkyl group, or a halogen atom,
A represents a group defined by formula (II), (III), or (IV) 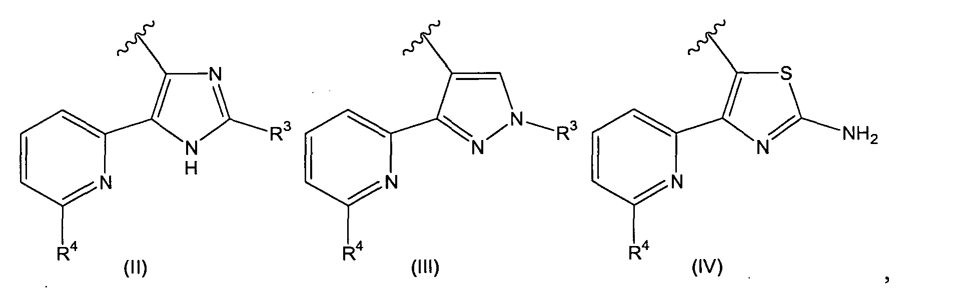
(wherein R³ represents a hydrogen atom, a C₁-C₆ alkyl group, a C₃-C₆ cycloalkyl group, or a C₁-C₆ alkyl group which is substituted with a C₃-C₆ cycloalkyl group, and R⁴ represents a hydrogen atom, a C₁-C₆ alkyl group, or a C₃-C₆ cycloalkyl group),
substituent group *a* represents the group consisting of a halogen atom, a C₁-C₆ alkyl group, and a C₁-C₆ halogenated alkyl group, and
substituent group *b* represents the group consisting of a hydroxy group, an amino group, a carbamoyl group, a C₃-C₆ cycloalkyl group, a C₁-C₆ alkoxy group, a C₂-C₇ alkylcarbonyloxy group, a di-(C₁-C₆ alkyl)amino group, a mono-C₁-C₆ alkylsulfonylamino group, a C₆-C₁₀ aryl group which may be substituted with one to three group(s) each independently selected from substituent group *a,* a heterocyclic group which may be substituted with one group selected from substituent group *a*, a heterocyclic carbonyl group which may be substituted with one group selected from substituent group *a,* a heterocyclic amino group which may be substituted with one group selected from substituent group *a*, a heterocyclic group which is substituted with one oxo group, a C₁-C₆ alkylthio group, a C₁-C₆ alkylsulfinyl group, and a C₁-C₆ alkylsulfonyl group.

2. The biaryl derivative or the pharmacologically acceptable salt thereof according to claim 1, wherein
R represents a hydrogen atom, a C₁-C₆ alkyl group, a heterocyclic group which may be substituted with one group selected from substituent group *a*, or a C₁-C₆ alkyl group which is substituted with one group selected from substituent group *b.*

3. The biaryl derivative or the pharmacologically acceptable salt thereof according to claim 1, wherein
R represents a hydrogen atom, a C₁-C₄ alkyl group, a partially or completely reduced 6-membered heterocyclic group, a C₁-C₆ alkyl group which is substituted with one partially or completely reduced 5-membered heterocyclic group, or a C₁-C₆ alkyl group which is substituted with one hydroxy group.

4. The biaryl derivative or the pharmacologically acceptable salt thereof according to claim 1, wherein
R represents a hydrogen atom, a methyl group, an ethyl group, a hydroxymethyl group, a 1-hydroxy-1-methylethyl group, or a 2-hydroxyethyl group.

5. The biaryl derivative or the pharmacologically acceptable salt thereof according to claim 1, wherein
R represents a hydrogen atom, a C₁-C₄ alkyl group, a C₁-C₄ halogenated alkyl group, a C₃-C₄ cycloalkyl group, a phenyl group which may be substituted with one group selected from substituent group *a*, a partially or completely reduced 6-membered heterocyclic group which may be substituted with one group selected from substituent group *a*, a C₁-C₄ alkyl group which is substituted with one group selected from substituent group *b,* or a nitro group.

6. The biaryl derivative or the pharmacologically acceptable salt thereof according to claim 1, wherein
R represents a hydrogen atom; a methyl group; an ethyl group; a trifluoromethyl group; a cyclopropyl group; a morpholino, piperazinyl, or tetrahydropyranyl group which may be substituted with one group selected from a fluorine atom, a chlorine atom, a methyl group, and an ethyl group; or a C₁-C₄ alkyl group which is substituted with one group selected from a hydroxy group and a C₂-C₇ alkylcarbonyloxy group.

7. The biaryl derivative or the pharmacologically acceptable salt thereof according to claim 1, wherein
R represents a hydrogen atom, a methyl group, an ethyl group, a cyclopropyl group, a 4-morpholino group, a 4-methyl-1-piperazinyl group, a 4-tetrahydropyranyl group, a 1-hydroxy-1-methylethyl group, a 1-acetoxy-1-methylethyl group, or a 2-hydroxyethyl group.

8. The biaryl derivative or the pharmacologically acceptable salt thereof according to any one of claims 1 to 7, wherein
L represents a single bond, an oxygen atom, an amino group, a sulfur atom, a sulfinyl group, a sulfonyl group, a carbonyl group, an oxycarbonyl group, an aminocarbonyl group, a carbonylamino group, an aminosulfonyl group, a sulfonylamino group, or an aminocarbonylamino group.

9. The biaryl derivative or the pharmacologically acceptable salt thereof according to any one of claims 1 to 7, wherein
L represents an oxygen atom, an amino group, a sulfur atom, a sulfinyl group, a sulfonyl group, a carbonyl group, an aminocarbonyl group, a carbonylamino group, an aminosulfonyl group, a sulfonylamino group, or an aminocarbonylamino group.

10. The biaryl derivative or the pharmacologically acceptable salt thereof according to any one of claims 1 to 7, wherein
L represents an oxygen atom, an amino group, a sulfur atom, a sulfinyl group, a sulfonyl group, an aminocarbonyl group, an aminosulfonyl group, a sulfonylamino group, or an aminocarbonylamino group.

11. The biaryl derivative or the pharmacologically acceptable salt thereof according to any one of claims 1 to 7, wherein
L represents a single bond, an oxygen atom, a sulfonyl group, an aminocarbonyl group, or an aminosulfonyl group.

12. The biaryl derivative or the pharmacologically acceptable salt thereof according to any one of claims 1 to 11, wherein
R¹ represents a C₆-C₁₀ aryl group which is substituted with one to three group(s) each independently selected from the group consisting of a group defined by formula R-L- and a di-(C₁-C₆ alkyl)amino group; or a heterocyclic group which may be substituted with one to three group(s) each independently selected from a group defined by formula R-L-.

13. The biaryl derivative or the pharmacologically acceptable salt thereof according to any one of claims 1 to 11, wherein
R¹ represents a C₆-C₁₀ aryl group which is substituted with one group selected from the group consisting of a group defined by formula R-L- and a di-(C₁-C₆ alkyl)amino group; or a 5-membered aromatic heterocyclic, 6-membered aromatic heterocyclic, or fused bicyclic heterocyclic group which may be substituted with one group selected from a group defined by formula R-L-.

14. The biaryl derivative or the pharmacologically acceptable salt thereof according to any one of claims 1 to 11, wherein
R¹ represents a phenyl group of which the 4-position is substituted with one group selected from the group consisting of a group defined by formula R-L- and a dimethylamino group; or a thienyl, pyrrolyl, pyrazolyl, imidazolyl, pyridyl, or 1,1-dioxido-2,3-dihydro-1-benzothienyl group which may be substituted with one group selected from a group defined by formula R-L-.

15. The biaryl derivative or the pharmacologically acceptable salt thereof according to any one of claims 1 to 11, wherein
R¹ represents a 4-methylsulfonylphenyl group, a 4-aminosulfonylphenyl group, a 4-methylaminosulfonylphenyl group, a 4-(4-morpholino)aminocarbonylphenyl group, a 4-[2-(1-pyrrolidinyl)ethoxy]phenyl group, a 4-dimethylaminosulfonylphenyl group, a 5 -amino sulfonyl-2-thienyl group, a 2-pyrrolyl group, a 3-pyrrolyl group, a 4-pyrazolyl group, a 1-methyl-4-pyrazolyl group, a 1-ethyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 2-(2-hydroxyethyl)-5-imidazolyl group, a 5-hydroxymethyl-2-pyridyl group, a 5-(1-hydroxy-1-methylethyl)-2-pyridyl group, a 5-(4-morpholino)-2-pyridyl group, or a 1,1-dioxido-2,3-dihydro-1-benzothien-5-yl group.

16. The biaryl derivative or the pharmacologically acceptable salt thereof according to any one of claims 1 to 11, wherein
R¹ represents a 4-methylsulfonylphenyl group, a 4-aminosulfonylphenyl group, a 4-methylaminosulfonylphenyl group, a 5-aminosulfonyl-2-thienyl group, a 3-pyrrolyl group, a 1-methyl-4-pyrazolyl group, a 1-(2-hydroxyethyl)-4-pyrazolyl group, a 1-methyl-4-imidazolyl group, a 4-imidazolyl group, a 2-hydroxymethyl-5-imidazolyl group, a 2-(1-hydroxy-1-methylethyl)-5-imidazolyl group, a 5-(1-hydroxy-1-methylethyl)-2-pyridyl group, or a 1,1-dioxido-2,3-dihydro-1-benzothien-5-yl group.

17. The biaryl derivative or the pharmacologically acceptable salt thereof according to any one of claims 1 to 11, wherein
R¹ represents a C₆-C₁₀ aryl group which is substituted with one or two group(s) selected from the group consisting of a group defined by formula R-L-, a di-(C₁-C₄ alkyl)amino group, a di-(C₁-C₄ alkyl)aminosulfonyl group, a hydroxyaminocarbonyl group, and a halogen atom; or a heterocyclic group which may be substituted with one group selected from the group consisting of a group defined by formula R-L-, a di-(C₁-C₆ alkyl)amino group, a di-(C₁-C₆ alkyl)aminosulfonyl group, a hydroxyaminocarbonyl group, and a halogen atom.

18. The biaryl derivative or the pharmacologically acceptable salt thereof according to any one of claims 1 to 11, wherein
R¹ represents a phenyl group of which the 4- or 3-position is substituted with one group selected from the group consisting of a group defined by formula R-L-, a di-(C₁-C₂ alkyl)aminosulfonyl group, and a halogen atom; or a thienyl, pyrrolyl, furyl, or pyridyl group which may be substituted with one group selected from the group consisting of a group defined by formula R-L-, a di-(C₁-C₂ alkyl)aminosulfonyl group, and a halogen atom.

19. The biaryl derivative or the pharmacologically acceptable salt thereof according to any one of claims 1 to 11, wherein
R¹ represents a phenyl group of which the 4-position is substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group; or a thienyl, pyrrolyl, furyl, or pyridyl group which may be substituted with one group selected from the group consisting of a fluorine atom, a methyl group, a nitro group, a methoxy group, an amino group, a methylthio group, a methylsulfinyl group, a methylsulfonyl group, an ethylsulfonyl group, a methoxycarbonyl group, a carbamoyl group, a (2-hydroxyethyl)aminocarbonyl group, an acetylamino group, a (1-hydroxy-1-methylethyl)carbonylamino group, a (1-acetoxy-1-methylethyl)carbonylamino group, an aminosulfonyl group, a methylaminosulfonyl group, a dimethylaminosulfonyl group, a methylsulfonylamino group, an ethylsulfonylamino group, a cyclopropylsulfonylamino group, a (4-morpholino)sulfonyl group, a (4-methyl-1-piperazinyl)sulfonyl group, a (4-morpholino)carbonyl group, a (4-morpholino)aminocarbonyl group, a (4-methyl-1-piperazinyl)aminocarbonyl group, a (4-tetrahydropyranyl)aminocarbonyl group, a (1-methyl-4-piperidino)aminocarbonyl group, a (4-morpholino)aminocarbonylamino group, and a (4-methyl-1-piperazinyl)aminocarbonylamino group.

20. The biaryl derivative or the pharmacologically acceptable salt thereof according to any one of claims 1 to 11, wherein
R¹ represents a 4-methoxyphenyl group, a 4-aminophenyl group, a 4-methylthiophenyl group, a 4-methylsulfinylphenyl group, a 4-methylsulfonylphenyl group, a 4-ethylsulfonylphenyl group, a 4-acetylaminophenyl group, a 4-(1-hydroxy-1-methylethyl)carbonylaminophenyl group, a 4-(1-acetoxy-1-methylethyl)carbonylaminophenyl group, a 4-carbamoylphenyl group, a 3-carbamoylphenyl group, a 4-(2-hydroxyethyl)aminocarbonylphenyl group, a 4-methylsulfonylaminophenyl group, a 4-ethylsulfonylaminophenyl group, a 4-cyclopropylsulfonylaminophenyl group, a 4-aminosulfonylphenyl group, a 4-methylaminosulfonylphenyl group, a 4-(4-morpholino)carbonylphenyl group, a 4-(4-morpholino)sulfonylphenyl group, a 4-(4-morpholino)aminocarbonylphenyl group, a 4-(4-methyl-1-piperazinyl)aminocarbonylphenyl group, a 4-(4-methyl-1-piperazinyl)sulfonylphenyl group, a 4-(4-tetrahydropyranyl)aminocarbonylphenyl group, a 4-(1-methyl-4-piperidino)aminocarbonylphenyl group, a 4-(4-morpholino)aminocarbonylaminophenyl group, a 4-(4-methyl-1-piperazinyl)aminocarbonylaminophenyl group, a 3-thienyl group, a 2-pyrrolyl group, a 3-furyl group, a 5-carbamoyl-2-pyridyl group, a 2-methoxy-5-pyridyl group, or a 4-pyridyl group.

21. The biaryl derivative or the pharmacologically acceptable salt thereof according to any one of claims 1 to 11, wherein
R¹ represents a 4-methoxyphenyl group, a 4-aminophenyl group, a 4-methylthiophenyl group, a 4-methylsulfinylphenyl group, a 4-methylsulfonylphenyl group, a 4-methylsulfonylaminophenyl group, a 4-ethylsulfonylaminophenyl group, a 4-cyclopropylsulfonylaminophenyl group, a 4-aminosulfonylphenyl group, a 4-(4-morpholino)aminocarbonylphenyl group, a 4-(4-methyl-1-piperazinyl)aminocarbonylphenyl group, a 4-(4-tetrahydropyranyl)aminocarbonylphenyl group, a 4-(1-methyl-4-piperidino)aminocarbonylphenyl group, a 4-(4-morpholino)aminocarbonylaminophenyl group, a 4-(4-methyl-1-piperazinyl)aminocarbonylaminophenyl group, a 3-thienyl group, a 2-pyrrolyl group, or a 5-carbamoyl-2-pyridyl group.

22. The biaryl derivative or the pharmacologically acceptable salt thereof according to any one of claims 1 to 21, wherein
R² represents a hydrogen atom, a methyl group, a fluorine atom, or a chlorine atom.

23. The biaryl derivative or the pharmacologically acceptable salt thereof according to any one of claims 1 to 21, wherein
R² represents a hydrogen atom or a fluorine atom.

24. The biaryl derivative or the pharmacologically acceptable salt thereof according to any one of claims 1 to 21, wherein
R² represents a fluorine atom.

25. The biaryl derivative or the pharmacologically acceptable salt thereof according to any one of claims 1 to 24, wherein
A represents a group defined by the aforementioned formula (II).

26. The biaryl derivative or the pharmacologically acceptable salt thereof according to any one of claims 1 to 24, wherein
A represents a group defined by the aforementioned formula (III).

27. The biaryl derivative or the pharmacologically acceptable salt thereof according to any one of claims 1 to 26, wherein
R³ represents a C₁-C₆ alkyl group or a C₃-C₆ cycloalkyl group.

28. The biaryl derivative or the pharmacologically acceptable salt thereof according to any one of claims 1 to 26, wherein
R³ represents an isopropyl group, an isobutyl group, or a cyclopropyl group.

29. The biaryl derivative or the pharmacologically acceptable salt thereof according to any one of claims 1 to 26, wherein
R³ represents an isopropyl group.

30. The biaryl derivative or the pharmacologically acceptable salt thereof according to any one of claims 1 to 26, wherein
R³ represents a hydrogen atom.

31. The biaryl derivative or the pharmacologically acceptable salt thereof according to any one of claims 1 to 30, wherein
R⁴ represents a hydrogen atom or a methyl group.

32. The biaryl derivative or the pharmacologically acceptable salt thereof according to any one of claims 1 to 30, wherein
R⁴ represents a methyl group.

33. The biaryl derivative or the pharmacologically acceptable salt thereof according to claim 1, being
2-{4-[4-fluoro-3-(1H-pyrrol-3-yl)phenyl]-2-isopropyl-1H-imidazol-5-yl}-6-methylpyridine,
2-{5-[4-fluoro-3-(1-methyl-1H-pyrazol-4-yl)phenyl]-2-isopropyl-1H-imidazol-4-yl}-6-methylpyridine,
2-{4-[4-fluoro-3-(1H-imidazol-4-yl)phenyl]-2-isopropyl-1H-imidazol-5-yl}-6-methylpyridine,
2-{4-[4-fluoro-3-(1-methyl-1H-imidazol-4-yl)phenyl]-2-isopropyl-1H-imidazol-5-yl} -6-methylpyridine,
2-{4-[6-fluoro-4'-(methylsulfbnyl)-1,1'-biphenyl-3-yl]-1H-pyrazol-3-yl}-6-methylpyridine,
2'-fluoro-5'-[3-(6-methylpyridin-2-yl)-1H-pyrazol-4-yl]-1,1'-biphenyl-4-sulfonamide,
2'-fluoro-N-methyl-5'-[3-(6-methylpyridin-2-yl)-1H-pyrazol-4-yl]-1,1'-biphenyl-4-sulfonamide,
2-{4-[4-fluoro-3-(1-methyl-1H-pyrazol-4-yl)phenyl]-1H-pyrazol-3-yl}-6-methylpyridine,
(4-{2-fluoro-5-[3-(6-methylpyridin-2-yl)-1H-pyrazol-4-yl]phenyl}-1H-imidazol-2-yl)methanol,
5-{2-fluoro-5-[3-(6-methylpyridin-2-yl)-1H-pyrazol-4-yl]phenyl}thiophene-2-sulfonamide,
2-(6-{2-fluoro-5-[3-(6-methylpyridin-2-yl)-1H-pyrazol-4-yl]phenyl}pyridin-3-yl)propan-2-ol,
2-(4-{2-fluoro-5-[3-(6-methylpyridin-2-yl)-1H-pyrazol-4-yl]phenyl}-1H-imidazol-2-yl)propan-2-ol,
2-(4-{2-fluoro-5-[3-(6-methylpyridin-2-yl)-1H-pyrazol-4-yl]phenyl}-1H-pyrazol-1-yl)ethanol,
2-{4-[4-fluoro-3-(1-methyl-1H-imidazol-4-yl)phenyl]-1H-pyrazol-3-yl}-6-methylpyridine, or
2-{4-[3-(1,1-dioxido-2,3-dihydro-1-benzothien-5-yl)-4-fluorophenyl]-1H-pyrazol-3-yl}-6-methylpyridine.

34. The biaryl derivative or the pharmacologically acceptable salt thereof according to claim 1, being
2-{4-[6-fluoro-4'-(methylsulfonyl)-1,1'-biphenyl-3-yl]-2-isopropyl-1H-imidazol-5-yl}-6-methylpyridine,
N-{2'-fluoro-S'-[2-isopropyl-5-(6-methylpyridin-2-yl)-1H-imidazol-4-yl]-1,1'-biphenyl-4-yl}cyclopropylsulfonamide,
N-(morpholin-4-yl)-4-{2-fluoro-5-[2-isopropyl-5-(6-methylpyridin-2-yl)-1H-imidazol-4-yl]-phenyl}benzamide,
2'-fluoro-5'-[2-isopropyl-5-(6-methylpyridin-2-yl)-1H-imidazol-4-yl]-N-(4-methylpiperazin-1-yl)-1,1'-biphenyl-4-carboxyamide,
N-{2'-fluoro-5'-[2-isopropyl-5-(6-methylpyridin-2-yl)-1H-imidazol-4-yl]-1,1'-biphenyl-4-yl}-N'-(4-methylpiperazin-1-yl)urea,
N-(tetrahydropyran-4-yl)-4- {2-fluoro-5-[2-isopropyl-5-(6-methylpyridin-2-yl)-1H-imidazol-4-yl]phenyl}benzamide,
N- {2'-fluoro-5'-[2-isopropyl-5-(6-methylpyridin-2-yl)-1H-imidazol-4-yl]-1,1'-biphenyl-4-yl}-N'-morpholin-4-ylurea, or
2'-fluoro-5'-[2-isopropyl-5-(6-methylpyridin-2-yl)-1H-imidazol-4-yl]-N-(1-methylpiperidin-4-yl)-1,1'-biphenyl-4-carboxyamide.

35. The biaryl derivative or the pharmacologically acceptable salt thereof according to claim 1, being
2- {2-isopropyl-4-[4'-(methylsulfonyl)-1,1'-biphenyl-3-yl]-1H-imidazol-5-yl} -6-methylpyridine,
N-{3'-[2-isopropyl-5-(6-methylpyridin-2-yl)-1H-imidazol-4-yl]-1,1'-biphenyl-4-yl}cyclopropylsulfonamide,
N-(4-methylpiperazin-1-yl)-4-{3-[2-isopropyl-5-(6-methylpyridin-2-yl)-1H-imidazol-4-yl]phenyl}benzamide,
N-{3'-[2-isopropyl-5-(6-methylpyridin-2-yl)-1H-imidazol-4-yl]-1,1'-biphenyl-4-yl}-N'-(methylpiperazin-1-yl)urea,
N-{3'-[2-isopropyl-5-(6-methylpyridin-2-yl)-1H-imidazol-4-yl]-1,1'-biphenyl-4-yl}-N'-morpholin-4-ylurea, or
3'-[2-isopropyl-5-(6-methylpyridin-2-yl)-1H-imidazol-4-yl]-N-(1-methylpiperidin-4-yl)-1,1'-biphenyl-4-carboxyamide.

36. A pharmaceutical composition containing a biaryl derivative or a pharmacologically acceptable salt thereof according to any one of claims 1 to 35 as an active ingredient.

37. A pharmaceutical composition containing a biaryl derivative or a pharmacologically acceptable salt thereof according to any one of claims 1 to 35 as an active ingredient, which is for suppressing production of an extracellular matrix in glomerular cells.

38. A pharmaceutical composition containing a biaryl derivative or a pharmacologically acceptable salt thereof according to any one of claims 1 to 35 as an active ingredient, which is for suppressing production of collagen in glomerular cells.

39. A pharmaceutical composition containing a biaryl derivative or a pharmacologically acceptable salt thereof according to any one of claims 1 to 35 as an active ingredient, which is for suppressing production of an extracellular matrix in liver stellate cells.

40. A pharmaceutical composition containing a biaryl derivative or a pharmacologically acceptable salt thereof according to any one of claims 1 to 35 as an active ingredient, which is for suppressing production of collagen in liver stellate cells.

41. A pharmaceutical composition containing a biaryl derivative or a pharmacologically acceptable salt thereof according to any one of claims 1 to 35 as an active ingredient, which is for suppressing production of an extracellular matrix in lung fibroblasts.

42. A pharmaceutical composition containing a biaryl derivative or a pharmacologically acceptable salt thereof according to any one of claims 1 to 35 as an active ingredient, which is for suppressing production of collagen in lung fibroblasts.

43. A pharmaceutical composition containing a biaryl derivative or a pharmacologically acceptable salt thereof according to any one of claims 1 to 35 as an active ingredient, which is for suppressing production of an extracellular matrix in skin fibroblasts.

44. A pharmaceutical composition containing a biaryl derivative or a pharmacologically acceptable salt thereof according to any one of claims 1 to 35 as an active ingredient, which is for suppressing production of collagen in skin fibroblasts.

45. A pharmaceutical composition containing a biaryl derivative or a pharmacologically acceptable salt thereof according to any one of claims 1 to 35 as an active ingredient, which is for prevention and/or treatment of chronic renal disease, acute renal disease, diabetic renal disorder, or any renal disease mainly caused by fibrosis.

46. A pharmaceutical composition containing a biaryl derivative or a pharmacologically acceptable salt thereof according to any one of claims 1 to 35 as an active ingredient, which is for prevention and/or treatment of liver fibrosis.

47. A pharmaceutical composition containing a biaryl derivative or a pharmacologically acceptable salt thereof according to any one of claims 1 to 35 as an active ingredient, which is for prevention and/or treatment of lung fibrosis.

48. A pharmaceutical composition containing a biaryl derivative or a pharmacologically acceptable salt thereof according to any one of claims 1 to 35 as an active ingredient, which is for prevention and/or treatment of general scleroderma, local scleroderma, keloid, discoid lupus erythematosus, or any skin disease mainly caused by fibrosis.

49. A pharmaceutical composition containing a biaryl derivative or a pharmacologically acceptable salt thereof according to any one of claims 1 to 35 as an active ingredient, which is for prevention and/or treatment of skin fibrosis.

50. Use of the biaryl derivative or the pharmacologically acceptable salt thereof according to any one of claims 1 to 35 for the manufacture of a pharmaceutical composition.

51. The use according to claim 50, wherein the pharmaceutical composition is one for prevention and/or treatment of chronic renal disease, acute renal disease, diabetic renal disorder, or any renal disease mainly caused by fibrosis.

52. The use according to claim 50, wherein the pharmaceutical composition is one for prevention and/or treatment of liver fibrosis.

53. The use according to claim 50, wherein the pharmaceutical composition is one for prevention and/or treatment of lung fibrosis.

54. The use according to claim 50, wherein the pharmaceutical composition is one for prevention and/or treatment of general scleroderma, local scleroderma, keloid, discoid lupus erythematosus, or any skin disease mainly caused by fibrosis.

55. The use according to claim 50, wherein the pharmaceutical composition is one for prevention and/or treatment of skin fibrosis.

56. A method of preventing and/or treating a disease by administering a pharmaceutically effective dose of a biaryl derivative or a pharmacologically acceptable salt thereof according to any one of claims 1 to 35 to a warm-blooded animal.

57. The method according to claim 56, wherein the disease is chronic renal disease, acute renal disease, diabetic renal disorder, or any renal disease mainly caused by fibrosis.

58. The method according to claim 56, wherein the disease is liver fibrosis.

59. The method according to claim 56, wherein the disease is lung fibrosis.

60. The method according to claim 56, wherein the disease is general scleroderma, local scleroderma, keloid, discoid lupus erythematosus, or any skin disease mainly caused by fibrosis.

61. The method according to claim 56, wherein the disease is skin fibrosis.

62. The method according to any one of claims 56 to 61, wherein the warm-blooded animal is a human.
